# EUROPEAN PATENT SPECIFICATION

(11) **EP 2 952 503 B1**
(45) Date of publication and mention of the grant of the patent: **23.10.2019**
(21) Application number: 14746481.2
(22) Date of filing: 30.01.2014
(51) Int. Cl.: C07D 405/14, C07D 311/80, C07D 221/12, C07D 221/18, C07D 223/18, C07D 225/08, C07D 335/10, C07D 401/04, C07D 237/36, C07D 405/04, C07D 413/04, C07D 241/44, C07D 243/04, C07D 243/12, C07D 243/38, C07D 471/04, C07D 471/06, C07D 471/10, C07D 487/04, C07D 491/06

(54) **CARBOXYLIC ACID DERIVATIVES AS HIV REPLICATION INHIBITOR**
CARBONSÄUREDERIVATE ALS HIV-REPLIKATIONSHEMMSTOFFE
DÉRIVÉS D'ACIDES CARBOXILIQUES EN TANT QU'INHIBITEURS DE LA RÉPLICATION DU VIH

(30) Priority: 31.01.2013 JP 2013016433; 29.03.2013 JP 2013071415; 28.06.2013 JP 2013135926; 30.09.2013 JP 2013202877
(43) Date of publication of application: 09.12.2015
(73) Proprietor: Shionogi & Co., Ltd., Osaka-shi, Osaka 541-0045 (JP)
(72) Inventor: TOMITA, Kenji, Toyonaka-shi Osaka 561-0825 (JP); TAODA, Yoshiyuki, Toyonaka-shi Osaka 561-0825 (JP); IWAKI, Tsutomu, Toyonaka-shi Osaka 561-0825 (JP); KAWASUJI, Takashi, Toyonaka-shi Osaka 561-0825 (JP); AKIYAMA, Toshiyuki, Toyonaka-shi Osaka 561-0825 (JP); SUGIYAMA, Shuichi, Toyonaka-shi Osaka 561-0825 (JP); TAMURA, Yoshinori, Toyonaka-shi Osaka 561-0825 (JP); IWATSU, Masafumi, Toyonaka-shi Osaka 561-0825 (JP)
(74) Representative: Vossius & Partner Patentanwälte Rechtsanwälte mbB
(86) International application number: PCT/JP2014/052030
(87) International publication number: WO 2014/119636

(56) References cited:
- EP-A1- 2 511 273
- WO-A1-2012/003497
- WO-A1-2013/002357
- WO-A1-2013/062028
- WO-A1-2013/157622
- JP-A- 2012 526 728

## Description

### TECHNICAL FIELD

The present invention relates to a novel compound having an antiviral activity, in more detail, an anti-HIV drug.

### BACKGROUND ART

Among viruses, human immunodeficiency virus (hereinafter abbreviated as HIV) that is a type of retrovirus is known to be a cause of acquired immunodeficiency syndrome (hereinafter abbreviated as AIDS). As a therapeutic agent of the AIDS, reverse transcriptase inhibitors (AZT, 3TC, etc.), protease inhibitors (indinavir, etc.), and integrase inhibitors (raltegravir, etc.) are mainly used so far, but problems of side effects such as kidney problems and emergence of resistant viruses have been found, and development of anti-HIV drugs having a mechanism of action different from those is expected.

In addition, in the treatment of AIDS, because resistant viruses easily emerge, it is reported that, multiple drug therapy is currently effective. As the anti-HIV drugs, three types of reverse transcriptase inhibitors, protease inhibitors and integrase inhibitors have been used clinically, but the agents having the same mechanism of action often exhibit cross-resistance, or merely show additive effects, and there is a demand for the development of anti-HIV drugs having a different mechanism of action.

In Patent Document 1, a compound having a carboxymethyl benzene skeleton as an HIV reverse transcriptase inhibitor has been reported. In addition, as HIV replication inhibitors relatively similar to that of the present invention in structure, carboxymethyl pyridine derivatives (Patent Documents 2 to 8, 14, 19, 23, 33), carboxymethyl pyrimidine derivatives (Patent Documents 8 to 11, 21, 28, 29), phenylacetic acid derivatives (Patent Documents 12 to 13, 25, 26, 30, 31), a tricyclic carboxymethyl pyridine derivative (Patent Document 15, 27), a carboxymethyl pyridone derivative (Patent Document 16, 22), a substituted five-membered ring compound (Patent Document 17), and a substituted six-membered ring compound (Patent Document 18) have been reported. In Patent Document18, example of fused tricyclic compounds is not described.

Non-Patent Document 1 and 2 describe compounds relatively similar to that of the present invention in structure, but each document relates to synthesis technology. Non-Patent Documents 3 describe compounds relatively similar to that of the present invention in structure, but the document relates to anti-inflammatory agents.

Furthermore, the patents related to HIV replication inhibitors have been filed by the present applicant (Patent Document 20, 24, 32 WO2013/157622).

### PRIOR ART DOCUMENTS

### PATENT DOCUMENTS

Patent Document 1: WO 2008/071587
Patent Document 2: WO 2007/131350
Patent Document 3: WO 2009/062285
Patent Document 4: WO 2009/062288
Patent Document 5: WO 2009/062289
Patent Document 6: WO 2009/062308
Patent Document 7: WO 2010/130034
Patent Document 8: WO 2010/130842
Patent Document 9: WO 2011/015641
Patent Document 10: WO 2011/076765
Patent Document 11: WO 2012/033735
Patent Document 12: WO 2012/003497
Patent Document 13: WO 2012/003498
Patent Document 14: WO 2012/065963
Patent Document 15: WO 2012/066442
Patent Document 16: WO 2012/102985
Patent Document 17: WO 2012/137181
Patent Document 18: WO 2012/140243
Patent Document 19: WO 2013/012649
Patent Document 20: WO 2013/002357
Patent Document 21: WO 2013/025584
Patent Document 22: WO 2013/043553
Patent Document 23: WO 2013/073875
Patent Document 24: WO 2013/062028
Patent Document 25: WO 2013/103724
Patent Document 26: WO 2013/103738
Patent Document 27: WO 2013/123148
Patent Document 28: WO 2013/134113
Patent Document 29: WO 2013/134142
Patent Document 30: WO 2013/159064
Patent Document 31: WO 2012/145728
Patent Document 32: WO 2013/157622
Patent Document 33: WO 2014/009794

### NON-PATENT DOCUMENTS

Non-Patent Document 1: Journal of the American Chemical Society, vol. 133, No. 45, pages 18183-18193 (2011)
Non-Patent Document 2: Journal of the American Chemical Society, vol. 130, No. 52, 17676-17677 (2008)
Non-Patent Document 3: European Journal of Medicinal Chemistry, vol. 12, No. 2, 161-171 (1977)

### SUMMARY OF THE INVENTION

### PROBLEMS TO BE SOLVED BY THE INVENTION

An object of the present invention is to provide a novel compound having antiviral activity. Preferably, the present invention provides an anti-HIV drug having an inhibitory effect on HIV replication. More preferably, the present invention also provides effective new anti-HIV drugs against mutant strains and resistant strains of HIV, the core structure of which differs from that of traditional anti-HIV drugs. Furthermore, the present invention also provides its synthesis intermediates.

### SOLUTIONS TO THE PROBLEMS

As a result of intensive studies, the present inventors have found a novel HIV replication inhibitor. Furthermore, the present inventors have found that the compound of the present invention and a pharmaceutical composition containing the same are useful as an antiviral drug (examples: an antiretroviral drug, an anti-HIV drug, an anti-HTLV-1 (Human T cell leukemia virus type 1: human T-cell leukemia virus type 1) drug, an anti-FIV (Feline immunodeficiency virus: feline AIDS virus) drug, an anti-SIV (Simian immunodeficiency virus: simian AIDS virus) drug), particularly an anti-HIV drug, an anti-AIDS drug, a therapeutic agent of the related diseases or the like, thereby accomplishing the present invention.

The present invention can be summarized by the following items:
1. A compound represented by the following formula: 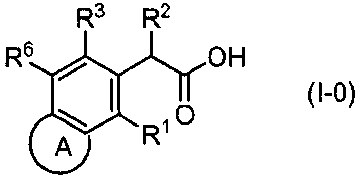 or its pharmaceutically acceptable salt,
   wherein
   ring A is substituted or unsubstituted heterocycle;
   R¹ is alkyl;
   or R¹ may be taken together with atom(s) constituting the ring A to form substituted or unsubstituted heterocycle;
   R² is alkyloxy, or substituted or unsubstituted cycloalkyloxy;
   R³ is selected from the following groups: 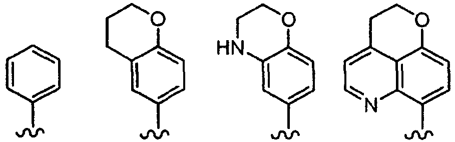 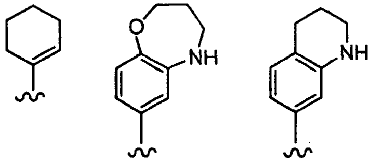 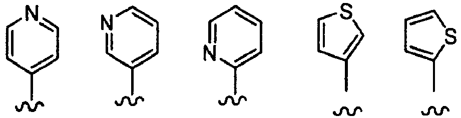 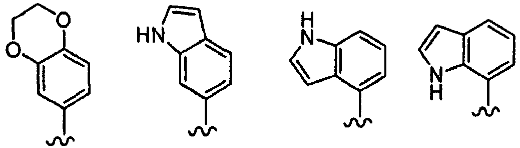 wherein the above groups may be substituted with same or different 1 to 4 substituent consisting of alkyl, alkoxy, halogen, hydroxy, hydroxyalkyl, alkoxyalkyl, haloalkyl, oxo, amino, mono or di alkylamino, aminoalkyl, mono or di alkylaminoalkyl and cyano; and
   R6 is alkyl.
2. The compound according to item 1 or its pharmaceutically acceptable salt, wherein ring A is monocyclic aromatic heterocycle or monocyclic non-aromatic heterocycle,
   wherein the ring may be fused with another aromatic carbocycle, non-aromatic carbocycle, aromatic heterocycle, or non-aromatic heterocycle; the ring may form a spiro ring together with another aromatic carbocycle, non-aromatic carbocycle, aromatic heterocycle or non-aromatic heterocycle; two atoms constituting ring A which are not adjacent to each other may be cross-linked with alkylene, alkenylene or alkynylene; and/or, rings may be substituted with one or more of R^{A};
   wherein R^{A} is each independently, halogen, cyano, nitro, oxo, azide, trimethylsilyl or -X^{A} -RA¹ ;
   X^{A} is a single bond, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -CO-O-NR^{A2}-, -O-CONR^{A2}-, -NR^{A2}-O-CO-, -CO-NR^{A2}-O-, -O-NR^{A2}-CO-, -NR^{A2}-CO-NR^{A3}-, -NR^{A2}-SO₂- or -SO₂-NR^{A2}-;
   R^{A1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A} is a single bond, R^{A1} is not a hydrogen atom;
   R^{A2} and R^{A3} are each independently a hydrogen atom, substituted or unsubstituted alkyl, substituted or unsubstituted alkenyl, or substituted or unsubstituted alkynyl;
   when X^{A} is -NR^{A2}-, -CO-NR^{A2}-, -CO-O-NR^{A2}-, -O-CO-NR^{A2}- or -SO₂-NR^{A2}-, R^{A1} and R^{A2} may be taken together with an adjacent nitrogen atom to form substituted or unsubstituted aromatic heterocyclyl, or substituted or unsubstituted non-aromatic heterocyclyl;
   when X^{A} is -NR^{A2}-CO-NR^{A3}-, R^{A1} and R^{A3} may be taken together with an adjacent nitrogen atom to form substituted or unsubstituted aromatic heterocyclyl, or substituted or unsubstituted non-aromatic heterocyclyl;
   R¹ and R^{A} may be taken together with an adjacent atom to form aromatic carbocycle, non-aromatic carbocycle, aromatic heterocycle or non-aromatic heterocycle, wherein the ring may be substituted with one or more of the same or different R^{A'};
   wherein R^{A'} is each independently, halogen, cyano, nitro, oxo, azide, trimethylsilyl or -X^{A'}-R^{A'1} ;
   X^{A'} is a single bond, -O-, -S-, -NR^{A'2}-, =N-, -CO-, -SO₂-, -O-CO-, -CO-O-, -NR^{A'2}-CO-, -CO-NR^{A'2}-, -NR^{A'2}-CO-O-, -CO-O-NR^{A'2}-, -O-CO-NR^{A'2}-, -NR^{A'2}-O-CO-, -CO-NR^{A'2}-O-, -O-NR^{A'2}-CO-, -NR^{A'2}-CO-NR^{A'3}-, -NR^{A'2}-SO₂- or -SO₂-NR^{A'2}-;
   R^{A'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A'} is a single bond, R^{A'1} is not a hydrogen atom;
   R^{A'2} and R^{A'3} are each independently, a hydrogen atom, substituted or unsubstituted alkyl, substituted or unsubstituted alkenyl, or substituted or unsubstituted alkynyl;
   X^{A'} is -NR^{A'2}-, -CO-NR^{A'2}-, -CO-O-NR^{A'2}-, -O-CONR^{A'2}- or -SO₂-NR^{A'2}-; R^{A'1} and R^{A'2} may be taken together with an adjacent nitrogen atom to form substituted or unsubstituted aromatic heterocyclyl, or substituted or unsubstituted non-aromatic heterocyclyl;
   when X^{A'} is -NR^{A'2}-CO-NR^{A'3}-, R^{A'1} and R^{A'3} may be taken together with an adjacent nitrogen atom to form substituted or unsubstituted aromatic heterocyclyl, or substituted or unsubstituted non-aromatic heterocyclyl.
3. The compound according to item 1 or 2 or its pharmaceutically acceptable salt, wherein ring A is the following ring: 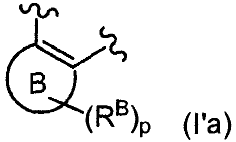 wherein
   ring B is monocyclic aromatic heterocycle or monocyclic non-aromatic heterocycle; wherein the ring may form a spiro ring together with another aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene;
   R^{B} is each independently, halogen, cyano, nitro, oxo, azide, trimethylsilyl or -X^{B}-RB¹;
      X^{B} is a single bond, -O-, -S-, -NR^{B2}-, =N-, -CO-, -SO₂-, -O-CO-, -CO-O-, -NRB²-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- or -SO₂-NR^{B2}-;
      R^{B1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom;
      R^{B2}, and R^{B3} are each independently a hydrogen atom, substituted or unsubstituted alkyl, substituted or unsubstituted alkenyl, or substituted or unsubstituted alkynyl;
      when X^{B} is -NR^{B2}-, -CO-NR^{B2}-, -CO-O-NR^{B2}-, -O-CO-NR^{B2}- or -SO₂-NR^{B2}-, R^{B1} and R^{B2} may be taken together with an adjacent nitrogen atom to form substituted or unsubstituted aromatic heterocyclyl, or substituted or unsubstituted non-aromatic heterocyclyl;
      when X^{B} is -NR^{B2}-CO-NR^{B3}-, R^{B1} and R^{B3} may be taken together with an adjacent nitrogen atom to form substituted or unsubstituted aromatic heterocyclyl, or substituted or unsubstituted non-aromatic heterocyclyl;
   p is any integer of 0 to 12;
   R¹ and R^{B} may be taken together with an adjacent atom to form aromatic carbocycle, non-aromatic carbocycle, aromatic heterocycle or non-aromatic heterocycle, wherein the ring may be substituted with one or more of the same or different R^{B'} ;
      R^{B'} is each independently, halogen, cyano, nitro, oxo, azide, trimethylsilyl or -X^{B'}-R^{B'1};
      wherein X^{B'} is a single bond, -O-, -S-, -NR^{B2}-, =N-, -CO-, -SO₂-, -O-CO-, -CO-O-, -NR^{B'2}-CO-, -CO-NR^{B'2}-, -NR^{B'2}-CO-O-, -CO-O-NR^{B'2}-, -O-CO-NR^{B'2}-, -NR^{B2}-O-CO-, -CO-NR^{B'2}-O-, -O-NR^{B'2}-CO-, -NR^{B'2}-CO-NR^{B'3}-, -NR^{B'2}-SO₂- or -SO₂-NR^{B'2}-;
      R^{B'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom;
      R^{B'2} and R^{B'3} are each independently, a hydrogen atom, substituted or unsubstituted alkyl, substituted or unsubstituted alkenyl, or substituted or unsubstituted alkynyl;
      when X^{B'} is -NR^{B'2}-, -CO-NR^{B'2}-, -CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl;
      when X^{B'} is -NR^{B'2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl.
4. The compound according to item 1 or 2 or its pharmaceutically acceptable salt, wherein ring A is any one of the following rings: 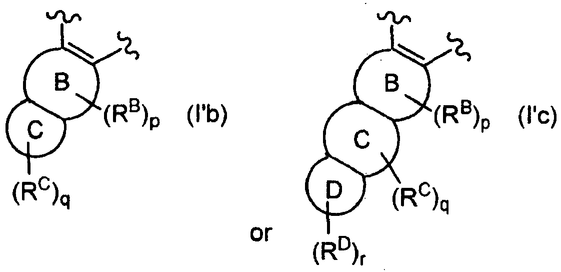 wherein
   ring B is each independently monocyclic aromatic heterocycle or monocyclic non-aromatic heterocycle, ring C and ring D are each independently monocyclic aromatic carbocycle, monocyclic non-aromatic carbocycle, monocyclic aromatic heterocycle or monocyclic non-aromatic heterocycle;
      wherein the ring(s) may form a spiro ring together with another aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene;
   R^{B} is each independently, halogen, cyano, nitro, oxo, azide, trimethylsilyl or -X^{B}-R^{B1};
      X^{B} is a single bond, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- or -SO₂-NR^{B2} -;
      R^{B1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom;
      R^{B2} and R^{B3} are each independently a hydrogen atom, substituted or unsubstituted alkyl, substituted or unsubstituted alkenyl, or substituted or unsubstituted alkynyl;
      when X^{B} is -NR^{B2}-, -CO-NR^{B2}-, -CO-O-NR^{B2}-, -O-CO-NR^{B2}- or -SO₂-NR^{B2}-, R^{B1} and R^{B2} may be taken together with an adjacent nitrogen atom to form substituted or unsubstituted aromatic heterocyclyl, or substituted or unsubstituted non-aromatic heterocyclyl;
      when X^{B} is -NR^{B2}-CO-NR^{B3}-, R^{B1} and R^{B3} may be taken together with an adjacent nitrogen atom to form substituted or unsubstituted aromatic heterocyclyl, or substituted or unsubstituted non-aromatic heterocyclyl;
   p is any integer of 0 to 12;
   R¹ and R^{B} may be taken together with an adjacent atom to form aromatic carbocycle, non-aromatic carbocycle, aromatic heterocycle or non-aromatic heterocycle, wherein the ring may be substituted with one or more of the same or different R^{B'} ;
      R^{B'} is each independently, halogen, cyano, nitro, oxo, azide, trimethylsilyl or -X^{B'}-R^{B'1};
      wherein X^{B'} is a single bond, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -CO-O-NR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- or -SO₂-NR^{B'2}-;
      R^{B'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom;
      R^{B'2} and R^{B'3} are each independently, a hydrogen atom, substituted or unsubstituted alkyl, substituted or unsubstituted alkenyl, or substituted or unsubstituted alkynyl;
      when X^{B'} is -NR^{B'2}-, -CO-NR^{B'2}-, -CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl;
      when X^{B'} is -NR^{B'2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl;
   s is any integer of 0 to 12;
   R^{C} is each independently, halogen, cyano, nitro, oxo, azide, trimethylsilyl or -X^{C}-R^{C1};
      X^{C} is a single bond, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-O-CO-, -CO-NR^{C2}-O-, -O-NR^{C2}-CO-, -NR^{C2}-CO-NR^{C3}-, -NR^{C2}-SO₂- or -SO₂-NR^{C2}-;
      R^{C1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{C} is a single bond, R^{C1} is not a hydrogen atom;
      R^{C2} and R^{C3} are each independently, a hydrogen atom, substituted or unsubstituted alkyl, substituted or unsubstituted alkenyl, or substituted or unsubstituted alkynyl;
      when X^{C} is -NR^{C2}-, -CO-NR^{C2}-, -CO-O-NR^{C2}-, -O-CO-NR^{C2}- or -SO₂-NR^{C2}-, R^{C1} and R^{C2} may be taken together with an adjacent nitrogen atom to form substituted or unsubstituted aromatic heterocyclyl, or substituted or unsubstituted non-aromatic heterocyclyl;
      when X^{C} is -NR^{C2}-CO-NR^{C3}-, R^{C1} and R^{C3} may be taken together with an adjacent nitrogen atom to form substituted or unsubstituted aromatic heterocyclyl, or substituted or unsubstituted non-aromatic heterocyclyl;
   q is any integer of 0 to 12;
   R^{B} and R^{C} may be taken together with an adjacent atom to form aromatic carbocycle, non-aromatic carbocycle, aromatic heterocycle or non-aromatic heterocycle, wherein the ring may be substituted with one or more of the same or different R^{B}; and
   R^{D} is each independently, halogen, cyano, nitro, oxo, azide, trimethylsilyl or -X^{D}-R^{D1};
      X^{D} is a single bond, -O-, -S-, -NR^{D2}-, =N-, -CO-, -SO₂-, -O-CO-, -CO-O-, -NR^{D2}-CO-, -CO-NR^{D2}-, -NR^{D2}-CO-O-, -CO-O-NR^{D2}-, -O-CO-NR^{D2}-, -NR^{D2}-O-CO-, -CO-NR^{D2}-O-, -O-NR^{D2}-CO-, -NR^{D2}-CO-NR^{D3}-, -NR^{D2}-SO₂- or -SO₂-NR^{D2}-;
      R^{D1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl or substituted or unsubstituted non-aromatic heterocyclyl, provided that when X^{D} is a single bond, R^{D1} is not a hydrogen atom;
      R^{D2} and R^{D3} are each independently, a hydrogen atom, substituted or unsubstituted alkyl, substituted or unsubstituted alkenyl, or substituted or unsubstituted alkynyl;
      when X^{D} is -NR^{D2}-, -CO-NR^{D2}-, -CO-O-NR^{D2}-, -O-CO-NR^{D2}- or -SO₂-NR^{D2}-, R^{D1} and R^{D2} may be taken together with an adjacent nitrogen atom to form substituted or unsubstituted aromatic heterocyclyl, or substituted or unsubstituted non-aromatic heterocyclyl,
      when X^{D} is -NR^{D2}-CO-NR^{D3}-, R^{D1} and R^{D3} may be taken together with an adjacent nitrogen atom to form substituted or unsubstituted aromatic heterocyclyl, or substituted or unsubstituted non-aromatic heterocyclyl;
   r is any integer of 0 to 12;
   R^{C} and R^{D} may be taken together with an adjacent atom to form aromatic carbocycle, non-aromatic carbocycle, aromatic heterocycle or non-aromatic heterocycle, wherein the ring may be substituted with one or more of the same or different R^{C}.
5. The compound according to item 4 or its pharmaceutically acceptable salt, wherein ring A is the following ring: 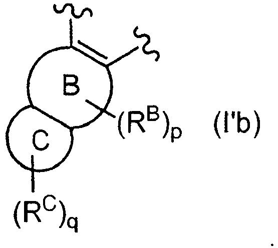
6. The compound according to any one of items 3 to 5 or its pharmaceutically acceptable salt, wherein p is one or more.
7. The compound according to item 3 or 6 represented by formula (I"a): 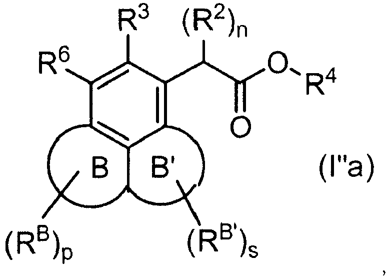 or its pharmaceutically acceptable salt,
   wherein ring B' is monocyclic aromatic carbocycle, monocyclic non-aromatic carbocycle, monocyclic aromatic heterocycle, or monocyclic non-aromatic heterocycle; p is any integer of 0 to 11; s is any integer of 0 to 11.
8. The compound according to item 1 represented by any one of following formulas: 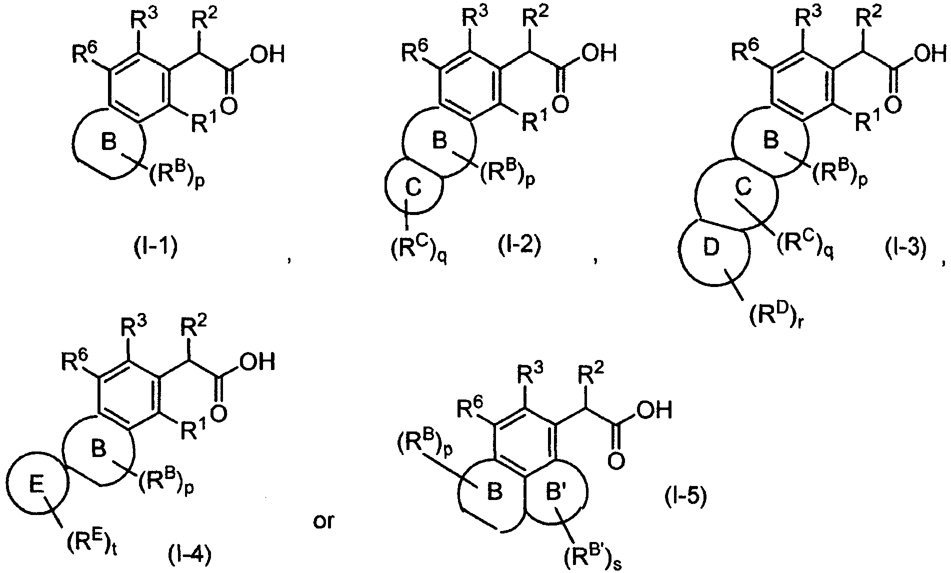 or its pharmaceutically acceptable salt,
   wherein
   R¹ is alkyl;
   R² is alkyloxy, or substituted or unsubstituted cycloalkyloxy;
   R³ is as defined in claim 1;
   R⁶ is alkyl;
   ring B is each independently monocyclic aromatic heterocycle or monocyclic non-aromatic heterocycle, ring C, ring D, ring E, and ring B' are each independently monocyclic aromatic carbocycle, monocyclic non-aromatic carbocycle, monocyclic aromatic heterocycle or monocyclic non-aromatic heterocycle;
   ring E is the same as defined as ring C;
   R^{B} and R^{E} are each independently the same meaning as R^{C} ;
   t is any integer of 0 to 12;
   others are the same meaning as described in item 4.
9. The compound according to item 1 represented by any one of following formulas: 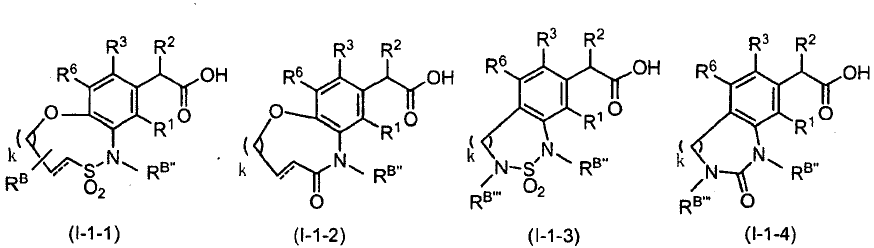 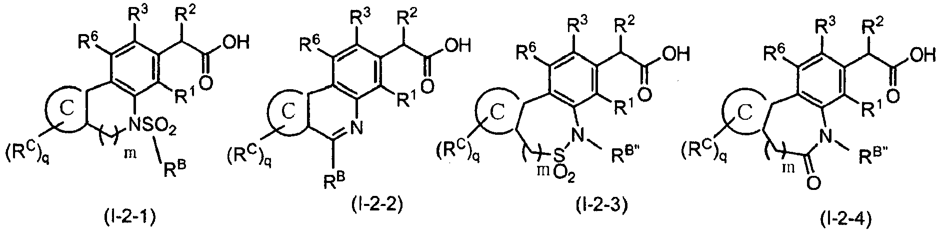 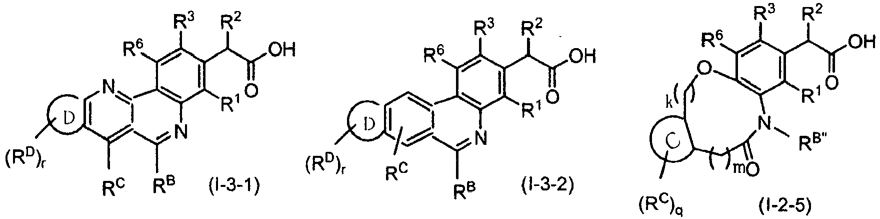 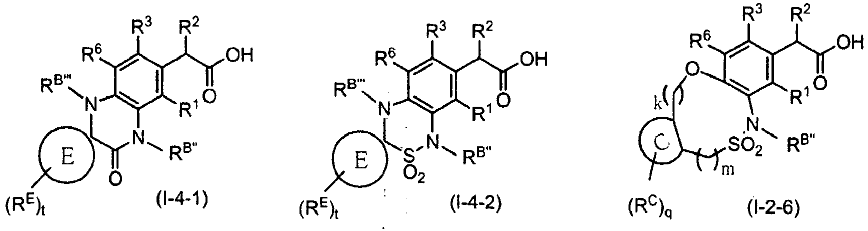 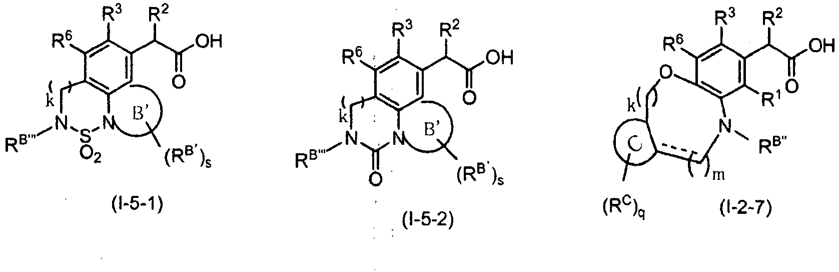 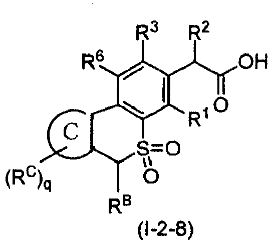 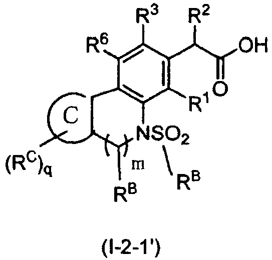 or its pharmaceutically acceptable salt,
   wherein
   ring C and ring D are each independently benzene or 5- to 8-membered heterocycle;
   ring B' is 5- to 8-membered heterocycle;
   ring E is optionally cross-linked 5- to 8-membered carbocycle;
   R^{B"} and R^{B'''} are each independently a hydrogen atom, alkyl, cycloalkyl, cycloalkylalkyl, formyl, alkoxycarbonyl, substituted or unsubstituted aryl, substituted or unsubstituted aralkylaralkyl, substituted or unsubstituted heterocyclyl, substituted or unsubstituted heterocyclylalkyl, or substituted sulfonyl;
   a broken line means the presence or absence of bond;
   k and m are each independently an integer of 0 to 4;
   others are the same meaning as described in item 8.
10. The compound according to item 9 represented by formulas (I-2-1), (I-2-2), (I-2-3) or (I-2-4), or its pharmaceutically acceptable salt.
11. A pharmaceutical composition comprising the compound according to any one of items 1 to 10 or its pharmaceutically acceptable salt.
12. The pharmaceutical composition according to item 11, having anti-HIV activity.
13. The compound according to any one of items 1 to 10 or its pharmaceutically acceptable salt, for use in the treatment or prevention of HIV infection.

Herein disclosed is further:
(1) A compound represented by formula (I): 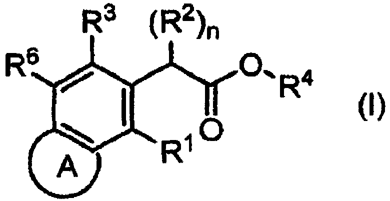 or its pharmaceutically acceptable salt,
   wherein
   ring A is substituted or unsubstituted carbocycle, or substituted or unsubstituted heterocycle;
   R¹ is halogen, cyano, nitro or -X¹-R¹¹;
      X¹ is a single bond, -O-, -S-, -NR¹²-, -CO-, -SO₂-, -O-CO-, -CO-O-, -NR¹²-CO-, - CO-NR¹²-, -NR¹²-CO-O-, -NR¹²-CO-NR¹⁸-, -NR¹²-SO₂- or -SO₂-NR¹²-;
      R¹¹ is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl;
      R¹² and R¹³ are each independently a hydrogen atom, substituted or unsubstituted alkyl, substituted or unsubstituted alkenyl, or substituted or unsubstituted alkynyl;
      when X¹ is -NR¹²-, -CO-NR¹²- or -SO₂-NR¹²-, R¹¹ and R¹² may be taken together with an adjacent nitrogen atom to form substituted or unsubstituted aromatic heterocyclyl, or substituted or unsubstituted non-aromatic heterocyclyl;
      when X¹ is -NR¹²-CO-NR¹⁸-, R¹¹ and R¹⁸ may be taken together with an adjacent nitrogen atom to form substituted or unsubstituted aromatic heterocyclyl, or substituted or unsubstituted non-aromatic heterocyclyl;
      R¹ may be taken together with atom(s) constituting the ring A to form substituted or unsubstituted carbocycle, or substituted or unsubstituted heterocycle;
   R² is each independently substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted cycloalkyloxy, substituted or unsubstituted alkylsulfanyl, substituted or unsubstituted alkenylsulfanyl, or substituted or unsubstituted alkynylsulfanyl;
   n is 1 or 2;
   R³ is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl;
   R⁴ is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl; and
   R⁶ is halogen, cyano, nitro or -X⁶-R⁶¹;
      X⁶ is a single bond, -O-, -S-, -NR⁶²-, -CO-, -SO₂-, -O-CO-, -CO-O-, -NR⁶²-CO-, - CO-NR⁶²-, -NR⁶²-CO-O-, -NR⁶²-CO-NR⁶³-, -NR⁶²-SO₂- or -SO₂-NR⁶²-;
      R⁶¹ is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl;
      R⁶² and R⁶³ are each independently, a hydrogen atom, substituted or unsubstituted alkyl, substituted or unsubstituted alkenyl, or substituted or unsubstituted alkynyl;
      when X⁶ is -NR⁶² -, -CO-NR⁶²- or -SO₂-NR⁶²-, R⁶¹ and R⁶² may be taken together with an adjacent nitrogen atom to form substituted or unsubstituted aromatic heterocyclyl, or substituted or unsubstituted non-aromatic heterocyclyl;
      when X⁶ is -NR⁶²-CO-NR⁶³-, R⁶¹ and R⁶³ may be taken together with an adjacent nitrogen atom to form substituted or unsubstituted aromatic heterocyclyl, or substituted or unsubstituted non-aromatic heterocyclyl;
   provided that the following compounds are excluded: 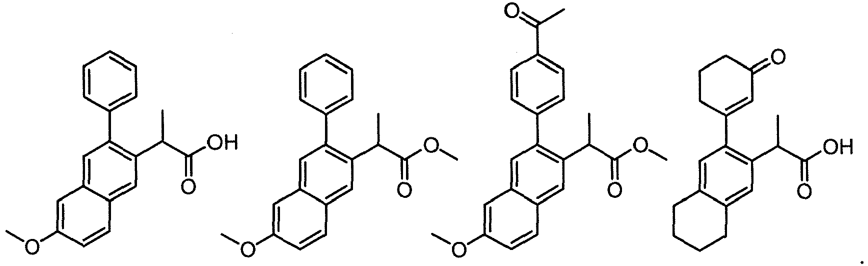
(2) The compound according to the above (1) or its pharmaceutically acceptable salt, wherein R⁴ is a hydrogen atom.
(3) The compound according to the above (1) or (2), or its pharmaceutically acceptable salt, wherein n is 1.
(4) The compound according to the above (3) or its pharmaceutically acceptable salt, wherein R² is substituted or unsubstituted alkyloxy, or substituted or unsubstituted cycloalkyloxy.
(5) The compound according to any one of the above (1) to (4) or its pharmaceutically acceptable salt, wherein ring A is monocyclic aromatic carbocycle, monocyclic non-aromatic carbocycle, monocyclic aromatic heterocycle or monocyclic non-aromatic heterocycle,
   wherein the ring may be fused with another aromatic carbocycle, non-aromatic carbocycle, aromatic heterocycle, non-aromatic heterocycle or its fused ring; the ring may form a spiro ring together with another aromatic carbocycle, non-aromatic carbocycle, aromatic heterocycle or non-aromatic heterocycle; two atoms constituting ring A which are not adjacent to each other may be cross-linked with alkylene, alkenylene or alkynylene; and/or, rings may be substituted with one or more of R^{A};
   wherein R^{A} is each independently, halogen, cyano, nitro, oxo, azide, trimethylsilyl or -X^{A}-R^{A1};
   X^{A} is a single bond, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -CO-O-NR^{A2}-, -O-CO-NR^{A2}-, -NR^{A2}-O-CO-, -CO-NR^{A2}-O-, -O-NR^{A2}-CO-, -NR^{A2}-CO-NR^{A3}-, -NR^{A2}-SO₂- or -SO₂-NR^{A2}-;
   R^{A1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A} is a single bond, R^{A1} is not a hydrogen atom;
   R^{A2} and R^{A3} are each independently a hydrogen atom, substituted or unsubstituted alkyl, substituted or unsubstituted alkenyl, or substituted or unsubstituted alkynyl;
   when X^{A} is -NR^{A2}-, -CO-NR^{A2}-, -CO-O-NR^{A2}-, -O-CO-NR^{A2}- or -SO₂-NR^{A2}-, R^{A1} and R^{A2} may be taken together with an adjacent nitrogen atom to form substituted or unsubstituted aromatic heterocyclyl, or substituted or unsubstituted non-aromatic heterocyclyl;
   when X^{A} is -NR^{A2}-CO-NR^{A3}-, R^{A1} and R^{A3} may be taken together with an adjacent nitrogen atom to form substituted or unsubstituted aromatic heterocyclyl, or substituted or unsubstituted non-aromatic heterocyclyl;
   R¹ and R^{A} may be taken together with an adjacent atom to form aromatic carbocycle, non-aromatic carbocycle, aromatic heterocycle or non-aromatic heterocycle, wherein the ring may be substituted with one or more of the same or different R^{A};
   wherein R^{A'} is each independently, halogen, cyano, nitro, oxo, azide, trimethylsilyl or -X^{A'}-R^{A'1};
   X^{A'} is a single bond, -O-, -S-, -NR^{A'2}-, =N-, -CO-, -SO₂-, -O-CO-, -CO-O-, - NR^{A'2}-CO-, -CO-NR^{A'2}-, -NR^{A'2}-CO-O-, -CO-O-NR^{A'2}-, -O-CO-NR^{A'2}-, -NR^{A'2}-O-CO-, -CO-NR^{A'2}-O-, -O-NR^{A'2}-CO-, -NR^{A'2}-CO-NR^{A'3}-, -NR^{A'2}-SO₂- or -SO₂-NR^{A'2}-
   ; R^{A'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A'} is a single bond, R^{A'1} is not a hydrogen atom;
   R^{A'2} and R^{A'3} are each independently, a hydrogen atom, substituted or unsubstituted alkyl, substituted or unsubstituted alkenyl, or substituted or unsubstituted alkynyl;
   X^{A'} is -NR^{A'2}-, -CO-NR^{A'2}-, -CO-O-NR^{A'2}-, -O-CO-NR^{A'2}- or -SO₂-NR^{A'2}-;
   R^{A'1} and R^{A'2} may be taken together with an adjacent nitrogen atom to form substituted or unsubstituted aromatic heterocyclyl, or substituted or unsubstituted non-aromatic heterocyclyl;
   when X^{A'} is -NR^{A'2}-CO-NR^{A'3}-, R^{A'1} and R^{A'3} may be taken together with an adjacent nitrogen atom to form substituted or unsubstituted aromatic heterocyclyl, or substituted or unsubstituted non-aromatic heterocyclyl.
(6) The compound according to any one of the above (1) to (5) or its pharmaceutically acceptable salt, wherein ring A is 5- to 12-membered ring which is fused with another 3- to 10-membered monocycle or another 8- to 18-membered fused ring and is optionally substituted with one or more of the same or different R^{A}.
(7) The compound according to any one of the above (1) to (5) or its pharmaceutically acceptable salt, wherein ring A is 5- to 12-membered ring to form a spiro ring together with another 5- to 10-memdered ring, which is optionally substituted with one or more of the same or different R^{A}.
(8) The compound according to any one of the above (1) to (5) or its pharmaceutically acceptable salt, wherein ring A is any one of the following rings: 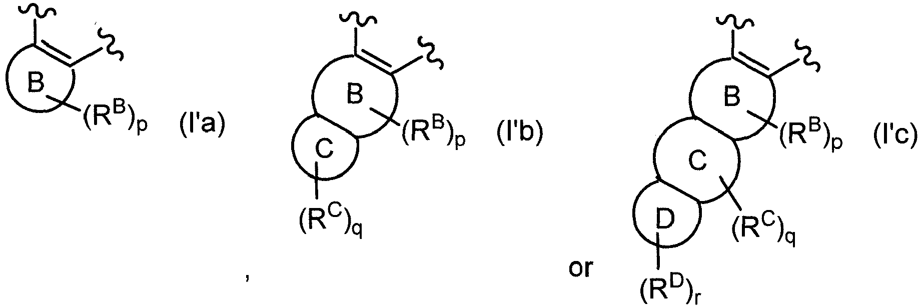 wherein
   ring B, ring C and ring D are each independently monocyclic aromatic carbocycle, monocyclic non-aromatic carbocycle, monocyclic aromatic heterocycle or monocyclic non-aromatic heterocycle; wherein the ring may form a spiro ring together with another aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene;
   R^{B} is each independently, halogen, cyano, nitro, oxo, azide, trimethylsilyl or -X^{B}-R^{B1} ;
      X^{B} is a single bond, -O-, -S-, -NR^{B2}-, =N-, -CO-, -SO₂-, -O-CO-, -CO-O-, - NR^{B2}-CO-, -CO-NR^{B2}-, -NR^{B2}-CO-O-, -CO-O-NR^{B2}-, -O-CO-NR^{B2}-, -NR^{B2}-O-CO-, - CO-NR^{B2}-O-, -O-NR^{B2}-CO-, -NR^{B2}-CO-NR^{B3}-, -NR^{B2}-SO₂- or -SO₂-NR^{B2}-;
      R^{B1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom;
      R^{B2} and R^{B3} are each independently a hydrogen atom, substituted or unsubstituted alkyl, substituted or unsubstituted alkenyl, or substituted or unsubstituted alkynyl;
      when X^{B} is -NR^{B2}-, -CO-NR^{B2}-, -CO-O-NR^{B2}-, -O-CO-NR^{B2}- or -SO₂-NR^{B2}-, R^{B1} and R^{B2} may be taken together with an adjacent nitrogen atom to form substituted or unsubstituted aromatic heterocyclyl, or substituted or unsubstituted non-aromatic heterocyclyl;
      when X^{B} is -NR^{B2}-CO-NR^{B3}-, R^{B1} and R^{B3} may be taken together with an adjacent nitrogen atom to form substituted or unsubstituted aromatic heterocyclyl, or substituted or unsubstituted non-aromatic heterocyclyl;
   p is any integer of 0 to 12;
   R¹ and R^{B} may be taken together with an adjacent atom to form aromatic carbocycle, non-aromatic carbocycle, aromatic heterocycle or non-aromatic heterocycle, wherein the ring may be substituted with one or more of the same or different R^{B'};
      R^{B'} is each independently, halogen, cyano, nitro, oxo, azide, trimethylsilyl or - X^{B'}-R^{B'1};
      wherein X^{B'} is a single bond, -O-, -S-, -NR^{B'2}-, =N-, -CO-, -SO₂-, -O-CO-, -COO-, -NR^{B'2}-CO-, -CO-NR^{B'2}-, -NR^{B'2}-CO-O-, -CO-O-NR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- or -SO₂-NR^{B'2}-;
      R^{B'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom;
      R^{B'2} and R^{B'3} are each independently, a hydrogen atom, substituted or unsubstituted alkyl, substituted or unsubstituted alkenyl, or substituted or unsubstituted alkynyl;
      when X^{B'} is -NR^{B'2}-, -CO-NR^{B'2}-, -CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl;
      when X^{B'} is -NR^{B'2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl;
   s is any integer of 0 to 12;
   R^{C} is each independently, halogen, cyano, nitro, oxo, azide, trimethylsilyl or -X^{C}-R^{C1};
      X^{C} is a single bond, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-O-CO-, -CO-NR^{C2}-O-, -O-NR^{C2}-CO-, -NR^{C2}-CO-NR^{C3}-, -NR^{C2}-SO₂- or -SO₂-NR^{C2}-;
      R^{C1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{C} is a single bond, R^{C1} is not a hydrogen atom;
      R^{C2} and R^{C3} are each independently, a hydrogen atom, substituted or unsubstituted alkyl, substituted or unsubstituted alkenyl, or substituted or unsubstituted alkynyl;
      when X^{C} is -NR^{C2}-, -CO-NR^{C2}-, -CO-O-NR^{C2}-, -O-CO-NR^{C2}- or -SO₂-NR^{C2}-, R^{C1} and R^{C2} may be taken together with an adjacent nitrogen atom to form substituted or unsubstituted aromatic heterocyclyl, or substituted or unsubstituted non-aromatic heterocyclyl;
      when X^{C} is -NR^{C2}-CO-NR^{C3}-, R^{C1} and R^{C3} may be taken together with an adjacent nitrogen atom to form substituted or unsubstituted aromatic heterocyclyl, or substituted or unsubstituted non-aromatic heterocyclyl;
   q is any integer of 0 to 12;
   R^{B} and R^{C} may be taken together with an adjacent atom to form aromatic carbocycle, non-aromatic carbocycle, aromatic heterocycle or non-aromatic heterocycle, wherein the ring may be substituted with one or more of the same or different R^{B}; and
   R^{D} is each independently, halogen, cyano, nitro, oxo, azide, trimethylsilyl or -X^{D}-R^{D1};
      X^{D} is a single bond, -O-, -S-, -NR^{D2}-, =N-, -CO-, -SO₂-, -O-CO-, -CO-O-, -NR^{D2}-CO-, -CO-NR^{D2}-, -NR^{D2}-CO-O-, -CO-O-NR^{D2-}, -O-CO-NR^{D2}-, -NR^{D2}-O-CO-, -CO-NR^{D2}-O-, -O-NR^{D2}-CO-, -NR^{D2}-CO-NR^{D3}-, -NR^{D2}-SO₂- or -SO₂-NR^{D2}-;
      R^{D1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl or substituted or unsubstituted non-aromatic heterocyclyl, provided that when X^{D} is a single bond, R^{D1} is not a hydrogen atom;
      R^{D2} and R^{D3} are each independently, a hydrogen atom, substituted or unsubstituted alkyl, substituted or unsubstituted alkenyl, or substituted or unsubstituted alkynyl;
      when X^{D} is -NR^{D2}-, -CO-NR^{D2}-, -CO-O-NR^{D2}-, -O-CO-NR^{D2}- or -SO₂ -NR^{D2}-, R^{D1} and R^{D2} may be taken together with an adjacent nitrogen atom to form substituted or unsubstituted aromatic heterocyclyl, or substituted or unsubstituted non-aromatic heterocyclyl,
      when X^{D} is -NR^{D2}-CO-NR^{D3}-, R^{D1} and R^{D3} may be taken together with an adjacent nitrogen atom to form substituted or unsubstituted aromatic heterocyclyl, or substituted or unsubstituted non-aromatic heterocyclyl;
   r is any integer 0 to 12;
   R^{C} and R^{D} may be taken together with an adjacent atom to form aromatic carbocycle, non-aromatic carbocycle, aromatic heterocycle or non-aromatic heterocycle, wherein the ring may be substituted with one or more of the same or different R^{C}.
(9) The compound according to the above (8) or its pharmaceutically acceptable salt, wherein ring A is the following ring: 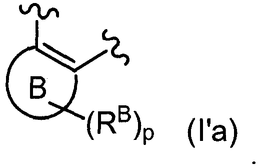
(10) The compound according to the above (8) or its pharmaceutically acceptable salt, wherein ring A is the following ring: 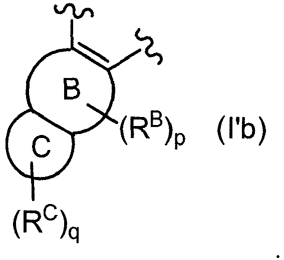
(11) The compound according to the above (8) or its pharmaceutically acceptable salt, wherein ring A is the following ring: 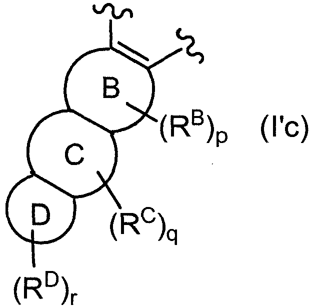
(12) The compound according to any one of the above (9) to (11) or its pharmaceutically acceptable salt, wherein ring B is 5- to 10-membered ring which may form a spiro ring together with another 3- to 10-membered ring.
(13) The compound according to any one of the above (9) to (11) or its pharmaceutically acceptable salt, wherein p is one or more.
(14) The compound according to the above (13) or its pharmaceutically acceptable salt, wherein R^{B} is each independently halogen, hydroxy, oxo, amino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl.
(15) The compound according to the above (10) or (11), or its pharmaceutically acceptable salt, wherein ring C is 3- to 10-membered ring.
(16) The compound according to the above (10) or (11), or its pharmaceutically acceptable salt, wherein q is one or more.
(17) The compound according to the above (16) or its pharmaceutically acceptable salt, wherein R^{C} is each independently halogen, hydroxy, oxo, amino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl.
(18) The compound according to the above (11) or its pharmaceutically acceptable salt, wherein ring D is 3- to 10-membered ring.
(19) The compound according to the above (11) or its pharmaceutically acceptable salt, wherein r is one or more.
(20) The compound according to the above (19) or its pharmaceutically acceptable salt, wherein R^{D} is each independently, halogen, hydroxy, oxo, amino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl.
(21) The compound according to the above (9) or its pharmaceutically acceptable salt, wherein p is an integer of 0 to 3.
(22) The compound according to the above (10) or its pharmaceutically acceptable salt, wherein p and q are each independently an integer of 0 to 3.
(23) The compound according to the above (11) or its pharmaceutically acceptable salt, wherein p, q and r are each independently an integer of 0 to 3.
(24) The compound according to any one of the above (1) to (23) or its pharmaceutically acceptable salt, wherein R¹ is halogen, cyano, nitro or -X¹-R¹¹;
   X¹ is a single bond, -O-, -S-, -NR¹²-, -CO-, -SO₂-, -O-CO-, -CO-O-, -NR¹²-CO-,-CO-NR¹²-, -NR¹²-CO-O-, -NR¹²-CO-NR¹³-, -NR¹²-SO₂- or -SO₂-NR¹²-;
   R¹¹ is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl;
   R¹² and R¹³ are each independently a hydrogen atom, substituted or unsubstituted alkyl, substituted or unsubstituted alkenyl, or substituted or unsubstituted alkynyl;
   when X¹ is -NR¹²-, -CO-NR¹²- or -SO₂-NR¹²-, R¹¹ and R¹² may be taken together with an adjacent nitrogen atom to form substituted or unsubstituted aromatic heterocyclyl, or substituted or unsubstituted non-aromatic heterocyclyl;
   when X¹ is -NR¹² -CO-NR¹³-, R¹¹ and R¹³ may be taken together with an adjacent nitrogen atom to form substituted or unsubstituted aromatic heterocyclyl, or substituted or unsubstituted non-aromatic heterocyclyl.
(25) The compound according to any one of the above (1) to (24) or its pharmaceutically acceptable salt, wherein R¹ is a hydrogen atom, halogen, substituted or unsubstituted alkyl, substituted or unsubstituted alkenyl, or substituted or unsubstituted alkynyl.
(26) The compound according to the above (25) or its pharmaceutically acceptable salt, wherein R¹ is substituted or unsubstituted alkyl.
(27) The compound according to any one of the above (9) or (12) to (14) represented by formula (I"a): 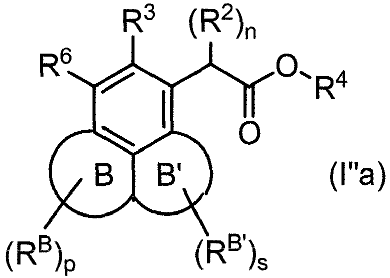 , or its pharmaceutically acceptable salt,
   wherein ring B' is monocyclic aromatic carbocycle, monocyclic non-aromatic carbocycle, monocyclic aromatic heterocycle, or monocyclic non-aromatic heterocycle; p is any integer of 0 to 11; s is any integer of 0 to 11.
(28) The compound according to any one of the above (10) or (12) to (17) represented by formula (I"b): 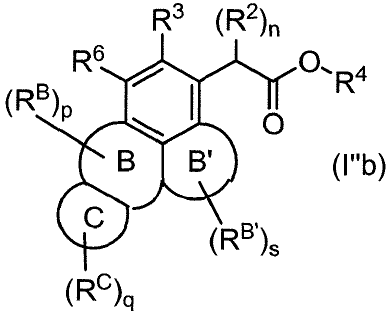 , or its pharmaceutically acceptable salt,
   wherein ring B' is monocyclic aromatic carbocycle, monocyclic non-aromatic carbocycle, monocyclic aromatic heterocycle or monocyclic non-aromatic heterocycle; p is any integer of 0 to 11; s is any integer of 0 to 11.
(29) The compound according to any one of the above (11) to (20) represented by formula (Ic"): 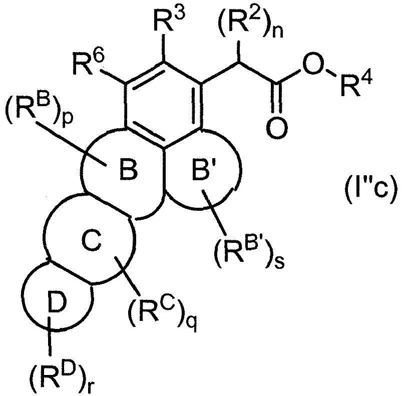 , or its pharmaceutically acceptable salt,
   wherein ring B' is monocyclic aromatic carbocycle, monocyclic non-aromatic carbocycle, monocyclic aromatic heterocycle or monocyclic non-aromatic heterocycle; p is any integer of 0 to 11; s is an integer of 0 to 11.
(30) The compound according to any one of the above (8) to (20) or (27) to (29) or its pharmaceutically acceptable salt, wherein R^{B'} is each independently, halogen, hydroxy, oxo, amino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl.
(31) The compound according to any one of the above (1) to (30) or its pharmaceutically acceptable salt, wherein R⁶ is substituted or unsubstituted alkyl.
(32) The compound according to any one of the above (1) to (31) or its pharmaceutically acceptable salt, wherein R³ is substituted or unsubstituted phenyl which may be fused, or substituted or unsubstituted 5- to 8-membered heterocyclyl which may be fused.
(33) The compound according to the above (1) represented by the following formula: 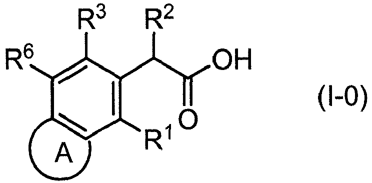 or its pharmaceutically acceptable salt,
   wherein
   ring A is substituted or unsubstituted heterocycle;
   R¹ is alkyl;
   or R¹ may be taken together with atom(s) constituting the ring A to form substituted or unsubstituted heterocycle;
   R² is alkyloxy, or substituted or unsubstituted cycloalkyloxy;
   R³ is substituted or unsubstituted phenyl which may be fused, or substituted or unsubstituted 5- to 8-membered heterocyclyl which may be fused;
   R⁶ is alkyl.
(34) The compound according to the above (1) represented by any one of following formulas: 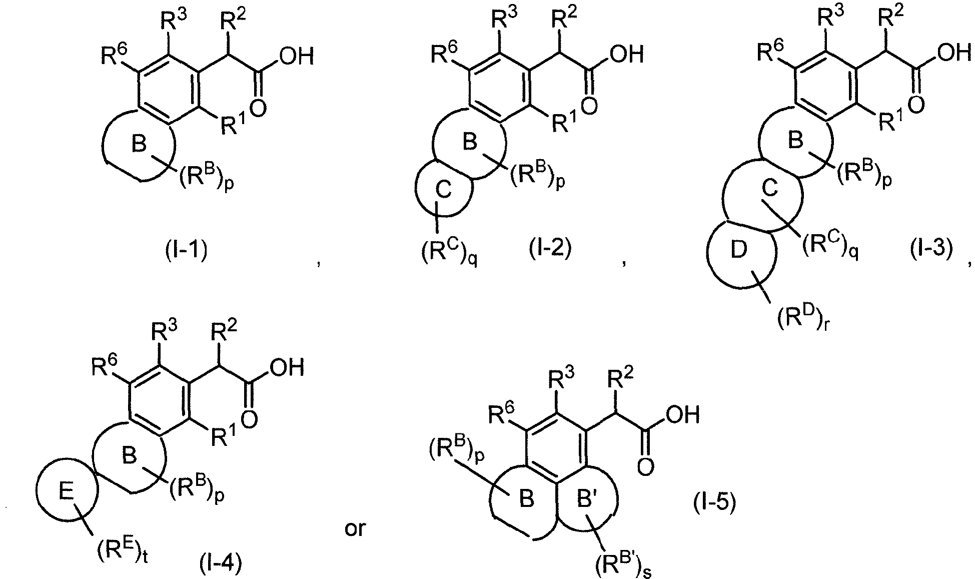 or its pharmaceutically acceptable salt,
   wherein
   R¹ is alkyl;
   R² is alkyloxy, or substituted or unsubstituted cycloalkyloxy;
   R³ is substituted or unsubstituted phenyl which may be fused, or substituted or unsubstituted 5- to 8-membered heterocyclyl which may be fused;
   R⁶ is alkyl;
   ring B, ring C, ring D, ring E, and ring B' are each independently monocyclic aromatic carbocycle, monocyclic non-aromatic carbocycle, monocyclic aromatic heterocycle or monocyclic non-aromatic heterocycle (each ring is preferably 5- to 11-membered ring, more preferably 6- to 10-membered ring, further preferably 6- to 8-membered ring);
   ring E is the same as defined as ring C;
   R^{B'} and R^{E} are each independently the same meaning as R^{C};
   t is any integer of 0 to 12;
   others are the same meaning as described above (8).
(35) The compound according to the above (1) represented by any one of following formulas: 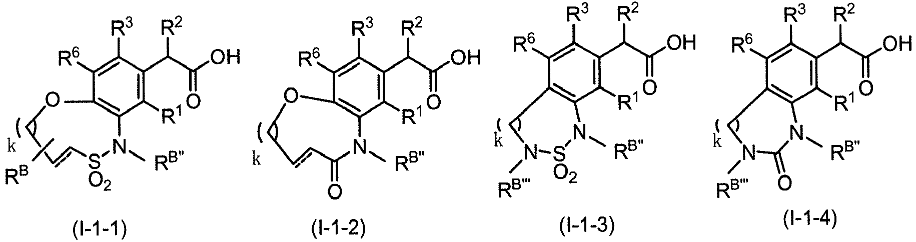 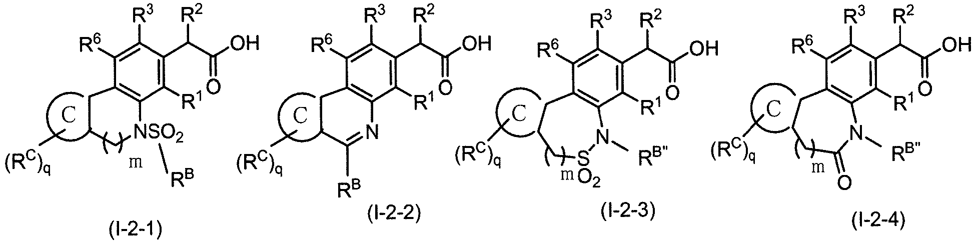 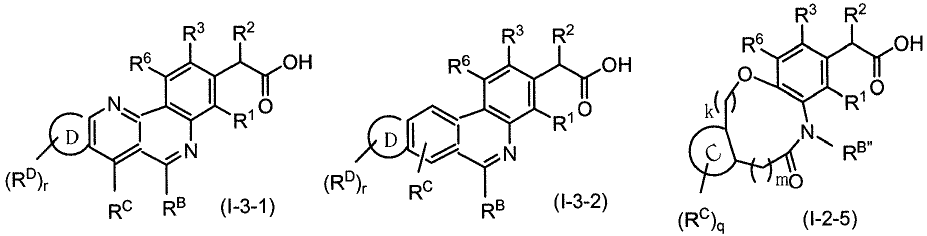 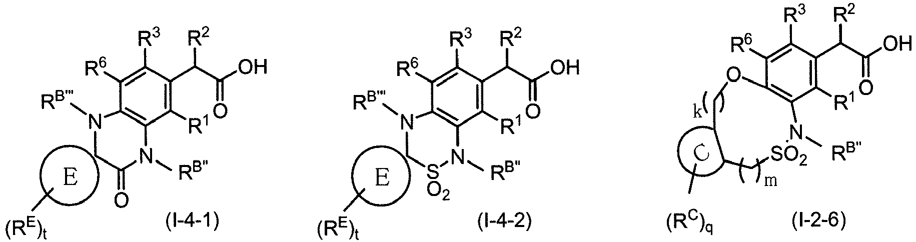 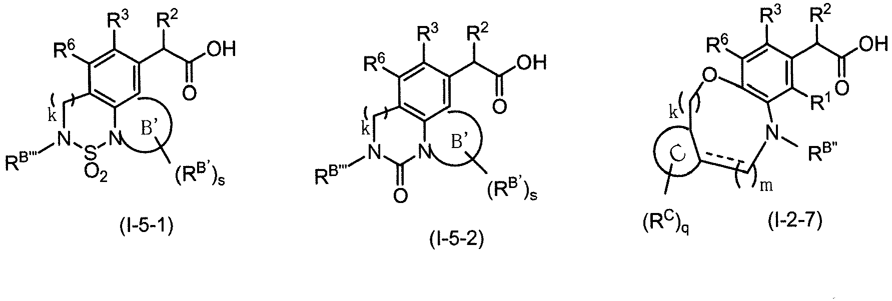 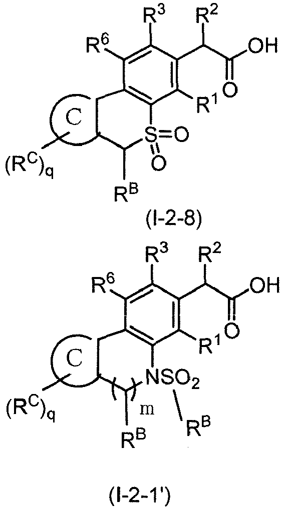 or its pharmaceutically acceptable salt,
   wherein
   ring C and ring D are each independently benzene or 5- to 8-membered heterocycle;
   ring B' is 5- to 8-membered heterocycle;
   ring E is optionally cross-linked 5- to 8-membered carbocycle;
   R^{B"} and R^{B'''} are each independently a hydrogen atom, alkyl, cycloalkyl, cycloalkylalkyl, formyl, alkoxycarbonyl, substituted or unsubstituted aryl, substituted or unsubstituted aralkylaralkyl, substituted or unsubstituted heterocyclyl, substituted or unsubstituted heterocyclylalkyl, or substituted sulfonyl; a broken line means the presence or absence of bond;
   k and m are each independently an integer of 0 to 4, preferably 0 to 2;
   others are the same meaning as described above (34).
(36) The compound according to the above (35) represented by formulas (I-2-1), (I-2-2), (I-2-3) or (I-2-4), or its pharmaceutically acceptable salt.
(37) The compound according to the above (35) or its pharmaceutically acceptable salt, wherein R^{B} and R^{C} each independently halogen, alkyl, haloalkyl, hydroxy, alkoxy, haloalkoxy, oxo, carboxy, or substituted or unsubstituted amino.
(38) The compound according to the above (1) represented by the following formula: 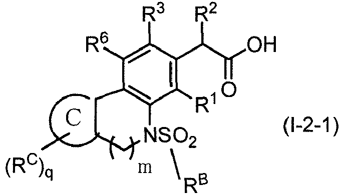 or its pharmaceutically acceptable salt,
   wherein
   R¹ is alkyl;
   R² is alkyloxy or cycloalkyloxy which may be substituted with methyl;
   R³ is substituted or unsubstituted phenyl which may be fused, or substituted or unsubstituted 5- to 8-membered heterocyclyl which may be fused;
   ring C is benzene or 5- to 8-membered heterocycle;
   R^{B} is alkyl;
   m is an integer of 0 to 4;
   R^{C} is each independently halogen, alkyl, alkoxy, haloalkyl, haloalkoxy, oxo, amino, or mono or di alkylamino;
   q is an integer of 0 to 4;
   m is an integer of 0 to 2, preferably 1;
   q is an integer of 0 to 2.
(39) The compound according to the above (38) represented by the following formula: 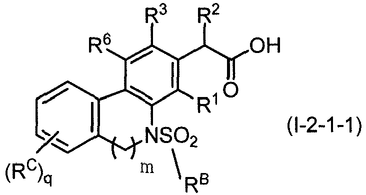 or its pharmaceutically acceptable salt,
   wherein each definition has the same meaning as described above (38);
   m is preferably a integer of 0 to 2, more preferably 1;
   q is preferably a integer of 0 to 2.
(40) The compound according to the above (38) or its pharmaceutically acceptable salt, wherein ring C is 5- to 8-membered heterocycle.
(41) The compound according to the above (38) represented by the following formulas: 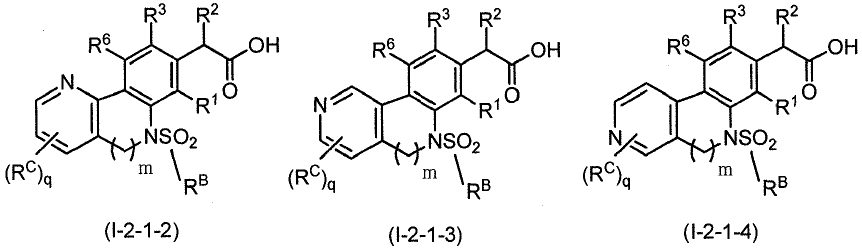 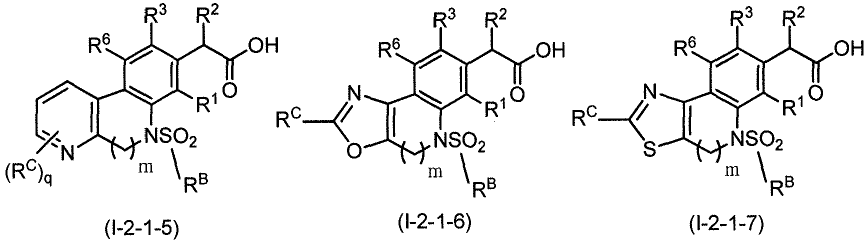 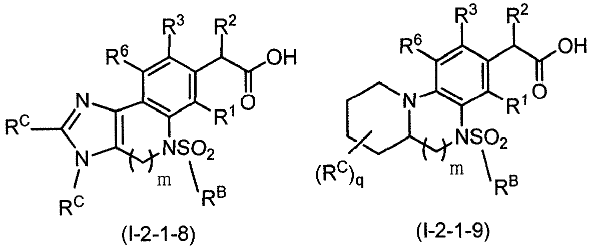 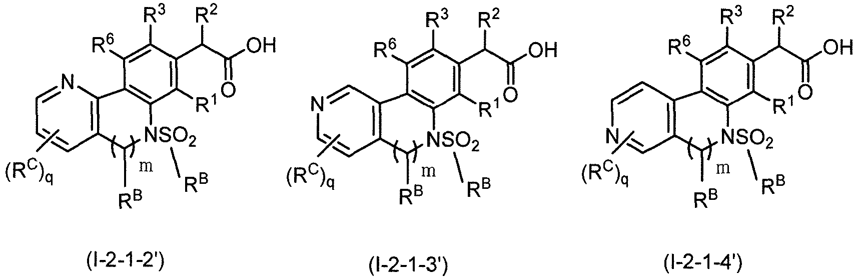 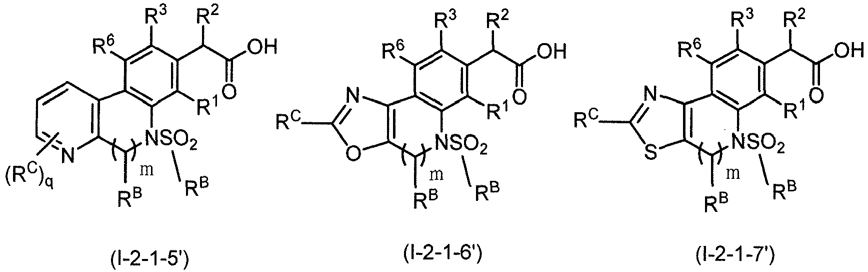 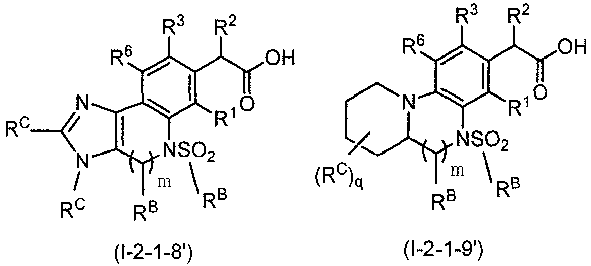 or its pharmaceutically acceptable salt,
   wherein q is an integer of 0 to 3; others are the same meaning as described above (38);
   m is preferably an integer of 0 to 2, more preferably 1;
   q is preferably an integer of 0 to 2.
(42) The compound according to any one of the above (1) to (41) or its pharmaceutically acceptable salt, wherein R³ is the following groups: 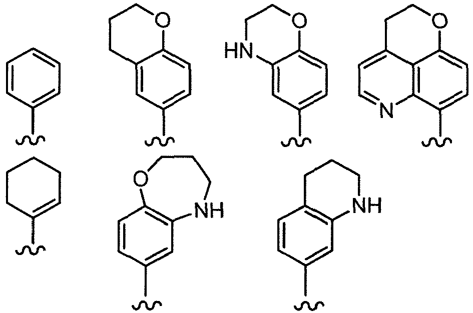 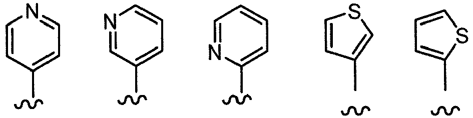 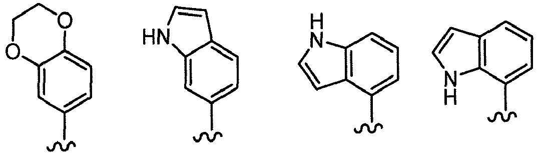 wherein the above groups may be substituted with same or different 1 to 4 substituent consisting of alkyl, alkoxy, halogen, hydroxy, hydroxyalkyl, alkoxyalkyl, haloalkyl, oxo, amino, mono or di alkylamino, aminoalkyl, mono or di alkylaminoalkyl and cyano.
(43) The compound according to any one of the above (1) to (41) or its pharmaceutically acceptable salt, wherein R³ is the following groups: 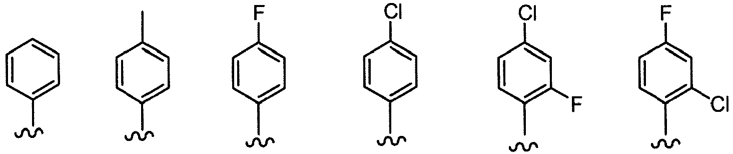 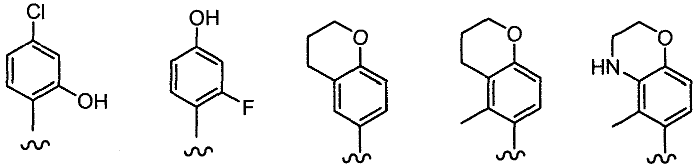 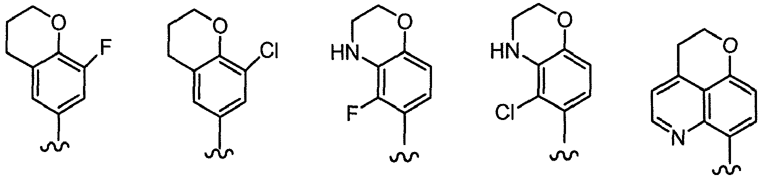 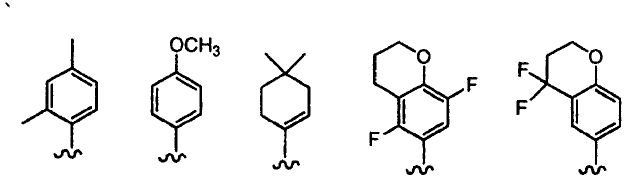 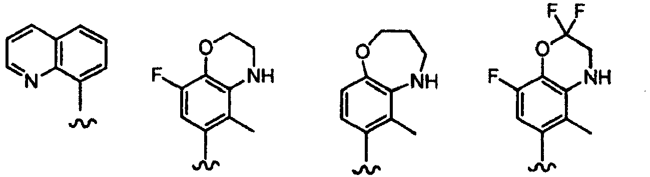 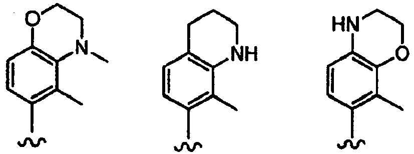 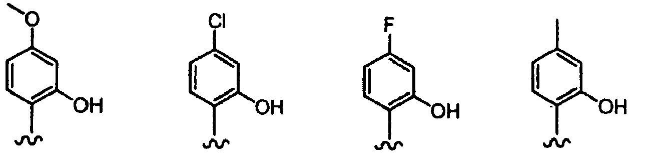 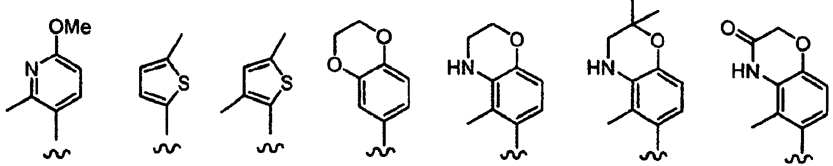 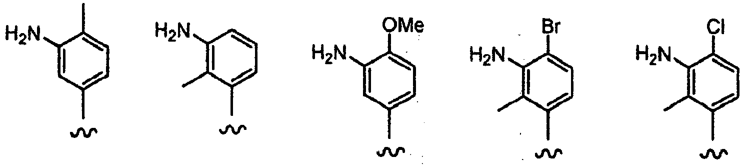 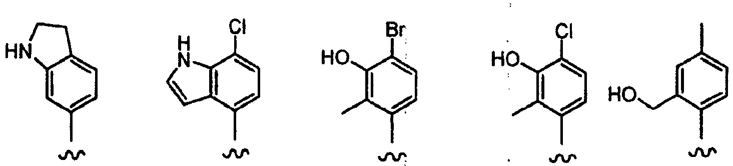
(44) A pharmaceutical composition comprising the compound according to any one of the above (1) to (43) or its pharmaceutically acceptable salt.
(45) The pharmaceutical composition according to the above (44), having anti-HIV activity.
(48) The compound according to any one of the above (1) to (43) or its pharmaceutically acceptable, for the treatment or prevention of viral infection.
(49) The compound according to the above (48) or its pharmaceutically acceptable salt, for the treatment or prevention of HIV infection.
(50) A use of the compound according to any one of the above (1) to (43) or its pharmaceutically acceptable salt, for the production of a therapeutic or prophylactic agent for viral infection.
(51) The use according to the above (60), for the production of a therapeutic or prophylactic agent for HIV infection.

### EFFECTS OF THE INVENTION

The compound of the present invention has a replication inhibitory activity on a virus, particularly HIV (example: HIV-1) and a resistant virus thereof. Accordingly, the compound of the present invention is useful in the prevention or treatment of viral infections (example: AIDS) and the like. The compound of the present invention is also useful as a synthetic intermediate for an antiviral drug.

### MODE FOR CARRYING OUT THE INVENTION

Each meaning of terms used herein is described below. Each term, alone or in combination with another word, is used in the same meaning.

The term of "halogen" includes a fluorine atom, a chlorine atom, a bromine atom and an iodine atom. A fluorine atom and a chlorine atom are preferable.

The term of "alkyl" includes a linear or branched hydrocarbon group having 1 to 15 carbon atom(s), preferably 1 to 10 carbon atom(s), more preferably 1 to 6 carbon atom(s), further preferably 1 to 4 carbon atom(s). For example, methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, n-decyl and the like are exemplified.

In a preferable embodiment of "alkyl", methyl, ethyl, n-propyl, isopropyl, n-butyl, isobutyl, sec-butyl, tert-butyl and n-pentyl are exemplified. In another preferable embodiment, methyl, ethyl, n-propyl, isopropyl and tert-butyl are exemplified.

The term of "alkenyl" includes a linear or branched hydrocarbon group having 2 to 15 carbon atoms, preferably 2 to 10 carbon atoms, more preferably 2 to 6 carbon atoms, further preferably 2 to 4 carbon atoms, and one or more double bond(s) at any available position. For example, vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, pentadecenyl and the like are exemplified.

In one preferable embodiment of "alkenyl", vinyl, allyl, propenyl, isopropenyl and butenyl are exemplified.

The term of "alkynyl" includes a linear or branched hydrocarbon group having 2 to 10 carbon atoms, preferably 2 to 8 carbon atoms, more preferably 2 to 6 carbon atoms, further preferably 2 to 4 carbon atoms, and one or more triple bond(s) at any available position. For example, ethynyl, propynyl, butynyl, pentynyl, hexynyl, heptynyl, octynyl, nonynyl, decynyl and the like are exemplified. These may have further a double bond at any available position.

In one preferable embodiment of "alkynyl", ethynyl, propynyl, butynyl, and pentynyl are exemplified.

The term of "alkylene" includes a linear or branched divalent hydrocarbon group having 1 to 15 carbon atom(s), preferably 1 to 10 carbon atom(s), more preferably 1 to 6 carbon atom(s), most preferably 1 to 4 carbon atom(s). For example, methylene, ethylene, trimethylene, propylene, tetramethylene, panta methylene, hexamethylene and the like are exemplified.

The term of "alkenylene" includes a linear or branched divalent hydrocarbon group having 2 to 15 carbon atoms, preferably having 2 to 10 carbon atoms, more preferably 2 to 6 carbon atoms, most preferably 2 to 4 carbon atoms, and one or more double bond(s) at any available position. For example, vinylene, propenylene, butenylene, pentenylene and the like are exemplified.

The term of "alkynylene" includes a linear divalent hydrocarbon group having 2 to 15 carbon atoms, preferably having 2 to 10 carbon atoms, more preferably 2 to 6 carbon atoms, most preferably 2 to 4 carbon atoms, and one or more triple bond(s) at any available position. For example, ethynylene, propynylene, butynylene, pentynylene, hexynylene and the like are exemplified.

The term of "carbocycle" includes a mono-, bi-, or more cyclic hydrocarbon group. In addition, the "carbocycle" also includes a fused ring, a spiro ring and a crosslinked ring. Constituent atom(s) of fused ring, the spiro ring and the crosslinked ring may include heteroatom(s). A mono carbocycle includes aromatic carbocycle and non-aromatic carbocycle.

As mono non-aromatic carbocycle, the mono non-aromatic carbocycle consisting of 3 to 16 carbon atoms is preferred, more preferably 3 to 12 carbon atoms, and further preferably 4 to 8 carbon atoms. For example, cycloalkane, cycloalkene and the like are exemplified.

As "cycloalkane", cyclopropane, cyclobutane, cyclopentane, cyclohexane, cycloheptane, cyclooctane, cyclononane and the like are exemplified.

As "cycloalkane", cyclopropene, cyclobutene, cyclopentene, cyclohexene, cycloheptene, cyclohexadiene and the like are exemplified.

The term of "heterocycle" includes a non-aromatic cyclic group which is monocyclic, or two or more rings, containing one or more the same or different of heteroatom(s) independently selected from oxygen, sulfur and nitrogen atoms, preferably 1 to 4 heteroatom(s). In addition, the "heterocycle" also includes a fused ring, a spiro ring and a crosslinked ring. Constituent atom(s) of fused ring, the spiro ring and the crosslinked ring may include heteroatom(s). A mono heterocycle includes aromatic heterocycle and non-aromatic heterocycle. A bi- or more cyclic heterocycle includes a fused ring wherein a heterocycle, which is monocyclic or polycyclic having two or more rings, is fused with a ring of the above "carbocycle". A heterocycle is preferably 5- to 7-membered ring or 8- to 12-membered ring.

"Carbocycle" and "heterocycle" also includes a cyclic group having a bridge or a cyclic group to form a spiro ring as follows:

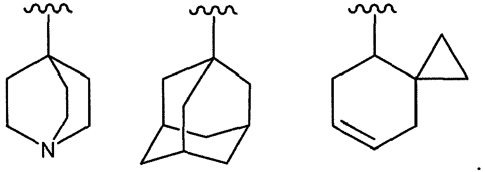

The term of "Crosslinked ring" includes a ring wherein two atoms constituting ring which is not adjacent to each other are bridged by alkylene, alkenylene, alkynylene and the like.

The term of "aromatic carbocyclyl" includes a mono-, bi-, or more cyclic aromatic hydrocarbon group. For example, phenyl, naphthyl, anthryl, phenanthryl, and the like are exemplified.

In one preferable embodiment of "aromatic carbocyclyl", phenyl is exemplified.

The term of "non-aromatic carbocyclyl" includes a mono-, bi-, or more cyclic, non-aromatic saturated hydrocarbon group or non-aromatic unsaturated hydrocarbon group. A bi- or more cyclic non-aromatic carbocyclyl includes a fused ring wherein a non-aromatic carbocycle, which is monocyclic or polycyclic having two or more rings, is fused with a ring of the above "aromatic carbocycle".

In addition, the "non-aromatic carbocyclyl" also includes a cyclic group having a bridge or a cyclic group to form a spiro ring as follows:

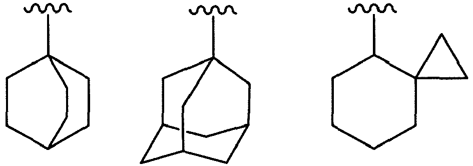

As a monocyclic non-aromatic carbocyclyl, 3 to 16 carbon atoms is preferred, more preferably 3 to 12 carbon atoms, further preferably 4 to 8 carbon atoms. For example, cycloalkyl, cycloalkenyl, and the like are exemplified.

As "cycloalkyl", the cycloalkyl consisting of 3 to 10 carbon atoms is preferred, and more preferably 3 to 7 carbon atoms. For example, cyclopropyl, cyclobutyl, cyclopentyl, cyclohexyl, cycloheptyl, cyclooctyl, cyclononyl, cyclodecyl, and the like are exemplified.

Examples of "cycloalkenyl" include cyclopropenyl, cyclobutenyl, cyclopentenyl, cyclohexenyl, cycloheptenyl, and cyclohexadienyl.

As a non-aromatic carbocyclyl of two or more rings, indanyl, indenyl, acenaphthyl, tetrahydronaphthyl, fluorenyl, dihydroindenyl and the like are exemplified.

The term of "aromatic heterocyclyl" includes an aromatic ring group which is monocyclic or polycyclic having two or more rings, containing one or more the same or different of heteroatom(s) independently selected from oxygen, sulfur and nitrogen atom(s) in the ring.

An "aromatic heterocyclyl" which is polycyclic having two or more rings includes a fused ring wherein an aromatic heterocycle, which is monocyclic or polycyclic having two or more rings is fused with a ring of the above "aromatic carbocycle".

As a monocyclic aromatic heterocyclyl, a 5- to 10-membered ring is preferred, more preferably 5- to 6- membered. For example, pyrrolyl, imidazolyl, pyrazolyl, pyridyle, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, thiadiazolyl and the like are exemplified.

As a bicyclic aromatic heterocyclyl, a 8- to 18-membered ring is preferred. For example, indolyl, isoindolyl, indazoryl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridinyl, thiazolopyridyle and the like are exemplified.

As an aromatic heterocyclyl which is polycyclic having three or more rings, a 11- to 26-membered ring is preferred. For example, carbazolyl, acridinyl, xanthenyl, phenothiazinyl, phenoxathiinyl, phenoxazinyl, dibenzofuryl and the like are exemplified.

The term of "non-aromatic heterocyclyl" includes a non-aromatic cyclic group which is monocyclic or polycyclic having two or more rings, containing one or more the same or different of heteroatom(s) independently selected from oxygen, sulfur and nitrogen atoms.

A "non-aromatic heterocyclyl" which is polycyclic having two or more rings includes a fused ring wherein a non-aromatic heterocyclyl which is monocyclic or polycyclic having two or more rings, is fused with a ring of the above "aromatic carbocyclyl", "non-aromatic carbocyclyl" and/or "aromatic heterocyclyl".

In addition, the "non-aromatic heterocyclyl" also includes a cyclic group having a bridge or a cyclic group to form a spiro ring as follows:

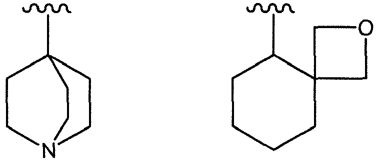

As a monocyclic non-aromatic heterocyclyl, a 3- to 8-membered ring is preferred, more preferably 5- to 6- membered. For example, dioxanyl, thiiranyl, oxiranyl, oxetanyl, oxathiolanyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl,tetrahydropyridazinyl, hexahydropyrimidinyl, dioxolanyl, dioxazinyl, aziridinyl, dioxolinyl, oxepanyl, thiolanyl, thiinyl, thiazinyl and the like are exemplified.

As a non-aromatic heterocyclyl which is polycyclic having two or more rings, for example, indolinyl, isoindolinyl, chromanyl, isochromanyl, dihydrobenzofuryl, benzodioxazolyl, benzodioxanyl, benzomorpholinyl and the like are exemplified.

The term of "hydroxyalkyl" includes a group wherein hydrogen atom(s) attached to one or more carbon atom(s) of above "alkyl" is (are) replaced with one or more hydroxy group(s). For example, hydroxymethy, 1-hydroxyethyl, 2-hydroxyethyl, 1-hydroxypropyl, 2-hydroxypropyl, 1,2-dihydroxyethyl and the like are exemplified.

In one preferable embodiment of "hydroxyalkyl", hydroxymethyl is exemplified.

The term of "alkyloxy" includes a group wherein an oxygen atom is substituted with the above "alkyl". For example, methyloxy, ethyloxy, n-propyloxy, isopropyloxy, n-butyloxy, tert-butyloxy, isobutyloxy, sec-butyloxy, pentyloxy, isopentyloxy, hexyloxy and the like are exemplified.

In one preferable embodiment of "alkyloxy", methyloxy, ethyloxy, n-propyloxy, isopropyloxy and tert-butyloxy are exemplified.

The term of "alkenyloxy" includes a group wherein an oxygen atom is substituted with the above "alkenyl". For example, vinyloxy, allyloxy, 1-propenyloxy, 2-butenyloxy, 2-pentenyloxy, 2-hexenyloxy, 2-heptenyloxy, 2-octenyloxy and the like are exemplified.

The term of "alkynyloxy" includes a group wherein an oxygen atom is substituted with the above "alkynyl". For example, ethynyloxy, 1-propynyloxy, 2-propynyloxy, 2-butynyloxy, 2-pentynyloxy, 2-hexynyloxy, 2-heptynyloxy, 2-octynyloxy and the like are exemplified.

The term of "haloalkyl" includes a group wherein hydrogen atom(s) attached to one or more carbon atom(s) of the above "alkyl" is (are) replaced with one or more above "halogen". For example, monofluoromethyl, monofluoroethyl, monofluoropropyl, 2,2,3,3,3-pentafluoropropyl, monochloromethyl, trifluoromethyl, trichloromethyl, 2,2,2-trifluoroethyl, 2,2,2-trichloroethyl, 1,2-dibromoethyl, 1,1,1-trifluoropropan-2-yl and the like are exemplified.

In one preferable embodiment of "haloalkyl", trifluoromethyl and trichloromethyl are exemplified.

The term of "haloalkyloxy" includes a group wherein an oxygen atom is substituted with one above "haloalkyl". For example, monofluoromethyloxy, monofluoroethyloxy, trifluoromethyloxy, trichloromethyloxy, trifluoroethyloxy, trichloroethyloxy and the like are exemplified.

In one preferable embodiment of "haloalkyloxy", trifluoromethyloxy and trichloromethyloxy are exemplified.

The term of "alkyloxyalkyl" includes a group wherein above "alkyl" is substituted with above "alkyloxy". For example, methyloxymethyl, methyloxyethyl, ethyloxymethyl and the like are exemplified.

The term of "alkyloxyalkyloxy" includes a group wherein above "alkyloxy" is substituted with above "alkyloxy". For example, methyloxymethyloxy, methyloxyethyloxy, ethyloxymethyloxy, ethyloxyethyloxy and the like are exemplified.

The term of "alkylcarbonyl" includes a group wherein a carbonyl is substituted with one above "alkyl". For example, methylcarbonyl, ethylcarbonyl, n-propylcarbonyl, isopropylcarbonyl, tert-butylcarbonyl, isobutylcarbonyl, sec-butylcarbonyl, pentylcarbonyl, isopentylcarbonyl, hexylcarbonyl and the like are exemplified.

In one preferable embodiment of "alkylcarbonyl", methylcarbonyl, ethylcarbonyl and n-propylcarbonyl are exemplified.

The term of "alkenylcarbonyl" includes a group wherein a carbonyl is substituted with one above "alkenyl". For example, ethylenylcarbonyl, propenylcarbonyl and the like are exemplified.

The term of "alkynylcarbonyl" includes a group wherein a carbonyl is substituted with one above "alkynyl". For example, ethynylcarbonyl, propynylcarbonyl and the like are exemplified.

The term of "monoalkylamino" includes a group wherein a hydrogen atom attached to a nitrogen atom of an amino group is replaced with above "alkyl". For example, methylamino, ethylamino, isopropylamino and the like are exemplified.

In one preferable embodiment of "monoalkylamino", methylamino and ethylamino are exemplified.

The term of "dialkylamino" includes a group wherein two hydrogen atoms attached to a nitrogen atom of an amino group are replaced with two above "alkyl". These two alkyl groups may be the same or different. For example, dimethylamino, diethylamino, N,N-diisopropylamino, N-methyl-N-ethylamino, N-isopropyl-N-ethylamino and the like are exemplified.

In one preferable embodiment of "dialkylamino", dimethylamino and diethylamino are exemplified.

The term of "alkylsulfonyl" includes a group wherein a sulfonyl is substituted with one above "alkyl". For example, methylsulfonyl, ethylsulfonyl, propylsulfonyl, isopropylsulfonyl, tert-butylsulfonyl, isobutylsulfonyl, sec-butylsulfonyl and the like are exemplified.

In one preferable embodiment of "alkylsulfonyl", methylsulfonyl and ethylsulfonyl are exemplified.

The term of "alkenylsulfonyl" includes a group wherein a sulfonyl is substituted with one above "alkenyl". For example, ethylenylsulfonyl, propenylsulfonyl and the like are exemplified.

The term of "alkynylsulfonyl" includes a group wherein a sulfonyl is substituted with one above "alkynyl". For example, ethynylsulfonyl, propynylsulfonyl and the like are exemplified.

The term of "monoalkylcarbonylamino" includes a group wherein a hydrogen atom attached to a nitrogen atom of an amino group is replaced with above "alkylcarbonyl". For example, methylcarbonylamino, ethylcarbonylamino, propylcarbonylamino, isopropylcarbonylamino, tert-butylcarbonylamino, isobutylcarbonylamino, sec-butylcarbonylamino and the like are exemplified.

In one preferable embodiment of "monoalkylcarbonylamino", methylcarbonylamino and ethylcarbonylamino are exemplified.

The term of "dialkylcarbonylamino" includes a group wherein two hydrogen atoms attached to a nitrogen atom of an amino group are replaced with above "alkylcarbonyl". The two "alkylcarbonyl" are identical or different. For example, dimethylcarbonylamino, diethylcarbonylamino, N, N-diisopropylcarbonylamino, and the like are exemplified.

In one preferable embodiment of "dialkylcarbonylamino", dimethylcarbonylamino and diethylcarbonylamino are exemplified.

The term of "monoalkylsulfonylamino" includes a group wherein a hydrogen atom attached to a nitrogen atom of an amino group is replaced with above "alkylsulfonyl". For example, methylsulfonylamino, ethylsulfonylamino, propylsulfonylamino, isopropylsulfonylamino, tert-butylsulfonylamino, isobutylsulfonylamino, sec-butylsulfonylamino and the like are exemplified.

In one preferable embodiment of "monoalkylsulfonylamino", methylsulfonylamino and ethylsulfonylamino are exemplified.

The term of "dilkylsulfonylamino" includes a group wherein two hydrogen atoms attached to a nitrogen atom of an amino group are replaced with above "alkylsulfonyl". For example, dimethylsulfonylamino, diethylsulfonylamino, N, N-diisopropylsulfonylamino and the like are exemplified.

In one preferable embodiment of "dialkylsulfonylamino", dimethylsulfonylamino and diethylsulfonylamino are exemplified.

The term of "alkylimino" includes a group wherein a hydrogen atom attached to a nitrogen atom of an imino group is replaced with above "alkyl". For example, methylimino, ethylimino, n-propylimino, isopropylimino and the like are exemplified.

The term of "alkenylimino" includes a group wherein a hydrogen atom attached to a nitrogen atom of an imino group is replaced with above "alkenyl". For example, ethylenylimino, propenylimino and the like are exemplified.

The term of "alkynylimino" includes a group wherein a hydrogen atom attached to a nitrogen atom of an imino group is replaced with above "alkynyl". For example, ethynylimino, propynylimino and the like are exemplified.

The term of "alkylcarbonylimino" includes a group wherein a hydrogen atom attached to a nitrogen atom of an imino group is replaced with above "alkylcarbonyl". For example, methylcarbonylimino, ethylcarbonylimino, n-propylcarbonylimino, isopropylcarbonylimino and the like are exemplified.

The term of "alkenylcarbonylimino" includes a group wherein a hydrogen atom attached to a nitrogen atom of an imino group is replaced with above "alkenylcarbonyl". For example, ethylenylcarbonylimino, propenylcarbonylimino and the like are exemplified.

The term of "alkynylcarbonylimino" includes a group wherein a hydrogen atom attached to a nitrogen atom of an imino group is replaced with above "alkynylcarbonyl". For example, ethynylcarbonylimino, propynylcarbonylimino and the like are exemplified.

The term of "alkyloxyimino" includes a group wherein a hydrogen atom attached to a nitrogen atom of an imino group is replaced with above "alkyloxy". For example, methyloxyimino, ethyloxyimino, n-propyloxyimino, isopropyloxyimino and the like are exemplified.

The term of "alkenyloxyimino" includes a group wherein a hydrogen atom attached to a nitrogen atom of an imino group is replaced with above "alkenyloxy". For example, ethylenyloxyimino, propenyloxyimino and the like are exemplified.

The term of "alkynyloxyimino" includes a group wherein a hydrogen atom attached to a nitrogen atom of an imino group is replaced with above "alkynyloxy". For example, ethynyloxyimino, propynyloxyimino and the like are exemplified.

The term of "alkylcarbonyloxy" includes a group wherein an oxygen atom is substituted with one above "alkylcarbonyl". For example, methylcarbonyloxy, ethylcarbonyloxy, propylcarbonyloxy, isopropylcarbonyloxy, tert-butylcarbonyloxy, isobutylcarbonyloxy, sec-butylcarbonyloxy and the like are exemplified.

In one preferable embodiment of "alkylcarbonyloxy", methylcarbonyloxy and ethylcarbonyloxy are exemplified.

The term of "alkenylcarbonyloxy" includes a group wherein an oxygen atom is substituted with one above "alkenylcarbonyl". For example, ethylenylcarbonyloxy, propenylcarbonyloxy and the like are exemplified.

The term of "alkynylcarbonyloxy" includes a group wherein an oxygen atom is substituted with one above "alkynylcarbonyl". For example, ethynylcarbonyloxy, propynylcarbonyloxy and the like are exemplified.

The term of "alkylsulfonyloxy" includes a group wherein an oxygen atom is substituted with one above "alkylsulfonyl". For example, methylsulfonyloxy, ethylsulfonyloxy, propylsulfonyloxy, isopropylsulfonyloxy, tert-butylsulfonyloxy, isobutylsulfonyloxy, sec-butylsulfonyloxy and the like are exemplified.

In one preferable embodiment of "alkylsulfonyloxy", methylsulfonyloxy and ethylsulfonyloxy are exemplified.

The term of "alkenylsulfonyloxy" includes a group wherein an oxygen atom is substituted with one above "alkenylsulfonyl". For example, ethylenylsulfonyloxy, propenylsulfonyloxy and the like are exemplified.

The term of "alkynylsulfonyloxy" includes a group wherein an oxygen atom is substituted with one above "alkynylsulfonyl". For example, ethynylsulfonyloxy, propynylsulfonyloxy and the like are exemplified.

The term of "alkyloxycarbonyl" includes a group wherein a carbonyl is substituted with one above "alkyloxy". For example, methyloxycarbonyl, ethyloxycarbonyl, propyloxycarbonyl, isopropyloxycarbonyl, tert-butyloxycarbonyl, isobutyloxycarbonyl, sec-butyloxycarbonyl, pentyloxycarbonyl, isopentyloxycarbonyl, hexyloxycarbonyl and the like are exemplified.
In one preferable embodiment of "alkyloxycarbonyl", methyloxycarbonyl, ethyloxycarbonyl and propyloxycarbonyl are exemplified.

The term of "alkenyloxycarbonyl" includes a group wherein a carbonyl is substituted with one above "alkenyloxy". For example, ethylenyloxycarbonyl, propenyloxycarbonyl and the like are exemplified.

The term of "alkynyloxycarbonyl" includes a group wherein a carbonyl is substituted with one above "alkynyloxy". For example, ethynyloxycarbonyl, propynyloxycarbonyl, and the like are exemplified.

The term of "alkylsulfanyl" includes a group wherein a hydrogen atom attached to a sulfur atom of a sulfanyl is replaced with one above "alkyl". For example, methylsulfanyl, ethylsulfanyl, n-propylsulfanyl, isopropylsulfanyl, tert-butylsulfanyl, isobutylsulfanyl and the like are exemplified.

The term of "alkenylsulfanyl" includes a group wherein a hydrogen atom attached to a sulfur atom of sulfanyl is replaced with one above "alkenyl". For example, ethylenylsulfanyl, propenylsulfanyl and the like are exemplified.

The term of "alkynylsulfanyl" includes a group wherein a hydrogen atom attached to a sulfur atom of sulfanyl is replaced with one above "alkynyl". For example, ethynylsulfanyl, propynylsulfanyl, and the like are exemplified.

The term of "alkylsulfinyl" includes a group wherein a sulfinyl is substituted with one above "alkyl". For example, methylsulfinyl, ethylsulfinyl, n-propylsulfinyl, isopropylsulfinyl and the like are exemplified.

The term of "alkenylsulfinyl" includes a group wherein a sulfinyl is substituted with one above "alkenyl". For example, ethylenylsulfinyl, propenylsulfinyl, and the like are exemplified.

The term of "alkynylsulfinyl" includes a group in which a sulfinyl is substituted with one above "alkynyl". For example, ethynylsulfinyl, propynylsulfinyl and the like are exemplified.

The term of "monoalkylcarbamoyl" includes a group wherein a hydrogen atom attached to a nitrogen atom of a carbamoyl group is replaced with above "alkyl". For example, methylcarbamoyl, ethylcarbamoyl and the like are exemplified.

The term of "dialkylcarbamoyl" includes a group wherein two hydrogen atoms attached to a nitrogen atom of a carbamoyl group are replaced with two above "alkyl". These two alkyl groups may be the same or different. For example, dimethylcarbamoyl, diethylcarbamoyl, and the like are exemplified.

The term of "monoalkylsulfamoyl" includes a group wherein a hydrogen atom attached to a nitrogen atom of a sulfamoyl is replaced with one above "alkyl". For example, methylsulfamoyl and the like are exemplified.

The term of "dialkylsulfamoyl" includes a group wherein two hydrogen atoms attached to a nitrogen atom of a sulfamoyl are replaced with two above "alkyl". These two alkyl groups may be the same or different. For example, dimethylcarbamoyl, diethylcarbamoyl and the like are exemplified.

The term of "trialkylsilyl" includes a group wherein a silicon atom is substituted with three above "alkyl". These three alkyl groups may be the same or different. For example, trimethylsilyl, triethylsilyl, tert-butyldimethylsilyl and the like are exemplified.

The alkyl portion of "aromatic carbocyclyl alkyl", "non-aromatic carbocyclyl alkyl", "aromatic heterocyclyl alkyl" and "non-aromatic heterocyclyl alkyl", "aromatic carbocyclyl alkyloxy", "non-aromatic carbocyclyl alkyloxy", "aromatic heterocyclyl alkyloxy" and"non-aromatic heterocyclyl alkyloxy", "aromatic carbocyclyl alkylsulfanyl", "non-aromatic carbocyclyl alkylsulfanyl", "aromatic heterocyclyl alkylsulfanyl" and "non-aromatic heterocyclyl alkylsulfanyl",
"aromatic carbocyclyl alkyloxycarbonyl", "non-aromatic carbocyclyl alkyloxycarbonyl", "aromatic heterocyclyl alkyloxycarbonyl" and"non-aromatic heterocyclyl alkyloxycarbonyl",
"aromatic carbocyclyl alkyloxyalkyl", "non-aromatic carbocyclyl alkyloxyalkyl", "aromatic heterocyclyl alkyloxyalkyl" and"non-aromatic heterocyclyl alkyloxyalkyl", and
"aromatic carbocyclyl alkylamino", "non-aromatic carbocyclyl alkylamino", "aromatic heterocyclyl alkylamino" and"non-aromatic heterocyclyl alkylamino" means the aforementioned "alkyl".

The term of "aromatic carbocyclyl alkyl" or aralkyl include an alkyl substituted with one or more above "aromatic carbocyclyl". Examples thereof include such as benzyl, phenethyl, phenylpropyl, benzhydryl, trityl, naphthylmethyl and a group of the formula of

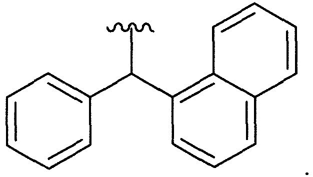

In one preferable embodiment of "aromatic carbocyclyl alkyl", benzyl, phenethyl and benzhydryl are exemplified.

The term of "non-aromatic carbocyclyl alkyl" includes an alkyl substituted with one or more above "non-aromatic carbocyclyl". Also, "non-aromatic carbocyclyl alkyl" includes a "non-aromatic carbocyclyl alkyl" wherein the alkyl portion thereof is substituted with one or more above "aromatic carbocyclyl". Examples thereof include cyclopropylmethyl, cyclobutylmethyl, cyclopentylmethyl, cyclohexylmethyl and a group of the formula of

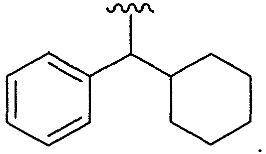

The term of "aromatic heterocyclyl alkyl" includes an alkyl substituted with one or more above "aromatic heterocyclyl". Also, "aromatic heterocyclyl alkyl" includes an "aromatic heterocyclyl alkyl" wherein the alkyl portion thereof is substituted with one or more above "aromatic carbocyclyl", and/or "non-aromatic carbocyclyl". Examples thereof include pyridylmethyl, furanylmethyl, imidazolylmethyl, indolylmethyl, benzothiophenylmethyl, oxazolylmethyl, isoxazolylmethyl, thiazolylmethyl, isothiazolylmethyl, pyrazolylmethyl, isopyrazolylmethyl, pyrrolidinylmethyl, benzoxazolylmethyl and groups of the formula of

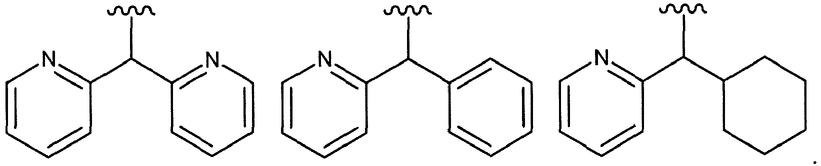

The term of "non-aromatic heterocyclyl alkyl" includes an alkyl substituted with one or more above "non-aromatic heterocyclyl". Also, "non-aromatic heterocyclyl alkyl" includes a "non-aromatic heterocyclyl alkyl" wherein the alkyl portion thereof is substituted with one or more above "aromatic carbocyclyl", "non-aromatic carbocyclyl" and/or "aromatic heterocyclyl". Examples thereof include tetrahydropyranylmethyl, morpholinylmethyl, morpholinylethyl, piperidinylmethyl, piperazinylmethyl and groups of the formula of

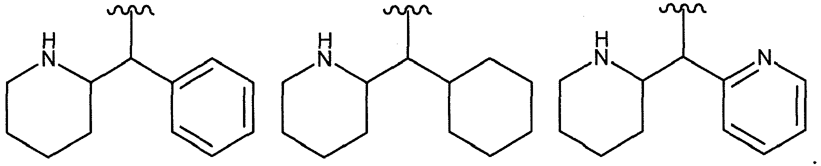

The term of "aromatic carbocyclyl alkyloxy" includes an alkyloxy substituted with one or more above "aromatic carbocyclyl". Examples thereof include such as benzyloxy, phenethyloxy, phenylpropynyloxy, benzhydryloxy, trityloxy, naphthylmethyloxy and a group of the formula of

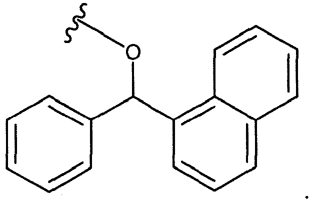

The term of "non-aromatic carbocyclyl alkyloxy" includes an alkyloxy substituted with one or more above "non-aromatic carbocyclyl". Also, "non-aromatic carbocyclyl alkyloxy" includes a "non-aromatic carbocyclyl alkyloxy" wherein the alkyl portion is substituted with one or more above "aromatic carbocyclyl". Examples thereof include cyclopropylmethyloxy, cyclobutylmethyloxy, cyclopentylmethyloxy, cyclohexylmethyloxy and a group of the formula of

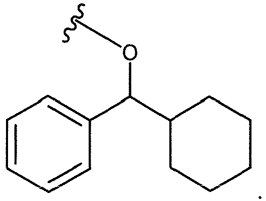

The term of "aromatic heterocyclyl alkyloxy" includes an alkyloxy substituted with one or more above "aromatic heterocyclyl". Also, "aromatic heterocyclyl alkyloxy" includes an "aromatic heterocyclyl alkyloxy" wherein the alkyl portion thereof is substituted with one or more above "aromatic carbocyclyl", and/or "non-aromatic carbocyclyl". Examples thereof include pyridylmethyloxy, furanylmethyloxy, imidazolylmethyloxy, indolylmethyloxy, benzothiophenylmethyloxy oxazolylmethyloxy, isoxazolylmethyloxy, thiazolylmethyloxy, isothiazolylmethyloxy, pyrazolylmethyloxy, isopyrazolylmethyloxy, pyrrolidinylmethyloxy, benzoxazolylmethyloxy and groups of the formula of

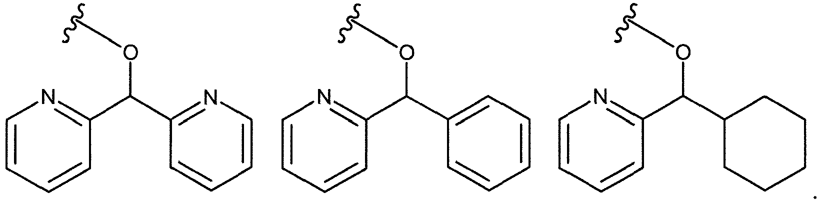

The term of "non-aromatic heterocyclyl alkyloxy" includes an alkyloxy substituted with one or more above "non-aromatic heterocyclyl". Also, "non-aromatic heterocyclyl alkyloxy" includes a "non-aromatic heterocyclyl alkyloxy" wherein the alkyl portion thereof is substituted with one or more above "aromatic carbocyclyl", "non-aromatic carbocyclyl" and/or "aromatic heterocyclyl". Examples thereof include tetrahydropyranylmethyloxy, morpholinylmethyloxy, morpholinylethyloxy, piperidinylmethyloxy, piperazinylmethyloxy and groups of the formula of

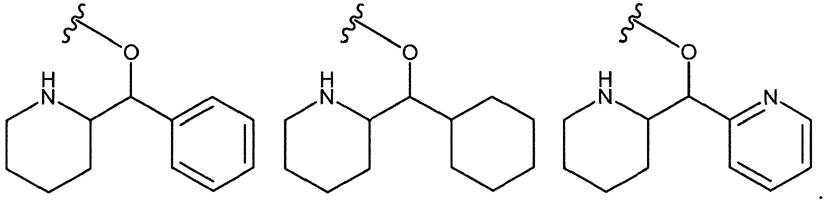

The term of "aromatic carbocyclyl alkylsulfanyl" includes an alkylsulfanyl substituted with one or more above "aromatic carbocyclyl". Examples thereof include benzylsulfanyl, phenethylsulfanyl, phenylpropynylsulfanyl, benzhydrylsulfanyl, tritylsulfanyl, naphthylmethylsulfanyl and the like.

The term of "non-aromatic carbocyclylalkylsulfanyl" includes an alkylsulfanyl substituted with one or more above "non-aromatic carbocyclyl". Also, "non-aromatic carbocyclyl alkylsulfanyl" includes a "non-aromatic carbocyclyl alkylsulfanyl" wherein the alkyl portion is substituted with one or more above "aromatic carbocyclyl". Examples thereof include cyclopropylmethylsulfanyl, cyclobutylmethylsulfanyl, cyclopentylmethylsulfanyl, and cyclohexylmethylsulfanyl.

The term of "aromatic heterocyclyl alkylsulfanyl" includes an alkylsulfanyl substituted with one or more above "aromatic heterocyclyl". Also, "aromatic heterocyclyl alkylsulfanyl" includes an "aromatic heterocyclyl alkylsulfanyl" wherein the alkyl portion is substituted with one or more above "aromatic carbocyclyl", and/or "non-aromatic carbocyclyl". Examples thereof include pyridylmethylsulfanyl, furanylmethylsulfanyl, imidazolylmethylsulfanyl, indolylmethylsulfanyl, benzothiophenylmethylsulfanyl, oxazolylmethylsulfanyl, isoxazolylmethylsulfanyl, thiazolylmethylsulfanyl, isothiazolylmethylsulfanyl, pyrazolylmethylsulfanyl, isopyrazolylmethylsulfanyl, pyrrolidinylmethylsulfanyl, benzoxazolylmethylsulfanyl and the like.

The term of "non-aromatic heterocyclyl alkylsulfanyl" includes an alkylsulfanyl substituted with one or more above "non-aromatic heterocyclyl". Also, "non-aromatic heterocyclyl alkylsulfanyl" includes a "non-aromatic heterocyclyl alkylsulfanyl" wherein the alkyl portion is substituted with one or more above "aromatic carbocyclyl", "non-aromatic carbocyclyl" and/or "aromatic heterocyclyl". Examples thereof include tetrahydropyranylmethylsulfanyl, morpholinylmethylsulfanyl, morpholinylethylsulfanyl, piperidinylmethylsulfanyl, piperazinylmethylsulfanyl and the like.

The term of "aromatic carbocyclyl alkyloxycarbonyl" includes an alkyloxycarbonyl substituted with one or more above "aromatic carbocyclyl". Examples thereof include benzyloxycarbonyl, phenethyloxycarbonyl, phenylpropynyloxycarbonyl, benzhydryloxycarbonyl, trityloxycarbonyl, naphthylmethyloxycarbonyl and a group of the formula of

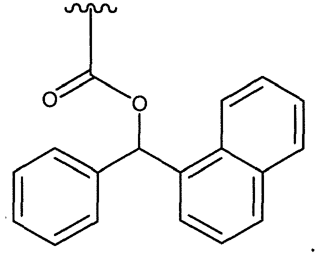

The term of "non-aromatic carbocyclyl alkyloxycarbonyl" includes an alkyloxycarbonyl substituted with one or more above "non-aromatic carbocyclyl". Also, "non-aromatic carbocyclyl alkyloxycarbonyl" includes a "non-aromatic carbocyclyl alkyloxycarbonyl" wherein the alkyl portion is substituted with one or more above "aromatic carbocyclyl". Examples thereof include cyclopropylmethyloxycarbonyl, cyclobutylmethyloxycarbonyl, cyclopentylmethyloxycarbonyl, cyclohexylmethyloxycarbonyl and a group of the formula of

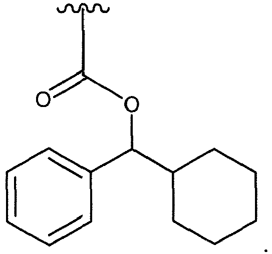

The term of "aromatic heterocyclyl alkyloxycarbonyl" includes an alkyloxycarbonyl substituted with one or more above "aromatic heterocyclyl". Also, "aromatic heterocyclyl alkyloxycarbonyl" includes an "aromatic heterocyclyl alkyloxycarbonyl" wherein the alkyl portion thereof is substituted with one or more above "aromatic carbocyclyl", and/or "non-aromatic carbocyclyl". Examples thereof include pyridylmethyloxycarbonyl, furanylmethyloxycarbonyl, imidazolylmethyloxycarbonyl, indolylmethyloxycarbonyl, benzothiophenylmethyloxycarbonyl, oxazolylmethyloxycarbonyl, isoxazolylmethyloxycarbonyl, thiazolylmethyloxycarbonyl, isothiazolylmethyloxycarbonyl, pyrazolylmethyloxycarbonyl, isopyrazolylmethyloxycarbonyl, pyrrolidinylmethyloxycarbonyl, benzoxazolylmethyloxycarbonyl and groups of the formula of

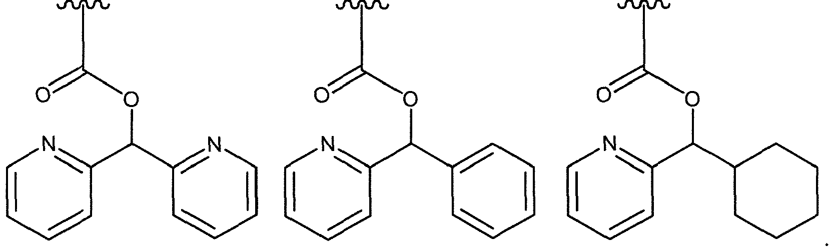

The term of "non-aromatic heterocyclyl alkyloxycarbonyl" includes an alkyloxycarbonyl substituted with one or more above "non-aromatic heterocyclyl". Also, "non-aromatic heterocyclyl alkyloxycarbonyl" includes a "non-aromatic heterocyclyl alkyloxycarbonyl" wherein the alkyl portion thereof is substituted with one or more above "aromatic carbocyclyl", "non-aromatic carbocyclyl" and/or "aromatic heterocyclyl". Examples thereof include tetrahydropyranylmethyloxy, morpholinylmethyloxy, morpholinylethyloxy, piperidinylmethyloxy, piperazinylmethyloxy and groups of the formula of

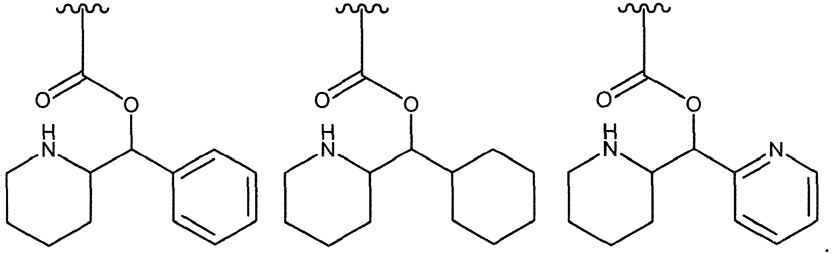

The term of "aromatic carbocyclyl alkyloxyalkyl" includes an alkyloxyalkyl substituted with one or more above "aromatic carbocyclyl". Examples thereof include benzyloxymethyl, phenethyloxymethyl, phenylpropynyloxymethyl, benzhydryloxymethyl, trityloxymethyl, naphthylmethyloxymethyl and a group of the formula of

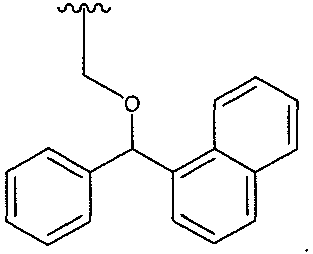

The term of "non-aromatic carbocyclyl alkyloxyalkyl" includes an alkyloxyalkyl substituted with one or more above "non-aromatic carbocyclyl". Also, "non-aromatic carbocyclyl alkyloxyalkyl" includes a "non-aromatic carbocyclyl alkyloxyalkyl" wherein the alkyl portion attached to a non-aromatic carbocycle is substituted with one or more above "aromatic carbocyclyl". Examples thereof include cyclopropylmethyloxymethyl, cyclobutylmethyloxymethyl, cyclopentylmethyloxymethyl, cyclohexylmethyloxymethyl and a group of the formula of

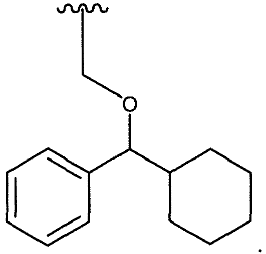

The term of "aromatic heterocyclyl alkyloxyalkyl" includes an alkyloxyalkyl substituted with one or more above "aromatic heterocyclyl". Also, "aromatic heterocyclyl alkyloxyalkyl" includes an "aromatic heterocyclyl alkyloxyalkyl" wherein the alkyl portion attached to aromatic heterocycle is substituted with one or more above "aromatic carbocyclyl" and/or "non-aromatic carbocyclyl". Examples thereof include pyridylmethyloxymethyl, furanylmethyloxymethyl, imidazolylmethyloxymethyl, indolylmethyloxymethyl, benzothiophenylmethyloxymethyl, oxazolylmethyloxymethyl, isoxazolylmethyloxymethyl, thiazolylmethyloxymethyl, isothiazolylmethyloxymethyl, pyrazolylmethyloxymethyl, isopyrazolylmethyloxymethyl, pyrrolidinylmethyloxymethyl, benzoxazolylmethyloxymethyl and groups of the formula of

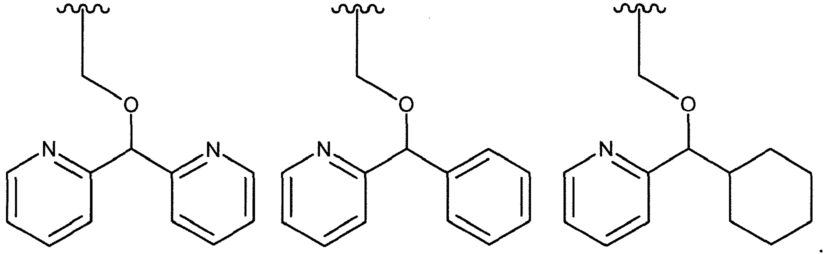

The term of "non-aromatic heterocyclyl alkyloxyalkyl" includes an alkyloxyalkyl substituted with one or more above "non-aromatic heterocyclyl". Also, "non-aromatic heterocyclyl alkyloxyalkyl" includes a "non-aromatic heterocyclyl alkyloxyalkyl" wherein the alkyl portion attached to non-aromatic heterocycle is substituted with one or more above "aromatic carbocyclyl", "non-aromatic carbocyclyl" and/or "aromatic heterocyclyl". Examples thereof include tetrahydropyranylmethyloxymethyl, morpholinylmethyloxymethyl, morpholinylethyloxymethyl, piperidinylmethyloxymethyl, piperazinylmethyloxymethyl and groups of the formula of

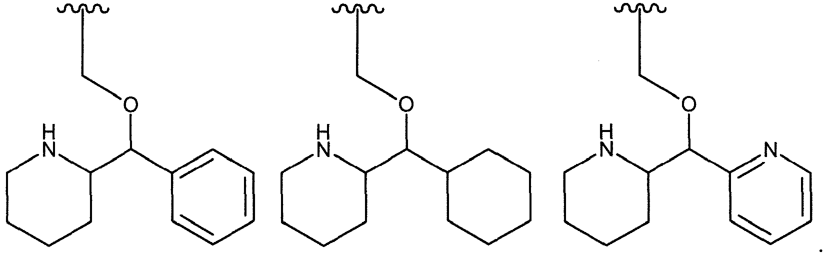

The term of "aromatic carbocyclyl alkylamino" includes a group wherein one or two hydrogen atom(s) attached to a nitrogen atom of an amino group is replaced with above "aromatic carbocyclyl alkyl". Examples include benzylamino, phenethylamino, phenylpropynylamino, benzhydrylamino, tritylamino, naphthylmethylamino, dibenzylamino and the like.

The term of "non-aromatic carbocyclyl alkylamino" includes a group wherein one or two hydrogen atom(s) attached to a nitrogen atom of an amino group is replaced with above "non-aromatic carbocyclyl alkyl". Examples include cyclopropylmethylamino, cyclobutylmethylamino, cyclopentylmethylamino, cyclohexylmethylamino and the like.

The term of "aromatic heterocyclyl alkylamino" includes a group wherein one or two hydrogen atom(s) attached to a nitrogen atom of an amino group is replaced with above "aromatic heterocyclyl alkyl". Examples include pyridylmethylamino, furanylmethylamino, imidazolylmethylamino, indolylmethylamino, benzothiophenylmethylamino, oxazolylmethylamino, isoxazolylmethylamino, thiazolylmethylamino, isothiazolylmethylamino, pyrazolylmethylamino, isopyrazolylmethylamino, pyrrolidinylmethylamino, benzoxazolylmethylamino and the like.

The term of "non-aromatic heterocyclyl alkylamino" includes a group wherein one or two hydrogen atom(s) attached to a nitrogen atom of an amino group is replaced with above "non-aromatic heterocyclyl alkyl". Examples include tetrahydropyranylmethylamino, morpholinylethylamino, piperidinylmethylamino, piperazinylmethyamino and the like.

The "aromatic carbocycle" portion of "aromatic carbocyclyl oxy", "aromatic carbocyclyl amino", "aromatic carbocyclyl carbonyl", "aromatic carbocyclyl oxycarbonyl", "aromatic carbocyclyl carbonylamino", "aromatic carbocyclyl sulfanyl" and "aromatic carbocyclyl sulfonyl" means the aforementioned "aromatic carbocyclyl".

The term of "aromatic carbocyclyl oxy" includes a group wherein an oxygen atom is substituted with one above "aromatic carbocycle". Examples include phenyloxy, naphthyloxy and the like.

The term of "aromatic carbocyclyl amino" includes a group wherein the nitrogen atom of amino is attached to above "aromatic carbocycle". Examples include phenylamino, naphthylamino and the like.

The term of "aromatic carbocyclyl carbonyl" includes a group wherein a carbonyl is substituted with one above "aromatic carbocycle". Examples include phenylcarbonyl, naphthylcarbonyl and the like.

The term of "aromatic carbocyclyl oxycarbonyl" includes a group wherein a carbonyl is substituted with one above "aromatic carbocyclyl oxy". Examples include phenyloxycarbonyl, naphthyloxycarbonyl and the like.

The term of "aromatic carbocyclyl carbonylamino" includes a group wherein the nitrogen atom of amino is attached to above "aromatic carbocycle carbonyl". Examples include phenylcarbonylamino, naphthylcarbonylamino and the like.

The term of "aromatic carbocyclyl sulfanyl" includes a group wherein a hydrogen atom attached to a sulfur atom of a sulfanyl is replaced with "aromatic carbocycle". Examples include phenylsulfanyl, naphthylsulfanyl and the like.

The term of "aromatic carbocyclyl sulfonyl" includes a group wherein a sulfonyl is substituted with one above "aromatic carbocycle". Examples include phenylsulfonyl, naphthylsulfonyl and the like.

The "non-aromatic carbocycle" portion of "non-aromatic carbocyclyl oxy", "non-aromatic carbocyclyl amino", "non-aromatic carbocyclyl carbonyl", "non-aromatic carbocyclyl oxycarbonyl", "non-aromatic carbocyclyl carbonylamino", "non-aromatic carbocyclyl sulfanyl" and "non-aromatic carbocyclyl sulfonyl" means the aforementioned "non-aromatic carbocyclyl".

The term of "non-aromatic carbocyclyl oxy" includes a group wherein an oxygen atom is substituted with one above "non-aromatic carbocycle". Examples include cyclopropyloxy, cyclohexyloxy, cyclohexenyloxy and the like.

The term of "non-aromatic carbocyclyl amino" includes a group wherein one or two hydrogen atom(s) attached to a nitrogen atom of an amino group is replaced with above "non-aromatic carbocycle". Examples include cyclopropylamino, cyclohexylamino, cyclohexenylamino, and the like.

The term of "non-aromatic carbocyclyl carbonyl" includes a group wherein a carbonyl is substituted with one above "non-aromatic carbocycle". Examples include cyclopropylcarbonyl, cyclohexylcarbonyl, cyclohexenylcarbonyl and the like.

The term of "non-aromatic carbocyclyl oxycarbonyl" includes a group wherein a carbonyl is substituted with one above "non-aromatic carbocyclyl oxy". Examples include cyclopropyloxycarbonyl, cyclohexyloxycarbonyl, cyclohexenyloxycarbonyl and the like.

The term of "non-aromatic carbocyclyl carbonylamino" includes a group wherein one or two hydrogen atom(s) attached to a nitrogen atom of an amino group is replaced with above "non-aromatic carbocyclecarbonyl". Examples include cyclopropylcarbonylamino, cyclohexylcarbonylamino, cyclohexenylcarbonylamino, and the like.

The term of "non-aromatic carbocyclyl sulfanyl" includes a group wherein a hydrogen atom attached to a sulfur atom of a sulfanyl is replaced with one above "non-aromatic carbocycle". Examples include cyclopropylsulfanyl, cyclohexylsulfanyl, cyclohexenylsulfanyl and the like.

The term of "non-aromatic carbocyclyl sulfonyl" includes a group wherein a sulfonyl is substituted with one above "non-aromatic carbocycle". Examples include cyclopropylsulfonyl, cyclohexylsulfonyl, cyclohexenylsulfonyl and the like.

The "aromatic heterocycle" portion of "aromatic heterocyclyl oxy", "aromatic heterocyclylamino", "aromatic heterocyclyl carbonyl", "aromatic heterocyclyl oxycarbonyl", "aromatic heterocyclylcarbonylamino", "aromatic heterocyclyl sulfanyl" and "aromatic heterocyclyl sulfonyl" means the aforementioned "aromatic heterocyclyl".

The term of "aromatic heterocyclyl oxy" includes a group wherein an oxygen atom is substituted with one above "aromatic heterocycle". Examples include pyridyloxy, oxazolyloxy and the like.

The term of "aromatic heterocyclyl amino" includes a group wherein one or two hydrogen atom(s) attached to a nitrogen atom of an amino group is replaced with "aromatic heterocycle". Examples include pyridylamino, oxazolylamino and the like.

The term of "aromatic heterocyclyl carbonyl" includes a group wherein a carbonyl is substituted with one above "aromatic heterocycle". Examples include pyridylcarbonyl, oxazolylcarbonyl and the like.

The term of "aromatic heterocyclyl oxycarbonyl" includes a group wherein a carbonyl is substituted with one above "aromatic heterocyclyl oxy". Examples include pyridyloxycarbonyl, oxazolyloxycarbonyl and the like.

The term of "aromatic heterocyclyl carbonylamino" includes a group wherein one or two hydrogen atom(s) attached to a nitrogen atom of an amino group is replaced with "aromatic heterocycle". Examples include pyridylcarbonylamino, oxazolylcarbonylamino and the like.

The term of "aromatic heterocyclyl sulfanyl" includes a group wherein a hydrogen atom attached to a sulfur atom of a sulfanyl is replaced with one above "aromatic heterocycle". Examples include pyridylsulfanyl, oxazolylsulfanyl and the like.

The term of "aromatic heterocyclyl sulfonyl" includes a group wherein a sulfonyl is substituted with one above "aromatic heterocycle". Examples include pyridylsulfonyl, oxazolylsulfonyl and the like.

The "non-aromatic heterocycle" portion of "non-aromatic heterocyclyl oxy", "non-aromatic heterocyclyl amino", "non-aromatic heterocyclyl carbonyl", "non-aromatic heterocyclyl oxycarbonyl", "non-aromatic heterocyclyl carbonylamio", "non-aromatic heterocyclyl sulfanyl" and "non-aromatic heterocyclyl sulfonyl" means the aforementioned "non-aromatic heterocyclyl".

The term of "non-aromatic heterocyclyl oxy" includes a group wherein "non-aromatic heterocycle" is attached to an oxygen atom. Examples include piperidinyloxy, tetrahydrofuryloxy and the like.

The term of "non-aromatic heterocyclyl amino" includes a group wherein one hydrogen atom attached to the nitrogen atom of an amino group is replaced with above "non-aromatic heterocycle". Examples include piperidinylamino, tetrahydrofurylamino and the like.

The term of "non-aromatic heterocyclyl carbonyl" includes a group wherein above "non-aromatic heterocycle" is attached to a carbonyl group. Examples include piperidinylcarbonyl, tetrahydrofurylcarbonyl and the like are exemplified.

The term of "non-aromatic heterocyclyl oxycarbonyl" includes a group wherein "non-aromatic heterocyclyl oxy" is attached to a carbonyl group. Examples include piperidinyloxycarbonyl, tetrahydrofuryloxycarbonyl and the like.

The term of "non-aromatic heterocyclyl carbonylamino" includes a group wherein one or two hydrogen atom(s) attached to the nitrogen atom of an amino group is replaced with above "non-aromatic heterocyclecarbonyl". Examples include piperidinylcarbonylamino, tetrahydrofurylcarbonylamino and the like.

The term of "non-aromatic heterocyclyl sulfanyl" includes a group wherein a hydrogen atom attached to a sulfur atom of a sulfanyl is replaced with one above "non-aromatic heterocycle". Examples include piperidinylsulfanyl, tetrahydrofurylsulfanyl and the like.

The term of "non-aromatic heterocyclyl sulfonyl" includes a group wherein a "non-aromatic heterocycle" is attached to a sulfonyl group. Examples include piperidinylsulfonyl, tetrahydrofurylsulfonyl and the like.

The substituents of "substituted or unsubstituted alkyl", "substituted or unsubstituted alkenyl", "substituted or unsubstituted alkynyl", "substituted or unsubstituted haloalkyl", "substituted or unsubstituted alkyloxy", "substituted or unsubstituted alkenyloxy", "substituted or unsubstituted alkynyloxy", "substituted or unsubstituted haloalkyloxy", "substituted or unsubstituted alkylcarbonyl", "substituted or unsubstituted alkenylcarbonyl", "substituted or unsubstituted alkynylcarbonyl", "substituted or unsubstituted monoalkylamino", "substituted or unsubstituted dialkylamino", "substituted or unsubstituted alkylsulfonyl", "substituted or unsubstituted alkenylsulfonyl", "substituted or unsubstituted alkynylsulfonyl", "substituted or unsubstituted monoalkylcarbonylamino", "substituted or unsubstituted dialkylcarbonylamino", "substituted or unsubstituted monoalkylsulfonylamino", "substituted or unsubstituted dialkylsulfonylamino", "substituted or unsubstituted alkylimino", "substituted or unsubstituted alkenylimino", "substituted or unsubstituted alkynylimino", "substituted or unsubstituted alkylcarbonylimino", "substituted or unsubstituted alkenylcarbonylimino", "substituted or unsubstituted alkynylcarbonylimino", "substituted or unsubstituted alkyloxyimino", "substituted or unsubstituted alkenyloxyimino", "substituted or unsubstituted alkynyloxyimino", "substituted or unsubstituted alkylcarbonyloxy", "substituted or unsubstituted alkenylcarbonyloxy", "substituted or unsubstituted alkynylcarbonyloxy", "substituted or unsubstituted alkylsulfonyloxy", "substituted or unsubstituted alkenylsulfonyloxy", "substituted or unsubstituted alkynylsulfonyloxy", "substituted or unsubstituted alkyloxycarbonyl", "substituted or unsubstituted alkenyloxycarbonyl", "substituted or unsubstituted alkynyloxycarbonyl", "substituted or unsubstituted alkylsulfanyl", "substituted or unsubstituted alkenylsulfanyl", "substituted or unsubstituted alkynylsulfanyl", "substituted or unsubstituted alkylsulfinyl", "substituted or unsubstituted alkenylsulfinyl", "substituted or unsubstituted alkynylsulfinyl", "substituted or unsubstituted monoalkylcarbamoyl", "substituted or unsubstituted dialkylcarbamoyl", "substituted or unsubstituted monoalkylsulfamoyl", "substituted or unsubstituted dialkylsulfamoyl", "substituted or unsubstituted alkylene", "substituted or unsubstituted alkenylene", "substituted or unsubstituted alkynylene" and the like include the group as follows. A carbon atom(s) at any possible position(s) can be substituted with one or more of the same or different substituent(s) selected from the following group, preferably 1 to 4 substituent(s), more preferably 1 to 3 substituent(s).

Substituent: halogen, hydroxy, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azido, hydrazino, ureido, amidino, guanidino, trialkylsilyl, alkyloxy, alkenyloxy, alkynyloxy, haloalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkylsulfonyloxy, alkenylsulfonyloxy, alkynylsulfonyloxy, alkyloxycarbonyl, alkenyloxycarbonyl, alkynyloxycarbonyl, alkylsulfanyl, alkenylsulfanyl, alkynylsulfanyl, monoalkylcarbamoyl, dialkylcarbamoyl, monoalkylsulfamoyl, dialkylsulfamoyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, substituted or unsubstituted non-aromatic heterocyclyloxy, substituted or unsubstituted aromatic carbocyclylamino, substituted or unsubstituted non-aromatic carbocyclylamino, substituted or unsubstituted aromatic heterocyclylamino, substituted or unsubstituted non-aromatic heterocyclylamino, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted aromatic carbocyclyloxycarbonyl, substituted or unsubstituted non-aromatic carbocyclyloxycarbonyl, substituted or unsubstituted aromatic heterocyclyloxycarbonyl, substituted or unsubstituted non-aromatic heterocyclyloxycarbonyl, aromatic carbocyclylcarbonylamino, non-aromatic carbocyclylcarbonylamino, aromatic heterocyclylcarbonylamino, non-aromatic heterocyclylcarbonylamino, aromatic carbocyclylalkyloxy, non-aromatic carbocyclylalkyloxy, aromatic heterocyclylalkyloxy, non-aromatic heterocyclylalkyloxy, aromatic carbocyclylalkylsulfanyl, non-aromatic carbocyclylalkylsulfanyl, aromatic heterocyclylalkylsulfanyl, non-aromatic heterocyclylalkylsulfanyl, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclylalkylamino, non-aromatic carbocyclylalkylamino, aromatic heterocyclylalkylamino, non-aromatic heterocyclylalkylamino, substituted or unsubstituted aromatic carbocyclylsulfanyl, substituted or unsubstituted non-aromatic carbocyclylsulfanyl, substituted or unsubstituted aromatic heterocyclylsulfanyl, substituted or unsubstituted non-aromatic heterocyclylsulfanyl, substituted or unsubstituted aromatic carbocyclylsulfonyl, substituted or unsubstituted non-aromatic carbocyclylsulfonyl, substituted or unsubstituted aromatic heterocyclylsulfonyl, and substituted or unsubstituted non-aromatic heterocyclylsulfonyl.

The substituents on the ring of "aromatic carbocycle", "non-aromatic carbocycle", "aromatic heterocycle", "non-aromatic heterocycle", "carbocycle" or "heterocycle" of "substituted or unsubstituted aromatic carbocyclyl", "substituted or unsubstituted non-aromatic carbocyclyl", "substituted or unsubstituted aromatic heterocyclyl" "substituted or unsubstituted non-aromatic heterocyclyl", "substituted or unsubstituted aromatic carbocyclyloxy", "substituted or unsubstituted non-aromatic carbocyclyloxy", "substituted or unsubstituted aromatic heterocyclyloxy", "substituted or unsubstituted non-aromatic heterocyclyloxy", "substituted or unsubstituted aromatic carbocyclylamino", "substituted or unsubstituted non-aromatic carbocyclylamino", "substituted or unsubstituted aromatic heterocyclylamino", "substituted or unsubstituted non-aromatic heterocyclylamino", "substituted or unsubstituted aromatic carbocyclylcarbonyl", "substituted or unsubstituted non-aromatic carbocyclylcarbonyl", "substituted or unsubstituted aromatic heterocyclylcarbonyl", "substituted or unsubstituted non-aromatic heterocyclylcarbonyl",
"substituted or unsubstituted aromatic carbocyclyloxycarbonyl", "substituted or unsubstituted non-aromatic carbocyclyloxycarbonyl", "substituted or unsubstituted aromatic heterocyclyloxycarbonyl", "substituted or unsubstituted non-aromatic heterocyclyloxycarbonyl",
"substituted or unsubstituted aromatic carbocyclylsulfanyl", "substituted or unsubstituted non-aromatic carbocyclylsulfanyl", "substituted or unsubstituted aromatic heterocyclylsulfanyl", "substituted or unsubstituted non-aromatic heterocyclylsulfanyl", "substituted or unsubstituted aromatic carbocyclylsulfonyl", "substituted or unsubstituted non-aromatic carbocyclylsulfonyl", "substituted or unsubstituted aromatic heterocyclylsulfonyl" and "substituted or unsubstituted non-aromatic heterocyclylsulfonyl" include the following group. An atom(s) at any possible position(s) on the ring can be substituted with one or more of the same or different substituent(s) selected from the following group, preferably 1 to 4 substituent(s), more preferably 1 to 3 substituent(s).

Substituent: halogen, hydroxy, carboxy, amino, imino, hydroxyamino, hydroxyimino, formyl, carbamoyl, sulfamoyl, sulfanyl, sulfino, sulfo, thioformyl, thiocarboxy, dithiocarboxy, thiocarbamoyl, cyano, nitro, azido, hydrazino, ureido, amidino, guanidino, trialkylsilyl, alkyl, alkenyl, alkynyl, haloalkyl, hydroxyalkyl, alkyloxy, alkenyloxy, alkynyloxy, haloalkyloxy, alkyloxyalkyl, alkyloxyalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkylsulfonyloxy, alkenylsulfonyloxy, alkynylsulfonyloxy, alkyloxycarbonyl, alkenyloxycarbonyl, alkynyloxycarbonyl, alkylsulfanyl, alkenylsulfanyl, alkynylsulfanyl, monoalkylcarbamoyl, dialkylcarbamoyl, monoalkylsulfamoyl, dialkylsulfamoyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy, aromatic carbocyclylamino, non-aromatic carbocyclylamino, aromatic heterocyclylamino, non-aromatic heterocyclylamino, aromatic carbocyclylcarbonyl, non-aromatic carbocyclylcarbonyl, aromatic heterocyclylcarbonyl, non-aromatic heterocyclylcarbonyl, aromatic carbocyclyloxycarbonyl, non-aromatic carbocyclyloxycarbonyl, aromatic heterocyclyloxycarbonyl, non-aromatic heterocyclyloxycarbonyl, aromatic carbocyclylcarbonylamino, non-aromatic carbocyclylcarbonylamino, aromatic heterocyclylcarbonylamino, non-aromatic heterocyclylcarbonylamino, aromatic carbocyclylalkyl, non-aromatic carbocyclylalkyl, aromatic heterocyclylalkyl, non-aromatic heterocyclylalkyl, aromatic carbocyclylalkyloxy, non-aromatic carbocyclylalkyloxy, aromatic heterocyclylalkyloxy, non-aromatic heterocyclylalkyloxy, aromatic carbocyclylalkylsukfanyl, non-aromatic carbocyclylalkylsukfanyl, aromatic heterocyclylalkylsukfanyl, non-aromatic heterocyclylalkylsukfanyl, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclylalkyloxyalkyl, non-aromatic carbocyclylalkyloxyalkyl, aromatic heterocyclylalkyloxyalkyl, non-aromatic heterocyclylalkyloxyalkyl, aromatic carbocyclylalkylamino, non-aromatic carbocyclylalkylamino, aromatic heterocyclylalkylamino, non-aromatic heterocyclylalkylamino, aromatic carbocyclylsukfanyl, non-aromatic carbocyclylsukfanyl, aromatic heterocyclylsukfanyl, non-aromatic heterocyclylsukfanyl, non-aromatic carbocyclylsulfonyl, aromatic carbocyclylsulfonyl, aromatic heterocyclesulfonyl, and non-aromatic heterocyclylsulfonyl.

"Substituted or unsubstituted non-aromatic carbocyclyl" and "substituted or unsubstituted non-aromatic heterocyclyl" can be substituted with "oxo". Namely, two hydrogen atoms attached to a carbon atom are replaced with oxo as follows:

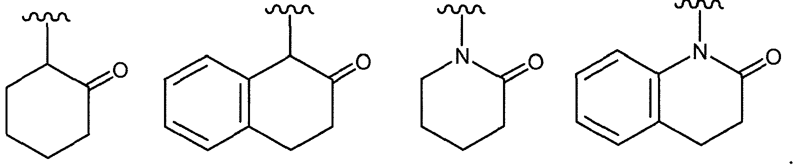

Further, "substituted or unsubstituted non-aromatic carbocyclyl" and "substituted or unsubstituted non-aromatic heterocyclyl" may be bridged with alkylene, alkenylene, or alkynylene, or form a spiro ring together with another ring such as cycloalkane, cycloalkene, cycloalkyne, oxirane, oxetane, and thiolane, as shown below

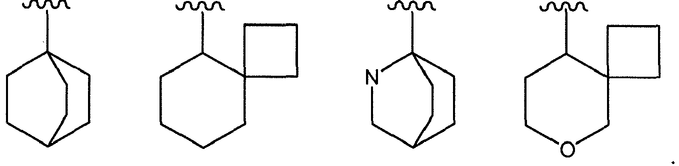

The non-aromatic carbocycle or non-aromatic heterocycle part of the above "substituted or unsubstituted non-aromatic carbocyclyloxy", "substituted or unsubstituted non-aromatic heterocyclyloxy", "substituted or unsubstituted non-aromatic carbocyclylamino", "substituted or unsubstituted non-aromatic heterocyclylamino", "substituted or unsubstituted non-aromatic carbocyclylcarbonyl", "substituted or unsubstituted non-aromatic heterocyclylcarbonyl", "substituted or unsubstituted non-aromatic carbocyclyloxycarbonyl", "substituted or unsubstituted non-aromatic heterocyclyloxycarbonyl", "substituted or unsubstituted non-aromatic carbocyclylcarbonylamino", "substituted or unsubstituted non-aromatic heterocyclylcarbonylamino", "substituted or unsubstituted non-aromatic carbocyclylsukfanyl", "substituted or unsubstituted non-aromatic heterocyclylsukfanyl", "substituted or unsubstituted non-aromatic carbocyclylsulfonyl", "substituted or unsubstituted non-aromatic heterocyclylsulfonyl", "substituted or unsubstituted carbocyclyl" and "substituted or unsubstituted heterocyclyl" may be substituted with "oxo" as mentioned above.

Preferred embodiment of the compound represented by the formula (I), which is also referred to as Compound (I), is shown below.

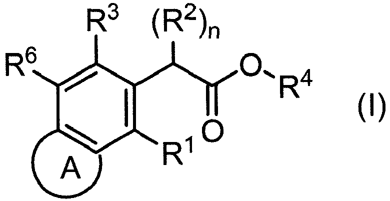

Ring A is preferably substituted or unsubstituted carbocycle, or substituted or unsubstituted heterocycle, more preferably, substituted or unsubstituted aromatic carbocycle, substituted or unsubstituted non-aromatic carbocycle, substituted or unsubstituted aromatic heterocycle, or substituted or unsubstituted non-aromatic heterocycle, wherein the ring may be fused with another substituted or unsubstituted aromatic carbocycle, substituted or unsubstituted non-aromatic carbocycle, substituted or unsubstituted aromatic heterocycle, substituted or unsubstituted non-aromatic heterocycle, or fused ring thereof, the ring may form a spiro ring together with another substituted or unsubstituted aromatic carbocycle, substituted or unsubstituted non-aromatic carbocycle, substituted or unsubstituted aromatic heterocycle, or substituted or unsubstituted non-aromatic heterocycle, and/or two atoms constituting ring A which is not adjacent to each other may be cross-linked with substituted or unsubstituted alkylene, substituted or unsubstituted alkenylene, or substituted or unsubstituted alkynylene.

R¹ may be taken together with constituent atom(s) of ring A to form substituted or unsubstituted carbocycle, or substituted or unsubstituted heterocycle.

Preferred embodiment of ring A includes substituted or unsubstituted ring shown in the followings 1) to 4):
1) 5- to 12-membered, preferably 5- to 10-membered, more preferably 7- to 10-membered monocyclic ring.
2) 5- to 10-membered ring fused with another 3- to 10-membered monocyclic ring or another 8- to 18-membered fused ring.
   Furthermore, 5- to 7-membered ring fused with another 5- to 7-membered monocyclic ring (e.g.: carbocycle, heterocycle), preferably heterocycle containing nitrogen atom(s) or sulfur atom(s).
3) 5- to 10-membered ring to form a spiro ring together with another 3- to 4-membered ring or 5- to 10-membered ring.
4) 11- to 12-membered ring which may be fused with another 3- to 10-membered monocyclic ring or 8- to 18-membered fused ring.

Ring A is more preferably aromatic carbocycle, non-aromatic carbocycle, aromatic heterocycle or non-aromatic heterocycle, wherein the ring may be fused with another aromatic carbocycle, non-aromatic carbocycle, aromatic heterocycle, non-aromatic heterocycle or fused ring thereof, the ring may form a spiro ring together with another aromatic carbocycle, non-aromatic carbocycle, aromatic heterocycle or non-aromatic heterocycle, and/or two atoms constituting Ring A which is not adjacent to each other may be cross-linked with alkylene, alkenylene or alkynylene.

Ring A is particularly preferably, optionally substituted 5- to 7-membered heterocycle. The heterocycle preferably have 1 to 2 of nitrogen atom(s), and is optionally fused with optionally substituted benzene or optionally substituted 5- to 7-membered heterocycle. Ring A is preferably optionally substituted with one or more, of R^{A}, preferably 1 to 4, of R^{A}. R^{A} is each independently, preferably, halogen, cyano, nitro, oxo or -X^{A}-R^{A1}.

X^{A} is preferably a single bond, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -COO-NR^{A2}-, -O-CO-NR^{A2}- -NR^{A2}-O-CO-, -CO-NR^{A2}-O-, -O-NR^{A2}-CO-, -NR^{A2}-CO-NR^{A3}-, -NR^{A2}-SO₂- or -SO₂ -NR^{A2}-. X^{A} is more preferably a single bond, -O-, -NR^{A2}-, -CO-, -SO₂- or -CO-O-. R^{A1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A} is a single bond, R^{A1} is not a hydrogen atom. R^{A1} is more preferably a hydrogen atom, substituted or unsubstituted alkyl, substituted or unsubstituted aromatic carbocyclyl, or substituted or unsubstituted non-aromatic carbocyclyl. R^{A2} and R^{A3} each independently, preferably, a hydrogen atom, substituted or unsubstituted alkyl, substituted or unsubstituted alkenyl, or substituted or unsubstituted alkynyl, more preferably, a hydrogen atom.

When X^{A} is -NR^{A2}-, -CO-NR^{A2}-, -CO-O-NR^{A2}-, -O-CO-NR^{A2}- or -SO₂-NR^{A2}-, R^{A1} and R^{A2} may be taken together with an adjacent nitrogen atom to form substituted or unsubstituted aromatic heterocyclyl, or substituted or unsubstituted non-aromatic heterocyclyl. When X^{A} is -NR^{A2}-CO-NR^{A3}-, R^{A1} and R^{A3} may be taken together with an adjacent nitrogen atom to form substituted or unsubstituted aromatic heterocyclyl, or substituted or unsubstituted non-aromatic heterocyclyl.

R^{A} is each independently, more preferably, halogen, hydroxy, oxo, amino, mono- or di-alkylamino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl. As these substituents, halogen, hydroxy, amino and the like are exemplified.

R¹ and R^{A} may be taken together with an adjacent atom to form aromatic carbocycle, non-aromatic carbocycle, aromatic heterocycle or non-aromatic heterocycle, wherein the ring is preferably 5- to 8-membered ring, more preferably 6- to 7-membered ring, and/or heterocycle, and may be substituted with same or different one or more R^{A'}. R^{A'} is each independently, preferably, halogen, cyano, nitro, oxo, azide, trimethylsilyl or -X^{A'}-R^{A'1}.

X^{A'} is preferably a single bond, -O-, -S-, -NR^{A'2}-, =N-, -CO-, -SO₂-, -O-CO-,-CO-O-, -NR^{A'2}-CO-, -CO-NR^{A'2}-, -NR^{A'2}-CO-O-, -CO-O-NR^{A2}-, -O-CO-NR^{A'2}-,-NR^{A'2}-O-CO-, -CO-NR^{A'2}-O-, -O-NR^{A'2}-CO-, -NR^{A'2}-CO-NR^{A'3}-, -NR^{A'2}-SO₂- or-SO₂-NR^{A'2}-.

R^{A'1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A'} is a single bond, R^{A'1} is not a hydrogen atom.

R^{A'2} and R^{A'3} are each independently, preferably, a hydrogen atom, substituted or unsubstituted alkyl, substituted or unsubstituted alkenyl, or substituted or unsubstituted alkynyl.

When X^{A'} is -NR^{A'2}-, -CO-NR^{A'2}-, -CO-O-NR^{A'2}-, -O-CO-NR^{A'2}- or -SO₂-NR^{A'2}-, R^{A'1} and R^{A'2} may be taken together with an adjacent nitrogen atom to form substituted or unsubstituted aromatic heterocyclyl, or substituted or unsubstituted non-aromatic heterocyclyl. When X^{A'} is -NR^{A'2}-CO-NR^{A'3}-, R^{A'1} and R^{A'3} may be taken together with an adjacent nitrogen atom to form substituted or unsubstituted aromatic heterocyclyl, or substituted or unsubstituted non-aromatic heterocyclyl.

Ring A is preferably any one of the following rings:

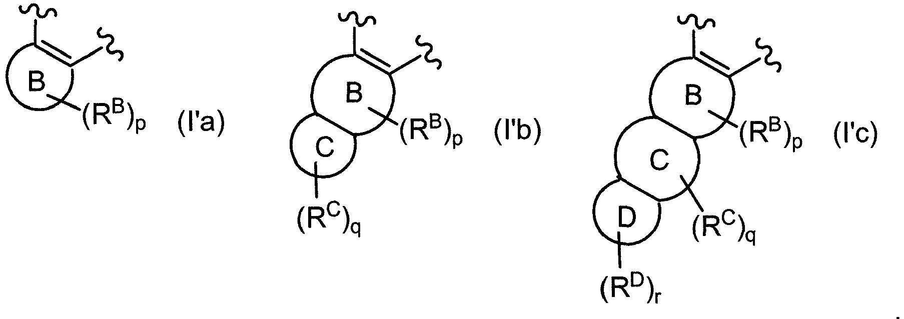

Ring B, ring C and ring D are each independently, monocyclic aromatic carbocycle, monocyclic non-aromatic carbocycle, monocyclic aromatic heterocycle or monocyclic non-aromatic heterocycle, preferably 6- to 12-membered ring, more preferably 6- to 8-membered ring, wherein the rings may form a spiro ring together with other aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene.

Ring B is preferably 5- to 10-membered ring, more preferably heterocycle, particularly preferably heterocycle containing nitrogen atom(s) which may form a spiro ring together with another 3- to 4-membered ring or 5- to 10-membered ring, and/or two atoms constituting Ring A which is not adjacent to each other may be cross-linked with substituted or unsubstituted alkylene, substituted or unsubstituted alkenylene, or substituted or unsubstituted alkynylene. Also ring B is preferably 11-to 12-membered ring.

When Ring A is the ring represented by formula (I'a), ring B is more preferably 7- to 10-membered ring.

When Ring A is the ring represented by formula (I'b), ring B is preferably 6- to 8-membered heterocycle.

Ring B preferably includes 1 to 3 double bond(s). Ring B is more preferably the following rings. When the following ring is a spito ring, the structure of the following ring corresponds to ring B-ring E.

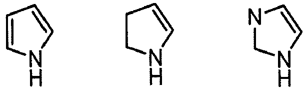

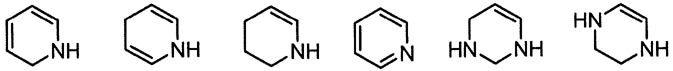

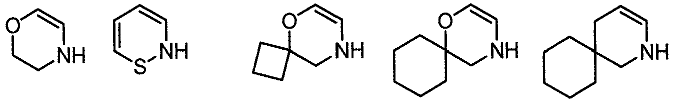

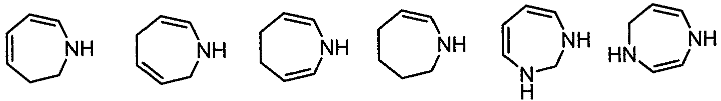

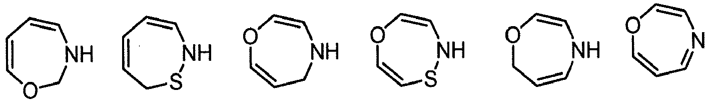

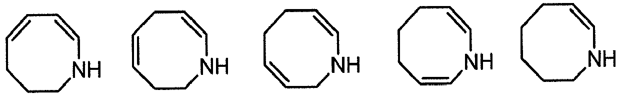

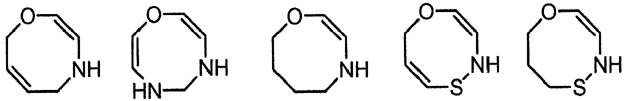

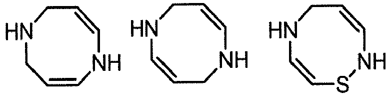

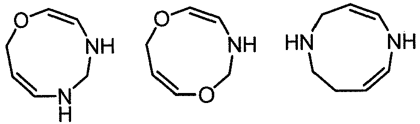

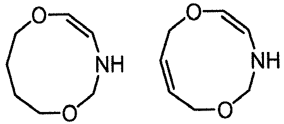

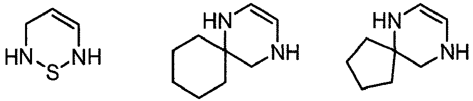

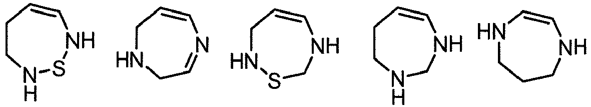

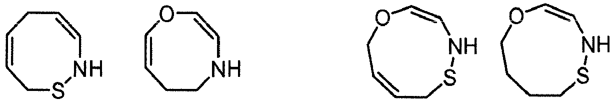

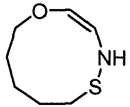

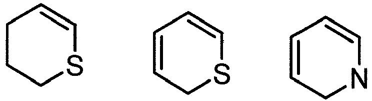

In the above formula, any one of bouble bonds preferably bounds to benze ne ring shown in the compound represented by formula (I).

Ring C is preferably 3- to 10-membered ring, more preferably 5- to 8-membered ring, further preferably 5- to 6-membered ring.

Ring C is preferably thiophene, thiolane, furan, tetrahydrofuran, pyrrolidine, pyrroline, pyrrole, imidazole, imidazolidine, imidazoline, pyrazole, pyrazolidine, pyrazoline, triazole, tetrazole, oxazole, oxazolidine, oxazoline, oxadiazole, isoxazole, thiazole, thiazolidine, thiadiazole, isothiazole, benzene, pyridine, pyrimidine, pyridazine, pyrazine, triazin or cycloalkene, more preferably thiophene, furan, pyrrolidine, triazole, tetrazole, isoxazole, benzene, pyridine or cyclohexene.

Ring C is also preferably thiadiazolidine or tetrahydropyridine.

Ring D is preferably 3- to 10-membered ring, more preferably 5- to 8-membered ring, further preferably 5- to 6-membered ring.

Ring D is preferably thiophene, thiolane, furan, tetrahydrofuran, pyrrolidine, pyrroline, pyrrole, imidazole, imidazolidine, imidazoline, pyrazole, pyrazolidine, pyrazoline, triazole, tetrazole, oxazole, oxazolidine, oxazoline, oxadiazole, isoxazole, thiazole, thiazolidine, thiadiazole, isothiazole, benzene, pyridine, pyrimidine, pyridazine, pyrazine, triazine, cycloalkane or cycloalkene, more preferably thiophene, isoxazole or benzene.

R^{B} is each independently, preferably, a hydrogen atom, halogen, cyano, nitro, oxo, azide, trimethylsilyl or -X^{B}-R^{B1}.

X^{B} is preferably a single bond, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- or -SO₂-NR^{B2}-.

R^{B1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom).

R^{B2} and R^{B3} are each independently, preferably, a hydrogen atom, substituted or unsubstituted alkyl, substituted or unsubstituted alkenyl, or substituted or unsubstituted alkynyl.

When X^{B} is -NR^{B2}-, -CO-NR^{B2}-, -CO-O-NR^{B2}-, -O-CO-NR^{B2}- or -SO₂-NR^{B2}-, R^{B1} and R^{B2} may be taken together with an adjacent nitrogen atom to form substituted or unsubstituted aromatic heterocyclyl, or substituted or unsubstituted non-aromatic heterocyclyl. When X^{B} is -NR^{B2}-CO-NR^{B3}-, R^{B1} and R^{B3} may be taken together with an adjacent nitrogen atom to form substituted or unsubstituted aromatic heterocyclyl, or substituted or unsubstituted non-aromatic heterocyclyl.

R^{B} is each independently, more preferably, a hydrogen atom, halogen, hydroxy, oxo, amino, alkylamino, imino, substituted or unsubstituted alkyl, substituted or unsubstituted alkenyl, substituted or unsubstituted alkynyl, substituted or unsubstituted cycloalkyl, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted alkylsulfonyl, substituted or unsubstituted alkenylsulfonyl, substituted or unsubstituted alkynylsulfonyl, substituted or unsubstituted aryl, substituted or unsubstituted aralkyl, substituted or unsubstituted heterocyclyl, substituted or unsubstituted heterocyclylalkyl, or substituted sulfonyl. R^{B} is more preferably a hydrogen atom, halogen, oxo, amino, imino, substituted or unsubstituted alkyl, substituted or unsubstituted alkyloxy, substituted or unsubstituted alkyloxycarbonyl, substituted or unsubstituted alkylsulfonyl, substituted or unsubstituted cycloalkyl, substituted or unsubstituted phenyl, substituted or unsubstituted benzyl, substituted or unsubstituted heterocyclyl, or substituted or unsubstituted heterocyclylalkyl, particularly preferably a hydrogen atom, oxo, imino, halogen, substituted or unsubstituted alkyl, substituted or unsubstituted alkoxy, substituted or unsubstituted alkyloxycarbonyl, substituted or unsubstituted alkylsulfonyl, substituted or unsubstituted phenyl, substituted or unsubstituted benzyl. R^{B} is most preferably a hydrogen atom, oxo, imino, halogen, substituted or unsubstituted methyl, substituted or unsubstituted methoxy, substituted or unsubstituted benzyl, substituted or unsubstituted phenethyl, substituted or unsubstituted benzyloxycarbonyl, or substituted or unsubstituted methylsulfonyl. The substituents of "substituted or unsubstituted" is preferably alkyl, cycloalkyl, cycloalkylalkyl, alkoxy, alkoxyalkyl, halogen, haloalkyl, amino, alkylamino, hydroxy, oxo, carboxy, phenyl or halophenyl.

R^{B} is also preferably hydroxyl or non-aromatic carbocyclyl, wherein non-aromatic carbocyclyl is preferably cycloalkyl, more preferably cyclohexyl.

When ring B is heterocycle containing nitrogen atom(s), one of preferable embodiment of R^{B} which bound to any carbon atom or sulfur atom adjacent to the nitrogen atom constituting ring B, is oxo as shown below.

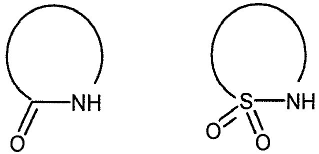

When ring B is heterocycle, one further preferred embodiment is the followings: 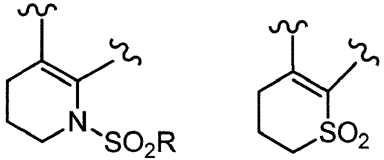 wherein R is alkyl and the like.

As an embodiment of p, the followings 1) and 2) are exemplified.
1) p is one or more.
2) p is 0.

p is preferably any integer of 0 to 12, more preferably any integer of 0 to 6, further preferably any integer of 0 to 3.

R¹ and R^{B} may be taken together with an adjacent atom to form aromatic carbocycle, non-aromatic carbocycle, aromatic heterocycle or non-aromatic heterocycle wherein the ring may be substituted with one or more of the same or different R^{B'}. The ring is more preferably aromatic heterocycle or non-aromatic heterocycle, further preferably non-aromatic heterocycle, particularly preferably dihydropyridine or tetrahydropyridine, most preferably 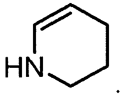 The ring is preferably unsubstituted ring.

R^{B'} includes each independently the same group as R^{B}, preferably halogen, cyano, nitro, oxo, azide, trimethylsilyl or -X^{B'}-R^{B'1}.

X^{B'} is preferably a single bond, -O-, -S-, -NR^{B'2}-, =N-, -CO-, -SO₂ -, -O-CO-,-CO-O-, -NR^{B'2}-CO-, -CO-NR^{B'2}-, -NR^{B'2}-CO-O-, -CO-O-NR^{B'2}-, -O-CO-NR^{B'2}-,-NR^{B'2}-O-CO-, -CO-NR^{B'2}-O-, -O-NR^{B'2}-CO-, -NR^{B'2}-CO-NR^{B'3}-, -NR^{B'2}-SO₂- or-SO₂-NR^{B'2}-.

R^{B'1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom

R^{B'2} and R^{B'3} are each independently, preferably, a hydrogen atom, substituted or unsubstituted alkyl, substituted or unsubstituted alkenyl, or substituted or unsubstituted alkynyl.

When X^{B'} is -NR^{B'2}-, -CO-NR^{B'2}-, -CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl. When X^{B'} is -NR^{B2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl.

s is preferably any integer of 0 to 11, more preferably any integer of 0 to 6, further preferably 0 or 1, particularly preferably 0.

R^{C} includes each independently the same group as R^{B}, preferably halogen, cyano, nitro, oxo, azide, trimethylsilyl or -X^{C}-R^{C1}.

X^{C} is preferably a single bond, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-O-CO-, -CO-NR^{C2}-O-, -O-NR^{C2}-CO-, -NR^{C2}-CO-NR^{C3}-, -NRC²-SO₂- or -SO₂-NR^{C2}-.

R^{C1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{C} is a single bond, R^{C1} is not a hydrogen atom.

R^{C2} and R^{C3} are each independently, preferably, a hydrogen atom, substituted or unsubstituted alkyl, substituted or unsubstituted alkenyl, or substituted or unsubstituted alkynyl.

When X^{C} is -NR^{C2}-, -CO-NR^{C2}-, -CO-O-NR^{C2}-, -O-CO-NR^{C2}- or -SO₂ -NR^{C2}-, R^{C}1 and R^{C2} may be taken together with an adjacent nitrogen atom to form substituted or unsubstituted aromatic heterocyclyl, or substituted or unsubstituted non-aromatic heterocyclyl. When X^{C} is -NR^{C2}-CO-NR^{C3}-, R^{C1} and R^{C3} may be taken together with an adjacent nitrogen atom to form substituted or unsubstituted aromatic heterocyclyl, or substituted or unsubstituted non-aromatic heterocyclyl.

R^{C} is each independently, more preferably, a hydrogen atom, halogen, hydroxy, oxo, amino, alkylamino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl, further preferably, a hydrogen atom, halogen, oxo, amino, imino, substituted or unsubstituted alkyl, substituted or unsubstituted alkyloxy, substituted or unsubstituted alkyloxycarbonyl, or substituted or unsubstituted alkylsulfonyl, particularly preferably, halogen, oxo, amino, or substituted or unsubstituted alkyl, most preferably fluoro, oxo, amino, methyl or trifluoromethyl. The substituents of "substituted or unsubstituted" is preferably alkyl, cycloalkyl, cycloalkylalkyl, alkoxy, alkoxyalkyl, halogen, haloalkyl, amino, alkylamino, hydroxy, oxo, carboxy, phenyl or halophenyl.

R^{C} is also preferably nitro, aromatic carbocyclyl, non-aromatic carbocyclyl, aromatic heterocyclyl or non-aromatic heterocyclyl, further preferably nitro, non-aromatic carbocyclyl or aromatic heterocyclyl, wherein non-aromatic carbocyclyl is preferably cycloalkyl, more preferably cyclohexyl, and aromatic heterocyclyl is preferably pyridyl.

As an embodiment of q, the followings 1) and 2) are exemplified.
1) q is one more.
2) q is 0.

q is preferably any integer of 0 to 12, more preferably any integer of 0 to 6, further preferably 0 or 1.

R^{B} and R^{C} may be taken together with an adjacent atom to form aromatic carbocycle, non-aromatic carbocycle, aromatic heterocycle or non-aromatic heterocycle, wherein the ring is preferably 5- to 10-membered ring, more preferably 5- to 8-membered ring, and may be substituted with one or more of the same or different R^{B}.

R^{D} includes each independently the same group as R^{B}, preferably halogen, cyano, nitro, oxo, azide, trimethylsilyl or -X^{D}-R^{D1}.

X^{D} is preferably a single bond, -O-, -S-, -NR^{D2}-, =N-, -CO-, -SO₂-, -O-CO-, -CO-O-, -NR^{D2}-CO-, -CO-NR^{D2}-, -NR^{D2}-CO-O-, -COO-NR^{D2}-, -OCO-NR^{D2}-, -NR^{D2}-O-CO-, -CO-NR^{D2}-O-, -O-NR^{D2}-CO-, -NR^{D2}-CO-NR^{D3}-, -NR^{D2}-SO₂ - or -SO₂-NR^{D2}-.

R^{D1} is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{D} is a single bond, R^{D1} is not a hydrogen atom.

R^{D2} and R^{D3} are each independently, preferably, a hydrogen atom, substituted or unsubstituted alkyl, substituted or unsubstituted alkenyl, or substituted or unsubstituted alkynyl.

When X^{D} is -NR^{D2}-, -CO-NR^{D2}-, -CO-O-NR^{D2}-, -O-CO-NR^{D2}- or -SO₂-NR^{D2}-, R^{D1} and R^{D2} may be taken together with an adjacent nitrogen atom to form substituted or unsubstituted aromatic heterocyclyl, or substituted or unsubstituted non-aromatic heterocyclyl. When X^{D} is -NR^{D2}-CO-NR^{D3}-, R^{D1} and R^{D3} may be taken together with an adjacent nitrogen atom to form substituted or unsubstituted aromatic heterocyclyl, or substituted or unsubstituted non-aromatic heterocyclyl.

R^{D} is each independently, preferably, halogen, hydroxy, oxo, amino, imino, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkyloxycarbonyl, substituted or unsubstituted alkenyloxycarbonyl, substituted or unsubstituted alkynyloxycarbonyl, substituted or unsubstituted alkylcarbonyl, substituted or unsubstituted alkenylcarbonyl, substituted or unsubstituted alkynylcarbonyl, substituted or unsubstituted alkylsulfonyl, substituted or unsubstituted alkenylsulfonyl, or substituted or unsubstituted alkynylsulfonyl, further preferably halogen, oxo, amino, imino, substituted or unsubstituted alkyl, substituted or unsubstituted alkyloxy, substituted or unsubstituted alkyloxycarbonyl, or substituted or unsubstituted alkylsulfonyl, particularly preferably, substituted or unsubstituted alkyl, or substituted or unsubstituted alkyloxy, most preferably methyl or methyloxy. The substituents of "substituted or unsubstituted" is preferably alkyl, cycloalkyl, cycloalkylalkyl, alkoxy, alkoxyalkyl, halogen, haloalkyl, amino, alkylamino, hydroxy, oxo, carboxy or phenyl.

As an embodiment of r, the followings 1) and 2) are exemplified.
1) r is one or more.
2) r is 0.

r is preferably any integer of 0 to 12, more preferably any integer of 0 to 6, further preferably 0 or 1.

R^{C} and R^{D} may be taken together with an adjacent atom to form aromatic carbocycle, non-aromatic carbocycle, aromatic heterocycle or non-aromatic heterocycle, wherein the ring may be substituted with one or more of the same or different R^{C}.

Ring B of formula (I'a) is further preferably the following rings. When ring B is a spito ring, the structure is ring B-ring E.

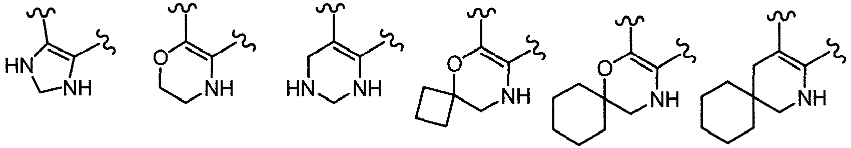

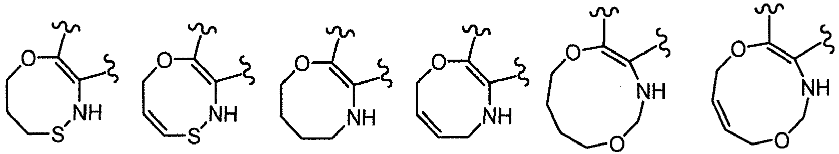

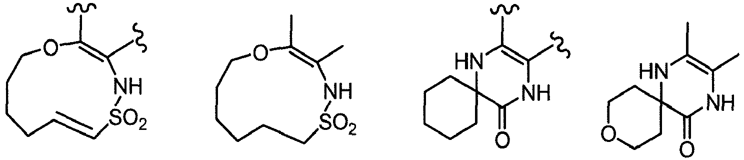

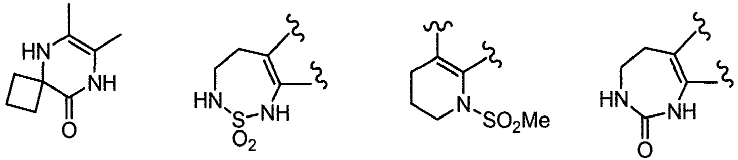

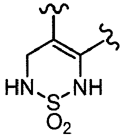

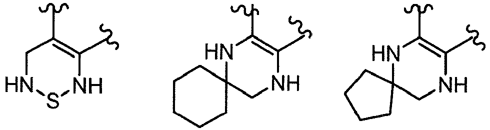

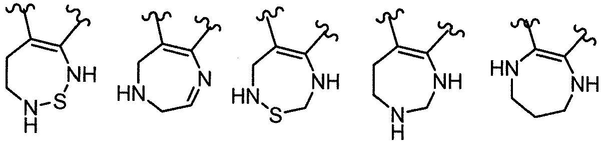

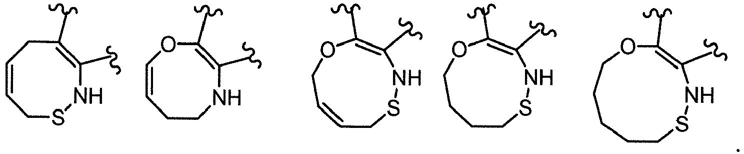

Ring B-ring C of formula (I'b) is preferably the following rings:

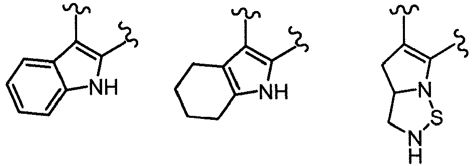

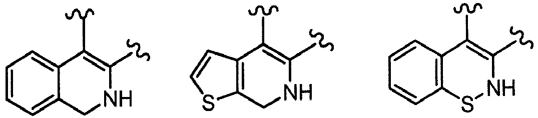

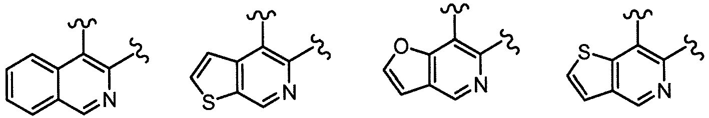

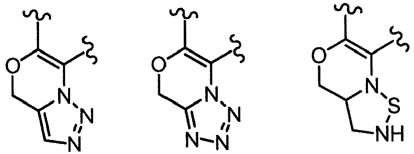

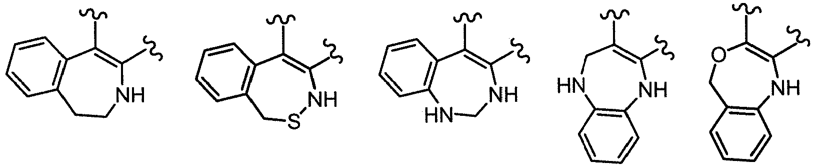

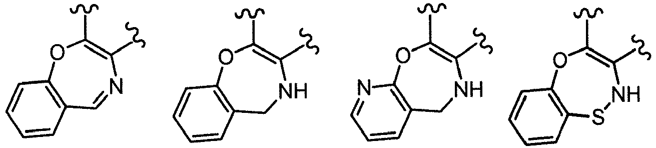

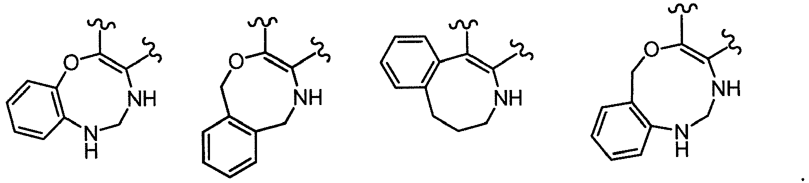

Ring B-ring C is more preferably the following rings: 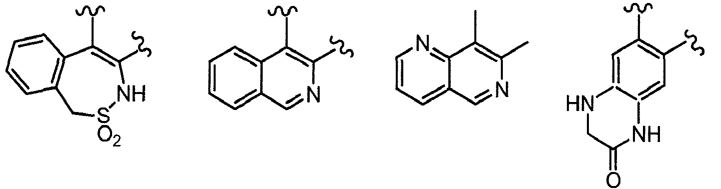 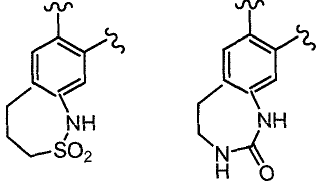 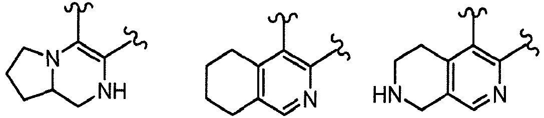 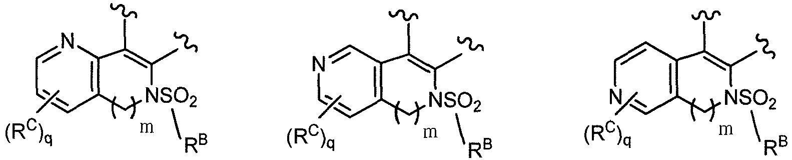 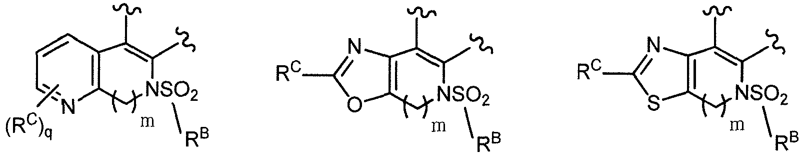 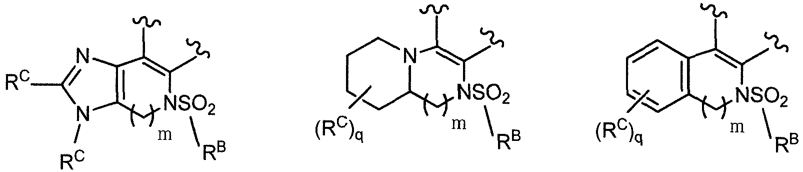 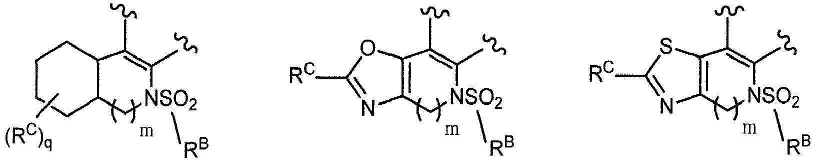 wherein each definition has the same meaning as described above.

"(-CH₂-)m" moiety in the above formula may be substituted with the substituent(s) of R^{B} and the like, preferably may be substituted with alkyl.

Ring B-ring C-ring D in the formula (I'c) is preferably the above preferred ring B-ring C substituted with ring D, more preferably the following rings:

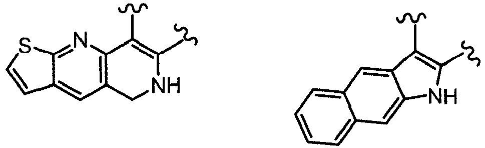

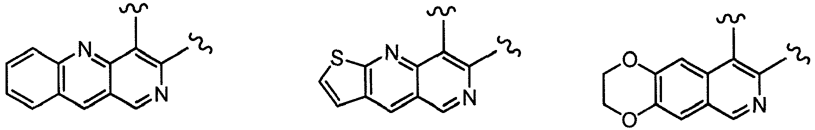

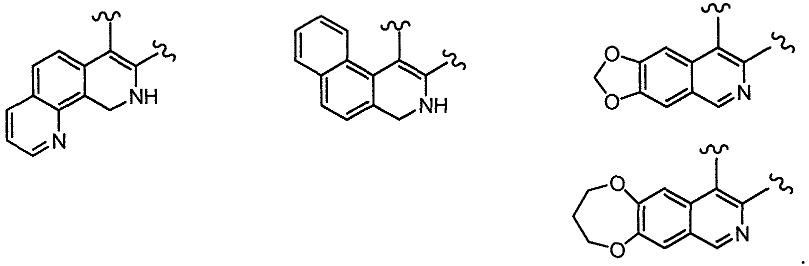

Ring E is defined as well as ring C, preferably carbocycle, more preferably 3- to 10-membered carbocycle, further preferably 3- to 8-membered carbocycle, particularly non-aromatic carbocycle, preferably saturated carbocycle.

R¹ is preferably relatively small group, more preferably halogen, cyano, nitro or -X¹-R¹¹.

X¹ is preferably a single bond, -O-, -S-, -NR¹²-, -CO-, -SO₂-, -O-CO-, -CO-O-,-NR¹²-CO-, -CO-NR¹²-, -NR¹²-CO-O-, -NR¹²-CO-NR¹³-, -NR¹²-SO₂ - or -SO₂-NR¹²-.

R¹¹ is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R¹² and R¹³ are each independently, preferably a hydrogen atom, substituted or unsubstituted alkyl, substituted or unsubstituted alkenyl, or substituted or unsubstituted alkynyl.

When X¹ is -NR¹²-, -CO-NR¹²- or -SO₂-NR¹²-, R¹¹ and R¹² may be taken together with an adjacent nitrogen atom to form substituted or unsubstituted aromatic heterocyclyl, or substituted or unsubstituted non-aromatic heterocyclyl. When X¹ is -NR¹²-CO-NR¹³-, R¹¹ and R¹³ may be taken together with an adjacent nitrogen atom to form substituted or unsubstituted aromatic heterocyclyl, or substituted or unsubstituted non-aromatic heterocyclyl.

R¹ is more preferably a hydrogen atom, halogen, substituted or unsubstituted alkyl, substituted or unsubstituted alkenyl, or substituted or unsubstituted alkynyl, more preferably substituted or unsubstituted alkyl, particularly preferably alkyl of 1 to 4 carbon atom(s), most preferably methyl.

R² is each independently, preferably, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted cycloalkyloxy, substituted or unsubstituted alkylsulfanyl, substituted or unsubstituted alkenylsulfanyl, or substituted or unsubstituted alkynylsulfanyl, more preferably substituted or unsubstituted alkyloxy, substituted or unsubstituted alkenyloxy, or substituted or unsubstituted alkynyloxy, further preferably substituted or unsubstituted alkyloxy, particularly preferably 1 to 6 carbon atom(s), further preferably alkyloxy of 1 to 4 carbon atom(s), most preferably tert-butyloxy. As the substituent(s) of R², methyl, ethyl, halogen, hydroxy, amino and the like are exemplified. The substituent(s) of cycloalkyloxy is(are) preferably methyl.

n is preferably 1 or 2, particularly preferably 1.

When n is 1, R² is preferably the following steric structure:

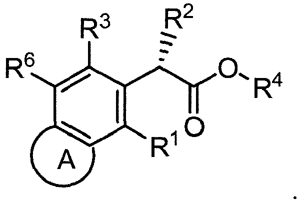

R³ is preferably cyclic group, specifically substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, or the group linked to these cyclic group via a linker (methylene, ethylene, heteroatom(s) (e.g.: O, S)), more preferably substituted or unsubstituted phenyl, substituted or unsubstituted cycloalkenyl, substituted or unsubstituted benzofuryl, substituted or unsubstituted benzodioxolyl, substituted or unsubstituted benzodioxanyl, substituted or unsubstituted dihydrobenzofuryl, substituted or unsubstituted chromanyl, substituted or unsubstituted benzomorpholinyl, or substituted or unsubstituted 5- to 7-membered saturated heterocyclyl containing nitrogen atom(s). R³ is preferably optionally substituted 5- to 7-membered ring, more preferably substituted or unsubstituted phenyl, or substituted or unsubstituted chromanyl. Also, each ring includes the ring fused with 1 or 2 of optionally substituted carbocycle (preferably 5- to 7-membered ring, e.g.: benzene) and/or optionally substituted heterocycle (preferably 5- to 7-membered ring, e.g.: pyran). As 5- to 7-membered saturated heterocyclyl containing nitrogen atom(s), pyrrolidinyl, piperidinyl, piperazinyl, morpholino and thiomorpholino are exemplified, and preferably the ring binds to benzene of core skeleton at N-position. Also, the above saturated heterocyclyl containing nitrogen atom(s) may be substituted with 1 to 3 of the same or different R³¹ and form 5- to 7-membered fused ring or 3- to 7-membered spiro ring.

When R³ is substituted or unsubstituted aromatic heterocyclyl fused with substituted or unsubstituted benzene, or substituted or unsubstituted non-aromatic heterocyclyl fused with substituted or unsubstituted benzene, the aromatic heterocyclyl or non-aromatic heterocyclyl preferably contain at least one oxygen atom.

When R³ has substituent(s), the substituent is preferably halogen, hydroxy, amino, cyano, oxo, alkyl, alkenyl, alkynyl, hydroxyalkyl, haloalkyl, alkyloxy, alkenyloxy, alkynyloxy, alkyloxyalkyl, haloalkyloxy, monoalkylamino, dialkylamino, alkylsulfanyl, alkenylsulfanyl, or alkynylsulfanyl, more preferably alkyl, alkoxy, halogen or hydroxy, further preferably alkyl (e.g.: methyl, ethyl, propyl or isopropyl, most preferably methyl.

Preferred R³ is specifically the following structure. 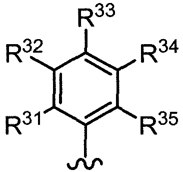 wherein R³¹, R³², R³³, R³⁴ and R³⁵ are each independently, preferably a hydrogen atom, halogen, hydroxy, amino, alkyl, cycloalkyl, alkenyl, alkynyl, haloalkyl, alkyloxy, alkenyloxy, alkynyloxyhaloalkyl, haloalkyloxy, carboxy, carbamoyl or alkylamino, more preferably a hydrogen atom, halogen, hydroxy, amino, alkyl or alkyloxy, further preferably a hydrogen atom, fluoro, chloro, bromo, hydroxy, amino, methyl, ethyl or methyloxy, particularly preferably, a hydrogen atom, halogen, hydroxy, methyl or ethyl.

As a form which R³ form a fused ring, R³¹ and R³², R³² and R³³, R³³ and R³⁴, and R³⁴ and R³⁵ may be each independently taken together with an adjacent atom to form substituted or unsubstituted aromatic carbocycle, substituted or unsubstituted non-aromatic carbocycle, substituted or unsubstituted aromatic heterocycle, or substituted or unsubstituted non-aromatic heterocycle.

The ring is preferably 5- to 8-memberedring, more preferably 5- or 6-membered ring, further preferably 6-membered ring. The ring may be cross-linked ring. The substituent(s) of "substituted or unsubstituted" is preferably alkyl, cycloalkyl, cycloalkylalkyl, alkoxy, alkoxyalkyl, halogen, halo alkyl, amino, alkylamino, hydroxy, oxo, carboxy, carbamoyl or phenyl, more preferably, halogen, alkyl, alkoxy, amino, hydroxyl and/or oxo, further preferably methyl, ethyl, F, Br, amino, hydroxyl and the like.

R³ is particularly preferably phenyl optionally fused with 1 to 2 of carbocycle or heterocycle (e.g.: 5- to 7-membered ring), more preferably the following group. The carbocycle, heterocycle and/or phenyl may have 1 to 3 of the same or different substituent(s) (e.g.: halogen, hydroxy, amino, alkylamino, alkyl, haloalkyl, oxo or alkyloxy).

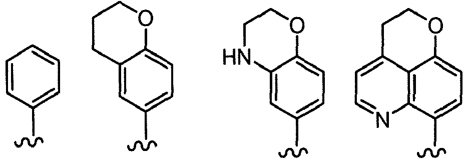

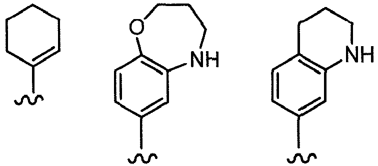

Preferred examples of R³ are below.

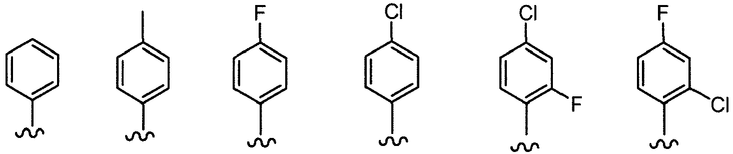

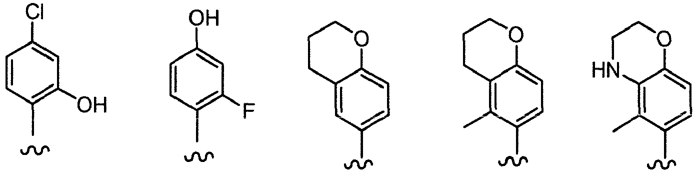

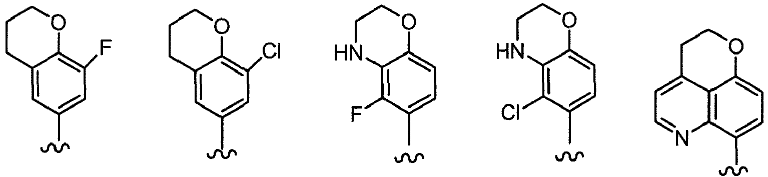

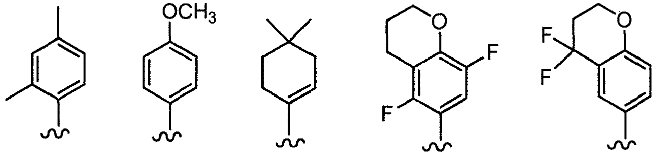

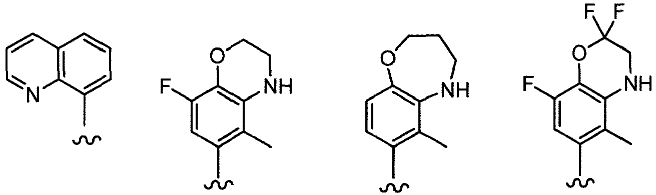

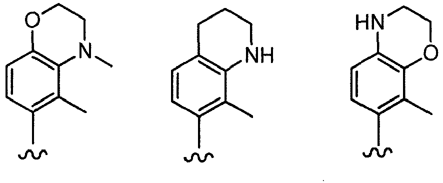

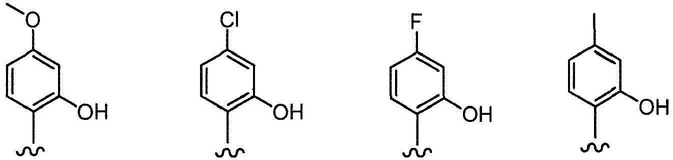

One embodiment of the isomers of compound (I) includes stereoisomers identified by the direction of R³ ring, but the present invention includes all of those isomers and racemics.

R⁴ is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, most preferably a hydrogen atom. Even if R⁴ is not a hydrogen atom, as far as R⁴ can be converted into a hydrogen atom, such compound is also useful as a synthetic intermediate.

R⁶ is preferably relatively small group, more preferably halogen, cyano, nitro or -X⁶-R⁶¹.

X⁶ is preferably a single bond, -O-, -S-, -NR⁶²-, -CO-, -SO₂-, -O-CO-, -CO-O-,-NR⁶²-CO-, -CO-NR⁶²-, -NR⁶²-CO-O-, -NR⁶²-CO-NR⁶³-, -NR⁶²-SO₂- or -SO₂-NR⁶²-, more preferably a single bond.

R⁶¹ is preferably a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl.

R⁶² and R⁶³ are each independently, preferably a hydrogen atom, substituted or unsubstituted alkyl, substituted or unsubstituted alkenyl, or substituted or unsubstituted alkynyl.

When X⁶ is -NR⁶²-, -CO-NR⁶²- or -SO₂-NR⁶²-, R⁶¹ and R⁶² may be taken together with an adjacent nitrogen atom to form substituted or unsubstituted aromatic heterocyclyl, or substituted or unsubstituted non-aromatic heterocyclyl. When X⁶ is -NR⁶²-CO-NR⁶³-, R⁶¹ and R⁶³ may be taken together with an adjacent nitrogen atom to form substituted or unsubstituted aromatic heterocyclyl, or substituted or unsubstituted non-aromatic heterocyclyl.

R⁶ is more preferably a hydrogen atom, halogen, substituted or unsubstituted alkyl, substituted or unsubstituted alkenyl, or substituted or unsubstituted alkynyl, more preferably substituted or unsubstituted alkyl, particularly preferably, alkyl of 1 to 4 carbon atom(s), most preferably methyl.

The compound represented by the formula (I) includes the following compounds represented by the formulas (I"a), (I"b) and (I"c):

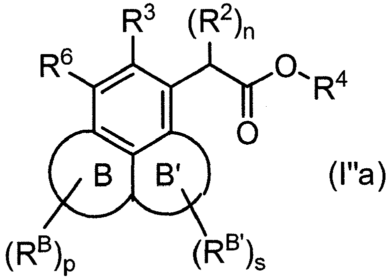

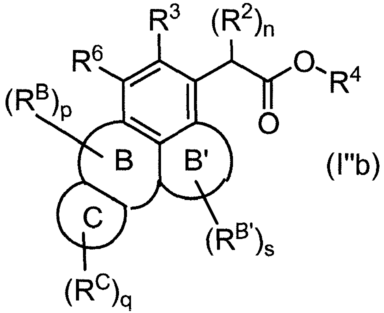

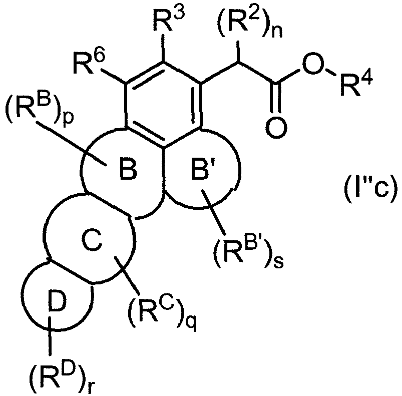

Ring B' includes the same as defined as ring B, more preferably benzene or 5-to 7-membered heterocycle. The heterocycle preferably contains total 1 to 4 of N, S, and/or O atom(s), more preferably 1 to 2 atome(s). The substituent(s) of ring B' includes the same as defined as that of ring B.

Ring B-ring B' in the formula (I"a) is preferably the following ring:

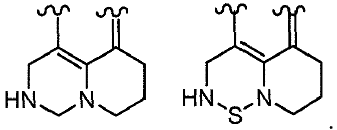

Ring C-ring B-ringB' in the formula (I"b) is preferably the following ring:

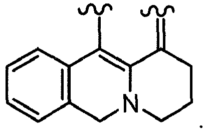

Ring A is preferably substituted o.r unsubstituted following ring: 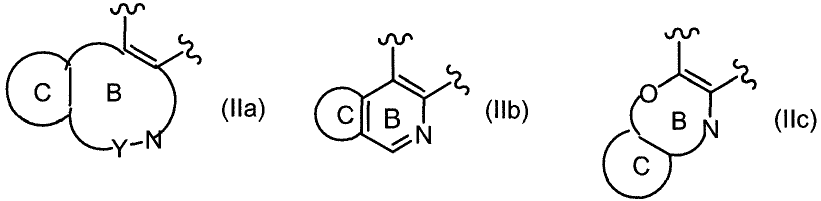 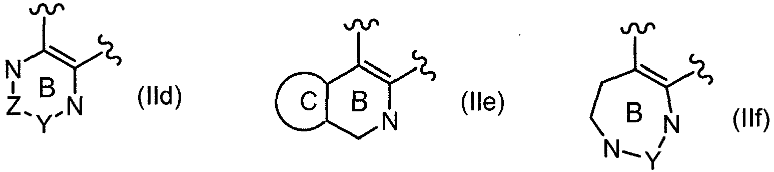 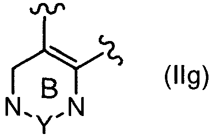 wherein
ring B and ring C are each independently 5- to 10-membered ring; Y and Z are each independently carbon atom, oxygen atom or sulfur atom wherein the carbon atom and sulfur atom may be substituted with alkyl, oxo and the like.

Ring C in the formulas (IIa) and (IIc) is preferably aromatic carbocycle or 5- to 7-membered heterocycle.

Ring B in the formula (IId) preferably form a spiro ring together with another 3- to 10-membered ring, preferably 3- to 7 membered carbocycle at the position of Z.

R^{B} which is attached to the nitrogen atom constitute of the ring B in the formula (IIe), is preferably -X^{B} -R^{B1}, wherein X^{B} is preferably -CO- or -SO₂-. R^{B1} is preferably alkyl, cycloalkyl, substituted or unsubstituted aralkyl (examples of substituent(s): alkyl, halogen, hydroxy).

Preferred embodiments of the compound represented by the formula (I) include 1) to 48) as follows. 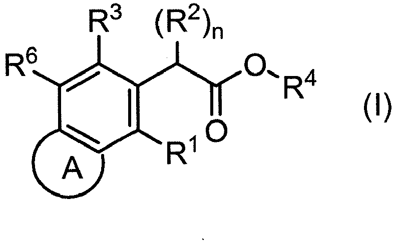 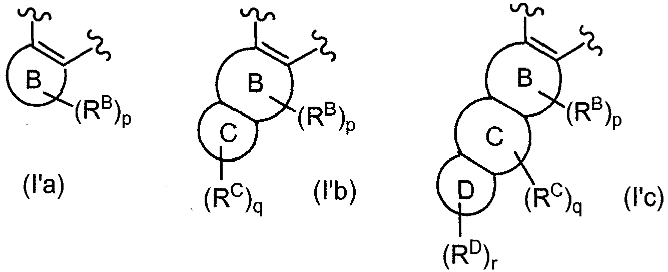
1) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'a), p is one or more.
2) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'a), p is one or more.
3) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'a), p is one or more.
4) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'a), p is one or more.
5) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'b), p and/or q are/is one or more.
6) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'b), p and/or q are/is one or more.
7) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'b), p and/or q are/is one or more.
8) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'b), p and/or q are/is one or more.
9) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'c), p, q and/or r are/is one or more.
10) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'c), p, q and/or r are/is one ormore.
11) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'c), p, q and/or r are/is one or more.
12) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'c), p, q and/or r are/is one or more.
13) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'a), p is 0.
14) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'a), p is 0.
15) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'a), p is 0.
16) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'a), p is 0.
17) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'b), p and q are 0.
18) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'b), p and q are 0.
19) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'b), p and q are 0.
20) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'b), p and q are 0.
21) Compound wherein R¹ is substituted or unsubstituted alkyl, R² is substituted or unsubstituted alkyloxy, n is 1, R³ is substituted or unsubstituted aromatic carbocyclyl, R⁴ is a hydrogen atom, R⁶ is substituted or unsubstituted alkyl, ring A is the ring represented by the formula (I'c), p, q and r are 0.
22) Compound wherein R¹ is substituted or unsubstituted alkyl, R² is substituted or unsubstituted alkyloxy, n is 1, R³ is substituted or unsubstituted non-aromatic carbocyclyl, R⁴ is a hydrogen atom, R⁶ is substituted or unsubstituted alkyl, ring A is the ring represented by the formula (I'c), p, q and r are 0.
23) Compound wherein R¹ is substituted or unsubstituted alkyl, R² is substituted or unsubstituted alkyloxy, n is 1, R³ is substituted or unsubstituted aromatic heterocyclyl, R⁴ is a hydrogen atom, R⁶ is substituted or unsubstituted alkyl, ring A is the ring represented by the formula (I'c), p, q and r are 0.
24) Compound wherein R¹ is substituted or unsubstituted alkyl, R² is substituted or unsubstituted alkyloxy, n is 1, R³ is substituted or unsubstituted non-aromatic heterocyclyl, R⁴ is a hydrogen atom, R⁶ is substituted or unsubstituted alkyl, ring A is the ring represented by the formula (I'c), p, q and r are 0. 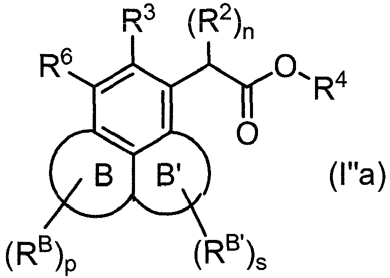 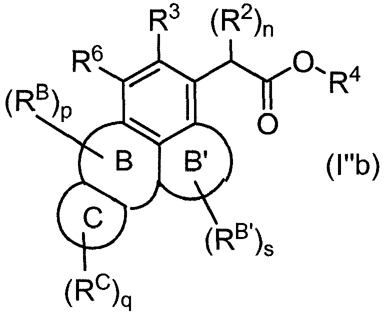 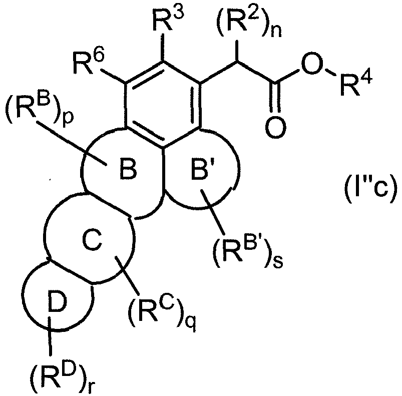
25) Compound represented by the formula (I"a) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p is one or more.
26) Compound represented by the formula (I"a) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p is one or more.
27) Compound represented by the formula (I"a) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p is one or more.
28) Compound represented by the formula (I"a) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p is one or more.
29) Compound represented by the formula (I"b) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p and/or q are/is one or more.
30) Compound represented by the formula (I"b) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p and/or q are/is one or more.
31) Compound represented by the formula (I"b) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p and/or q are/is one or more.
32) Compound represented by the formula (I"b) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p and/or q are/is one or more.
33) Compound represented by the formula (I"c) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p, q and/or r are/is one or more.
34) Compound represented by the formula (I"c) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p, q and/or r are/is one or more.
35) Compound represented by the formula (I"c) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p, q and/or r are/is one or more.
36) Compound represented by the formula (I"c) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p, q and/or r are/is one or more.
37) Compound represented by the formula (I"a) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p is 0.
38) Compound represented by the formula (I"a) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p is 0.
39) Compound represented by the formula (I"a) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p is 0.
40) Compound represented by the formula (I"a) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p is 0.
41) Compound represented by the formula (I"b) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p and q are 0.
42) Compound represented by the formula (I"b) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p and q are 0.
43) Compound represented by the formula (I"b) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p and q are 0.
44) Compound represented by the formula (I"b) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p and q are 0.
45) Compound represented by the formula (I"c) wherein R² is substituted or unsubstituted alkyloxy, n is 1, R³ is substituted or unsubstituted aromatic carbocyclyl, R⁴ is a hydrogen atom, R⁶ is substituted or unsubstituted alkyl, p, q and r are 0.
46) Compound represented by the formula (I"c) wherein R² is substituted or unsubstituted alkyloxy, n is 1, R³ is substituted or unsubstituted non-aromatic carbocyclyl, R⁴ is a hydrogen atom, R⁶ is substituted or unsubstituted alkyl, p, q and r are 0.
47) Compound represented by the formula (I"c) wherein R² is substituted or unsubstituted alkyloxy, n is 1, R³ is substituted or unsubstituted aromatic heterocyclyl, R⁴ is a hydrogen atom, R⁶ is substituted or unsubstituted alkyl, p, q and r are 0.
48) Compound represented by the formula (I"c) wherein R² is substituted or unsubstituted alkyloxy, n is 1, R³ is substituted or unsubstituted non-aromatic heterocyclyl, R⁴ is a hydrogen atom, R⁶ is substituted or unsubstituted alkyl, p, q and r are 0.

Preferred embodiments of ring B include B1) to B8) as follows.
B1) Aromatic heterocycle wherein p is one or more, one of R^{B} is substituted or unsubstituted alkyl.
B2) Non-aromatic heterocycle wherein p is one or more, one of R^{B} is substituted or unsubstituted alkyl.
B3) Aromatic heterocycle wherein p is one or more, one of R^{B} is oxo.
B4) Non-aromatic heterocycle wherein p is one or more, one of R^{B} is oxo.
B5) Aromatic heterocycle which forms a spiro ring together with another ring.
B6) Non-aromatic heterocycle which forms a spiro ring together with another ring.
B7) Aromatic heterocycle wherein p is 0.
B8) Non-aromatic heterocycle wherein p is 0.

Preferred embodiments of ring C include C1) to C12) as follows.
C1) Aromatic carbocycle wherein q is one or more, one of R^{C} is substituted or unsubstituted alkyl.
C2) Non-aromatic carbocycle wherein q is one or more, one of R^{C} is substituted or unsubstituted alkyl.
C3) Aromatic heterocycle wherein q is one or more, one of R^{C} is substituted or unsubstituted alkyl.
C4) Non-aromatic heterocycle wherein q is one or more, one of R^{C} is substituted or unsubstituted alkyl.
C5) Aromatic carbocycle wherein q is one or more, one of R^{C} is halogen.
C6) Non-aromatic carbocycle wherein q is one or more, one of R^{C} is halogen.
C7) Aromatic heterocycle wherein q is one or more, one of R^{C} is halogen.
C8) Non-aromatic heterocycle wherein q is one or more, one of R^{C} is halogen.
C9) Aromatic carbocycle wherein q is 0.
C10) Non-aromatic carbocycle wherein q is 0.
C11) Aromatic heterocycle wherein q is 0.
C12) Non-aromatic heterocycle wherein q is 0.

Preferred embodiments of ring D include D1) to D8) as follows.
D1) Aromatic carbocycle wherein r is one or more, one of R^{D} is substituted or unsubstituted alkyl.
D2) Non-aromatic carbocycle wherein r is one or more, one of R^{D} is substituted or unsubstituted alkyl.
D3) Aromatic heterocycle wherein r is one or more, one of R^{D} is substituted or unsubstituted alkyl.
D4) Non-aromatic heterocycle wherein r is one or more, one of R^{D} is substituted or unsubstituted alkyl.
D5) Aromatic carbocycle wherein r is 0.
D6) Non-aromatic carbocycle wherein r is 0.
D7) Aromatic heterocycle wherein r is 0.
D8) Non-aromatic heterocycle wherein r is 0.

Preferred embodiments of ring B' include B'1) to B'8) as follows.
B'1) Aromatic heterocycle wherein s is one or more, one of R^{B'} is substituted or unsubstituted alkyl.
B'2) Non-aromatic heterocycle wherein s is one or more, one of R^{B'} is substituted or unsubstituted alkyl.
B'3) Aromatic heterocycle wherein s is one or more, one of R^{B'} is oxo.
B'4) Non-aromatic heterocycle wherein s is one or more, one of R^{B'} is oxo.
B'5) Aromatic heterocycle which forms a spiro ring together with another ring.
B'6) Non-aromatic heterocycle which forms a spiro ring together with another ring. B'7) Aromatic heterocycle wherein s is 0.
B'8) Non-aromatic heterocycle wherein s is 0.

The compound represented by the formula (I) can take any combination selected from any oen of 1) to 48), any one of B1) to B8), any one of C1) to C12), any one of D1) to D8) and any one of B'1) to B'8) in the above.

When ring A is the ring represented by the formula (I'c), the ring can take any combination selected from any one of B1) to B8) and any one of C1) to C12) in the above.

When ring A is the ring represented by the formula (I'd), the ring can take any combination selected from any one of B1) to B8), any one of C1) to C12) and any one of D1) to D8) in the above.

Preferred embodiments of the compound represented by the formula (I) include compound in the above 1) to 24), wherein R¹ is replaced by a hydrogen atom, halogen, substituted or unsubstituted alkenyl, or substituted or unsubstituted alkynyl.

Also, preferred embodiments of the compound represented by the formula (I) include compound in the above 1) to 48), wherein R² is replaced by substituted or unsubstituted alkyl, substituted or unsubstituted alkenyl, substituted or unsubstituted alkynyl, substituted or unsubstituted alkenyloxy, substituted or unsubstituted alkynyloxy, substituted or unsubstituted alkylsulfanyl, substituted or unsubstituted alkenylsulfanyl, or substituted or unsubstituted alkynylsulfanyl.

Also, preferred embodiments of the compound represented by the formula (I) include compound in the above 1) to 48), wherein R⁶ is replaced by a hydrogen atom, halogen, substituted or unsubstituted alkenyl, or substituted or unsubstituted alkynyl.

Compound (I) is more preferably the compound represented by the above formula (1-0), (I-1), (1-2), (1-3), (1-4) or (1-5), further preferably the compound represented by the formula (I-1), (1-2) or (1-3), wherein each definition has the same meaning as compound (I), preferably compound (I-0). Examples of these compounds are below. 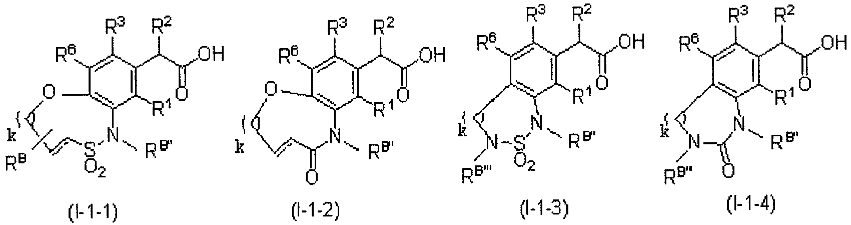 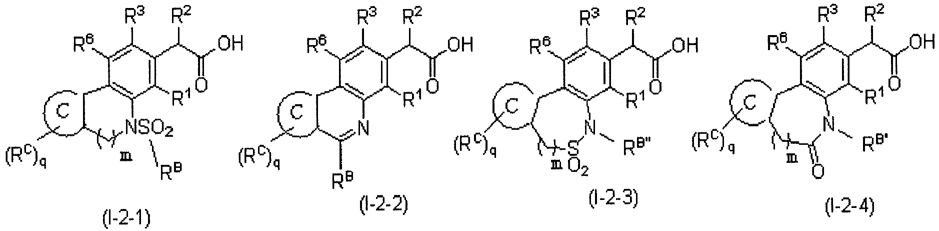 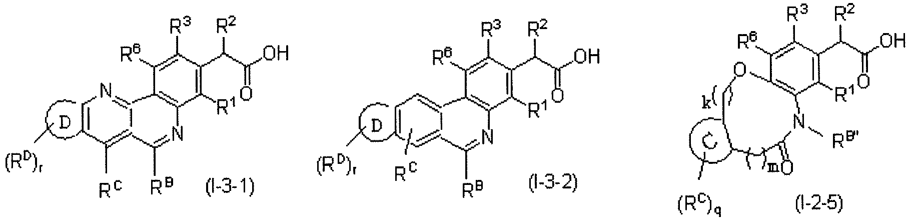 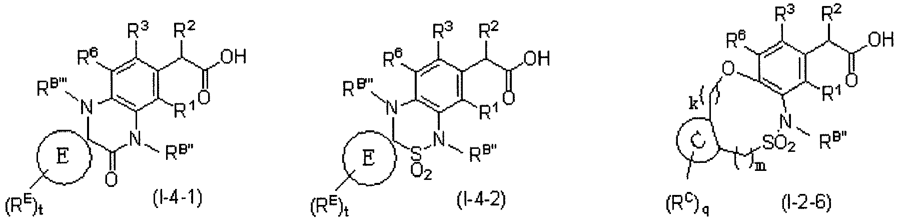 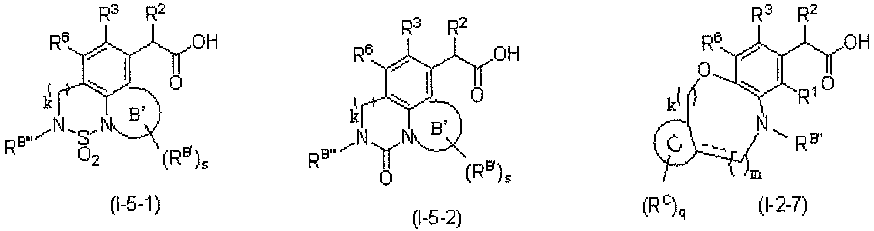 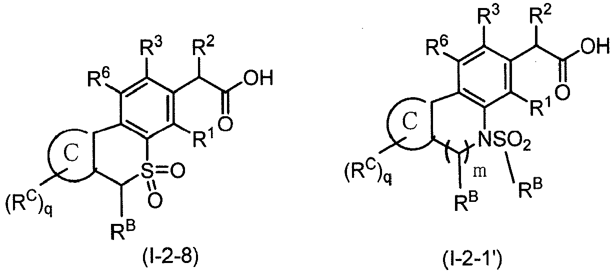 wherein each definition has the same meaning as described above. Preferred embodiment is below:
R¹ is alkyl, preferably C1∼C4 alkyl;
R² is alkyloxy, preferably C1∼C6 alkyloxy or optionally substituted cycloalkyloxy, preferably C3∼C7 cycloalkyloxy optionally substituted with methyl;
R³ is substituted or unsubstituted phenyl, or substituted or unsubstituted 5- to 7-membered heterocycle, or its fused ring, wherein the ring fuzed to said pheny or heterocycle is preferably 1 to 2 of benzene and/or 5- to 7-membered heterocycle. The substituent(s) of R³ has(have) the same meaning as described above.;
R⁶ is alkyl;
ring C, ring D, and ring B' are each independently, monocyclic aromatic carbocycle (e.g.: benzene), monocyclic non-aromatic carbocycle (e.g.: cyclohexane, cycloheptane, cyclohexene, cycloheptene), monocyclic aromatic heterocycle or monocyclic non-aromatic heterocycle, preferably 5- to 8-membered ring;
ring E is optionally crosslinked carbocycle, more preferably C3∼C8 carbocycle, further preferably 5- to 8-membered carbocycle;
R^{B}, R^{C} , R^{B'} and R^{E} are each independently, a hydrogen atom, halogen, oxo, hydroxy, carboxy, imino, substituted or unsubstituted amino (examples of substituent: alkyl), substituted or unsubstituted alkyl, substituted or unsubstituted alkoxy (examples of substituent: halogen, hydroxy, alkoxy, phenyl), substituted or unsubstituted cycloalkyl, substituted or unsubstituted cycloalkylalkyl, formyl, substituted or unsubstituted alkoxycarbonyl, substituted or unsubstituted aryl, substituted or unsubstituted aralkyl, substituted or unsubstituted heterocyclyl, substituted or unsubstituted heterocyclyl alkyl (preferred examples of substituent: alkyl, hydroxy, alkoxy, halogen), or substituted sulfonyl (preferred examples of substituent: alkyl, phenyl, cycloalkyl, cycloalkylalkyl, aralkyl), more preferably halogen, oxo, amino, substituted or unsubstituted alkyl, or substituted or unsubstituted alkoxy;
As to R^{B}, especially alkyl (e.g.: methyl) is preferred;
R^{C} is especially a hydrogen atom, oxo, imino, halogen, substituted or unsubstituted alkyl, substituted or unsubstituted alkoxy, substituted or unsubstituted alkyloxycarbonyl, substituted or unsubstituted alkylsulfonyl, substituted or unsubstituted phenyl, or substituted or unsubstituted benzyl. R^{C} is most preferably a hydrogen atom, oxo, imino, halogen or substituted or unsubstituted methyl, substituted or unsubstituted methoxy, substituted or unsubstituted benzyl, substituted or unsubstituted phenethyl, substituted or unsubstituted benzyloxycarbonyl or substituted or unsubstituted methylsulfonyl; the substituent(s) of "substituted or unsubstituted" is preferably alkyl, cycloalkyl, cycloalkylalkyl, alkoxy, alkoxyalkyl, halogen, haloalkyl, amino, alkylamino, hydroxy, oxo, carboxy, phenyl or halophenyl;
R^{B"} and R^{B'''} are each independently a hydrogen atom, alkyl, cycloalkyl, cycloalkylalkyl, formyl, alkoxycarbonyl, substituted or unsubstituted aryl , substituted or unsubstituted aralkyl, substituted or unsubstituted heterocyclyl, substituted or unsubstituted heterocyclylalkyl (preferred examples of substituent on the each ring: alkyl, halogen, hydroxy, alkoxy), or substituted sulfonyl (preferred examples of substituent: alkyl, cycloalkyl, cycloalkylalkyl, phenyl, alkylphenyl);
a broken line means the presence or absence of bond;
t is an integer of 0 to 12, preferably 0 to 4, more preferably 0 to 2;
q, r, s, k and m are each independently an integer of 0 to 4, preferably 0 to 2;
As to m, especially 1 is preferred.

The modification of each substituent on R³, ring B, ring B', ring C, ring D and ring E is effective for not only improving main activity of the compound but also improving metabolism and reducing side effects.

The present invention particularly preferably provides the Compound (1-2).

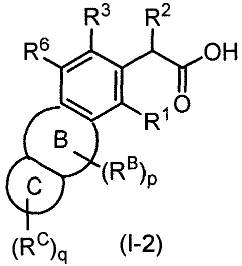

In Compound (1-2), ring B preferably 5- to 7-membered heterocycle and constituent atoms of each ring include more preferably at least nitrogen atom and/or sulfur atom;
ring B more preferably include the partial structure of -N(SO₂ R^{B})-, -N=CR^{B}- or-N(RB")SO₂ -;
ring C is preferably benzene or 5- to 7-membered heterocycle (e.g.: pyridine);
R^{B} and R^{C} are each independently, preferably halogen, alkyl, haloalkyl, hydroxy, alkoxy, haloalkoxy, oxo, carboxy, or substituted or unsubstituted amino (examples of substituent: alkyl);
R^{B} is preferably -SO₂ -alkyl, alkyl (e.g.: methyl);
R^{C} is preferably 1 or 2 of alkyl (e.g.: methyl), alkoxy (e.g.: methoxy) and/or halogen (e.g.: F);
p and q are each independently, preferably 1 or 2.
R^{B} and R^{C} may be taken together to form substituted or unsubstituted 5- to 10-memberd carbocycle or heterocycle, preferably 6- to 8-membered carbocycle or heterocycle; the substituent(s) of "substituted or unsubstituted" is preferably alkyl, cycloalkyl, cycloalkylalkyl, alkoxy, alkoxyalkyl, halogen, haloalkyl, amino, alkylamino, hydroxy, oxo, carboxy, phenyl or halophenyl.

Compound (1-2) is more preferably the above Compound (1-2-1), (1-2-2), (1-2-3), (1-2-4), (1-2-5), (1-2-6) or (I-2-7), particularly preferably, (1-2-1), (1-2-2) or (1-2-3). Compund (I-2-1) is preferably Compound (1-2-1-1) to (1-2-1-9) or (I-2-1-2') to (I-2-1-9`), particularly preferably (1-2-1-1) to (1-2-1-5). In these compounds, the preferred embodiment of R^{B} and R^{C} is below.

Furthermore, R³ of the Compound (I) is preferably any one of the groups examplified below, wherein R³ may be each independently substituted with 1 to 4 of the same or different group(s) selected from alkyl, alkoxy, halogen, hydroxy, hydroxyalkyl, alkoxyalkyl, haloalkyl, oxo, amino, mono- and di-alkylamino, and cyano.

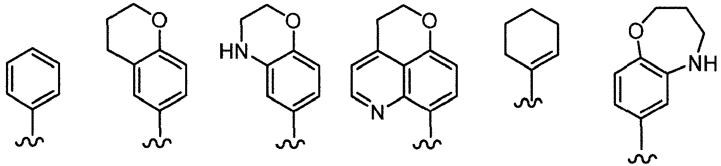

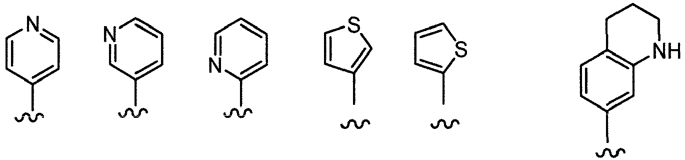

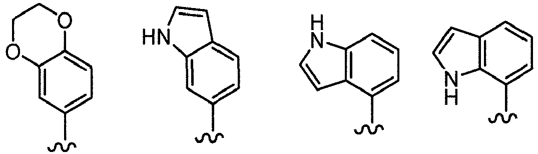

A characteristic of the compound according to the present invention is to have an inhibitory effect on HIV replication, in which, in the formula (I),
(1) the benzene ring, the main skeleton, is substituted with at least one cyclic group (R3),
(2) the benzene ring is fused with another ring (A), and
(3) the benzene ring has a side chain represented by -C(R²)nCOOR⁴ (n = 1, 2).

Another characteristic of the compound according to the present invention is that substituted or unsubstituted alkyloxy, or substituted or unsubstituted cycloalkyloxy is preferably introduced as R² in the formula (I), and/or R⁴ is a hydrogen atom, thereby having a high inhibitory effect on HIV replication.

In one preferred embodiments of ring A, 1) ring A is more than 7-membered, 2) ring A has substituent(s) except a hydrogen atom, and/or 3) ring A is fused with another ring.

When ring A is 6-membered ring, the ring is preferably fused with another ring preferably fused with benzene or 5- to 7-membered heterocycle, more preferably the ring A or the fused ring has substituent(s).

The compound of the present invention is significantly improved on HIV replication inhibiting activity, various pharmacokinetics, and/or safety of pharmaceuticals by these structural features. Furthermore, these profiles have been significantly improved by ring A, R³ and preferred combinations.

In Compound (I), the compound wherein R⁴ is group except a hydrogen atom, preferably alkyl, is also useful as an intermediate of HIV replication inhibitors.

The compound represented by the formula (I) is not limited to a specific isomer, and includes all possible isomers (e.g., keto-enol isomers, imine-enamine isomers, diastereoisomers, atropisomers, optical isomers, rotamers, etc.), racemates or mixtures thereof. Although, these isomers are often easily separated by optical resolution, crystallization, chromatographic separation and the like, these may be displayed in the same flat structural. Also, when these can be separated by chromatographic separation, these are distinguishable by peak time (RT).

One or more hydrogens, carbons and/or other atoms of the compounds represented by the formula (I) may be substituted by an isotope of hydrogen, carbon and/or other atom. Examples of the isotope include hydrogen, carbon, nitrogen, oxygen, phosphorus, sulfur, fluorine, iodine and chlorine, like ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl. The compound represented by the formula (I) also includes compounds substituted with the isotope. The compound substituted with the isotope is also useful as a pharmaceutical, and includes all radiolabeled materials of the compounds represented by the formula (I). Also, a "radiolabeling method" for producing the "radiolabeled material" is also included in the present invention, and it is useful as a research and/or diagnostic tool in metabolism pharmacokinetic studies and binding assays.

The radiolabeled material of the compound represented by the formula (I) can be prepared by a method well known in the art. For example, a tritium-labeled compound represented by the formula (I) can be prepared, for example, by introducing tritium into a particular compound represented by the formula (I) by catalytic dehalogenation using tritium. This method includes reacting a precursor in which the compound represented by the formula (I) is properly substituted with halogen with tritium gas, in the presence of an appropriate catalyst, for example, Pd/C, in the presence or absence of a base. As the appropriate method for preparing other tritium-labeled compound, document of Isotopes in the Physical and Biomedical Sciences, Vol. 1, Labeled Compounds (Part A), Chapter 6 (1987) can be referred. A ¹⁴C-labeled compound can be prepared by using a raw material having a ¹⁴C carbon.

Examples of the pharmaceutically acceptable salt of the compound represented by the formula (I) include salts of the compound represented by the formula (I) with an alkali metal (e.g., lithium, sodium, potassium, etc.), an alkaline earth metal (e.g., calcium, barium, etc.), magnesium, a transition metal (e.g., zinc, iron, etc.), ammonia, an organic base (e.g., trimethylamine, triethylamine, dicyclohexylamine, ethanolamine, diethanolamine, triethanolamine, meglumine, diethanolamine, ethylenediamine, pyridine, picoline, quinolone, etc.) and an amino acid, or salts with an inorganic acid (e.g., hydrochloric acid, sulfuric acid, nitric acid, carbonic acid, hydrobromic acid, phosphoric acid, hydroiodic acid, etc.) and an organic acid (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid, etc.). Examples include, particularly, salts with hydrochloric acid, sulfuric acid, phosphoric acid, tartaric acid, or methanesulfonic acid, and the like. These salts can be formed by a method usually carried out.

The compound represented by the formula (I) of the present invention and a pharmaceutically acceptable salt thereof may form a solvate (e.g., hydrate, etc.) and/or a crystalline polymorph, and the present invention also includes various kinds of solvates and crystalline polymorphs. "Solvate" may be coordinated with solvent molecules (e.g., water molecules, etc.) in any number, relative to the compound represented by the formula (I). The compound represented by the formula (I) or a pharmaceutically acceptable salt thereof is left in the air, whereby it may absorb water and the adsorbed water may attach thereto, or it may form a hydrate. In addition, there is a case that the compound represented by the formula (I) or a pharmaceutically acceptable salt thereof is recrystallized to form a crystalline polymorph thereof.

The compound represented by the formula (I) of the present invention or a pharmaceutically acceptable salt thereof may form a prodrug, and the present invention also includes such various prodrugs. The prodrug is a derivative of the compound of the present invention having a group that can chemically or metabolically decompose, and is a compound to be the pharmaceutically active compound of the present invention in vivo by solvolysis or under physiological conditions. The prodrug includes compounds which are converted to a compound represented by the formula (I) in response to enzymatic oxidation, reduction, hydrolysis, or the like, under physiological conditions in vivo, compounds which are converted to a compound represented by the formula (I) by being hydrolyzed by gastric acid or the like, and the like. A method for selecting an appropriate prodrug derivative and a method for producing the same are described, for example, in Design of Prodrugs, Elsevier, Amsterdam 1985. The prodrugs themselves may have an activity.

When the compound represented by the formula (I) or a pharmaceutically acceptable salt thereof has a hydroxyl group, for example, prodrugs such as acyloxy derivatives and sulfonyloxy derivatives produced by reacting a compound having a hydroxyl group with an appropriate acyl halide, an appropriate acid anhydride, an appropriate sulfonyl chloride, an appropriate sulfonyl anhydride and a mixed anhydrides or by reacting using a condensing agent are exemplified. Examples include CH₃COO-, C₂H₅COO-, t-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO-, CH₃SO₃-, CH₃CH₂SO₃-, CF₃SO₃-, CH₂FSO₃-, CF₃CH₂SO₃-, p-CH₃-O-PhSO₃-, PhSO₃-, p-CH₃PhSO₃-, and the like.

### (Method for Producing Compound of Present Invention)

The compound of the present invention can be produced, for example, according to the general synthesis method described below. In addition, extraction, purification and the like may be performed by a treatment performed in a normal experiment of organic chemistry.

The synthesis of the compound of the present invention can be carried out while referring to the method known in the art. The present invention also provides intermediates and final compounds in the following general synthetic methods. The type of substituent(s) in each compound and preferred embodiments are as defined above.

In the following general synthetic methods, R^{B} and/or R^{C} may be a hydrogen atom.

### 1) Synthesis of Compounds A-1 and A-2

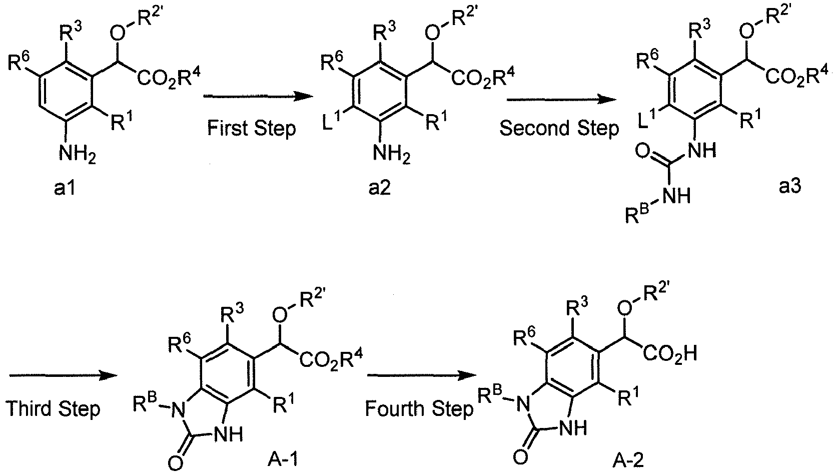

wherein R¹, R³, R⁴, R⁶ and R^{B} have the same meaning as described above; R²' is substituted or unsubstituted alkyl, substituted or unsubstituted alkenyl, or substituted or unsubstituted alkynyl; L¹ is leaving group such as halogen.

### First Step

In a solvent such as dichloromethane, THF, toluene, acetonitrile or DMF, bromine or a halogenating reagent such as NBS, NCS or NIS is added to Compound a1, and when L¹ is bromo, the mixture is reacted at -30°C to 50°C, and preferably at - 10°C to 20°C, for 0.1 hours to 10 hours, and preferably 0.5 hours to 2 hours, whereby Compound a2 can be obtained. When L¹ is chloro or iodine, the mixture is reacted at 10°C to 150°C, and preferably at 60°C to 120°C, for 0.5 hours to 24 hours, and preferably 1 hour to 6 hours, whereby Compound a2 can be obtained.

### Second Step

In a solvent such as dichloromethane, dichloroethane, THF or toluene, a base such as triethylamine, N-methylmorpholine or N,N-diisopropylethylamine is added to Compound a2, and then triphosgene is sequentially added, and the mixture is reacted at -30°C to 50°C, and preferably at -10°C to 20°C, for 0.1 hours to 10 hours, and preferably 0.5 hours to 2 hours, whereby isocyanate can be obtained. Also, in a solvent such as acetonitrile, DMF or DMA, carbonyl imidazole is added to Compound a2, and the mixture is reacted at -30°C to 100°C, and preferably at 0°C to 50°C, for 0.1 hours to 10 hours, and preferably 0.5 hours to 2 hours, whereby isocyanate can be obtained. The obtained isocyanate is not isolated. To the reaction mixture, an amine that is commercially available or synthesized by a known method is added, and the mixture is reacted at -30°C to 100°C, and preferably at 0°C to 50°C, for 0.1 hours to 24 hours, and preferably 0.5 hours to 12 hours, whereby Compound a3 can be obtained.

### Third Step

In a solvent such as toluene, xylene, DMF or DMA, a base such as 2,6-lutidine, N,N-diisopropylethylamine or diazabicycloundecene, and copper iodide (I) are added to Compound a3, and the mixture is reacted in a sealed tube at 30°C to 250°C, and preferably at 80°C to 200°C, for 0.1 hours to 10 hours, and preferably 0.5 hours to 2 hours, whereby Compound A-1 can be obtained.

### Fourth Step

In a solvent such as methanol, ethanol, THF or DMSO, or in a mixed solvent thereof, a base such as potassium hydroxide, sodium hydroxide or lithium hydroxide is added to Compound A-1, and the mixture is reacted at 0°C to 150°C, and preferably at 20°C to 100°C, for 0.1 hours to 24 hours, and preferably 1 hour to 6 hours, whereby Compound A-2 can be obtained.

Also, in a solvent such as methanol, ethanol, ethyl acetate or THF, a catalyst such as 5% or 10% palladium carbon, palladium hydroxide or platinum dioxide is added to Compound A-1, and the mixture is reacted under a hydrogen atmosphere and under 1 to 10 atmospheres, and preferably 1 to 3 atmospheres, at 0°C to 60°C, and preferably at 20°C to 40°C, for 0.1 hours to 24 hours, and preferably 1 hour to 12 hours, whereby Compound A-2 can be obtained.

Compound a1 can be synthesized by the following method. 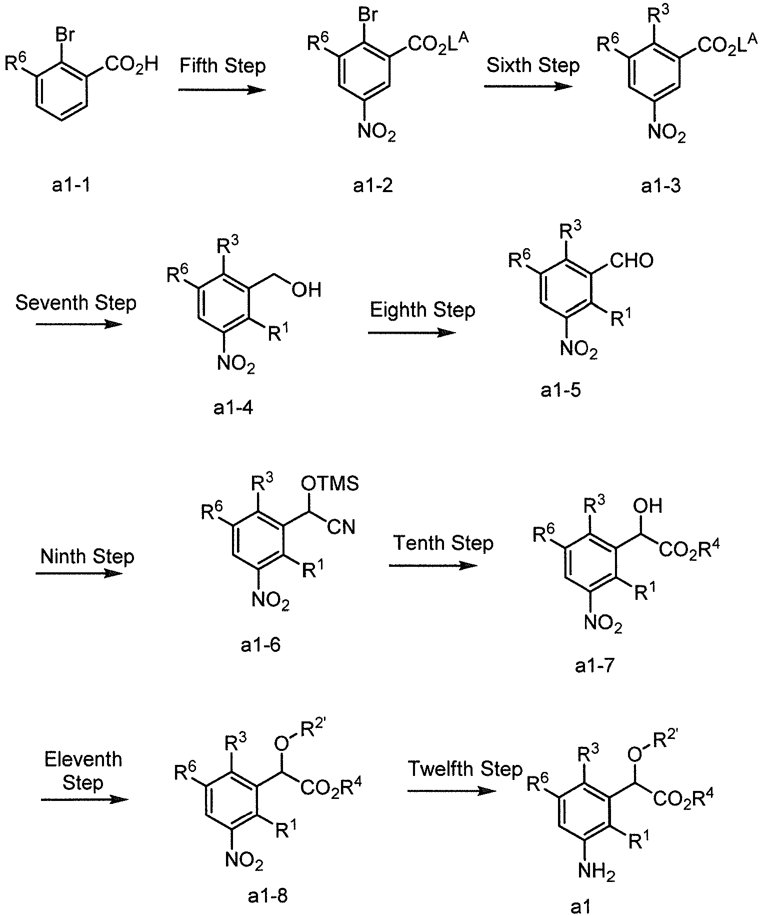 wherein R¹, R²', R³, R⁴ and R⁶ have the same meaning as described above; L^{A} is substituted or unsubstituted alkyl; L^{B} is leaving group such as halogen.

### Fifth Step

To Compound al-1 that is commercially available or synthesized by a known method is added, in a solvent such as concentrated sulfuric acid or acetic acid, nitric acid, fuming nitric acid or the like under ice-cooling, and the mixture is reacted at - 20°C to 60°C, and preferably at 0°C to 25°C, for 0.5 hours to 6 hours, and preferably 1 hour to 3 hours, whereby a nitro compound can be obtained. This compound is reacted in a solvent such as thionyl chloride and phosphorus oxychloride, at 20°C to 120°C, and preferably at 80°C to 100°C, for 0.5 hours to 6 hours, and preferably 1 hour to 3 hours, and the solvent is concentrated under reduced pressure, whereby a crude acid chloride can be obtained. Subsequently, in a L^{A}OH solvent, the crude chloride is reacted at 20°C to 120°C, and preferably at 50°C to 80°C, for 0.5 hours to 6 hours, and preferably 1 hour to 3 hours, whereby Compound a1-2 can be obtained.

### Sixth Step

Compound a1-3 can be obtained by a coupling reaction of Compound a1-2 with R³-L⁴. As L⁴, a boronic acid, a boronic acid ester, an alkyltin, a zinc halide and the like are exemplified. As the reaction, Suzuki cross-coupling, Ullmann cross-coupling, Negishi cross-coupling, Stille coupling, and the like are exemplified.

In a solvent such as dioxane, DMF, DME, THF or water, or in a mixed solvent thereof, a palladium catalyst such as Pd(PPh₃)₄, Pd(OAc)₂, Pd(PPh₃)₂Cl₂ or Pd(dppf)₂Cl₂, a base such as potassium carbonate, sodium carbonate or potassium phosphate, and a boronic acid, a boronic acid ester, an alkyltin or a zinc halide that is commercially available or synthesized by a known method, are added to Compound a1-2, and the mixture is reacted under a nitrogen atmosphere at 0°C to 150°C, and preferably at 60°C to 120°C, for 0.5 hours to 24 hours, and preferably 1 hour to 12 hours, whereby Compound a1-3 can be obtained.

### Seventh Step

In a solvent such as methylene chloride, dichloroethane or tetrahydrofuran, a toluene solution of diisobutylaluminum hydride or a tetrahydrofuran solution of lithium aluminum hydride is added to Compound a1-3, and the mixture is reacted at - 100°C to 50°C, and preferably at -60°C to 0°C, for 0.5 hours to 10 hours, and preferably 1 hour to 3 hours, whereby Compound a1-4 can be obtained.

### Eighth Step

In a solvent such as methylene chloride, acetone or DMSO, an oxidant such as a Dess-Martin reagent, manganese dioxide or sulfur trioxide pyridine is added to Compound a1-4, and the mixture is reacted at -20°C to 50°C, and preferably at 0°C to 30°C, for 0.5 hours to 10 hours, and preferably 1 hour to 3 hours to be oxidized, whereby Compound al-5 can be obtained.

### Ninth Step

In a solvent such as methylene chloride, dichloroethane or toluene, zinc iodide and TMSCN are added to Compound a1-5, and the mixture is reacted at -20°C to 50°C, and preferably at 0°C to 30°C, for 0.1 hours to 10 hours, and preferably 0.5 hours to 2 hours, whereby Compound a1-6 can be obtained.

### Tenth Step

In R⁴OH, an acid such as concentrated sulfuric acid or concentrated hydrochloric acid is added to Compound a1-6, and the mixture is reacted at 0°C to 150°C, and preferably at 80°C to 110°C, for 1 hour to 24 hours, and preferably 6 hours to 12 hours, for deprotection of a TMS group, hydrolysis to the carboxylic acid of nitrile group, followed by esterification, whereby Compound a1-7 can be obtained.

### Eleventh Step

In a solvent such as THF, DMF or toluene, a base such as sodium hydride, potassium tert-butoxide or sodium methoxide and R²'-I, R²'-Br, R²'-Cl or the like are added to Compound a1-7, and the mixture is reacted at -20°C to 100°C, and preferably at 0°C to 60°C, for 1 hour to 24 hours, and preferably 3 hours to 12 hours, whereby Compound a1-8 can be obtained.

Also, tert-butyl ester, tert-pentyl ether, methylcyclobutane ether and the like can be also obtained by adding 1 to 3 equivalents of a aqueous perchloric acid solution in a solvent such as tert-butyl acetate, tert-pentyl acetate or methylenecyclobutane, and reacting the mixture at 0°C to 60°C, and preferably at 15°C to 30°C, for 0.1 hours to 10 hours, and preferably 0.5 hours to 2 hours.

### Twelfth Step

In a solvent such as methanol, ethanol, THF, ethyl acetate or acetic acid, or in a mixed solvent thereof, a catalyst such as 5% or 10% palladium carbon, palladium hydroxide or platinum dioxide is added to Compound a1-8, and the mixture is reacted under a hydrogen atmosphere at atmospheric or more pressure, at 0°C to 50°C, and preferably at 15°C to 25°C, for 0.1 hours to 48 hours, and preferably 1 hour to 24 hours, whereby Compound a1 can be obtained.

Also, in a mixed solvent of an organic solvent such as methanol, ethanol or THF and water, a metal such as iron, zinc or tin is added to compound a12, under acidic conditions of hydrochloric acid or acetic acid, under alkaline conditions of potassium hydroxide or sodium hydroxide, or under neutral conditions of ammonium chloride, and the mixture is reacted at 0°C to 120°C, and preferably at 25°C to 80°C, for 0.1 hours to 24 hours, and preferably 1 hour to 6 hours, whereby Compound a1 can be also obtained.

### 2) Synthesis of Compounds B-1 and B-2

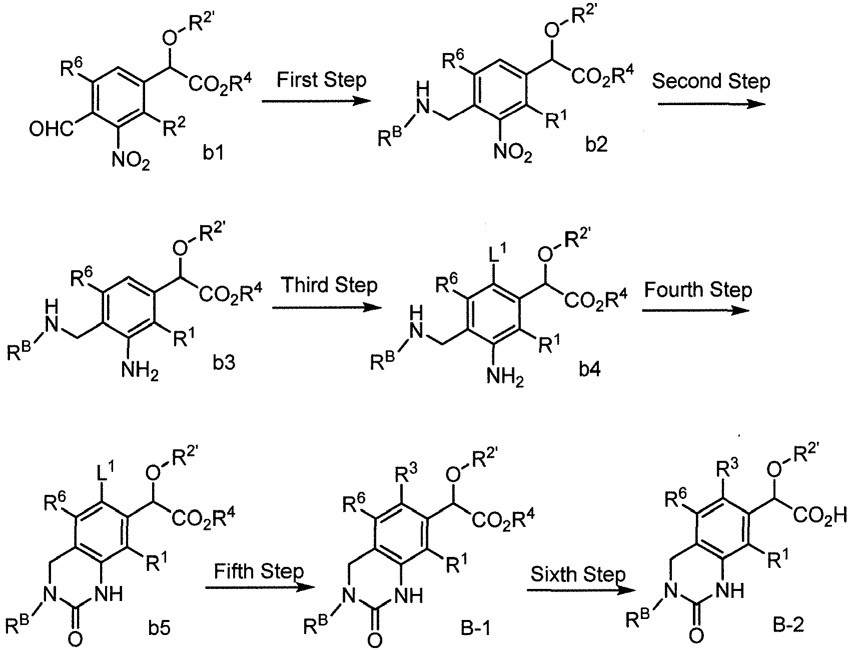

wherein each definition has the same meaning as described above.

### First Step

In a solvent such as dichloromethane, dichloroethane, THF, methanol or ethanol, acetic acid, TFA or the like, and an amine that is commercially available or synthesized by a known method, are added to Compound b1, and the mixture is reacted at 0°C to 150°C, and preferably at 20°C to 60°C, for 0.1 hours to 24 hours, and preferably 1 hour to 6 hours, whereby a imine can be obtained. The obtained imine is not isolated. To the reaction mixture, a reducing agent such as sodium cyanoborohydride or sodium triacetoxyborohydride is added, and the mixture is reacted at -30°C to 50°C, and preferably at 0°C to 20°C, for 0.1 hours to 24 hours, and preferably 0.5 hours to 12 hours, whereby Compound b2 can be obtained.

### Second Step

In a solvent such as methanol, ethanol, THF, ethyl acetate or acetic acid, or in a mixed solvent thereof, a catalyst such as 5% or 10% palladium carbon, palladium hydroxide or platinum dioxide is added to Compound b2, and the mixture is reacted under a hydrogen atmosphere at atmospheric or more pressure, at 0°C to 50°C, and preferably at 15°C to 25°C, for 0.1 hours to 48 hours, and preferably 1 hour to 24 hours, whereby Compound b3 can be obtained.

In this condition, the reaction may be promoted by adding acetic acid, hydrochloric acid or the like. In a mixed solvent of an organic solvent such as methanol, ethanol or THF and water, a metal such as iron, zinc or tin is added to Compound b2, under acidic conditions of hydrochloric acid or acetic acid, under alkaline conditions of potassium hydroxide or sodium hydroxide, or under neutral conditions of ammonium chloride, and the mixture is reacted at 0°C to 150°C, and preferably at 25°C to 80°C, for 0.1 hours to 24 hours, and preferably 1 hour to 6 hours, whereby Compound b3 can be also obtained.

### Third Step

Compound b4 can be obtained from Compound b3 in the same manner as described above in the first step in "1) Synthesis of Compounds A-1 and A-2".

### Fourth Step

In a solvent such as dichloroethane, toluene, xylene, dioxane, DMF or DMA, a base such as N,N-diisopropylethylamine, 2,6-lutidine or diazabicycloundecene, and carbonyl imidazole, triphosgene or the like are added to Compound b4, and the mixture is reacted at 20°C to 180°C, and preferably at 80°C to 120°C, for 0.5 hours to 24 hours, and preferably 4 hours to 12 hours, whereby Compound b5 can be obtained.

### Fifth Step

Compound B-1 can be obtained by a coupling reaction of Compound b5 with L⁴-R³. As the reaction, Suzuki cross-coupling, Ullmann cross-coupling, Negishi cross-coupling, Stille coupling, and the like are exemplified. As L⁴, a boronic acid, a boronic acid ester, an alkyltin, a zinc halide and the like are exemplified.

In a solvent such as dioxane, DMF, DME, tetrahydrofuran or water, or in a mixed solvent thereof, a palladium catalyst such as Pd(PPh₃)₄, Pd(OAc)₂, Pd(PPhs)₂Cl₂, Pd(dppf)₂Cl₂ or Pd(dtbpf), a base such as potassium carbonate, sodium carbonate, cesium carbonate or potassium phosphate, and a boronic acid, a boronic acid ester, an alkyltin or a zinc halide that is commercially available or prepared by a known method are added to compound b1, and the mixture is reacted under a nitrogen atmosphere at 0°C to 150°C, and preferably at 60°C to 120°C, for 0.5 hours to 24 hours, and preferably 1 hour to 12 hours, whereby Compound B-1 can be obtained.

### Sixth Step

Compound B-2 can be obtained from Compound B-1 in the same manner as described above in the fourth step in "1) Synthesis of Compounds A-1 and A-2".

Compound b1 can be synthesized by the following method. 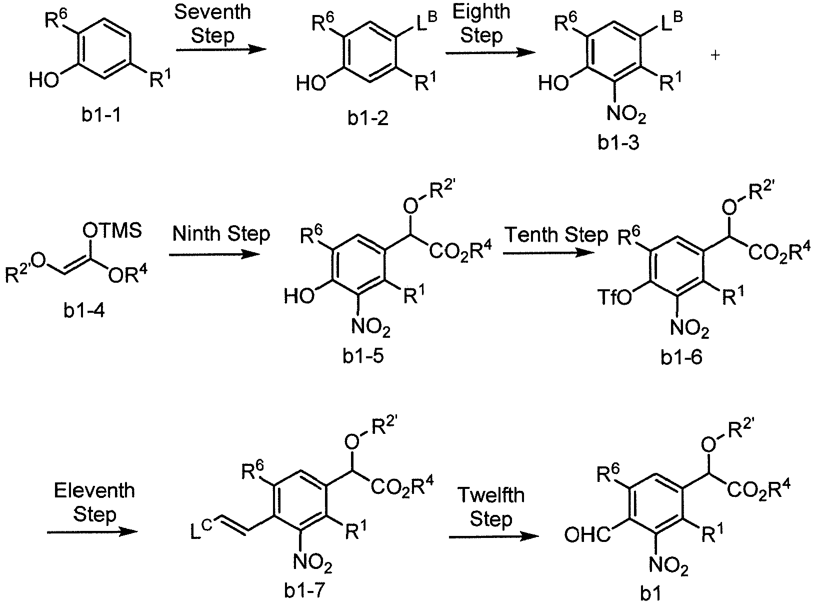 wherein R¹, R²', R⁴, R⁶ and L^{B} have the same meaning as described above; L^{C} is hydrogen or phenyl.

### Seventh Step

Compound b1-2 can be obtained from Compound b1-1 that is commercially available or synthesized by a known method in the same manner as described above in the eighth step"1) Synthesis of Compounds A-1 and A-2".

### Eighth Step

In a solvent such as concentrated sulfuric acid or acetic acid, nitric acid, fuming nitric acid or the like is added to Compound b1-2 under ice-cooling, and the mixture is reacted at -20°C to 60°C, and preferably at 0°C to 25°C, for 0.5 hours to 6 hours, and preferably 1 hour to 3 hours, whereby Compound b1-3 can be obtained.

### Ninth Step

In a solvent such as DMF, DMA, THF or dioxane, or in a mixed solvent thereof, a phosphine such as tri-tert-butylphosphine, tricyclohexylphosphine or triphenylphosphine, a catalyst such as dibenzylideneacetone palladium, palladium acetate or dichlorobistriphenylphosphine palladium, zinc fluoride and separately prepared silyl enol ether (b1-4) are added to Compound b1-3, and the mixture is reacted at 30°C to 130°C, and preferably at 50°C to 150°C, for 0.1 hours to 6 hours, and preferably 0.5 hours to 1 hour, whereby Compound b1-5 can be obtained.

### Tenth Step

In a solvent such as dichloromethane, 1,2-dichloroethane or tetrahydrofuran, or in a mixed solvent thereof, a base such as pyridine, lutidine or triethylamine, and a trifluoromethanesulfonylating agent such as trifluoromethanesulfonic anhydride or a comin's reagent are added to Compound b1-5, and the mixture is reacted at -50°C to 50°C, and preferably at -30°C to 30°C, for 0.1 hours to 4 hours, and preferably 0.5 hours to 1 hour, whereby Compound bl-6 can be obtained.

### Eleventh Step

In a solvent such as dioxane, DMF, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and trans-ethynylboronic acid, vinyl boronate, vinyl trialkyltin, or the like are added to Compound bl-6, and the mixture is reacted under a nitrogen atmosphere at 0°C to 150°C, and preferably at 60°C to 120°C, for 0.5 hours to 24 hours, and preferably 1 hour to 12 hours, whereby Compound b1-7 can be obtained.

### Twelfth Step

In dichloromethane or methanol or a mixed solvent thereof, an ozone gas is passed through Compound b1-7 at -100°C to 0°C, and preferably at -78°C to 50°C, for 0.5 hours to 6 hours, and preferably 1 hour to 3 hours, and thereafter dimethylsulfide trimethylphosphite, or the like is added, and the mixture is stirred at the same temperature for 0.1 hours to 3 hours, and preferably 0.5 hours to 1 hour, whereby Compound b1 can be obtained.

### 3) Synthesis of Compounds C-1 and C-2

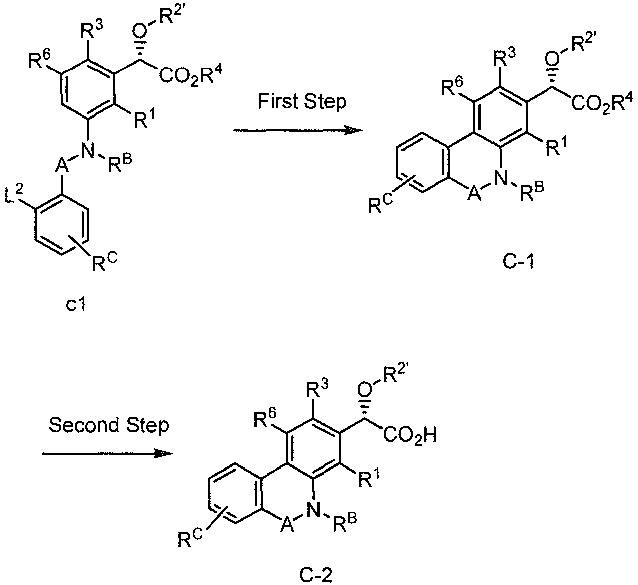

or 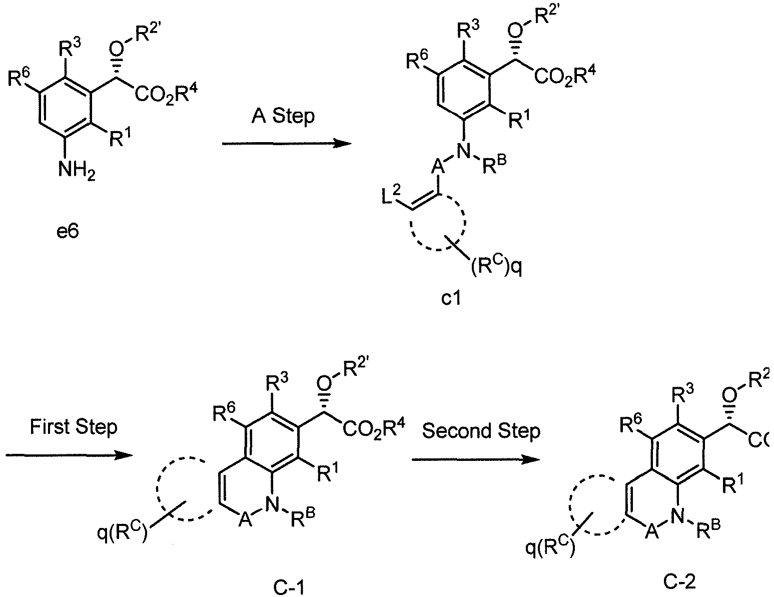 wherein R¹, R^{2'}, R³, R⁴, R⁶, R^{B} and R^{C} have the same meaning as described above; L² is leaving group such as halogen; A is a single bond, -SO₂-, -CO-, -O-CO-,-NH-CO- or alkylene optionally substituted with R^{B}; q is any integer of 1 to 4; dashlined portion is a ring C.

### A Step

In a solvent such as DMF, THF or dioxane, or in a mixed solvent thereof, a base such as pyridine or triethylamine, and a acylating agent such as acetyl chloride or substituted benzoyl chloride, a sulfonylating agent such as methanesulfonyl chloride or substituted benzenesulfonyl chloride, a urea agent such as ethyl isocyanate, or a carbamate agent such as allyloxycarbonyl chloride that is commercially available or prepared by a known method are added to Compound e6, and the mixture is reacted at -20°C to 50°C, and preferably at 0°C to 30°C, for 0.1 hours to 10 hours, and preferably 0.5 hours to 2 hours. To the obtained compound, in a solvent such as DMF, DME, THF, acetone or acetonitrile, a base such as potassium carbonate, sodium carbonate, cesium carbonate or sodium hydride, and a ring C alkyl halide optionally substituted with R^{C} or R^{B} that is commercially available or prepared by a known method (e.g., benzyl halide or heteroaryl methyl halide) or the like are added, and the mixture is reacted at 0°C to 100°C, and preferably at 20°C to 50°C, for 0.5 hours to 24 hours, and preferably 1 hour to 5 hours, whereby Compound C-1 can be obtained. Also, when R^{B} of Compound C-1 is alkylsulfonyl or the like, to the obtained compound, in a solvent such as THF, toluene or dichloromethane, Mitsunibu reagent such as DEAD, DIAD or bis(2-methoxyethyl) azodicarboxylate, such as triphenylphosphine, tri-n-butyl phosphine or tributylphosphine, and benzyl alcohol optionally substituted with R^{C} or heteroaryl methyl alcohol that is commercially available or prepared by a known method are added, and the mixture is reacted at -20°C to 100°C, and preferably at 0°C to 30°C, for 0.5 hours to 24 hours, and preferably 1 hour to 5 hours, whereby Compound C-1 can be obtained.

### First Step

In a solvent such as DMF or DMA, a base such as potassium carbonate, sodium carbonate or cesium carbonate, a palladium catalyst such as Pd(PPh₃)₄, Pd(OAc)₂, Pd(PPh₃)₂Cl₂, Pd(dppf)₂Cl₂ or Pd(dtbpf), and a ligand such as tricyclohexylphosphine, triphenylphosphine are added to Compound c1 that is commercially available or prepared by a known method, and the mixture is reacted at 0°C to 150°C, and preferably at 80°C to 130°C, for 1 hour to 24 hours, and preferably 6 hours to 12 hours, whereby Compound C-1 can be obtained.

### Second Step

Compound C-2 can be obtained from Compound C-1 in the same manner as described above in the fourth step in "1) Synthesis of Compounds A-1 and A-2".

### 4) Synthesis of Compounds D-1 and D-2

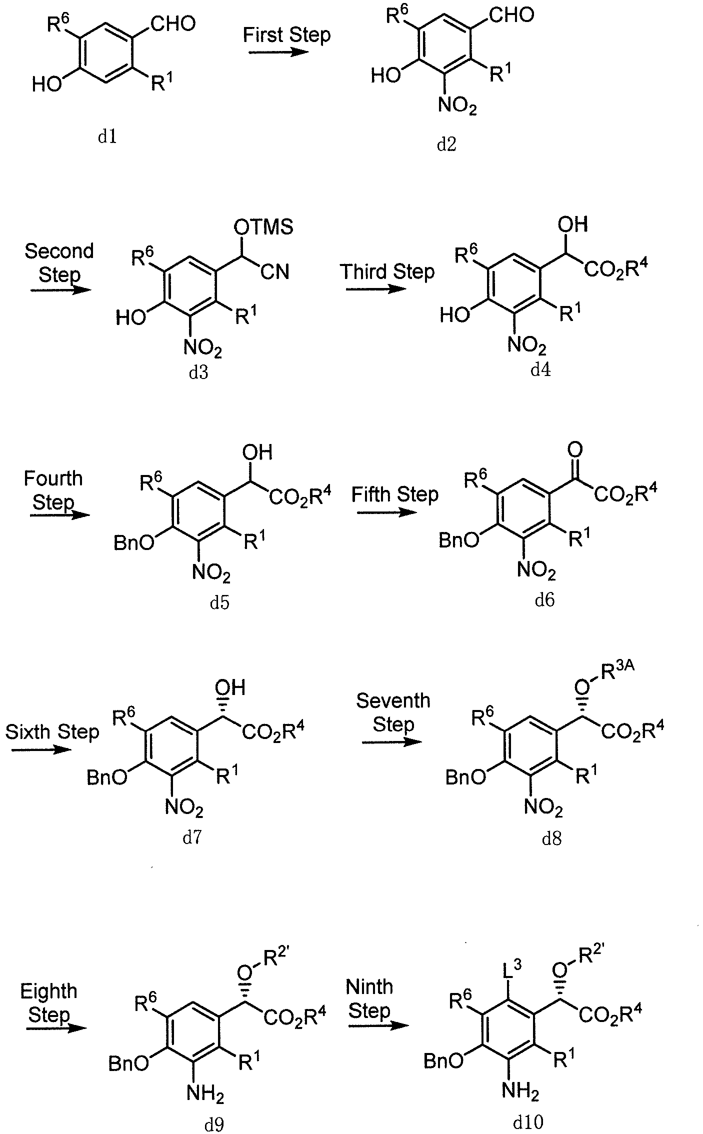

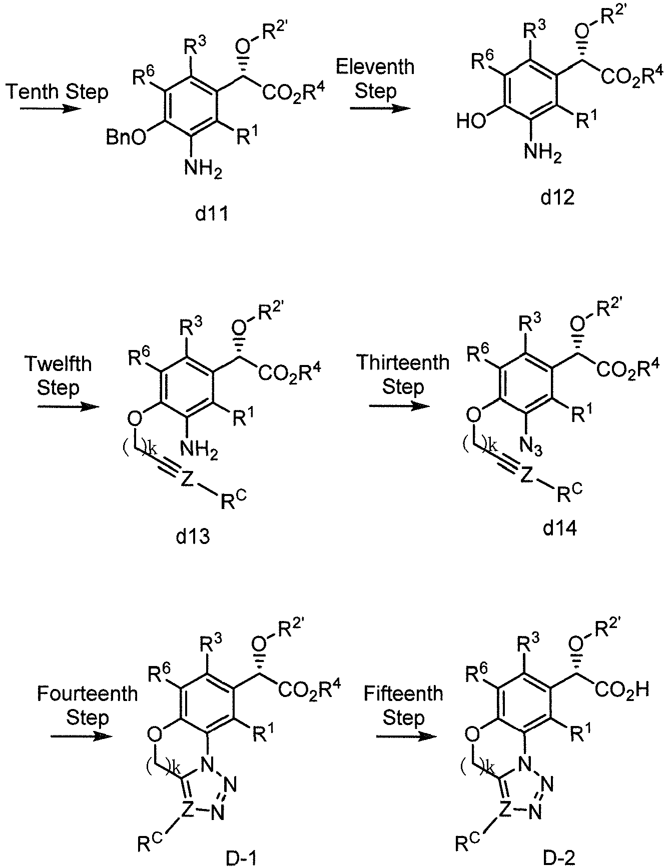

wherein R¹, R^{2'}, R³, R⁴, R⁶ and R^{C} have the same meaning as described above; L³ is leaving group such as halogen; Z is carbon atom or nitrogen atom; k is any integer of 1 to 5.

### First Step

To a solvent such as concentrated sulfuric acid or acetic acid of Compound d1 that is commercially available or prepared by a known method is added nitric acid, fuming nitric acid or the like under ice-cooling, and the mixture is reacted at -20°C to 60°C, and preferably at 0°C to 25°C, for 0.5 hours to 6 hours, and preferably 1 hour to 3 hours, whereby Compound d2 can be obtained. Also, Compound d2 can be obtained by adding a metal salt such as potassium nitrate or sodium nitrate under ice-cooling in concentrated sulfuric acid, and reacting the mixture at -20°C to 60°C, and preferably at 0°C to 25°C, for 0.5 hours to 6 hours, and preferably 1 hour to 3 hours.

### Second Step

In a solvent such as dichloromethane, dichloroethane or toluene, zinc iodide and TMSCN are added to Compound d2, and the mixture is reacted at -20°C to 50°C, and preferably at 0°C to 30°C, for 0.1 hours to 10 hours, and preferably 0.5 hours to 2 hours, whereby Compound d3 can be obtained. Also, Compound d3 can be obtained by reacting a metal salt such as zinc iodide, TMSCI, sodium cyanide or potassium cyanide in a solvent such as acetonitrile and DMF at -20°C to 50°C, and preferably at 0°C to 30°C, for 0.1 hours to 10 hours, and preferably 0.5 hours to 2 hours.

### Third Step

In R⁴OH, an acid such as concentrated sulfuric acid or concentrated hydrochloric acid is added to Compound d3, and the mixture is reacted at 0°C to 150°C, and preferably at 80°C to 110°C, for 1 hour to 24 hours, and preferably 6 hours to 12 hours, for deprotection of a TMS group, hydrolysis to the carboxylic acid of nitrile group, followed by esterification, whereby Compound d4 can be obtained.

### Fourth Step

In a solvent such as DMF, DME, tetrahydrofuran, acetone or acetonitrile, a base such as potassium carbonate, sodium carbonate, cesium carbonate or sodium hydride, and benzyl bromide or benzyl chloride are added to Compound d4, and the mixture is reacted at 0°C to 100°C, and preferably at 20°C to 50°C, for 0.5 hours to 24 hours, and preferably 1 hour to 5 hours, whereby Compound d5 can be obtained. Also, Compound d5 can be obtained by adding diethyl azodicarboxylate or diisopropyl azodicarboxylate, and benzyl alcohol in a solvent such as tetrahydrofuran, toluene or dichloromethane, and reacting the mixture at 0°C to 100°C, and preferably at 20°C to 50°C, for 0.5 hours to 24 hours, and preferably 1 hour to 5 hours.

### Fifth Step

In a solvent such as dichloromethane, dichloroethane or chloroform, an oxidizing agent such as a Dess-Martin reagent, manganese dioxide or pyridinium chlorochromate is added to Compound d5, and the mixture is reacted at 0°C to 80°C, and preferably at 20°C to 45°C, for 0.5 hours to 5 hours, and preferably 1 hour to 3 hours, whereby Compound d6 can be obtained. Also, Compound d6 can be obtained by general Swern oxidation.

### Sixth Step

In a solvent such as benzene, toluene, xylene, dichloromethane or dichloroethane, a (R)-CBS reagent is added as an asymmetric source, and a reducing agent such as catechol borane or 9-borabicyclo[3.3.1]nonane is sequentially added to Compound d6, and the mixture is reacted at -100°C to 0°C, and preferably at -78°C to -50°C, for 0.5 hours to 6 hours, and preferably 1 hour to 3 hours, whereby Compound d7 can be obtained.

### Seventh Step

In a solvent such as tetrahydrofuran, DMF or toluene, a base such as sodium hydride, potassium tert-butoxide or sodium methoxide and R^{2'}-I, R^{2'}-Br, R^{2'}-Cl or the like that is commercially available or prepared by a known method are added to Compound d7, and the mixture is reacted at -20°C to 100°C, and preferably at 0°C to 60°C, for 1 hour to 24 hours, and preferably 3 hours to 12 hours, whereby Compound d8 can be obtained.

Also, tert-butyl ester and the like can be also obtained by adding 1 to 3 equivalents of a 70% aqueous perchloric acid solution in tert-butyl acetate, and reacting the mixture at 0°C to 60°C, and preferably at 15°C to 30°C, for 0.1 hours to 10 hours, and preferably 0.5 hours to 2 hours.

### Eighth Step

In a solvent such as methanol, ethanol, THF or ethyl acetate, a catalyst such as 5% or 10% palladium carbon, palladium hydroxide or platinum dioxide is added to Compound d8, and the mixture is reacted under a hydrogen atmosphere at 0°C to 50°C, and preferably at 15°C to 25°C, for 0.1 hours to 48 hours, and preferably 1 hour to 24 hours, whereby Compound d9 can be obtained.

In this condition, the reaction may be promoted by adding acetic acid, hydrochloric acid or the like. In a mixed solvent of an organic solvent such as methanol, ethanol or THF and water, a metal such as iron, zinc or tin is added to Compound d8, under acidic conditions of hydrochloric acid or acetic acid, under alkaline conditions of potassium hydroxide or sodium hydroxide, or under neutral conditions of ammonium chloride, and the mixture is reacted at 0°C to 120°C, and preferably at 25°C to 80°C, for 0.1 hours to 24 hours, and preferably 1 hour to 12 hours, whereby Compound d9 can be also obtained.

### Ninth Step

In a solvent such as dichloromethane, THF, toluene, acetonitrile or DMF, bromine or a halogenating reagent such as NBS, NCS and NIS is added to Compound d9, and when L³ is bromo, the mixture is reacted at -30°C to 50°C, and preferably at - 10°C to 20°C, for 0.1 hours to 10 hours, and preferably 0.5 hours to 2 hours, whereby Compound d10 can be obtained. When L³ is chloro or iodine, the mixture is reacted at 10°C to 150°C, and preferably at 60°C to 120°C, for 0.5 hours to 24 hours, and preferably 1 hour to 6 hours, whereby Compound d10 can be obtained.

### Tenth Step

From Compound d10, Compound d11 can be obtained in the same manner as in the fifth step in "2) Synthesis of Compounds B-1 and B-2" described above.

When the cyclization does not progress, in a solvent such as dioxane, DMF, DME, tetrahydrofuran or ethyl acetate, or in a mixed solvent, an acid such as acetic acid or a base such as cesium carbonate is added to the reaction mixture, and the mixture is reacted at 0°C to 150°C, and preferably at 20°C to 100°C, for 0.5 hours to 24 hours, and preferably 1 hour to 12 hours, whereby Compound d11 can be obtained.

### Eleventh Step

In a solvent such as methanol, ethanol or THF, a catalyst such as 5% or 10% palladium carbon, palladium hydroxide or platinum dioxide is added to Compound d11, and the mixture is reacted under a hydrogen atmosphere at 0°C to 50°C, and preferably at 20°C to 40°C, for 0.1 hours to 24 hours, and preferably 1 hour to 12 hours, whereby Compound d12 can be obtained.

### Twelfth Step

In a solvent such as DMF, DME, THF, acetone or acetonitrile, a base such as potassium carbonate, sodium carbonate, cesium carbonate or sodium hydride, and an alkylating agent that is commercially available or prepared by a known method are added to Compound d12, and the mixture is reacted at 0°C to 100°C, and preferably at 20°C to 50°C, for 0.5 hours to 24 hours, and preferably 1 hour to 5 hours, whereby Compound d13 can be obtained.

### Thirteenth Step

In a solvent such as acetonitrile, THF or DMF, a diazotization reagent such as tert-butyl nitrite or isopentyl nitrite is added to Compound d13, and then trimethylsilyl azide is sequentially added, and the mixture is reacted at 0°C to 80°C, and preferably at 20°C to 50°C, for 0.1 hours to 6 hours, and preferably 0.5 hours to 3 hours, whereby Compound d14 can be obtained.

### Fourteenth Step

In a solvent such as toluene, acetonitrile or DMF, Compound d14 is reacted at 0°C to 110°C, and preferably at 20°C to 80°C, for 0.1 hours to 24 hours, and preferably 0.5 hours to 6 hours, whereby Compound d15 can be obtained.

Also, in a solvent such as water or phosphate buffer, a copper salt such as copper iodide or copper chloride is added to Compound d14, and the mixture is reacted at 0°C to 110°C, and preferably at 20°C to 80°C, for 0.1 hours to 24 hours, and preferably 0.5 hours to 6 hours, whereby Compound d15 can be obtained.

### Fifteenth Step

Compound D-2 can be obtained from Compound D-1 in the same manner as described above in the fourth step in "1) Synthesis of Compounds A-1 and A-2".

### 5) Synthesis of Compounds E-1 and E-2

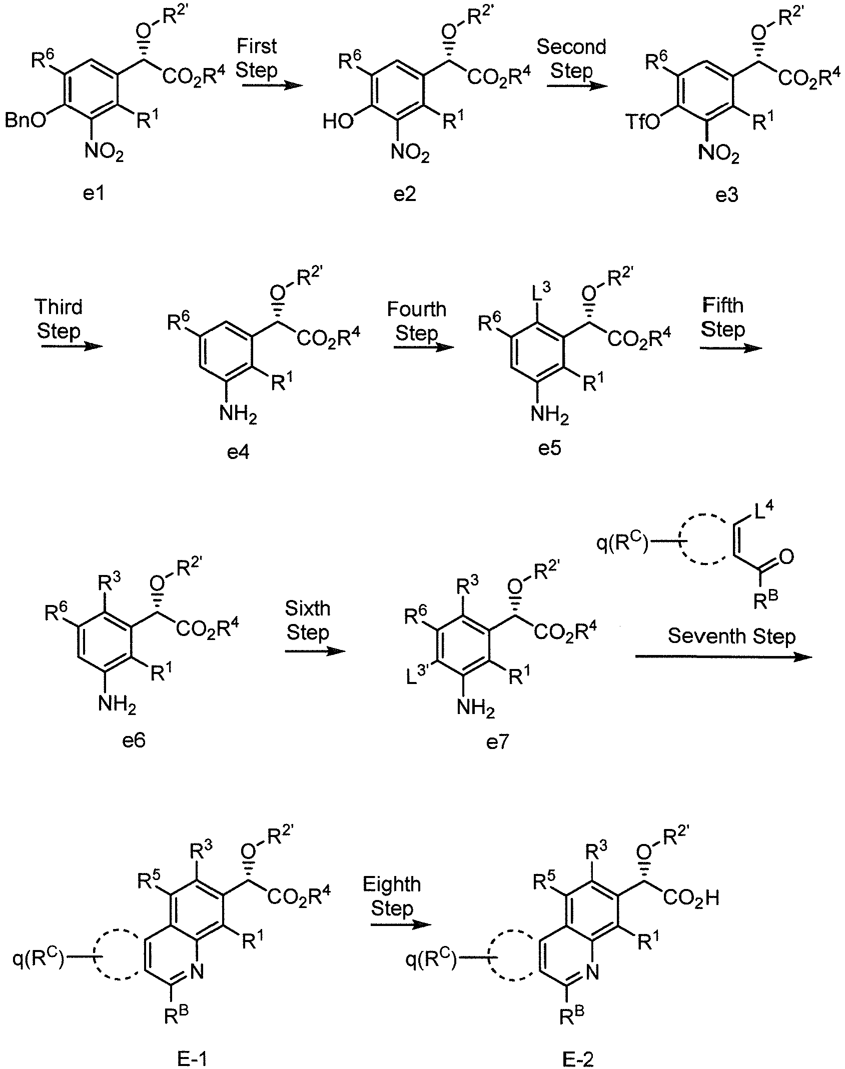

wherein R¹, R^{2'}, R³, R⁴, R⁶, R^{B}, R^{C} and L³ have the same meaning as described above; L⁴ is leaving group such as halogen; L^{3'} is leaving group such as halogen; dashlined portion is a ring C.

### First Step

Compound e2 can be obtained from Compound e1 in the same manner as described above in the eleventh step in "4) Synthesis of Compounds D-1 and D-2".

### Second Step

In a solvent such as dichloromethane, dichloroethane or THF, or in a mixed solvent thereof, a base such as pyridine, lutidine or triethylamine, and a trifluoromethanesulfonylating reagent such as trifluoromethanesulfonyl chloride, trifluoromethanesulfonic anhydride or N-phenylbistrifluoromethanesulfonimide or a nonaflating reagent such as nonafluorobutanesulfonyl chloride or nonafluorobutanesulfonic anhydride are added to Compound e2, and the mixture is reacted at -50°C to 50°C, and preferably at -30°C to 30°C, for 0.1 hours to 4 hours, and preferably 0.5 hours to 1 hour, whereby Compound e3 can be obtained.

### Third Step

In a solvent such as methanol, ethanol, ethyl acetate or tetrahydrofuran, a base such as triethylamine, N-methylmorpholine or DIEA and a catalyst such as 5% or 10% palladium carbon, palladium hydroxide or platinum dioxide are added to Compound e3, and the mixture is reacted under a hydrogen atmosphere and under 1 to 10 atmospheres, and preferably 2 to 5 atmospheres, at 0°C to 60°C, and preferably at 20°C to 40°C, for 0.1 hours to 24 hours, and preferably 1 hour to 12 hours, whereby Compound e4 can be obtained.

Also, Compound e4 can be also obtained by adding formic acid, a base such as triethylamine or tributylamine, a ligand such as triphenylphosphine, dppf or dppp, and a palladium catalyst such as Pd(OAc)₂, Pd(PPh₃)₄ or bistriphenylphosphine palladium dichloride to Compound e3, in a solvent such as toluene, DMF or dioxane, and reacting the mixture at 20°C to 200°C, and preferably at 60°C to 120°C, for 0.1 hours to 24 hours, and preferably 1 hour to 12 hours.

### Fourth Step

Compound e5 can be obtained from Compound e4 in the same manner as described above in the ninth step in "4) Synthesis of Compounds D-1 and D-2".

### Fifth Step

Compound e6 can be obtained from Compound e5 in the same manner as described above in the tenth step in "4) Synthesis of Compounds D-1 and D-2".

### Sixth Step

Compound e7 can be obtained from Compound e6 in the same manner as described above in the ninth step in "4) Synthesis of Compounds D-1 and D-2" described above.

### Seventh Step

Compound E-1 can be obtained from Compound e7 in the same manner as described above in the tenth step in "4) Synthesis of Compounds D-1 and D-2".

### Eighth Step

Compound E-2 can be obtained from Compound E-1 in the same manner as described above in the fourth step in "1) Synthesis of Compounds A-1 and A-2".

### 6) Synthesis of Compounds F-1 and F-2

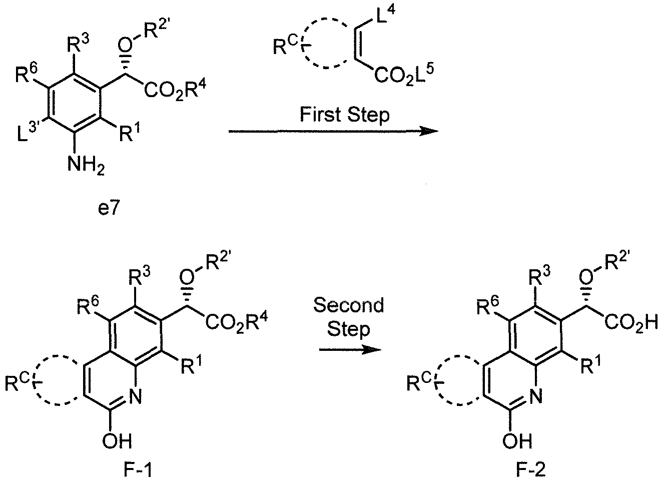

wherein R¹, R^{2'}, R³, R⁴, R⁶, R^{C}, L^{3'} and L⁴ have the same meaning as described above; L⁵ is substituted or unsubstituted alkyl; dashlined portion is a ring C; R^{C} is preferably be present 1 to 4 (the same shall apply heresfter).

### First Step

Compound F-1 can be obtained from Compound e7 in the same manner as described above in the tenth step in "4) Synthesis of Compounds D-1 and D-2".

### Second Step

Compound F-2 can be obtained from Compound F-1 in the same manner as described above in the fourth step in "1) Synthesis of Compounds A-1 and A-2".

### 7) Synthesis of Compounds G-1 and G-2

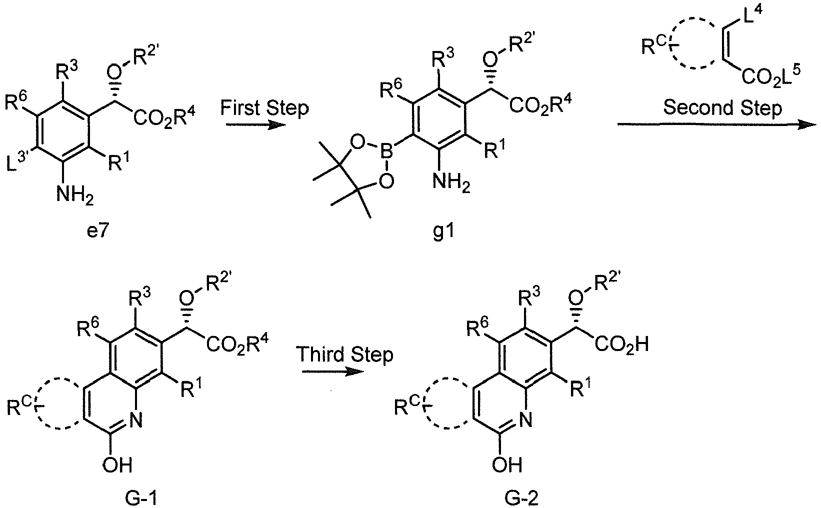

wherein each symbol has the same meaning as described above; dashlined portion is a ring C.

### First Step

In a solvent such as dioxane, DMF, DME, tetrahydrofuran or DMSO, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium acetate, sodium acetate, potassium carbonate or potassium phosphate, and bispinacalato diboron are added to Compound e7, and the mixture is reacted under a nitrogen atmosphere at 0°C to 150°C, and preferably at 60°C to 120°C, for 0.5 hours to 24 hours, and preferably 1 hour to 12 hours, whereby Compound g1 can be obtained.

### Second Step

In a solvent such as dioxane, DMF, DME, tetrahydrofuran or water, or in a mixed solvent, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and a halide or trifluoromethanesulfonic acid ester that is commercially available or prepared by a known method are added to Compound g1, and the mixture is reacted under a nitrogen atmosphere at 0°C to 150°C, and preferably at 60°C to 120°C, for 0.5 hours to 24 hours, and preferably 1 hour to 12 hours, whereby Compound G-1 can be obtained. When the cyclization does not progress, in a solvent such as dioxane, DMF, DME, tetrahydrofuran or ethyl acetate, or in a mixed solvent, an acid such as acetic acid or a base such as cesium carbonate is added to the reaction mixture, and the mixture is reacted at 0°C to 150°C, and preferably at 20°C to 100°C, for 0.5 hours to 24 hours, and preferably 1 hour to 12 hours, whereby Compound G-1 can be obtained.

### Third Step

Compound G-2 can be obtained from Compound G-1 in the same manner as described above in the fourth step in "1) Synthesis of Compounds A-1 and A-2".

### 8) Synthesis of Compounds H-1 and H-2

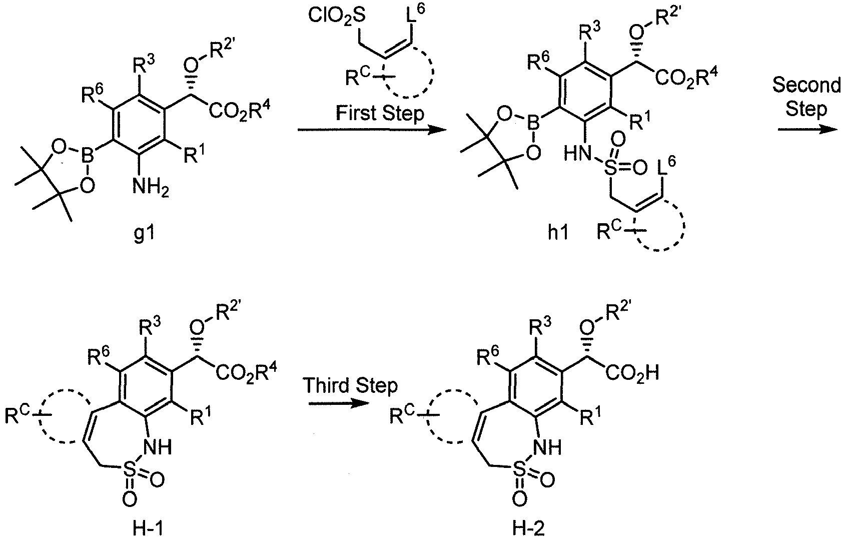

wherein R¹, R^{2'}, R³, R⁴, R⁶ and R^{C} have the same meaning as described above; L⁶ is leaving group such as halogen, substituted or unsubstituted alkylsulfonyloxy; dashlined portion is a ring C.

### First Step

In a solvent such as pyridine or lutidine, a substituted sulfonyl chloride that is commercially available or synthesized by a known method is added to Compound g1, and the mixture is reacted at 20°C to 100°C, and preferably at 50°C to 100°C, for 1 hour to 24 hours, and preferably 2 hours to 5 hours, whereby Compound h1 can be obtained.

### Second Step

In a solvent such as dioxane, DMF, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), and a base such as potassium carbonate, sodium carbonate, cesium carbonate or potassium phosphate are added to Compound h1, and the mixture is reacted under a nitrogen atmosphere at 0°C to 150°C, and preferably at 60°C to 120°C, for 0.5 hours to 24 hours, and preferably 1 hour to 12 hours, whereby Compound H-1 can be obtained.

### Third Step

Compound H-2 can be obtained from Compound H-1 in the same manner as described above in the fourth step in "1) Synthesis of Compounds A-1 and A-2".

### 9) Synthesis of Compound I-1

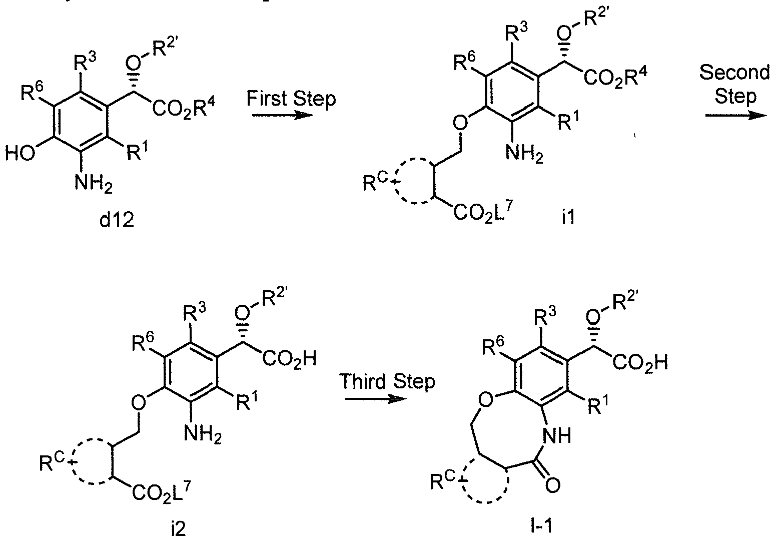

wherein R¹, R^{2'}, R³, R⁴, R⁶ and R^{C} have the same meaning as described above; L⁷ is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl or substituted or unsubstituted non-aromatic heterocyclyl; dashlined portion is a ring C.

### First Step

Compound i1 can be obtained from Compound d12 in the same manner as described above in the twelfth step in "4) Synthesis of Compounds D-1 and D-2".

### Second Step

Compound i2 can be obtained from Compound i1 in the same manner as described above in the fifteenth step in "4) Synthesis of Compounds D-1 and D-2".

### Third Step

In a soluvent such as dioxane, DMF, DME or THF, or in a mixed solvent thereof, a condensing agent such as N,N'-dicyclohexylcarbodiimide or N,N'-diisopropylcarbodiimide, a condensation accelerator such as 1-hydroxyazabenzotriazole or 1-hydroxybenzotriazole, and a base such as triethylamine or N,N-diisopropylethylamine are added to Compound i2, and the mixture is reacted at 0°C to 150°C, and preferably at 20°C to 60°C, for 15 hours to 72 hours, and preferably 12 hours to 24 hours, whereby Compound I-1 can be obtained.

### 10) Synthesis of Compounds J-1 and J-2

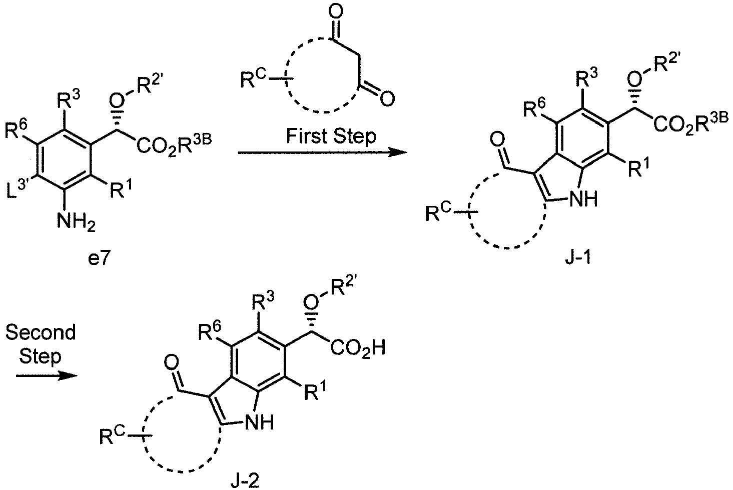

wherein each symbol has the same meaning as described above; dashlined portion is a ring C.

### First Step

In a solvent such as toluene, xylene or dichloromethane, or in a mixed soluvent thereof, 1,3-diketone and an acid such as acetic acid or para-toluene sulfonic acid are added to Compound e7, and the mixture is reacted at 0°C to 150°C, and preferably at 60°C to 120°C, for 30 minutes to 24 hours, and preferably 1 hour to 2 hours, whereby imine can be obtained. In a solvent such as dioxane, DMF or DMA, or in a mixed soluvent thereof, a catalyst such as Pd(OAc)₂, a ligand such as 1,3-bis(diphenylphosphino)propane, a base such as sodium acetate or potassium acetate, and such as tetraethyl ammonium chloride are added to the imine, and the mixture is reacted at 50°C to 150°C, and preferably at 80°C to 120°C, for 1 hour to 24 hours, and preferably 2 hours to 4 hours, whereby Compound J-1 can be obtained.

### Second Step

Compound J-2 can be obtained from Compound J-1 in the same manner as described above in the fourth step in "1) Synthesis of Compounds A-1 and A-2".

### 11) Synthesis of Compounds K-1 and K-2

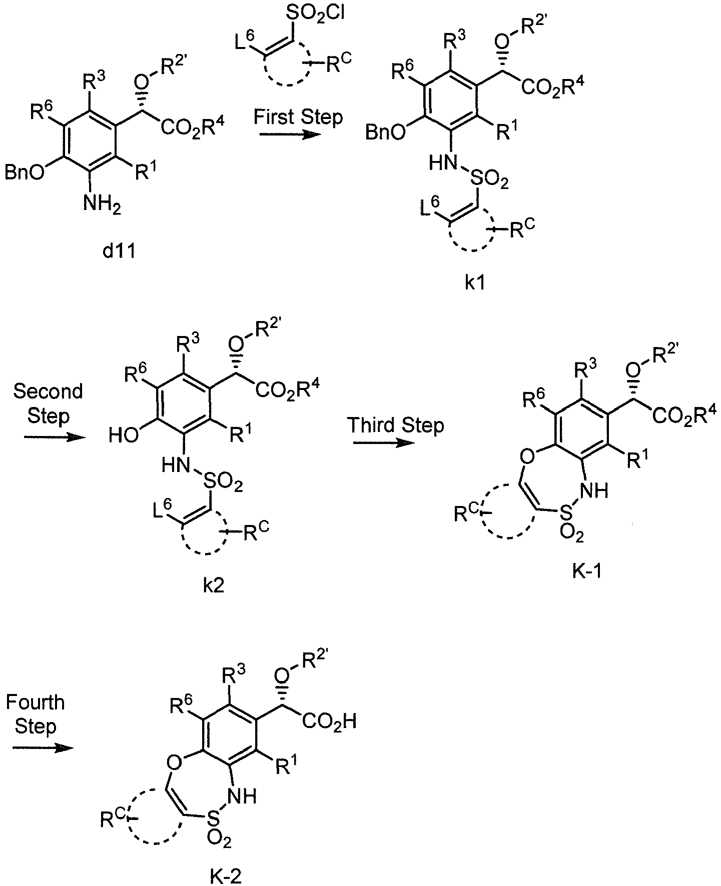

wherein each symbol has the same meaning as described above; dashlined portion is a ring C.

### First Step

Compound k1 can be obtained from Compound d11 in the same manner as described above in the first step in "8) Synthesis of Compounds H-1 and H-2".

### Second Step

Compound k2 can be obtained from Compound k1 in the same manner as described above in the eleventh step in "4) Synthesis of Compounds D-1 and D-2".

### Third Step

In a solvent such as DMSO, DMF or DMA, or in a mixed solvent thereof, a base such as sodium hydride sodium hydroxide or tert-butoxypotassium is added to Compound k2, and the mixture is reacted at 0°C to 150°C, and preferably at 60°C to 120°C, for 0.5 hours to 24 hours, and preferably 1 hour to 6 hours, whereby Compound K-1 can be obtained.

Also, in a solvent such as toluene, dioxane or DMF, or in a mixed solvent thereof, a palladium catalyst such as tris(dibenzylideneacetone)dipalladium, Pd(OAc)₂ or Pd(PPh₃)₄, a ligand such as tris ortho-tolyl phosphine, tri-tert-butyl phosphine or tris-2-furyl phosphine, and a base such as triethylamine, cesium carbonate or sodium tert-butoxide are added to Compound k2, and the mixture is reacted at 20°C to 180°C, and preferably at 80°C to 160°C, for 30 minutes to 48 hours, and preferably 1 hour to 24 hours, whereby Compound K-1 can be obtained.

Also, in a solvent such as DMSO, DMF or dioxane, or in a mixed solvent thereof, a catalyst such as copper iodide, a ligand such as L-proline, and a base such as sodium carbonate, potassium phosphate or triethylamine are added to Compound k2, and the mixture is reacted at 20°C to 180°C, and preferably at 60°C to 120°C, for 1 hour to 96 hours, and preferably 24 hours to 72 hours, whereby Compound K-1 can be obtained.

### Fourth Step

Compound K-2 can be obtained from Compound K-1 in the same manner as described above in the fourth step in "1) Synthesis of Compounds A-1 and A-2".

### 12) Synthesis of Compounds L-1 and L-2

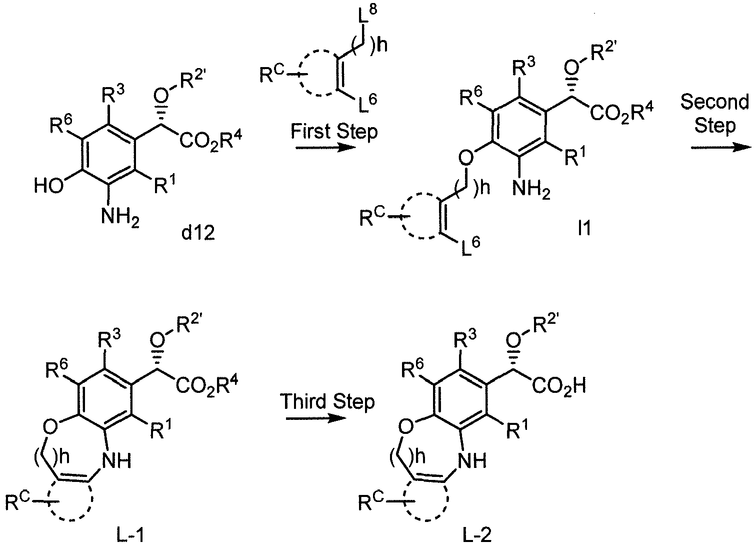

wherein R¹, R^{2'}, R³, R⁴, R⁶, L6 and R^{C} have the same meaning as described above; L⁸ is halogen or substituted or unsubstituted alkylsulfonyloxy;h is any integer of 1 to 4; dashlined portion is a ring C.

### First Step

Compound 11 can be obtained from Compound d12 in the same manner as described above in the twelfth step in "4) Synthesis of Compounds D-1 and D-2".

### Second Step

Compound L-1 can be obtained from Compound 11 in the same manner as described above in the third step in "11) Synthesis of Compounds K-1 and K-2".

### Third Step

Compound L-2 can be obtained from Compound L-1 in the same manner as described above in the fourth step in "1) Synthesis of Compounds A-1 and A-2".

### 13) Synthesis of Compounds M-1 and M-2

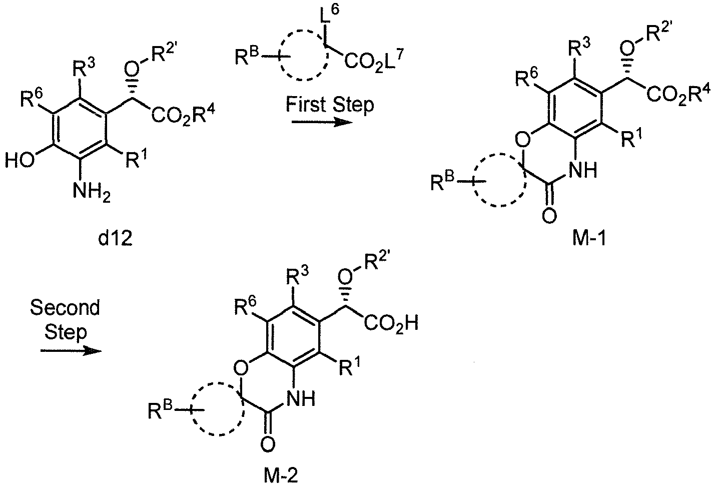

wherein each symbol has the same meaning as described above; dashlined portion is a ring E (wherein a ring E is a spiro ring).

### First Step

Compound M-1 can be obtained from Compound d12 in the same manner as described above in the twelfth step in "4) Synthesis of Compounds D-1 and D-2".

### Second Step

Compound M-2 can be obtained from Compound M-1 in the same manner described above as in the fourth step in "1) Synthesis of Compounds A-1 and A-2".

### 14) Synthesis of Compounds N-1 to N-3

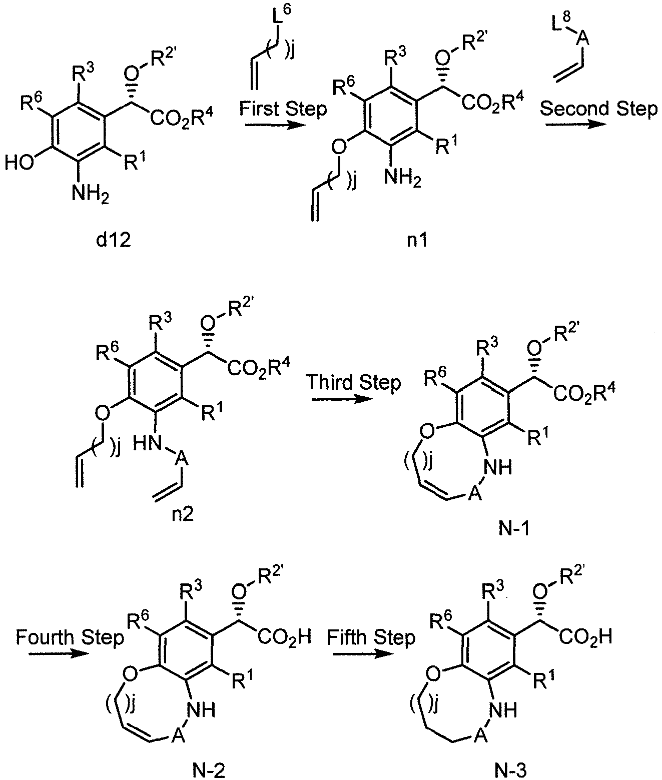

wherein A is a single bond, -SO₂-, -CO-, -O-CO-, -NH-CO-, or alkylene; j is any integer of 1 to 3; the others have the same meaning as described above.

### First Step

Compound n1 can be obtained from Compound d12 in the same manner as described above in the twelfth step in "4) Synthesis of Compounds D-1 and D-2".

### Second Step

In a solvent such as DMF, THF or dioxane, a base such as sodium hydride or triethylamine, and an alkylating agent such as an allyl bromide, an acylating agent such as acryloyl chloride, a sulfonylating agent such as 2-chloroethyl sulfonyl chloride, a urea agent such as allyl isocyanate or a carbamate agent such as allyloxycarbonyl chloride are added to Compound n1, and the mixture is reacted at - 20°C to 50°C, and preferably at 0°C to 30°C, for 0.1 hours to 10 hours, and preferably 0.5 hours to 2 hours, whereby Compound n2 can be obtained.

### Third Step

In a solvent such as dichloromethane or dichloroethane, or in a mixed solvent thereof, Grubbs catalyst is added to Compound n2, and the mixture is reacted at 0°C to 50°C, and preferably at 15°C to 25°C, for 0.1 hours to 48 hours, and preferably 0.1 hours to 24 hours, whereby Compound N-1 can be obtained.

### Fourth Step

Compound N-2 can be obtained from Compound N-1 in the same manner as described above in the fourth step in "1) Synthesis of Compounds A-1 and A-2".

### Fifth Step

In a solvent such as methanol, ethanol, THF or ethyl acetate, a catalyst such as 5% or 10% palladium carbon, palladium hydroxide or platinum dioxide is added to Compound N-2, and the mixture is reacted under a hydrogen atmosphere at 0°C to 50°C, and preferably at 15°C to 25°C, for 0.1 hours to 48 hours, and preferably 1 hour to 24 hours, whereby Compound N-3 can be obtained.

### 15) Synthesis of Compounds O-1 and O-2

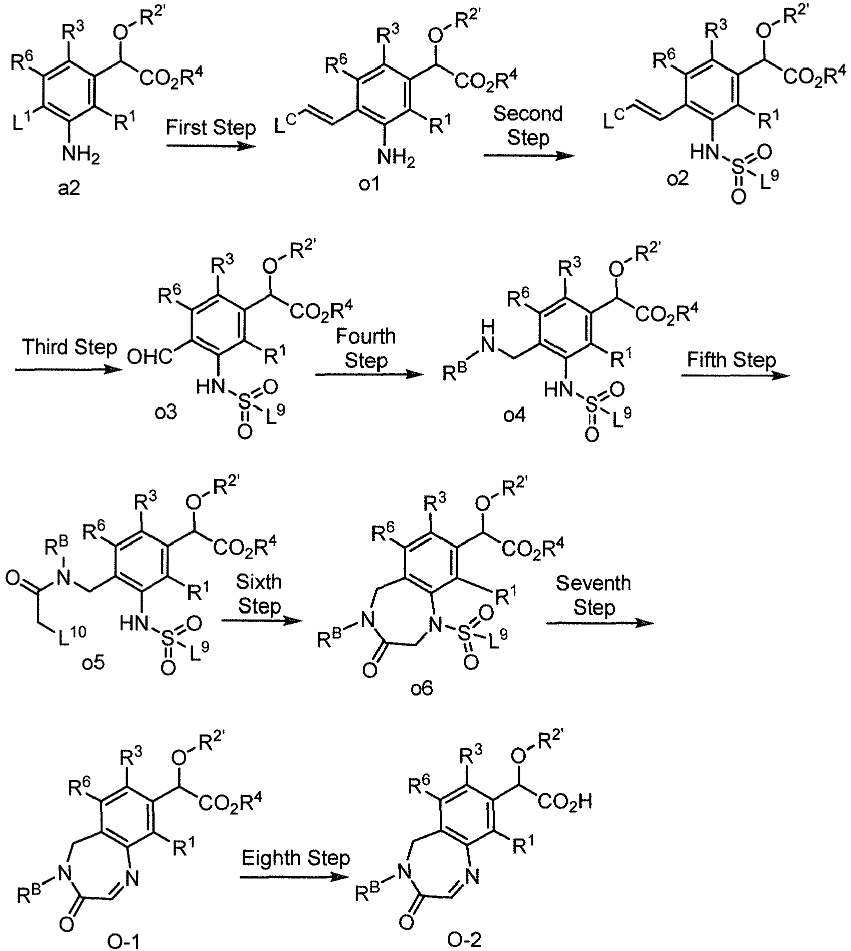

wherein R¹, R^{2'}, R³, R⁴, R⁶, L¹, L^{C} and R^{B} have the same meaning as described above; L⁹ is alkyl or substituted or unsubstituted phenyl; L¹⁰ is halogen.

### First Step

Compound (1-2) can be obtained by a coupling reaction of Compound a2 with Z-L^{C}. As the reaction, Suzuki cross-coupling, Ullmann cross-coupling, Negishi cross-coupling, Stille coupling, and the like are exemplified. As Z, a boronic acid, a boronic acid ester, an alkyltin, a zinc halide and the like are exemplified. In a solvent such as dioxane, DMF, DMA, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and a vinylboronic acid substituted with L^{C}, a vinylboronic acid ester substituted with L^{C}, an alkylvinyltin substituted with L^{C} or a vinylzinc halide substituted with L^{C} that is commercially available or prepared by a known method are added to Compound (1-1), and the mixture is reacted under a nitrogen atmosphere at 0°C to 180°C, and preferably at 60°C to 120°C, for 0.5 hours to 24 hours, and preferably 1 hour to 12 hours, whereby Compound o1 can be obtained.

### Second Step

In a solvent such as pyridine, lutidine or collidine, L⁹-SO₂Cl or (L⁹-SO₂)₂ that is commercially available or synthesized by a known method is added to Compound o1, and the mixture is reacted at 0°C to 120°C, and preferably at 20°C to 60°C, for 0.1 hours to 48 hours, and preferably 1 hour to 24 hours, whereby Compound o2 can be obtained.

### Third Step

In a solvent such as dichloromethane, chloroform or methanol, or in a mixed solvent thereof, pyridine is added to Compound o2, and an ozone gas is passed through the mixture at -100°C to 0°C, and preferably at -78°C to 30°C, for 0.1 hours to 5 hours, and preferably 0.5 hours to 2 hours, and thereafter dimethylsulfide, trimethylphosphite, or the like is added, and the mixture is stirred at -20°C to 60°C, and preferably at 0°C to 20°C for 0.1 hours to 5 hours, and preferably 0.5 hours to 2 hours, whereby Compound o3 can be obtained.

Also, in a mixed solvent of diethyl ether, THF, acetone or the like and water, a catalytic amount of osmium tetroxide or dipotassium osmate dehydrate, and an oxidizing agent such as N-methylmorpholine oxide or N-trimethylamine oxide are added to Compound o2, and the mixture is reacted at -20°C to 60°C, and preferably at 0°C to 20°C, for 0.5 hours to 24 hours, and preferably 2 hours to 12 hours, whereby diol compound can be obtained. Thereafter in the same solvent as above, sodium periodate is added to diol compound, the mixture is stirred at -20°C to 60°C, and preferably at 0°C to 20°C for 0.5 hours to 24 hours, and preferably 2 hours to 12 hours, whereby Compound o3 can be obtained.

### Fourth Step

In a solvent such as dichloromethane, dichloroethane, THF, methanol or ethanol, acetic acid, TFA or the like, and an amine that is commercially available or synthesized by a known method, are added to Compound o3, and the mixture is reacted at 0°C to 150°C, and preferably at 20°C to 60°C, for 0.1 hours to 24 hours, and preferably 1 hour to 6 hours, whereby a imine can be obtained. The obtained imine is not isolated. To the reaction mixture, a reducing agent such as sodium cyanoborohydride or sodium triacetoxyborohydride is added, and the mixture is reacted at -30°C to 50°C, and preferably at 0°C to 20°C, for 0.1 hours to 24 hours, and preferably 0.5 hours to 12 hours, whereby Compound o4 can be obtained.

### Fifth Step

In a solvent such as dichloromethane, dichloroethane, THF, methanol or ethanol, a bace such as pyridine, triethylamine or N-methylmorppholine, and a halogenating reagent such as acetyl chloride, are added to Compound o4, and the mixture is reacted at -50°C to 80°C, and preferably at -10°C to 20°C, for 0.1 hours to 12 hours, and preferably 0.5 hours to 3 hours, whereby Compound o5 can be obtained.

### Sixth Step

In a solvent such as DMF, DMA, THF, toluene or dioxane, a bace such as potassium carbonate, sodium carbonate or cesium carbonate is added to Compound o5, and the mixture is reacted at 0°C to 120°C, and preferably at 40°C to 80°C, for 0.1 hours to 6 hours, and preferably 0.5 hours to 2 hours, whereby Compound o6 can be obtained.

### Seventh Step

In a solvent such as methanol, ethanol, THF or DMSO, or in a mixed solvent thereof, a base such as potassium hydroxide, sodium hydroxide or lithium hydroxide is added to Compound o6, and the mixture is reacted at 0°C to 150°C, and preferably at 20°C to 100°C, for 0.1 hours to 48 hours, and preferably 1 hour to 24 hours, whereby Compound O-1 can be obtained.

### Eighth Step

Compound O-2 can be obtained from Compound O-1 in the same manner as described above in the fourth step in "1) Synthesis of Compounds A-1 and A-2".

### 16) Synthesis of Compounds P-1 and P-2

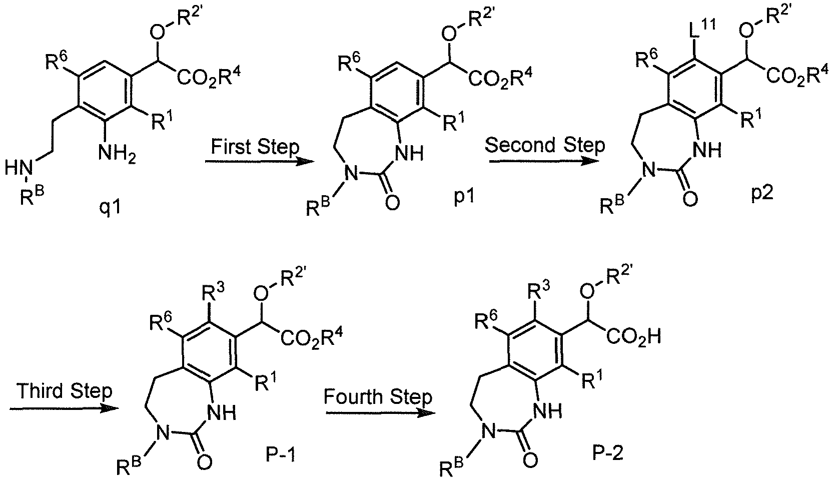

wherein R¹, R^{2'}, R³, R⁴, R⁶ and R^{B} have the same meaning as described above; L¹¹ is leaving group such as halogen.

### First Step

In a solvent such as dichloromethane, toluene, xylene, dioxane, DMF or DMA, a base such as N,N-diisopropylethylamine, 2,6-lutidine or diazabicycloundecene, and carbonyldiimidazole, triphosgene, or the like are added to Compound q1 that can be obtained in the same manner as described below "17) Synthesis of Compounds Q-1 and Q-2", and the mixture is reacted at 20°C to 180°C, and preferably at 80°C to 120°C, for 0.5 hours to 24 hours, and preferably 4 hours to 12 hours, whereby Compound p1 can be obtained.

### Second Step

In a solvent such as dichloromethane, THF, toluene, acetonitrile or DMF, bromine or a halogenating reagent such as NBS, NCS and NIS is added to Compound p1, and when L¹¹ is bromo, the mixture is reacted at -30°C to 50°C, and preferably at -10°C to 20°C, for 0.1 hours to 10 hours, and preferably 0.5 hours to 2 hours, whereby Compound p2 can be obtained. When L¹¹ is chloro or iodine, the mixture is reacted at 10°C to 150°C, and preferably at 60°C to 120°C, for 0.5 hours to 24 hours, and preferably 1 hour to 6 hours, whereby Compound p2 can be obtained.

### Third Step

Compound p3 can be obtained by a coupling reaction of Compound p2 with Z-R³. As the reaction, Suzuki cross-coupling, Ullmann cross-coupling, Negishi cross-coupling, Stille coupling, and the like are exemplified. As Z, a boronic acid, a boronic acid ester, an alkyltin, a zinc halide and the like are exemplified.

In a solvent such as dioxane, DMF, DME, tetrahydrofuran or water, or in a mixed solvent thereof, a palladium catalyst such as Pd(PPh₃)₄, Pd(OAc)₂, Pd(PPhg)₂Cl₂, Pd(dppf)₂Cl₂ or Pd(dtbpf), a base such as potassium carbonate, sodium carbonate, cesium carbonate or potassium phosphate, and a boronic acid, a boronic acid ester, an alkyltin or a zinc halide that is commercially available or prepared by a known method are added to Compound p2, and the mixture is reacted under a nitrogen atmosphere at 0°C to 180°C, and preferably at 60°C to 120°C, for 0.5 hours to 24 hours, and preferably 1 hour to 12 hours, whereby Compound P-1 can be obtained.

### Fourth Step

Compound P-2 can be obtained from Compound P-1 in the same manner as described above in the fourth step in "1) Synthesis of Compounds A-1 and A-2".

### 17) Synthesis of Compounds Q-1 and Q-2

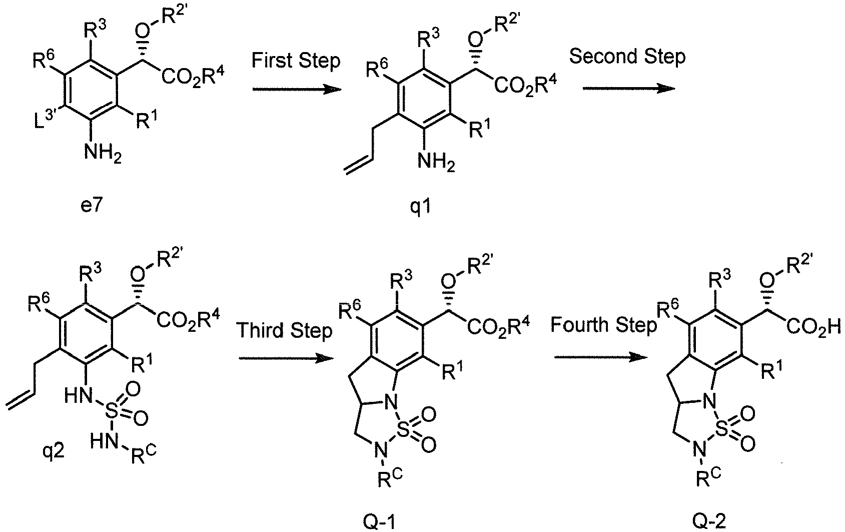

wherein each symbol has the same meaning as described above.

### First Step

Compound q1 can be obtained by a coupling reaction of Compound e7 with Z-allyl. As the reaction, Suzuki cross-coupling, Negishi cross-coupling, Stille coupling, and the like are exemplified. As Z, a boronic acid, a boronic acid ester, an alkyltin, a zinc halide and the like are exemplified.

In a solvent such as dioxane, DMF, DMA, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and a allylboronic acid, a allylboronic acid ester, an alkylallyltin or a allylzinc halide that is commercially available or prepared by a known method are added to Compound e7, and the mixture is reacted under a nitrogen atmosphere at 0°C to 180°C, and preferably at 60°C to 120°C, for 0.5 hours to 24 hours, and preferably 1 hour to 12 hours, whereby Compound q1 can be obtained.

### Second Step

In a solvent such as pyridine, lutidine or collidine, R¹ -NHSO₂ Cl that is commercially available or synthesized by a known method is added to Compound q1, and the mixture is reacted at 0°C to 120°C, and preferably at 20°C to 60°C, for 0.1 hours to 48 hours, and preferably 1 hour to 24 hours, whereby Compound q2 can be obtained.

### Third Step

In a solvent such as dioxane, DMF, DMA, DME, THF or water, or in a mixed solvent thereof, a base such as potassium carbonate, sodium carbonate, cesium carbonate or potassium phosphate, and a copper reagent such as Cu(OAc)₂, neodecanoate copper(II) are added to Compound q2, and the mixture is reacted at 40°C to 180°C, and preferably at 80°C to 120°C, for 0.5 hours to 24 hours, and preferably 1 hour to 12 hours, whereby Compound Q-1 can be obtained.

### Fourth Step

Compound Q-2 can be obtained from Compound Q-1 in the same manner as described above in the fourth step in "1) Synthesis of Compounds A-1 and A-2".

### 18) Synthesis of Compounds R-1 and R-2

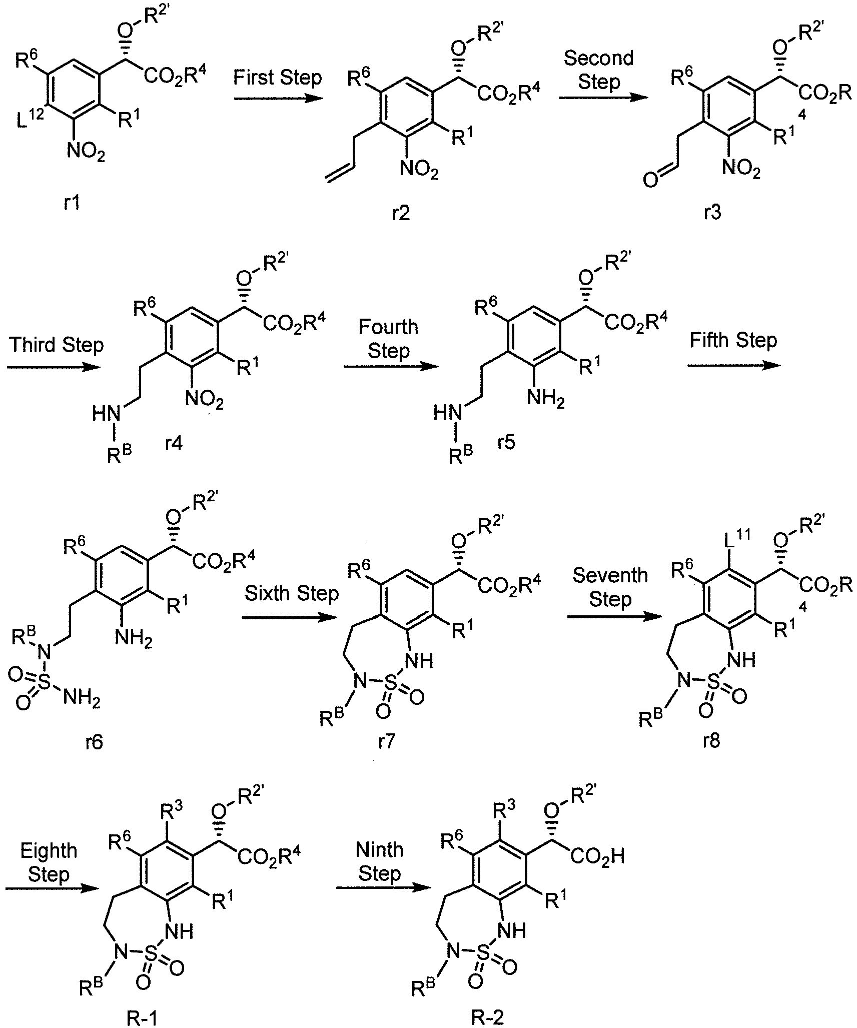

wherein R¹, R^{2'}, R³, R⁴, R⁶, R^{B} and L¹¹ have the same meaning as described above; L¹² is leaving group such as halogen.

### First Step

Compound r2 can be obtained by a coupling reaction of Compound r1 with Z-allyl. As the reaction, Suzuki cross-coupling, Negishi cross-coupling, Stille coupling, and the like are exemplified. As Z, a boronic acid, a boronic acid ester, an alkyltin, a zinc halide and the like are exemplified.

In a solvent such as dioxane, DMF, DMA, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and a allylboronic acid, a allylboronic acid ester, an alkylallyltin or a allylzinc halide that is commercially available or prepared by a known method are added to Compound r1, and the mixture is reacted under a nitrogen atmosphere at 0°C to 180°C, and preferably at 60°C to 120°C, for 0.5 hours to 24 hours, and preferably 1 hour to 12 hours, whereby Compound r2 can be obtained.

### Second Step

In a solvent such as dichloromethane, chloroform or methanol, or in a mixed solvent thereof, pyridine is added to Compound r2, and an ozone gas is passed through the mixture at -100°C to 0°C, and preferably at -78°C to 30°C, for 0.1 hours to 5 hours, and preferably 0.5 hours to 2 hours, whereby Compound r3 can be obtained.

Also, in a mixed solvent of diethyl ether, THF, acetone or the like and water, a catalytic amount of osmium tetroxide or dipotassium osmate dehydrate, and an oxidizing agent such as N-methylmorpholine oxide or N-trimethylamine oxide are added to Compound r2, and the mixture is reacted at -20°C to 60°C, and preferably at 0°C to 20°C, for 0.5 hours to 24 hours, and preferably 2 hours to 12 hours, whereby diol compound can be obtained. Thereafter in the same solvent as above, sodium periodate is added to diol compound, the mixture is stirred at -20°C to 60°C, and preferably at 0°C to 20°C for 0.5 hours to 24 hours, and preferably 2 hours to 12 hours, whereby Compound r3 can be obtained.

### Third Step

In a solvent such as dichloromethane, dichloroethane, THF, methanol or ethanol, acetic acid, TFA or the like, and an amine that is commercially available or synthesized by a known method, are added to Compound r3, and the mixture is reacted at 0°C to 150°C, and preferably at 20°C to 60°C, for 0.1 hours to 24 hours, and preferably 1 hour to 6 hours, whereby a imine can be obtained. The obtained imine is not isolated. To the reaction mixture, a reducing agent such as sodium cyanoborohydride or sodium triacetoxyborohydride is added, and the mixture is reacted at -30°C to 50°C, and preferably at 0°C to 20°C, for 0.1 hours to 24 hours, and preferably 0.5 hours to 12 hours, whereby Compound r4 can be obtained.

### Fourth Step

In a mixed solvent of an organic solvent such as methanol, ethanol or THF and water, a metal such as iron, zinc or tin is added to Compound r4, under acidic conditions of hydrochloric acid or acetic acid, under alkaline conditions of potassium hydroxide or sodium hydroxide, or under neutral conditions of ammonium chloride, and the mixture is reacted at 0°C to 120°C, and preferably at 25°C to 80°C, for 0.1 hours to 24 hours, and preferably 1 hour to 12 hours, whereby Compound r5 can be also obtained.

### Fifth Step

In a solvent such as pyridine, lutidine or collidine, sulfamide is added to Compound r5, and the mixture is reacted at 20°C to 150°C, and preferably at 50°C to 120°C, for 0.1 hours to 48 hours, and preferably 1 hour to 24 hours, whereby Compound r6 can be obtained.

### Sixth Step

In a solvent such as toluene, xylene, DMF or THF, a base such as triethylamine or N,N-diisopropylethylamine is added to Compound r6, and the mixture is reacted at 20°C to 150°C, and preferably at 50°C to 120°C, for 0.1 hours to 48 hours, and preferably 1 hour to 24 hours, whereby Compound r7 can be obtained.

### Seventh Step

In a solvent such as dichloromethane, THF, toluene, acetonitrile or DMF, bromine or a halogenating reagent such as NBS, NCS and NIS is added to Compound r7, and when L¹¹ is bromo, the mixture is reacted at -30°C to 50°C, and preferably at - 10°C to 20°C, for 0.1 hours to 48 hours, and preferably 2 hours to 24 hours, whereby Compound r8 can be obtained. When L¹¹ is chloro or iodine, the mixture is reacted at 10°C to 150°C, and preferably at 60°C to 120°C, for 0.5 hours to 48 hours, and preferably 1 hour to 24 hours, whereby Compound r8 can be obtained.

### Eighth Step

Compound R-1 can be obtained by a coupling reaction of Compound r8 with Z-R⁴. As the reaction, Suzuki cross-coupling, Ullmann cross-coupling, Negishi cross-coupling, Stille coupling, and the like are exemplified. As Z, a boronic acid, a boronic acid ester, an alkyltin, a zinc halide and the like are exemplified.

In a solvent such as dioxane, DMF, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), a base such as potassium carbonate, sodium carbonate, cesium carbonate or potassium phosphate, and a boronic acid, a boronic acid ester, an alkyltin or a zinc halide that is commercially available or prepared by a known method are added to Compound r8, and the mixture is reacted under a nitrogen atmosphere at 0°C to 150°C, and preferably at 60°C to 120°C, for 0.5 hours to 24 hours, and preferably 1 hour to 12 hours, whereby Compound R-1 can be obtained.

### Ninth Step

Compound R-2 can be obtained from Compound R-1 in the same manner as described above in the fourth step in "1) Synthesis of Compounds A-1 and A-2".

### 19) Synthesis of Compounds S-1 and S-2

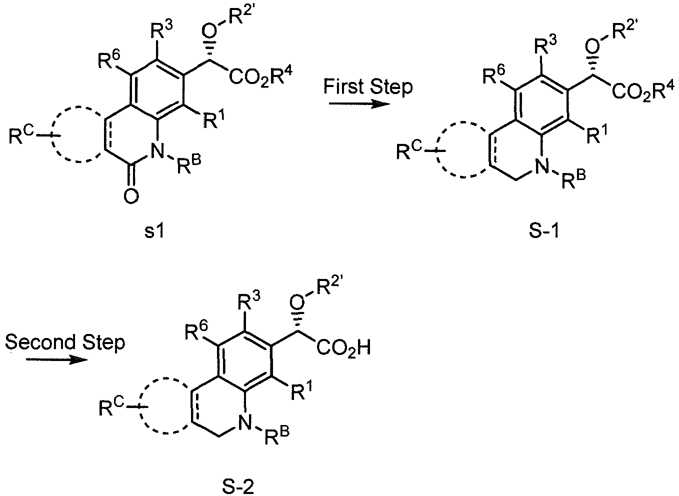

wherein each symbol has the same meaning as described above; dashlined portion is a ring C.

### First Step

In a solvent such as dichloromethane, THF or toluene, or in a mixed solvent thereof, a reducing agent such as borane or diisobutylaluminum hydride is added to Compound s1, and the mixture is reacted at -20°C to 100°C, and preferably at 0°C to 30°C, for 0.5 hours to 24 hours, and preferably 1 hour to 6 hours, whereby Compound S-1 can be obtained.

### Second Step

Compound S-2 can be obtained from Compound S-1 in the same manner as described above in the fourth step in "1) Synthesis of Compounds A-1 and A-2".

### 20) Synthesis of Compounds T-1 to T-3

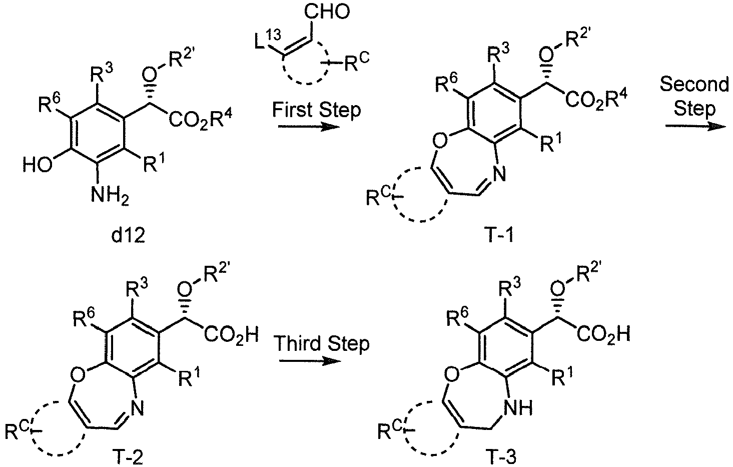

wherein R¹, R^{2'}, R³, R⁴, R⁶ and R^{C} have the same meaning as described above; L¹³ is leaving group such as halogen or substituted or unsubstituted alkylsulfonyloxy; dashlined portion is a ring C.

### First Step

In a solvent such as dichloromethane, toluene or xylene, or in a mixed soluvent thereof, an acid such as para-toluene sulfonic acid or acetic acid, or a dehydrating agent such as anhydrous magnesium sulfate or anhydrous sodium sulfate is added to Compound d12, and the mixture is reacted at 0°C to 150°C, and preferably at 20°C to 120°C, for 30 minutes to 24 hours, and preferably 1 hour to 6 hours, whereby imine can be obtained. In a solvent such as DMSO, DMF or DMA, or in a mixed soluvent thereof, a base such as sodium hydride, sodium hydroxide, potassium carbonate or triethylamine is added to the imine, and the mixture is reacted at 0°C to 150°C, and preferably at 60°C to 120°C, for 0.5 hours to 24 hours, and preferably 1 hour to 6 hours, whereby Compound T-1 can be obtained.

### Second Step

Compound T-2 can be obtained from Compound T-1 in the same manner as described above in the fourth step in "1) Synthesis of Compounds A-1 and A-2".

### Third Step

Compound T-3 can be obtained from Compound T-2 in the same manner as described above in the fifth step in "14) Synthesis of Compounds N-1 to N-3".

### 21) Synthesis of Compounds U-1 to U-3

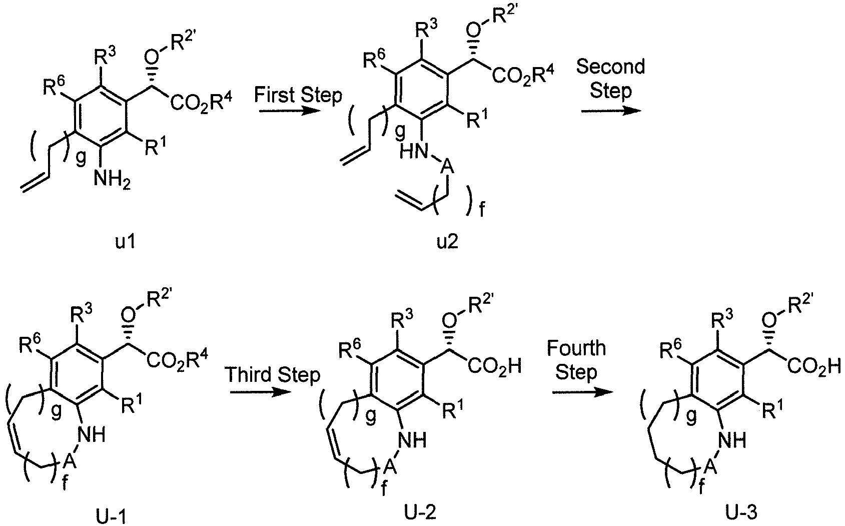

wherein R¹, R^{2'}, R³, R⁴, R⁶ and A have the same meaning as described above; f and g are each independently any integer of 0 to 6.

### First Step

Compound u2 can be obtained from Compound u1 in the same manner as described above in the second step in "14) Synthesis of Compounds N-1 to N-3".

### Second Step

Compound U-1 can be obtained from Compound u2 in the same manner as described above in the third step in "14) Synthesis of Compounds N-1 to N-3".

### Third Step

Compound U-2 can be obtained from Compound U-1 in the same manner as described above in the fourth step in "1) Synthesis of Compounds A-1 and A-2".

### Fourth Step

Compound U-3 can be obtained from Compound U-2 in the same manner as described above in the fifth step in "14) Synthesis of Compounds N-1 to N-3".

### 22) Synthesis of Compounds V-1 to V-3

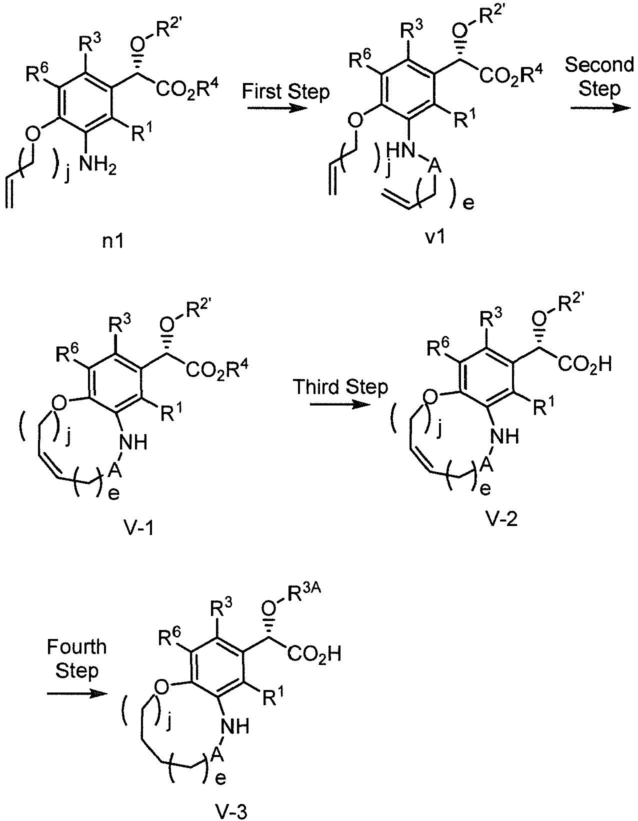

wherein R¹, R^{2'}, R³, R⁴, R⁶, j and A have the same meaning as described above; e is any integer of 0 to 5.

### First Step

Compound v1 can be obtained from Compound n1 in the same manner as described above in the second step in "14) Synthesis of Compounds N-1 to N-3".

### Second Step

Compound V-1 can be obtained from Compound v1 in the same manner as described above in the third step in "14) Synthesis of Compounds N-1 to N-3".

### Third Step

Compound V-2 can be obtained from Compound V-1 in the same manner as described above in the fourth step in "1) Synthesis of Compounds A-1 and A-2".

### Fourth Step

Compound V-3 can be obtained from Compound V-2 in the same manner as described above in the fifth step in "14) Synthesis of Compounds N-1 to N-3".

### 23) Synthesis of Compounds W-1 and W-2

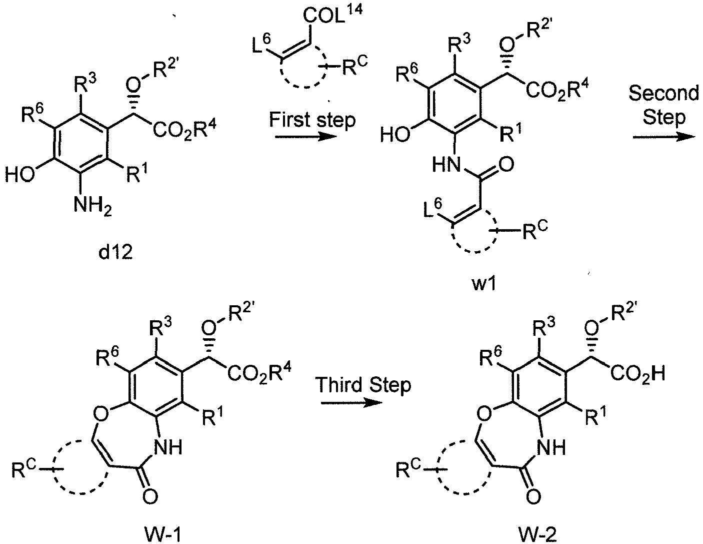

wherein R¹, R^{2'}, R³, R⁴, R⁶, R^{C} and L⁶ have the same meaning as described above; L¹⁴ is leaving group such as halogen; dashlined portion is a ring C.

### First Step

In a solvent such as DMF, THF or dioxane, or in a mixed solvent thereof, a base such as sodium hydride or triethylamine, and an acylating agent such as acid chloride that is commercially available or prepared by a known method are added to Compound d12, and the mixture is reacted at -20°C to 50°C, and preferably at 0°C to 30°C, for 0.1 hours to 10 hours, and preferably 0.5 hours to 2 hours, whereby Compound w1 can be obtained.

Also, in a soluvent such as dioxane, DMF, DME or THF, or in a mixed solvent thereof, carboxylic acid that is commercially available or synthesized by a known method, a condensing agent such as N,N'-dicyclohexylcarbodiimide or N,N'-diisopropylcarbodiimide, a condensation accelerator such as 1-hydroxybenzotriazole or 1-hydroxyazabenzotriazole, and a base such as triethylamine or N,N-diisopropylethylamine are added to Compound d12, and the mixture is reacted at 0°C to 150°C, and preferably at 20°C to 60°C, for 15 hours to 72 hours, and preferably 12 hours to 24 hours, whereby Compound w1 can be obtained.

### Second Step

Compound W-1 can be obtained from Compound w1 in the same manner as described above in the third step in "11) Synthesis of Compounds K-1 and K-2".

### Third Step

Compound W-2 can be obtained from Compound W-1 in the same manner as described above in the fourth step in "1) Synthesis of Compounds A-1 and A-2".

### 24) Synthesis of Compounds X-1 to X-3

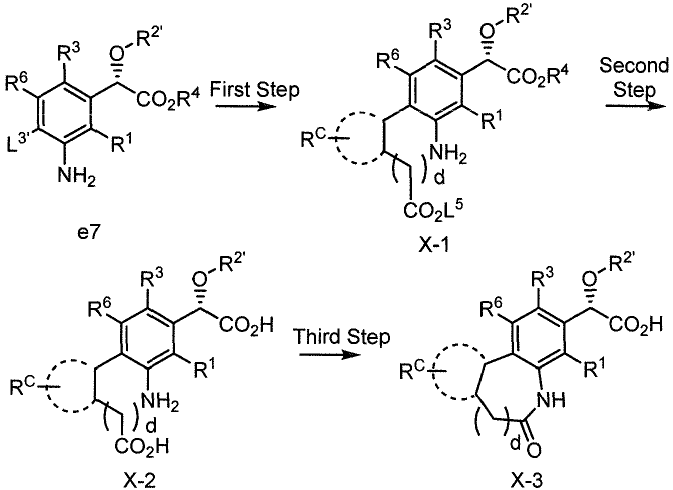

wherein R¹, R^{2'}, R³, R⁴, R⁶, R^{C}, L³' and L⁵ have the same meaning as described above; d is any integer of 0 to 6; dashlined portion is a ring C.

### First Step

Compound X-1 can be obtained from Compound e7 in the same manner as described above in the fifth step in "2) Synthesis of Compounds B-1 and B-2" or in the first step to second step in "7) Synthesis of Compounds G-1 and G-2".

### Second Step

Compound X-2 can be obtained from Compound X-1 in the same manner as described above in the fourth step in "1) Synthesis of Compounds A-1 and A-2".

### Third Step

In a soluvent such as dioxane, DMF, DME or THF, or in a mixed solvent thereof, a condensing agent such as N,N'-dicyclohexylcarbodiimide or N,N'-diisopropylcarbodiimide, a condensation accelerator such as 1-hydroxybenzotriazole or 1-hydroxyazabenzotriazole, and a base such as triethylamine or N,N-diisopropylethylamine are added to Compound X-2, and the mixture is reacted at 0°C to 150°C, and preferably at 20°C to 60°C, for 1 hour to 72 hours, and preferably 12 hours to 24 hours, whereby Compound X-3 can be obtained.

When an active ester is obtained, in a soluvent such as methanol or ethanol, a base such as potassium carbonate is added to the active ester, and the mixture is reacted at 0°C to 150°C, and preferably at 20°C to 60°C, for 15 hours to 72 hours, and preferably 12 hours to 24 hours, whereby ester is derived. Thereafter, in a soluvent such as methanol, ethanol, dioxane, DMF, DME or THF, or in a mixed soluvent thereof, a base such as sodium hydroxide or lithium hydroxide is added to the ester, and the mixture is reacted at 0°C to 150°C, and preferably at 20°C to 60°C, for 0.5 hours to 72 hours, and preferably 1 hour to 2 hours, whereby Compound X-3 can be obtained. Also, in a soluvent such as methanol, ethanol, dioxane, DMF, DME or THF, or in a mixed soluvent thereof, a base such as sodium hydroxide or lithium hydroxide is added to the active ester, and the mixture is reacted at 0°C to 150°C, and preferably at 20°C to 60°C, for 0.5 hours to 72 hours, and preferably 1 hour to 2 hours, whereby Compound X-3 can be obtained.

### 25) Synthesis of Compounds Y-1 and Y-2

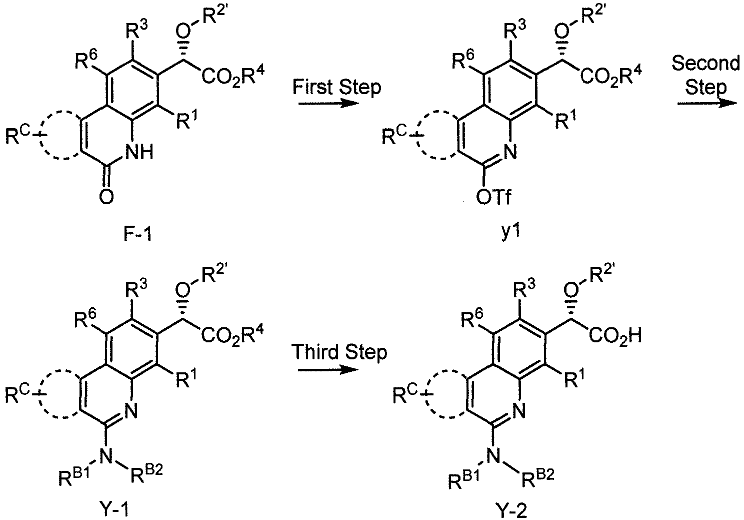

wherein each symbol has the same meaning as described above; dashlined portion is a ring C.

### First Step

In a solvent such as dichloromethane, THF or dichloroethane, or in a mixed solvent thereof, a base such as pyridine, lutidine or triethylamine, and a trifluoromethanesulfonyl
ating agent such as trifluoromethanesulfonic anhydride or a comin's reagent are added to Compound F-1, and the mixture is reacted at -50°C to 50°C, and preferably at -30°C to 30°C, for 0.1 hours to 4 hours, and preferably 0.5 hours to 1 hour, whereby Compound y1 can be obtained.

### Second Step

In a solvent such as DMF, DMA, THF or dioxane, or in a mixed solvent thereof, a base such as triethylamine, lutidine or pyridine, a palladium catalyst such as dibenzylideneacetone palladium, Pd(OAc)₂ or Pd(PPh₃)₂Cl₂, a ligand such as Xantphos or tri-tert-butyl phosphine, and an amine that is commercially available or prepared by a known method are added to Compound y1, and the mixture is reacted at 50°C to 150°C, and preferably at 70°C to 130°C, for 0.1 hours to 8 hours, and preferably 0.5 hours to 2 hours, whereby Compound Y-1 can be obtained.

### Third Step

Compound Y-2 can be obtained from Compound Y-1 in the same manner as described above in the fourth step in "1) Synthesis of Compounds A-1 and A-2".

### 26) Synthesis of Compounds Z-1 and Z-2

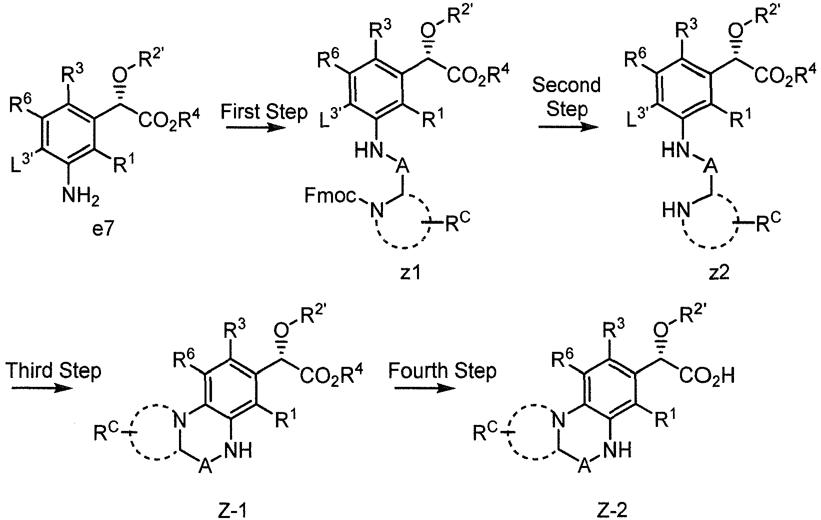

wherein each symbol has the same meaning as described above; dashlined portion is a ring C.

### First Step

Compound z1 can be obtained from Compound e7 in the same manner as described above in the second step in "14) Synthesis of Compounds N-1 to N-3".

### Second Step

In a solvent such as DMF, DMA, THF, dioxane or dichloromethane, or in a mixed solvent thereof, a base such as diethylamine or piperidine is added to Compound z1, and the mixture is reacted at 0°C to 100°C, and preferably at 20°C to 50°C, for 0.5 hours to 8 hours, and preferably 1 hour to 2 hours, whereby Compound z2 can be obtained.

### Third Step

In a solvent such as DMF, DMA, toluene or dioxane, or in a mixed solvent thereof, a base such as cesium carbonate, potassium tert-butoxide or triethylamine, a palladium catalyst such as tris(dibenzylideneacetone)dipalladium, dibenzylideneacetone palladium or Pd(OAc)₂, and a ligand such as tris ortho-tolyl phosphine, 2,2'-bis(diphenylphosphino)-1,1'-binaphthyl or 1,3-bis (2,6-diisopropylphenyl)-4,5-dihydro-imidazolium tetrafluoroborate are added to Compound z2, and the mixture is reacted under microwave irradiation at 100°C to 200°C, and preferably at 120°C to 160°C, for 5 minutes to 1 hour, and preferably 10 minutes to 30 minutes, whereby Compound Z-1 can be obtained.

### Fourth Step

Compound Z-2 can be obtained from Compound Z-1 in the same manner as described above in the fourth step in "1) Synthesis of Compounds A-1 and A-2".

### 27) Synthesis of Compounds AA-1 and AA-2

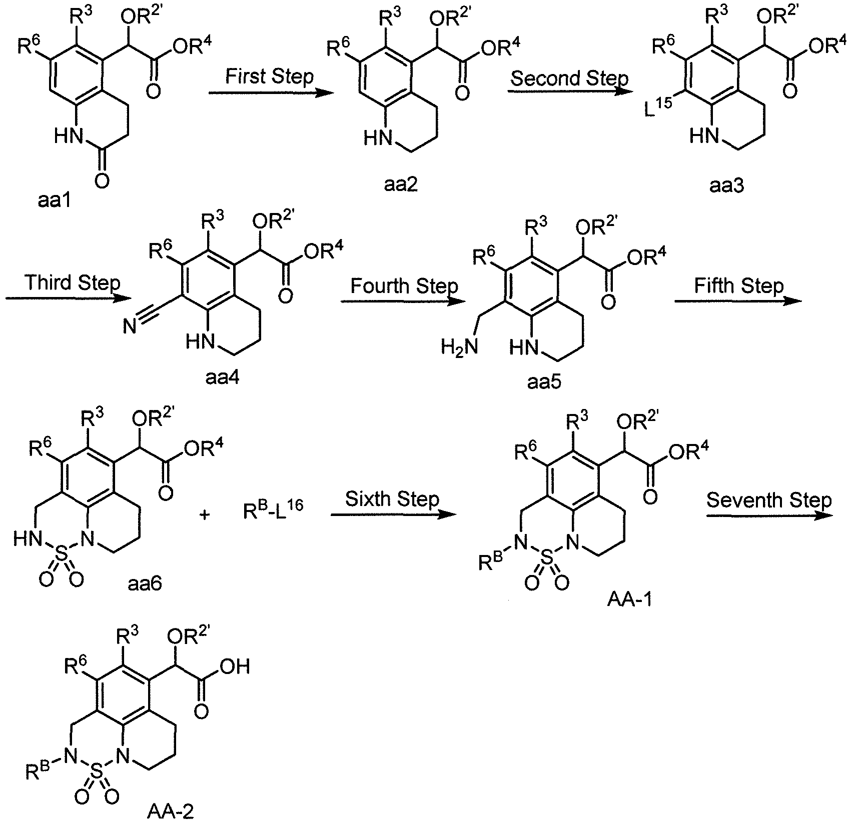

wherein R^{2'}, R³, R⁴, R⁶ and R^{B} have the same meaning as described above; L¹⁵ and L¹⁶ are leaving group such as halogen.

### First Step

In a solvent such as THF, toluene or dichloromethane, or in a mixed solvent thereof, a reducing agent such as borane-tetrahydrofuran complex, borane-diethyl ether complex or borane-dimethyl sulfide complex is added to Compound aa1, and the mixture is reacted at 10°C to 110°C, and preferably at 30°C to 90°C, for 0.1 hours to 12 hours, and preferably 0.5 hours to 2 hours, whereby Compound aa2 can be obtained.

### Second Step

In a solvent such as dichloromethane, acetonitrile or DMF or a mixed solvent thereof, bromine or a halogenating reagent such as NBS, NCS and NIS is added to Compound aa2, and when L3' is bromo, the mixture is reacted at -30°C to 50°C, and preferably at -10°C to 20°C, for 0.1 hours to 8 hours, and preferably 0.5 hours to 1 hour, whereby Compound aa3 can be obtained.

### Third Step

In a solvent such as DMF, DMA, THF, dioxane or water, or in a mixed solvent thereof, a cyanide such as zinc cyanide, copper cyanide or sodium cyanide, and a catalyst such as Pd(PPh₃)₄, Pd(OAc)₂ or Pd(PPh₃)₂Cl₂ are added to Compound aa3, and the mixture is reacted at 50°C to 150°C, and preferably at 70°C to 130°C, for 0.1 hours to 12 hours, and preferably 0.5 hours to 2 hours, whereby Compound aa4 can be obtained.

### Fourth Step

In a solvent such as methanol, ethyl acetate or acetic acid, or in a mixed solvent thereof, a catalyst such as Pd/C or Pd(OH)₂ is added to Compound aa4, and the mixture is reacted under a hydrogen atmosphere at 30°C to 130°C, and preferably at 50°C to 110°C, for 0.1 hours to 12 hours, and preferably 0.5 hours to 2 hours, whereby Compound aa5 can be obtained.

### Fifth Step

In a solvent such as benzene, toluene or xylene, or in a mixed solvent thereof, a sulfonylating agent such as amido sulfate or thionyl chloride, and a base such as triethylamine, lutidine or pyridine are added to Compound aa5, and the mixture is reacted at 50°C to 150°C, and preferably at 70°C to 130°C, for 0.1 hours to 12 hours, and preferably 0.5 hours to 2 hours, whereby Compound aa6 can be obtained.

### Sixth Step

In a solvent such as DMF, DMA or THF, or in a mixed solvent thereof, a base such as sodium hydride, potassium carbonate or cesium carbonate, and an alkylating agent such as alkyl chloride, alkyl bromide or alkyl triflate are added to Compound aa6, and the mixture is reacted at -30°C to 50°C, and preferably at -10°C to 20°C, for 0.1 hours to 8 hours, and preferably 0.5 hours to 1 hour, whereby Compound AA-1 can be obtained.

### Seventh Step

Compound AA-2 can be obtained from Compound AA-1 in the same manner as described above in the fourth step in "1) Synthesis of Compounds A-1 and A-2".

A compound wherein sulfonylurea structure of Compound AA-1 and AA-2 is replased with urea structure, can be obtained by using carbonylating agent such as urea, CDI or diethyl carbonate, instead of sulfonylating agent in the same manner as in the fifth step.

Compound aa1 can be synthesized by the following method. 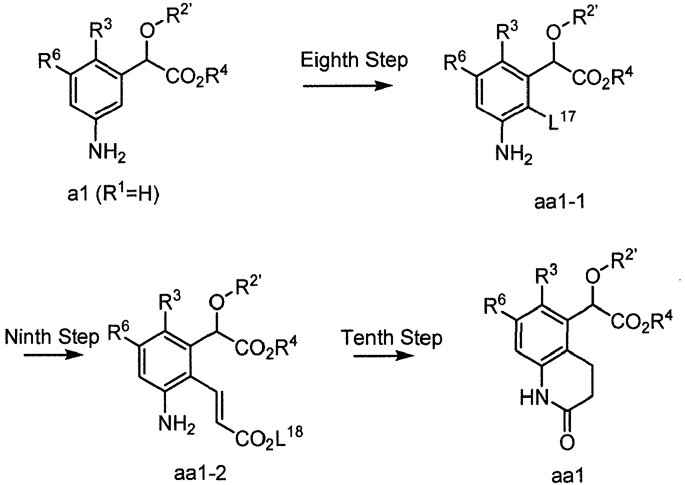 wherein R^{2'} , R³, R⁴ and R⁶ have the same meaning as described above; L¹⁷ is leaving group such as halogen; L¹⁸ is substituted or unsubstituted alkyl.

### Eighth Step

In a solvent such as dichloromethane, THF, toluene, acetonitrile or DMF, bromine or a halogenating reagent such as NBS, NCS and NIS is added to Compound a1 (wherein, R¹ is hydrogen), and when L¹⁷ is bromo, the mixture is reacted at -30°C to 50°C, and preferably at -10°C to 20°C, for 0.1 hours to 10 hours, and preferably 0.5 hours to 2 hours, whereby Compound aa1-1 can be obtained. When L¹¹ is chloro or iodine, the mixture is reacted at 10°C to 150°C, and preferably at 60°C to 120°C, for 0.5 hours to 24 hours, and preferably 1 hour to 6 hours, whereby Compound aa1-1 can be obtained.

### Ninth Step

In a solvent such as DMF, DMA, THF, dioxane or water, or in a mixed solvent thereof, a base such as K₂CO₃, Na₂CO₃ or K₃PO₄, and (E)-3-boranyl acrylic acid ester that is commercially available or prepared by a known method are added to Compound aa1-1, and the mixture is reacted at 50°C to 150°C, and preferably at 70°C to 130°C, for 0.1 hours to 8 hours, and preferably 0.5 hours to 2 hours, whereby Compound aa1-2 can be obtained.

### Tenth Step

In a solvent such as methanol, ethanol, DMF or acetic acid, or in a mixed solvent thereof, palladium carbon is added to Compound aa1-2, and the mixture is reacted under a hydrogen atmosphere at 0°C to 100°C, and preferably at 20°C to 50°C, for 1 hour to 24 hours, and preferably 2 hours to 6 hours, whereby Compound aa1 can be obtained.

### 28) Synthesis of Compounds AB-1 and AB-2

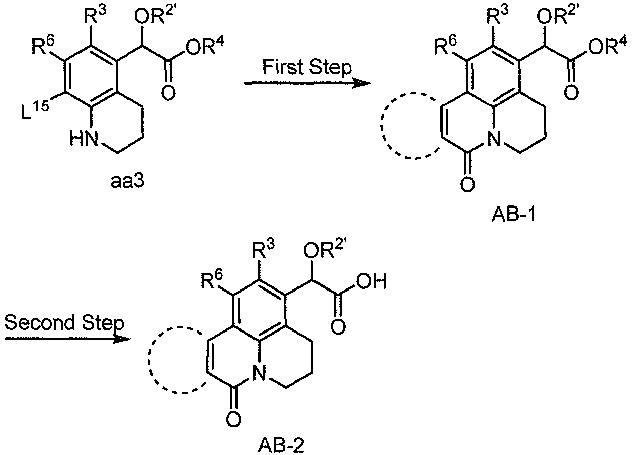

wherein each symbol has the same meaning as described above; dashlined portion is a ring C.

### First Step

In a solvent such as DMF, DMA, THF, dioxane or water, or in a mixed solvent thereof, a palladium catalyst such as Pd(PPh₃)₄, or Pd(PPh₃)₂Cl₂, a base such as K₂ CO₃, Na₂ CO₃ or K₃ PO₄, and a boronic acid or a boronic acid ester are added to Compound aa3, and the mixture is reacted at 50°C to 150°C, and preferably at 70°C to 130°C, for 0.1 hours to 8 hours, and preferably 0.5 hours to 2 hours, whereby Compound AB-1 can be obtained.

### Second Step

Compound AB-2 can be obtained from Compound AB-1 in the same manner as described above in the fourth step in "1) Synthesis of Compounds A-1 and A-2".

### 29) Synthesis of Compounds AC-1 and AC-2

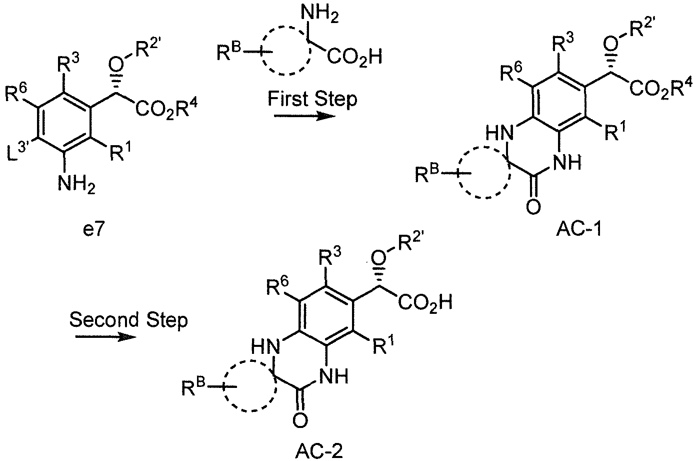

wherein each symbol has the same meaning as described above; dashlined portion is a part of a spiro ring constituting the ring B.

### First Step

Compound AC-1 can be obtained from Compound e7 in the same manner as described above in the third step in "1) Synthesis of Compounds A-1 and A-2". In this process, DMSO may be used as a solvent and cesium carbonate may be used as a base.

### Second Step

Compound AC-2 can be obtained from Compound AC-1 in the same manner as described above in the fourth step in "1) Synthesis of Compounds A-1 and A-2".

### 30) Synthesis of Compounds AD-1 and AD-2

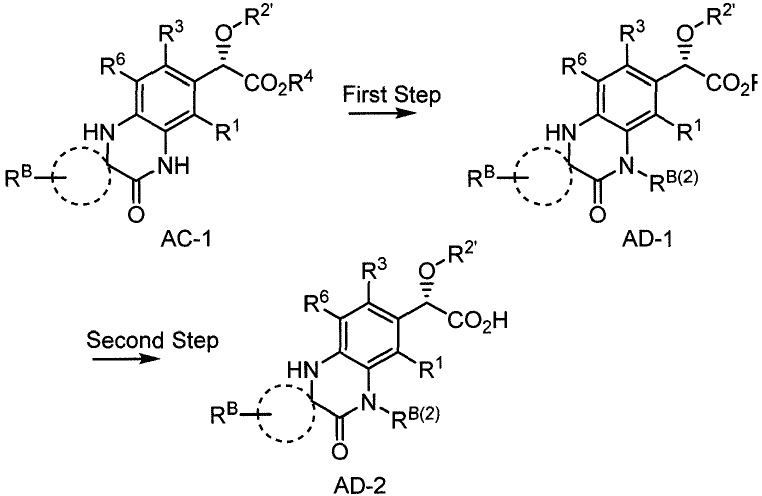

wherein each symbol has the same meaning as described above; R^{B(2)} has the same meaning as R^{B}; dashlined portion is a ring E (a spiro ring).

### First Step

Compound AD-1 can be obtained from Compound AC-1 in the same manner as described above in the twelfth step in "4) Synthesis of Compounds D-1 and D-2".

### Second Step

Compound AD-2 can be obtained from Compound AD-1 in the same manner as described above in the fourth step in "1) Synthesis of Compounds A-1 and A-2".

### 31) Synthesis of Compounds AD-3 and AD-4

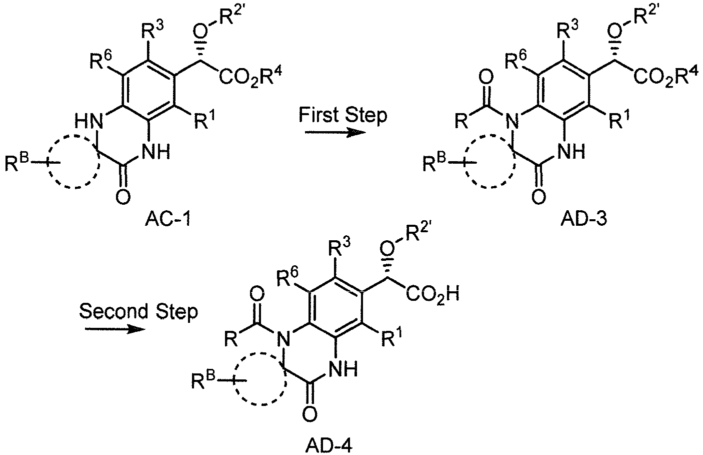

wherein R is acyl residue; dashlined portion is a ring E (a spiro ring); the others have the same meaning as described above.

### First Step

In a solvent such as DMF, THF or dioxane, or in a mixed solvent thereof, a base such as pyridine or triethylamine, and an acylating agent such as acetyl chloride or substituted benzoyl chloride are added to Compound AC-1, and the mixture is reacted at -20°C to 50°C, and preferably at 0°C to 30°C, for 0.1 hours to 10 hours, and preferably 0.5 hours to 2 hours, whereby Compound AD-3 can be obtained.

### Second Step

Compound AD-4 can be obtained from Compound AD-3 in the same manner as described above in the fourth step in "1) Synthesis of Compounds A-1 and A-2".

### 32) Synthesis of Compounds AE-1 and AE-2

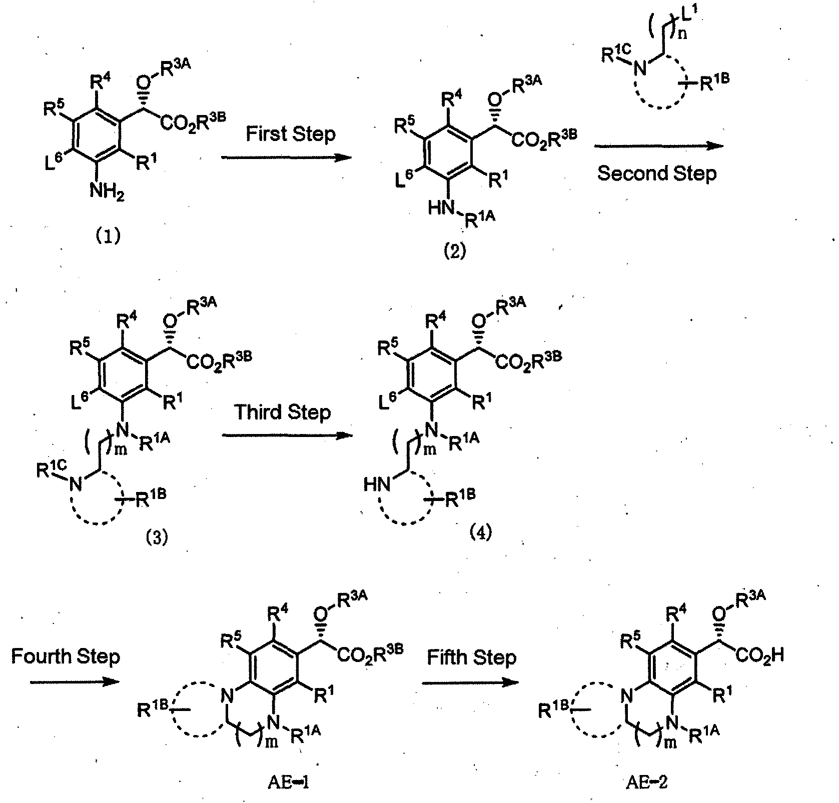

wherein L² is leaving group such as halogen; R^{1A} is substituted alkyl, substituted alkenyl or the like; the others have the same meaning as described above.

### First Step

Compound (2) can be obtained from Compound (1) in the same manner as described above in the second step in "14) Synthesis of Compounds N-1 to N-3".

### Second Step

Compound (3) can be obtained from Compound (2) in the same manner as described above in the twelfth step in "4) Synthesis of Compounds D-1 to D-2".

### Third Step

Compound (4) can be obtained from Compound (3) in the same manner as described above in the second step in "26) Synthesis of Compounds Z-1 to Z-2".

Also, in a solvent such as DMF, DMA, toluene or dioxane, or in a mixed solvent thereof, a base such as DBU or lithium hydroxide, and thiol such as mercaptoethanol or mercaptoacetic acid are added to Compound (3), and the mixture is reacted at 0°C to 100°C, and preferably at 20°C to 60°C, for 5 minutes to 24 hours, and preferably 1 hour to 6 hours, whereby Compound (4) can be obtained.

### Fourth Step

Compound AE-1 can be obtained from Compound (4) in the same manner as described above in the third step in "26) Synthesis of Compounds Z-1 to Z-2".

### Fifth Step

Compound AE-2 can be obtained from Compound AE-1 in the same manner as described above in the fourth step in "1) Synthesis of Compounds A-1 and A-2".

### 33) Synthesis of Compounds AF-1 and AF-2

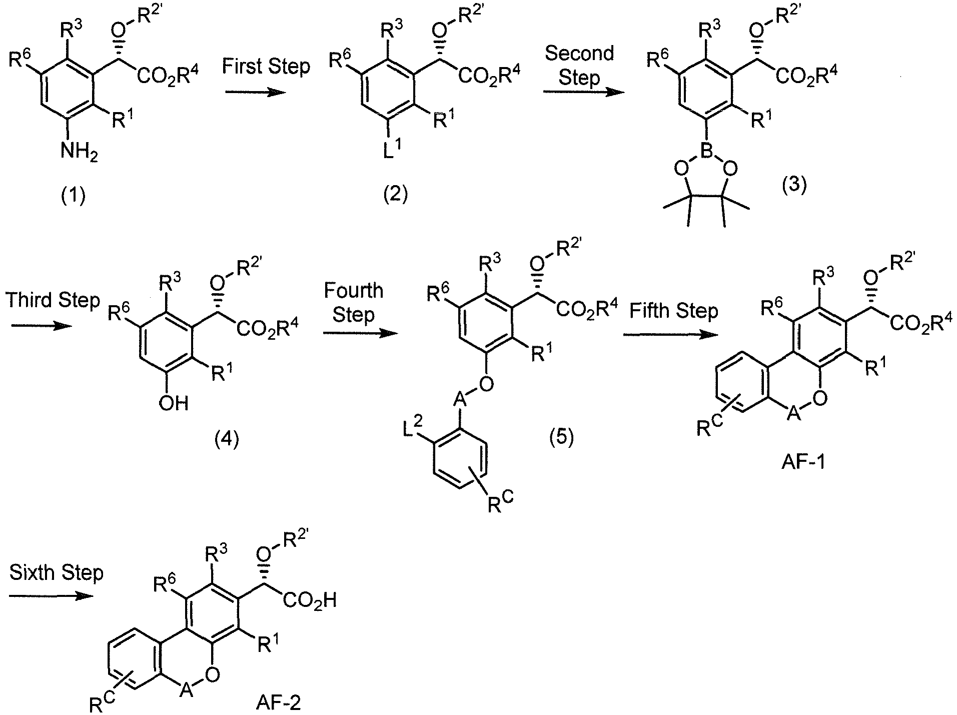

wherein each symbol has the same meaning as described above.

### First Step

In a solvent such as acetonitrile, methanol, acetone or water, or in a mixed solvent thereof, a nitrite ester such as tert-butyl nitrite or isopentyl nitrite, and a cupric halide such as cupric chloride, cupric bromide or cupric iodide are added to Compound (1), and the mixture is reacted at 0°C to 100°C, and preferably at 20°C to 50°C, for 0.5 hours to 8 hours, and preferably 2 hours to 4 hours, whereby Compound (2) can be obtained.

### Second Step

In a solvent such as dioxane, DMF, DME, tetrahydrofuran or water, or in a mixed solvent thereof, a palladium catalyst such as Pd(PPh₃)₄, Pd(OAc)₂, Pd(PPh₃)₂Cl₂, Pd(dppf)Cl₂ dichloromethane complex or Pd(dtbpf), a base such as potassium acetate, sodium acetate, potassium carbonate or potassium phosphate, and bis(pinacolato)diboron are added to Compound (2), and the mixture is reacted under a nitrogen atmosphere at 0°C to 150°C, and preferably at 60°C to 120°C, for 0.5 hours to 24 hours, and preferably 1 hour to 12 hours, whereby Compound (3) can be obtained.

### Third Step

In a solvent such as dioxane, DMF, DME, THF DMSO, or in a mixed solvent thereof, a base such as sodium hydroxide or potassium hydroxide, and hydrogen peroxide solution are added to Compound (3), and the mixture is reacted at -20°C to 30°C, and preferably at -10°C to 20°C, for 0.5 hours to 12 hours, and preferably 0.5 hours to 2 hours, whereby Compound (4) can be obtained.

### Fourth Step

In a solvent such as DMF, DME, THF, acetone or acetonitrile, a base such as potassium carbonate, sodium carbonate, cesium carbonate or sodium hydride, and benzyl bromide or benzyl chloride that is commercially available or synthesized by a known method are added to Compound (4), and the mixture is reacted at 0°C to 100°C, and preferably at 20°C to 50°C, for 0.5 hours to 24 hours, and preferably 1 hour to 5 hours, whereby Compound (5) can be obtained.

### Fifth Step

Compound AF-1 can be obtained in the same manner as in the second step in "3) Synthesis of Compounds C-1 and C-2" described above.

### Sixth Step

Compound AF-2 can be obtained in the same manner as in the third step in "3) Synthesis of Compounds C-1 and C-2" described above.

### 34) Synthesis of Compounds AB-3 and AB-4

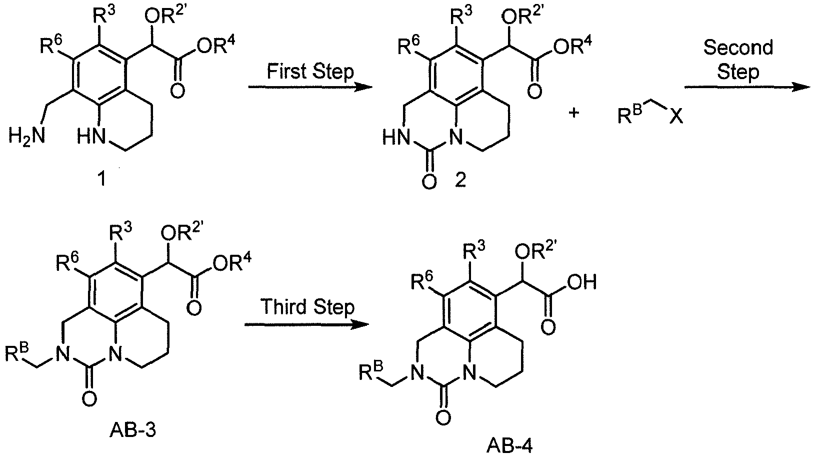

wherein X is leaving group such as halogen; the others have the same meaning as described above.

### First Step

Compound 2 can be obtained by using carbonylating agent such as urea, CDI or diethyl carbonate, instead of sulfonylating agent in the same manner as in the fifth step in 27).

### Second Step

Compound AB-3 can be obtained in the same manner as in the sixth step in 27).

### Third Step

Compound AB-4 can be obtained in the same manner as in the seventh step in 27).

### 35) Synthesis of Compounds AC-1 and AC-2

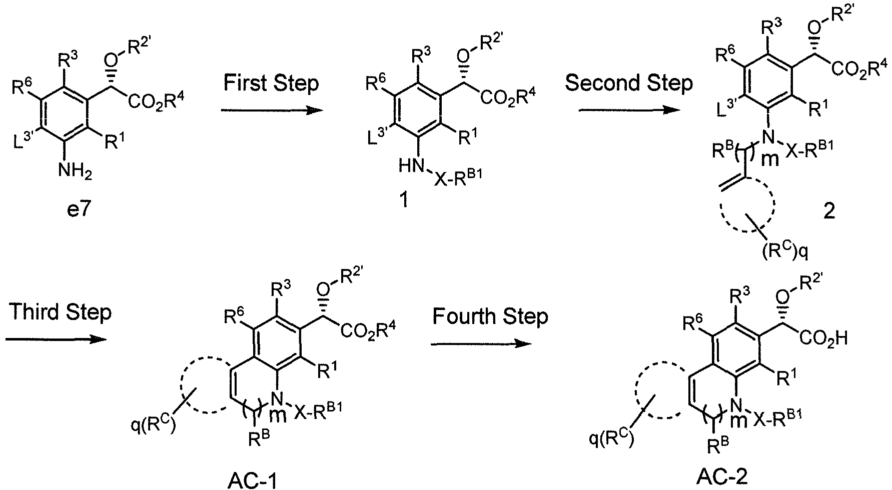

wherein X is -SO₂-, -CO-, -CONH-, -COO- and the like; R^{B1} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl or substituted or unsubstituted non-aromatic heterocyclyl; q is an integer of 1 to 4; m is an integer of 1 to 4; dashlined portion is a ring C; the others have the same meaning as described above; R^{B} is may be hydrogen.

### First Step

In a solvent such as dichloromethane, dichloroethane, THF, toluene or pyridine, a base such as triethylamine, N-methylmorpholine or pyridine, and R^{B1}-SO₂Cl, R^{B1}-COCl, R^{B1}-NCO or R^{B1}-OCOCl that is commercially available or synthesized by a known method are added to Compound e7, and the mixture is reacted at -20°C to 100°C, and preferably at 0°C to 30°C, for 0.1 hours to 10 hours, and preferably 0.5 hours to 2 hours, whereby Compound 1 can be obtained.

### Second Step

In a solvent such as DMF, DME, THF, acetone or acetonitrile, a base such as potassium carbonate, sodium carbonate, cesium carbonate or sodium hydride, and a ring C alkyl halide optionally substituted with R^{C} or R^{B} that is commercially available or prepared by a known method (e.g., benzyl halide) are added to Compound 1, and the mixture is reacted at 0°C to 100°C, and preferably at 20°C to 50°C, for 0.5 hours to 24 hours, and preferably 1 hour to 5 hours, whereby Compound 2 can be obtained. Also, when -X- of Compound 1 is -SO₂-, in a solvent such as THF, toluene or dichloromethane, Mitsunibu reagent such as DEAD, DIAD or bis(2-methoxyethyl) azodicarboxylate, triphenylphosphine, tri-n-butyl phosphine or tributylphosphine, and benzyl alcohol optionally substituted with R^{C} that is commercially available or prepared by a known method (R^{B}=H) are added to Compound 1, and the mixture is reacted at -20°C to 100°C, and preferably at 0°C to 30°C, for 0.5 hours to 24 hours, and preferably 1 hour to 5 hours, whereby Compound 2 can be obtained.

### Third Step

Compound AC-1 can be obtained from Compound d2 in the same manner as described above in the first step in "3) Synthesis of Compounds C-1 and C-2".

### Fourth Step

Compound AC-2 can be obtained from Compound AC-1 in the same manner as described above in the fourth step in "1) Synthesis of Compounds A-1 and A-2".

### 36) Synthesis of Compounds AF-1 and AF-2

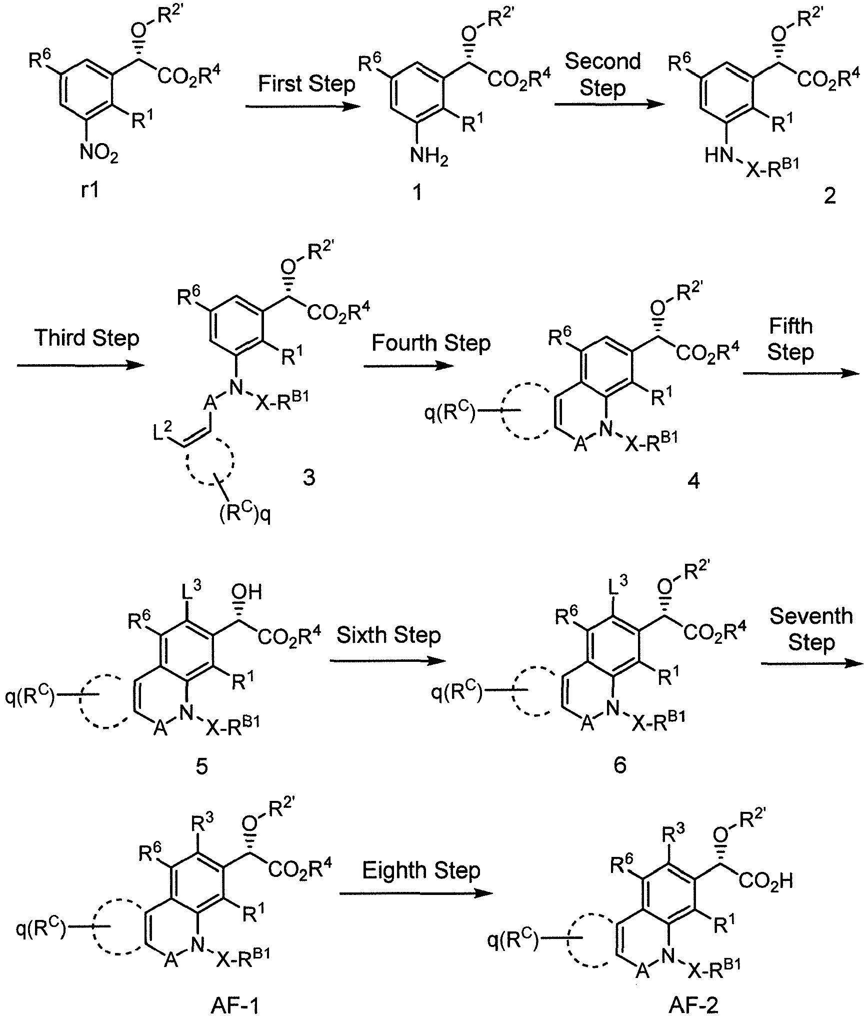

wherein each symbol has the same meaning as described above; preferably A is alkylene optionally substituted with R^{B} (especially methylene); preferably X is -SO₂-; preferably R^{B 1} is alkyl.

### First Step

In a solvent such as methanol, ethanol, THF, dioxane or ethyl acetate, or in a mixed solvent thereof, a base such as triethylamine, N-methylmorpholine or pyridine, and a catalyst such as 5% or 10% palladium carbon or palladium hydroxide are added to Compound r1, and the mixture is reacted under a hydrogen atmosphere and under 1 to 10 atmospheres, and preferably 3 to 5 atmospheres, at 0°C to 50°C, and preferably at 15°C to 25°C, for 0.1 hours to 48 hours, and preferably 4 hours to 24 hours, whereby Compound 1 can be obtained.

### Second Step

In a solvent such as dichloromethane, dichloroethane, THF, toluene or pyridine, a base such as triethylamine, N-methylmorpholine or pyridine, and R^{B 1}-SO₂ Cl, R^{B 1}-COCl, R^{B 1 -} NCO or R^{B 1} -OCOCl that is commercially available or prepared by a known method are added to Compound 1, and the mixture is reacted at -20°C to 100°C, and preferably at 0°C to 30°C, for 0.1 hours to 10 hours, and preferably 0.5 hours to 2 hours, whereby Compound 2 can be obtained.

### Third Step

In a solvent such as DMF, DME, THF, acetone or acetonitrile, a base such as potassium carbonate, sodium carbonate, cesium carbonate or sodium hydride, and a ring C alkyl halide optionally substituted with R^{C} that is commercially available or prepared by a known method (e.g., benzyl halide) are added to Compound 2, and the mixture is reacted at 0°C to 100°C, and preferably at 20°C to 50°C, for 0.5 hours to 24 hours, and preferably 1 hour to 5 hours, whereby Compound 3 can be obtained. Also, when -X- of Compound 2 is -SO₂-, in a solvent such as THF, toluene or dichloromethane, Mitsunibu reagent such as DEAD, DIAD or bis(2-methoxyethyl) azodicarboxylate, triphenylphosphine, tri-n-butyl phosphine or tributylphosphine, and benzyl alcohol substituted with R^{C} that is commercially available or prepared by a known method are added to Compound 2, and the mixture is reacted at -20°C to 100°C, and preferably at 0°C to 30°C, for 0.5 hours to 24 hours, and preferably 1 hour to 5 hours, whereby Compound 3 can be obtained

### Fourth Step

Compound d4 can be obtained from Compound d3 in the same manner as described above in the first step in "3) Synthesis of Compounds C-1 and C-2".

### Fifth Step

In a solvent of acetic acid, concentrated sulfuric acid or concentrated hydrochloric acid, and a halogenating reagent such as NBS, 1,3-dibromo-5,5-dimethyl hydantoin, 1,3-diiodo-5,5-dimethyl hydantoin, dibromoisocyanuric acid or diiodo isocyanuric acid are added to Compound 4, and the mixture is reacted at -20°C to 60°C, and preferably at 0°C to 30°C, for 0.1 hours to 12 hours, and preferably 0.5 hours to 2 hours, whereby Compound 5 can be obtained.

### Sixth Step

Compound 6 can be obtained from Compound 5 in the same manner as described above in the eleventh step in "1) Synthesis of Compounds A-1 and A-2".

### Seventh Step

Compound AF-1 can be obtained from Compound 6 in the same manner as described above in the fifth step in "2) Synthesis of Compounds B-1 and B-2".

### Eighth Step

Compound AF-2 can be obtained from Compound 7 in the same manner as described above in the fourth step in "1) Synthesis of Compounds A-1 and A-2".

### 37) Synthesis of Compounds AG-1 and AG-2

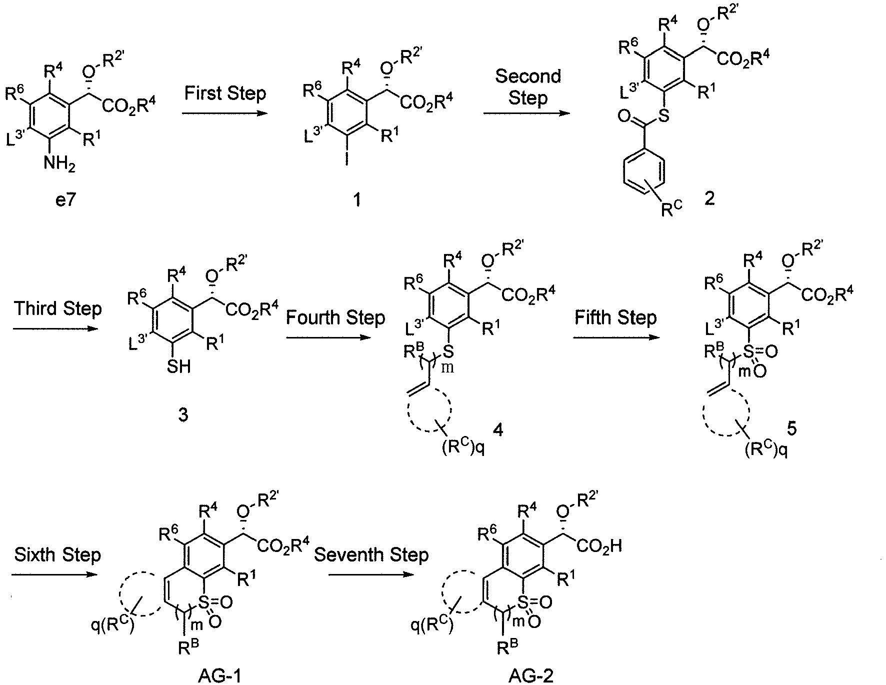

wherein each symbol has the same meaning as described above.

### First Step

In a solvent such as acetonitrile, methanol, acetone or water, or in a mixed solvent thereof, a nitrite ester such as tert-butyl nitrite or isopentyl nitrite, and a iodide salt such as cupric iodide or potassium iodide are added to Compound e7, and the mixture is reacted at 0°C to 100°C, and preferably at 20°C to 60°C, for 0.5 hours to 8 hours, and preferably 2 hours to 4 hours, whereby Compound 1 can be obtained.

### Second Step

In a solvent such as toluene, 1,4-dioxane, DMF or water, or in a mixed solvent thereof, a thiocarboxylic acid such as thiobenzoic acid substituted with RC or thioacetate substituted with RC that is commercially available or prepared by a known method, a ligand such as 1,10-phenanthroline or N,N,N',N'-tetramethylethylenediamine, a base such as N-ethyl-N-diisopropylamine or triethylamine, and a copper halide such as copper iodide (I) or copper bromide (I) are added to Compound 1, and the mixture is reacted at 80°C to 150°C, and preferably at 100°C to 130°C, for 2 hours to 24 hours, and preferably 4 hours to 8 hours, whereby Compound 2 can be obtained.

### Third Step

In a solvent such as methanol, THF, acetone or water, or in a mixed solvent thereof, a base such as potassium carbonate or sodium hydroxide is added to Compound 2, and the mixture is reacted at 0°C to 50°C, and preferably at 10°C to 30°C, for 15 minutes to 4 hours, and preferably 0.5 hours to 2 hours, whereby Compound 3 can be obtained.

### Fourth Step

In a solvent such as DMF, THF, DMSO or water, or in a mixed solvent thereof, a ring C alkyl halide optionally substituted with R^{C} or R^{B} that is commercially available or prepared by a known method (e.g., benzyl bromide or benzyl chloride), and a base such as potassium carbonate or sodium hydride are added to Compound 3, and the mixture is reacted at 0°C to 60°C, and preferably at 10°C to 30°C, for 10 minutes to 12 hours, and preferably 0.5 hours to 4 hours, whereby Compound 4 can be obtained.

### Fifth Step

In a solvent such as dichloromethane, tetrachloromethane or water, or in a mixed solvent thereof, an oxidant such as a meta-chloroperbenzoic acid or sodium periodate, and a base such as potassium carbonate or sodium hydride are added to Compound 4, and the mixture is reacted at -20°C to 50°C, and preferably at 0°C to 30°C, for 1 hour to 12 hours, and preferably 2 hours to 6 hours, whereby Compound 5 can be obtained.

### Sixth Step

Compound 6 can be obtained from Compound 5 in the same manner as described above in the first step in "3) Synthesis of Compounds C-1 and C-2".

### Seventh Step

Compound 7 can be obtained from Compound 6 in the same manner as described above in the fourth step in "1) Synthesis of Compounds A-1 and A-2".

### 38) Synthesis of Compounds AH-1 and AH-2

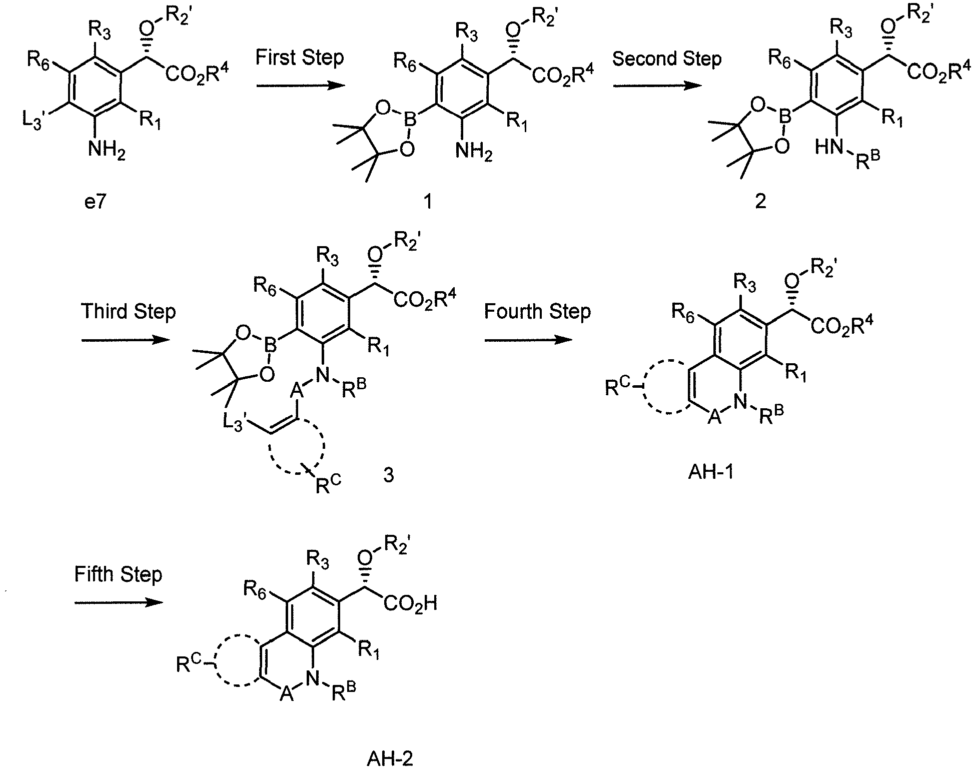

wherein each symbol has the same meaning as described above; preferably A is alkylene optionally substituted with R^{B} or the like.

### First Step

Compound 1 can be obtained from Compound e7 in the same manner as in the first step in "7) Synthesis of Compound G-2".

### Second Step

In a solvent such as dichloromethane, chloroform, THF, acetone or acetonitrile, a base such as pyridine, lutidine or triethylamine, and substituted sulfonyl chloride that is commercially available or synthesized by a known method are added to Compound 1, and the mixture is reacted at 0°C to 100°C, and preferably at 0°C to 20°C, for 0.5 hours to 24 hours, and preferably 0.5 hours to 5 hours, whereby Compound 2 can be obtained.

### Third Step

In a solvent such as DMF, DME, THF, acetone or acetonitrile, a base such as potassium carbonate, sodium carbonate, cesium carbonate or sodium hydride, and a ring C alkyl halide optionally substituted with R^{C} that is commercially available or prepared by a known method (e.g., picolyl halide) or the like are added to Compound 2, and the mixture is reacted at 0°C to 100°C, and preferably at 20°C to 50°C, for 0.5 hours to 24 hours, and preferably 1 hour to 5 hours, whereby Compound 3 can be obtained. Also, when R^{B} of Compound 2 is alkylsulfonyl or the like, in a solvent such as THF, toluene or dichloromethane, Mitsunibu reagent such as DEAD, DIAD or bis(2-methoxyethyl) azodicarboxylate, triphenylphosphine, tr-n-butyl phosphine or tributylphosphine, and benzyl alcohol optionally substituted with R^{C} that is commercially available or prepared by a known method are added to Compound 2, and the mixture is reacted at -20°C to 100°C, and preferably at 0°C to 30°C, for 0.5 hours to 24 hours, and preferably 1 hour to 5 hours, whereby Compound 3 can be obtained.

### Fourth Step

In a solvent such as dioxane, DMF, DME, tetrahydrofuran or water, or in a mixed solvent thereof, a palladium catalyst such as Pd(PPh₃)₄, Pd(OAc)₂, Pd(PPh₃)₂Cl₂, Pd(dppf)₂Cl₂ or Pd(dtbpf), and a base such as potassium carbonate, sodium carbonate, cesium carbonate or potassium phosphate are added to Compound 3, and the mixture is reacted under a nitrogen atmosphere at 0°C to 150°C, and preferably at 60°C to 120°C, for 0.5 hours to 24 hours, and preferably 1 hour to 12 hours, whereby Compound 4 can be obtained.

### Fifth Step

Compound 5 can be obtained from Compound 4 in the same manner as described above in the fourth step in "1) Synthesis of Compounds A-1 and A-2".

### 39) Synthesis of Compound (3)

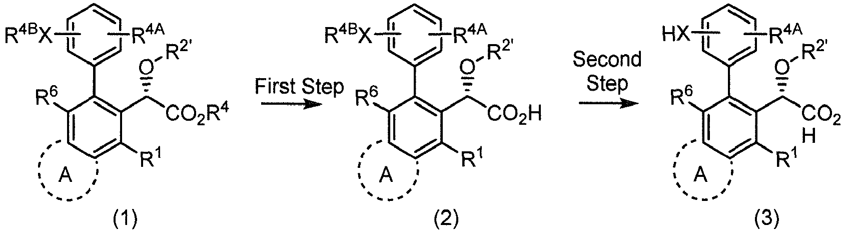

wherein R^{4A} is halogen; R^{4B} is alkyl, aralkyl or the like; X is O; dashlined portion is a ring A; the others have the same meaning as described above.

### First Step

Compound (2) can be obtained from Compound (1) in the same manner as described above in the third step in "1) Synthesis of Compounds A-1 and A-2".

### Second Step

Compound (3) can be obtained by the following method.

### (Method 1)

In a solvent such as methanol, ethanol, ethyl acetate or tetrahydrofuran, a catalyst such as 5% or 10% palladium carbon, palladium hydroxide or platinum dioxide is added to Compound (2), and the mixture is reacted under a hydrogen atmosphere and under 1 to 10 atmospheres, and preferably 1 to 3 atmospheres, at 0°C to 60°C, and preferably at 20°C to 40°C, for 0.1 hours to 48 hours, and preferably 1 hour to 12 hours, whereby Compound (3) can be obtained.

### (Method 2)

In a solvent such as methanol, ethanol, THF or DMSO, or in a mixed solvent thereof, a base such as potassium hydroxide, sodium hydroxide or lithium hydroxide is added to Compound (2), and the mixture is reacted at 0°C to 150°C, and preferably at 20°C to 100°C, for 0.1 hours to 24 hours, and preferably 0.5 hours to 6 hours, whereby Compound (3) can be obtained.

### (Method 3)

In a solvent such as THF, acetonitrile or hexamethyl phosphoric triamide, or in a mixed solvent thereof, a fluoride agent such as tetrabutylammonium fluoride or hydrogen fluoride is added to Compound (2), and the mixture is reacted at 0°C to 150°C, and preferably at 20°C to 80°C, for 0.5 hours to 3 hours, and preferably 0.5 hours to 3 hours, whereby Compound (3) can be obtained.

### (Method 4)

In a solvent such as acetonitrile, dichloromethane or toluene, a Lewis acid such as phosphorus tribromide, trimethylsilyl iodide or aluminum trichloride is added to Compound (2), the mixture is reacted at 0°C to 150°C, and preferably 20°C to 80°C, for 0.1 hours to 24 hours, and preferably 1 hour to 6 hours, whereby Compound (3) can be obtained.

### 40) Synthesis of Compound (4)

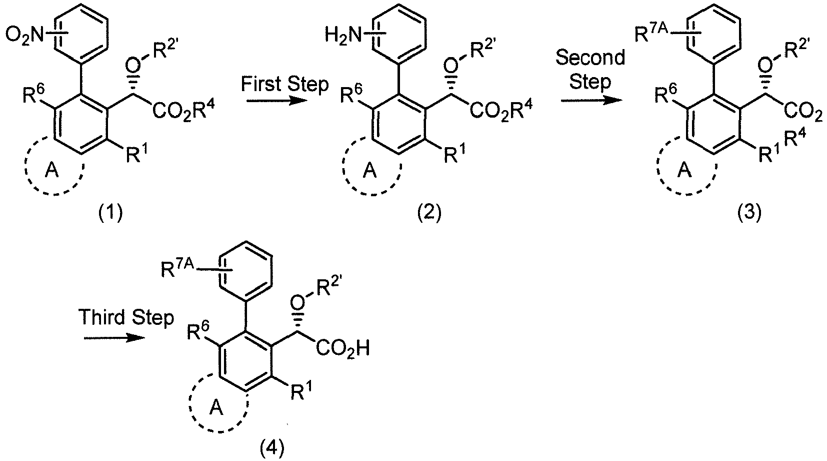

### First Step

Compound (2) can be obtained from Compound (1) in the same manner as described above in the second step in "2) Synthesis of Compounds B-1 and B-2".

### Second Step

In a solvent such as acetonitrile, THF or DMF, a diazotization reagent such as tert-butyl nitrite or isopentyl nitrite is added to Compound (2), and then a cupric halide such as cupric chloride (I), cupric bromide (I) or cupric iodide (I), or such as trimethylsilyl chloride or trimethylsilyl azide is sequentially added, and the mixture is reacted at 0°C to 80°C, and preferably at 20°C to 50°C, for 0.1 hours to 6 hours, and preferably 0.5 hours to 3 hours, whereby Compound (3) can be obtained. Compound (3) may be a mixture of two compounds (e.g., a compound wherein R^{7A} is H and a compound wherein R^{7A} is halogen).

### Third Step

Compound (4) can be obtained from Compound (3) in the same manner as described above in the fourth step in "1) Synthesis of Compounds A-1 and A-2". Compound (4) may be a mixture of two compounds (e.g., a compound wherein R^{7A} is H and a compound wherein K^{7A} is halogen), and each single compound can be obtained by purification such as column chromatography.

### 41) Synthesis of Compound (3)

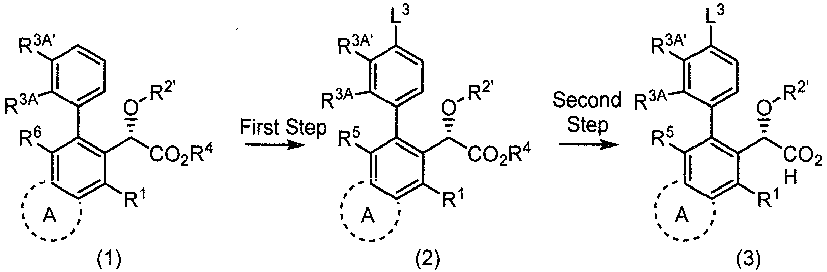

wherein L³ is halogen; R^{3A} is alkyl or the like; R^{3A'} is NH₂, OH or the like.

### First Step

Compound (2) can be obtained from Compound (1) in the same manner as described above in the ninth step in "4) Synthesis of Compounds D-1 and D-2".

### Second Step

Compound (3) can be obtained from Compound (2) in the same manner as described above in the fourth step in "1) Synthesis of Compounds A-1 and A-2".

The compound of the present invention has an inhibitory effect on HIV replication, thus is useful as a therapeutic agent and/or prophylactic agent of viral infections such as AIDS.

In HIV replication inhibition activity of the compound of the present invention, for example, in the following Experimental Example 1, preferably, EC50 value is 100nM or less, more preferably 50nM or less, more preferably 20nM or less, particularly preferably10nM less. EC90 value is also available in the evaluation of this activity. Also, preferred compound has strong virus mutations resistance. More preferred compound has high C24/EC50 value (C24: blood concentration after administration 24 hours).

Preferably, the compound of the present invention has any or all of excellent characteristics described below.
a) Has a weak inhibitory effect on CYP enzymes (e.g., CYP1A2, CYP2C9, CYP2C19, CYP2D6, CYP3A4, etc.).
b) Shows good pharmacokinetics such as high bioavailability and moderate clearance.
c) Has high metabolic stability.
d) Shows no irreversible inhibitory effect on a CYP enzyme (e.g., CYP3A4) within a concentration range in the measurement conditions described herein.
e) Has no mutagenicity.
f) Has low risk on the cardiovascular system.
g) Shows high solubility.
h) Shows strong efficacy also against resistant viruses.
i) Has high stability in aqueous solution.
j) Has high light stability and/or low or no phototoxicity.

When administering the pharmaceutical composition of the present invention, it can be administered in any method of orally and parenterally methods. For oral administration, the pharmaceutical composition may be prepared into a commonly used dosage form such as tablets, granules, powders and capsules, according to a conventional method, and administered. In parenteral administration, the pharmaceutical composition can be suitably administered in any commonly used dosage form such as injections. The compound of the present invention preferably has high oral absorbability, thus can be suitably used as an oral agent.

Various pharmaceutical additives such as excipients, binders, disintegrating agents and lubricants suitable for the dosage form can be mixed as necessary in an effective amount of the compound of the present invention, to make the compound into a pharmaceutical composition.

It is desirable that the dosage amount of the pharmaceutical composition of the present invention is set in consideration of the patient's age, weight, the type and degree of disease, the route of administration, and the like, and when orally administered to an adult, the dosage amount is normally in the range of 0.05 to 100 mg/kg/day, and preferably 0.1 to 10 mg/kg/day. In the case of parenteral administration, the dosage amount greatly varies depending on the route of administration, but is normally in the range of 0.005 to 10 mg/kg/day, and preferably 0.01 to 1 mg/kg/day. This dosage amount may be administered in once to several times a day.

The compound of the present invention can be used in combination with a reverse transcriptase inhibitor, a protease inhibitor, an integrase inhibitor, other anti-HIV drug, or the like (hereinafter, abbreviated as concomitant drug), for the purpose of enhancement of action of the compound, reduction of the dosage amount of the compound, or the like. At this time, the time of administration of the compound of the present invention and the concomitant drug is not limited, and, these may be administered simultaneously, or may be administered with a time difference, to the administration subject. Furthermore, the compound of the present invention and the concomitant drug may be administered as two types of preparations containing each active ingredient, or may be administered as a single preparation containing both active ingredients.

The dosage amount of the concomitant drug can be appropriately selected based on the clinically used dose. In addition, the blending ratio of the compound of the present invention to the concomitant drug can be appropriately selected depending on the administration subject, administration route, target disease, symptoms, combination and the like. For example, when the administration subject is a human, 0.01 to 100 parts by weight of the concomitant drug may be used, based on 1 part by weight of the compound of the present invention.

In addition, the compound of the present invention can be used, in the field of gene therapy, to prevent infection of retroviral vectors from spreading to other parts than the object tissues when using a retroviral vector based on HIV and MLV. In particular, when a vector is transmitted to cells and the like in a test tube and then returned to the body, by administering the compound of the present invention in advance, it is possible to prevent unnecessary infection in the body.

Examples of the reverse transcriptase inhibitor include AZT, 3TC, didanosine, zalcitabine, Sanirubujin, abacavir, tenofovir, emtricitabine, Nebirabin, efavirenz, capravirine, etravirine, delavirdine, and the like.

Examples of the protease inhibitor include indinavir, ritonavir, saquinavir, nelfinavir, amprenavir, Atanazabiru, lopinavir, fosamprenavir, darunavir, atazanavir, Burekanabiru, Tipuranabiru, and the like.

Examples of the integrase inhibitor include raltegravir, Erubitegurabiru, JTK-656, Dolutegravir (S-349572), S-265744, and the like.

Examples of other anti-HIV drugs include entry inhibitors such as maraviroc and Bikuribiroku, fusion inhibitors such as enfuvirtide, sifuvirtide, albuvirtide, and the like.

### EXAMPLES

Hereinbelow, the present invention will be described in more detail with reference to examples and reference examples of the present invention and test examples, but the present invention is not limited by these examples.

In addition, abbreviations used herein represent the following meanings.
- Ac:: acetyl
- n-Bu:: n-butyl
- t-Bu:: tert-butyl
- Bn:: benzyl
- DMA:: N,N-dimethylacetamide
- DME:: dimethoxyethane
- DMF:: N,N-dimethylformamide
- DCM:: dichloromethane
- DMSO:: dimethylsulfoxide
- dppf:: 1,1'-bis(diphenylphosphino)ferrocene
- dppp:: 1,3-bis(diphenylphosphino)propane
- dtbpf:: 1,1'-di-tert-butyl phosphinoferrocene
- Et:: ethyl
- Fmoc:: 9-fluorenylmethyloxycarbonyl
- Me:: methyl
- Ms:: methanesulfonyl
- NBS:: N-bromosuccinimide
- NCS:: N-chlorosuccinimide
- NIS:: N-iodosuccinimide
- Ph:: phenyl
- TBS:: tert-butyldimethylsilyl
- THF:: tetrahydrofuran
- Tf:: trifluoromethanesulfonyl
- TFA:: trifluoroacetic acid
- TMS:: trimethylsilyl
- Ts:: p-toluenesulfonyl

The NMR analysis obtained in each example was carried out in 300 MHz or 400 MHz, and was measured using DMSO-d₆, CDCl₃.

The term RT in the table represents a retention time at LC/MS: liquid chromatography/mass spectrometry, and was measured under the following conditions. For the compound that may exist as two isomers in the mobile phase, two measurement peaks may be obtained.

### (Measurement Conditions)

Column: ACQUITY UPLC (Registered trademark) BEH C18 (1.7µm i.d.2.1x50mm) (Waters)
Flow rate: 0.8 mL/min
UV detection wavelength: 254nm
Mobile phase: [A]: a 0.1% formic acid-containing aqueous solution, [B]: a 0.1% formic acid-containing acetonitrile solution
Gradient: a linear gradient of 5% to 100% solvent [B] was carried out in 3.5 minutes, and 100% solvent [B] was kept for 0.5 minutes.

Compounds described as *, was measured by the following measurement conditions.
Column: Shim-pack XR-ODS (2.2µm, i.d.50x3.0mm) (Shimadzu)
Flow rate: 1.6 mL/min
UV detection wavelength: 254nm
Mobile phase: [A]: a 0.1% formic acid-containing aqueous solution, [B]: a 0.1% formic acid-containing acetonitrile solution
Gradient: a linear gradient of 10% to 100% solvent [B] was carried out in 3 minutes, and 100% solvent [B] was kept for 0.5 minutes.

Compounds described as ▲, were measured by the following measurement conditions.
Column: Shim-pack XR-ODS (2.2µm, i.d.50x3.0mm) (Shimadzu)
Flow rate: 1.6 mL/min
UV detection wavelength: 254nm
Mobile phase: [A]: a 0.1% formic acid-containing aqueous solution, [B]: a 0.1% formic acid-containing acetonitrile solution
Gradient: a linear gradient of 10% to 100% solvent [B] was carried out in 8 minutes, and 100% solvent [B] was kept for 0.5 minutes.

Compounds described as *2, were measured by the following measurement conditions.
Column: ACQUITY UPLC (Registered trademark) BEH C18 (1.7µm i.d.2. 1x50mm)(Waters)
   Flow rate: 0.8 mL/min
   UV detection wavelength: 254nm
Mobile phase: [A]: a 0.1% formic acid-containing aqueous solution, [B]: a 0.1% formic acid-containing acetonitrile solution
Gradient: a linear gradient of 5% to 100% solvent [B] was carried out in 9.5 minutes, and 100% solvent [B] was kept for 0.5 minutes.

### Example 1 Synthesis of Compound 3

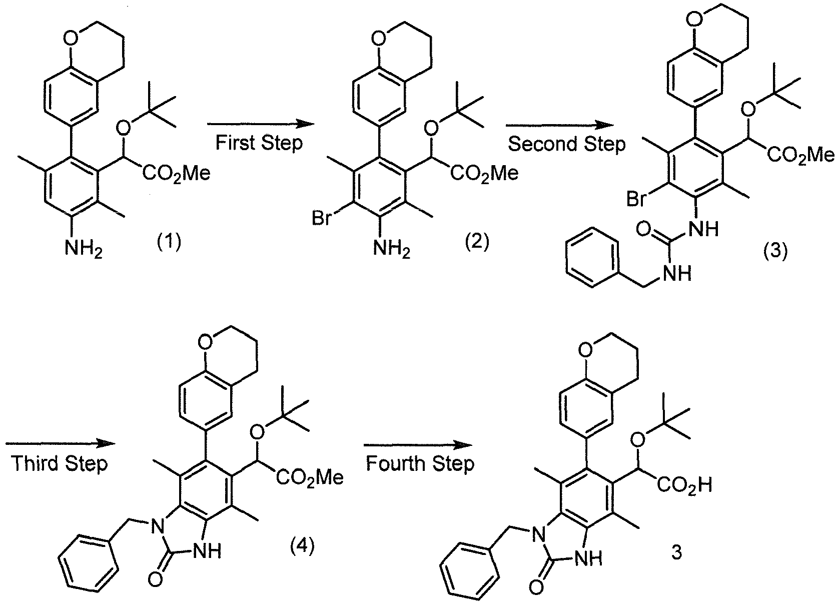

### First Step Synthesis of Compound (2)

Compound (1) (6.50 g, 16.0 mmoL) was dissolved in DMF (50.0 mL), and a solution of NBS (3.10 g, 17.6 mmoL) in DMF (10.0 mL) was added dropwise thereto at 0°C. After being stirred at same temperature for 10 minutes, the mixture was was poured into ice water and extracted with ethyl acetate. The obtained organic layer was washed with brine, dried over anhydrous sodium sulfate, and concentrated under reduced pressure. The obtained residue was purified by silica gel column chromatography (petroleum ether-ethyl acetate) to obtain Compound (2) (5.10 g, 65%) as a yellow solid.
¹ H NMR (CDCl₃) δ: 0.97 (s, 9H), 2.06-2.09 (m, 5H), 2.28 (s, 3H), 2.69-2.83 (m, 2H), 3.66 (d, 3H), 4.23 (s, 2H), 5.01 (d, 1H), 6.78-6.87 (m, 2H), 6.98 (m, 1H).
LC/MS (ESI): m/z = 476 [M+H]+.

### Second Step Synthesis of Compound (3)

Compound (2) (150 mg, 0.315 mmoL) was dissolved in dichloromethane (2.00 mL), and triethylamine (0.044 mL, 0.315 mmoL) was added thereto, and the mixture was cooled under ice bath. To the mixture was added triphosgene (37.4 mg, 0.126 mmoL), and the mixture was warmed up to room temperature and stirred for 15 minutes. The mixture was cooled under ice bath, and a solution of benzylamine (0.103 mL, 0.945 mmoL) in dichloromethane (2.00 mL) was added thereto, and the mixture was warmed up to room temperature and stirred for 225 minutes. The mixture was poured into the mixture of ice water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The obtained residue was purified by silica gel column chromatography (hexane-ethyl acetate) to obtain Compound (3) (200 mg, >100%) as a pale orange solid. ¹ H NMR (CDCl₃) δ: 0.92 (s, 9H), 1.98-2.08 (m, 2H), 2.12 (s, 3H), 2.39 (s, 3H), 2.63-2.84 (m, 2H), 3.58-3.61 (m, 3H), 4.08-4.24 (m, 2H), 4.41-4.47 (m, 2H), 4.63 (br s, 1H), 4.99-5.02 (m, 1H), 5.94 (s, 1H), 6.79-6.95 (m, 3H), 7.24-7.30 (m, 5H).

### Third Step Synthesis of Compound (4)

Compound (3) (192 mg, 0.315 mmoL), copper iodide (I) (90.0 mg, 0.473 mmoL) and N,N-diisopropylethylamine (0.110 mL, 0.630 mmoL) were suspended in DMA (2.50 mL), and the mixture was stirred in a sealed tube at 150°C for 135 minutes. Ice water and 2moL/L aqueous solution of hydrochloric acid were added thereto and the mixture was extracted with ethyl acetate. The obtained organic layer was washed with brine, dried over anhydrous sodium sulfate, and concentrated under reduced pressure. The obtained residue was purified by silica gel column chromatography (hexane-ethyl acetate) to obtain Compound (4) (145 mg, 87.1%) as a pale yellow foam.
¹H NMR (CDCl₃) δ: 0.98(s, 9H), 1.93-1.96 (m, 3H), 1.96-2.05 (m, 2H), 2.43 (s, 3H), 2.63-2.77 (m, 2H), 3.62-3.67 (m, 3H), 4.20-4.25 (m, 2H),4.40 (m, 1H), 5.00 (m, 1H), 5.25-5.37 (m, 2H), 6.73-6.99 (m, 3H), 7.18-7.32 (m, 5H), 9.00 (m, 1H).

### Fourth Step Synthesis of Compound 3

Compound (4) (140 mg, 0.265 mmoL) was dissolved in ethanol (2.65 mL), and 2moL/L aqueous solution of sodium hydroxide (0.662 mL, 1.32 mmoL) was added thereto, and the mixture was stirred at 100°C for 2 hours 50 minutes. The mixture was poured into ice water and obtained aqueous layer was adjusted with 2moL/L aqueous solution of hydrochloric acid to about pH=3 and extracted with ethyl acetate. The obtained organic layer was washed with brine, dried over anhydrous sodium sulfate, and concentrated under reduced pressure. The obtained residue was purified by silica gel column chromatography (chloroform-methanol) and dried at 60°C under reduced pressure to obtain Compound3 (110 mg, 80.7%) as a pale orange solid.
¹ H NMR (DMSO-d₆) δ: 0.88(s, 9H), 1.75-1.86 (m, 5H), 2.50-2.75 (m, 2H), 4.14 (s, 2H), 4.84 (m, 1H), 5.10-5.30 (m, 2H), 6.70-6.85 (m, 2H),6.88 (m, 1H), 7.10-7.40 (m, 5H), 11.02 (s, 1H), 12.35 (br s, 1H). MS(ESI) m/z: 515 [M + H]+

### Example 2 Synthesis of Compound 7

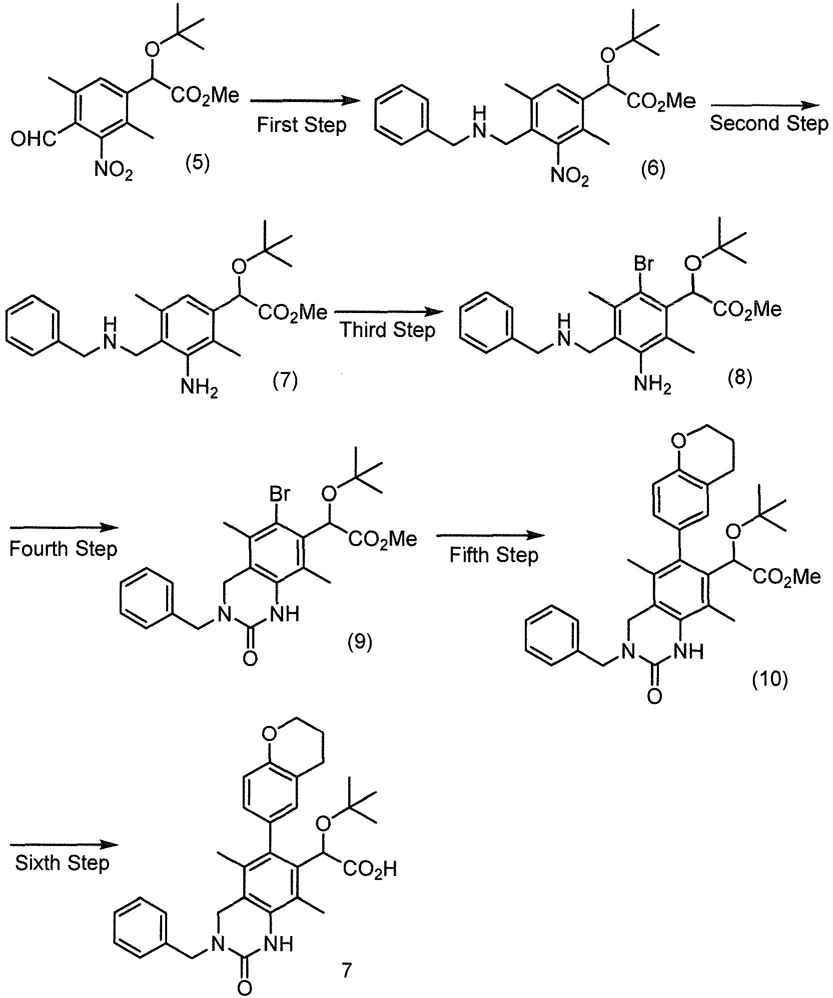

### First Step Synthesis of Compound (6)

Compound (5) (100 mg, 0.309 mmoL) was dissolved in dichloroethane (2 mL), acetic acid (0.0350 mL, 0.619 mmoL) and benzylamine (0.0340 mL, 0.309 mmoL) were added thereto, the mixutre was stirred at 60°C for 3 hours 35 minutes. The mixture was cooled at room temperature, and sodium triacetoxyborohydride (103 mg, 0.464 mmoL) was added thereto, and the mixture was stirred at room temperature for 16 hours 15 minutes. The mixture was poured into the mixture of ice water and a saturated aqueous solution of sodium hydrogen carbonate and extracted with ethyl acetate. The obtained organic layer was washed with brine, dried over anhydrous sodium sulfate, and concentrated under reduced pressure. The obtained residue was purified by silica gel column chromatography (hexane-ethyl acetate) to obtain Compound (6) (175 mg, >100%) as a pale yellow oil.
¹H NMR (CDCl₃) δ: 1.22 (s, 9H), 2.27 (s, 3H), 2.36 (s, 3H), 3.58 (s, 2H), 3.67 (s, 3H), 3.81 (s, 3H), 5.18 (s, 1H), 7.20-7.35 (m, 5H), 7.40 (s, 1H).

### Second Step Synthesis of Compound (7)

Compound (6) (128 mg, 0.309 mmoL) was dissolved in acetic acid (2.00 mL), zinc (202 mg, 3.09 mmoL) was added thereto, the mixture was stirred at 60°C for 2 hours 5 minutes. The mixture was poured into the mixture of ice water and an aqueous solution of sodium bicarbonate and extracted with ethyl acetate. The obtained organic layer was washed with brine, dried over anhydrous sodium sulfate, and concentrated under reduced pressure. The obtained residue was purified by silica gel column chromatography (hexane-ethyl acetate) to obtain Compound (7) (76 mg, 64.0%) as a colorless oil.
¹H NMR (DMSO-d₆) δ: 1.14 (s, 9H), 2.04 (s, 3H), 2.12 (s, 3H), 3.50-3.65 (m, 6H), 4.95-5.25 (m, 3H), 6.48 (s, 1H), 7.20-7.40 (m, 5H).

### Third Step Synthesis of Compound (8)

Compound (7) (361 mg, 0.939 mmoL) was dissolved in dichloromethane (4.00 mL), and the mixture was cooled under ice bath. To the mixture was added a solution of bromine (0.0480 mL, 0.939 mmoL) in dichloromethane (4.00 mL), the mixture was stirred at same temperature for 1 hour. The mixture was poured into the mixture of ice water and a saturated aqueous solution of hydrogen carbonate and extracted with ethyl acetate. The obtained organic layer was washed with brine, dried over anhydrous sodium sulfate, and concentrated under reduced pressure. The obtained residue was purified by silica gel column chromatography (hexane-ethyl acetate) to obtain Compound (8) (133 mg, 30.6%) as a colorless oil.
¹H NMR (DMSO-d₆) δ: 1.14 (s, 9H), 2.04 (s, 3H), 2.32 (s, 3H), 3.57 (s, 4H), 3.65 (s, 2H), 3.74 (s, 2H), 4.95-5.25 (m, 2H), 5.85 (s, 1H), 7.20-7.40 (m, 5H).

### Fourth Step Synthesis of Compound (9)

Compound (8) (165 mg, 0.356 mmoL) and carbonyldiimidazole (289 mg, 1.780 mmoL) were dissolved in dioxane (5.00 mL), N,N-diisopropylethylamine (0.622 mL, 3.56 mmoL) was added thereto, the mixture was stirred at 120°C for 14 hours 25 minutes. The mixture was poured into the mixture of ice water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The obtained residue was purified by silica gel column chromatography (hexane-ethyl acetate) to obtain Compound (9) (139 mg, 79.8%) as a white crystalline powder.
¹H NMR (CDCl₃) δ: 1.21 (s, 9H), 2.20 (s, 3H), 2.23 (s, 3H), 3.68 (s, 3H), 4.32 (s, 2H), 4.60-4.75 (m, 2H), 5.95 (s, 1H), 6.55 (s, 1H), 7.26-7.40 (m, 5H).

### Fifth Step Synthesis of Compound (10)

Compound (9) (136mg, 0.278mmoL) and 6-chromaneboronic acib (74.2 mg, 0.417 mmoL) were dissolved in dioxane (4.20 mL), 2moL/L aqueous solution of potassium carbonate (0.417 mL, 0.834 mmoL) was added thereto, the operation of degassing and nitrogen substitution was repeated three times. To the mixture was added PdCl₂ (dppf) (22.7 mg, 0.0280 mmoL), and again, repeated three times the operation of the degassing and nitrogen substitution, and the mixture was stirred at 100°C for 3 hours 45 minutes. The mixture was cooled to room temperature, poured into the mixture of ice water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The obtained residue was purified by silica gel column chromatography (hexane-ethyl acetate) to obtain Compound (10) (106 mg, 70.3%) as a pale yellow foam.
¹H NMR (CDCl₃) δ: 0.96 (s, 9H), 1.69-1.75 (m, 3H), 1.95-2.15 (m, 2H), 2.25 (s, 3H), 2.65-2.95 (m, 2H), 3.60-3.70 (m, 3H), 4.20-4.30 (m, 3H), 4.30 (s, 2H), 4.60-4.73 (m, 2H), 5.00 (m, 1H), 6.57 (s, 1H), 6.45-7.00 (m, 3H), 7.26-7.40 (m, 5H).

### Sixth Step Synthesis of Compound 7

Compound (10) (104 mg, 0.192 mmoL) was dissolved in ethanol (1.92 mL), 2moL/L aqueous solution of sodium hydroxide (0.479 mL, 0.958 mmoL) was added thereto, and the mixture was stirred at 100°C for 2 hours 35 minutes. The mixture was cooled to room temperature and poured into ice water, and obtained aqueous layer was adjusted with 2moL/L aqueous solution of hydrochloric acid to about pH=3 and extracted with ethyl acetate. The obtained organic layer was washed with brine, dried over anhydrous sodium sulfate, and concentrated under reduced pressure. The obtained residue was purified by silica gel column chromatography (chloroform-methanol) and dried at 60°C under reduced pressure to obtain Compound 7 (83 mg, 81.9%) as a pale yellow crystalline powder.
¹H NMR (DMSO-d₆) δ: 0.86 (s, 9H), 1.68 (s, 3H), 1.80-2.00 (m, 2H), 2.18 (s, 3H), 2.50-2.80 (m, 2H), 4.10-4.20 (m, 2H), 4.29 (s, 2H), 4.50-4.65 (m, 2H), 4.87 (m, 1H), 6.40-6.80 (m, 2H), 6.90 (m, 1H), 7.25-7.40 (m, 5H), 8.21 (s, 1H), 12.37 (br s, 1H).
MS(ESI) m/z:529 [M+H]+

### Example 3 Synthesis of Compound 16

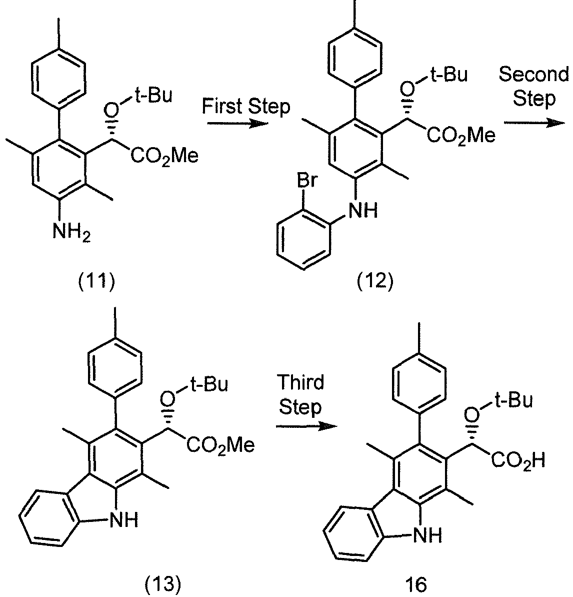

### First Step Synthesis of Compound (12)

Compound (11) (300 mg, 0.844 mmol) which was synthesized by the same manner as Compound (33), 1,2-dibromobenzene (199 mg, 0.844 mmol), xantphos (37 mg, 0.063 mmol), cesium carbonate (550 mg, 1.69 mmol) and Pd₂ (dba)₃ (39 mg, 0.042 mmol) were added to toluene (3 ml), and the mixture was heated to reflux for 18 hours under nitrogen atmosphere. The mixture was poured into water and extracted with ethyl acetate. The obtained organic layer was washed with brine, dried over anhydrous sodium sulfate, and concentrated under reduced pressure. The obtained residue was purified by silica gel column chromatography to obtain Compound (12) (282 mg, 66%) as a colorless foam.
MS: m/z = 510, 512 [M+H] ⁺

### Second Step Synthesis of Compound (13)

Compound (12) (276 mg, 0.541 mmol), palladium acetate (12 mg, 0.054 mmol), potassium carbonate (149 mg, 1.08 mmol), tricyclohexylphosphonium tetrafluoroborate (40 mg, 0.11 mmol) were added to dimethylacetamide (5 ml), and the mixture was stirred at 130°C for 2 hours under nitrogen atmosphere. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (13) (112 mg, 48%) as a pale yellow solid.
MS: m/z = 452 [M+Na] ⁺

### Third Step Synthesis of Compound 16

Compound (13) (109 mg, 0.254 mmol) was dissolved in the mixture of methanol (3 ml) and THF (3 ml), 2mol/L aqueous solution of sodium hydroxide (1.27 ml, 2.54 mmol) was added thereto, and the mixture was stirred at 80°C for 3 hours. The mixture was added to dilute hydrochloric acid and extracted with ethyl acetate. The obtained organic layer was dried over anhydrous sodium sulfate and concentrated under reduced pressure. The obtained residue was recrystallized from diisopropyl ether to obtain Compound 16 (78 mg, 74%).
¹H-NMR (DMSO-d₆) δ: 0.88 (s, 9H), 2.41 (s, 6H), 2.58 (s, 3H), 5.02 (s, 1H), 7.14 (m, 2H), 7.29 (m, 3H), 7.38 (t, J = 7.6 Hz, 1H), 7.54 (d, J = 8.0 Hz, 1H), 8.10 (d, J = 7.9 Hz, 1H), 11.13 (s, 1H).
MS: m/z = 438 [M+Na] ⁺

### Example 4 Synthesis of Compound 20

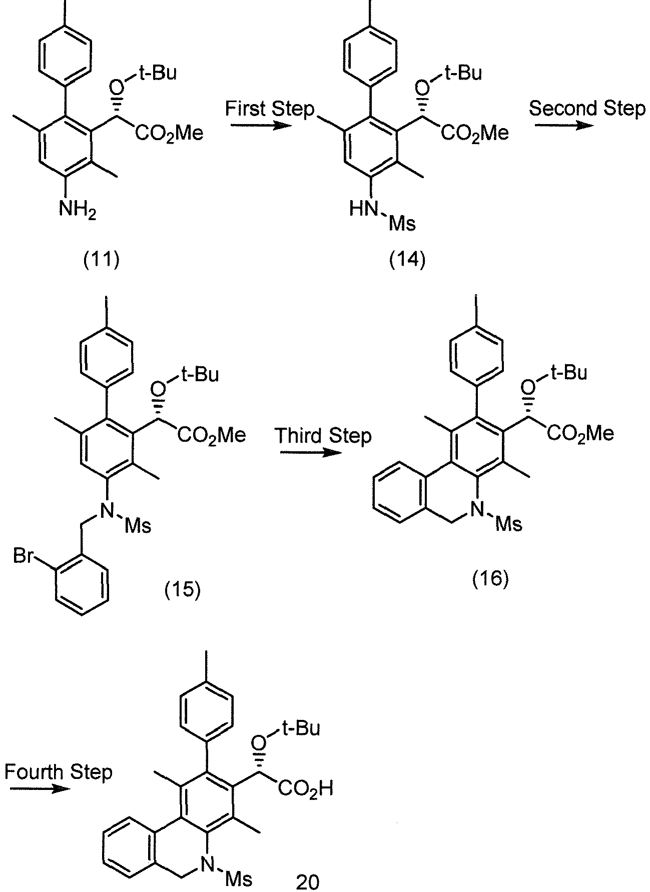

### First Step Synthesis of Compound (14)

Compound (11) (500 mg, 1.41 mmol) was dissolved in pyridine (5 ml), methanesulfonyl chloride (483 mg, 0.329 mmol) was added thereto at room temperature, and the mixture was stirred at room temperature for 18 hours. The mixture was poured into water and extracted with ethyl acetate. The obtained organic layer was washed with dilute hydrochloric acid, dried over anhydrous sodium sulfate, and concentrated under reduced pressure. The obtained residue was purified by silica gel column chromatography to obtain Compound (14) (538 mg, 88%) as a colorless foam.

### Second Step Synthesis of Compound (15)

Compound (14) (300 mg, 0.692 mmol) was dissolved in DMF (3 ml), 1-brome-2-(bromomethyl)benzene (190 mg, 0.761 mmol) and cesium carbonate (451 mg, 1.38 mmol) were added thereto at room temperature, and the mixture was stirred for 4 hours. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (15) (382 mg, 92%) as a pale yellow solid.
MS: m/z = 624, 626 [M+Na] +

### Third Step Synthesis of Compound (16)

Compound (15) (356 mg, 0.591 mmol), palladium acetate (13 mg, 0.059 mmol), potassium carbonate (163 mg, 1.18 mmol) and tricyclohexylphosphonium tetrafluoroborate (44 mg, 0.12 mmol) were added to dimethylacetamide (6.5 ml), and the mixture was stirred at 130°C for 2 hours under nitrogen atmosphere. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (16) (288 mg, 93%) as a pale yellow solid.
MS: m/z = 544 [M+Na] +

### Fourth Step Synthesis of Compound 20

Compound (16) (285 mg, 0.546 mmol) was dissolved in the mixture of methanol (6 ml) and THF (4 ml), 2mol/L aqueous solution of sodium hydroxide (2.73 ml, 5.46 mmol) was added thereto, and the mixture was stirred at 80°C for 3 hours. The mixture was poured into dilute hydrochloric acid and extracted with ethyl acetate. The obtained organic layer was dried over anhydrous sodium sulfate, and concentrated under reduced pressure. The obtained residue was recrystallized from diisopropyl ether-hexane to obtain Compound 20 (193 mg, 70%).
MS: m/z = 530 [M+Na] +

### Example 5 Synthesis of Compound 22

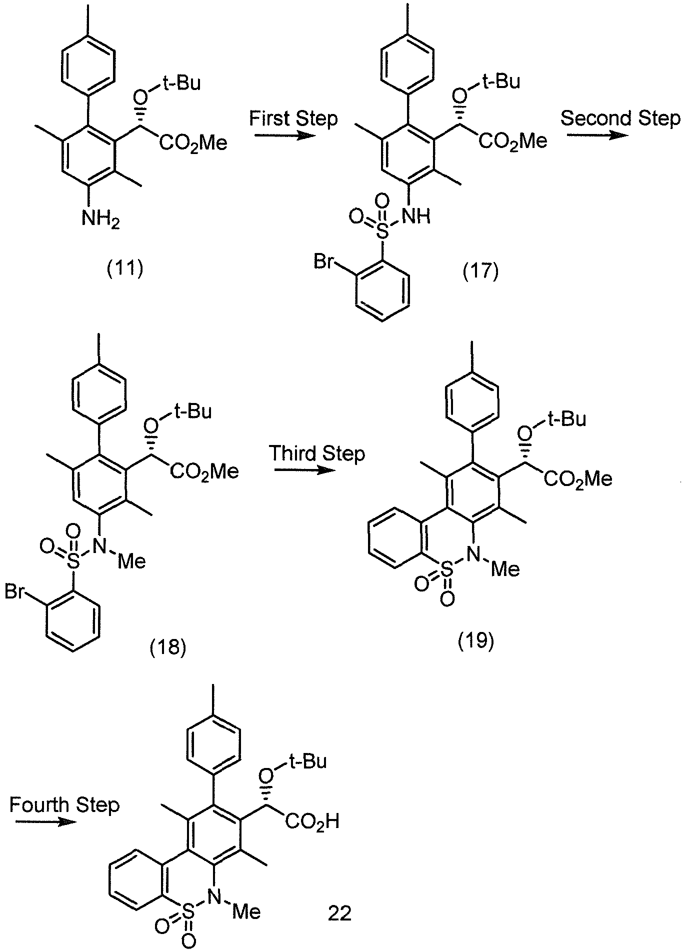

### First Step Synthesis of Compound (17)

Compound (11) (300 mg, 0.844 mmol) was dissolved in pyridine (5 ml), 2-bromobenzene-1-sulfonyl chloride (237 mg, 0.928 mmol) was added thereto at room temperature, and the mixture was stirred for 18 hours. The mixture was poured into water and extracted with ethyl acetate. The obtained organic layer was washed with dilute hydrochloric acid, dried over anhydrous sodium sulfate, and concentrated under reduced pressure. The obtained residue was purified by silica gel column chromatography to obtain Compound (17) (422 mg, 87%) as a colorless foam.
MS: m/z = 596, 598 [M+Na] +

### Second Step Synthesis of Compound (18)

Compound (17) (413 mg, 0.719 mmol) was dissolved in DMF (5 ml), iodomethane (255 mg, 0.112 mmol) and cesium carbonate (703 mg, 2.16 mmol) were added thereto at room temperature, and the mixture was stirred for 4 hours. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (18) (370 mg, 88%) as a colorless foam.
MS: m/z = 610, 612 [M+Na] +

### Third Step Synthesis of Compound (19)

Compound (18) (365 mg, 0.620 mmol), palladium acetate (14 mg, 0.062 mmol), potassium carbonate (171 mg, 1.24 mmol) and tricyclohexylphosphonium tetrafluoroborate (46 mg, 0.12 mmol) were added to dimethylacetamide (7 ml), and the mixture was stirred at 130°C for 4 hours under nitrogen atmosphere. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (19) (283 mg, 90%) as a pale yellow solid.
MS: m/z = 530 [M+Na] +

### Fourth Step Synthesis of Compound 22

Compound (19) (262 mg, 0.516 mmol) was dissolved in a mixture of methanol (4 ml) and THF (2 ml), 2mol/L aqueous solution of sodium hydroxide (2.58 ml, 5.16 mmol) was added thereto, and the mixture was stirred at 80°C for 3 hours. The mixture was poured into dilute hydrochloric acid and extracted with ethyl acetate. The obtained organic layer was dried over anhydrous sodium sulfate, and concentrated under reduced pressure. The obtained residue was recrystallized from diisopropyl ether-hexane to obtain Compound 22 (177 mg, 70%).
MS(*): m/z = 516 [M+Na] +

### Example 6 Synthesis of Compound 23

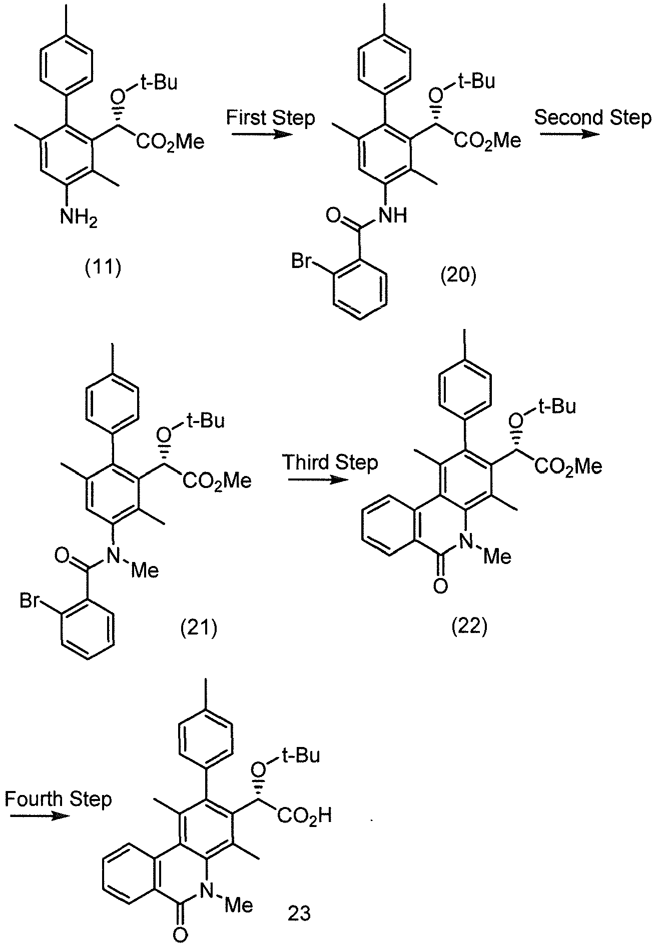

### First Step Synthesis of Compound (20)

Compound (11) (200 mg, 0.563 mmol) was dissolved in pyridine (3 ml), 2-bromobenzoyl chloride (136 mg, 0.619 mmol) was added thereto at room temperature, and the mixture was stirred for 18 hours. The mixture was poured into water and extracted with ethyl acetate. The obtained organic layer was washed with dilute hydrochloric acid, dried over anhydrous sodium sulfate, and concentrated under reduced pressure. The obtained residue was purified by silica gel column chromatography to obtain Compound(20)(250 mg, 83%) as a colorless foam.
MS: m/z = 560, 562 [M+Na] +

### Second Step Synthesis of Compound (21)

Compound (20) (243 mg, 0.451 mmol) was dissolved in DMF (2.5 ml), sodium hydride (60% in oil, 54.1 mg, 1.35 mmol) was added thereto at 0°C, and the mixture was stirred at room temperature for 10 minutes. Iodomethane (192 mg, 1.35 mmol) was added thereto at room temperature and the mixture was stirred foe 30 minutes. The mixture was poured into dilute hydrochloric acid and extracted with ethyl acetate. The obtained organic layer was washed with water, dried over anhydrous sodium sulfate, and concentrated under reduced pressure. The obtained residue was purified by silica gel column chromatography to obtain Compound (21) (216 mg, 87%) as a colorless foam.
MS: m/z = 552, 554 [M+H] +

### Third Step Synthesis of Compound (22)

Compound (21) (208 mg, 0.376 mmol), palladium acetate (8.5 mg, 0.038 mmol), potassium carbonate (104 mg, 0.753 mmol) and tricyclohexylphosphonium tetrafluoroborate (28 mg, 0.075 mmol) were added to dimethylacetamide (4 ml), and the mixture was stirred at 130°C for 4 hours under nitrogen atmosphere. The mixture was poured into water and extracted with ethyl acetate. The obtained organic layer was washed with water, dried over anhydrous sodium sulfate, and concentrated under reduced pressure. The obtained residue was purified by silica gel column chromatography to obtain Compound (22) (99 mg, 56%) as a pale yellow solid.
MS: m/z = 472 [M+H] +

### Fourth Step Synthesis of Compound 23

Compound (22) (95 mg, 0.201 mmol) was dissolved in a mixture of methanol (2 ml) and THF (1 ml), 2mol/L aqueous solution of sodium hydroxide (1.01 ml, 2.01 mmol) was added thereto, and the mixture was stirred at 80°C for 3 hours. The mixture was poured into dilute hydrochloric acid and extracted with ethyl acetate. The obtained organic layer was dried over anhydrous sodium sulfate, and concentrated under reduced pressure. The obtained residue was recrystallized from diisopropyl ether-hexane to obtain Compound 23 (65 mg, 71%).
1H-NMR (DMSO-D6) δ: 0.91 (s, 9H), 2.41 (s, 6H), 2.46 (s, 6), 3.53 (s, 3H), 4.96 (s, 1H), 7.25 (m, 2H), 7.34 (d, J = 8.0 Hz, 2H), 7.61 (t, J = 7.5 Hz, 1H), 7.74-7.78 (m, 1H), 8.26 (d, J = 8.3 Hz, 1H), 8.34 (dd, J = 7.8, 1.2 Hz, 1H), 12.65 (br s, 1H).
MS(*): m/z = 458 [M+H] +

### Example 7 Synthesis of Compound 21

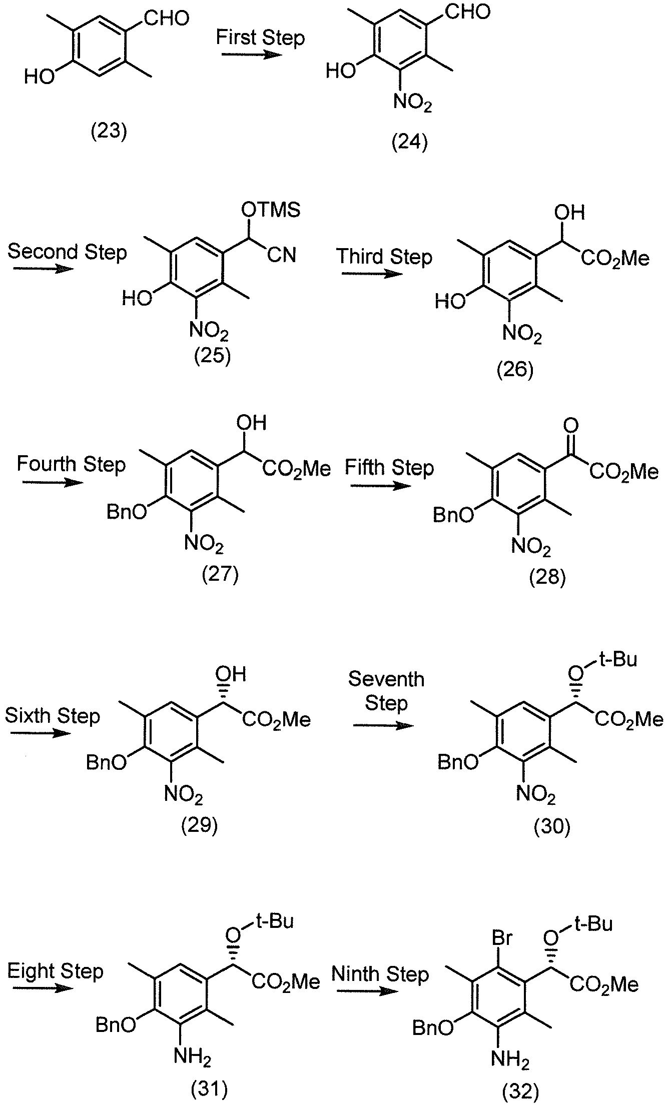

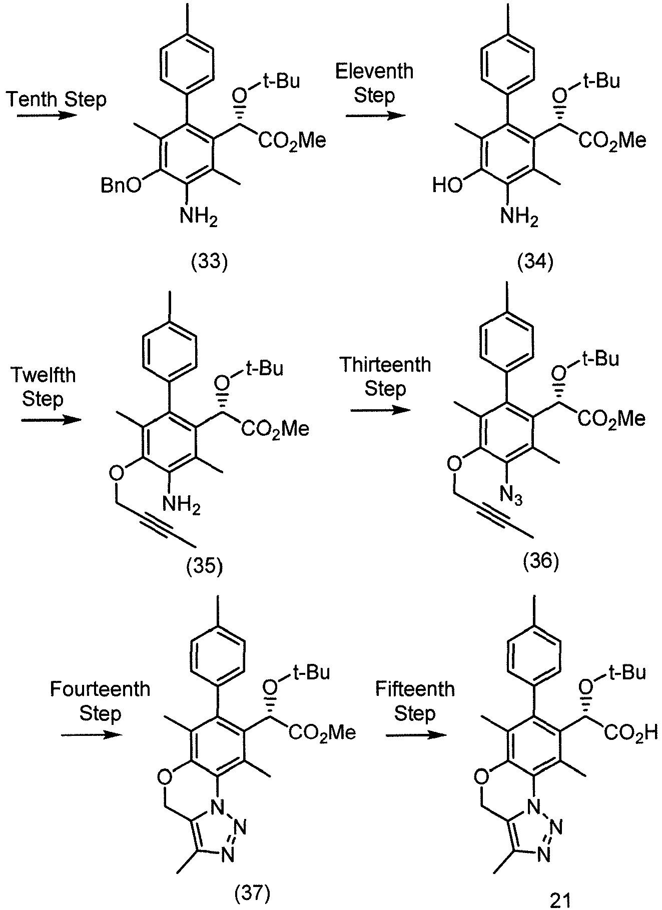

### First Step Synthesis of Compound (24)

To a solution of Compound (23) (25.0 g, 166 mmol) in sulfuric acid (350 mL) was added potassium nitrate (18.5 g, 183 mmol) under ice bath, and the mixture was stirred at 0°C for 1.5 hours. The mixture was poured into water and the presipitated solid was filtered to obtain Compound (24) (31.8 g, 98%) as a yellow solid.
MS(ESI) m/z: 194.30[M-H]-

### Second Step Synthesis of Compound (25)

To a solution of Compound (24) (25.0 g, 128 mmol) in dichloromethane (750 mL) were added zinc chloride (40.9 g, 128 mmol) and TMSCN (51.5 mL, 384 mmol) under ice bath, and the mixture was stirred at 0°C for 20 minutes. To the mixture were added a saturated aqueous solution of sodium bicarbonate (100 mL) and water (600 mL), and the mixture was extracted with dichloromethane (500 mL×3). The obtained organic layer was washed with water and brine, dried over sodium sulfate, and concentrated under reduced pressure to obtain a crude Compound (25) (47 g) as a brown oil.
MS(ESI) m/z: 292.95[M-H]-

### Third Step Synthesis of Compound (26)

To the crude Compound (25) (45.6 g) were added 5∼10% solution of hydrogen chloride in methanol (456 mL) and water (2.2 mL, 123 mmol), and the mixture was stirred under heat reflux for 20 hours. The mixture was concentrated under reduced pressure, water (300mL) was added thereto, and the mixture was extracted with ethyl acetate (300mL×3). The obtained organic layer was washed with water and brine, dried over sodium sulfate, and concentrated under reduced pressure. To the residue were added diisopropyl ether and hexane, and the presipitated solid was filtered to obtain Compound (26) (28.6 g, 91% in 2 steps) as a yellow solid.
¹H-NMR(CDCl₃, 400MHz)δ:2.28(s,3H), 2.50(s,3H), 3.41(s,1H), 3.78(s,3H), 5.37(s,1H), 7.33(s,1H), 9.48(s, 1H)
MS(ESI) m/z: 254.05[M-H]-

### Fourth Step Synthesis of Compound (27)

To a solution of Compound (26) (29.0 g, 114 mmol) in DMF (290 mL) were added potassium carbonate (31.4 g, 227 mmol) and benzylbromide (14.9 mL, 125 mmol), and the mixture was stirred at room temperature for 45 minutes. To the mixture were added 2mol/L aqueous solution of hydrochloric acid (300 mL) and water (300 mL), and the mixture was extracted with ethyl acetate (300 mL×3). The obtained organic layer was washed with water and brine, dried over sodium sulfate, and concentrated under reduced pressure. The residue was purified by silica gel column chromatography to obtain Compound (27) (38.0 g, 97%) as a yellow oil. ¹H-NMR(CDCl₃, 400MHz)δ:2.30(s,3H), 2.32(s,3H), 3.46(d, 1H,J=4.3Hz), 3.80(s,3H), 4.93(s,2H), 5.33(d,1H,J=4.3Hz), 7.30(s,1H), 7.37-7.44(m,5H)

### Fifth Step Synthesis of Compound (28)

To a solution of Compound (27) (38.0 g, 110 mmol) in dichloromethane (380 mL) was added mangane dioxide (47.8 g, 550 mmol), and the mixture was stirred under heat reflux for 2 hours. The mixture was filtered through Celite and the filtrate was concentrated to obtain Compound (28) (32.9 g, 87%) as a yellow solid.
¹ H-NMR(CDCl₃, 400MHz)δ:2.37(s,3H), 2.45(s,3H), 3.98(s,3H), 5.00(s,2H), 7.37-7.40(m,5H), 7.65(s,1H)

### Sixth Step Synthesis of Compound (29)

To a solution of Compound (28) (32.5 g, 95 mmol) in dichloromethane (325mL) were added 1M-(R)-CBS reagent (18.9 mL, 18.9 mmol) and catechol borane (20.2 mL, 189 mmol) at -78°C, and the mixture was stirred at -78°C for 20 minutes. To the mixture was added a saturated aqueous solution of ammonium chloride (100 mL), and the mixture was extracted with ethyl acetate (200mL×3). The obtained organic layer was washed with a saturated aqueous solution of sodium bicarbonate and brine, dried over sodium sulfate, and concentrated under reduced pressure. The residue was purified by silica gel column chromatography to obtain Compound (29) (24.8 g, 76%) as a yellow oil.
¹H-NMR(CDCl₃, 400MHz)δ:2.30(s,3H), 2.32(s,3H), 3.46(d,1H,J=4.3Hz), 3.80(s,3H), 4.93(s,2H), 5.33(d,1H,J=4.3Hz), 7.30(s,1H), 7.37-7.44(m,5H)

### Seventh Step Synthesisi of Compound (30)

To a solution of Compound (29) (24.5 g, 70.9 mmol) in tert-butyl acetate (245mL) was added perchloric acid (18.3 mL, 213 mmol) under ice bath, and the mixture was stirred at 0°C for 10 minutes. To the mixture was added a saturated aqueous solution of sodium bicarbonate (400mL), and the mixture was extracted with ethyl acetate (300 mL×3). The obtained organic layer was washed with water and brine, dried over sodium sulfate, and concentrated under reduced pressure. The residue was purified by silica gel column chromatography to obtain Compound (30) (21.2 g, 74%) as a yellow oil.
¹H-NMR(CDCl₃, 400MHz)δ:1.24(s,9H), 2.31(s,3H), 2.34(s,3H), 3.70(s,3H), 4.93(s,2H), 5.17(s,1H), 7.37-7.40(m,5H), 7.53(s,1H)

### Eighth Step Synthesis of Compound (31)

Compound (30) (38.8 g, 97.0 mmol) was dissolved in acetic acid (194mL), zinc (25.3g, 386mmol) was added thereto at room temperature, and the mixture was stirred at 60°C for 2.5hours. The mixture was concentrated, a saturated aqueous solution of sodium hydrogen carbonate (500mL) was added thereto, and the mixture was extracted with ethyl acetate (1000mL). The obtained organic layer was washed with a saturated aqueous solution of sodium hydrogen carbonate (500 mL) and brine (30 mL), dried over anhydrous magnesium sulfate to obtain a crude Compound (31) (36.0 g) as a brown oil.
MS: m/z = 372.2 [M+H] ⁺

### Ninth Step Synthesis of Compound (32)

Compound (31) (36.0 g, 97.0 mmol) was dissolved in acetonitrile (180mL), NBS (16.7 g, 94.0 mmol) was added under ice bath, and the mixture was stirred for 1.5 hours. Water (500 mL) was added thereto and the mixture was extracted with ethyl acetate (1000 mL). The obtained organic layer was washed with brine (300mL) and dried over anhydrous magnesium sulfate to obtain a crude Compound (32) (44.0 g) as a brown oil.
MS: m/z = 450.2, 452.2 [M+H] ⁺

### Tenth Step Synthesis of Compound (33)

Compound (32) (41.9g, 93.0mmol) was dissolved in a mixture of DMA (210 mL) and water (21 mL), 4-methylphenyl boronic acid (17.7 g, 130 mmol), [1,1'-bis(di-tert-butylphosphino)ferrocene] palladium dichloride (6.06 g, 9.30 mmol) and potassium carbonate (25.7 g, 186 mmol) were added thereto, and the mixture was stirred at 130°C for 2 hours under nitrogen atmosphere. Water (400 mL) was added thereto and the mixture was extracted with ethyl acetate (1000 mL). The obtained organic layer was washed with water (500mL×2) and brine (500mL), dried over anhydrous magnesium sulfate, and concentrated under reduced pressure. The residue was purified by silica gel column chromatography (hexane-ethyl acetate) to obtain Compound (33) (15.1 g, 33% in 3 steps) as a brown foam.
¹H-NMR(CDCl₃, 400MHz)δ:0.96(s,9H), 1.91(s,3H), 2.25(s,3H), 2.41(s,3H), 3.67(s,3H), 3.89(s,2H), 4.84(d,J=11.3Hz,1H), 5.00(s,1H), 7.06(d,J=7.8Hz,1H), 7.18(d,J=7.8Hz,1H), 7.21(s,2H), 7.31-7.43(m,3H), 7.39(d,J=7.4Hz,1H)
MS: m/z = 462.2 [M+H] ⁺

### Eleventh Step Synthesis of Compound (34)

To a solution of Compound (33) (14.6 g, 31.7 mmol) in methanol (146 mL) was added palladium hydroxide (4.45g, 20%wt, 50%wet), and the mixture was stirred under a hydrogen atmosphere at room temperature for 3 hours. The mixture was filtered through Celite, and the filtrate was concentrated. The residue was purified by silica gel column chromatography (hexane-ethyl acetate) to obtain Compound (34) (4.38 g, 37%) as a colorless solid.
MS: m/z = 372.3 [M+H] ⁺

### Twelfth Step Synthesis of Compound (35)

Compound (34) (300 mg, 0.808 mmol) was dissolved in DMF (6 mL), and sodium hydride (35.5 mg, 60%Wt, 0.888 mmol) was added thereto under ice bath, and the mixture was stirred for 10 minutes. 1-bromo-2-butyne (0.081 mL, 0.808 mmol)was added thereto under ice bath, and the mixture was stirred for 45 minutes. A saturated aqueous solution of ammonium chloride (3 mL) and water (50 mL) were added thereto, and the mixture was extracted with ethyl acetate (100 mL). The obtained organic layer was washed with water (50 mL×2) and brine(50mL), dried over anhydrous magnesium sulfate, and concentrated under reduced pressure. The residue was purified by silica gel column chromatography (hexane-ethyl acetate) to obtain Compound (35) (243 mg, 71%) as a yellow solid.
¹H-NMR(CDCl₃, 400MHz)δ:0.95(s,9H), 1.87-1.91(m,6H), 2.23(s,3H), 2.40(s,3H), 3.66(s,3H), 3.88(s,2H), 4.42-4.47(m,2H), 4.97(s,1H), 7.04(d,J=7.8Hz,1H), 7.14-7.23(m,3H)
MS: m/z = 424.4 [M+H] ⁺

### Thirteenth Step Synthesis of Compound (36)

Compound (35) (232 mg, 0.548 mmol) was dissolved in acetonitrile (4.54 mL), and tert-butyl nitrite (0.099 mL, 0.822 mmol) and trimethylsilyl azide (0.087 mL, 0.657 mmol) were added thereto under ice bath, and the mixture was stirred for 30 minutes. The mixture was concentrated under reduced pressure. The residue was purified by silica gel column chromatography (hexane-ethyl acetate) to obtain Compound (36) (242 mg, 98%) as a colorless foam.
¹ H-NMR(CDCl₃, 400MHz)δ:0.94(s,9H), 1.87(t,J=2.3Hz,3H), 1.92(s,3H), 2.30(s,3H), 2.41(s,3H), 3.68(s,3H), 4.53-4.57(m,2H), 4.96(s,1H), 7.03(dd,J=7.8,1.8Hz,1H), 7.13-7.25(m,3H)
MS: m/z = 472.3 [M+Na] ⁺

### Fourteenth Step Synthesis of Compound (37)

Compound (36) (253 mg, 0.563 mmol) was dissolved in toluene (5mL), and the mixture was stirred under heat reflux for 45 minutes. The mixture was concentrated to obtain Compound (37) (241 mg, 95%) as a colorless foam.
¹H-NMR(CDCl₃, 400MHz)δ:0.97(s,9H), 1.91(s,3H), 2.40(s,3H), 2.43(s,3H), 2.83(s,3H), 3.72(s,2H), 5.08(s,1H), 5.15(d,J=13.3Hz,1H), 5.22(d,J=13.3Hz,1H), 7.07(dd,J=7.8,1.8Hz, 1H), 7.15-7.28(m,3H)
MS: m/z = 450.3 [M+H] ⁺

### Fifteenth Step Synthesis of Compound 21

Compound (37) (240 mg, 0.534 mmol) was dissolved in ethanol (5.34 mL), and 2mol/L aqueous solution of sodium hydroxide (1.34 mL, 2.67 mmol) was added thereto, and the mixture was stirred under heat reflux for 30 minutes. 1mol/L aqueous solution of hydrochloric acid (2.65 mL) and brine (30 mL) were added thereto, the mixture was extracted with ethyl acetate (50 mL). The obtained organic layer was washed with brine (30 mL), dried over anhydrous magnesium sulfate, and concentrated under reduced pressure. The residue was purified by silica gel column chromatography (chloroform-methanol) to obtain Compound 21 (217 mg, 93%) as a colorless foam.
¹H-NMR(CDCl₃, 400MHz)δ:1.01(s,9H), 1.93(s,3H), 2.41(s,3H), 2.42(s,3H), 2.82(s,3H), 5.10(d,J=13.3Hz,1H), 5.21(s,1H), 5.29(d,J=13.3Hz,1H), 7.08(d,J=7.7Hz,1H), 7.22-7.27(m,2H), 7.41(d,J=7.7Hz,1H)
MS: m/z = 436.4 [M+H] ⁺

### Example 8 Synthesis of Compound 12

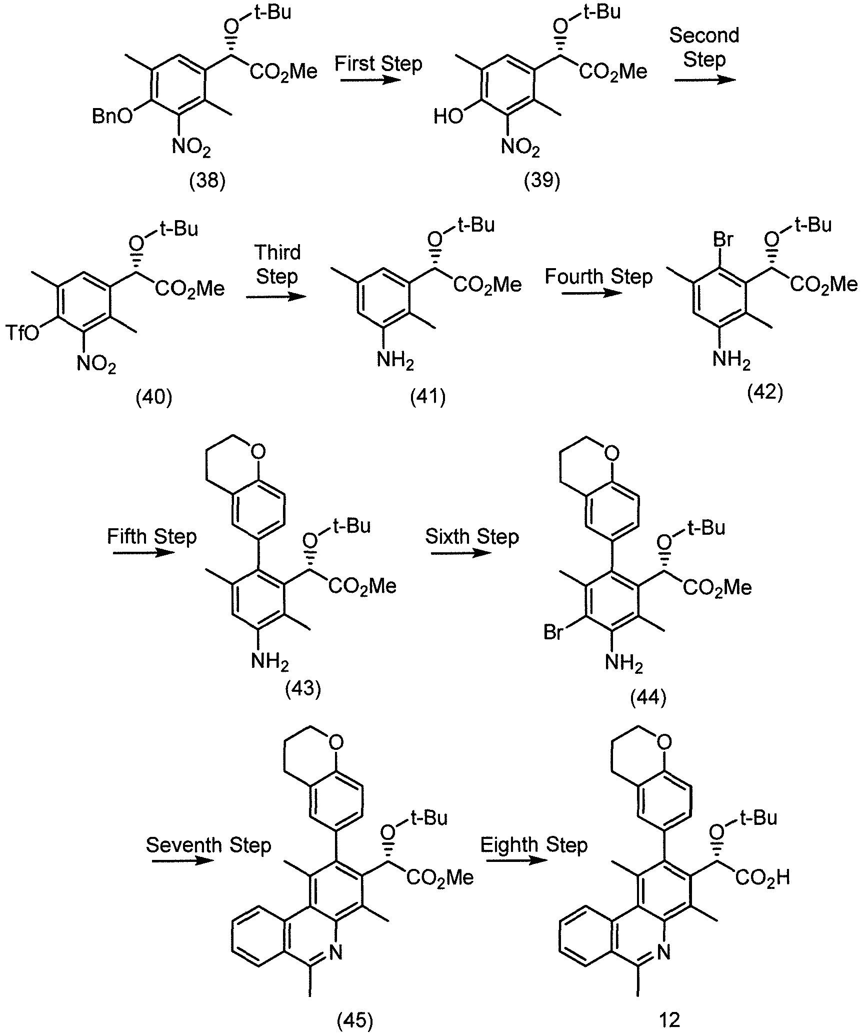

### First Step Synthesis of Compound (39)

To a solution of Compound (38) (21.2 g, 52.8 mmol) in ethyl acetate (212 mL) was added Pd/C (2.12 g, 10%wt, 50%wet), and the mixture was stirred under a hydrogen atmosphere at room temperature for 6 hours. The mixture was filtered through Celite, and the filtrate was concentrated under reduced pressure. The residue was purified by silica gel column chromatography to obtain Compound (39) (16.2 g, 99%) as a yellow oil.
¹H-NMR(CDCl₃, 400MHz)δ:1.23(s,9H), 2.29(s,3H), 2.51(s,3H), 3.68(s,3H), 5.22(s,1H), 7.60(s, 1H), 9.51(s, 1H)
MS(ESI) m/z: 315.35[M-H]-

### Second Step Synthesis of Compound (40)

To a solution of Compound (39) (16.2 g, 52 mmol) in dichloromethane (160 mL) were added pyridine (8.42 mL, 104 mmol) and trifluoromethansulfonic anhydride (13.2 mL, 78 mmol) under ice bath, and the mixture was stirred at 0°C for 10 minutes. A saturated aqueous solution of ammonium chloride (100 mL) was added thereto, and the mixture was extracted with dichloromethane (100 mL×2). The obtained organic layer was washed with water and brine, dried over sodium sulfate, and concentrated under reduced pressure. The residue was purified by silica gel column chromatography to obtain Compound (40) (21.2g, 92%) as a pale pink solid.

### Third Step Synthesis of Compound (41)

Compound (40) (10.0 g, 22.6 mmol) was dissolved in ethanol (150 ml), and 10%Pd(OH)₂/ C (3.17 g, 2.26 mmol) and triethylamine (3.13 ml, 22.6 mmol) were added thereto, and the mixture was stirred under a hydrogen atmosphere under 5 atmospheres at room temperature for 18 hours. The mixture was filtered through Celite and the filtrate was concentrated under reduced pressure. The residue was purified by silica gel column chromatography (hexane-ethyl acetate) to obtain Compound (41) (5.02 g, 84%) as a white solid.
¹H-NMR (CDCl₃) δ=1.25(s,9H), 2.17(s,3H), 2.23(s,3H), 3.55(br s,2H), 3.66(s,3H), 5.20(s,1H), 6.47(s,1H), 6.81(s, H).

### Fourth Step Synthesis of Compound (42)

Compound (41) (5.02 g, 18.9 mmol) was dissolved in acetonitrile (60 ml), and N-bromosuccinimide (3.40g, 18.9mmol) was added thereto at 0°C, and the mixture was stirred for 2 hours. The mixture was poured into water and extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (42) (4.95 g, 76%) as a white solid.
¹H-NMR (CDCl₃) δ:1.25(s,9H), 2.18(s,3H), 2.34(s,3H), 3.57(br s,2H), 3.67(s,3H), 5.95(s,1H), 6.57(s,1H).

### Fifth Step Synthesis of Compound (43)

Compound (42) (4.80g, 13.9mmol) was dissolved in a mixutre of DMA (24 mL) and water (2.4 mL), and 2-(chroman-6-yl)-4,4,5,5-tetramethyl-1,3,2-dioxaborolane (5.44 g, 20.9 mmol), [1,1'-bis(di-tert-butylphosphine)ferrocene] palladium dichloride (1.82 g, 2.79 mmol) and potassium carbonate (3.85g, 2.79mmol) were added thereto, and the mixture was stirred under a nitrogen atmosphere at 130°C for 1.5 hours. Water (200 mL) was added thereto and the mixture was extracted with ethyl acetate (200 mL). The obtained organic layer was washed with water (150 mL×2) and brine (100 mL), dried over anhydrous magnesium sulfate, and concentrated under reduced pressure. The residue was purified by silica gel column chromatography (hexane-ethyl acetate) to obtain Compound (43) (5.04 g, 91%) as a brown foam.
MS: m/z = 398.3 [M+H] ⁺

### Sixth Step Synthesis of Compound (44)

Compound (43) (1.73 g, 4.35 mmol) was dissolved in acetonitrile (17.3 mL), and NBS (775 mg, 4.35 mmol) was added thereto under ice bath, and the mixture was stirred under ice bath for 1 hour. Water (50 mL) was added thereto and the mixture was extracted with ethyl acetate (150 mL). The obtained organic layer was washed with brine (50mL), dried over anhydrous magnesium sulfate, and concentrated under reduced pressure. The residue was purified by silica gel column chromatography (hexane-ethyl acetate) to obtain Compound (44) (1.91g, 92%) as a colorless foam.
MS: m/z = 476.2, 478.2 [M+H] ⁺

### Seventh Step Synthesis of Compound (45)

Compound (44) (100mg, 0.210mmol) was dissolved in a solution of DMA (0.5 mL) and water (0.05 mL), and 2-acetyl phenyl boronic acid (51.6 mg, 0.315 mmol), [1,1'-bis(di-tert-butylphosphino)ferrocene] palladium dichloride (27.4 mg, 0.042 mmol) and potassium carbonate (58.0 mg, 0.420 mmol) were added thereto, and the mixture was stirred in a sealed tube at 130°C for 1.5 hours. Water (50mL) was added thereto and the mixture was extracted with ethyl acetate (50 mL). The obtained organic layer was washed with water (50 mL×2) and brine (50 mL), dried over anhydrous magnesium sulfate, and concentrated under reduced pressure. The residue was purified by silica gel column chromatography (hexane-ethyl acetate) to obtain Compound (45) (40.4 mg, 39%) as a brown foam.
MS: m/z = 498.2 [M+H] ⁺

### Eighth Step Synthesis of Compound 12

Compound (45) (40.0 mg, 0.080 mmol) was dissolved in ethanol (0.8 mL), and 2mol/L aqueous solution of sodium hydroxide (0.2 mL, 0.400 mmol) was added thereto, and the mixture was stirred under heat reflux for 1.5 hours. 1mol/L aqueous solution of hydrochloric acid (30 mL) was added thereto and the mixture was extracted with ethyl acetate (50 mL). The obtained organic layer was washed with brine (30 mL), dried over anhydrous magnesium sulfate, and concentrated under reduced pressure. The residue was purified by silica gel column chromatography (chloroform-methanol) to obtain Compound 12 (36.9 mg, 95%) as a brown foam.
MS: m/z = 484.0 [M+H] ⁺

### Example 9 Synthesis of Compound 8

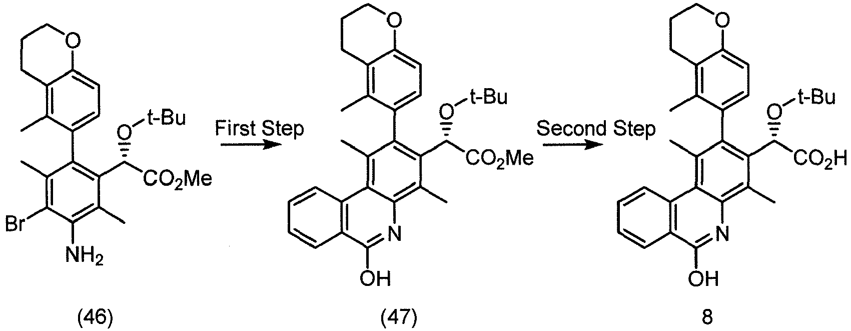

### First Step Synthesis of Compound (47)

Compound (46) (69.6 mg, 0.142 mmol) which was synthesized by the same manner as Compound (44), was dissolved in a mixture of DMA (0.5 mL) and water (0.05 mL), and 2-(4,4,5,5-tetramethyl-1,3,2-dioxaborolan-2-yl) ethyl benzoate (59.0 mg, 0.213 mmol), [1,1'-bis(di-tert-butylphosphino)ferrocenel palladium dichloride (18.5 mg, 0.029 mmol) and potassium carbonate (39.0 mg, 0.284 mmol) were added thereto, and the mixture was stirred in a sealed tube at 130°C for 2 hours. Water (50 mL) was added thereto, and the mixture was extracted with ethyl acetate (50 mL). The obtained organic layer was washed with water (50 mL×2) and brine (50 mL), dried over anhydrous magnesium sulfate, and concentrated under reduced pressure. The residue was purified by silica gel column chromatography (hexane-ethyl acetate) to obtain Compound (47) (29.6 mg, 41%) as a brown foam.
MS: m/z = 514.3 [M+H] ⁺

### Second Step Synthesis of Compound 8

Compound (47) (29.0 mg, 0.056 mmol) was dissolved in ethanol (1mL), and 2mol/L aqueous solution of sodium hydroxide (0.25 mL, 0.500 mmol) was added thereto at room temperature, and the mixture was stirred under heat reflux for 1.5 hours. 1mol/L aqueous solution of hydrochloric acid (3 mL) and brine (30 mL) were added thereto, and the mixture was extracted with ethyl acetate (30 mL). The obtained organic layer was dried over anhydrous magnesium sulfate, and concentrated under reduced pressure. The residue was purified by silica gel column chromatography (chloroform-methanol) to obtain Compound 8 (28.0 mg, 99%) as a colorless foam.
MS: m/z = 500.3 [M+H] ⁺

### Example 10 Synthesis of Compound 9

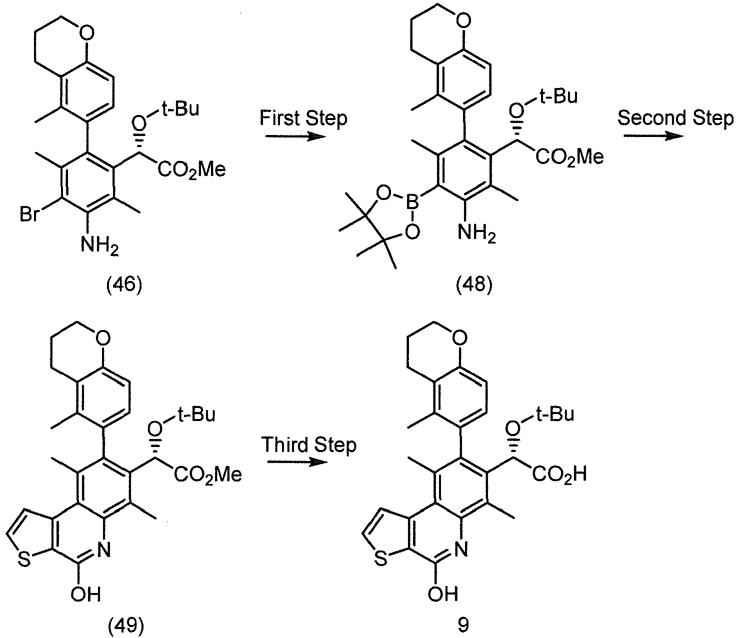

### First Step Synthesis of Compound (48)

Compound (46) (1.51 g, 3.08 mmol) was dissolved in DMF (11.2 mL), and bis(pinacolato)diboron (3.91 g, 15.4 mmol), [1,1'-bis(diphenylphosphino)ferrocene] palladium dichloride dichloromethane complex (336 mg, 0.411 mmol) and potassium acetate (1.51 g, 15.4 mmol) were added thereto, and the mixture was stirred in a sealed tube at 110°C for 17 hours. Water (100 mL) was added thereto, and the mixture was extracted with ethyl acetate (100 mL). The obtained organic layer was washed with water (100 mL×2) and brine (100 mL), dried over anhydrous magnesium sulfate, and concentrated under reduced pressure. The residue was purified by silica gel column chromatography (hexane-ethyl acetate) to obtain Compound (48) (1.12 g, 68%) as a yellow solid.
MS: m/z = 538.4 [M+H] ⁺

### Second Step Synthesis of Compound (49)

Compound (48) (116 mg, 0.216 mmol) was dissolved in a solution of DMA (0.75mL) and water (0.075 mL), and 3-iodo-thiophene-2-carboxylic acid methyl ester (87.1 mg, 0.324 mmol), [1,1'-bis(di-tert-butylphosphino)ferrocene] palladium dichloride (39.4mg, 0.060mmol) and potassium carbonate (60.0 mg, 0.144 mmol) were added thereto, and the mixture was stirred in a sealed tube at 130°C for 2 hours. Water (50 mL) was added thereto, and the mixture was extracted with ethyl acetate (50 mL). The obtained organic layer was washed with water (50 mL×2) and brine (50 mL), dried over anhydrous magnesium sulfate, and concentrated under reduced pressure. The residue was dissolved in a solution of ethyl acetate (3 mL) and acetic acid (0.125 mL), and the mixture was stirred at room temperature for 17 hours. A saturated aqueous solution of sodium hydrogen carbonate (30 mL) was added thereto, and the mixture was extracted with ethyl acetate (50 mL). The obtained organic layer was washed with brine (30 mL), dried over anhydrous magnesium sulfate, and concentrated under reduced pressure. The residue was purified by silica gel column chromatography (hexane-ethyl acetate) to obtain Compound (49) (80.3 mg, 73%) as a colorless solid.
MS: m/z = 520.3 [M+H] ⁺

### Third Step Synthesis of Compound 9

Compound (49) (80.0 mg, 0.154 mmol) was dissolved in ethanol (1.54 mL), and 2mol/L aqueous solution of sodium hydroxide (0.385 mL, 0.770 mmol) was added thereto, and the mixture was stirred under heat reflux for 2 hours. 1mol/L aqueous solution of hydrochloric acid (30 mL) was added thereto, and the mixture was extracted with ethyl acetate (50 mL). The obtained organic layer was washed with brine (30 mL), dried over anhydrous magnesium sulfate, and concentrated under reduced pressure. The residue was purified by silica gel column chromatography (chloroform-methanol) to obtain Compound 3 (62.2 mg, 80%) as a colorless solid.
MS: m/z = 506.2 [M+H] ⁺

### Example 11 Synthesis of Compound 10

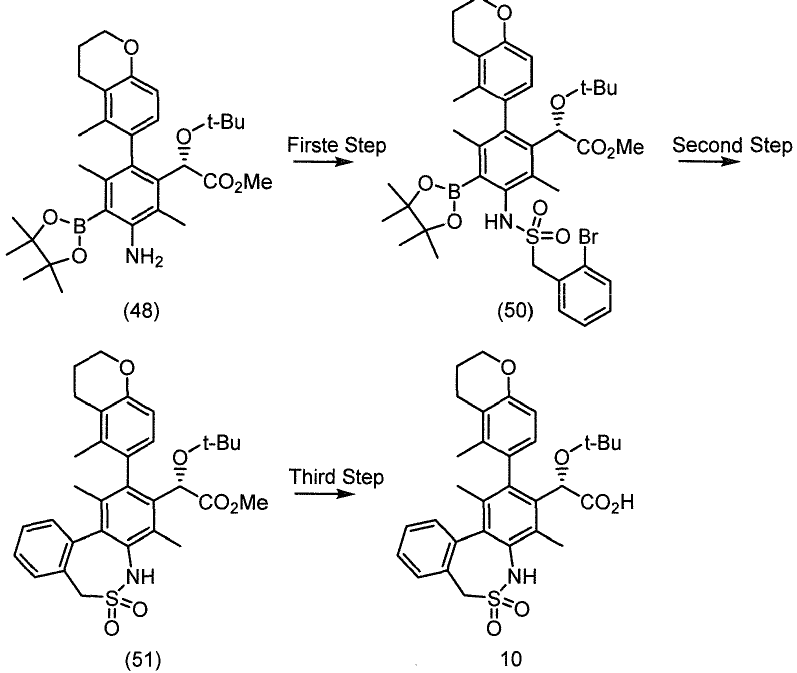

### First Step Synthesis of Compound (50)

Compound (48) (232 mg, 0.432 mmol) was dissolved in pyridine (3 mL), and the mixture was heated at 100°C, and 2-bromobenzyl sulfonyl chloride (349 mg, 1.30 mmol) was added thereto, and the mixture was stirred at 100°C for 1 hour. The mixture was cooled to room temperature, and water (25 mL) was added thereto, and the mixture was extracted with ethyl acetate (50 mL). The obtained organic layer was washed with 1mol/L aqueous solution of hydrochloric acid (30 mL), water (30 mL) and brine (30 mL), dried over anhydrous magnesium sulfate, and concentrated under reduced pressure. The residue was purified by silica gel column chromatography (hexane-ethyl acetate) to obtain Compound (50) (139 mg, 42%) as a brown foam.
MS: m/z = 768.3, 770.3 [M+H] ⁺

### Second Step Synthesis of Compound (51)

Compound (50) (142 mg, 0.184 mmol) was dissolved in a solution of DMA (1.42 mL) and watre (0.142 mL), and [1,1'-bis(di-tert-butylphosphino)ferrocene]palladium dichloride (24.0 mg, 0.037 mmol) and potassium carbonate (50.9 mg, 0.369 mmol) were added thereto, and the mixture was stirred in a sealed tube at 130°C for 1.5 hours. 1mol/L aqueous solution of hydrochloric acid (30mL) was added thereto, and the mixture was extracted with ethyl acetate (50 mL). The obtained organic layer was washed with 1mol/L aqueous solution of hydrochloric acid (30 mL) and brine (30 mL), dried over anhydrous magnesium sulfate, and concentrated under reduced pressure. The residue was purified by silica gel column chromatography (hexane-ethyl acetate) to obtain Compound (51) (37.4 mg, 36%) as a brown foam.
MS: m/z = 562.3 [M-H] ⁺

### Third Step Synthesis of Compound 10

Compound (51) (42.2 mg, 0.075 mmol) was dissolved in ethanol (1mL), and 2mol/L aqueous solution of sodium hydroxide (0.25 mL, 0.500 mmol) was added thereto at room temperature, and the mixture was stirred under heat reflux for 2.5 hours. 1mol/L aqueous solution of hydrochloric acid (30 mL) was added thereto, and the mixture was extracted with ethyl acetate (50 mL). The obtained organic layer was washed with brine (30 mL), dried over anhydrous magnesium sulfate, and concentrated under reduced pressure. The residue was purified by silica gel column chromatography (chloroform-methanol) to obtain Compound 10 (25.9 mg, 63%) as a colorless foam.
MS: m/z = 548.3 [M-H] ⁺

### Example 12 Synthesis of Compound 18

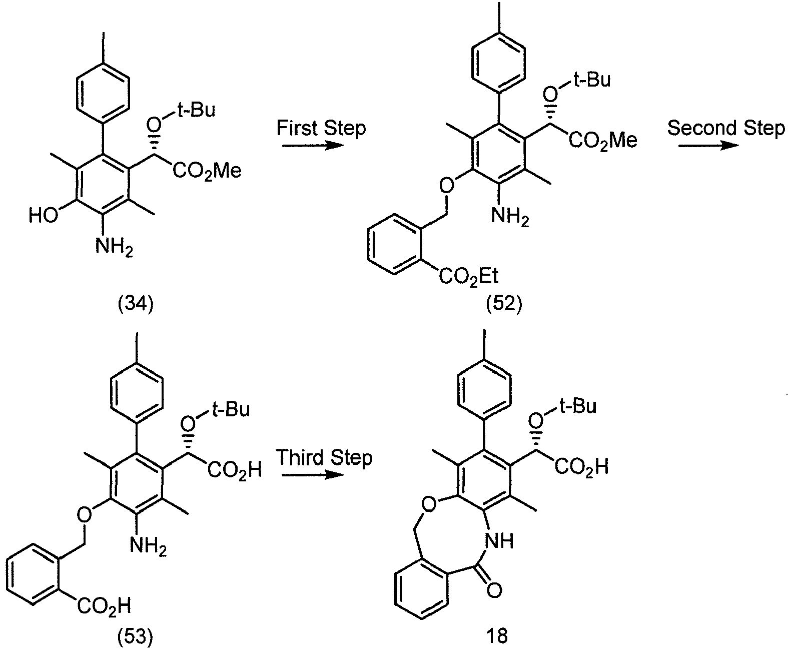

### First Step Synthesis of Compound (52)

Compound (34) (300 mg, 0.808 mmol) was dissolved in DMF (6 mL), and sodium hydride (35.5 mg, 60%Wt, 0.888 mmol) was added thereto under ice bath, and the mixture was stirred for 10 minutes. Ethyl 2-(bromomethyl)benzoate (196 mg, 0.808 mmol) was added thereto under ice bath, and the mixture was stirred for 45 minutes. A saturated aqueous solution of ammonium chloride (3 mL) and water (50 mL) were added thereto, and the mixture was extracted with ethyl acetate (100 mL). The obtained organic layer was washed with water (50 mL) and brine (50 mL), dried over anhydrous magnesium sulfate, and concentrated under reduced pressure. The residue was purified by silica gel column chromatography (hexane-ethyl acetate) to obtain Compound (52) (274 mg, 63%) as a brown oil.
MS: m/z = 534.5 [M+H] ⁺

### Second Step Synthesis of Compound (53)

Compound (52) (283 mg, 0.530 mmol) was dissolved in ethanol (5.30 mL), and 2mol/L aqueous solution of sodium hydroxide (1.33 mL, 2.65 mmol) was added thereto at room temperature, and the mixture was stirred under heat reflux for 1.5 hours. 1mol/L aqueous solution of hydrochloric acid (2.65mL) and brine (30 mL) were added thereto, and the mixture was extracted with ethyl acetate (50 mL). The obtained organic layer was washed with brine (30 mL), dried over anhydrous magnesium sulfate, and concentrated under reduced pressure to obtain crude Compound (53) (257 mg, 99%) as a yellow foam.
MS: m/z = 492.4 [M-H] ⁻

### Third Step Synthesis of Compound 18

Compound (53) (240 mg, 0.534 mmol), 1-ethyl-3-(3-dimethylaminopropyl) carbodiimide hydrochloride (198mg, 1.03mmol) and 1-hydroxybenzotriazole (140 mg, 1.03 mmol) were dissolved in DMF (12.7 mL), and N,N-diisopropylethylamine (0.180 mL, 1.03 mmol) was added thereto, and the mixture was stirred at room temperature for 66 hours. 1mol/L aqueous solution of hydrochloric acid (50 mL) was added thereto, and the mixture was extracted with ethyl acetate (150 mL). The obtained organic layer was washed with 1mol/L aqueous solution of hydrochloric acid (50mL×2) and brine (50 mL), dried over anhydrous magnesium sulfate, and concentrated under reduced pressure. The residue was purified by silica gel column chromatography (chloroform-methanol) to obtain Compound 18 (90.8 mg, 37%) as a colorless foam.
¹ H-NMR(CDCl₃, 400MHz)δ:0.70(s,9H), 1.86(s,3H), 2.18(s,3H), 2.34(s,3H), 4.75(s, 1H), 5.03-5.26(m,1H), 5.40-5.62(m,1H), 6.88-7.37(m,8H), 9.29(s,1H), 12.4(s,1H)
MS: m/z = 474.2 [M+H] ⁺

### Example 13 Synthesis of Compound 25

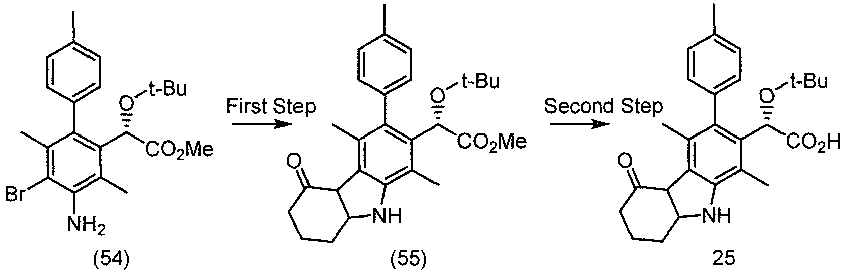

### First Step Synthesis of Compound (55)

Compound (54) (100 mg, 0.230 mmol) which was synthesized by the same matter as Compound (44), was dissolved in toluene (2 mL), and 1,3-cyclohexanedione (25.8 mg, 0.230 mmol) and para-toluene sulfonic acid monohydrate (4.38 mg, 0.023 mmol) were added thereto, and the mixture was stirred under heat reflux for 1.5 hours. Water (50 mL) was added thereto, and the mixture was extracted with ethyl acetate (50 mL). The obtained organic layer was washed with brine (50 mL), dried over anhydrous magnesium sulfate, and concentrated under reduced pressure. The residue was dissolved in DMA (2 mL), and palladium acetate (5.17 mg, 0.023 mmol), 1,3-bis(diphenylphosphino)propane (9.50 mg, 0.23 mmol), sodium acetate (76.0 mg, 0.921 mmol) and tetraethylammonium chloride (381 mg, 2.30 mmol) were added thereto, and the mixture was stirred in a sealed tube at 120°C for 4 hours. Water (50 mL) was added thereto, and the mixture was extracted with ethyl acetate (50 mL). The obtained organic layer was washed with water (50 mL×2) and brine (50 mL), dried over anhydrous magnesium sulfate, and concentrated under reduced pressure. The residue was purified by silica gel column chromatography (hexane-ethyl acetate) to obtain Compound (55) (29.1 mg, 28%) as a brown solid.
1 H-NMR (CDCl₃) δ: 0.95 (s, 9H), 2.12-2.22 (m, 2H), 2.42 (s, 3H), 2.52-2.63 (m, 8H), 2.96 (t, J = 6.2 Hz, 2H), 3.64 (s, 3H), 5.07 (s, 1H), 7.11 (d, J = 7.9 Hz, 1H), 7.16-7.24 (m, 3H), 8.97 (s, 1H).
MS: m/z = 448.3 [M+H] ⁺

### Second Step Synthesis of Compound 25

Compound (55) (29.0 mg, 0.065 mmol) was dissolved in ethanol (0.648 mL), and 2mol/L aqueous solution of sodium hydroxide (0.162 mL, 0.324 mmol) was added thereto, and the mixture was stirred under heat reflux fo 45 minutes. 1mol/L aqueous solution of hydrochloric acid (3 mL) and brine (30 mL) were added thereto, and the mixture was extracted with ethyl acetate (50 mL). The obtained organic layer was dried over anhydrous magnesium sulfate, and concentrated under reduced pressure. The residue was purified by silica gel column chromatography (chloroform-methanol) to obtain Compound 25 (24.4 mg, 87%) as a colorless solid.
¹H-NMR (CDCl₃) δ: 0.96 (s, 9H), 2.09-2.18 (m, 2H), 2.41 (s, 6H), 2.50-2.61 (m, 5H), 2.83-2.93 (m, 2H), 5.23 (s, 1H), 7.15 (d, J = 7.6 Hz, 1H), 7.20-7.26 (m, 2H), 7.37 (d, J = 6.8 Hz, 1H), 8.68 (s, 1H), 9.88 (s, 0H).
MS: m/z = 434.2 [M-H]⁻

### Example 14 Synthesis of Compound 29

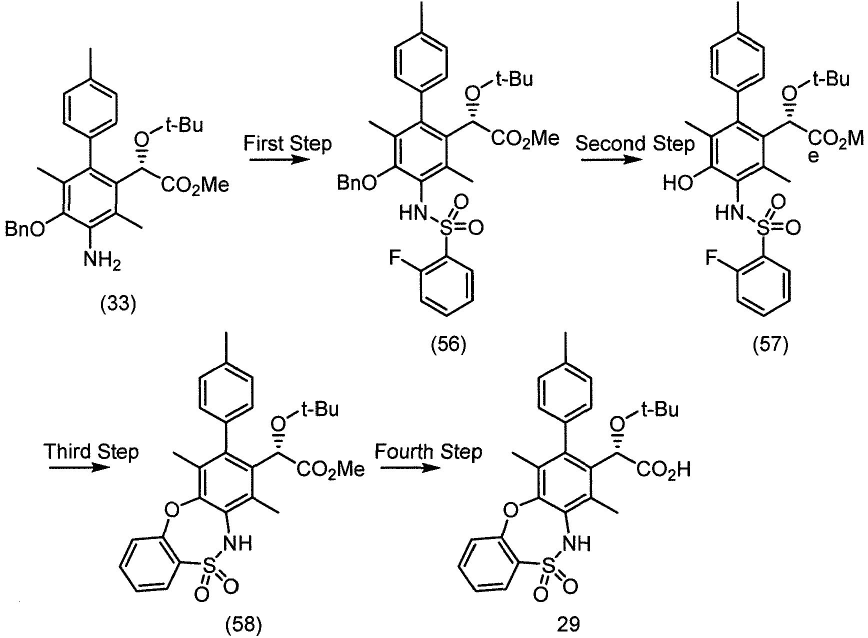

### First Step Synthesis of Compound (56)

Compound (33) (50.0 mg, 0.108 mmol) was dissolved in pyridine (1 mL), and the mixture was heated at 100°C, and 2-fluorobenzene sulfonyl chloride (0.043 mL, 0.325 mmol) was added thereto, and the mixture was stirred at 100°C for 1.5 hours. The mixture was cooled to room temperature, and water (30 mL) was added thereto, and the mixture was extracted with ethyl acetate (50 mL). The obtained organic layer was washed with 1mol/L aqueous solution of hydrochloric acid (30 mL), water (30 mL), a saturated aqueous solution of sodium hydrogen carbonate (30 mL) and brine (30 mL), dried over anhydrous magnesium sulfate, and concentrated under reduced pressure. The residue was purified by silica gel column chromatography (hexane-ethyl acetate) to obtain Compound (56) (62.7 mg, 93%) as a colorless foam.
¹H-NMR (CDCl₃) δ: 0.94 (s, 9H), 2.20 (s, 3H), 2.41 (s, 3H), 3.67 (s, 3H), 4.81 (s, 2H), 4.98 (s, 1H), 6.43 (s, 1H), 6.99-7.05 (m, 2H), 7.11-7.18 (m, 2H), 7.19-7.24 (m, 2H), 7.31-7.37 (m, 5H), 7.41-7.48 (m, 1H), 7.75 (ddd, J = 7.5, 7.5, 1.5 Hz, 1H).
MS: m/z = 618.3 [M-H]⁻

### Second Step Synthesis of Compound (57)

To a solution of Compound (56) (60.0 mg, 0.097 mmol) in methanol (2 mL) was added palladium hydroxide (13.6mg, 20%wt, 50%wet), and the mixture was stirred under a hydrogen atmosphere at room temperature for 15 hours. The mixture was filtered through celite, and the filtrate was concentrated to obtain Compound (57)
(41.3 mg, 81%) as a brown foam.
¹H-NMR (CDCl₃) δ: 0.81 (s, 9H), 1.82 (s, 3H), 1.89 (s, 3H), 2.40 (s, 3H), 3.58 (s, 3H), 4.86 (s, 1H), 6.36 (s, 1H), 6.92 (s, 1H), 7.01-7.05 (m, 1H), 7.10-7.14 (m, 1H), 7.18-7.24 (m, 4H), 7.57-7.64 (m, 1H), 7.78 (ddd, J = 7.5, 7.5, 1.1 Hz, 1H).
MS: m/z = 528.2 [M-H]⁻

### Third Step Synthesis of Compound (58)

Compound (57) (40.0mg, 0.076mmol) was dissolved in DMF (1 mL), and sodium hydride (9.06 mg, 60%Wt, 0.227 mmol) was added thereto under ice bath, and the mixture was stirred at 80°C for 2 hours. 1mol/L aqueous solution of hydrochloric acid (30 mL) was added thereto, and the mixture was extracted with ethyl acetate (50 mL). The obtained organic layer was washed with brine (30 mL), dried over anhydrous magnesium sulfate, and concentrated under reduced pressure to obtain crude Compound (58) as a brown oil.
MS: m/z = 508.2 [M-H]⁻

### Fourth Step Synthesis of Compound 29

The crude Compound (58) was dissolved in ethanol (1mL), and 2mol/L aqueous solution of sodium hydroxide (0.250 mL, 0.500 mmol) was added thereto at room temperature, and the mixture was stirred under heat reflux for 3 hours. 1mol/L aqueous solution of hydrochloric acid (30 mL) was added thereto, and the mixture was extracted with ethyl acetate (50mL). The obtained organic layer was washed with brine (30 mL), dried over anhydrous magnesium sulfate, and concentrated under reduced pressure. The residue was purified by silica gel column chromatography (chloroform-methanol) to obtain Compound 29 (32.0 mg, 86% in 2 steps) as a colorless foam.
¹ H-NMR (CDCl₃) δ: 0.96 (s, 9H), 2.07 (s, 3H), 2.41 (s, 3H), 2.52 (s, 3H), 5.11 (s, 1H), 6.69 (s, 1H), 7.02 (d, J = 7.4 Hz, 1H), 7.27-7.39 (m, 5H), 7.51 (ddd, J = 7.5, 7.5, 1.2 Hz, 1H), 7.84 (dd, J = 7.9, 0.9 Hz, 1H).
MS: m/z = 494.1 [M-H]⁻

### Example 15 Synthesis of Compound 30

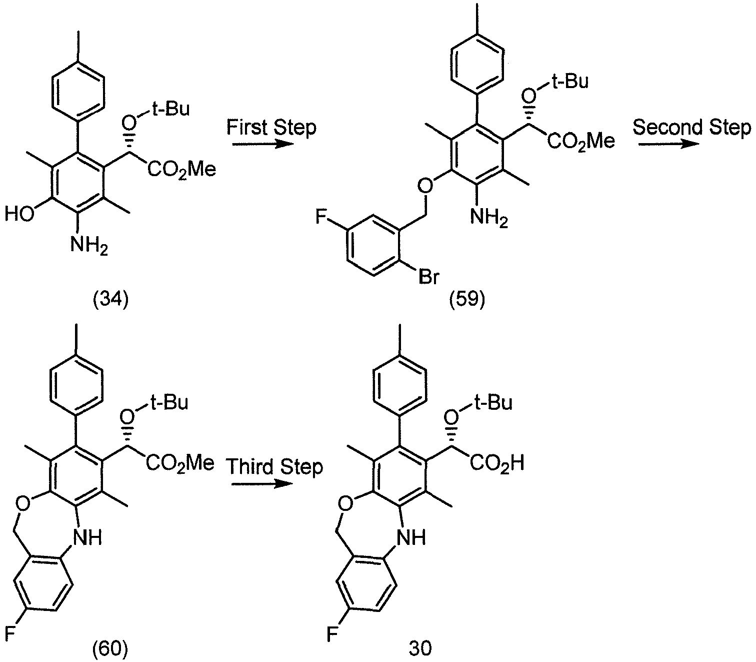

### First Step Synthesis of Compound (59)

Compound (34) (200 mg, 0.538 mmol) was dissolved in DMF (4 mL), and sodium hydride (23.7mg, 60%Wt, 0.592mmol) was added thereto under ice bath, and the mixture was stirred for 15 minutes. 2-bromo-5-fluorobenzyl bromide (144 mg, 0.538 mmol) was added thereto under ice bath, and the mixture was stirred for 1 hour. A saturated aqueous solution of ammonium chloride (3 mL) and water (50 mL) were added thereto, and the mixture was extracted with ethyl acetate (100 mL). The obtained organic layer was washed with water (50 mL×2) and brine (50 mL), dried over anhydrous magnesium sulfate, and concentrated under reduced pressure. The residue was purified by silica gel column chromatography (hexane-ethyl acetate) to obtain Compound (59) (223 mg, 74%) as a brown foam.
¹H-NMR (CDCl₃) δ: 0.96 (s, 9H), 1.88 (s, 3H), 2.25 (s, 3H), 2.41 (s, 3H), 3.68 (s, 3H), 3.82 (s, 2H), 4.87 (s, 2H), 5.00 (s, 1H), 6.93 (td, J = 8.3, 3.0 Hz, 1H), 7.07 (d, J = 7.7 Hz, 1H), 7.16-7.23 (m, 3H), 7.48 (dd, J = 9.4, 3.0 Hz, 1H), 7.52 (dd, J = 8.8, 5.1 Hz, 1H).
MS: m/z = 558.2, 560.2 [M+H] ⁺

### Second Step Synthesis of Compound (60)

Compound (59) (50.0 mg, 0.090 mmol), tris(dibenzylideneacetone)dipalladium (8.2 mg, 0.009 mmol), sodium tert-butoxide (17.2 mg, 0.179 mmol) and potassium carbonate (24.8 mg, 0.179 mmol) was dissolved in toluene (1 mL), and tert-butylphosphine (0.9 mg, 0.004 mmol) was added thereto at room temperature, and the mixture was stirred in a sealed tube at 95°C for 4 hours. 1mol/L aqueous solution of hydrochloric acid (3 mL) and brine (30 mL) were added thereto, and the mixture was extracted with ethyl acetate (50 mL). The obtained organic layer was dried over anhydrous magnesium sulfate, and concentrated under reduced pressure to obtain crude Compound (60) as a brown oil.
MS: m/z = 478.2 [M+H] ⁺

### Third Step Synthesis of Compound 30

The crude Compound (60) was dissolved in ethanol (1 mL), and 2mol/L aqueous solution of sodium hydroxide (0.250 mL, 0.500 mmol) was added thereto at room temperature, and the mixture was stirred under heat reflux for 30 minutes. 1mol/L aqueous solution of hydrochloric acid (3 mL) and brine (30 mL) were added thereto, and the mixture was extracted with ethyl acetate (50 mL). The obtained organic layer was dried over anhydrous magnesium sulfate, and concentrated under reduced pressure. The residue was purified by silica gel column chromatography (chloroform-methanol) to obtain Compound 30 (37.2 mg, 90% in 2 steps) as a brown foam.
¹H-NMR (CDCl₃) δ: 0.97 (s, 9H), 1.93 (s, 3H), 2.28 (s, 3H), 2.40 (s, 3H), 5.00 (d, J = 13.6 Hz, 1H), 5.05 (d, J = 13.6 Hz, 1H), 5.14 (s, 1H), 5.85 (s, 1H), 6.73-6.81 (m, 2H), 6.88 (td, J = 8.3, 2.7 Hz, 1H), 7.07 (d, J = 7.5 Hz, 1H), 7.18-7.24 (m, 2H), 7.35 (d, J = 7.4 Hz, 1H), 9.85 (br s, 1H).
MS: m/z = 464.2 [M+H]⁺

### Example 16 Synthesis of Compound 33

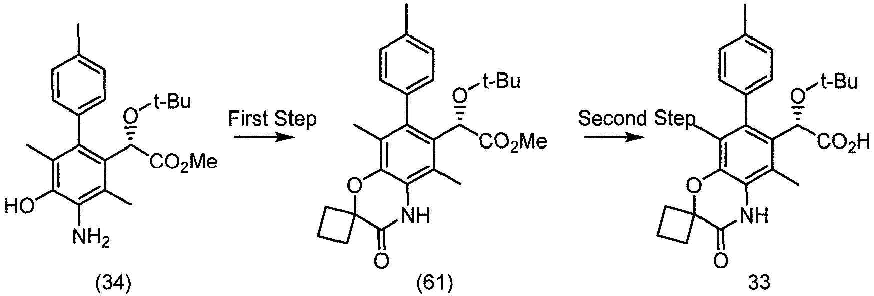

### First Step Synthesis of Compound (61)

Compound (34) (300 mg, 0.808 mmol) was dissolved in DMF (3 mL), and sodium hydride (35.5 mg, 60%Wt, 0.888 mmol) was added thereto under ice bath, and the mixture was stirred for 15 minutes. Ethyl 1-bromocyclobutanecarboxylate (0.392 mL, 2.42 mmol) was added thereto under ice bath, and the mixture was stirred at room temperature for 4.5 hours. A saturated aqueous solution of ammonium chloride (3 mL) and water (30 mL) were added thereto, and the mixture was extracted with ethyl acetate (50mL). The obtained organic layer was washed with brine (30 mL), dried over anhydrous magnesium sulfate, and concentrated. The residue was purified by silica gel column chromatography (hexane-ethyl acetate) to obtain Compound (61) (35.0 mg, 10%) as a brown foam.
¹H-NMR (CDCl₃) δ: 0.95 (s, 9H), 1.90 (s, 3H), 1.94-2.03 (m, 2H), 2.26-2.36 (m, 5H), 2.42 (s, 3H), 2.61-2.73 (m, 2H), 3.67 (s, 3H), 4.98 (s, 1H), 7.04 (dd, J = 7.6, 1.4 Hz, 1H), 7.16-7.25 (m, 3H), 7.44 (s, 1H).
MS: m/z = 452.2 [M+H]⁺

### Second Step Synthesis of Compound 33

Compound (61) (35.0 mg, 0.078 mmol) was dissolved in ethanol (1 mL). 2mol/L aqueous solution of sodium hydroxide (0.250 mL, 0.500 mmol) was added thereto, and the mixture was stirred under heat reflux for 30 minutes. 1mol/L aqueous solution of hydrochloric acid (3 mL) and brine (30 mL) were added thereto, and the mixture was extracted with ethyl acetate (50 mL). The obtained organic layer was dried over anhydrous magnesium sulfate, and concentrated. The residue was purified by silica gel column chromatography (chloroform-methanol) to obtain Compound 33 (24.1 mg, 71%) as a pale brown solid.
¹H-NMR (CDCl₃) δ: 0.97 (s, 9H), 1.89-2.04 (m, 5H), 2.23-2.38 (m, 5H), 2.41 (s, 3H), 2.49-2.58 (m, 1H), 2.74-2.83 (m, 1H), 5.10 (s, 1H), 7.05 (d, J = 7.7 Hz, 1H), 7.21 (d, J = 7.7 Hz, 1H), 7.26 (d, J = 7.7 Hz, 1H), 7.38 (d, J = 7.7 Hz, 1H), 8.72 (s, 1H), 10.88 (s, 1H).
MS: m/z = 438.2 [M+H]⁺

### Example 17 Synthesis of Compounds 34 and 35

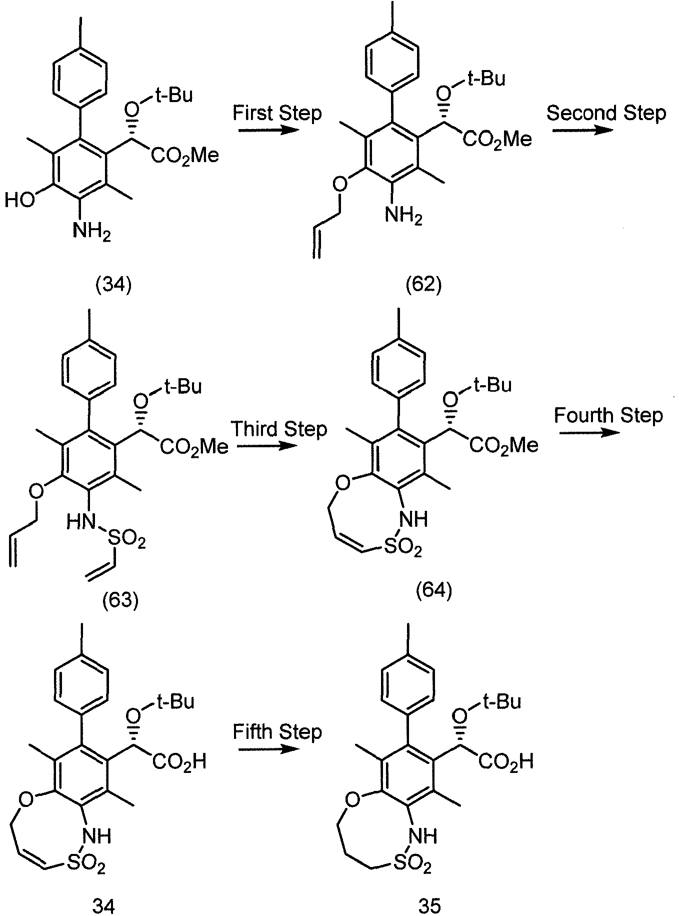

### First Step Synthesis of Compound (62)

Compound (34) (1.00 mg, 2.69 mmol) was dissolved in DMF (20 mL), and sodium hydride (118 mg, 60%Wt, 2.96 mmol) was added thereto under ice bath, and the mixture was stirred for 20 minutes. Allyl bromide (0.233 mL, 2.69 mmol) was added thereto under ice bath, and the mixture was stirred for 45 minutes. A saturated aqueous solution of ammonium chloride (3 mL) and water (50 mL) was added thereto, and the mixture was extracted with ethyl acetate (100 mL). The obtained organic layer was washed with water (50 mL×2) and brine (50 mL), dried over anhydrous magnesium sulfate, and concentrated. The residue was purified by silica gel column chromatography (hexane-ethyl acetate) to obtain Compound (62) (733 mg, 64%) as a brown oil.
¹H-NMR (CDCl₃) δ: 1.88 (s, 3H), 2.23 (s, 3H), 2.40 (s, 3H), 3.66 (s, 3H), 3.81 (s, 2H), 4.30-4.39 (m, 2H), 4.98 (s, 1H), 5.26 (dd, J = 10.4, 1.4 Hz, 1H), 5.45 (dd, J = 17.1, 1.4 Hz, 1H), 6.07-6.18 (m, 1H), 7.05 (d, J = 7.8 Hz, 1H), 7.15-7.22 (m, 3H).
MS: m/z = 412.2 [M+H]⁺

### Second Step Synthesis of Compound (63)

Compound (62) (606 mg, 1.47 mmol) was dissolved in dichloromethane (10 mL), and a solution of 2-chloroethane sulfonyl chloride (0.186 mL, 1.77 mmol) in dichloromethane (1mL) and a solution of pyridine (0.357 mL, 4.42 mmol) in dichloromethane (1mL) were added thereto under ice bath, and the mixture was warmed up to room temperature and stirred for 2.5 hours. Water (50 mL) was added thereto and the mixture was extracted with ethyl acetate (100 mL). The obtained organic layer was washed with 1mol/L aqueous solution of hydrochloric acid (50 mL), water (50 mL), a saturated aqueous solution of sodium hydrogen carbonate (50 mL) and brine (50 mL), dried over anhydrous magnesium sulfate, and concentrated. The residue was purified by silica gel column chromatography (hexane-ethyl acetate) to obtain Compound (63) (565 mg, 74%) as a colorless foam.
¹H-NMR (CDCl₃) δ: 0.94 (s, 9H), 1.88 (s, 3H), 2.42 (s, 3H), 2.43 (s, 3H), 3.67 (s, 3H), 4.40 (d, J = 5.6 Hz, 2H), 4.99 (s, 1H), 5.30 (d, J = 10.4 Hz, 1H), 5.44 (dd, J = 17.2, 1.3 Hz, 1H), 5.90 (d, J = 9.9 Hz, 1H), 6.03 (s, 1H), 6.06-6.16 (m, 1H), 6.22 (d, J = 16.6 Hz, 1H), 6.82 (dd, J = 16.6, 9.9 Hz, 1H), 7.04 (d, J = 7.9 Hz, 1H), 7.16 (d, J = 7.8 Hz, 1H), 7.20-7.25 (m, 2H).
MS(ESI) m/z: 500.2[M-H]-

### Third Step Synthesis of Compound (64)

Compound (63) (547 mg, 1.09 mmol) was dissolved in dichloromethane (54.7mL), and second generation Grubbs catalyst (278 mg, 0.327 mmol) was added thereto at room temperature, and the mixture was stirred for 25 hours. The mixture was concentrated. The residue was purified by silica gel column chromatography (hexane-ethyl acetate) to obtain Compound (64) (113 mg, 21%) as a brown solid.
¹H-NMR (CDCl₃) δ: 0.95 (s, 9H), 1.89 (s, 3H), 2.40 (s, 3H), 2.42 (s, 3H), 3.68 (s, 3H), 4.77-4.89 (m, 2H), 4.99 (s, 1H), 6.15 (s, 1H), 6.37 (dt, J = 11.0, 4.0 Hz, 1H), 6.74 (d, J = 11.2 Hz, 1H), 7.03 (dd, J = 7.7, 1.4 Hz, 1H), 7.16 (dd, J = 7.7, 1.4 Hz, 1H), 7.18-7.26 (m, 2H).
MS(ESI) m/z: 472.2[M-H]-

### Fourth Step Synthesis of Compound 34

Compound (64) (105 mg, 0.222 mmol) was dissolved in ethanol (2.22 mL). 2mol/L aqueous solution of sodium hydroxide (0.554 mL, 1.11 mmol) was added thereto, and the mixture was stirred under heat reflux for 1 hour. 1mol/L aqueous solution of hydrochloric acid (3 mL) and brine (30 mL) were added thereto and the mixture was extracted with ethyl acetate (50 mL). The obtained organic layer was dried over anhydrous magnesium sulfate and concentrated. The residue was purified by silica gel column chromatography (chloroform-methanol) to obtain Compound 34 (44.5 mg, 44%) as a brown foam.
¹H-NMR (CDCl₃) δ: 0.99 (s, 9H), 1.92 (s, 3H), 2.42 (s, 3H), 2.49 (s, 3H), 3.57 (dd, J = 15.2, 7.7 Hz, 1H), 3.72 (dd, J = 15.2, 7.7 Hz, 1H), 4.85 (ddd, J = 7.7, 7.7, 7.7 Hz, 1H), 5.14 (s, 1H), 6.39 (s, 1H), 6.74 (d, J = 7.7 Hz, 1H), 7.07 (d, J = 6.5 Hz, 1H), 7.23-7.28 (m, 2H), 7.37 (d, J = 6.5 Hz, 1H).
MS: m/z = 458.2 [M-H]⁻

### Fifth Step Synthesis of Compound 35

Compound 34 (30.0 mg, 0.065 mmol) was dissolved in methanol (1.5 mL). palladium hydroxide (18.3 mg, 10%Wt, 0.013 mmol) was added thereto at room temperature and the mixture was stirred under a hydrogen atmosphere for 1 hour. The mixture was filtered through celite and the filtrate was concentrated. The residue was purified by silica gel column chromatography (chloroform-methanol) to obtain Compound 35 (18.7 mg, 62%) as a colorless solid.
¹H-NMR (CDCl₃) δ: 0.97 (s, 9H), 1.93 (s, 3H), 2.03-2.18 (m, 2H), 2.41 (s, 3H), 2.47 (s, 3H), 3.23-3.43 (m, 2H), 4.07-4.17 (m, 1H), 4.23-4.34 (m, 1H), 5.10 (s, 1H), 6.20 (s, 1H), 7.07 (d, J = 5.1 Hz, 1H), 7.20-7.27 (m, 2H), 7.37 (d, J = 5.1 Hz, 1H).
MS: m/z = 460.2 [M-H]⁻.

### Example 18 Synthesis of Compounds 46 and 47

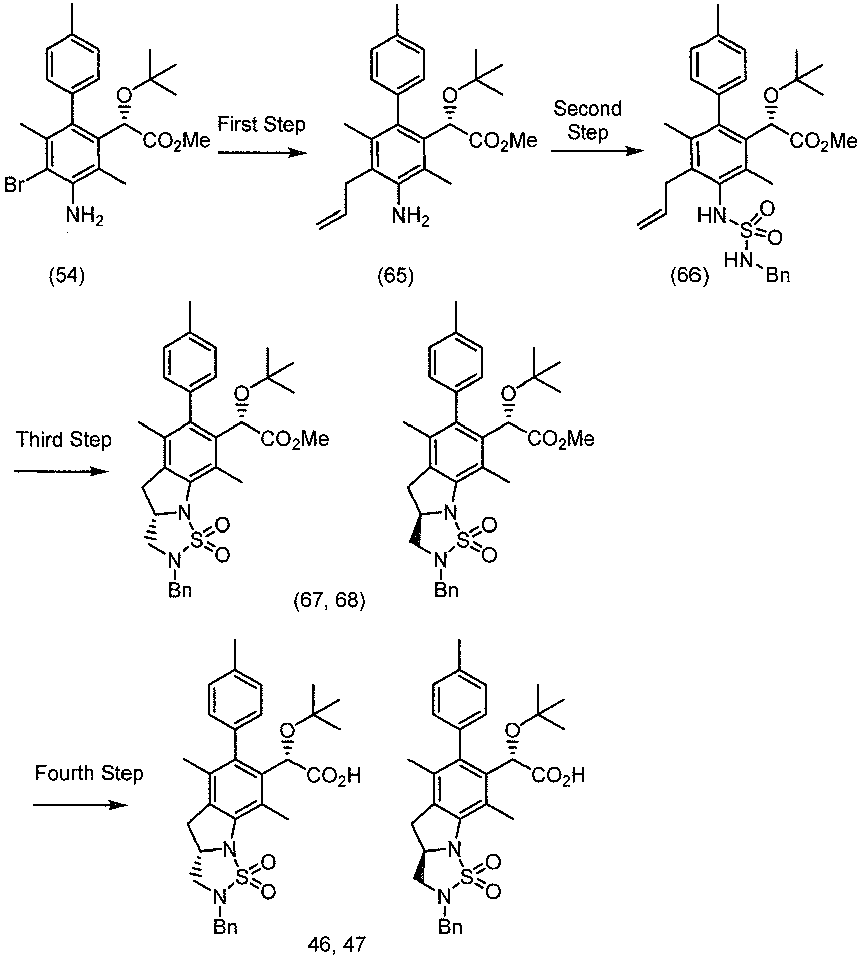

### First Step Synthesis of Compound (65)

Compound (54) (210 mg, 0.483 mmol) and allyl tetrabutyltin (240 mg, 0.725 mmol) were dissolved in DMF (2 mL), and lithium chloride (2mg, 0.047mmol) was added thereto, and the operation of degassing and nitrogen substitution was repeated three times. PdCl₂ (dtbpf)(31 mg, 0.048 mmol) was added thereto, and again, repeated three times the operation of the degassing and nitrogen substitution, and the mixture was stirred at 100 °C for 30 minutes. The mixture was cooled to room temperature and diluted with ethyl acetate (5 mL), and a saturated aqueous solution of potassium fluoride was added thereto. The mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (66) (135 mg, 74.1%).
¹H NMR (CDCl₃) δ: 0.95 (s, 9H), 1.89 (s, 3H), 2.24 (s, 3H), 2.40 (s, 3H), 3.39 (d, J = 5.6 Hz, 2H), 3.65 (s, 3H), 3.68 (s, 2H), 5.00 (s, 1H), 5.05-5.07 (m, 1H), 5.09 (s. 1H), 5.88-5.97 (m, 1H), 7.05 (d, J = 7.6 Hz, 2H), 7.16 (d, J = 7.6 Hz, 2H),7.20 (s, 2H).

### Second Step Synthesis of Compound (66)

Compound (65) (536 mg, 1.36 mmol) was dissolved in pyridine (5 mL), and benzyl sulfamoyl chloride (557 mg, 2.71 mmol) which was synthesized by a known method, was added thereto under ice bath. The mixture was stirred at room temperature for 1 hour. Water was added thereto, and the mixture was extracted with ethyl acetate. The obtained organic layer was washed with 1mol/L aqueous solution of hydrochloric acid, dried over anhydrous sodium sulfate, and concentrated under reduced pressure. The residue was purified by silica gel column chromatography (hexane-ethyl acetate) to obtain Compound (66)(766 mg, 100%).
¹H NMR (CDCl₃) δ: 0.95 (s, 9H), 1.89 (s, 3H), 2.41 (s, 3H), 2.51 (s, 3H),3.66 (s, 3H), 3.68 (d, J = 5.6 Hz, 2H), 4.36 (d, J = 6.0 Hz, 2H), 4.70-4,77 (m, 1H), 4.95 (d, J = 12.9 Hz, 1H), 5.02 (s, 1H), 5.06 (d, J = 10.4 Hz, 1H), 5.87-5.97 (m, 1H), 5.99-6.02 (m, 1H), 7.04 (d, J = 8.0 Hz, 1H), 7.16-7.27 (m, 3H), 7.29-7.33 (m, 1H), 7.36 (d, J = 4.0 Hz, 4H).

### Third Step Synthesis of Compounds (67) and (68)

Compound (66) (342 mg, 0.606 mmol) was dissolved in DMF (4 mL), and copper acetate (II) (165 mg, 0.908 mmol) and potassium carbonate (251 mg, 1.82 mmol) were added thereto. The mixture was stirred at 100°C for 2 hours. The mixture was cooled to room temperature, and water was added thereto, and the mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (67) (38 mg, 11.2%) and Compound (68) (59 mg, 17.3%). Compound (67)
¹H NMR (CDCl₃) δ: 0.99 (s, 9H), 1.82 (s, 3H), 2.41 (s, 3H), 2.47 (s, 3H), 3.06 (dd, J = 15.6, 8.4 Hz, 1H), 3.16 (dd, J = 15.6, 9.2 Hz, 1H), 3.24 (d, J = 10.0 Hz, 1H), 3.44 (dd, J = 9.6, 6.0 Hz, 1H), 3.68 (s, 3H), 3.86 (d. J = 13.2 Hz, 1H), 4.50 (d, J = 13.2 Hz, 1H), 4.64 (dd, J = 14.8, 7.6 Hz, 1H), 4.97 (s, 1H), 7.10 (dd, J = 16.8, 7.2 Hz, 2H), 7.22 (t, J = 6.8 Hz, 2H), 7.32-7.41 (m, 5H).
Compound (68)
¹H NMR (CDCl₃) δ: 0.94 (s, 9H), 1.83 (s, 3H), 2.41 (s, 3H), 2.57 (s, 3H), 3.05 (dd, J = 15.2, 8.4 Hz, 1H), 3.16 (dd, J = 15.6, 9.2 Hz, 1H), 3.24 (d, J = 10.0 Hz, 1H),
3.44 (dd, J = 9.6, 5.6 Hz, 1H), 3.68 (s, 3H), 3.93 (d. J = 13.2 Hz, 1H), 4.50 (d, J = 13.2 Hz, 1H), 4.64 (dd, J = 14.4, 8.0 Hz, 1H), 5.08 (s, 1H), 7.03 (d, J = 7.6 Hz, 1H), 7.18 (d, J = 7.6 Hz, 1H), 7.24 (t, J = 8.4 Hz, 2H), 7.31-7.38 (m, 5H).

### Fourth Step Synthesis of Compounds 47 and 48

Compound (67) (38 mg, 0.068 mmol) was dissolved in ethanol (2 mL), and 2mol/L aqueous solution of sodium hydroxide (1 mL, 2 mmol) was added thereto, and the mixture was stirred at 80°C for 2 hours. The mixture was cooled to room temperature and diluted with water (5 mL), and 2mol/L aqueous solution of hydrochloric acid (2 mL, 4 mmol) was added thereto, and the mixture was extracted with ethyl acetate. The obtained organic layer was dried over anhydrous sodium sulfate and concentrated under reduced pressure. The residue was purified by silica gel column chromatography (hexane-ethyl acetate) to obtain Compound 47 (29 mg, 78.3%).
¹H NMR (CDCl₃) δ: 1.02 (s, 9H), 1.86 (s, 3H), 2.41 (s, 3H), 2.42 (s, 3H), 3.07 (dd, J = 15.6, 8.4 Hz, 1H), 3.15 (dd, J = 15.6, 9.2 Hz, 1H), 3.25 (d. J = 10.4 Hz, 1H), 3.44 (dd, J = 9.6, 6.0 Hz, 1H), 3.97 (d, J = 13.2 Hz, 1H), 4.50 (d, J = 13.6 Hz, 1H), 4.65 (dd, J = 14.8, 6.8 Hz, 1H), 5.10 (s, 1H), 7.09 (d, J = 8.0 Hz, 1H), 7.23 (d, J = 8.4 Hz, 1H), 7.33-7.39 (m, 6H).
MS(ESI) m/z:547.0 [M - H]-(*)

Compound 48 was obtained from Compound (68) by the same matter.
¹H NMR (CDCl₃) δ:0.99 (s, 9H), 1.82 (s, 3H), 2.41 (s, 3H), 2.47 (s, 3H), 3.05 (dd,J = 16.6, 8.4 Hz, 1H), 3.12 (dd, J = 15.6, 9.2 Hz, 1H), 3.44 (dd, J = 9.6, 6.0 Hz), 3.68 (s, 3H), 3.96 (d, J = 13.2 Hz, 1H), 4.50 (d, J = 13.2 Hz, 1H), 4.64 (d, J = 14.8, 7.2 Hz, 1H), 4.97 (s, 1H), 7.08 (d, J = 7.2 Hz, 1H), 7.13 (d, J = 7.2 Hz, 1H), 7.22 (t, J = 6.8 Hz, 2H), 7.32-7.41 (m, 5H).
MS(ESI) m/z:547.1 [M - H]-(*)

### Example 19 Synthesis of Compound 49

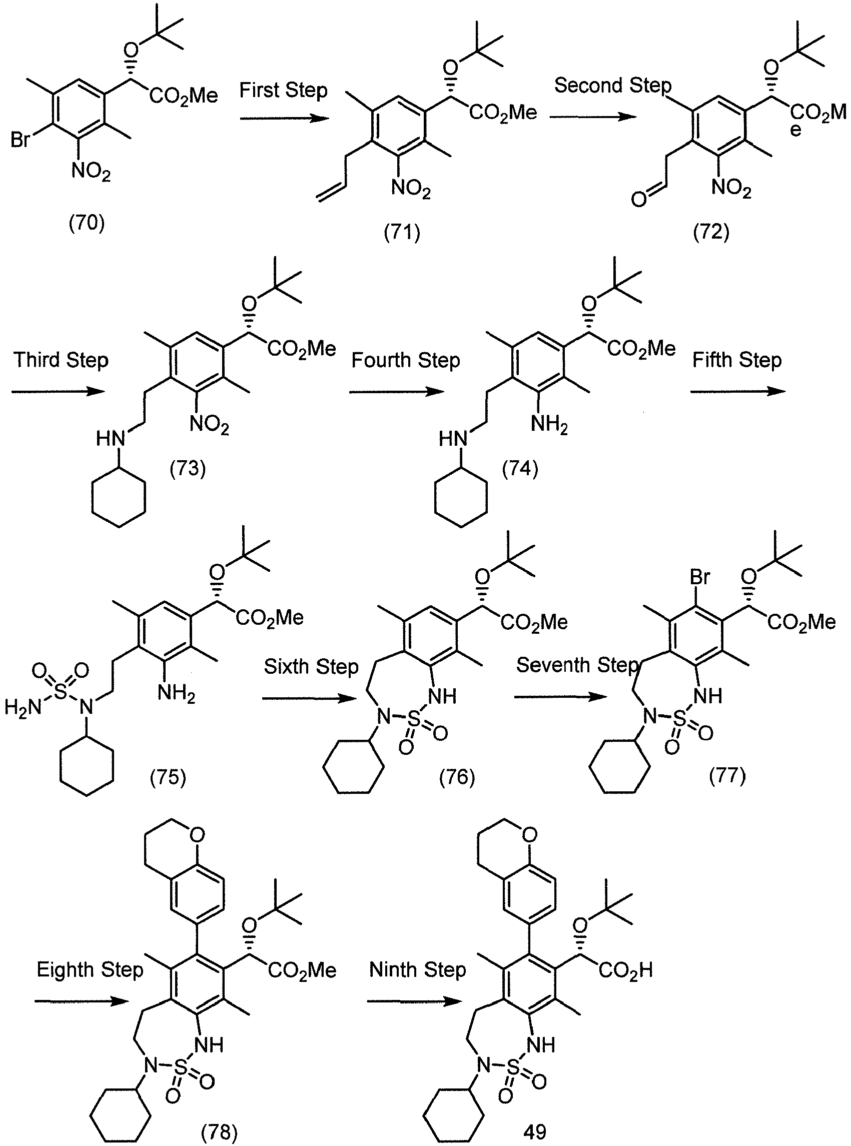

### First Step Synthesis of Compound (71)

Compound (70) (1.00 g, 2.67 mmol) and allyl tetrabutyltin (1.33 g, 4.02 mmol) were dissolved in DMF (5 mL), and lithium chloride (11 mg, 0.259 mmol) was added thereto, and the operation of degassing and nitrogen substitution was repeated three times. PdCl₂ (dtbpf) (174 mg, 0.267 mmol) was added thereto, and again, repeated three times the operation of the degassing and nitrogen substitution, and the mixture was stirred at 100°C for 1 hour. The mixture was cooled to room temperature and diluted with ethyl acetate (10 mL), and a saturated aqueous solution of potassium fluoride was added thereto. The mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (71) (618 mg, 69.0%).
¹H NMR (CDCl₃) δ:1.23 (s, 9H), 2.28 (s, 3H), 2.33 (s, 3H), 3.25 (d, J = 6.0 Hz, 2H), 3.69 (s, 3H), 4.97 (d, J = 17.2 Hz, 1H), 5.06 (d, J = 10.0 Hz, 1H), 5.19 (s, 1H), 5.76-5.85 (m, 1H), 7.49 (s, 1H).

### Second Step Synthesis of Compound (72)

Compound (71) (1.00 g, 2.98 mmol) and pyridine (707 mg, 8.94 mmoL) were dissolved in dichloromethane (20 mL) and the mixture was cooled to -78°C under dryice bath. An ozone gas was passed through the mixture for 30 minutes and then a nitrogen gas for 10 minutes. The mixture was warmed up to room temperature, and a saturated aqueous solution of sodium hydrogen carbonate was added thereto. The mixture was extracted with dichloromethane. The obtained organic layer was dried over anhydrous sodium sulfate and concentrated under reduced pressure. The residue was purified by silica gel column chromatography (hexane-ethyl acetate) to obtain Compound (72) (707 mg, 70.3%).
¹H NMR (CDCl₃) δ:1.24 (s, 9H), 2.28 (s, 3H), 2.32 (s, 3H), 3.60 (s, 2H), 3.70 (s, 3H), 5.21 (s, 1H), 7.58 (s, 1H), 9.66 (s, 1H).

### Third Step Synthesis of Compound (73)

Compound (72) (350 mg, 1.04 mmol), cyclohexylamine (206 mg, 2.08 mmol) and acetic acid (20 mg, 0.333 mmol) were dissolved in dichloromethane (6 mL), and sodium triacetoxyborohydride (331 mg, 1.56 mmol) was added thereto under ice bath, and the mixture was stirred at room temperature for 15 hours. Water was added thereto and the mixture was extracted with dichloromethane. The obtained organic layer was dried over anhydrous sodium sulfate and concentrated under reduced pressure to obtain crude Compound (73) (453 mg). The compound was used in the next reaction without purification.

### Fourth Step Synthesis of Compound (74)

The above crude compound (453 mg) was dissolved in acetic acid (5 mL), and zinc (1.02 g, 15.6 mmol) was added thereto at room temperature, and the mixture was stirred at same temperature for 1 hour. The mixture was diluted with ethyl acetate (20mL) and filtered. The filtrate was concentrated under reduced pressure. The residue was dissolved in dichloromethane and the mixture was washed with a saturated aqueous solution of sodium bicarbonate. The obtained organic layer was dried over anhydrous sodium sulfate and concentrated under reduced pressure to obtain crude Compound (74). The compound was used in the next reaction without purification.

### Fifth Step Synthesis of Compound (75)

The above crude compound was dissolved in pyridine (3 mL), and sulfamide (300 mg, 3.12 mmol) was added thereto, and the mixture was stirred under heat reflux for 1 hour. The mixture was cooled to room temperature, and water was added thereto. The mixture was extracted with ethyl acetate. The obtained organic layer was dried over anhydrous sodium sulfate and concentrated under reduced pressure to obtain crude Compound (75). The compound was used in the next reaction without purification.

### Sixth Step Synthesis of Compound (76)

The above crude compound was dissolved in xylene (3 mL), and triethylamine (315 mg, 3.11 mmol) was added thereto, and the mixture was stirred under heat reflux for 2 hours. The mixture was cooled to room temperature, and water was added thereto. The mixture was extracted with ethyl acetate. The obtained organic layer was washed with 1mol/L aqueous solution of hydrochloric acid, dried over anhydrous sodium sulfate, and concentrated under reduced pressure. The residue was purified by silica gel column chromatography (hexane-ethyl acetate) to obtain Compound (76(226 mg, 48.0% in 4 steps).
¹H NMR (CDCl₃) δ:0.88-1.01 (m, 1H) 1.13-1.28 (m, 4H), 1.22 (s, 9H), 1.37-1.44 (m, 1H), 1.51-1.59 (m, 2H), 1.66-1.71 (m, 2H), 2.29 (s, 3H), 2.42 (s, 3H), 3.03 (t, J = 6.4 Hz, 2H), 3.34-3.48 (m, 2H), 3.65 (s, 3H), 3.69-3.75 (m, 1H), 5.22 (s, 1H), 5.70 (s, 1H), 7.35 (s, 1H).

### Seventh Step Synthesis of Compound (77)

Compound (76) (226 mg, 0.499 mmol) was dissolved in DMF (2 mL), and NBS (89 mg, 0.500 mmol) was added thereto at room temperature, and the mixture was stirred at same temperature for 24 hours. Water was added thereto and the mixture was extracted with ethyl acetate. The obtained organic layer was washed with water, dried over anhydrous sodium sulfate, and concentrated under reduced pressure to obtain crude Compound (77). The compound was used in the next reaction without purification.

### Eighth Step Synthesis of Compound (78)

The above crude compound and chroman-6-yl boronic acid (125 mg, 0.702 mmol) were dissolved in a solution of DMF (3 mL) and water (0.3 mL), and potassium carbonate (195 mg, 1.41 mmoL) was added thereto, the operation of degassing and nitrogen substitution was repeated three times. PdCl₂ (dtbpf) (31 mg, 0.048 mmoL) was added thereto, and again, repeated three times the operation of the degassing and nitrogen substitution, and the mixture was stirred at 100°C for 30 minutes. The mixture was cooled to room temperature and water was added thereto. The mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (78) (226 mg, 74.5% in 2 steps).
¹H NMR (CDCl₃) δ:0.80-0.99 (m, 1H), 0.96 (s, 9H), 1.10-1.28 (m, 6H), 1.44-1.51 (m, 1H), 1.56-1.60 (m, 2H), 1.69-1.73 (m, 2H), 1.94 (d, J = 4.4 Hz, 2H), 2.02-2.08 (m, 2H), 2.05 (s, 3H), 2.42 (s, 3H), 2.68-2.85 (m, 2H), 3.11 (t, J = 6.0 Hz, 2H), 3.43 (t, J = 6.0 Hz), 3.63 (s, 1.33H), 3.66 (s, 1.67H), 3.76 (t, J = 11.2 Hz, 1H), 4.24 (brs, 2H), 5.01 (d, J = 9.6 Hz), 5.79 (s, 1H), 6.75-6.85 (m, 2H), 6.90-6.95 (m, 1H).

### Ninth Step Synthesis of Compound 49

Compound (78) (205 mg, 0.351 mmol) was dissolved in ethanol (2 mL), and 2mol/L aqueous solution of sodium hydroxide (1 mL, 2 mmol) was added thereto, and the mixture was stirred at 80°C for 2 hours. The mixture was cooled to room temperature and diluted with water (5 mL), and 2mol/L aqueous solution of hydrochloric acid (2 mL, 4 mmol) was added thereto. The mixture was extracted with ethyl acetate. The obtained organic layer was dried over anhydrous sodium sulfate and concentrated under reduced pressure. The residue was purified by silica gel column chromatography (hexane-ethyl acetate) to obtain Compound 49 (130 mg, 65.0%).
¹H NMR (CDCl₃) δ:0.84-0.90 (m, 1H), 1.00 (s, 9H), 1.08-1.33 (m, 6H), 1.97-1.98 (m, 3H), 2.01-2.07 (m, 2H), 2.40 (s, 3H), 2.70-2.85 (m, 2H), 3.08-3.16 (m, 2H), 3.01-3.41 (m, 1H), 3.45-3.54 (m, 1H), 3.69-3.77 (m, 1H), 4.22-4.25 (m, 2H), 5.17 (s, 1H), 5.72-5.73 (m, 1H), 6.71-6.86 (m, 2H), 7.11 (brs, 1H).
MS(ESI) m/z:570.7 [M - H]-(*)

### Example 20 Synthesis of Compound 50

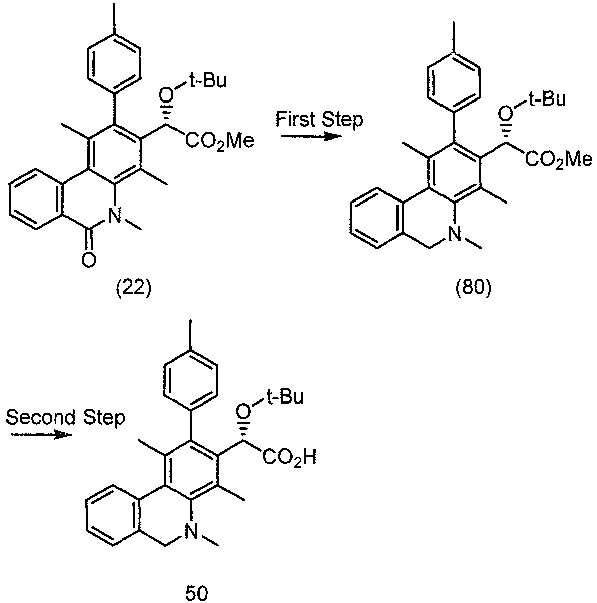

### First Step Synthesis of Compound (80)

Compound (22) (50.0 mg, 0.106 mmol) was dissolved in THF (1 mL), and 2mol/L solution of dimethyl sulfide borane complex (0.106 mL, 0.212 mmol) in THF was added thereto, and the mixture was warmed up to room temperature and stirred. A saturated aqueous solution of sodium hydrogen carbonate (30 mL) was added thereto, and the mixture was extracted with ethyl acetate (50 mL). The obtained organic layer was washed with brine (30 mL), dried over anhydrous sodium sulfate, and concentrated. The residue was purified by silica gel column chromatography (hexane-ethyl acetate) to obtain Compound (80) (43.5mg, 90%) as a colorless foam.
¹H-NMR (CDCl₃) δ: 0.97 (s, 9H), 2.21 (s, 3H), 2.36 (s, 3H), 2.43 (s, 6H), 3.71 (s, 3H), 3.95 (d, J = 14.9 Hz, 1H), 4.08 (d, J = 14.9 Hz, 1H), 5.01 (s, 1H), 7.19 (d, J = 8.3 Hz, 1H), 7.22-7.33 (m, 6H), 7.68 (dd, J = 6.0, 1.8 Hz, 1H).
MS(ESI) m/z: 458.2[M+H]+

### Second Step Synthesis of Compound 50

Compound (80) (43.0 mg, 0.094 mmol) was dissolved in ethanol (1mL), and 2mol/L aqueous solution of sodium hydroxide (0.250 mL, 0.500 mmol) was added thereto, and the mixture was stirred under heat reflux for 45 minutes. 1mol/L aqueous solution of hydrochloric acid (3mL) and brine (30 mL) were added thereto and the mixture was extracted with ethyl acetate (50 mL). The obtained organic layer was dried over anhydrous magnesium sulfate and concentrated. The residue was purified by silica gel column chromatography (chloroform-methanol) to obtain Compound 50 (31.1 mg, 75%) as a brown foam.
¹H-NMR (CDCl₃) δ: 0.99 (s, 9H), 2.22 (s, 3H), 2.35 (s, 3H), 2.41 (s, 3H), 2.42 (s, 3H), 3.93 (d, J = 14.9 Hz, 1H), 4.09 (d, J = 14.9 Hz, 1H), 5.14 (s, 1H), 7.20 (d, J = 7.2 Hz, 1H), 7.23-7.34 (m, 5H), 7.45 (d, J = 7.2 Hz, 1H), 7.69 (dd, J = 6.3, 2.0 Hz, 1H), 9.90 (s, 1H).
MS: m/z = 444.2 [M+H]+

### Example 21 Synthesis of Compounds 38 and 39

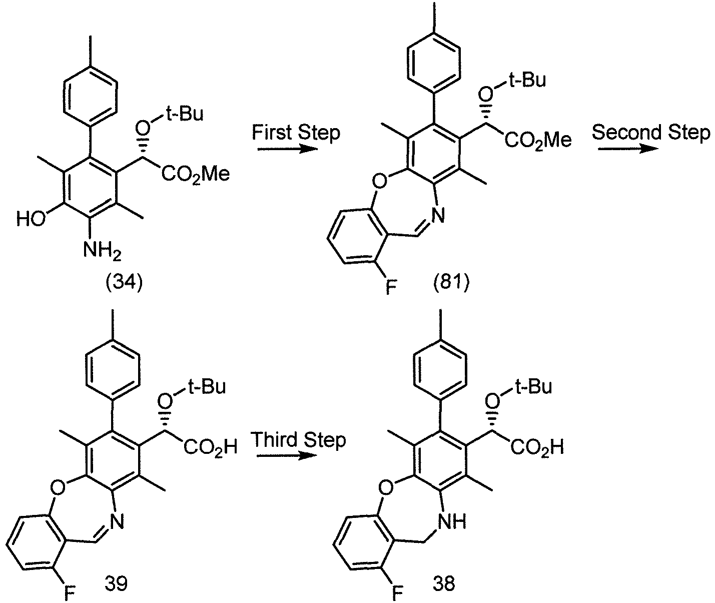

### First Step Synthesis of Compound (81)

Compound (34) (293 mg, 0.788 mmol) was suspended in toluene (5 mL), and 2,6-difluorobenzaldehyde (112 mg, 0.788 mmol) and p-toluenesulfonic acid monohydrate (7.50 mg, 0.039 mmol) were added thereto, and the mixture was stirred under heat reflux for 30 minutes. Triethylamine (0.125 mL) was added thereto and the mixture was concentrated. The residue was dissolved in ethanol (6 mL), and triethylamine (2.19 mL, 15.8 mmol) was added thereto, and the mixture was stirred under heat reflux for 1.5 hours. A saturated aqueous solution of ammonium chloride (30 mL) was added thereto and the mixture was extracted with ethyl acetate. The obtained organic layer was washed with water (30 mL), a saturated aqueous solution of sodium hydrogen carbonate (30 mL) and brine (30 mL), dried over anhydrous magnesium sulfate, and concentrated. The residue was purified by silica gel column chromatography (hexane-ethyl acetate) to obtain Compound (81) (210 mg, 56%) as a colorless foam.
¹H-NMR (CDCl₃) δ: 0.94 (s, 9H), 2.06 (s, 3H), 2.42 (s, 3H), 2.46 (s, 3H), 3.67 (s, 3H), 5.00 (s, 1H), 6.93 (dd, J = 8.2, 8.2 Hz, 1H), 6.98 (d, J = 8.2 Hz, 1H), 7.05 (dd, J = 7.7, 1.6 Hz, 1H), 7.16-7.25 (m, 3H), 7.38 (ddd, J = 8.2, 8.2, 6.5 Hz, 1H), 8.81 (s, 1H).
MS(ESI) m/z: 476.2[M+H]+

### Second Step Synthesis of Compound 39

Compound (81) (107 mg, 0.226 mmol) was dissolved in ethanol (2.26 mL), and 2mol/L aqueous solution of sodium hydroxide (0.565 mL, 1.13 mmol) was added thereto, and the mixture was stirred under heat reflux 1 hour. 1mol/L aqueous solution of hydrochloric acid (3mL) and brine (30 mL) were added thereto and the mixture was extracted with ethyl acetate (50mL). The obtained organic layer was dried over anhydrous magnesium sulfate and concentrated. The residue was purified by silica gel column chromatography (chloroform-methanol) to obtain Compound 39 (80.8 mg, 78%) as a brown foam.
¹H-NMR (CDCl₃) δ: 0.98 (s, 9H), 2.08 (s, 3H), 2.41 (s, 3H), 2.45 (s, 3H), 5.13 (s, 1H), 6.93 (dd, J = 8.3, 8.3 Hz, 1H), 6.97 (d, J = 8.3 Hz, 1H), 7.07 (d, J = 6.8 Hz, 1H), 7.20-7.26 (m, 2H), 7.34 (d, J = 7.2 Hz, 1H), 7.39 (ddd, J = 8.3, 8.3, 6.5 Hz, 1H), 8.82 (s, 1H).
MS: m/z = 462.4 [M+H]+

### Third Step Synthesis of Compound 38

Compound 39 (48.5 mg, 0.105 mmol) was dissolved in methanol (1.5 mL), and palladium hydroxide (29.5 mg, 10%Wt, 0.021 mmol) was added thereto at room temperature, and the mixture was stirred under a hydrogen atmosphere for 15 hours. The mixture was filtered through celite and the filtrate was concentrated. The residue was purified by silica gel column chromatography (chloroform-methanol) to obtain Compound 38 (28.4 mg, 58%) as a brown foam.
¹H-NMR (CDCl₃) δ: 0.95 (s, 9H), 2.09 (s, 3H), 2.14 (s, 3H), 2.40 (s, 3H), 4.57 (d, J = 14.6 Hz, 1H), 4.66 (d, J = 14.6 Hz, 1H), 5.10 (s, 1H), 6.85 (dd, J = 8.2, 8.2 Hz, 1H), 7.00 (d, J = 8.2 Hz, 1H), 7.07 (d, J = 7.5 Hz, 1H), 7.15-7.24 (m, 3H), 7.33 (d, J = 7.5 Hz, 1H).
MS: m/z = 464.4 [M+H]+

### Example 22 Synthesis of Compound 61

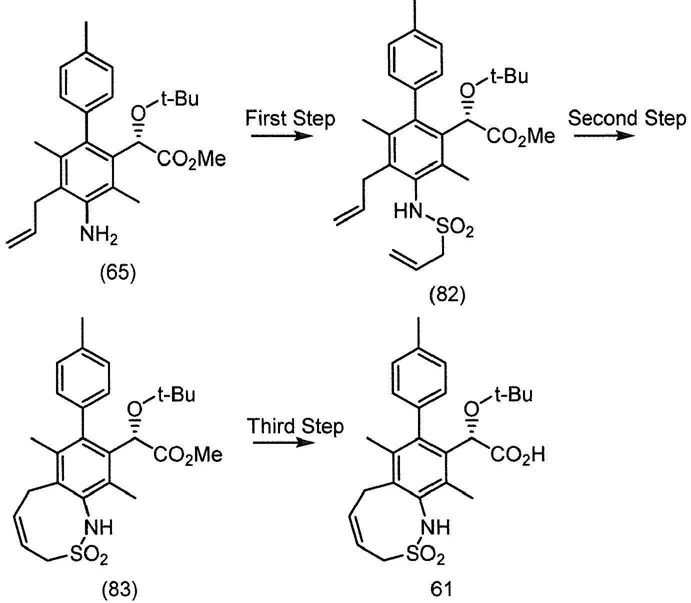

### First Step Synthesis of Compound (82)

Compound (65) (93.0 mg, 0.218 mmol) was dissolved in dichloromethane (2mL), and pyridine (0.053 mL, 0.657 mmol) and a solution of allylsulfonyl chloride (47.0 mg, 0.334 mmol) in dichloromethane (1mL) were added thereto under ice bath, and the mixture was warmed up to room temperature and stirred for 7 hours. A saturated aqueous solution of sodium hydrogen carbonate (10 mL) was added thereto and the mixture was extracted with ethyl acetate (30 mL). The obtained organic layer was washed with lmol/L aqueous solution of hydrochloric acid (10 mL) and brine (15 mL×2), dried over anhydrous magnesium sulfate, and concentrated. The residue was purified by silica gel column chromatography (hexane-ethyl acetate) to obtain Compound (82) (46.9mg, 42%) as a brown foam.
MS(ESI) m/z: 498.10[M-H]-

### Second Step Synthesis of Compound (83)

Compound (82) (34.6 mg, 0.069 mmol) was dissolved in dichloromethane (7 mL). A solution of second generation Grubbs catalyst (9.5 mg, 0.004 mmol) in dichloromethane (0.2 mL) was added thereto at room temperature. The mixture was stirred under a nitrogen atmosphere for 25 hours and concentrated. The residue was purified by silica gel column chromatography (hexane-ethyl acetate) to obtain Compound (83) (29.4 mg, 90%) as a brown foam.
MS(ESI) m/z: 470.10[M-H]-

### Third Step Synthesis of Compound 61

Compound (83) (32.5 mg, 0.069 mmol) was dissolved in a solution of methanol (1 mL), THF (1 mL) and water (1 mL). Lithium hydroxide (10.0 mg, 0.418 mmol) was added thereto at room temperature, and the mixture was stirred at 50°C for 24 hours. 1mol/L aqueous solution of hydrochloric acid (0.75 mL) and brine (10 mL) were added thereto and the mixture was extracted with ethyl acetate (40 mL). The obtained organic layer was washed with brine (10 mL), dried over anhydrous magnesium sulfate, and concentrated. The residue was purified by silica gel column chromatography (chloroform-methanol) to obtain Compound 61 (31.6 mg, 100%) as a colorless solid.
MS: m/z = 456.05 [M-H]-(▲)

### Example 23 Synthesis of Compound 52

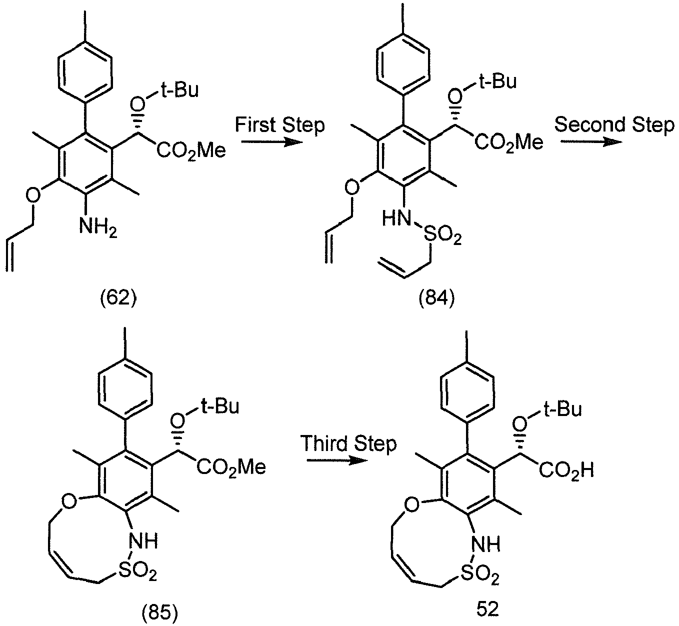

### First Step Synthesis of Compound (84)

Compound (62) (704 mg, 1.71 mmol) was dissolved in pyridine (10 mL). Allylsulfonyl chloride (1.49 mg, 6.20 mmol) was added thereto under ice bath and the mixture was stirred at room temperature over night. The mixture was stirred at 50°C 1 hour. The mixture was poured into a solution of water (40 mL) and 1mol/L aqueous solution of hydrochloric acid (14 mL), and extracted with ethyl acetate (80mL). The obtained organic layer was washed with water (40 mL) and brine (40 mL), dried over anhydrous magnesium sulfate, and concentrated. The residue was purified by silica gel column chromatography (hexane-ethyl acetate) to obtain Compound (84) (302 mg, 34%) as a yellow foam.
MS(ESI) m/z: 514 [M+H]+

### Second Step Synthesis of Compound (85)

Compound (84) (160 mg, 0.310 mmol) was dissolved in dichloromethane (15 mL). Second generation Grubbs catalyst (26.3 mg, 0.031 mmol) was added thereto at room temperature. The mixture was stirred for 5.5 hours and concentrated. The residue was purified by silica gel column chromatography (hexane-ethyl acetate) to obtain Compound (85) (125 mg, 83%) as a brown foam.
MS(ESI) m/z: 486 [M-H]-

### Third Step Synthesis of Compound 52

Compound (85) (109 mg, 0.224 mmol) was dissolved in a solution of THF (1.5 mL), methanol (1 mL) and water (1.5 mL). Lithium hydroxide (26.8 mg, 1.12 mmol) was added thereto at room temperature, and the mixture was stirred at 50°C for 9.5 hours. 2mol/L aqueous solution of hydrochloric acid (1 mL) and water (20 mL) were added thereto and the mixture was extracted with ethyl acetate (40mL). The obtained organic layer was washed with brine (20 mL), dried over anhydrous magnesium sulfate, and concentrated. The residue was purified by silica gel column chromatography (chloroform-methanol) to obtain Compound 52 (59.0 mg, 56%) as a brown foam.
MS: m/z = 472 [M-H]⁻(*)

### Example 24 Synthesis of Compound 54

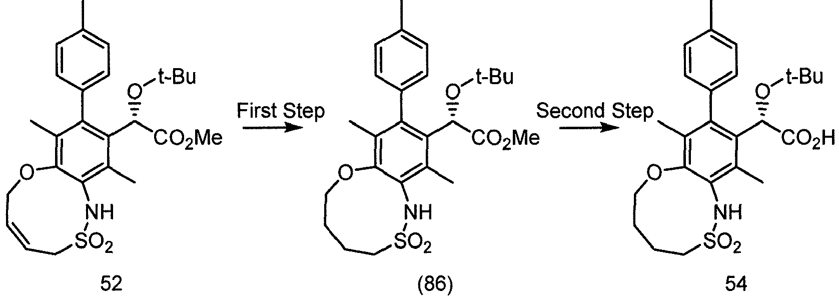

### First Step Synthesis of Compound (86)

Compound 52 (125 mg, 0.256 mmol) was dissolved in a solution of ethyl acetate (2 mL) and methanol (2 mL). Pd/C (25 mg, 10%Wt) was added thereto and the mixture was stirred under a hydrogen atmosphere at room temperature for 3 hours. The mixture was filtered through celite and the filtrate was concentrated. The residue was purified by silica gel column chromatography (hexane-ethyl acetate) to obtain Compound (86) (51.0 mg, 41%) as a colorless foam.
MS: m/z = 488 [M-H]-

### Second Step Synthesis of Compound 54

Compound (86) (51.0 mg, 0.104 mmol) was dissolved in a solution of THF (1 mL), methanol (1 mL) and waater (1 mL). Lithium hydroxide (15.0 mg, 0.625 mmol) was added thereto at room temperature, and the mixture was stirred at 60°C for 9.5 hours. 2mol/L aqueous solution of hydrochloric acid (1 mL) and water (20 mL) were added thereto and the mixture was extracted with ethyl acetate (40 mL). The obtained organic layer was washed with brine (20 mL), dried over anhydrous magnesium sulfate, and concentrated. The residue was purified by silica gel column chromatography (chloroform-methanol) to obtain Compound 54 (37.0 mg, 75%) as a brown foam.
MS: m/z = 474 [M-H]-(*)

### Example 25 Synthesis of Compound 67

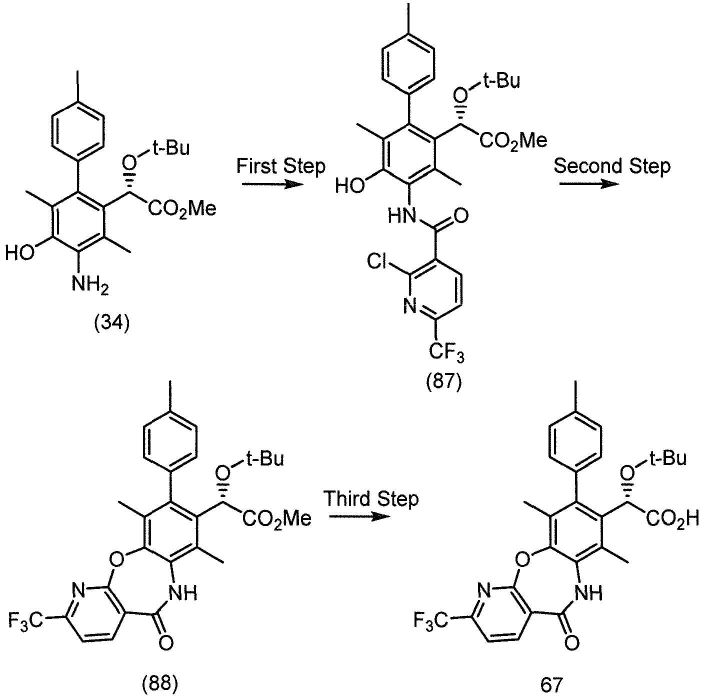

### First Step Synthesis of Compound (87)

Compound (34) (100 mg, 0.269 mmol), 2-chloro-6-trifluoromethyl nicotinic acid (60.7mg, 0.269mmol) and 1-hydroxybenzotriazole monohydrate (41.2 mg, 0.269 mmol) were dissolved in DMF (2 mL), and 1-ethyl-3-(3-dimethylaminopropyl) carbodiimide hydrochloride (51.6 mg, 0.269 mmol) was added thereto, and the mixture was stirred at room temperature for 4 hours. A saturated aqueous solution of ammonium chloride (30 mL) was added thereto and the mixture was extracted with ethyl acetate (50 mL). The obtained organic layer was washed with water (30 mL), a saturated aqueous solution of sodium hydrogen carbonate (30 mL) and brine (50 mL), dried over anhydrous magnesium sulfate, and concentrated. The residue was purified by silica gel column chromatography (hexane-ethyl acetate) to obtain Compound (87) (134 mg, 86%) as a colorless foam.
¹H-NMR (CDCl₃) δ: 0.96 (s, 9H), 1.95 (s, 3H), 2.43 (s, 3H), 2.45 (s, 3H), 3.67 (s, 3H), 5.02 (s, 1H), 7.06 (d, J = 7.8 Hz, 1H), 7.16-7.25 (m, 3H), 7.33 (s, 1H), 7.83 (d, J = 7.9 Hz, 1H), 8.44 (s, 1H), 8.53 (d, J = 7.9 Hz, 1H).
MS: m/z = 577.4 [M-H]-

### Second Step Synthesis of Compound (88)

Compound (87) (130 mg, 0.225 mmol) was dissolved in DMF (2.6 mL). Potassium carbonate (62.1 mg, 0.449 mmol) was added thereto at room temperature, and the mixture was stirred at 100°C for 45 minutes. A saturated aqueous solution of ammonium chloride (30 mL) was added thereto and the mixture was extracted with ethyl acetate (50 mL). The obtained organic layer was washed with water (30 mL×2) and brine (30 mL), dried over anhydrous magnesium sulfate, and concentrated. The residue was purified by silica gel column chromatography (hexane-ethyl acetate) to obtain Compound (88) (110 mg, 91%) as a colorless solid.
¹H-NMR (CDCl₃) δ: 0.94 (s, 9H), 2.23 (s, 3H), 2.42 (d, J = 5.9 Hz, 6H), 3.67 (s, 3H), 5.02 (s, 1H), 7.04 (d, J = 7.4 Hz, 1H), 7.13 (d, J = 7.4 Hz, 1H), 7.20-7.25 (m, 2H), 7.61 (s, 1H), 7.67 (d, J = 7.8 Hz, 1H), 8.50 (d, J = 7.8 Hz, 1H).
MS: m/z = 541.4 [M-H]-

### Third Step Synthesis of Compound 67

Compound (88) (105 mg, 0.194 mmol) was dissolved in ethanol (1.94 mL). 2mol/L aqueous solution of sodium hydroxide (0.484 mL, 0.968 mmol) was added thereto at room temperature, and the mixture was stirred under heat reflux for 45 minutes. 1mol/L aqueous solution of hydrochloric acid (3 mL) and brine (30 mL) were added thereto and the mixture was extracted with ethyl acetate (50 mL). The obtained organic layer was washed with brine (30 mL), dried over anhydrous magnesium sulfate, and concentrated. The residue was purified by silica gel column chromatography (chloroform-methanol) to obtain Compound 67 (66.6 mg, 65%) as a yellow solid.
¹H-NMR (CDCl₃) δ: 0.98 (s, 9H), 2.26 (s, 3H), 2.36 (s, 3H), 2.42 (s, 3H), 5.15 (s, 1H), 7.07 (d, J = 6.8 Hz, 1H), 7.20-7.31 (m, 3H), 7.68 (d, J = 7.7 Hz, 1H), 8.16 (s, 1H), 8.48 (d, J = 7.7 Hz, 1H), 10.27 (s, 0H).
MS: m/z = 527.4 [M-H]-

### Example 26 Synthesis of Compound 42

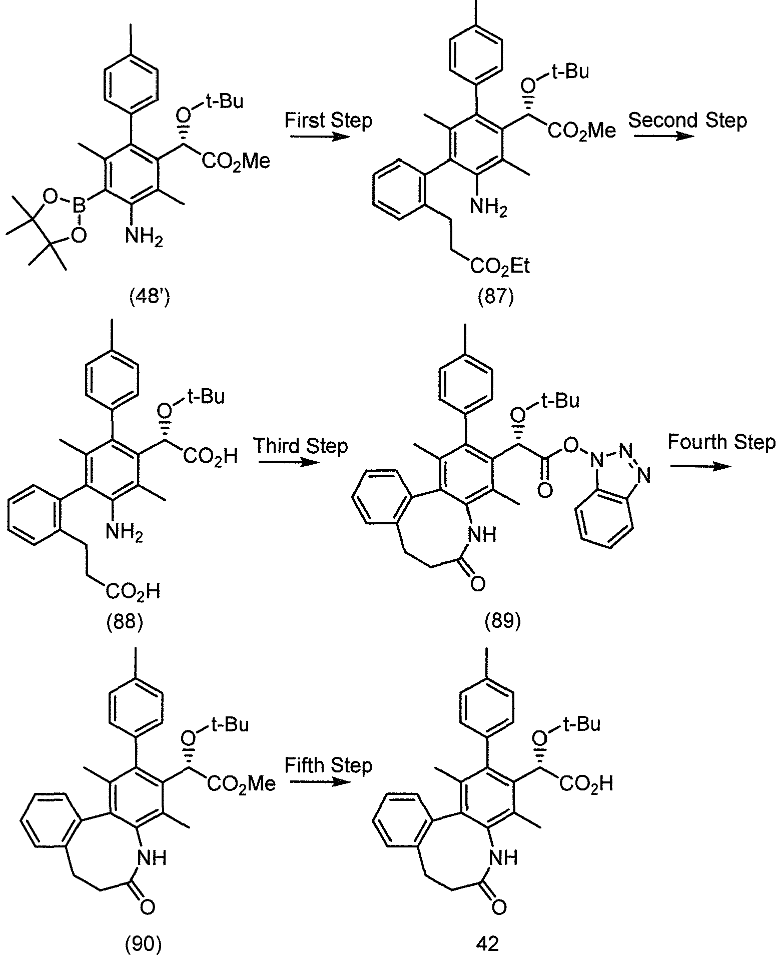

### First Step Synthesis of Compound (87)

Compound (48') (292 mg, 0.607 mmol) which was synthesized from Compound (54) by the same manner as Compound (48), was dissolved in a solution of DMA (6 mL) and (0.6 mL), and 3-(2-bromophey)propionic acid ethyl ester (187 mg, 0.728 mmol), [1,1'-bis(di-tert-butylphosphino)ferrocene]palladium dichloride (79.0 mg, 0.121 mmol) and potassium carbonate (168 mg, 1.21 mmol) were added thereto, and the mixture was stirred under a nitrogen atmosphere at 130°C for 1.5 hours. Water (30 mL) was added thereto and the mixture was extracted with ethyl acetate (60mL). The obtained organic layer was washed with water (30 mL) and brine (30 mL), dried over anhydrous magnesium sulfate, and concentrated. The residue was purified by silica gel column chromatography (hexane-ethyl acetate) to obtain Compound (87) (89.0g, 28%) as a yellow foam.
MS: m/z = 532 [M+H]+

### Second Step Synthesis of Compound (88)

Compound (87) (123 mg, 0.231 mmol) was dissolved in ethanol (3 mL). 2mol/L aqueous solution of sodium hydroxide (0.694 mL, 1.39 mmol) was added thereto at room temperature, and the mixture was stirred at 50°C for 10.5 hours. 2mol/L aqueous solution of hydrochloric acid (3 mL) and water (20 mL) were added thereto and the mixture was extracted with ethyl acetate (40 mL). The obtained organic layer was washed with brine (20 mL) and dried over anhydrous magnesium sulfate to obtain Compound (88) (117 mg, 103%) as a yellow foam.
MS: m/z = 490 [M+H]+

### Third Step Synthesis of Compound (89)

Compound (88) (113 mg, 0.231 mmol), 1-ethyl-3-(3-dimethylaminopropyl)carbodiimide hydrochloride (88.0 mg, 0.462 mmol) and 1-hydroxybenzotriazole (62.4 mg, 0.462 mmol) were dissolved in DMF (5.7 mL), and N,N-diisopropylethylamine (0.081 mL, 0.462 mmol) was added thereto, and the mixture was stirred at room temperature for 29 hours. 2mol/L aqueous solution of hydrochloric acid (10 mL) and water (10 mL) were added thereto and the mixture was extracted with ethyl acetate (40 mL). The obtained organic layer was washed with water (30 mL) and brine (30 mL), dried over anhydrous magnesium sulfate, and concentrated. The residue was purified by silica gel column chromatography (chloroform-methanol) to obtain Compound (89) (78.0 mg, 57%) as a yellow foam.

### Fourth Step Synthesis of Compound (90)

Compound (89) (78.0 mg, 0.132 mmol) was dissolved in methanol (2 mL). 2mol/L aqueous solution of potassium carbonate (0.331 mL, 0.662 mmol) was added thereto at room temperature, and the mixture was stirred at same temperature for 17.5 hours. 2mol/L aqueous solution of hydrochloric acid (4 mL) and water (20 mL) were added thereto and the mixture was extracted with ethyl acetate (40 mL). The obtained organic layer was washed with brine (30 mL), dried over anhydrous magnesium sulfate, and concentrated. The residue was purified by silica gel column chromatography (hexane-ethyl acetate) to obtain Compound (90) (52.0 mg, 81%) as a colorless foam.
MS: m/z = 486 [M+H]+

### Fifth Step Synthesis of Compound 42

Compound (90) (52.0 mg, 0.107 mmol) was dissolved in ethanol (2 mL). 2mol/L aqueous solution of sodium hydroxide (0.268 mL, 0.535 mmol) was added thereto at room temperature, and the mixture was stirred at same temperature for 87.5 hours. 2mol/L aqueous solution of hydrochloric acid (2 mL) and water (20 mL) were added thereto and the mixture was extracted with ethyl acetate (40 mL). The obtained organic layer was washed with brine (20 mL), dried over anhydrous magnesium sulfate, and concentrated. The residue was purified by silica gel column chromatography (chloroform-methanol) to obtain Compound 42 (42.0 mg, 83%) as a yellow foam.
MS: m/z = 472 [M-H]-

### Example 27 Synthesis of Compounds 64 and 81

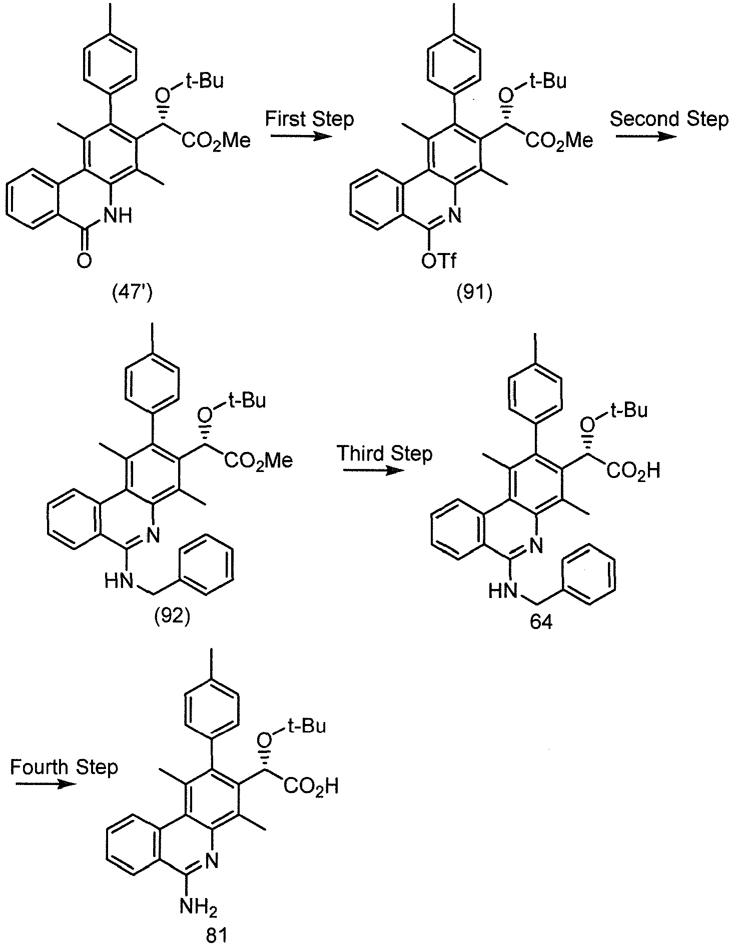

### First Step Synthesis of Compound (91)

Compound (47') (173 mg, 0.378 mmol) which was synthesized from Compound (54) by the same manner as Compound (47), was dissolved in dichloromethane (1.73 mL), and pyridine (0.040 mL, 0.492 mmol) and trifluoromethansulfonic anhydride (0.070 mL, 0.416 mmol) were added thereto under ice bath, and the mixture was stirred for 1 hour. 1mol/L aqueous solution of hydrochloric acid (30 mL) was added thereto and the mixture was extracted with ethyl acetate (50 mL). The obtained organic layer was washed with water (30 mL) and brine (30 mL), dried over anhydrous magnesium sulfate, and concentrated. The residue was purified by silica gel column chromatography (hexane-ethyl acetate) to obtain Compound (91) (206 mg, 94%) as a colorless foam.
¹H-NMR (CDCl₃) δ: 0.96 (s, 9H), 2.47 (s, 3H), 2.62 (s, 3H), 2.82 (s, 3H), 3.73 (s, 3H), 5.15 (s, 1H), 7.22-7.25 (m, 1H), 7.29-7.34 (m, 3H), 7.77 (dd, J = 7.5, 7.5 Hz, 1H), 7.86-7.90 (m, 1H), 8.28 (dd, J = 8.5, 1.7 Hz, 1H), 8.77 (d, J = 8.5 Hz, 1H).
MS: m/z = 590.2 [M+H]+

### Second Step Synthesis of Compound (92)

Compound (91) (50.0 mg, 0.085 mmol) was dissolved in toluene (1 mL). Benzylamine (0.019 mL, 0.170 mmol), cesium carbonate (55.3 mg, 0.170 mmol), xantphos (7.36 mg, 0.013 mmol) and bis(dibenzylideneacetone) palladium (4.9 mg, 0.008 mmol) were added thereto at room temperature, and the mixture was stirred under heat reflux for 3 hours. Water (30 mL) was added thereto and the mixture was extracted with ethyl acetate (50 mL). The obtained organic layer was washed with brine (30 mL), dried over anhydrous magnesium sulfate, and concentrated. The residue was purified by silica gel column chromatography (hexane-ethyl acetate) to obtain Compound (92) (25.8 mg, 56%) as a brown oil.
MS: m/z = 547.5 [M+H]+

### Third Step Synthesis of Compound 64

Compound (92) (25.0 mg, 0.046 mmol) was dissolved in ethanol (1 mL). 2mol/L aqueous solution of sodium hydroxide (0.250 mL, 0.500 mmol) was added thereto, and the mixture was stirred under heat reflux for 45 minutes. 1mol/L aqueous solution of hydrochloric acid (0.5 mL) and brine (30 mL) were added thereto and the mixture was extracted with ethyl acetate (50 mL). The obtained organic layer was dried over anhydrous magnesium sulfate, and concentrated. The residue was purified by silica gel column chromatography (chloroform-methanol) to obtain Compound 64 (24.3 mg, 100%) as a yellow foam.
¹H-NMR (CDCl₃) δ: 0.97 (s, 9H), 2.44 (s, 3H), 2.52 (s, 3H), 2.75 (s, 3H), 4.92 (d, J = 14.5 Hz, 1H), 4.99 (d, J = 14.5 Hz, 1H), 5.26 (s, 1H), 5.62 (s, 1H), 7.26-7.39 (m, 6H), 7.47-7.59 (m, 4H), 7.66 (dd, J = 7.4, 7.4 Hz, 1H), 7.87 (d, J = 7.9 Hz, 1H), 8.57 (d, J = 8.4 Hz, 1H), 9.96 (s, 1H).
MS: m/z = 533.5 [M+H]+

### Fourth Step Synthesis of Compound 81

Compound 64 (56.0 mg, 0.105 mmol) was dissolved in a solution of methanol (1 mL) and acetic acid (0.1 mL). Palladium hydroxide (29.5mg, 10%Wt) was added thereto, and the mixture was stirred under a hydrogen atmosphere at room temperature for 16 hours. The mixture was filtered through celite, and the filtrate was concentrated. The residue was purified by diol silica gel column chromatography (chloroform-methanol) to obtain Compound 81 (7.7 mg, 17%) as a colorless solid.
¹H-NMR (MeOD) δ: 0.86 (s, 9H), 2.37 (s, 3H), 2.44 (s, 3H), 2.62 (s, 3H), 5.06 (s, 1H), 7.19 (d, J = 7.8 Hz, 1H), 7.25-7.29 (m, 3H), 7.76 (dd, J = 7.7, 7.7 Hz, 1H), 7.96 (dd, J = 7.7, 7.7 Hz, 1H), 8.44 (d, J = 7.7 Hz, 1H), 8.60 (d, J = 7.7 Hz, 1H).
MS: m/z = 443.4 [M+H]+

### Example 28 Synthesis of Compound 73

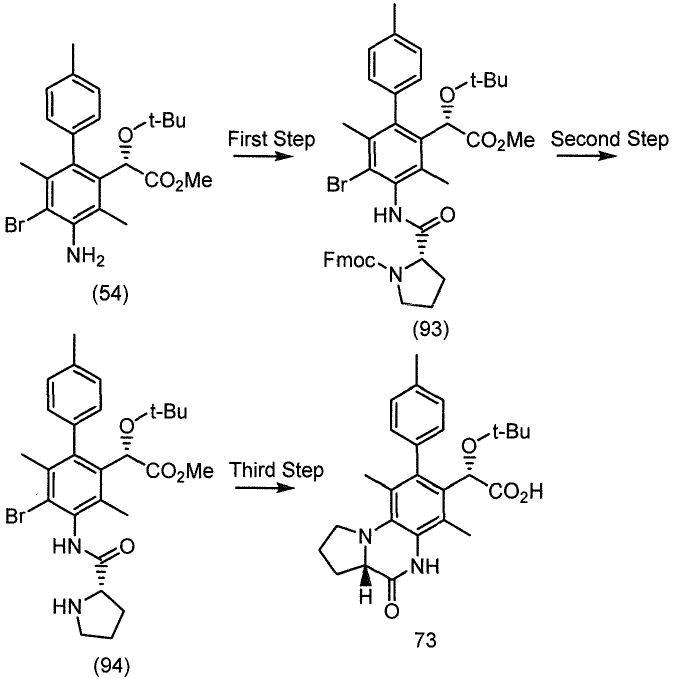

### First Step Synthesis of Compound (93)

Compound (54) (100 mg, 0.230 mmol) was dissolved in pyridine (2 mL), and Fmoc-L-proline acid chloride (316 mg, 0.888 mmol) was added thereto at room temperature, and the mixture was stirred at same temperature for 1 hour. 1mol/L aqueous solution of hydrochloric acid (30 mL) was added thereto and the mixture was extracted with ethyl acetate (50 mL). The obtained organic layer was washed with water (30 mL), a saturated aqueous solution of sodium hydrogen carbonate (30 mL) and brine (30 mL), dried over anhydrous magnesium sulfate, and concentrated. The residue was purified by silica gel column chromatography (hexane-ethyl acetate) to obtain Compound (93) (109 mg, 59%) as a colorless foam.
MS: m/z = 753.4/755.4 [M+H]+

### Second Step Synthesis of Compound (94)

Compound (93) (100 mg, 0.133 mmol) was dissolved in dichloromethane (1 mL). Diethylamine (1 mL) was added thereto at room temperature, and the mixture was stirred at same temperature for 1.5 hours and concentrated. The residue was purified by silica gel column chromatography (chloroform-methanol) to obtain Compound (94) (60.7 mg, 86%) as a colorless oil.
¹H-NMR (CDCl₃) δ: 0.95 (s, 9H), 1.72-1.84 (m, 1H), 1.86-1.97 (m, 1H), 2.07 (s, 3H), 2.14-2.24 (m, 2H), 2.31 (s, 3H), 2.42 (s, 3H), 3.07-3.15 (m, 2H), 3.67 (s, 3H), 3.96 (dd, J = 8.3, 5.8 Hz, 1H), 4.99 (s, 1H), 7.01-7.08 (m, 1H), 7.13-7.18 (m, 1H), 7.19-7.25 (m, 2H), 9.44 (s, 1H).
MS: m/z = 531.4/533.4 [M+H]+

### Third Step Synthesis of Compound 73

Compound (94) (55.0 mg, 0.103 mmol) was dissolved in dioxane (1 mL). Tris(dibenzylideneacetone) palladium(9.48mg, 0.010mmol), 1,3-bis(2,6-diisopropylphenyl)-4,5-dihydro-1H-imidazolium tetrafluoroborate (9.90 mg, 0.021 mmol) and tert-butoxypotassium (46.4 mg, 0.414 mmol) were added thereto, and the mixture was stirred under microwave irradiation at 160 °C for 10 minutes. 1mol/L aqueous solution of hydrochloric acid (3 mL) and brine (30 mL) were added thereto and the mixture was extracted with ethyl acetate (50 mL). The obtained organic layer was dried over anhydrous magnesium sulfate and concentrated. The residue was purified by silica gel column chromatography (chloroform-methanol) to obtain Compound 73 (10.0 mg, 22%) as a yellow solid.
¹H-NMR (CDCl₃) δ: 0.97 (s, 9H), 1.76-1.85 (m, 1H), 1.91 (s, 3H), 1.95-2.01 (m, 1H), 2.16-2.24 (m, 1H), 2.27 (s, 3H), 2.41 (s, 3H), 2.70-2.78 (m, 1H), 2.95 (ddd, J = 8.5, 8.5, 4.8 Hz, 1H), 3.58 (dd, J = 16.1, 7.3 Hz, 1H), 3.87 (d, J = 7.4 Hz, 1H), 5.15 (s, 1H), 7.07 (d, J = 7.5 Hz, 1H), 7.20 (d, J = 7.5 Hz, 1H), 7.25 (d, J = 7.5 Hz, 1H), 7.40 (d, J = 7.5 Hz, 1H), 8.32 (s, 1H).
MS: m/z = 437.4 [M+H]+

### Example 29 Synthesis of Compound 51

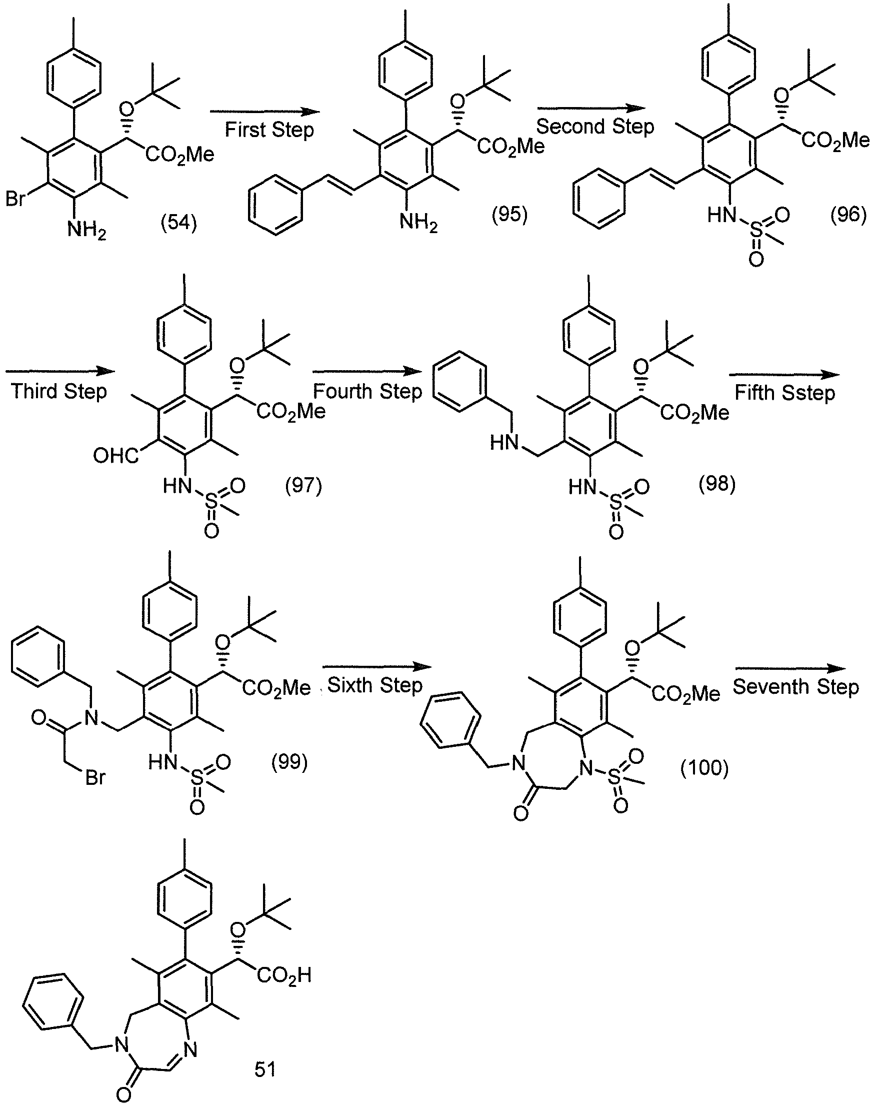

### First Step Synthesis of Compound (95)

Compound (54) (4.34 g, 10.0 mmoL) and (E)-styrylboronic acid (1.78 g, 12.0 mmoL) were suspended in a solution of DMF (43.4 mL) and water (4.34mL), potassium carbonate (4.15 g, 30.0 mmoL) was added thereto, and the operation of degassing and nitrogen substitution was repeated three times. PdCl₂ (dtbpf) (326 mg, 0.500 mmoL) was added thereto, and again, repeated three times the operation of the degassing and nitrogen substitution, and the mixture was stirred at 120°C for 30 minutes. The mixture was cooled to room temperature, poured into a solution of ice-water and 2 mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (95) (4.26g, 93.1%) as a pale yellow foam.
¹H NMR (CDCl₃) δ: 0.98 (s, 9H), 1.91 (s, 3H), 2.26 (s, 3H), 2.41 (s, 3H), 3.68 (s, 3H), 3.92 (s, 2H), 5.02 (s, 1H), 6.81 (d, J = 16.0, 1H), 7.00-7.40 (m, 8H), 7.50-7.56 (m, 2H).

### Second Step Synthesis of Compound (96)

Compound (95) (2.86 g, 6.25 mmoL) was dissolved in pyridine (28.6 mL), and methanesulfonyl chloride (1.46mL, 18.8mmoL) was added thereto, and the mixture was stirred at room temperature for 2 hours 10 minutes. The mixture was poured into a solution of ice-water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was crystallied from hexane-ethyl acetate to obtain Compound (96) (3.00 g, 89.5%)as pale orange crystalline powder.
¹H NMR (CDCl₃)δ:0.97 (s, 9H), 1.55 (s, 3H), 2.42 (s, 3H), 2.50 (s, 3H), 3.03 (s, 3H), 3.69 (s, 3H), 5.04 (s, 1H), 5.98 (s, 1H), 6.66 (d, J = 16.0 Hz, 1H), 7.00-7.43 (m, 8H), 7.40-7.52 (m, 2H).

### Third Step Synthesis of Compound (97)

Compound (96) (3.00 g, 5.59 mmoL) and pyridine (1.35 mL, 16.8 mmoL) were dissolved in dichloromethane (50.0 mL), and the mixture was cooled to -78°C under dryice bath. An ozone gas was passed through the mixture for 31 minutes and then a nitrogen gas for 17 minutes. The mixture was warmed up to room temperature, and the mixture was stirred for 1 hour 25 minutes. The mixture was poured into a solution of ice-water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (97) (690 mg, 26.7%) as a yellow foam.
¹H NMR (CDCl₃)δ:0.97(s, 9H), 2.25 (s, 3H), 2.43 (s, 3H), 2.49 (s, 3H), 2.97 (s, 3H), 3.69 (s, 3H), 5.05 (s, 1H), 7.00-7.30 (m, 4H), 6.73-6.99 (m, 3H), 8.64 (s, 1H), 10.55 (s, 1H).

### Fourth Step Synthesis of Compound (98)

Compound (97) (690 mg, 1.50 mmoL) was dissolved in dichloroethane (7.00 mL), and acetic acid (0.171 mL, 2.99 mmoL) and benzylamine (0.196 mL, 1.79 mmoL) were added thereto, and the mixture was stirred at 60°C for 2 hours 48 minutes. The mixture was cooled to room temperature, and sodium triacetoxyborohydride (500 mg, 2.24 mmoL) was added thereto, and the mixture was stirred at room temperature for 3 hours 20 minutes. The mixture was poured into a solution of ice-water and a saturated aqueous solution of sodium hydrogen carbonate and extracted with ethyl acetate. The obtained organic layer was washed with brine, dried over anhydrous sodium sulfate, and concentrated under reduced pressure. The residue was purified by silica gel column chromatography (hexane-ethyl acetate) to obtain Compound (98) (633 mg, 76.6%) as a pale yellow foam.
¹H NMR (CDCl₃)δ:0.94(s, 9H), 1.89 (s, 3H), 2.41 (s, 3H), 2.44 (s, 3H), 3.13 (s, 3H), 3.66 (s, 3H), 3.87 (s, 2H), 4.05 (s, 2H), 4.98 (s, 1H), 7.01 (m, 1H), 7.10-7.40 (m, 9H).

### Fifth Step Synthesis of Compound (99)

Compound (98) (83.0 mg, 0.150 mmoL) was dissolved in dichloromethane (2.00 mL), and the mixture was cooled under ice- water bath including sodium chloride. Pyridine (0.0240 mL, 0.300 mmoL) and bromoacetyl chloride (0.0220 mL, 0.225 mmoL) were added thereto, and the mixture was stirred under ice-water bath including sodium chloride for 57 minutes. The mixture was poured into a solution of ice-water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (99) (96.0 mg, 94.9%) as a yellow oil.
¹H NMR (CDCl₃)δ:0.96(s, 9H), 1.85 (s, 3H), 2.42 (s, 6H), 3.07 (s, 2H), 3.69 (s, 3H), 3.85 (s, 2H), 4.53 (s, 2H), 4.70-5.00 (m, 2H), 5.02 (s, 1H), 6.75-7.45 (m, 10H).

### Sixth Step Synthesis of Compound (100)

Compound (99) (94.0 mg, 0.140 mmoL) was dissolved in DMF (2.00 mL), and potassium carbonate (38.6 mg, 0.279 mmoL) was added thereto, and the mixture was stirred at 50°C for 1 hour 8 minutes. The mixture was poured into a solution of ice-water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (100) (42.0mg, 50.8%) and the diastereomer (31.0mg, 37.5%) as a each white foam.
LC/MS(ESI)r.t.= 2.65 min, m/z:593.15 [M + H]+ and r.t.= 2.66 min, m/z:593.15 [M + H]+.

### Seventh Step Synthesis of Compound 51

Compound (100) (40.0 mg, 0.0670 mmol) was dissolved in ethanol (1.35 mL), and 2mol/L aqueous solution of sodium hydroxide (0.337 mL, 0.675 mmoL) was added thereto, and the mixture was stirred at 90°C for 3 hours 16 minutes. The mixture was cooled to room temperature and poured into ice water, and obtained aqueous layer was adjusted with 2moL/L aqueous solution of hydrochloric acid to about pH=3 and extracted with ethyl acetate. The obtained organic layer was washed with brine, dried over anhydrous sodium sulfate, and concentrated under reduced pressure. The residue was purified by silica gel column chromatography (chloroform-methanol) and dried at 60°C under reduced pressure to obtain Compound 51 (23.0 mg, 68.4%) as a white foam.
¹H NMR (DMSO-d₆)δ:0.86(s, 9H), 1.55 (br s, 1H), 2.36 (s, 6H), 4.25 (br s, 2H), 4.51 (m, 1H), 4.82 (br s, 2H), 6.86 (br s, 1H),7.00-7.35 (m, 8H), 8.29 (s, 1H), 12.57 (br s, 1H). MS(ESI) m/z:499.3 [M + H]+

### Example 30 Synthesis of Compound 53

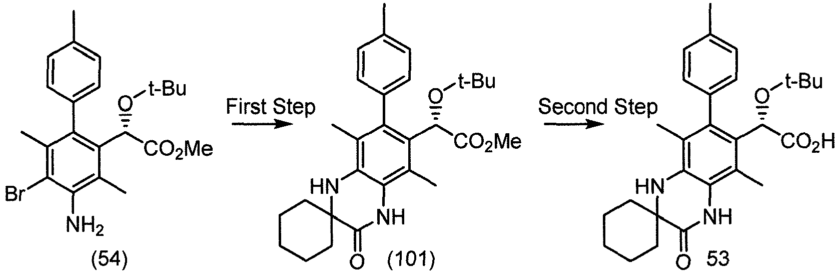

### First Step Synthesis of Compound (101)

Compound (54) (500 mg, 1.15 mmol) was dissolved in DMSO (10 mL), and 1-aminocyclohexane carboxylic acid (330 mg, 0.30 mmol), cesium carbonate (750 mg, 0.30 mmol) and copper iodide (66 mg, 0.345 mmol) were added thereto, and the mixture was stirred in a sealed tube at 130°C for 7 hours. The mixture was cooled to room temperature, and water (100 mL) was added thereto. The mixture was extracted with ethyl acetate (100 mL). The obtained organic layer was washed with water (100 mL) and brine (100 mL), dried over anhydrous magnesium sulfate, and concentrated. The residue was purified by silica gel column chromatography (hexane-ethyl acetate) to obtain Compound (101) (262 mg, 47%) as a colorless solid.
MS: m/z = 478 [M+H]⁺

### Second Step Synthesis of Compound 53

Compound (101) (126 mg, 0.263 mmol) was dissolved in a solution of methanol (1 mL) and THF (3 mL), and 2mol/L aqueous solution of sodium hydroxide (1.32 mL, 2.54 mmol) was added thereto at room temperature, and the mixture was stirred at 60°C for 7 hours. 2mol/L aqueous solution of hydrochloric acid (1.3mL) and brine (30 mL) were added thereto and the mixture was extracted with ethyl acetate (50mL). The obtained organic layer was dried over anhydrous magnesium sulfate and concentrated. The residue was purified by HPLC to obtain Compound 53 (28 mg, 23%) as a colorless solid.
¹H-NMR (CDCl₃) δ: 0.95 (s, 9H), 1.35-1.50 (m, 4H), 1.68-1.80 (m, 6H),1.88 (s, 3H), 2.00-2.10 (m, 1H), 2.14 (s, 3H), 2.41 (s, 3H), 4.17(s, 1H), 5.08 (s, 1H), 7.05 (d, J = 8.0 Hz, 1H), 7.20-7.26 (m, 2H), 7.38 (d, J = 4.0 Hz, 1H), 7.55 (s, 1H)
MS: m/z = 449 [M-H]⁻

### Example 31 Synthesis of Compound 71

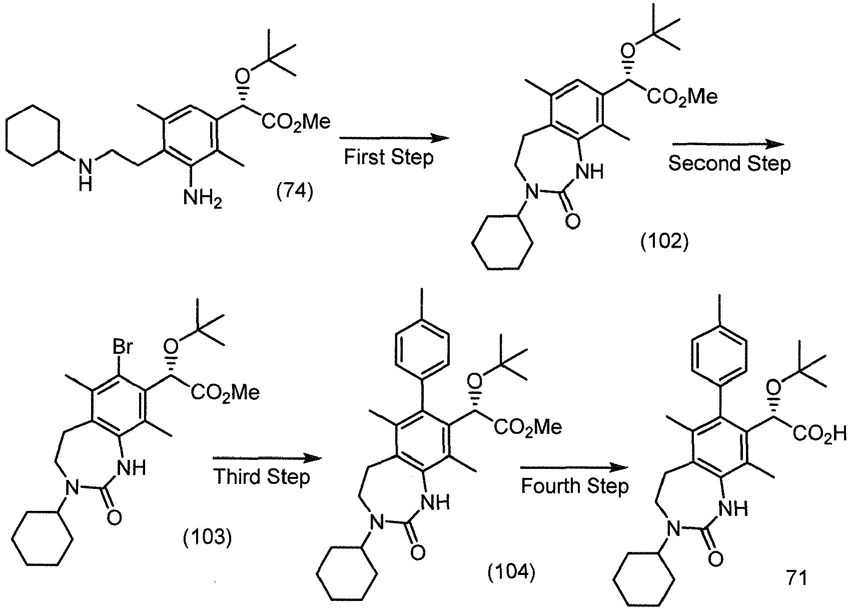

### First Step Synthesis of Compound (102)

Compound (74) (260 mg, 0.666 mmoL) was dissolved in dioxane (6.00 mL), and N,N-diisopropylethylamine (1.16 mL, 6.66 mmoL) and carbonyldiimidazole (540 mg, 3.33 mmoL) were added thereto, and the mixture was stirred at 120°C for 1 hour 22 minutes. The mixture was cooled to room temperature, poured into a solution of ice water and 2mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (102) (195 mg, 70.3%) as a white solid.
¹H NMR (CDCl₃) δ: 1.00-1.75 (m, 19H), 2.22 (s, 3H), 2.32 (s, 3H), 2.80-2.95 (m, 2H), 3.30-3.50 (m, 2H), 3.67 (s, 3H), 4.14 (m, 1H), 5.19 (s, 1H), 6.24 (s, 1H), 7.02 (s, 1H).

### Second Step Synthesis of Compound (103)

Compound (102) (193 mg, 0.463 mmoL) was dissolved in DMF (2.00 mL), and the mixture was cooled under ice bath. NBS (83.0 mg, 0.463 mmoL) was added thereto and the mixture was stirred at room temperature for 1 hour 22 minutes. The mixture was poured into a solution of ice water and 2moL/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (103) (215 mg, 93.7%) as a pale yellow solid.
¹H NMR (CDCl₃) δ: 1.00-1.75 (m, 19H), 2.33 (s, 3H), 2.42 (s, 3H), 2.90-3.10 (m, 2H), 3.35-3.60 (m, 2H), 3.69 (s, 3H), 4.13 (m, 1H), 6.01 (s, 1H), 6.23 (s, 1H).

### Third Step Synthesis of Compound (104)

Compound (103) (100 mg, 0.202 mmoL) and 4-methylphenyl boronic acid (41.2 mg, 0.303 mmoL) were suspended in a solution of DMF (1.00 mL) and water (0.100 mL), and potassium carbonate (112 mg, 0.807 mmoL) was added thereto, the operation of degassing and nitrogen substitution was repeated three times.
PdCl₂ (dtbpf) (13.2 mg, 0.807 mmoL) was added thereto, and again, repeated three times the operation of the degassing and nitrogen substitution, and the mixture was stirred at 120°C for 17 minutes. The mixture was cooled to room temperature, poured into a solution of oce water and 2mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (104) (101mg, 98.8%) as a pale yellow foam.
¹H NMR (CDCl₃) δ: 0.90 (s, 9H), 1.00-1.85 (m, 10H), 1.86 (s, 3H), 2.36 (s, 3H), 2.41 (s, 3H), 2.90-3.05 (m, 2H), 3.40-3.50 (m, 2H), 3.67 (s, 3H), 4.13 (m, 1H), 4.99 (s, 1H), 6.33 (s, 1H), 6.95-7.10 (m, 2H), 7.15-7.25 (m, 2H).

### Fourth Step Synthesis of Compound 71

Compound (104) (99.0 mg, 0.19 5mmoL) was dissolved in ethanol (1.95 mL), and 2moL/L aqueous solution of sodium hydroxide (0.488 mL, 0.977 mmoL) was added thereto, and the mixture was stirred at 90°C for 1 hour 27 minutes. The mixture was cooled to room temperature and poured into ice water, and obtained aqueous layer was adjusted with 2moL/L aqueous solution of hydrochloric acid to about pH=3 and extracted with ethyl acetate. The obtained organic layer was washed with brine, dried over anhydrous sodium sulfate, and concentrated under reduced pressure. The residue was purified by silica gel column chromatography (chloroform-methanol) and dried at 60°C under reduced pressure to obtain Compound 71 (76.0 mg, 79.0%)as a white crystalline powder.
¹H NMR (DMSO-d₆)δ:0.84 (s, 9H), 1.00-1.80 (m, 10H), 1.82 (s, 3H), 2.24 (s, 3H), 2.37 (s, 3H), 2.85-2.95 (m, 2H), 3.30-3.50 (m, 2H), 3.42 (m, 1H), 4.84 (s, 1H), 7.00-7.15 (m, 2H), 7.20-7.35 (m, 3H), 12.46 (br s, 1H). MS(ESI) m/z:493.3 [M + H]+

### Example 32 Synthesis of Compound 78

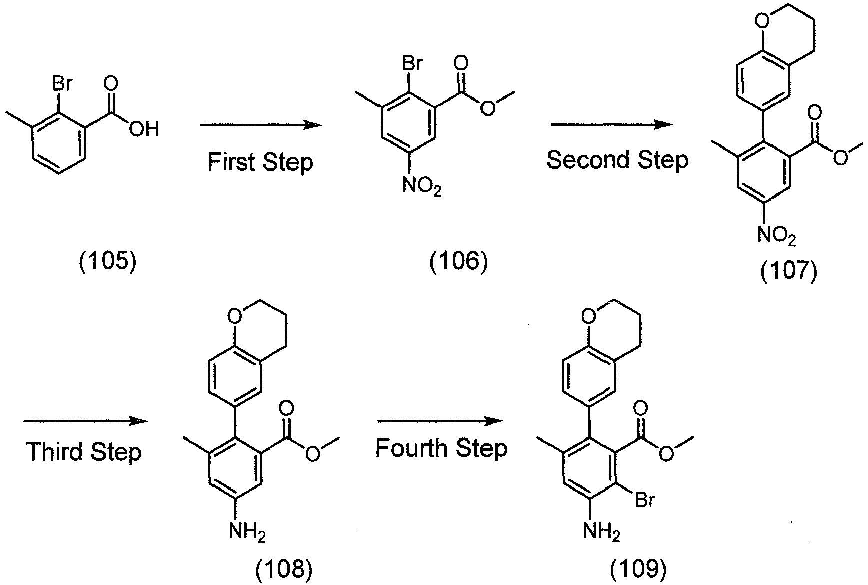

### First Step Synthesis of Compound (106)

Compound (105) (100 g, 456 mmol) and conc sulfuric acid (300 mL) were stirred under ice-water bath including sodium chloride, and white fuming nitric acid (40 mL, 456 mmol) was added dropwise thereto at 0°C. The mixture was stirred for 2 hours and poured into ice, and precipitated solid was filtered. The obtain solid was dissolved in ethyl acetate (500 mL) and the mixture was washed with water and brine, dried over sodium sulfate, and concentrated. The residue was dissolved in thionyl chloride (270 mL), and the mixture was stirred under heat reflux for 1 hour and concentrated. To the residue was added methanol (400 mL), and the mixture was stirred under heat reflux for 1 hour and concentrated. The residue was recrystallized from ethyl acetate to obtain Compound (106) (72 g).
¹H-NMR(CD3OD) δ:2.59(s,3H), 3.98(s,3H), 8.32(s,2H)

### Second Step Synthesis of Compound (107)

Compound (106) (50 g, 183 mmol), 2-(chromane-6-yl)-4,4,5,5-tetramethyl-1,3,2-dioxaborolane (57.8 g, 220 mmol) and Cs₂ CO₃ (178 g, 550 mmol) were dissolved in a solution of dioxane (400 mL) and water (80 mL), and Pd(dppf)Cl₂ (2 g, 2.4 mmol) was added thereto under a nitrogen atmosphere at room temperature, and the mixture was stirred with heating at 90°C for 14 hours. The mixture was cooled to room temperature, and water was added thereto, and the mixture was extracted with ethyl acetate. The obtained organic layer was washed with brine, dried over anhydrous sodium sulfate. The result was purified by silica gel column chromatography (petroleum ether : ethyl acetate = 8:1) to obtain Compound (107) (47.2g).
LC-MS (ESI): m/z=328[M+H]⁺.

### Third Step Synthesis of Compound (108)

Compound (107) (47.2 g, 145 mmol) was dissolved in methanol (200 mL), and Pd/C (20 g, 10%Wt) was added thereto under a nitrogen atmosphere. The mixture was stirred under a hydrogen atmosphere at room temperature for 14 hours. The mixture was filtered through celite, and the filtrate was concentrated to obtain crude Compound (108) (42 g). The residue was used in the next reaction without purification.
1HNMR (CDCl₃):2.04-2.06(m, 2H), 2.24(s,3H), 2.80(s,2H), 3.66(s,3H),4.23-4.25(m, 2H),6.80-6.85(m, 3H), 8.23(s,1H), 8.46 (s,1 H).

### Fourth Step Synthesis of Compound (109)

Compound (108) (42 g, 142 mmol) was dissolved in DMF (200 mL), and a solution of NBS (25.2 g, 142 mmol) in DMF (50 mL) was added dropwise thereto under ice bath, and the mixture was stirred for 10 minutes. Water was added thereto and the mixture was extracted with ethyl acetate. The obtained organic layer was washed with brine, dried over sodium sulfate, and concentrated. The residue was purified by silica gel column chromatography (petroleum ether : ethyl acetate = 8:1) to obtain Compound (109) (23 g).
LC-MS (ESI): m/z=376[M+H]+.
1HNMR (CDCl₃): 2.01-2.04(m,5H), 2.75-2.78(m,2H), 3.59(s,3H), 4.19-4.21(m,2H), 6.71-6.76(m,2H), 6.85-6.89 (m,2 H).

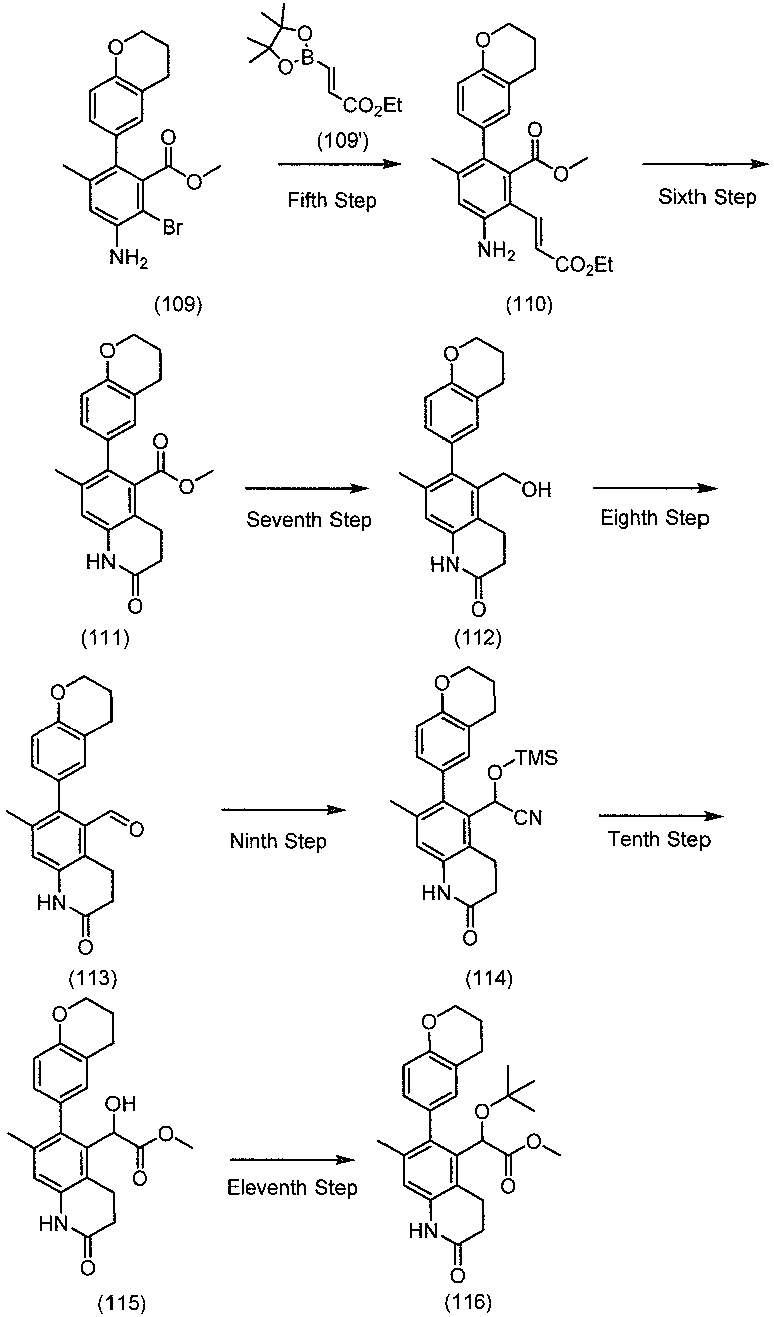

### Fifth Step Synthesis of Compound (110)

To a solution of Compound (109) in 1,4-dioxane/water (100 mL/10 mL) were added cesium carbonate (48.5 g, 149 mmol) and Pd(dppf)Cl₂, and Compound (109') (15.7 g, 69.4 mmol) was added thereto under a nitrogen atmosphere, and the mixture was stirred at 85°C for 14 hours. The mixture was cooled to room temperature, and water was added thereto, and the mixture was extracted with ethyl acetate. The obtained organic layer was dried over sodium sulfate and concentrated. The residue was purified by silica gel column chromatography to obtain Compound (110) (15.0 g).
LC/MS (ESI): m/z=396 [M+H]⁺

### Sixth Step Synthesis of Compound (111)

Compound (110) (15.0 g, 38 mmol) was dissolved in ethanol (500 mL), and Pd/C (7 g, 10%Wt), and the mixture was stirred under a hydrogen atmosphere at 40°C for 4 hours. The mixture was stirred at 80°C and then cooled to room temperature. The mixture was filtered through celite and washed with dichloromethane (200 mL×3), and the filtrate was dried over sodium sulfate and concentrated to obtain crude Compound (111) (9.5 g). The compound was used in the next reaction without purification.
LC/MS (ESI): m/z=351 [M+H]⁺

### Seventh Step Synthesis of Compound (112)

Compound (111) (9.5 g, 27 mmol) was dissolved in dichloromethane, and 1M solution of diisobutylaluminum hydride in toluene (81 mL, 81 mmol) was added dropwise thereto at -60 °C, and the mixture was stirred for 2 hours. The mixture was warmed up to room temperature, and 1M aqueous solution of hydrochloric acid was added thereto. The mixture was extracted with ethyl acetate (300 mL×3). The obtained organic layer was washed with water and brine, dried over sodium sulfate, and concentrated. The residue was purified by silica gel column chromatography to obtain Compound (112) (4.8 g).
LC/MS (ESI): m/z=323 [M+H]⁺

### Eighth Step Synthesis of Compound (113)

Compound (112) (4.8 g, 14.9 mmol) was dissolved in DMSO, and 2-iodoxybenzoic acid (8.3 g, 29.7 mmol) was added thereto, and the mixture was stirred at 45°C. Water was added thereto and the mixture was extracted with ethyl acetate. The obtained organic layer was washed with water and brine, dried over sodium sulfate, and concentrated. The residue was purified by silica gel column chromatography to obtain Compound (113) (3.5 g).
LC/MS (ESI): m/z=321 [M+H]⁺

### Ninth Step Synthesis of Compound (114)

Compound (113) (3.5 g, 10.9 mmol) was dissolved in dichloromethane (200 mL), and zinc iodide (3.5 g, 11.0 mmol) was added thereto, and then TMSCN (3.3 g, 32.7 mmol) was added thereto under ice bath. The mixture was stirred, and water (100 mL) was added thereto, and the mixture was extracted with dichloromethane (100 mL×2). The obtained organic layer was dried over sodium sulfate and concentrated to obtain crude Compound (112) (5.0 g). The compound was used in the next reaction without purification.

### Tenth Step Synthesis of Compound (115)

Compound (114) (5g, 10.9mmol) was dissolved in methanol (250 mL), and hydrogen chloride gas was blown thereto, and the mixture was stirred at 60°C for 14 hours. The mixture was cooled to room temperature, and water was added thereto, and the mixture was extracted with ethyl acetate (100 mL×3). The obtained organic layer was dried over sodium sulfate and concentrated to obtain crude Compound (115) (2.6 g). The compound was used in the next reaction without purification.

### Eleventh Step Synthesis of Compound (116)

Compound (115) (2.6 g, 6.8 mmol) was dissolved in tert-butyl acetate (100 mL), and perchloric acid (8.0 g, 80 mmol, 70%) was added thereto under a nitrogen atmosphere, and the mixture was stirred at room temperature for 30 minutes. The mixture was pored into brine and extracted with ethyl acetate. (100 mL×3). The obtained organic layer was dried over sodium sulfate, and concentrated. The residue was purified by silica gel column chromatography to obtain Compound (116) (2.4 g). 1H-NMR (400 MHz, DMSO-d6) δ:10.01 (s, 1H), 6.88-6.73 (m, 4H), 4.95 (d, 1H, J=5.6 Hz), 4.18 (s, 2H), 3.62-3.58 (m, 3H), 3.07 (m, 1H),2.83-2.76 (m, 3H), 2.35-2.31 (m, 3H), 1.99-1.89 (m, 5H), 0.90 (s, 9H).

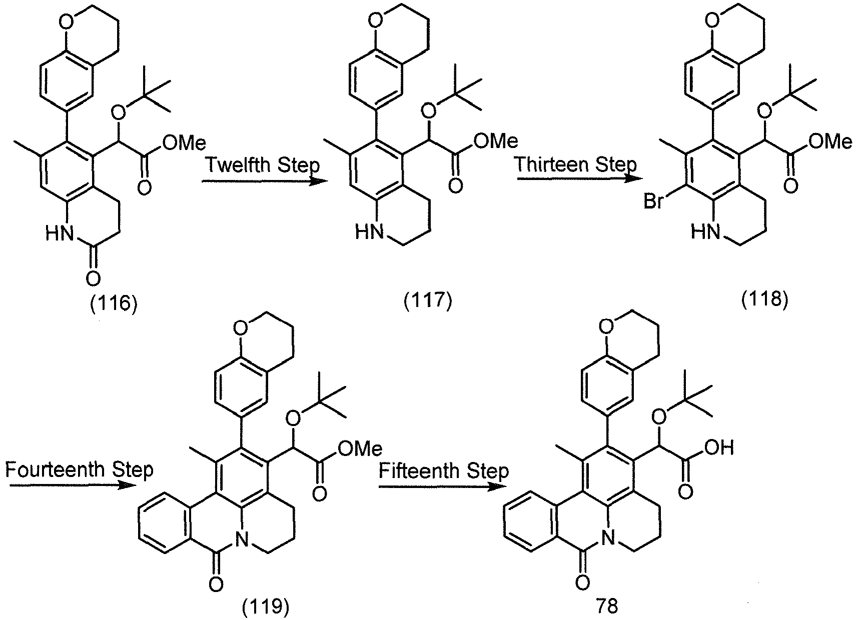

### Twelfth Step Synthesis of Compound (117)

To a solution of Compound (116) (2.9 g, 6.63 mmol) in THF (30 mL) was added 1mol/L solution of borane in THF (19.9mL, 19.9mmol) under a nitrogen atmosphere, and the mixture was stirred at 50°C for 1 hour. Methanol (5 mL) and water (25 mL) were added thereto under ice bath, and the mixture was extracted with ethyl acetate (20 mL×2). The obtained organic layer was washed with water and brine, dried over sodium sulfate, and concentrated. The residue was purified by silica gel column chromatography (hexane-ethyl acetate) to obtain Compound (117) (2.70 g, 96.2%) as a white foam.
MS(ESI) m/z: 424.15(M+H⁺)

### Thirteenth Step Synthesis of Compound (118)

To a solution of Compound (117) (2.6 g, 6.14 mmol) in dichloromethane (26 mL) was added NBS (1.20 g, 6.75 mmol) under ice bath, and the mixture was stirred for 1 hour. A saturated aqueous solution of sodium bicarbonate (20 mL) was added thereto, and the mixture was extracted with dichloromethane (20 mL×2). The obtained organic layer was washed with water and brine, dried over sodium sulfate, and concentrated. The residue was purified by silica gel column chromatography (hexane-ethyl acetate) to obtain Compound (118) (2.73 g, 88.5%) as a white foam.
MS(ESI) m/z: 502.05(M+H⁺)

### Fourteenth Step Synthesis of Compound (119)

To a solution of Compound (118) (100 mg, 0.199 mmol) in DMF (1 mL) were added 2mol/L aqueous solution of potassium carbonate (0.30 mL, 0.59 mmol), Ethyl 2-(4,4,5,5-tetramethyl-1,3,2-dioxaborolane-2-yl) benzoate (110mg, 0.398mmol) and [1,1-bis(di-tert-butylphosphino)ferrocene] palladium dichloride (13.0 mg, 0.02 mmol), and the mixture was stirred under a nitrogen atmosphere at 120°C for 30 minutes. Water (2 mL) was added thereto, and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain crude compound. To the crude compound was added acetic acid (1 mL), and the mixture was stirred at room temperature for 30 minutes and concentrated to obtain crude Compound (119).
MS(ESI) m/z: 526.15(M+H⁺)

### Fifteenth Step Synthesis of Compound 78

To a solution of crude compound (119) in methanol (1 mL) was added 2mol/L aqueous solution of sodium hydroxide (1 mL, 1.99 mmol), and the mixture was stirred at 60°C for 3 hours. 2mol/L aqueous solution of hydrochloric acid (2 mL) was added thereto, and the mixture was extracted with ethyl acetate (10 mL×2). The obtained organic layer was washed with water and brine, dried over sodium sulfate, and concentrated. The residue was purified by silica gel column chromatography (chloroform) to obtain Compound 78 (45.1 mg, 44.1% in 3 steps) as a white foam. MS(ESI) m/z: 512.15 (M+H⁺)(*)
¹H-NMR(400MHz, CDCl₃): δ1.01(9H, s), 1.98-2.18(4H, m), 2.48(1.5H, s), 2.50(1.5H, s), 2.63-2.94(3H, m), 3.35-3.44(1H, m), 4.13-4.38(4H, m), 5.31(1H, s), 6.89(1H, dd, J=8.8Hz, 2.3Hz), 6.98-7.11(1H, m), 7.22(1H, s), 7.57(1H, dd, J=7.8Hz, 7.8Hz), 7.68(1H, dd, J=7.8Hz, 7.8Hz), 8.21(1H, d, J=8.1Hz), 8.55(1H, d, J=8.1Hz), 9.68(1H, brs)

### Example 33 Synthesis of Compound 79

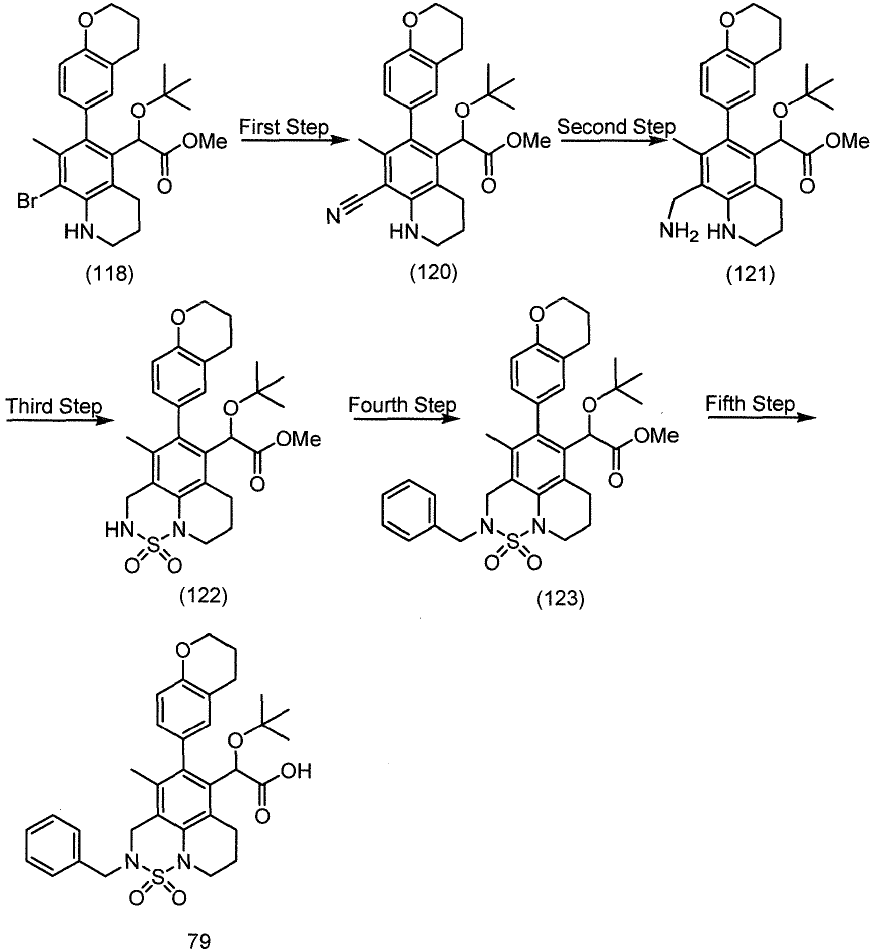

### First Step Synthesis of Compound (120)

To a solution of Compound (118) (300 mg, 0.597 mmol) in DMF (3 mL) were added zinc cyanide (140 mg, 1.19 mmol) and Pd(PPh₃)₄ (69.0 mg, 0.06 mmol), and the mixture was stirred under a nitrogen atmosphere at 120°C for 3 hours. Water (6 mL) was added thereto and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain Compound (120) (262 mg, 97.8%) as a white foam.
MS(ESI) m/z: 449.15 (M+H⁺)

### Second Step Synthesis of Compound (121)

To a solution of Compound (120) (200 mg, 0.446 mmol) in acetic acid (2 mL) was added Pd/C (20mg, 10%Wt), and the mixture was stirred under a hydrogen atmosphere at 80°C for 1 hour. The mixture was filtered through celite, and the filtrate was concentrated to obtain crude Compound (121).

### Third Step Synthesis of Compound (122)

To a solution of crude Compound (121) in xylene (2 mL) were added sulfamic acid (86mg, 0.893mmol) and triethylamine (0.186 mL, 1.34 mmol), and the mixture was stirred under heat reflux for 1.5 hours and then concentrated. The residue was purified by silica gel column chromatography (hexane-ethyl acetate) to obtain Compound (122) (121.4 mg, 52.9% in 2 steps) as a white foam.
MS(ESI) m/z: 537.10 (M+Na⁺)

### Fourth Step Synthesis of Compound (123)

To a solution of Compound (122) (50 mg, 0.097 mmol) in DMF (1 mL) were added sodium hydride (7.7 mg, 0.194 mmol) and benzyl bromide (0.023 mL, 0.194 mmol), and the mixture was stirred at room temperature for 30 minutes. 2mol/L aqueous solution of hydrochloric acid (2 mL) was added thereto and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain Compound (123) (57 mg, 97.0%) as a white foam.
MS(ESI) m/z: 627.30 (M+Na⁺)

### Fifth Step Synthesis of Compound 79

To a solution of Compound (123) (55 mg, 0.091 mmol) in methanol (1 mL) was added 2mol/L aqueous solution of sodium hydroxide (0.455 mL, 0.909 mmol), and the mixture was stirred at 60°C for 1 hour. 2mol/L aqueous solution of hydrochloric acid (2 mL) was added thereto and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain Compound 79 (50 mg, 93.1%) as a white foam.
MS(ESI) m/z: 591.10 (M+H⁺)(*)
¹H-NMR(400MHz, CDCl₃): δ1.02(9H, s), 1.69(0.5H, s), 1.71(0.5H, s), 1.90-2.17(4H, m), 2.45-2.59(1H, m), 2.67-2.87(2H, m), 3.06-3.17(1H, m), 3.54-3.65(1H, m), 3.91-4.05(2H, m), 4.19-4.53(5H, m), 5.19(0.5H, s), 5.20(0.5H, s), 6.72-6.87(2H, m), 7.13(1H, brs), 7.29-7.41(5H, m), 9.66(1H, brs)

### Example 34 Synthesis of Compound 80

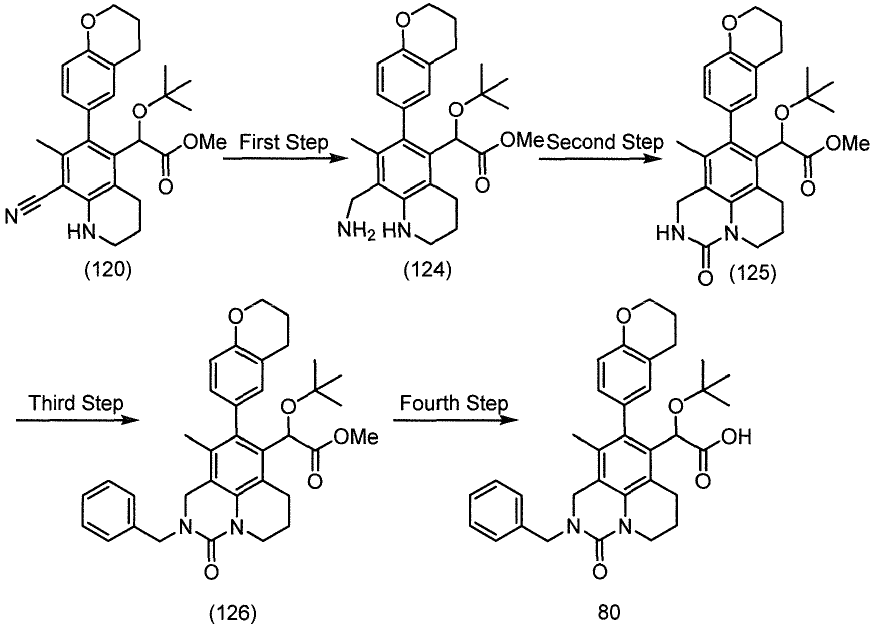

### First Step Synthesis of Compound (124)

To a solution of Compound (120) (230 mg, 0.513 mmol) in acetic acid (2 mL) was added Pd/C (23 mg, 10%Wt), and the mixture was stirred under a hydrogen atmosphere at 80°C for 1 hour. The mixture was filtered through celite, and the filtrate was concentrated to obtain crude Compound (124).

### Second Step Synthesis of Compound (125)

To a solution of the crude Compound (124) in xylene (2 mL) were added urea (61.6 mg, 1.03 mmol) and triethylamine (0.213 mL, 1.54 mmol), and the mixture was stirred under heat reflux for 2 hours and concentrated. The residue was purified by silica gel column chromatography (hexane-ethyl acetate) to obtain Compound (125) (64.5 mg, 26.3% in 2 steps) as a white foam.
MS(ESI) m/z: 479.20(M+Na⁺)

### Third Step Synthesis of Compound (126)

To a solution of Compound (125) (60 mg, 0.125 mmol) in DMF (1 mL) were added sodium hydride (10.0 mg, 0.251 mmol) and benzyl bromide (0.03 mL, 0.251 mmol), and the mixture was stirred at room temperature for 1 hour. 2mol/L aqueous solution of hydrochloric acid (2 mL) was added thereto and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain Compound (126) (40 mg, 56.1%) as a white foam.
MS(ESI) m/z: 589.15 (M+Na⁺)

### Fourth Step Synthesis of Compound 80

To a solution of Compound (126) (40 mg, 0.07 mmol) in methanol (1 mL) was added 2mol/L aqueous solution of sodium hydroxide (0.35 mL, 0.703 mmol), and the mixture was stirred at 60°C for 1 hours. 2mol/L aqueous solution of hydrochloric acid (2 mL) was added thereto and the mixture was extracted with ethyl acetate (10 mL×2). The obtained organic layer was washed with water and brine, dried over sodium sulfate, and concentrated. The residue was purified by silica gel column chromatography (hexane-ethyl acetate) to obtain Compound 80 (33 mg, 84.6%) as a white foam.
MS(ESI) m/z: 555.20 (M+H⁺)
¹H-NMR(400MHz, CDCl₃): δ1.01(9H, s), 1.74(1.5H, s), 1.75(1.5H, s), 1.79-1.93(1H, m), 1.95-2.09(4H, m), 2.42-2.54(1H, m), 2.67-2.85(2H, m), 2.93-3.05(1H, m), 3.55-3.72(1H, m), 4.18-4.27(4H, m), 4.62-4.74(2H, m), 5.16(1H, s), 6.78(1H, d, J=8.3Hz), 6.82(1H, d, J=8.3Hz), 7.12(1H, brs), 7.27-7.38(5H, m), 9.62(1H, brs)

### Example 35 Synthesis of Compound 87

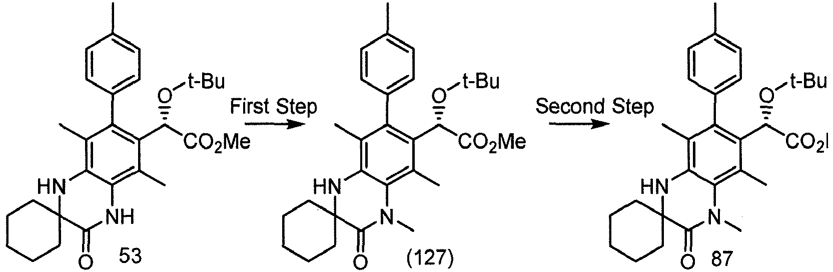

### First Step Synthesis of Compound (127)

Compound 53 (50 mg, 0.104 mmol) was dissolved in acetone (1 mL), and cesium carbonate (68 mg, 0.209 mmol) and methyl iodide (44 mg, 0.31 mmol) were added thereto, and the mixture was stirred at room temperature for 7 hours. Water (10 mL) was added thereto and the mixture was extracted with ethyl acetate (10 mL). The obtained organic layer was washed with water (10 mL) and brine (10 mL), dried over anhydrous magnesium sulfate, and concentrated to obtain crude compound (55 mg) as a colorless solid. The compound was used in the next reaction without purification.
MS: m/z = 493 [M+H]⁺

### Second Step Synthesis of Compound 87

Compound (127) (55 mg, 0.104 mmol) was dissolved in a solution of methanol (0.5 mL) and THF (1.5 mL), and 2mol/L aqueous solution of sodium hydroxide (0.52 mL, 1.04 mmol) were added thereto, and the mixture was stirred at 60°C for 10 hours. 2mol/L aqueous solution of hydrochloric acid (0.52 mL) and brine (15 mL) were added thereto and the mixture was extracted with ethyl acetate (25 mL). The obtained organic layer was dried over anhydrous magnesium sulfate, and concentrated. The residue was purified by HPLC to obtain Compound 87 (19 mg, 38%) as a colorless solid.
¹H-NMR (CDCl₃) δ: 0.97 (s, 9H), 1.10-1.70 (m, 10H),1.86 (s, 3H), 2.26 (s, 3H), 2.41 (s, 3H), 3.31 (s, 3H), 4.16(s, 1H), 5.13 (s, 1H), 7.00 (d, J = 8.0 Hz, 1H), 7.19-7.26 (m, 2H), 7.45-7.55 (m, 1H)
MS: m/z = 479[M+H]⁺

### Example 36

The following compounds were synthesized according to the above examples.

**[Table 1]**

| **No.** | **Structure** | **RT (min)** | | **MS [M+H]+** | |
|---|---|---|---|---|---|
| 1 | 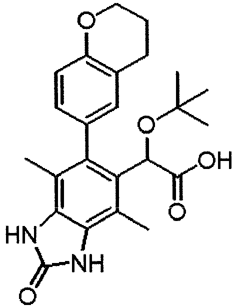 | 1.76 | | 423 | [M-H]- |
| 2 | 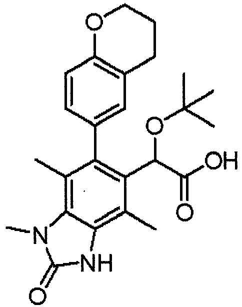 | 1.95 | | 438 | |
| 4 | 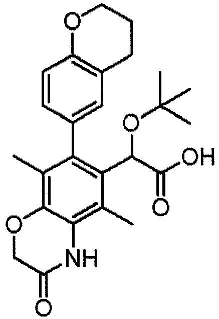 | 2.14 | | 440 | |
| 5 | 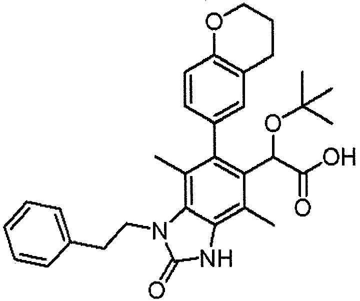 | 2.44 | | 529 | |

**[Table 2]**

| **No.** | **Structure** | **RT (min)** | | **MS [M+H]+** | |
|---|---|---|---|---|---|
| 6 | 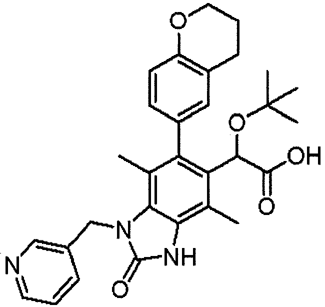 | 1.55 | | 516 | |
| 11 | 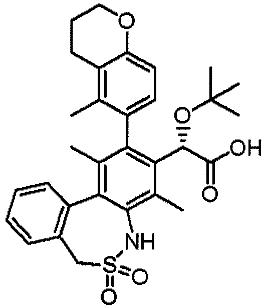 | 2.45 | | 548 | [M-H]- |
| 13 | 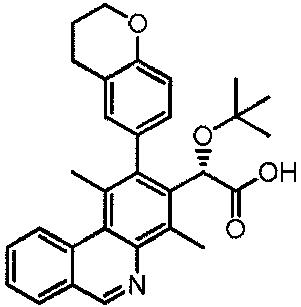 | 2.58/2.62 | | 470 | |
| 14 | 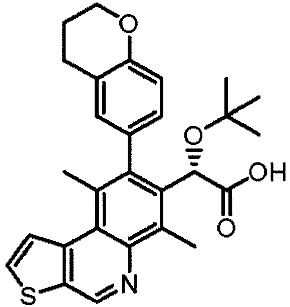 | 2.31/2.35 | | 476 | |

**[Table 3]**

| **No.** | **Structure** | **RT (min)** | | **MS [M+H]+** | |
|---|---|---|---|---|---|
| 15 | 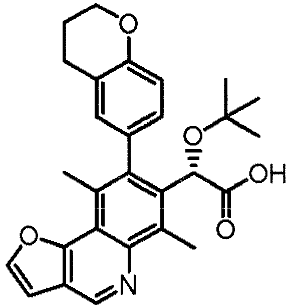 | 2.06/2.10 | | 460 | |
| 17 | 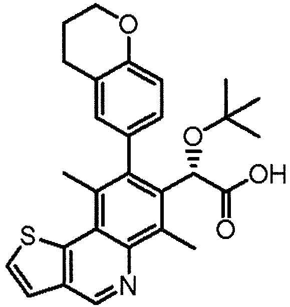 | 2.27/2.31 | | 476 | |
| 19 | 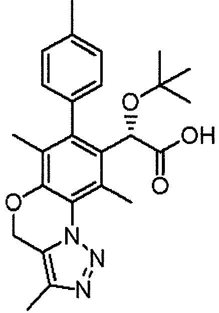 | 2.53 | | 436 | |
| 24 | 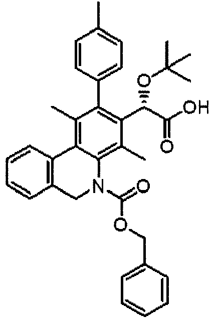 | 2.92 | * | 586 | [M+Na]+ |

**[Table 4]**

| **No.** | **Structure** | **RT (min)** | | **MS [M+H]+** | |
|---|---|---|---|---|---|
| 26 | 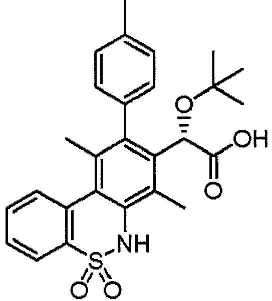 | 2.38 | * | 502 | [M+Na]+ |
| 27 | 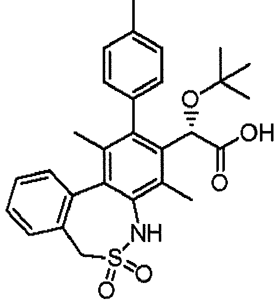 | 2.5 | | 492 | [M-H]- |
| 28 | 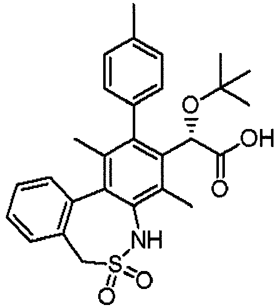 | 2.53 | | 492 | [M-H]- |
| 31 | 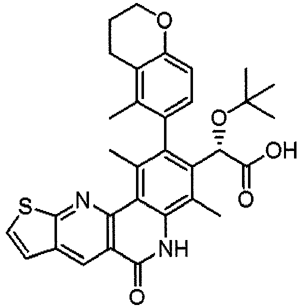 | 2.85 | | 557 | |

**[Table 5]**

| **No.** | **Structure** | **RT (min)** | | **MS [M+H]+** | |
|---|---|---|---|---|---|
| 32 | 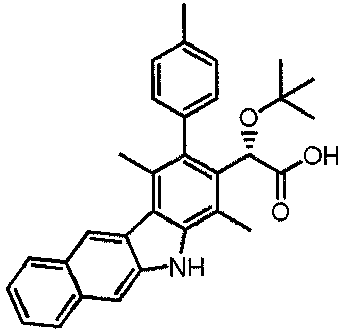 | 2.98 | * | 466 | |
| 36 | 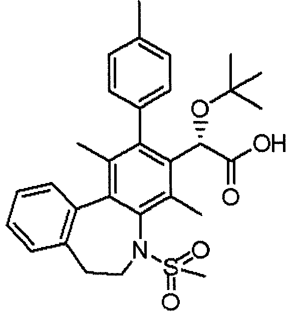 | 2.69 | * | 544 | [M+Na]+ |
| 37 | 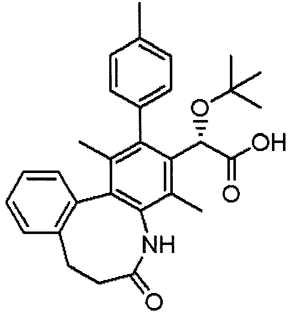 | 2.37/2.43 | * | 472 | |

**[Table 6]**

| **No.** | **Structure** | **RT (min)** | | **MS [M+H]+** | |
|---|---|---|---|---|---|
| 40 | 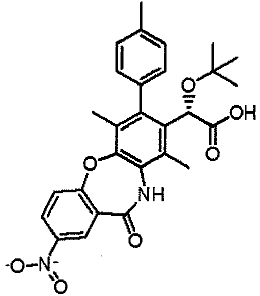 | 5.48 | * | 503 | [M-H]- |
| 41 | 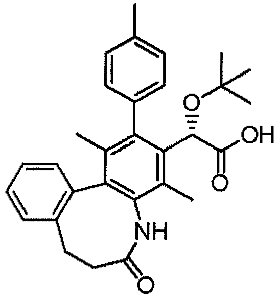 | 2.43 | | 472 | |
| 43 | 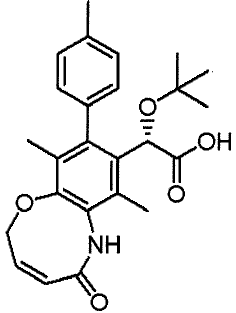 | 2.04 | * | 424 | |
| 44 | 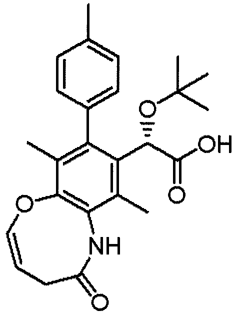 | 2.12 | * | 424 | |

**[Table 7]**

| **No.** | **Structure** | **RT (min)** | | MS **[M+H]+** | |
|---|---|---|---|---|---|
| 45 | 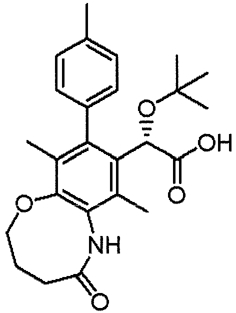 | 2.06 | * | 426 | |
| 48 | 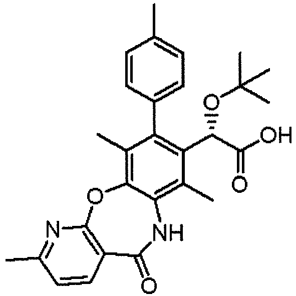 | 2.41 | | 475 | |
| 55 | 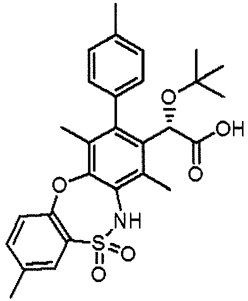 | 2.6 | | 508 | [M-H]- |
| 56 | 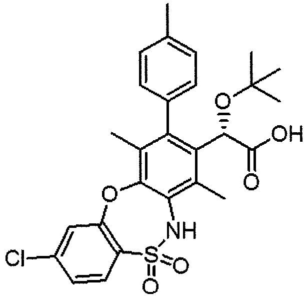 | 2.71 | | 528 | [M-H]- |

**[Table 8]**

| **No.** | **Structure** | **RT (min)** | | **MS [M+H]+** | |
|---|---|---|---|---|---|
| 57 | 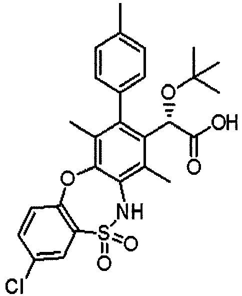 | 2.67 | | 528 | [M-H]- |
| 58 | 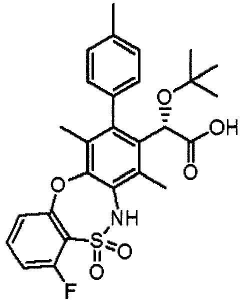 | 2.45 | | 512 | [M-H]- |
| 59 | 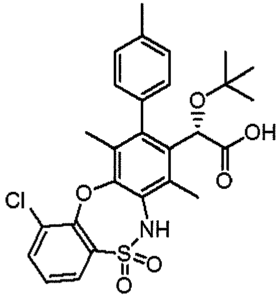 | 2.7 | | 528 | [M-H]- |
| 60 | 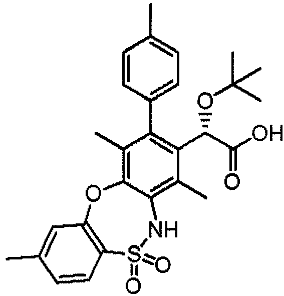 | 2.58 | | 508 | [M-H]- |

**[Table 9]**

| **No.** | **Structure** | **RT (min)** | | **MS [M+H]+** | |
|---|---|---|---|---|---|
| 62 | 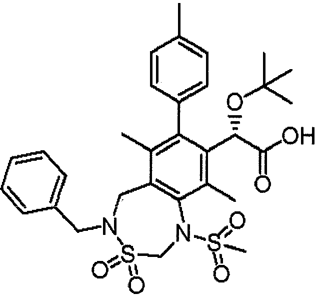 | 2.68 | | 613 | [M-H]- |
| 63 | 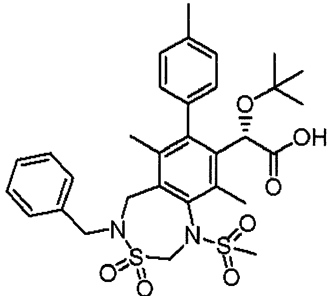 | 2.67 | | 613 | [M-H]- |
| 65 | 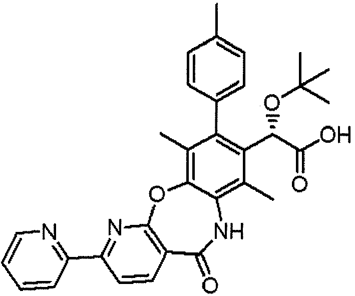 | 2.64 | | 538 | |
| 66 | 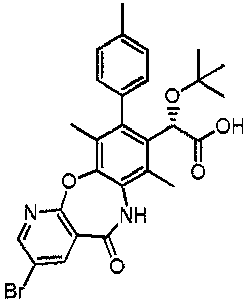 | 2.68 | | 537, 539 | |

**[Table 10]**

| **No.** | **Structure** | **RT (min)** | | **MS [M+H]+** | |
|---|---|---|---|---|---|
| 68 | 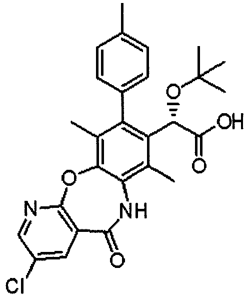 | 2.65 | | 494 | [M-H]- |
| 69 | 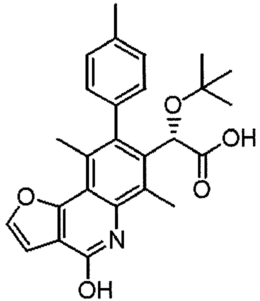 | 2.34 | * | 434 | |
| 72 | 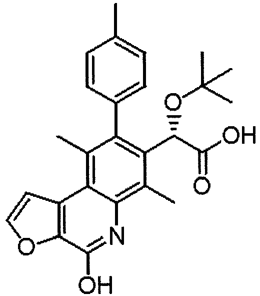 | 2.22 | * | 434 | |
| 74 | 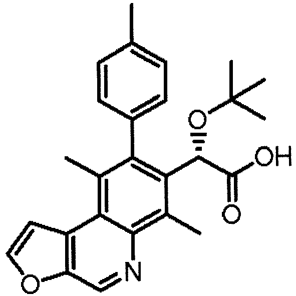 | 2.45 | * | 419 | |

**[Table 11]**

| **No.** | **Structure** | **RT (min)** | | **MS [M+H]+** | |
|---|---|---|---|---|---|
| 75 | 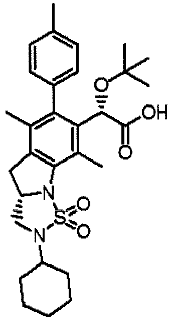 | 2.8 | * | 539 | [M-H]- |
| 76 | 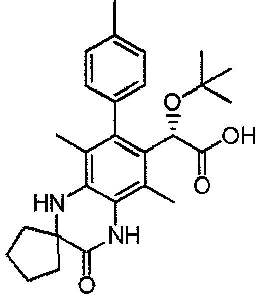 | 2.49 | | 451 | |
| 77 | 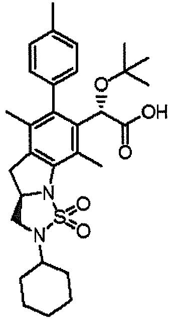 | 2.79 | * | 539 | [M-H]- |
| 82 | 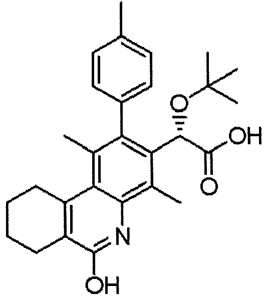 | 2.57 | * | 448 | |

**[Table 12]**

| **No.** | **Structure** | **RT (min)** | | **MS [M+H]+** | |
|---|---|---|---|---|---|
| 83 | 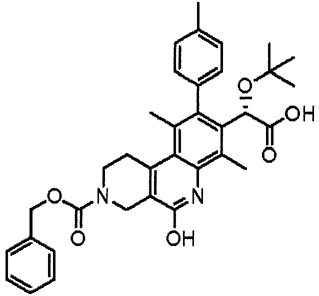 | 2.61 | * | 583 | |
| 84 | 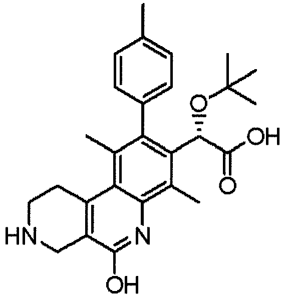 | 1.25 | * | 449 | |
| 85 | 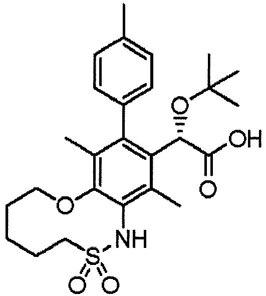 | 2.47 | | 488 | [M-H]- |
| 86 | 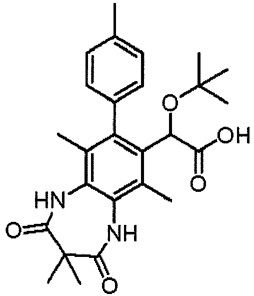 | 2.02 | * | 453 | |

### Example 37

The following compounds can be synthesized according to the above examples. 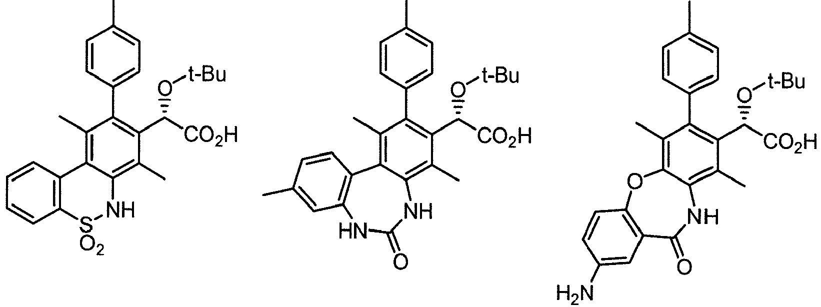 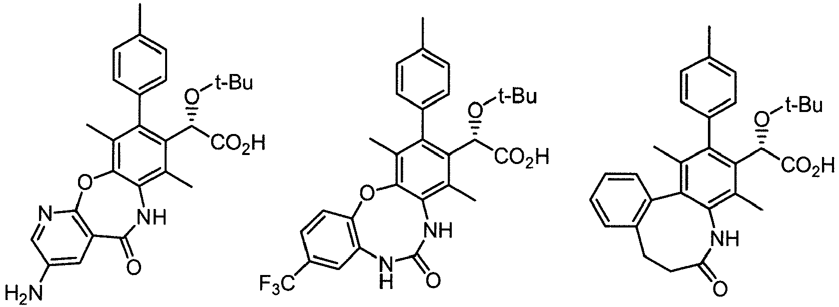 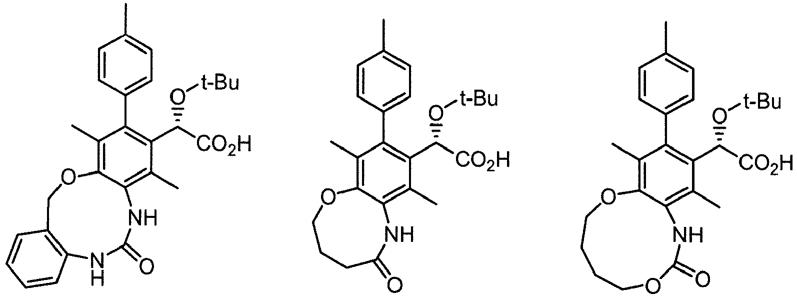 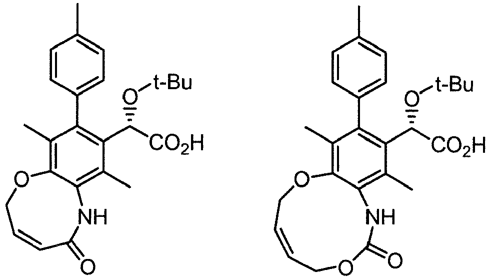 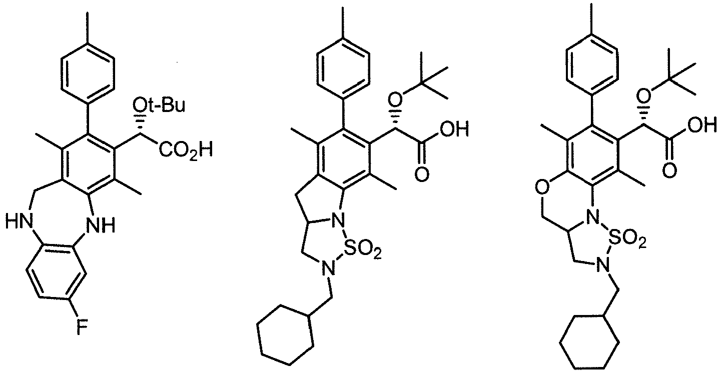 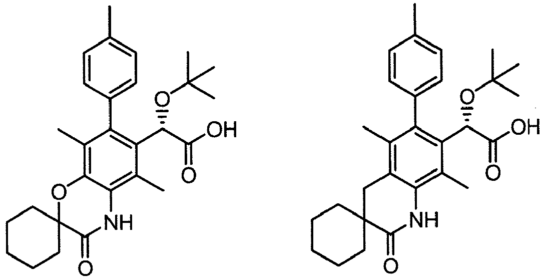 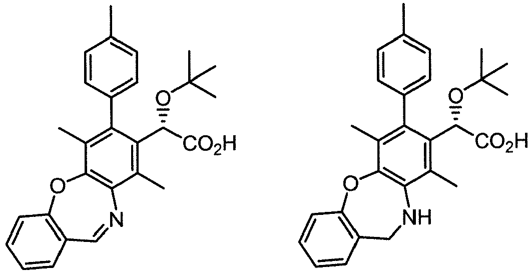 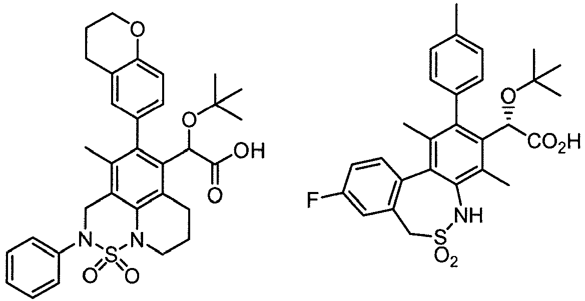 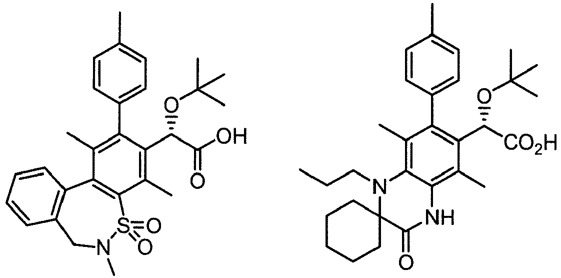 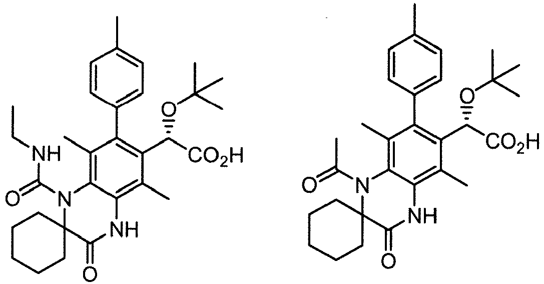 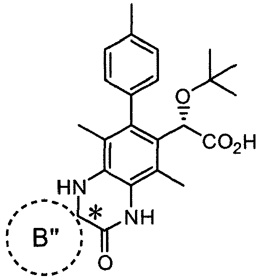 wherein B" is the followings; * shows a bond position 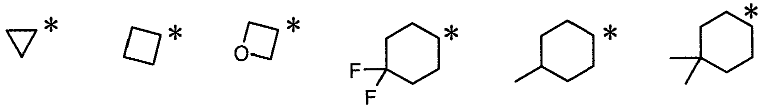 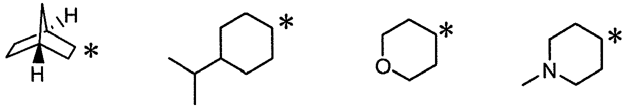 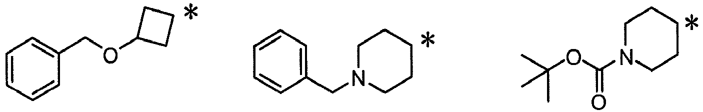 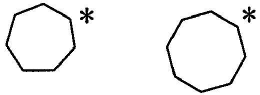 Example 38 Synthesis of Compound 92

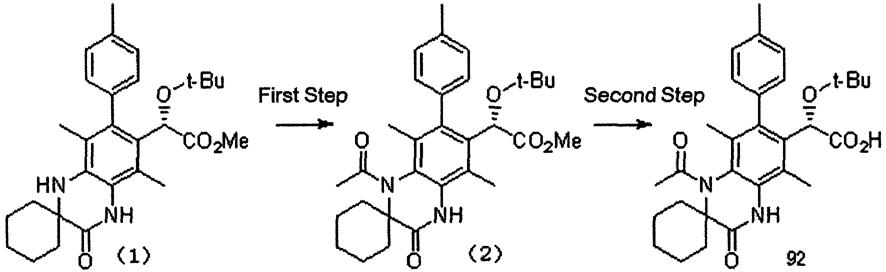

### First Step Synthesis of Compound (2)

Compound (1) (50 mg, 0.104 mmol) was dissolved in dichloromethane (1 mL), and triethylamine (43 µL, 0.313 mmol) and acetyl chloride (22 µL, 0.313 mmol) were added thereto, and the mixture was stirred at room temperature for 24 hours. Water (10 mL) was added thereto and the mixture was extracted with ethyl acetate (10 mL). The obtained organic layer was washed with water (10 mL) and brine (10 mL), dried over anhydrous magnesium sulfate, and concentrated to obtain crude Compound (2) (60 mg) as a colorless solid. The compound was used in the next reaction without purification.
MS: m/z = 521 [M+H]⁺

### Second Step Synthesis of Compound 92

Compound (2) (60mg) was dissolved in a solution of methanol (0.5 mL) and THF (1.5 mL), and 2mol/L aqueous solution of sodium hydroxide (0.52 mL, 1.04 mmol) was added thereto at room temperature, and the mixture was stirred at 60°C for 4 hours. 2mol/L aqueous solution of hydrochloric acid (0.52 mL) and brine (15 mL) were added thereto and the mixture was extracted with ethyl acetate (25 mL). The obtained organic layer was dried over anhydrous magnesium sulfate, and concentrated. The residue was purified by HPLC to obtain Compound 92 (8 mg, 15%) as a colorless solid.
¹H-NMR (CDCl₃) δ: 0.98 (s, 9H), 1.10-1.90 (m, 10H), 1.89 (s, 3H), 2.08 (s, 3H), 2.42 (s, 3H), 2.25(s,3H), 3.21 (s, 1H), 5.13 (s, 1H), 6.90-7.50 (m, 4H)
MS: m/z = 507[M+H]⁺

### Example 39 Synthesis of Compound 93

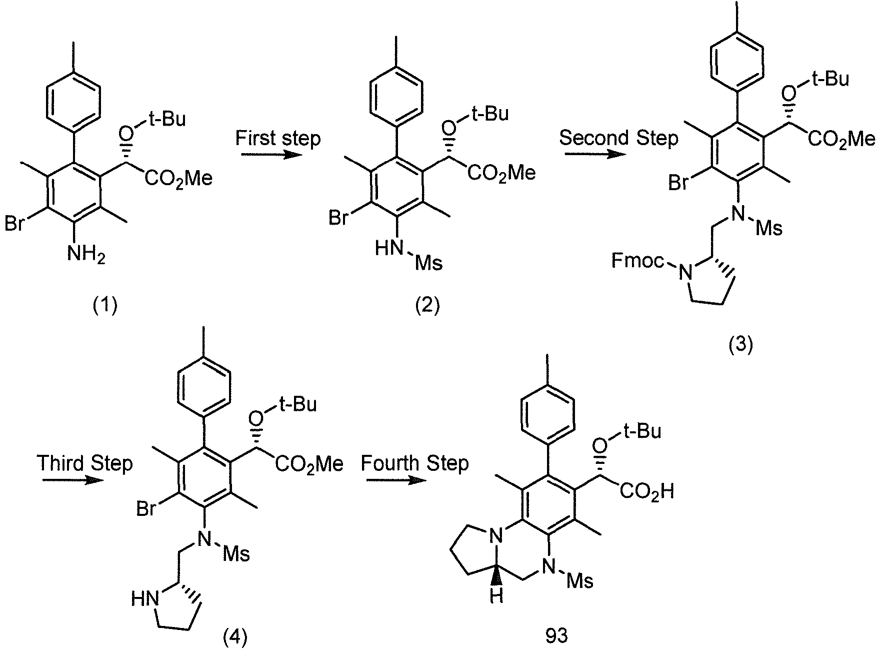

### First Step Synthesis of Compound (2)

Compound (1) (500 mg, 1.15 mmol) was dissolved in pyridine (10 mL), methanesulfonyl chloride (0.323 mL, 4.14 mmol) was added thereto at 100°C, and the mixture was stirred for 45 minutes. Water (50 mL) was added thereto and the mixture was extracted with ethyl acetate (150 mL). The obtained organic layer was washed with 1mol/L aqueous solution of hydrochloric acid (50 mL), water (50 mL) and brine (50 mL), dried over anhydrous magnesium sulfate, and concentrated. The residue was purified by silica gel column chromatography (hexane-ethyl acetate) to obtain Compound (2) (81.4 mg, 14%) as a brown foam.
¹H-NMR (CDCl₃) δ: 0.95 (s, 9H), 2.10 (s, 3H), 2.42 (s, 3H), 2.55 (s, 3H), 3.17 (s, 3H), 3.68 (s, 3H), 5.00 (s, 1H), 6.19 (s, 1H), 7.05 (d, J = 7.6 Hz, 1H), 7.15 (d, J = 7.8 Hz, 1H), 7.21-7.25 (m, 2H).
MS: m/z = 510.3/512.2 [M-H]-

### Second Step Synthesis of Compound (3)

Compound (2) (80.0 mg, 0.156 mmol), Fmoc-L-prolinol (151 mg, 0.468 mmol) and triphenylphosphine (123 mg, 0.468 mmol) were dissolved in THF (1.6 mL), and a solution of DEAD (diethyl azodicarboxylate) in toluene (2.2mol/L, 0.213mL, 0.468mmol) was added dropwise thereto, and the mixture was stirred at room temperature for 1 hour. Methanol (0.5 mL) was added thereto and the mixture was concentrated. The residue was purified by silica gel column chromatography (hexane-ethyl acetate) to obtain Compound (3) (76.3 mg, 60%) as a brown oil.
MS: m/z = 817.4/819.4 [M+H]+

### Third Step Synthesis of Compound (4)

Compound (3) (76.3 mg, 0.093 mmol) was dissolved in dichloromethane (1 mL). Diethylamine (1 mL) was added thereto at room temperature, and the mixture was stirred at same temperature for 2 hours. The mixture was concentrated. The residue was purified by silica gel column chromatography (chloroform-methanol) to obtain Compound (4) (43.9 mg, 79%) as a colorless foam.
MS: m/z = 595.4/597.4 [M+H]+

### Fourth Step Synthesis of Compound 93

Compound (4) (42.0 mg, 0.071 mmol) was dissolved in dioxane (1 mL). Tris(dibenzylideneacetone) palladium (12.9 mg, 0.014 mmol), 1,3-bis(2,6-diisopropylphenyl)-4,5-dihydro-1Himidazolium tetrafluoroborate (13.5 mg, 0.028 mmol) and tert-butoxypotassium (31.7 mg, 0.282 mmol) were added thereto at room temperature, and the mixture was stirred in a sealed tube at 110°C for 4 hours. 1mol/L aqueous solution of hydrochloric acid (3 mL) and brine (30 mL) were added thereto and the mixture was extracted with chloroform (30 mL×2). The obtained organic layer was dried over anhydrous magnesium sulfate and concentrated. The residue was purified by silica gel column chromatography (chloroform-methanol) and HPLC (0.1% formic acid-containing aqueous solution-acetonitrile) to obtain Compound 93 (2.6 mg, 7%) as a brown oil.
MS: m/z = 501.4 [M+H]+

### Example 40 Synthesis of Compound 170

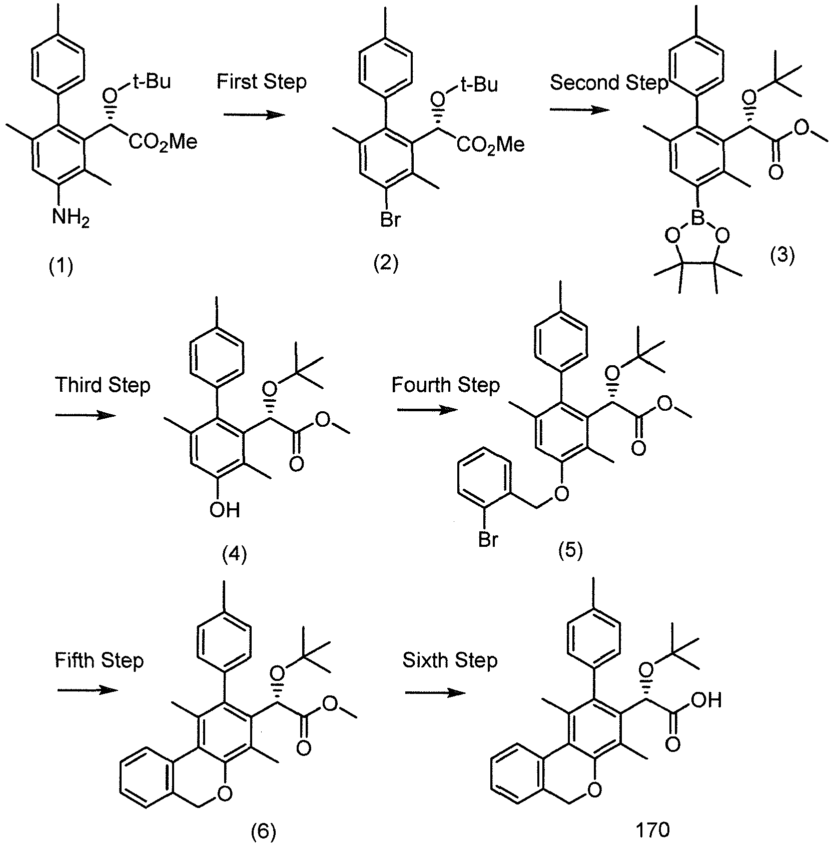

### First Step

Compound (1) (200 mg, 0.563 mmol) was dissolved in acetonitrile (4 mL). Tert-butyl nitrite (0.101 mL, 0.844 mmol) and copper chloride (I) (121 mg, 0.844 mmol) were added thereto under ice bath, and the mixture was warmed up to room temperature and stirred for 5.5 hours. Water (30 mL) was added thereto and the mixture was extracted with ethyl acetate (50 mL). The obtained organic layer was washed with brine (30 mL), dried over anhydrous magnesium sulfate, and concentrated. The residue was purified by silica gel column chromatography (hexane-ethyl acetate) to obtain Compound (2) (52.6 mg, 23%) as a colorless solid.
¹H-NMR (CDCl₃) δ: 0.95 (s, 9H), 1.93 (s, 3H), 2.41 (s, 3H), 2.45 (s, 3H), 3.68 (s, 3H), 5.03 (s, 1H), 7.06 (d, J = 7.4 Hz, 1H), 7.15-7.25 (m, 3H), 7.43 (s, 1H)

### Second Step

Compound (2) (69 mg, 0.165 mmol) was dissolved in 1,4-dioxane (1.4 mL). 4,4,4',4',5,5,5',5'-octamethyl-2,2'-bi(1,3,2-dioxaborolane) (54 mg, 0.214 mmol), potassium acetate (48 mg, 0.494 mmol) and Pd(dppf)Cl₂ dichloromethane complex (6.7 mg, 0.0082 mmol) were added thereto at room temperature, and the mixture was stirred under heat reflux for 2 hours. Water (2 mL) was added thereto and the mixture was extracted with chloroform (2 mLx3). The obtained organic layer was dried over anhydrous magnesium sulfate and concentrated. The residue was purified by silica gel column chromatography (hexane-ethyl acetate) to obtain Compound (3) (89 mg) as a colorless solid.

### Third Step

Compound (3) (89 mg) was dissolved in THF (1.5 mL). 30% hydrogen peroxide solution (0.020 mL, 0.198 mmol) and 2N aqueous solution of sodium hydroxide (0.099 mL, 0.198 mmol) were added thereto under ice bath, and the mixture was stirred for 45 minutes. A saturated aqueous solution of sodium thiosulfate (5 mL) was added thereto and the mixture was extracted with chloroform (2 mLx4). The obtained organic layer was dried over anhydrous magnesium sulfate and concentrated. The residue was purified by silica gel column chromatography (hexane-ethyl acetate) to obtain Compound (4) (55 mg, 0.154 mmol) as a colorless solid.

### Fourth Step

Compound (4) (54 mg, 0.151 mmol) was dissolved in DMF (0.8 mL). Cesium carbonate (74 mg, 0.227 mmol) and 1-bromo2-(bromomethyl)benzene (0.099 mL, 0.198 mmol) was added thereto at room temperature, and the mixture was warmed up to 40°C and stirred for 90 minutes. A saturated aqueous solution of ammonium chloride (5 mL) was added thereto and the mixture was extracted with ethyl acetate (5 mLx2). The obtained organic layer was washed with water (5 mL), dried over anhydrous magnesium sulfate, and concentrated. The residue was purified by silica gel column chromatography (hexane-ethyl acetate) to obtain crude Compound (5) (87 mg) as a colorless solid.

### Fifth and Sixth Step

Compound 170 was obtained in the same manner as in the second step and third step in Example 3.

### Compound 170

¹H-NMR (CDCl₃) δ: 0.99 (9H, s), 2.26 (3H, s), 2.28 (3H, s), 2.42 (3H, s), 4.90 (1H, J = 12.3 Hz, d), 5.05 (1H, J = 12.3 Hz, d), 5.13 (1H, s), 7.20-7.48 (7H, m), 7.69 (1H, J = 8.0 Hz, d).

### Example 41

The following compounds were synthesized according to the above examples.

**[Table 13]**

| **No.** | **Structure** | **RT (min)** | | **MS [M+H]+** | |
|---|---|---|---|---|---|
| 88 | 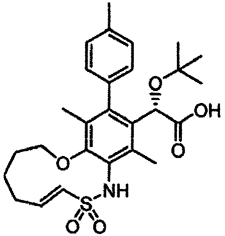 | 2.62 | | 502.4 | [M-H]- |
| 89 | 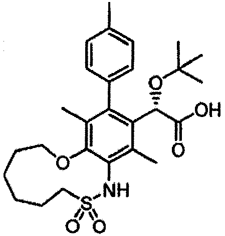 | 2.56 | | 500.4 | [M-H]- |
| 90 | 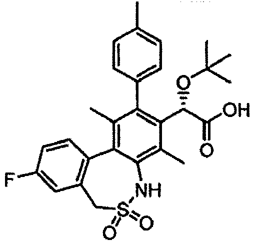 | 2.54 | | 510.4 | [M-H]- |
| 91 | 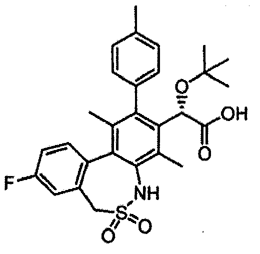 | 2.59 | | 510.4 | [M-H]- |

**[Table 14]**

| **No.** | **Structure** | **RT (min)** | | **MS [M+H]+** | |
|---|---|---|---|---|---|
| 94 | 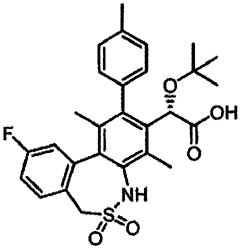 | 2.54 | | 510.1 | [M-H]- |
| 95 | 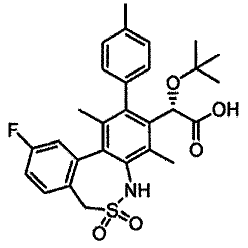 | 2.55 | | 510.1 | [M-H]- |
| 96 | 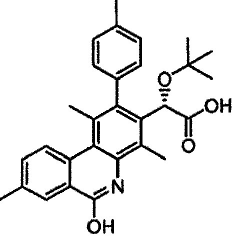 | 2.65 | * | 458.3 | |
| 97 | 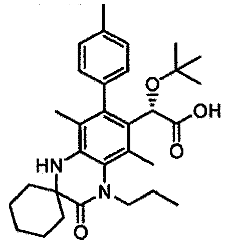 | 2.96 | | 507 | |

**[Table 15]**

| **No.** | **Structure** | **RT (min)** | | **MS [M+H]+** | |
|---|---|---|---|---|---|
| 98 | 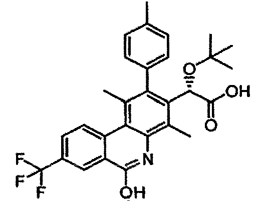 | 2.78 | * | 512.2 | |
| 99 | 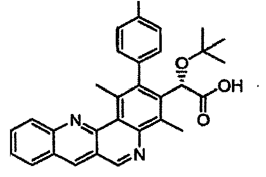 | 3.1 | * | 479.3 | |
| 100 | 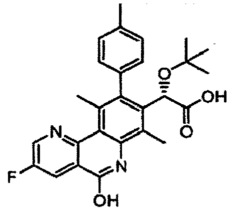 | 2.65 | * | 463.2 | |
| 101 | 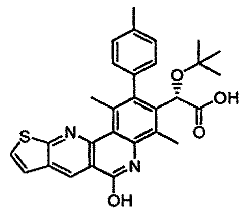 | 2.81 | * | 501.2 | |
| 102 | 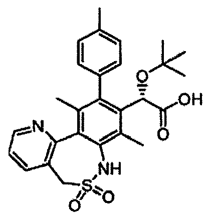 | | | 495.4 | |

**[Table 16]**

| **No.** | **Structure** | **RT (min)** | | **MS [M+H]+** | |
|---|---|---|---|---|---|
| 103 | 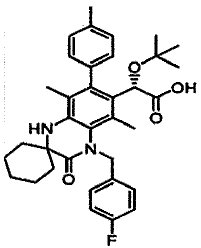 | 3.07 | | 573 | |
| 104 | 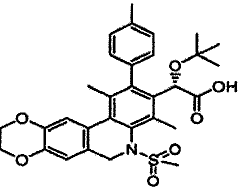 | 2.51 | * | 564 | [M-H]- |
| 105 | 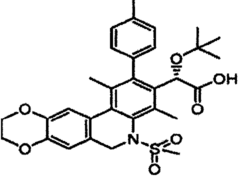 | 2.45 | | 590 | [M+Na]+ |
| 106 | 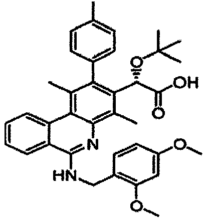 | 2.62 | | 593.4 | |
| 107 | 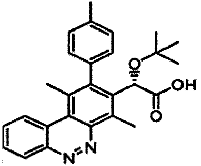 | 2.7 | | 430.5 | |

**[Table 17]**

| **No.** | **Structure** | **RT (min)** | | **MS [M+H]+** | |
|---|---|---|---|---|---|
| 108 | 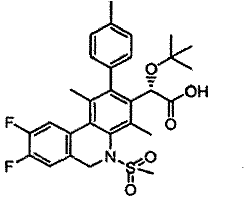 | 2.63 | * | 566 | [M+Na]+ |
| 109 | 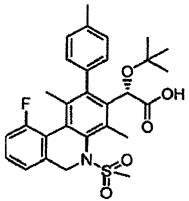 | 2.62 | * | 548 | [M+Na]+ |
| 110 | 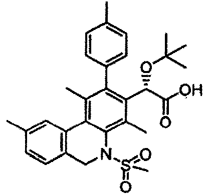 | 2.74 | * | | |
| 111 | 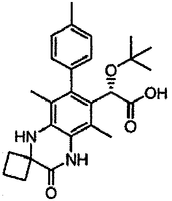 | 2.84 | | 437 | |
| 112 | 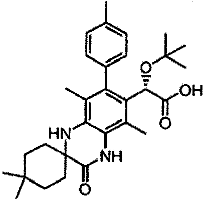 | 2.91 | | 493 | |

**[Table 18]**

| **No.** | **Structure** | **RT (min)** | | **MS [M+H]+** | |
|---|---|---|---|---|---|
| 113 | 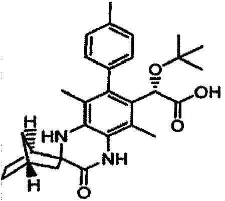 | 2.55 | | 477 | |
| 114 | 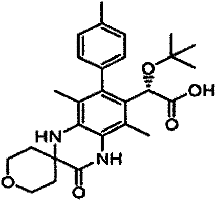 | 2.16 | | 467 | |
| 115 | 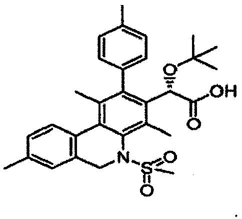 | 2.75 | * | 544 | [M+Na]+ |
| 116 | 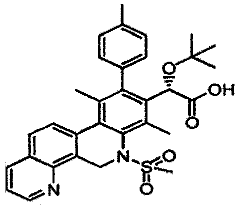 | 2.55 | *2 | 559 | |
| 117 | 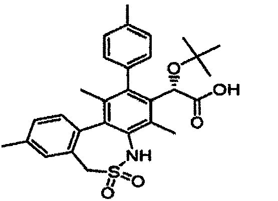 | 5.8 | *2 | 506 | [M-H]- |

**[Table 19]**

| **No.** | **Structure** | **RT (min)** | | MS [M+H]+ | |
|---|---|---|---|---|---|
| 118 | 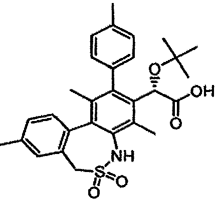 | 5.86 | *2 | 506 | [M-H]- |
| 119 | 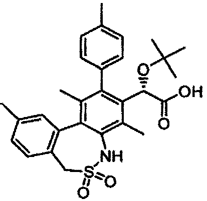 | 5.79 | *2 | 506 | [M-H]- |
| 120 | 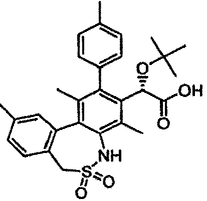 | 5.81 | *2 | 506 | [M-H]- |
| 121 | 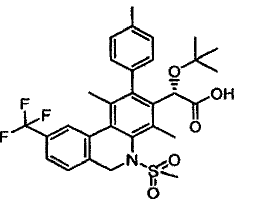 | 2.76 | * | 598 | [M+Na]+ |
| 122 | 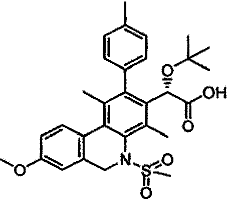 | 2.6 | * | 560 | [M+Na]+ |

**[Table 20]**

| **No.** | **Structure** | **RT (min)** | | **MS [M+H]+** | |
|---|---|---|---|---|---|
| 123 | 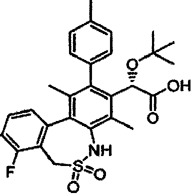 | 2.53 | | 510.3 | [M-H]- |
| 124 | 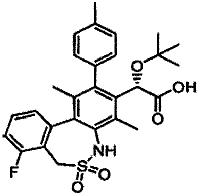 | 2.61 | | 510.4 | [M-H]- |
| 125 | 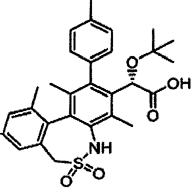 | 2.51 | * | 506.4 | [M-H]- |
| 127 | 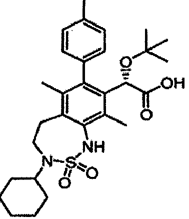 | 2.68 | * | 527.7 | [M-H]- |
| 128 | 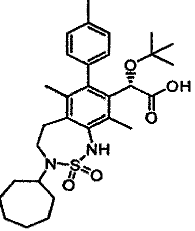 | 2.78 | * | 541.8 | [M-H]- |

**[Table 21]**

| **No.** | **Structure** | **RT (min)** | | **MS [M+H]+** | |
|---|---|---|---|---|---|
| 129 | 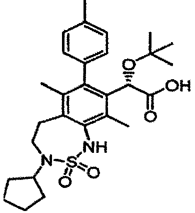 | 2.59 | * | 513.5 | [M-H]- |
| 130 | 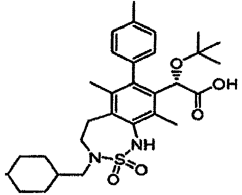 | 2.86 | * | 541.6 | [M-H]- |
| 131 | 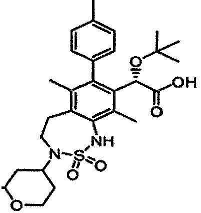 | 2.21 | * | 529.3 | [M-H]- |
| 132 | 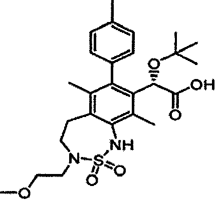 | 2.26 | * | 503.5 | [M-H]- |
| 133 | 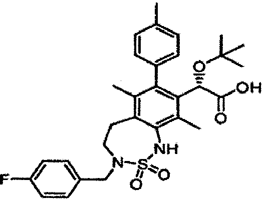 | 2.59 | * | 553.1 | [M-H]- |

**[Table 22]**

| **No.** | **Structure** | **RT (min)** | | **MS [M+H]+** | |
|---|---|---|---|---|---|
| 134 | 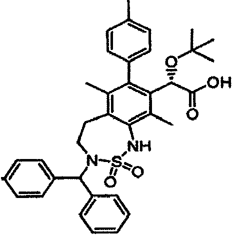 | 2.84 | * | 611.3 | [M-H]- |
| 135 | 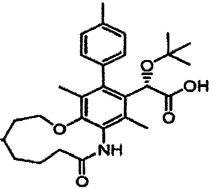 | 2.35 | | 468.3 | |
| 136 | 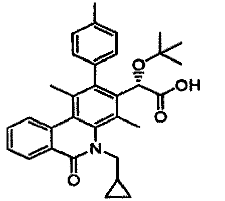 | 2.88 | | 498.3 | |
| 139 | 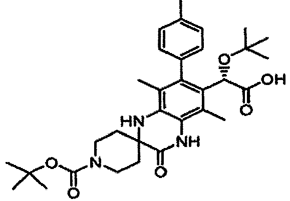 | 2.65 | | 566.3 | |
| 140 | 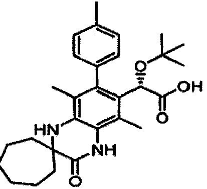 | 2.75 | | 479.3 | |

**[Table 23]**

| **No.** | **Structure** | **RT (min)** | | **MS [M+H]+** | |
|---|---|---|---|---|---|
| 141 | 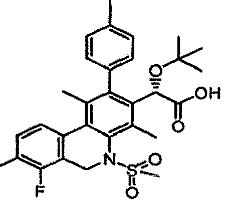 | 2.74 | * | 562 | [M+Na]+ |
| 142 | 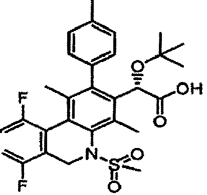 | 2.6 | * | 566 | [M+Na]+ |
| 143 | 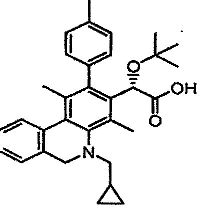 | 3.17 | | 484.5 | |
| 144 | 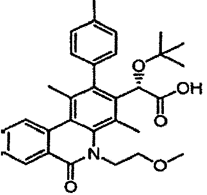 | 2.63 | | 502.3 | |
| 145 | 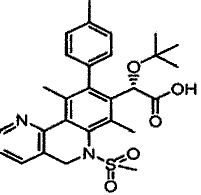 | 2.4 | | 510 | |

**[Table 24]**

| **No.** | **Structure** | **RT (min)** | | **MS [M+H]+** | |
|---|---|---|---|---|---|
| 146 | 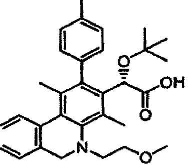 | 2.98 | | 488.1 | |
| 147 | 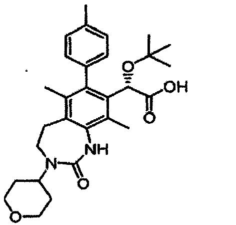 | 2.26 | * | 493.3 | [M-H]- |
| 148 | 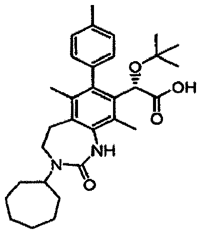 | 2.9 | * | 505.3 | [M-H]- |
| 149 | 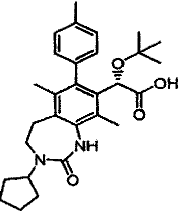 | 2.66 | * | 477.3 | [M-H]- |
| 150 | 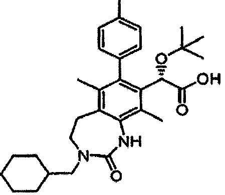 | 2.88 | * | 505.3 | [M-H]- |

**[Table 25]**

| **No.** | **Structure** | **RT (min)** | | **MS [M+H]+** | |
|---|---|---|---|---|---|
| 151 | 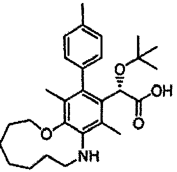 | 2.79 | | 454.2 | |
| 152 | 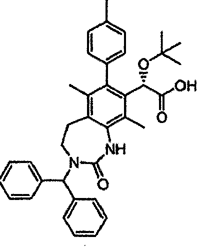 | 2.93 | * | 565.3 | [M-H]- |
| 153 | 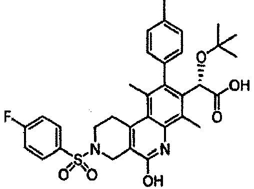 | 2.54 | | 607.3 | |
| 154 | 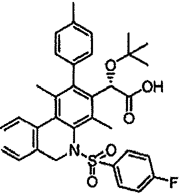 | 2.98 | | 586 | [M-H]+ |
| 155 | 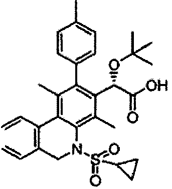 | 2.8 | | 532 | [M-H]+ |

**[Table 26]**

| **No.** | **Structure** | **RT (min)** | | **MS [M+H]+** | |
|---|---|---|---|---|---|
| 156 | 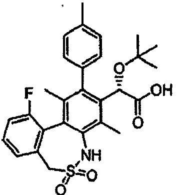 | 2.51 | | 510 | [M-H]+ |
| 157 | 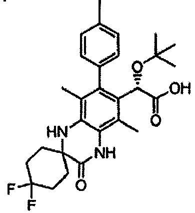 | 2.54 | | 501 | |
| 158 | 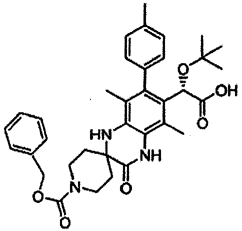 | 2.66 | | 600 | |
| 159 | 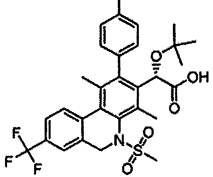 | 2.75 | * | 598 | [M+Na]+ |
| 160 | 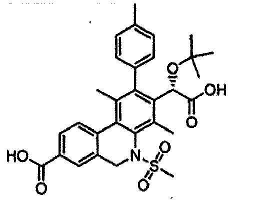 | 2.19 | * | 506 | {M-COOH]- |

**[Table 27]**

| **No.** | **Structure** | **RT (min)** | | **MS [M+H]+** | |
|---|---|---|---|---|---|
| 161 | 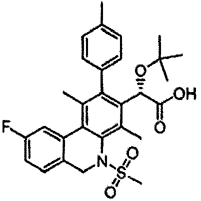 | 2.61 | * | 548 | [M+Na]+ |
| 162 | 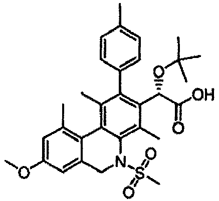 | 2.66 | * | 574 | [M+Na]+ |
| 163 | 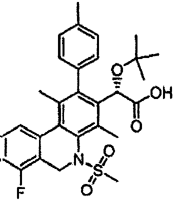 | 2.6 | * | 548 | [M+Na]+ |
| 164 | 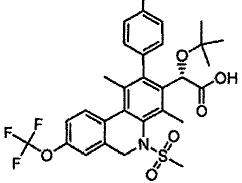 | 2.79 | * | 614 | [M+Na]+ |
| 165 | 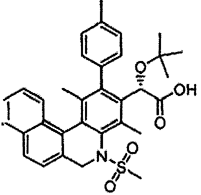 | 2.74 | * | 580 | [M+Na]+ |

**[Table 28]**

| **No.** | **Structure** | **RT (min)** | | **MS [M+H]+** | |
|---|---|---|---|---|---|
| 166 | 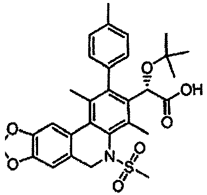 | 2.51 | * | 574 | [M+Na]+ |
| 167 | 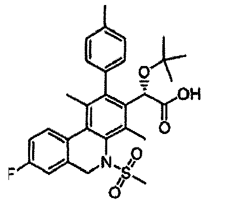 | 2.61 | * | 548 | [M+Na]+ |
| 168 | 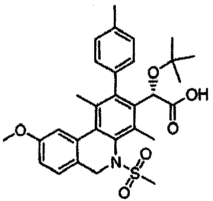 | 2.57 | * | 560 | [M+Na]+ |
| 169 | 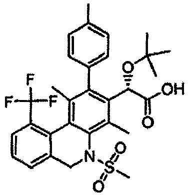 | 2.75 | * | 598 | [M+Na]+ |
| 170 | 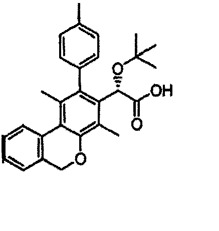 | 2.88 | * | 453.1 | [M+Na]+ |

**[Table 29]**

| **No.** | **Structure** | **RT (min)** | | **MS [M+H]+** | |
|---|---|---|---|---|---|
| 171 | 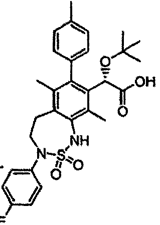 | 2.53 | * | 539.2 | [M-H]- |
| 172 | 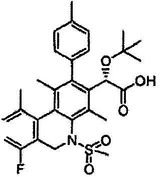 | 2.67 | * | 562 | [M+Na]+ |
| 173 | 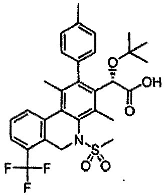 | 2.79 | * | 574 | [M-H]+ |
| 174 | 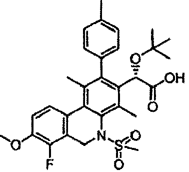 | 2.56 | * | 578 | [M+Na]+ |
| 175 | 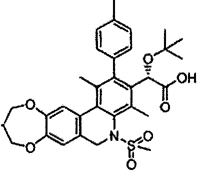 | 2.59 | * | 602 | [M+Na]+ |

**[Table 30]**

| **No.** | **Structure** | **RT (min)** | | **MS [M+H]+** | |
|---|---|---|---|---|---|
| 176 | 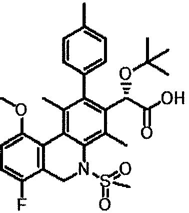 | 2.57 | * | 578 | [M+Na]+ |
| 177 | 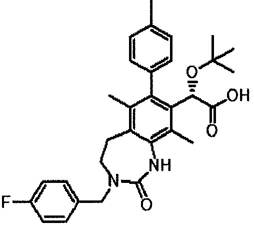 | 2.63 | * | 517.4 | [M-H]- |
| 178 | 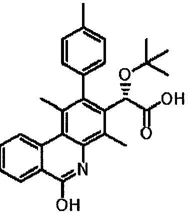 | 2.54 | | 444 | |
| 179 | 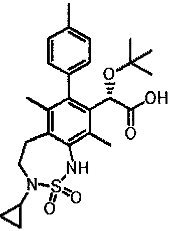 | 2.42 | * | 485.2 | [M-H]- |
| 180 | 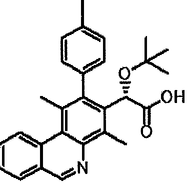 | 2.71 | | 428 | |

**[Table 31]**

| **No.** | **Structure** | **RT (min)** | | **MS [M+H]+** | |
|---|---|---|---|---|---|
| 181 | 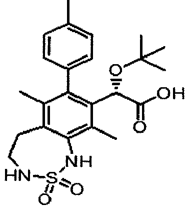 | 2.08 | * | 445.2 | [M-H]- |
| 182 | 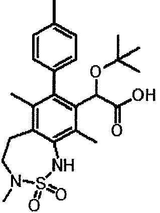 | 2.25 | * | 459.2 | [M-H]- |
| 183 | 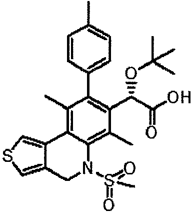 | 2.55 | * | 536 | [M+Na]+ |
| 184 | 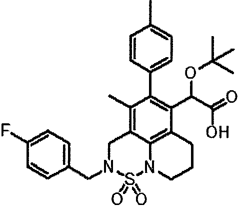 | 2.89 | | 567.3 | |
| 185 | 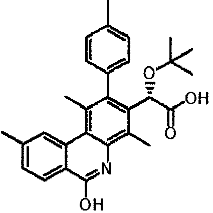 | 2.21 | | 458 | |

**[Table 32]**

| **No.** | **Structure** | **RT (min)** | | **MS [M+H]+** | |
|---|---|---|---|---|---|
| 186 | 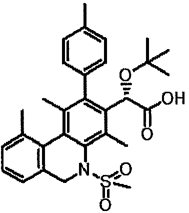 | 2.68 | | 544 | [M+Na]+ |
| 187 | 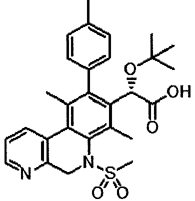 | 2.25 | | 509 | |
| 188 | 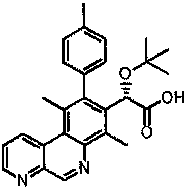 | 2.48 | | 429 | |
| 189 | 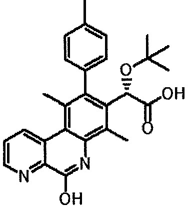 | 0.88 | | 445 | |
| 190 | 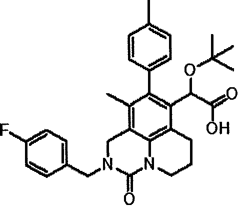 | 1.99 | | 531.3 | |

**[Table 33]**

| **No.** | **Structure** | **RT (min)** | | **MS [M+H]+** | |
|---|---|---|---|---|---|
| 191 | 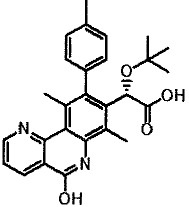 | 1.57 | | 445 | |
| 192 | 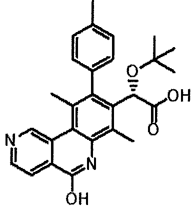 | 1.36 | | 445 | |
| 193 | 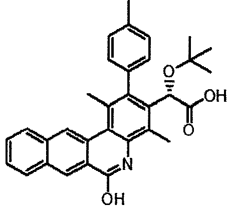 | 2.95 | | 494.5 | |
| 194 | 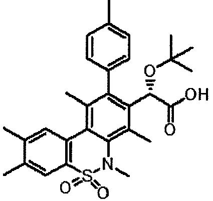 | 2.84 | * | 544 | [M+Na]+ |
| 195 | 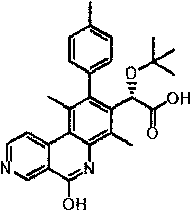 | 2.06 | | 445 | |

**[Table 34]**

| **No.** | **Structure** | **RT (min)** | | **MS [M+H]+** | |
|---|---|---|---|---|---|
| 196 | 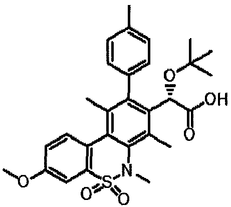 | 2.64 | * | 546 | [M+Na]+ |
| 197 | 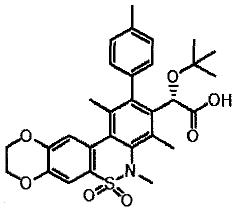 | 2.53 | * | 478 | [M-OBu]+ |
| 198 | 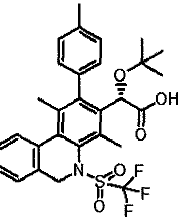 | 2.94 | * | 625 | [M+MeCN+Na]+ |

### Example 42 Synthesis of Compound 213

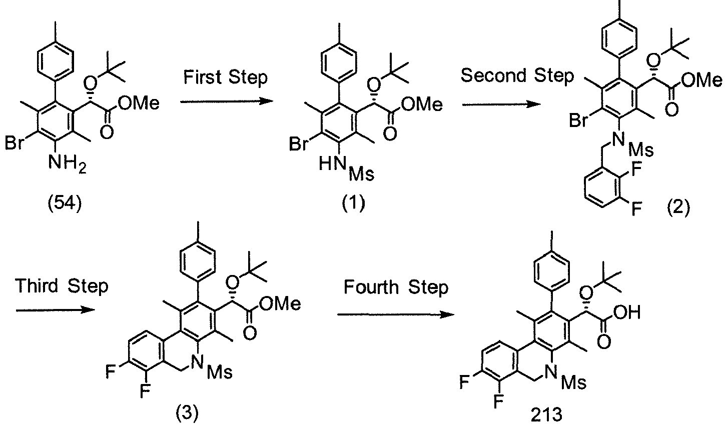

### First Step

Compound (54) (500 mg, 1.15 mmol) was dissolved in pyridine (3 mL), and the mixture was cooled under ice bath. Methanesulfonyl chloride (0.269 mL, 3.45 mmol) was added thereto, and the mixture was warmed up to room temperature and stirred for 18 hours. The mixture was poured into a solution of ice water and 2mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated. The residue was purified by silica gel column chromatography (hexane-ethyl acetate) to obtain Compound (1) (419 mg, 71%) as a colorless foam.
MS(ESI) m/z:534, 536 [M + Na]+

### Second Step

Compound (1) (255 mg, 0.498 mmol) was dissolved in DMF (2.6 mL), and cesium carbonate (324 mg, 0.995 mmol) and 1-bromomethyl-2,3-difluorobenzene (113 mg, 0.547 mmol) were added thereto, and the mixture was stirred at room temperature for 18 hours. The mixture was poured into a solution of ice water and 2 mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (2) (306 mg, 96%) as colorless foam.
¹H NMR (CDCl₃) δ:0.87 (s, 4.5H), 0.90 (s, 4.5H), 2.05 (s, 3H), 2.08 (s, 1.5H), 2.09 (s, 1.5H), 2.42 (s, 3H), 3.28 (s, 1.5 H), 3.30 (s, 1.5H), 3.62 (s, 1.5H), 3.63 (s, 1.5H), 4.79-4.96 (m, 3H), 6.96-7.22 (m, 7H).

### Third Step

Compound (2) (298 mg, 0.467 mmol) was dissolved in DMA (5 mL), and tricyclohexylphosphonium tetrafluoroborate (34.4 mg, 0.093 mmol), potassium carbonate (129 mg, 0.933 mmol) and Pd(OAc)₂ (10.5 mg, 0.0470 mmol) were added thereto, and the operation of degassing and nitrogen substitution was repeated three times. The mixture was stirred under a nitrogen atmosphere at 130°C for 3 hours. The mixture was cooled to room temperature, poured into a solution of ice water and 2 mol/L aqueous solution of hydrochloric acid and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound(3) (237 mg, 91%) as a white foam.
MS(ESI) m/z:580 [M + Na]+

### Fourth Step

Compound (3) (237 mg, 0.425 mmol) was dissolved in a solution of methanol (4 mL) and THF (2 mL), and 2 mol/L aqueous solution of sodium hydroxide (2.13 mL, 4.26 mmol) was added thereto, and the mixture was stirred at 90°C for 4 hours. The mixture was cooled to room temperature, poured into ice water, and obtained aqueous layer was adjusted with 2mol/L aqueous solution of hydrochloric acid to about pH=3 and extracted with ethyl acetate. The obtained organic layer was washed with brine, dried over anhydrous sodium sulfate, and concentrated under reduced pressure. The residue was recrystallized from diisopropyl ether and hexane, and dried at 60°C under reduced pressure to obtain Compound 213 (160 mg, 69%) as a colorless powder.
MS(ESI) m/z:566 [M+Na]+

### Example 43 Synthesis of Compound 224

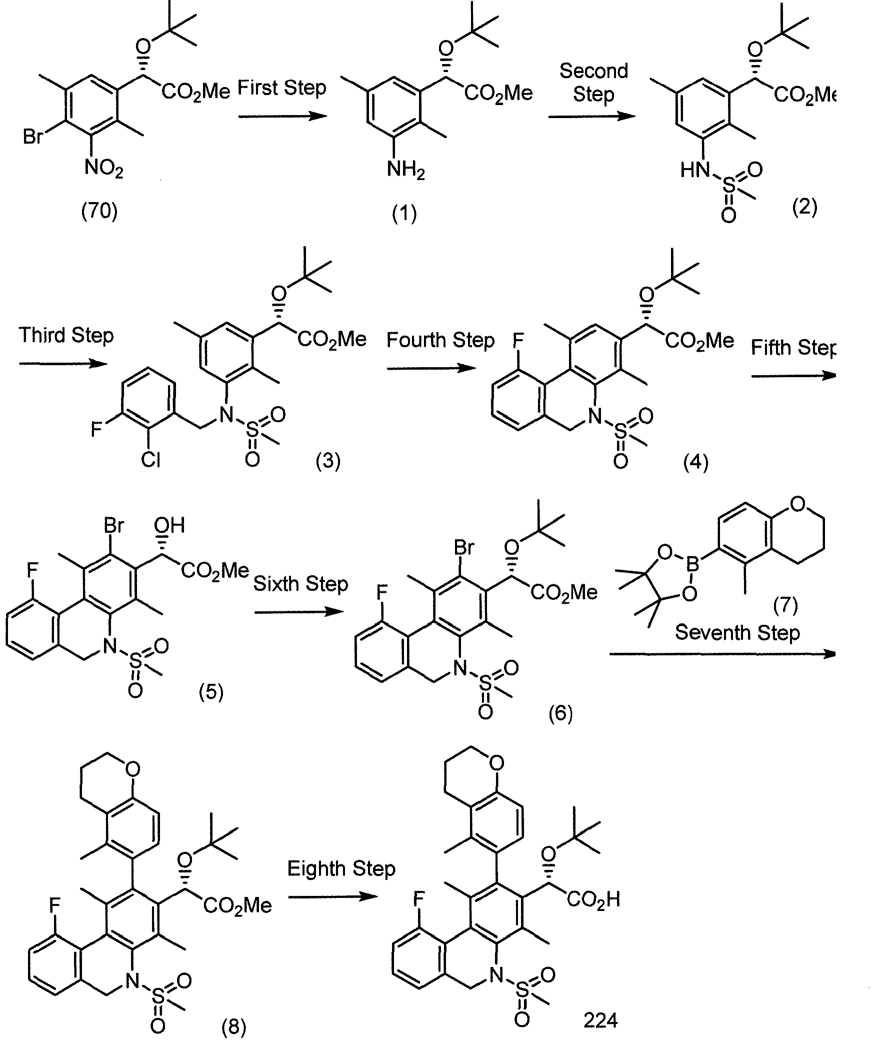

### First Step

Compound (70) (20.0 g, 53.4 mmol) was dissolved in a solution of ethanol (150 mL) and THF (50.0 mL), and triethylamine (7.41 mL, 53.4 mmol) and palladium hydroxide (7.51g, 5.34mmol, 10%Wt) were added thereto, and the operation of degassing and hydrogen substitution was repeated three times. The mixture was stirred under a hydrogen atmosphere at medium pressure, at room temperature for 20 hours 45 minutes. The mixture was filtered through celite, and the filtrate was concentrated under reduced pressure. The residue was poured into ice water and the mixture was extracted with ethyl acetate. The obtained organic layer was washed with brine, dried over anhydrous sodium sulfate, concentrated under reduced pressure, and dried at room temperature under reduced pressure to obtain crude Compound (1) (14.72 g) pale green solid.
¹H NMR (CDCl₃) δ: 1.23 (s, 9H), 2.17 (s, 3H), 2.23 (s, 3H), 3.55 (br s, 2H), 3.66 (s, 3H), 5.20 (s, 1H), 6.47 (s, 1H), 6.81 (s, 1H).

### Second Step

The crude Compound (1) (14.72 g) was dissolved in pyridine (70.8 mL), and the mixture was cooled under ice water. Methanesulfonyl chloride (4.99 mL, 64.1mmol) was added thereto, and the mixture was warmed up to room temperature and stirred for 1 hour 35 minutes. The mixture was poured into a solution of ice water and 2mol/L aqueous solution of hydrochloric acid and the mixture was extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (2) (17.78 g, 96.9% in 2 steps) as a pale yellow solid.
¹H NMR (CDCl₃) δ:1.23 (s, 9H), 2.33 (s, 3H), 2.34 (s, 3H), 3.01 (s, 3H), 3.67 (s, 3H), 5.21 (s, 1H),6.13 (br s, 1H), 7.21 (s, 1H), 7.29 (s, 1H).

### Third Step

Compound (2) (500 mg, 1.46 mmol) was dissolved in DMF (5.00 mL), and cesium carbonate (949 mg, 2.91 mmol) and 1-bromomethyl-2-chloro-3-fluorobenzene (411 mg, 1.75 mmol) were added thereto, and the mixture was stirred at room temperature for 2 hours 25 minutes. The mixture was poured into a solution of ice water and 2mol/L aqueous solution of hydrochloric acid and the mixture was extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound(3) (680 mg, 96.1%) as a white foam.
MS(ESI) m/z:530.2 [M + HCOO]-

### Fourth Step

Compound (3) (200 mg, 0.412 mmol) was dissolved in DMA (3.00 mL), and tricyclohexylphosphonium tetrafluoroborate (30.3 mg, 0.0820 mmol), potassium carbonate (114 mg, 0.823mmol) and Pd(OAc)₂ (9.24 mg, 0.410 mmol) were added thereto, and the operation of degassing and nitrogen substitution was repeated three times. The mixture was stirred under a nitrogen atmosphere at 130°C for 56 minutes. The mixture was cooled to room temperature, poured into a solution of ice water and 2mol/L aqueous solution of hydrochloric acid, and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound(4) (165mg, 89.2%) as a white foam.
MS(ESI) m/z:467.3 [M + NH₄]+

### Fifth Step

Compound (4) (535 mg, 1.19 mmol) and 1,3-dibromo-5,5-dimethylhydantoin (289 mg, 1.01 mmol) were suspended in acetic acid (10.7 mL), and the mixture was cooled under ice bath. Conc sulfuric acid (1.07 mL, 19.3mmol) was added thereto, and the mixture was warmed up to room temperature and stirred for 1 hour 37 minutes. The mixture was poured into a solution of ice water and a saturated aqueous solution of sodium hydrogen carbonate and extracted with ethyl acetate. The obtained organic layer was washed with brine, dried over anhydrous sodium sulfate, and concentrated under reduced pressure. The residue was purified by silica gel column chromatography (hexane-ethyl acetate) to obtain Compound (5) (356 mg, 63.3%) as a white foam.
MS(ESI) m/z:516.2 [M + HCOO]-

### Sixth Step

Compound (5) (436 mg, 0.923 mmol) was dissolved in tert-butyl acetic acid (12.3 mL), and 70% aqueous solution of perchloric acid (0.238 mL, 2.77 mmoL) was added thereto, and the mixture was stirred at room temperature for 22 minutes. The mixture was poured into a solution of ice water and a saturated aqueous solution of sodium hydrogen carbonate and extracted with ethyl acetate. The obtained organic layer was washed with brine, dried over anhydrous sodium sulfate, and concentrated under reduced pressure. The residue was purified by silica gel column chromatography (hexane-ethyl acetate) to obtain Compound(6) (270 mg, 55.4%) as a white solid.
MS(ESI) m/z:572.0 [M + HCOO]-

### Seventh Step

Compound (6) (250 mg, 0.473 mmol) and Compound (7) (195 mg, 0.710mmol) were dissolved in a solution of DMF (2.50 mL) and water (0.250 mL), and potassium carbonate (262 mg, 1.89 mmol) and PdCl₂ (dtbpf) (30.8 mg, 0.0470 mmol) were added thereto, and the operation of degassing and nitrogen substitution was repeated three times. The mixture was stirred under a nitrogen atmosphere at 120°C for 21 minutes. The mixture was cooled to room temperature, poured into a solution of ice water and 2mol/L aqueous solution of hydrochloric acid, and extracted with ethyl acetate. The obtained organic layer was washed with a saturated aqueous solution of sodium hydrogen carbonate and brine, dried over anhydrous sodium sulfate, and concentrated under reduced pressure. The residue was purified by silica gel column chromatography (hexane-ethyl acetate) to obtain Compound (8) (261 mg, 92.6%) as a brown foam.
MS(ESI) m/z:640.6 [M + HCOO]-

### Eighth Step

Compound (8) (255 mg, 0.428 mmol) was dissolved in ethanol (16.8 mL), and 2mol/L aqueous solution of sodium hydroxide (1.07 mL, 2.14mmol) was added thereto, and the mixture was stirred at 90°C for 2 hours 20 minutes. The mixture was cooled to room temperature and poured into ice water, and obtained aqueous layer was adjusted with 2moL/L aqueous solution of hydrochloric acid to about pH=3 and extracted with ethyl acetate. The obtained organic layer was washed with brine, dried over anhydrous sodium sulfate, and concentrated under reduced pressure. The residue was purified by silica gel column chromatography (chloroform-methanol) and dried at 60°C under reduced pressure to obtain Compound 224 (177 mg, 71.1%) as a pale yellow powder.
MS(ESI) m/z:580.4 [M - H]-

### Example 44 Synthesis of Compound 291

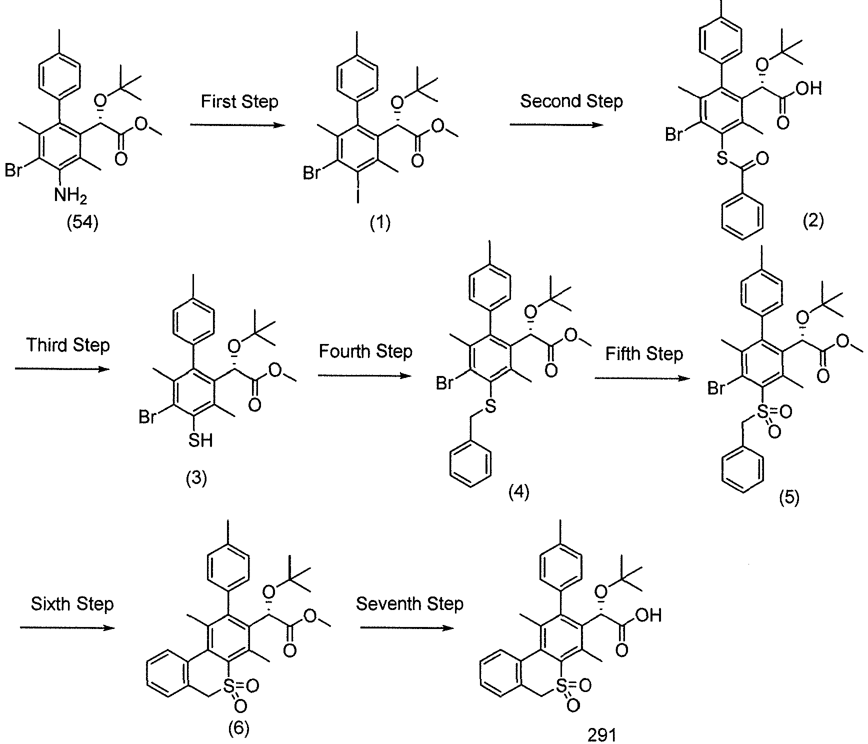

### First Step

Compound (54) (360 mg, 0.829 mmol) was dissolved in acetonitrile (7.2 mL). Tert-butyl nitrite (0.179 mL, 1.492 mmol) and copper iodide (I) (284 mg, 1.492 mmol) were added thereto, and the mixture was stirred for 1.5 hours and then at 60°C for 4 hours. Water (10 mL) was added thereto and the mixture was extracted with hexane (10 mLx2). The obtained organic layer was dried over anhydrous magnesium sulfate, and concentrated. The residue was purified by silica gel column chromatography (hexane-ethyl acetate) to obtain crude Compound (1) (295 mg).
MS(ESI) m/z:567[M+Na]+

### Second Step

The crude Compound (1) (414 mg) was dissolved in toluene (4.1 mL). Thiobenzoic acid (126 mg, 0.911 mmol), 1,10-phenanthroline (27.4 mg, 0.152 mmol), N-ethyl-N-diisopropylamine (0.27 mL, 1.52 mmol) and copper iodide (I) (14.5 mg, 0.076 mmol) were added thereto at room temperature, and the mixture was stirred under heat reflux for 3 hours. A saturated aqueous solution of sodium bicarbonate (10 mL) was added thereto under ice bath and the mixture was extracted with hexane-ethyl acetate (3:1, 10 mLx2). The obtained organic layer was washed with water, dried over anhydrous magnesium sulfate, and concentrated. The residue was purified by silica gel column chromatography (hexane-ethyl acetate) to obtain Compound (2) (148 mg, 0.266 mmol) as a colorless oil.
MS(ESI) m/z:577[M+Na]+

### Third Step

Compound (2) (23 mg, 0.041 mmol) was dissolved in methanol (0.9 mL). Potassium carbonate (11.4 mg, 0.083 mmol) was added thereto and the mixture was stirred for 40 minutes. A saturated aqueous solution of ammonium chloride (2 mL) was added thereto and the mixture was extracted with chloroform (2 mLx5). The obtained organic layer was dried over anhydrous magnesium sulfate, and concentrated. The residue was purified by silica gel column chromatography (hexane-ethyl acetate) to obtain crude Compound (3) (15mg).
MS(ESI) m/z:473[M+Na]+

### Fourth Step

The crdue Compound (3) (15mg) was dissolved in DMF (0.6 mL). Potassium carbonate (14 mg, 0.100 mmol) and benzyl bromide (0.012 mL, 0.100 mmol) were added thereto at room temperature, and the mixture was stirred for 30 minutes. Water (4 mL) was added thereto and the mixture was extracted with ethyl acetate (4 mLx2). The obtained organic layer was washed with water (5 mL), dried over anhydrous magnesium sulfate, and concentrated. The residue was purified by silica gel column chromatography (hexane-ethyl acetate) to obtain Compound (4) (17 mg, 0.031 mmol, 95% in 2 steps) as a colorless oil.
MS(ESI) m/z:563[M+Na]+

### Fifth Step

Compound (4) (17 mg, 0.031 mmol) was dissolved in dichloromethane (0.8 mL). 70% meta-chloroperoxybenzoic acid (18mg, 0.074mmol) was added thereto at room temperature, and the mixture was stirred for 10 minutes. Sodium hydrogen carbonate was added thereto and the mixture was stirred for 2 hours. A saturated aqueous solution of sodium thiosulfate (2 mL) and a saturated aqueous solution of sodium bicarbonate (2 mL) were added thereto and the mixture was extracted with chloroform (2 mLx4). The obtained organic layer was dried over anhydrous magnesium sulfate and concentrated. The residue was purified by silica gel column chromatography (hexane-ethyl acetate) to obtain Compound (5) (14 mg, 0.025 mmol, 83%) as a colorless foam.
MS(ESI) m/z:499 [M-OC(CH₃)₃]+

### Sixth Step

Compound (5) (17 mg, 0.031 mmol) was dissolved in N,N-dimethylacetamide (1mL). Tricyclohexylphosphonium tetrafluoroborate (14.4 mg, 0.039 mmol), potassium carbonate (6.8 mg, 0.049 mmol) and Pd(OAc)₂ (4.4 mg, 0.020 mmol) were added thereto at room temperature and the mixture was stirred at 130°C for 2 hours. Water (5 mL) was added thereto and the mixture was extracted with ethyl acetate (5 mLx2). The obtained organic layer was washed with water, dried over anhydrous magnesium sulfate, and concentrated. The residue was purified by silica gel column chromatography (hexane-ethyl acetate) to obtain Compound (6) (7.9 mg, 0.016 mmol, 66%) as a yellow foam.
MS(ESI) m/z:515 [M+Na]+

### Seventh Step

Compound (6) (7.5 mg, 0.015 mmol) was dissolved in a solution of THF (0.5 mL) and methanol (0.5 mL). 2mol/L aqueous solution of sodium hydroxide (0.038 mL, 0.076 mmol) was added thereto, and the mixture was stirred at 80°C for 2 hours. 2mil/L aqueous solution of hydrochloric acid (0.038 mL, 0.076 mmol) was added thereto and the mixture was extracted with ethyl acetate (5 mLx2). The obtained organic layer was dried over anhydrous magnesium sulfate and concentrated. The residue was purified by silica gel column chromatography (hexane-ethyl acetate) to obtain Compound 291 (6.6 mg, 0.014 mmol, 91%) as a colorless solid.
¹H-NMR (CDCl₃) δ: 1.03 (9H, s), 2.13 (3H, s), 2.44 (3H, s), 2.78 (3H, s), 4.29 (1H, brs), 4.49 (1H, brs), 5.19 (1H, s), 7.19 (1H, s), 7.28-7.49 (8H, m).

### Example 45 Synthesis of Compound 214

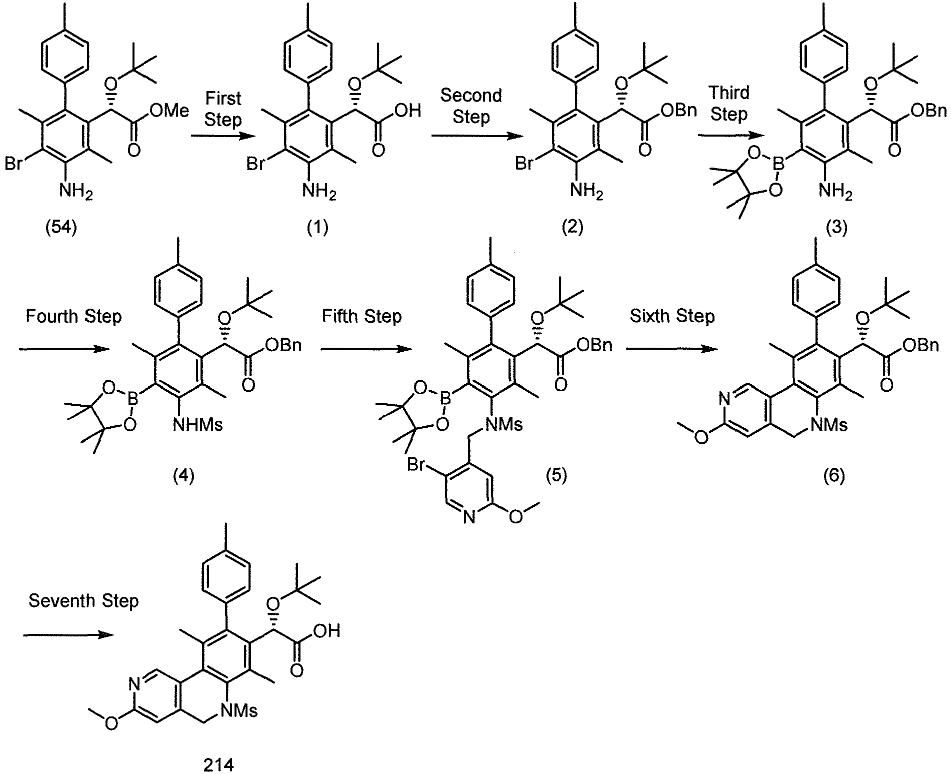

### First Step

Compound (54) (1.30 g, 2.99 mmol) was dissolved in a solution of ethanol (12 mL) and THF (4 mL), and 2mol/L aqueous solution of sodium hydroxide (4 mL, 8 mmol) was added thereto, and the mixture was stirred at 90°C for 1 hour. The mixture was cooled to room temperature, and water (20 ml) was added thereto, and the mixture was adjusted with 2mol/L aqueous solution of hydrochloric acid to pH=3 and extracted with dichloromethane. The obtained organic layer was dried over anhydrous sodium sulfate and concentrated under reduced pressure to obtain crude Compound (1).

### Second Step

The crude Compound (1) was dissolved in DMF (10 mL), and potassium carbonate (1.24 g, 8.97 mmol) and benzyl bromide (563 mg, 3.29 mmol) were added thereto, and the mixture was stirred at room temperature for 1 hour. Water was added thereto and the mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound (2) (1.53 g, 100%)as a colorless foam.
¹H NMR (CDCl₃) 8:0.94 (s, 9H), 2.03 (s, 3H), 2.21 (s, 3H), 2.38 (s, 3H), 4.17 (s, 2H), 5.00 (s, 1H), 5.06 (d, J = 12.0 Hz, 1.5 H), 5.15 (d, J = 12.0 Hz, 1.5H), 6.99-7.08 (m, 3H), 7.15-7.20 (m, 3H), 7.30-7.34 (m, 3H).

### Third Step

Compound (2) (1.53 g, 2.99 mmol) was dissolved in DMF (5 mL), and bis(pinacolato)diboron (2.30g, 8.97mmol), [1,1'-bis(diphenylphosphino)ferrocene] palladium dichloride dichloromethane complex (246 mg, 0.299 mmol) and potassium acetate (889 mg, 8.97 mmol) were added thereto, and the mixture was stirred at 110°C for 0.5 hour. The mixture was cooled to room temperature, and water was added thereto, and the mixture was extracted with ethyl acetate. The obtained organic layer was washed with water, dried over anhydrous magnesium sulfate, and concentrated. The residue was purified by silica gel column chromatography (hexane-ethyl acetate) to obtain Compound (3) (1.61 g, 96%) as a yellow foam.
MS(ESI) m/z:558.1 [M + H]+

### Fourth Step

Compound (3) (1.61 g, 2.89 mmol) was dissolved in dichloromethane (15 mL), and triethylamine (1.46 g, 14.5 mmol) and methanesulfonyl chloride (430 mg, 3.76 mmol) were added thereto under ice bath, and the mixture was stirred at same temperature for 2 hours. Water was added thereto and the mixture was extracted with dichloromethane. The obtained organic layer was dried over anhydrous magnesium sulfate and concentrated. The residue was purified by silica gel column chromatography (hexane-ethyl acetate) to obtain Compound (4) (1.06 g, 58%) as a yellow foam.
¹H NMR (CDCl₃) δ:0.94 (s, 9H), 1.39-1.42 (m, 12H), 2.04 (s, 3H), 2.06 (s, 3H), 2.38 (s, 6H), 2.86 (s, 3H), 5.01 (s, 1 H), 5.08 (d, J = 16.0 Hz, 1H), 5.13 (d, J = 16.0 Hz, 1H), 6.78 (s, 1H), 6.97-7.05 (m, 3H), 7.17-7.26 (m, 3H), 7.30-7.35 (m, 3H).

### Fifth Step

Compound (4) (200 mg, 0.315 mmol) and (5-bromo-2-methoxypyridine4-yl) methanol (89 mg, 0.410 mmol) were dissolved in THF (2 mL), and triphenylphosphine (107 mg, 0.410 mmol) and 40% solution of DEAD in toluene (119 mg, 0.410 mmol) were added thereto at room temperature, and the mixture was stirred at same temperature for 0.5 hour. The mixture was concentrated. The residue was purified by silica gel column chromatography (hexane-ethyl acetate) to obtain Compound (5) (259 mg, 99%) as a colorless foam.
¹H NMR (CDCl₃) 8:0.93 (s, 9H), 1.36-1.41 (m, 12H), 1.59-1.63 (m, 3H), 1.81 (s, 1.5H), 1.97 (s, 1.5H), 2.03-2.06 (m, 3H), 2.36 (s, 1.5H), 2.38 (s, 1.5H), 2.90 (s, 1.5H), 3.05 (s, 1.5H), 3.83 (s, 1.5H), 3.86 (s, 1.5H), 4.80-4.99 (m, 3H), 5.09-5.26 (m, 2H), 6.64 (s, 0.5H), 6.77 (s, 0.5H), 6.95-6.98 (m, 2H), 7.01-7.06 (m, 1H), 7.15-7.37 (m, 5H), 8.17 (s, 1H).

### Sixth Step

Compound (5) (259 mg, 0.309 mmol) was dissolved in a solution of DMF (3 mL) and water (0.3 mL), and potassium carbonate (86 mg, 0.618 mmol) and [1,1'-bis(diphenylphosphino)ferrocene]palladium dichloride dichloromethane complex (20 mg, 0.031 mmol) were added thereto, and the mixture was stirred at 110°C for 1 hour. The mixture was cooled to room temperature, and water was added thereto, and the mixture was extracted with ethyl acetate. The obtained organic layer was washed with water, dried over anhydrous magnesium sulfate, and concentrated. The residue was purified by silica gel column chromatography (hexane-ethyl acetate) to obtain Compound (6) (47 mg, 24%) as a colorless foam.
¹H NMR (CDCl₃) 8:0.95 (s, 9H), 2.15 (s, 3H), 2.21 (s, 3H), 2.41 (s, 3H), 2.48 (s, 3H), 3.99 (s, 3H), 4.30 (d, J = 16 Hz, 1H), 4.90 (d, J = 16 Hz, 1H), 5.00 (s, 1H), 5.15-5.25 (m, 2H), 6.78 (s, 1H), 7.06-7.09 (m, 1H), 7.16-7.18 (m, 2H), 7.21-7.34 (m, 5H), 8.57 (s, 1H).

### Seventh Step

Compound (6) (47 mg, 0.075 mmol) was dissolved in methanol (2 mL), and Pd/C (47mg, 50.6%Wt, 10%wet) was added thereto, and the mixture was stirred under a hydrogen atmosphere at room temperature for 0.5 hour. The mixture was filtered through celite, and the filtrate was concentrated. The residue was purified by silica gel column chromatography (hexane-ethyl acetate) to obtain Compound 214 (29 mg, 72%) as a white solid.
MS(ESI) m/z:539.4 [M + H]+

### Example 46 Synthesis of Compound 305

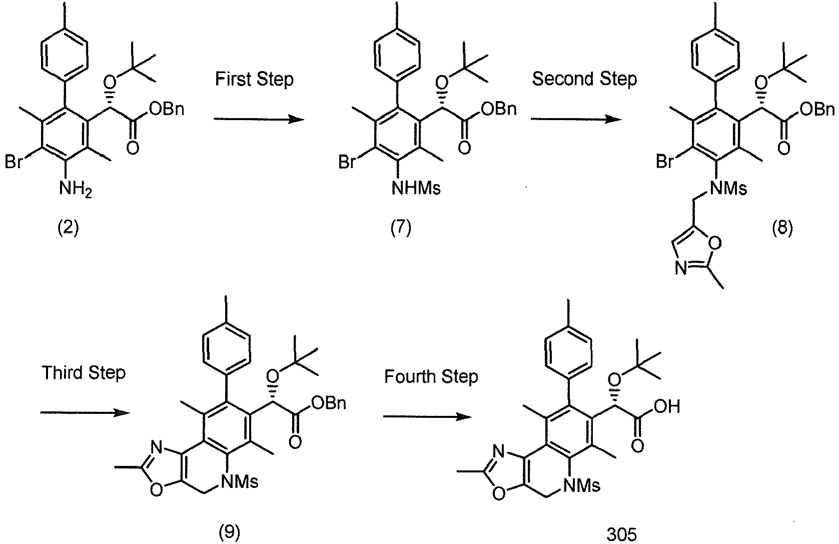

### First Step

Compound (2) (1.88 g, 3.68 mmol) was dissolved in pyridine (20 mL), and methanesulfonyl chloride (1.27 g, 11.0 mmol) was added thereto under ice bath, and the mixture was stirred at room temperature for 8 hours. Water was added thereto and the mixture was extracted with ethyl acetate. The obtained organic layer was washed with 1mol/L aqueous solution of hydrochloric acid, dried over anhydrous magnesium sulfate, and concentrated. The residue was purified by silica gel column chromatography (hexane-ethyl acetate) to obtain Compound (7) (1.53 g, 71%) as a colorless foam.
MS(ESI) m/z:587.1 [M - H]-

### Second Step

Compound (7) (300 mg, 0.510 mmol) and (2-methyloxazole-5-yl)methanol (115 mg, 1.02 mmol) were dissolved in THF (3 mL), triphenylphosphine (268 mg, 1.02 mmol) and bis(2-methoxyethyl) azodicarboxylate (239 mg, 1.02 mmol) were added thereto at room temperature, and the mixture was stirred at same temperature for 1 hour. Water was added thereto and the mixture was extracted with ethyl acetate. The obtained organic layer was washed with water, dried over anhydrous magnesium sulfate, and concentrated. The residue was purified by silica gel column chromatography (hexane-ethyl acetate) to obtain Compound (8) (298 mg, 86%) as a colorless foam.
¹H NMR (CDCl₃) δ:0.92 (s, 9H), 2.04-2.07 (m, 6H), 2.20 (s, 1.5H), 2.22 (s, 1.5H), 2.39-2.43 (m, 6H), 3.22 (s, 1.5H), 3.23 (s, 1.5H), 4.73-4.87 (m, 2H), 4.98 (s, 1H), 5.07-5.18 (m, 2H), 6.63 (s, 0.5H), 6.70 (s, 0.5H), 6.93-7.06 (m, 3H), 7.20-7.52 (m, 7H).

### Third Step

Compound (8) (151 mg, 0.221 mmol) was dissolved in DMA (5 mL), and tricyclohexylphosphonium tetrafluoroborate (49 mg, 0.133 mmol), potassium carbonate (92 mg, 0.663 mmol) and Pd(OAc)₂ (15 mg, 0.066 mmol) were added thereto, and the operation of degassing and nitrogen substitution was repeated three times. The mixture was stirred under a nitrogen atmosphere at 130°C for 0.5 hour. The mixture was cooled to room temperature, and water was added thereto. The mixture was extracted with ethyl acetate. The obtained organic layer was washed with water, dried over anhydrous sodium sulfate, and concentrated under reduced pressure. The residue was purified by silica gel column chromatography (hexane-ethyl acetate) to obtain Compound(9) (70 mg, 59%) as a white foam.
MS(ESI) m/z:603.2 [M + H]+

### Fourth Step

Compound (9) (87 mg, 0.144 mmol) was dissolved in THF (3 mL), and Pd/C (44mg, 10%Wt) was added thereto. The mixture was stirred under a hydrogen atmosphere at room temperature for 10 mimnutes. The mixture was filtered through celite, and the filtrate was concentrated. The residue was purified by silica gel column chromatography (hexane-ethyl acetate) to obtain Compound 305 (54 mg, 73%) as a white solid.
MS(ESI) m/z:513.2 [M + H]+

### Example 47 Synthesis of Compound 288

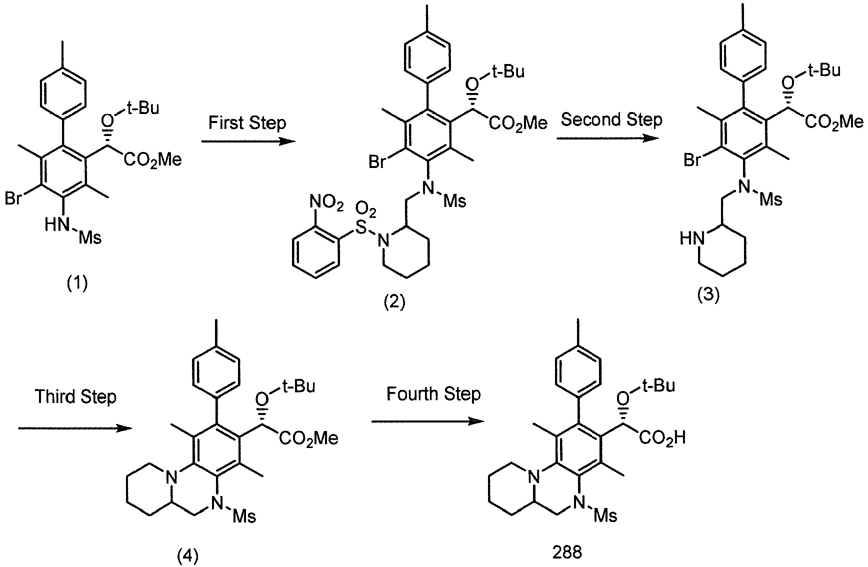

### First Step

Compound (1) (850 mg, 1.66 mmol), N-(2-nitrobenzene sulfonyl) piperidine-2-yl methanol (996 mg, 3.32 mmol) and triphenylphosphine (870 mg, 3.32 mmol) were dissolved in THF (8.5 mL), and bis(2-methoxyethyl)diazen-1,2-dicarboxylate (777 mg, 3.32mmol) was added thereto under ice bath. The mixture was warmed up to room temperature and stirred for 1 hour. Water (50 mL) was added thereto and the mixture was extracted with ethyl acetate (100 mL). The obtained organic layer was washed with water (50 mL) and brine (50 mL), dried over anhydrous magnesium sulfate, and concentrated. The residue was purified by silica gel column chromatography (hexane-ethyl acetate) to obtain Compound (2) (1.21 g, 86%) as a colorless oil.
MS: m/z = 794.4/796.5 [M+H]+

### Second Step

Compound (2) (1.13 g, 1.42 mmol) was dissolved in DMF (11.3mL). Mercaptoacetic acid (0.198mL, 2.84mmol) and lithium hydroxide monohydrate (239 mg, 5.69 mmol) were added thereto, and the mixture was stirred at room temperature for 1 hour. Water (50 mL) was added thereto and the mixture was extracted with ethyl acetate (100 mL). The obtained organic layer was washed with water (50 mL×2) and brine (50 mL), dried over anhydrous magnesium sulfate, and concentrated. The residue was purified by silica gel column chromatography (hexane-ethyl acetate) to obtain Compound (3) (840 mg, 94%) as a colorless oil.
MS: m/z = 609.4/611.4 [M+H]+

### Third Step

Compound (3) (580 mg, 0.951 mmol) was dissolved in dioxane (11.6 mL). RuPhos (133 mg, 0.285 mmol), cesium carbonate (930 mg, 2.85 mmol) and Pd₂(dba)₃ (87.0 mg, 0.095 mmol) were added thereto at room temperature, and the mixture was stirred under a nitrogen atmosphere at 100°C for 8 hours. Water (50 mL) was added thereto and the mixture was extracted with ethyl acetate (100 mL). The obtained organic layer was washed with water (50 mL) and brine (50 mL), dried over anhydrous magnesium sulfate, and concentrated. The residue was purified by silica gel column chromatography (hexane-ethyl acetate) to obtain Compound (4) (392 mg, 75%) as a colorless foam.
MS: m/z = 529.5 [M+H]+

### Fourth Step

Compound (4) (370 mg, 0.700 mmol) was dissolved in ethanol (7.4 mL). 2mmol/L aqueous solution of sodium hydroxide (3.50 mL, 7.00 mmol) was added thereto at room temperature, and the mixture was stirred under heat reflux for 1.5 hours. 1mol/L aqueous solution of hydrochloric acid (7 mL) and brine (30 mL) were added thereto and the mixture was extracted with ethyl acetate (50 mL). The obtained organic layer was dried over anhydrous magnesium sulfate, and concentrated. The residue was purified by silica gel column chromatography (chloroform-methanol) and HPLC (0.1% formic acid-containing aqueous solution-acetonitrile) to obtain Compound 288 (169 mg, 47%, 116mg, 32%) as a brown oil. MS: m/z = 515.4 [M+H]+

### Example 48

The following compounds were synthesized according to the above example.

**[Table 35]**

| No. | **Structure** | RT (min) | | MS [M+H]+ | |
|---|---|---|---|---|---|
| 199 | 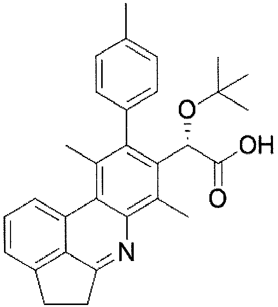 | 2.38 | | 454.4 | |
| 200 | 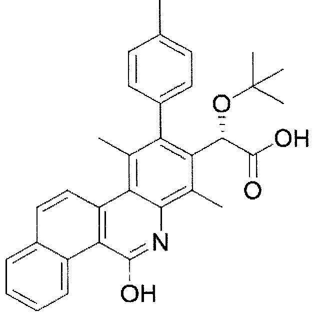 | 2.98 | | 494.3 | |
| 201 | 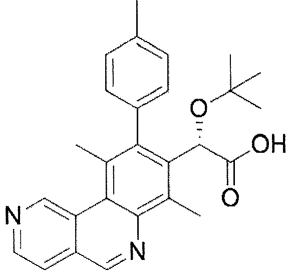 | 2.29 | * | 527.1 | [M-H]- |
| 202 | 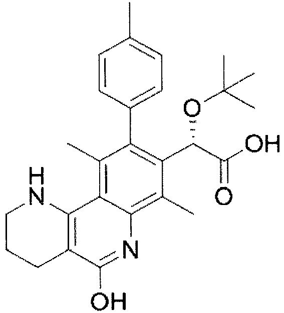 | 2.10 | | 449.4 | |

**[Table 36]**

| | | | | | |
|---|---|---|---|---|---|
| 203 | 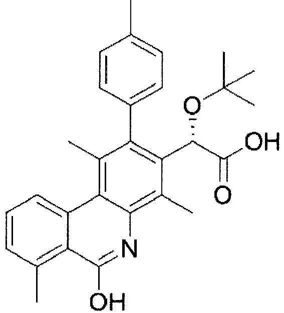 | 2.84 | | 458 | |
| 204 | 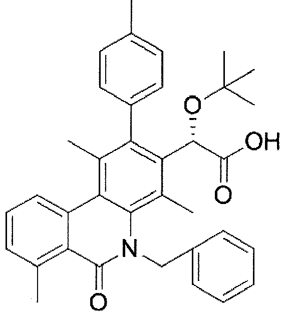 | 3.21 | | 548 | |
| 205 | 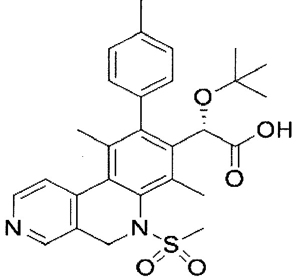 | 1.78 | * | 509.4 | |

**[Table 37]**

| | | | | | |
|---|---|---|---|---|---|
| 206 | 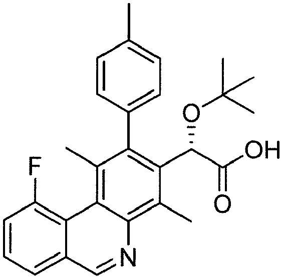 | 2.95 | | 446 | |
| 207 | 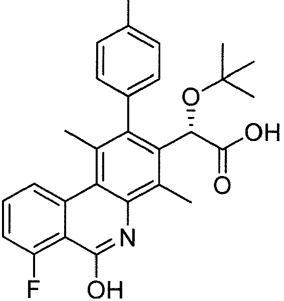 | 2.52 | | 462 | |

**[Table 38]**

| | | | | | |
|---|---|---|---|---|---|
| 208 | 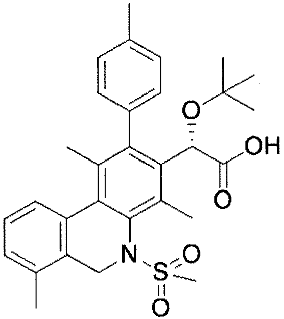 | 2.73 | * | 544 | [M+Na]+ |
| 209 | 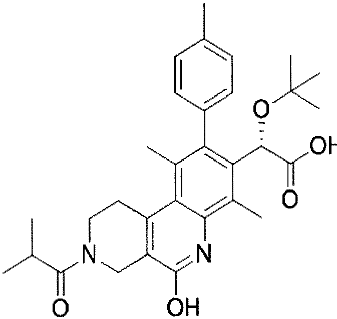 | 2.24 | | 519 | |
| 210 | 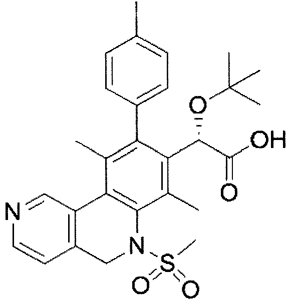 | 1.80 | * | 509.4 | |
| 211 | 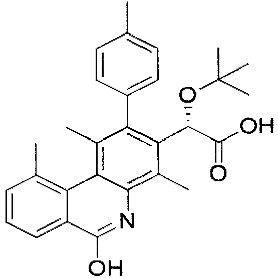 | 2,67 | | 458 | |

**[Table 39]**

| | | | | | |
|---|---|---|---|---|---|
| 212 | 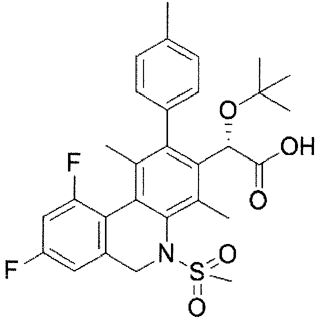 | 2.64 | * | 566 | [M+Na]+ |
| 215 | 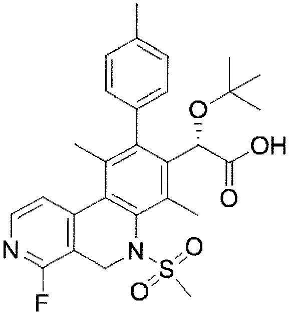 | 2.33 | * | 527.3 | |
| 216 | 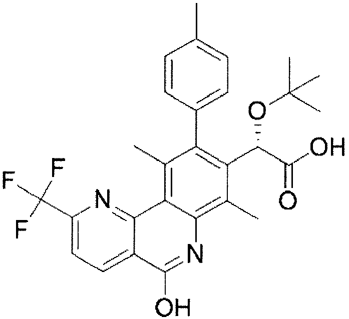 | 2.93 | | 513 | |
| 217 | 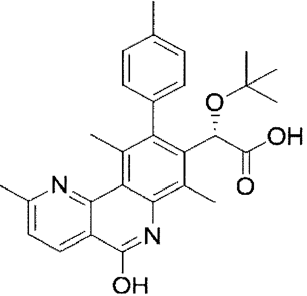 | 2.76 | | 459 | |

**[Table 40]**

| | | | | | |
|---|---|---|---|---|---|
| 218 | 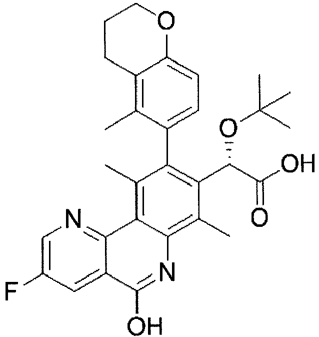 | 2.69 | | 519.4 | |
| 220 | 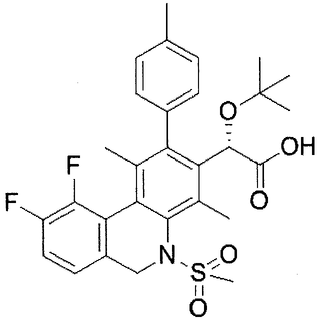 | 2.62 | * | 566 | [M+Na]+ |
| 221 | 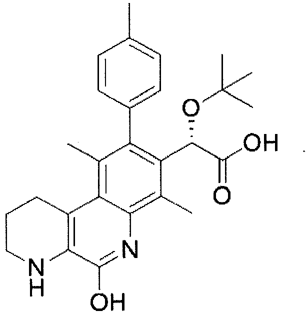 | 2.50 | | 449.4 | |
| 222 | 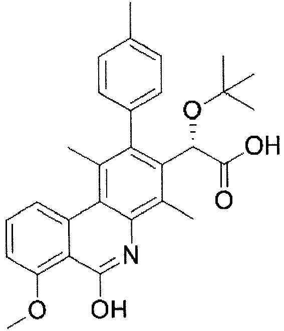 | 2.46 | | 474 | |

**[Table 41]**

| | | | | | |
|---|---|---|---|---|---|
| 223 | 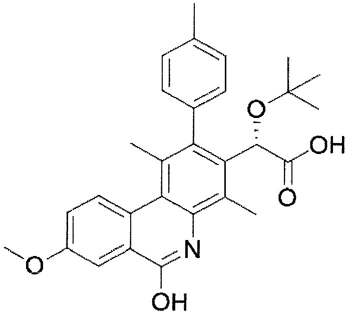 | 2.69 | | 474 | |
| 226 | 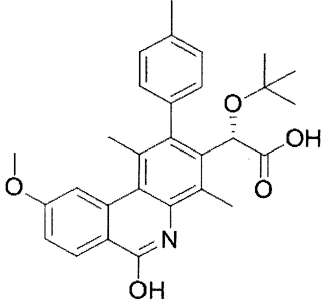 | 2.61 | | 474 | |
| 227 | 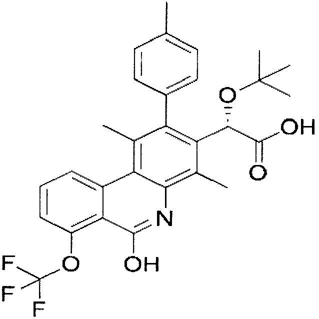 | 2.90 | | 528 | |
| 228 | 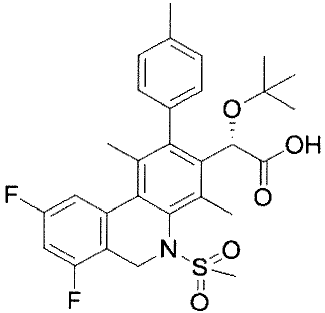 | 2.64 | * | 566 | [M+Na]+ |

**[Table 42]**

| | | | | | |
|---|---|---|---|---|---|
| 229 | 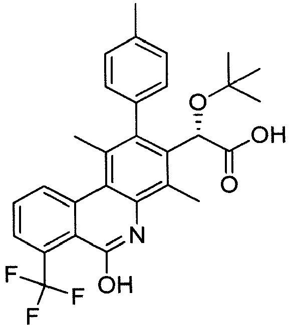 | 2.88 | | 512 | |
| 230 | 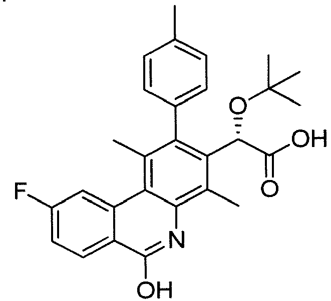 | 2.73 | | 462 | |
| 231 | 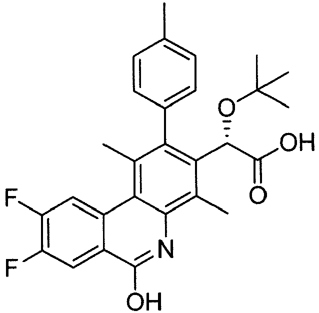 | 2.82 | | 480 | |
| 232 | 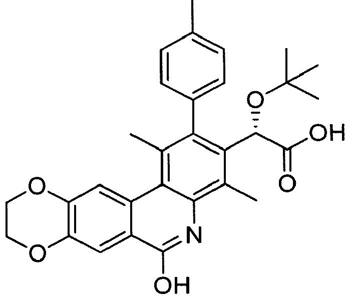 | 2.55 | | 502 | |

**[Table 43]**

| | | | | | |
|---|---|---|---|---|---|
| 233 | 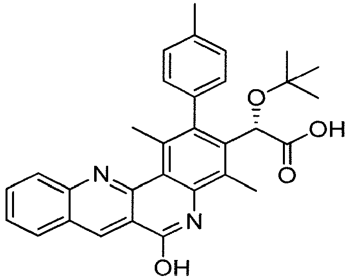 | 3.10 | | 495 | |
| 235 | 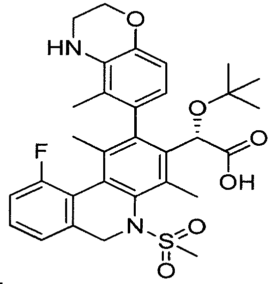 | 2.41 | | 583.4 | |
| 236 | 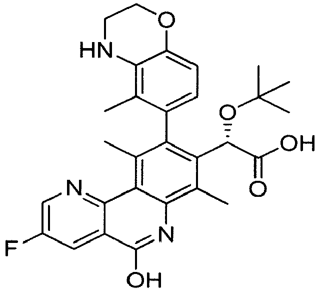 | 2.11 | | 520 | |
| 237 | 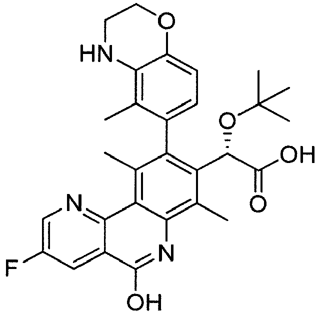 | 2.39 | | 520 | |

**[Table 44]**

| | | | | | |
|---|---|---|---|---|---|
| 238 | 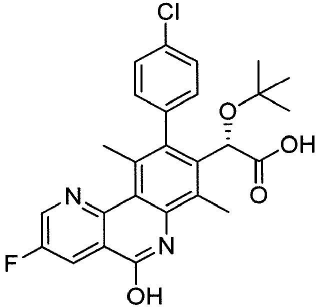 | 2.75 | | 483 | |
| 239 | 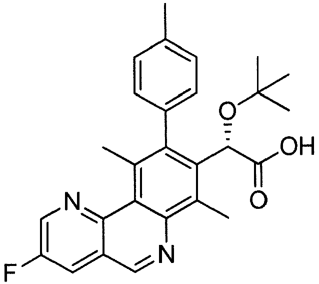 | 2.97 | | 447.4 | |
| 240 | 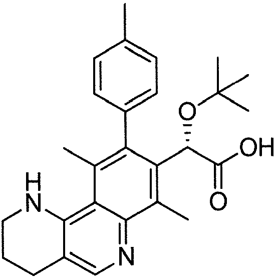 | 1.56 | | 433.4 | |
| 241 | 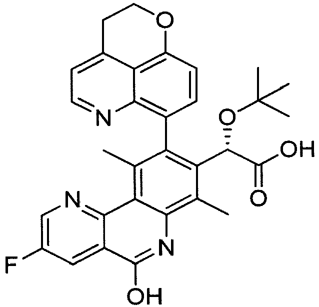 | 1.79 | | 542 | |

**[Table 45]**

| | | | | | |
|---|---|---|---|---|---|
| 242 | 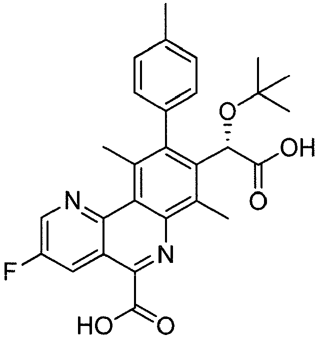 | 2.90 | | 491 | |
| 243 | 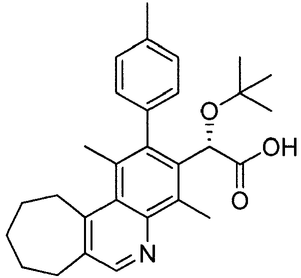 | 2.38 | | 446 | |
| 244 | 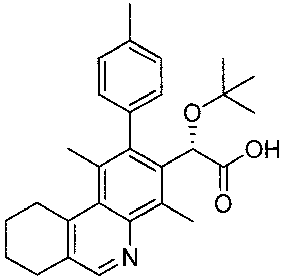 | 2.34 | | 432 | |
| 245 | 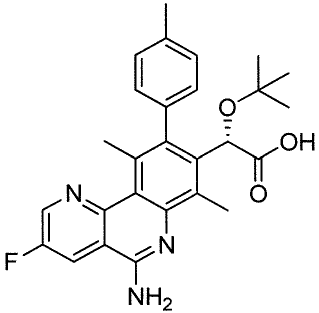 | 2.27 | | 462.4 | |

**[Table 46]**

| | | | | | |
|---|---|---|---|---|---|
| 246 | 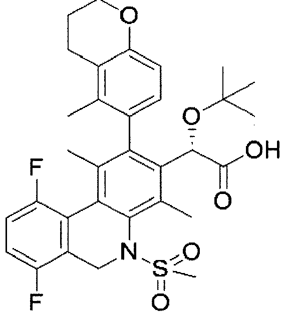 | 2.63 | * | 622 | [M+Na]+ |
| 248 | 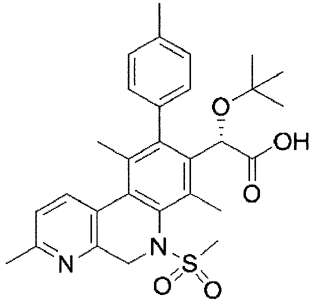 | 2.24 | * | 523.3 | |
| 249 | 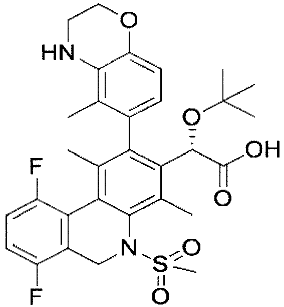 | 2.38 | * | 599 | [M-H]- |
| 251 | 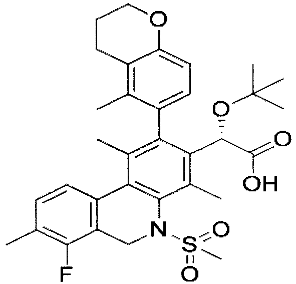 | 2.90 | | 594.3 | [M-H]- |

**[Table 47]**

| | | | | | |
|---|---|---|---|---|---|
| 254 | 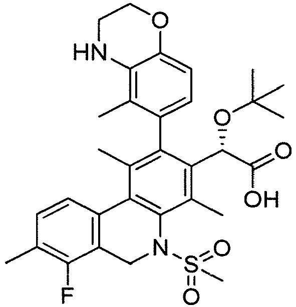 | 2.70 | | 597.4 | |
| 255 | 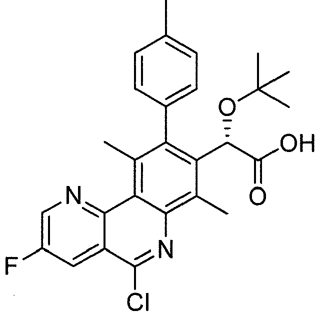 | 3.36 | | 481 | |
| 256 | 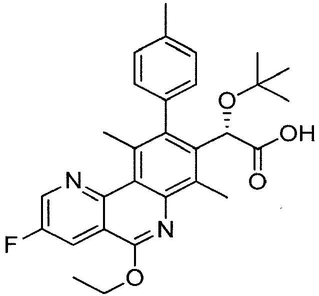 | 3.54 | | 491 | |
| 257 | 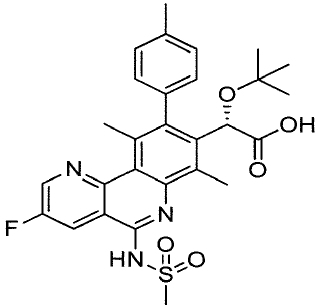 | 2.86 | | 540 | |

**[Table 48]**

| | | | | | |
|---|---|---|---|---|---|
| 258 | 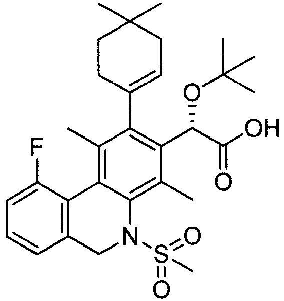 | 3.02 | | 542.4 | [M-H]- |
| 259 | 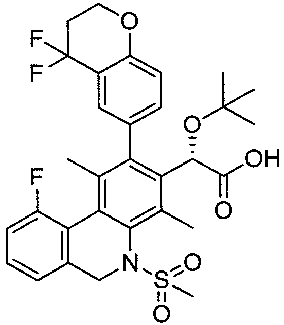 | 2.53 and 2.55 | | 602.4 | [M-H]- |
| 260 | 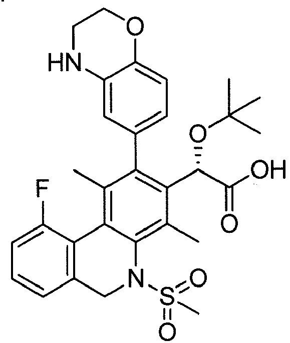 | 2.15 and 2.36 | | 567.5 | [M-H]- |
| 262 | 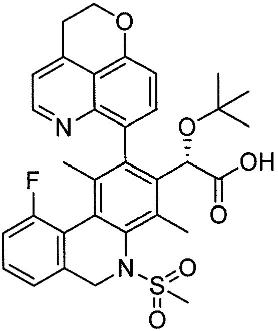 | 1.80 | | 605.4 | |

**[Table 49]**

| | | | | | |
|---|---|---|---|---|---|
| 263 | 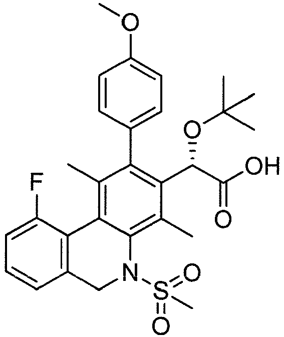 | 2.50 | | 540.5 | [M-H]- |
| 264 | 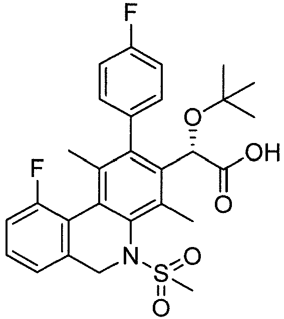 | 2.56 | | 528.4 | [M-H]- |
| 265 | 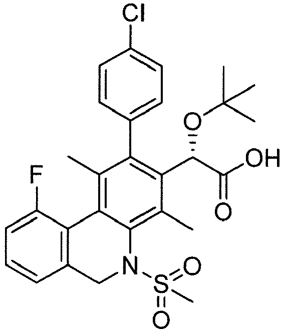 | 2.71 | | 544.5 | [M-H]- |
| 266 | 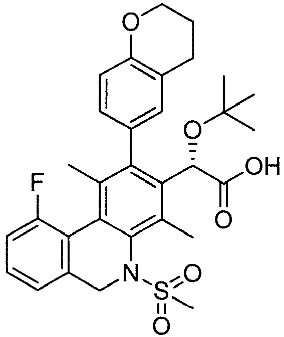 | 2.58 and 2.62 | | 566.5 | [M-H]- |

**[Table 50]**

| | | | | | |
|---|---|---|---|---|---|
| 273 | 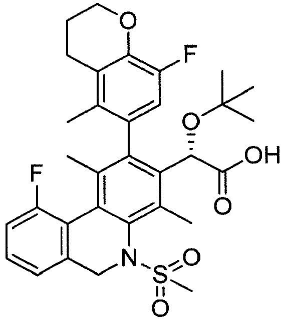 | 2.65 | | 598 | [M-H]- |
| 275 | 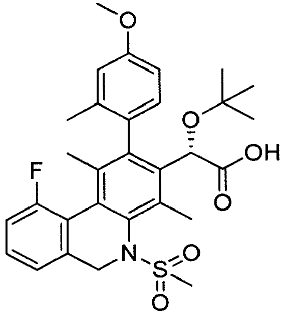 | 2.62 | | 554 | [M-H]- |
| 278 | 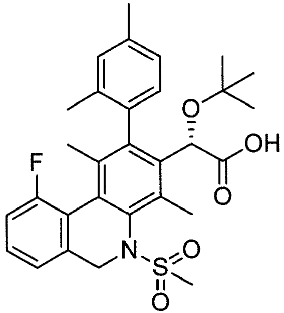 | 2.80 | | 538 | [M-H]- |
| 279 | 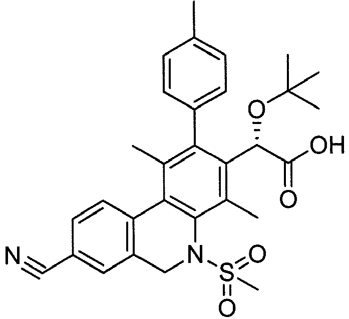 | 2.50 | | 533.3 | |

**[Table 51]**

| | | | | | |
|---|---|---|---|---|---|
| 282 | 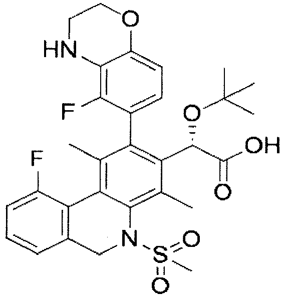 | 2.38 | | 587 | |
| 283 | 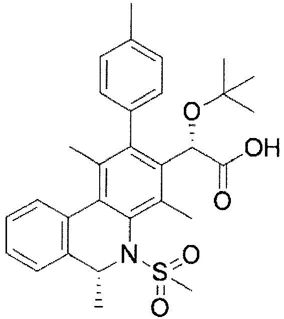 | 2.87 | * | 522 | |
| 284 | 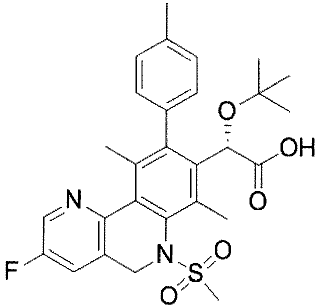 | 2.51 | * | 527.3 | |
| 285 | 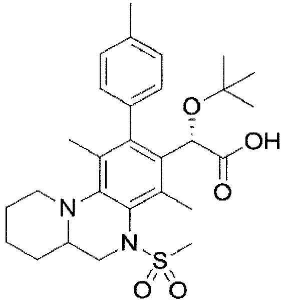 | 2.76 and 2.82 | | 515.4 | |

**[Table 52]**

| | | | | | |
|---|---|---|---|---|---|
| 286 | 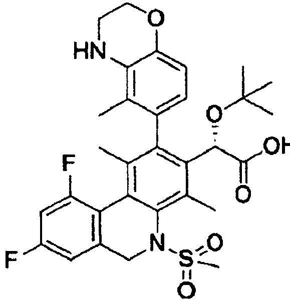 | 2.39 | * | 623 | [M+Na]+ |
| 287 | 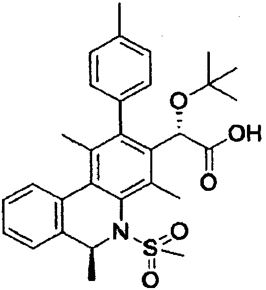 | 2.66 | * | 544 | [M+Na]+ |
| 290 | 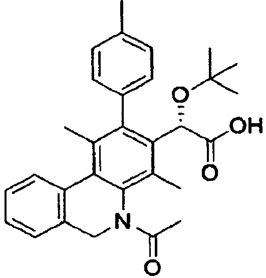 | 2.59 | | 472.4 | |
| 292* | 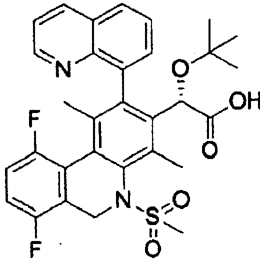 | 2.28 | | 581 | |

| | | | | | |
|---|---|---|---|---|---|
| *reference example | | | | | |

**[Table 53]**

| | | | | | |
|---|---|---|---|---|---|
| 293 | 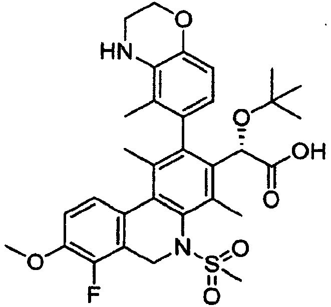 | 2.34 and 2.53 | | 613.4 | |
| 295* | 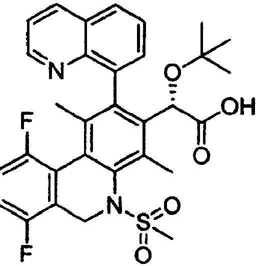 | 1.93 | | 581 | |
| 296 | 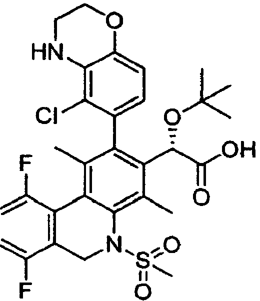 | 2.46 | | 621 | |
| 297 | 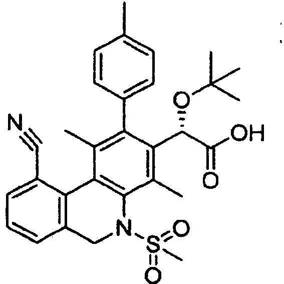 | 2.46 | | 534.4 | |

| | | | | | |
|---|---|---|---|---|---|
| *reference example | | | | | |

**[Table 54]**

| | | | | | |
|---|---|---|---|---|---|
| 298 | 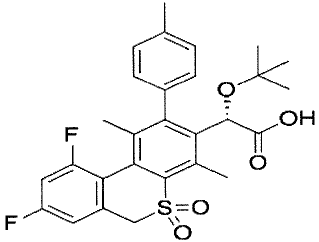 | 2.59 | * | 537.2 | [M+Na]+ |
| 299 | 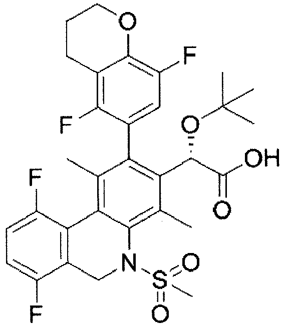 | 2.57 | | 622 | |
| 300 | 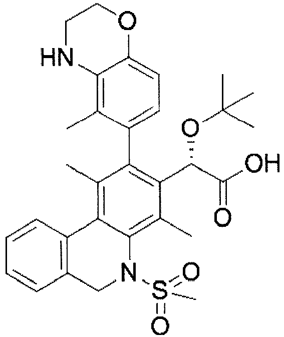 | 2.44 | * | 491 | [M-OBu]+ |
| 301 | 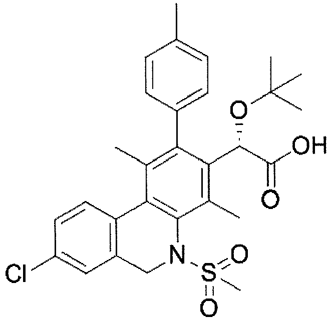 | 2.85 | | 540.3 | [M-H]- |

**[Table 55]**

| | | | | | |
|---|---|---|---|---|---|
| 302 | 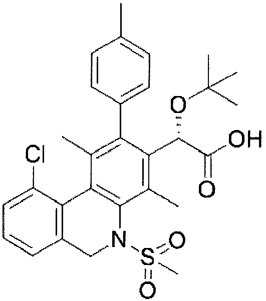 | 2.88 | | 540.3 | [M-H]- |
| 304 | 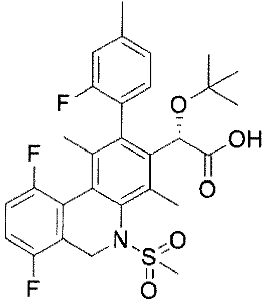 | 2.61 | | 562 | |
| 306 | 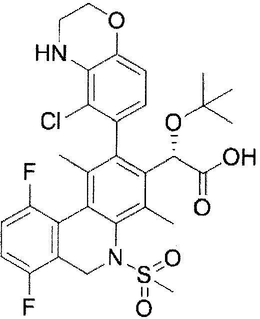 | 2.36 | | 621 | |
| 307 | 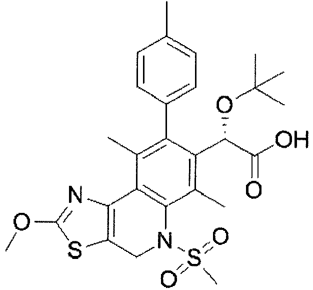 | 2.61 and 2.75 | * | 545.2 | |

**[Table 56]**

| | | | | | |
|---|---|---|---|---|---|
| 308 | 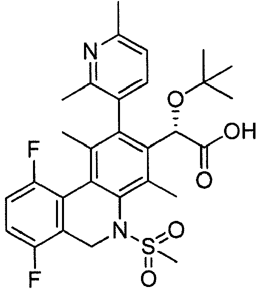 | 1.64 | | 559 | |
| 309 | 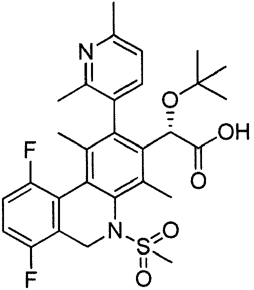 | 1.57 | | 559 | |
| 310 | 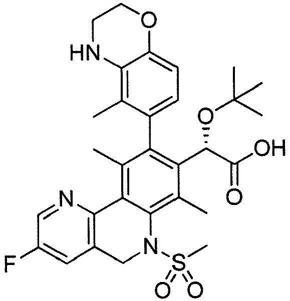 | 2.37 | | 584.4 | |
| 311 | 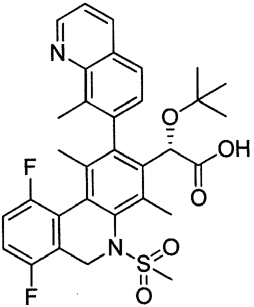 | 2.18 | | 595 | |

Compound 311 is a reference example

**[Table 57]**

| | | | | | |
|---|---|---|---|---|---|
| 312 | 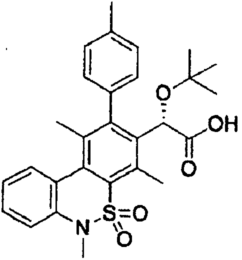 | 2.61 | * | 494.4 | |
| 313* | 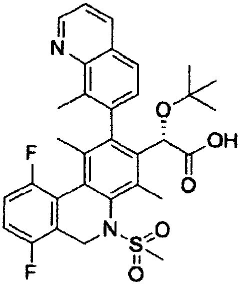 | 2.11 | | 595 | |
| 314 | 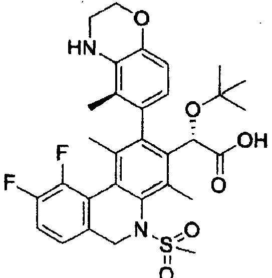 | 2.41 | * | 623 | [M+Na]+ |
| 315 | 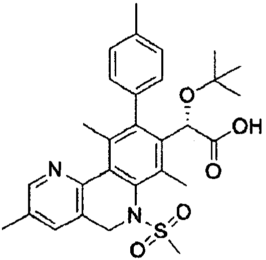 | 2.48 | * | 523.2 | |

| | | | | | |
|---|---|---|---|---|---|
| *reference example | | | | | |

**[Table 58]**

| | | | | | |
|---|---|---|---|---|---|
| 316 | 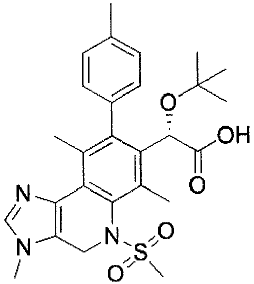 | 1.57 and 1.74 | * | 512.2 | |
| 317 | 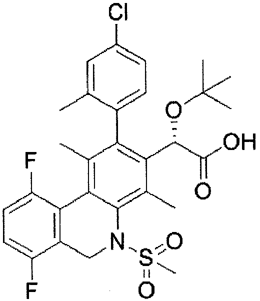 | 2.89 | | 578 | |
| 318 | 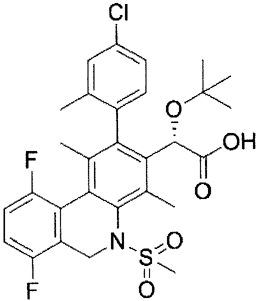 | 2.86 | | 578 | |
| 319 | 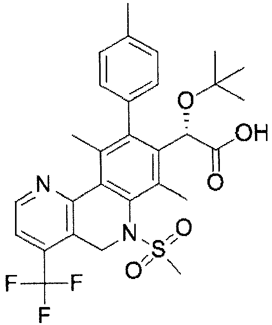 | 2.64 | * | 577.4 | |

**[Table 59]**

| | | | | | |
|---|---|---|---|---|---|
| 320 | 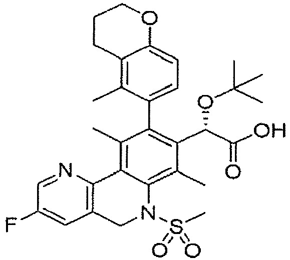 | 2.64 | | 583.4 | |
| 321 | 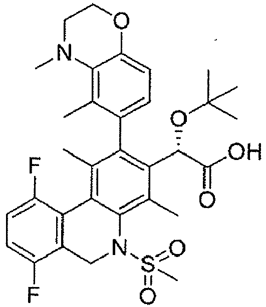 | 2.57 | | 615 | |
| 322 | 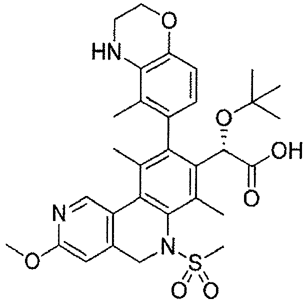 | 2.19 and 2.33 | | 596.4 | |
| 323 | 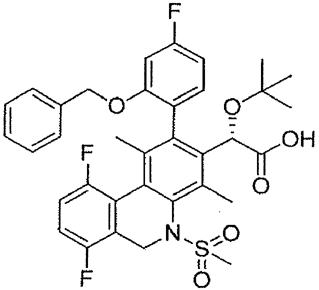 | 2.82 | | 652 | [M-H]- |

### Example 49 Synthesis of Compound 324

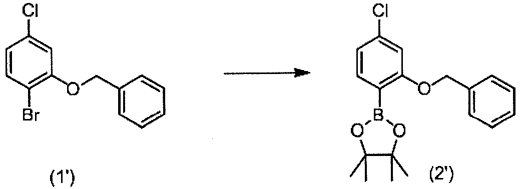

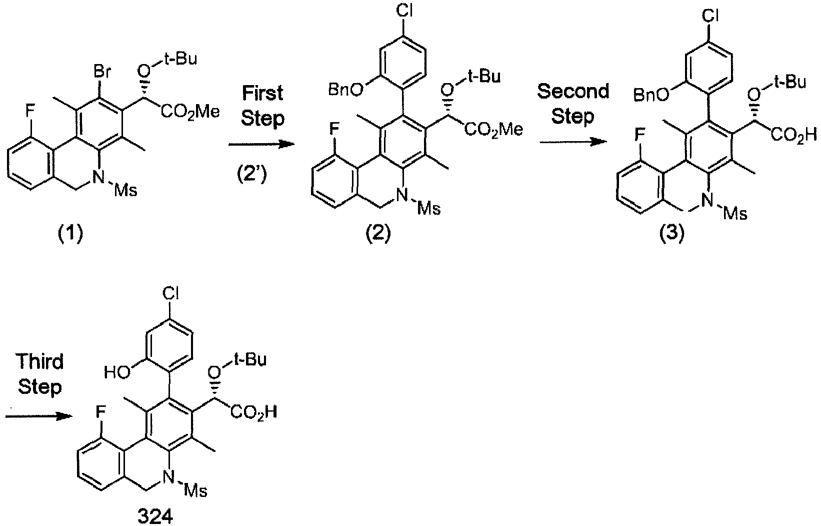

### (Synthesis of Compound (2'))

Compound (1') (6.64 g, 22.3 mmol) was dissolved in DMF (50 mL), and potassium acetate (6.57 g, 66.9 mmol), bis(pinacolato)diboron (7.37 g, 29.0 mmol), potassium carbonate (2.20 g, 15.9 mmol) and [1,1'-bis(diphenylphosphino)ferrocene] palladium dichloride (911 mg, 1.12 mmol) were added thereto. The mixture was stirred under a nitrogen atmosphere at 100°C for 4 hours. The mixture was cooled to room temperature and ethyl acetate (100 mL) was added thereto. The insoluble material was filtered. To the filtrate was added water (100 mL) and the mixture was extracted with ethyl acetate. The obtained organic layer was washed with water, dried over anhydrous magnesium sulfate, and concentrated. The residue was purified by silica gel column chromatography (hexane-ethyl acetate) to obtain Compound (2') (3.85 g, 50%) as a brown solid.
¹H-NMR (CDCl₃) δ: 1.36 (s, 12H), 5.10 (s, 2H), 6.93-6.97 (m, 2H), 7.28-7.42 (m, 3H), 7.59-7.63 (m, 3H).

### First Step Synthesis of Compound (2)

Compound (1) (500 mg, 0.946 mmol) was dissolved in a solution of DMF (5.0 mL) and water (0.5 mL), and Compound (2') (185 mg, 0.284 mmol), potassium carbonate (523 mg, 3.79 mmol) were added thereto. The mixture was stirred under a nitrogen atmosphere at 120°C for 15 mimnutes. Water (50 mL) was added thereto and the mixture was extracted with ethyl acetate (50 mL). The obtained organic layer was washed with water (50 mL) and brine (50 mL), dried over anhydrous magnesium sulfate, and concentrated to obtain crude Compound (2) (473mg) as a brown foam.

### Second Step Synthesis of Compound (3)

Compound (2) (470 mg, 0.705 mmol) was dissolved in ethanol (4.7 mL), and 2mol/L aqueous solution of sodium hydroxide (1.8 mL, 3.60 mmol) was added thereto at room temperature. The mixture was stirred under heat reflux for 0.5 hour. 2mol/L aqueous solution of hydrochloric acid (1.8 mL) and brine (50 mL) were added thereto and the mixture was extracted with ethyl acetate (50 mL). The obtained organic layer was dried over anhydrous magnesium sulfate and concentrated. The residue was purified by silica gel column chromatography (chloroform-methanol) to obtain Compound (3) (237 mg, 38% in 2steps) as a brown foam.
MS: m/z = 650 [M-H]-

### Third Step Synthesis of Compound 324

Compound (3) (237 mg, 0.363mmol) was dissolved in methanol (2.4 mL), and 10% Platinum carbon (213mg, 0.110mmol) was added thereto at room temperature. The mixture was stirred under a hydrogen atmosphere at room temperature for 18 hours. The mixture was filtered through celite, and the filtrate was concentrated. The residue was purified by silica gel column chromatography (chloroform-methanol) and preparative thin layer chromatography (chloroform-methanol) to obtain Compound (4) (7.9 mg, 3.9%) as a yellow foam.
Compound(4) MS: m/z = 560 [M-H]⁻

### Example 50 Synthesis of Compounds 325 and 326

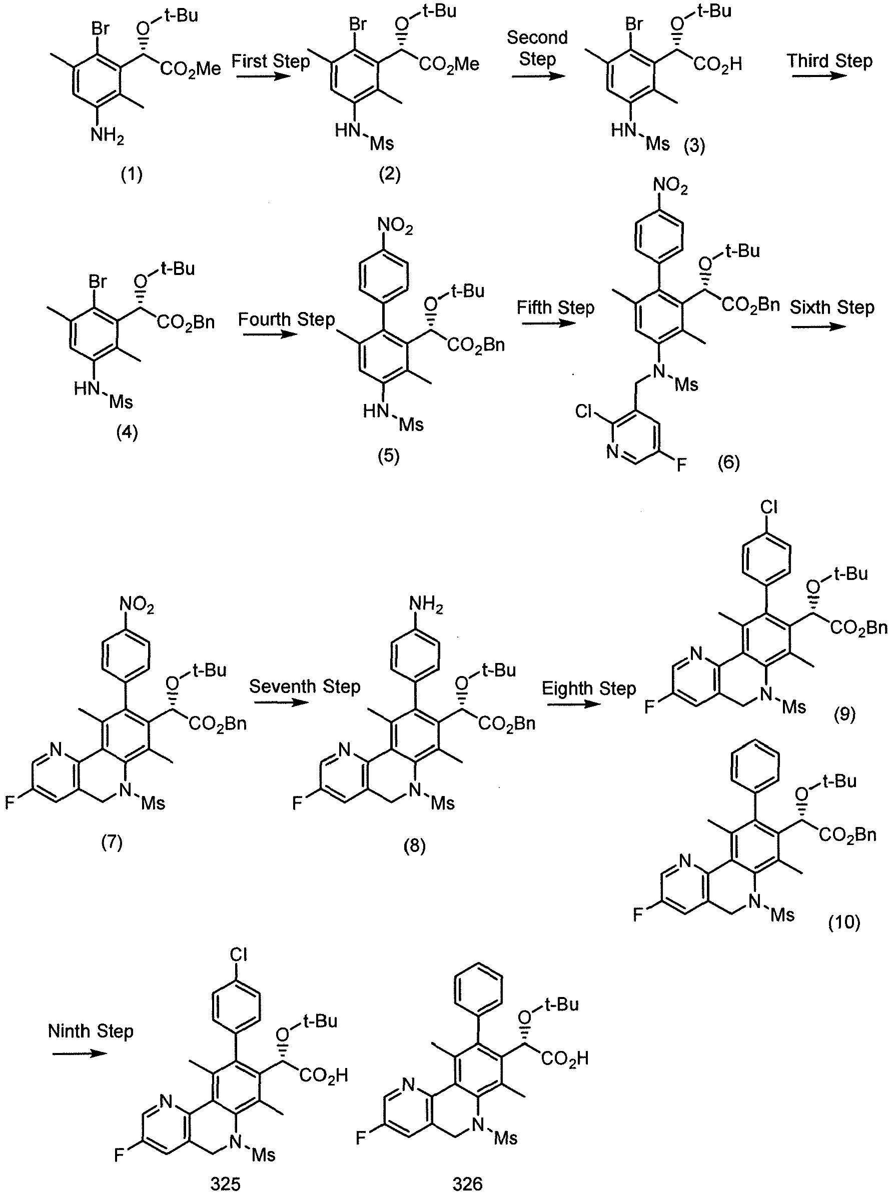

### First Step Synthesis of Compound (2)

Compound (1) (30.0 g, 87.0 mmol) was dissolved in dichloromethane (300 mL), pyridine (28.1 mL, 349 mmol) and methanesulfonyl chloride (20.4 mL, 261 mmol) were added thereto under ice bath. The mixture was warmed up to room temperature and stirred for 4 hours. Water (200 mL) and 1mol/L aqueous solution of hydrochloric acid (500 mL) were added thereto and the mixture was extracted with ethyl acetate (1000 mL). The obtained organic layer was washed with 1mol/L aqueous solution of hydrochloric acid (500mL), water (500 mL) and brine (250 mL), dried over anhydrous magnesium sulfate, and concentrated to obtain crude Compound (2) (41.6g) as a colorless oil.
¹H-NMR (CDCl₃, 400 MHz) δ: 1.21 (s, 9H), 2.33 (s, 3H), 2.44 (s, 3H), 3.00 (s, 3H), 3.69 (s, 3H), 6.00 (s, 1H), 6.22 (s, 1H), 7.37 (s, 1H).
MS: m/z = 420.3, 422.3 [M+H]+

### Second Step Synthesis of Compound (3)

Compound (2) (36.8 g, 87.0 mmol) was dissolved in ethanol (520 mL), and 2mol/L aqueous solution of sodium hydroxide (131 mL, 261 mmol) was added thereto at room temperature. The mixture was stirred under heat reflux for 1.5 hours and concentrated. To the residue was added 1mol/L aqueous solution of hydrochloric acid (500 mL) and the mixture was extracted with ethyl acetate (1000 mL). The obtained organic layer was washed with brine (300 mL), dried over anhydrous magnesium sulfate, and concentrated to obtain crude Compound (3) (36.0 g) as a brown oil.
MS: m/z = 406.2, 408.1 [M+H]+

### Third Step Synthesis of Compound (4)

Compound (3) (35.6 g, 87.0 mmol) was dissolved in DMF (240 mL), and potassium bicarbonate (13.1 g, 131 mmol) was added thereto at room temperature. The mixture was stirred at same temperature for 5 mimnutes and then a solution of benzyl bromide (10.1 mL, 85.0 mmol) in DMF (116 mL) was added thereto. The mixture was stirred for 16.5 hours. Water (350 mL) was added thereto and the mixture was extracted with ethyl acetate (1200 mL). The obtained organic layer was washed with water (500 mL×2) and brine (400 mL), dried over anhydrous magnesium sulfate, and concentrated to obtain Compound (4) (37.4g, 86% in 3steps) as a brown oil.
¹H-NMR (CDCl₃) δ: 1.21 (s, 9H), 2.19 (s, 3H), 2.44 (s, 3H), 2.89 (s, 3H), 5.07 (d, J = 12.6 Hz, 1H), 5.21 (d, J = 12.6 Hz, 1H), 6.06 (s, 1H), 6.11 (s, 1H), 7.16-7.21 (m, 2H), 7.25-7.29 (m, 3H), 7.37 (s, 1H).
MS: m/z = 496.4, 498.4 [M-H]+

### Fourth Step Synthesis of Compound (5)

Compound (4) (3.96 g, 7.94 mmol) was dissolved in a solution of DMA (19.8 mL) and water (1.98 mL), and 4-nitrophenyl boronic acid (1.99 g, 11.9 mmol), [1,1'-bis(di-tert-butylphosphino)ferrocene]palladium dichloride (259 mg, 0.397 mmol) and potassium carbonate (2.20 g, 15.9 mmol) were added thereto. The mixture was stirred under a nitrogen atmosphere at 130°C for 45 mimnutes. Water (100 mL) was added thereto and the mixture was extracted with ethyl acetate (150 mL). The obtained organic layer was washed with water (100cmL×2) and brine (100mL), dried over anhydrous magnesium sulfate, and concentrated. The residue was purified by silica gel column chromatography (hexane-ethyl acetate) to obtain Compound (5) (3.44g, 80%) as a brown foam.
¹H-NMR (CDCl₃) δ: 0.96 (s, 9H), 1.92 (s, 3H), 2.28 (s, 3H), 3.02 (s, 3H), 4.83 (s, 1H), 5.11 (s, 2H), 6.19 (s, 1H), 7.16-7.22 (m, 2H), 7.29-7.37 (m, 5H), 7.41 (s, 1H), 8.09 (dd, J = 8.7, 2.0 Hz, 1H), 8.27 (dd, J = 8.7, 2.0 Hz, 1H).
MS: m/z = 539.3 [M-H]+

### Fifth Step Synthesis of Compound (6)

Compound (5) (1.50 g, 2.77 mmol) was dissolved in THF (15 mL), 2-chlori-6-fluoro-3-(hydroxymethyl)pyridine (538 mg, 3.33 mmol), di-(2-methoxyethyl) azodicarboxylate (780 mg, 3.33 mmol) and triphenylphosphine (873 mg, 3.33 mmol) were added thereto at room temperature. The mixture was stirred at same temperature for 15 hours. Water (100 mL) was added thereto and the mixture was extracted with ethyl acetate (150 mL). The obtained organic layer was washed with water (100 mL×2) and brine (100mL), dried over anhydrous magnesium sulfate, and concentrated. The residue was purified by silica gel column chromatography (hexane-ethyl acetate) to obtain Compound (6) (1.67 g, 88%) as a brown foam.
MS: m/z = 684.3 [M+H]+

### Sixth Step Synthesis of Compound (7)

Compound (6) (1.67 g, 2.44 mmol) was dissolved in DMA (16.7 mL), and tricyclohexylphosphonium tetrafluoroborate (539 mg, 1.47 mmol), sodium hydrogen carbonate (410 mg, 4.88 mmol) and palladium acetate (164 mg, 0.732 mmol) were added thereto at room temperature. The mixture was stirred under a nitrogen atmosphere at 130°C for 5 hours. Water (100 mL) was added thereto and the mixture was extracted with ethyl acetate (150mL). The obtained organic layer was washed with water (100mL) and brine (50 mL), dried over anhydrous magnesium sulfate, and concentrated. The residue was purified by silica gel column chromatography (hexane-ethyl acetate) to obtain Compound (7) (778 mg, 49%) as a brown foam.
MS: m/z = 648.3 [M+H]+

### Seventh Step Synthesis of Compound (8)

Compound (7) (775 mg, 1.20 mmol) was dissolved in acetic acid (7.75m L), and zinc powder (391 mg, 5.98 mmol) was added thereto at room temperature. The mixture was stirred at 60°C for 1 hour and then concentrated. To the residue was added a saturated aqueous solution of sodium hydrogen carbonate (50 mL) and the mixture was extracted with ethyl acetate (100 mL). The obtained organic layer was washed with brine (50 mL), dried over anhydrous magnesium sulfate, and concentrated. The residue was purified by silica gel column chromatography (hexane-ethyl acetate) to obtain Compound (8) (390 mg, 53%) as a colorless foam.
MS: m/z = 618.3 [M+H]+

### Eighth Step Synthesis of Compound (9) and Compound (10)

Compound (8) (105 mg, 0.170 mmol) was dissolved in acetonitrile (2.1 mL), and tert-butyl nitrite (0.0408 mL, 0.340 mmol) and copper chloride (II) (45.7 mg, 0.340 mmol) were added thereto under ice bath. The mixture was warmed up to room temperature and stirred for 1 hour. Water (30 mL) was added thereto and the mixture was extracted with ethyl acetate (50 mL). The obtained organic layer was washed with brine (30 mL), dried over anhydrous magnesium sulfate, and concentrated. The residue was purified by silica gel column chromatography (hexane-ethyl acetate) to obtain the mixture of Compound (9) and Compound (10) (58.4 mg) as a brown foam.
Compound (9) MS: m/z = 637.2 [M+H]+
Compound (10) MS: m/z = 603.3 [M+H]+

### Ninth Step Synthesis of Compound 325 and Compound 326

The mixture of Compound (9) and Compound (10) (56.0 mg) was dissolved in methanol (2 mL), and 10% platinum carbon (17.2 mg, 0.009 mmol) was added thereto at room temperature. The mixture was stirred under a hydrogen atmosphere at room temperature for 1 hour. The mixture was filtered through celite, and the filtrate was concentrated. The residue was purified by silica gel column chromatography (chloroform-methanol) and HPLC (0.1% formic acid-containing aqueous solution-acetonitrile) to obtain Compound 325 (18.3 mg, 19% in 2steps) as a pink solid and Compound 326 (9.2mg, 11% in 2steps) as a pink solid.
Compound 325 MS: m/z = 547.2 [M+H]+
Compound 326 MS: m/z = 513.2 [M+H]+

### Example 51 Synthesis of Compound 327

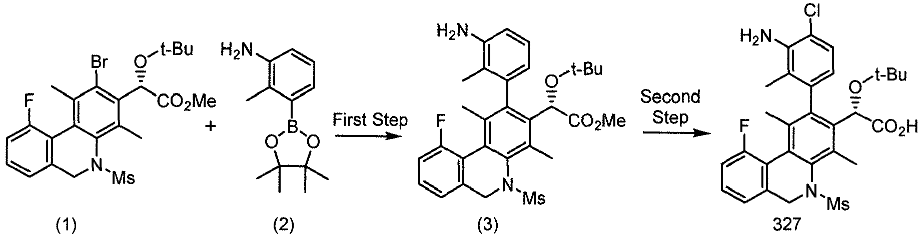

### First Step Synthesis of Compound (3)

Compound (1) (300 mg, 0.568 mmol) was dissolved in a solution of DMF (3.0 mL) and water (0.3 mL), and Compound (2) (199 mg, 0.85 mmol), [1,1'-bis(di-tert-butylphosphino)ferrocene]palladium dichloride (74 mg, 0.114 mmol) and potassium carbonate (314 mg, 2.27 mmol) were added thereto. The mixture was stirred under a nitrogen atmosphere at 120°C for 15 mimnutes. Water (30 mL) and ethyl acetate (30 mL) were added thereto and the mixture was extracted with ethyl acetate. The obtained organic layer was washed with water (30 mL) and brine (30 mL), dried over anhydrous magnesium sulfate, and concentrated. The residue was purified by silica gel column chromatography (hexane-ethyl acetate) to obtain Compound (3) (196 mg, 62%) as a white foam.
MS: m/z = 555 [M+H]+

### Second Step Synthesis of Compound 327

Compound (3) (50 mg, 0.090 mmol) was dissolved in DMF (0.5 mL), and NCS (14.4 mg, 0.108 mmol) was added thereto. The mixture was stirred at 60°C for 5.5 hours. Water (20 mL) and ethyl acetate (20 mL) were added thereto and the mixture was extracted with ethyl acetate. The obtained organic layer was washed with water (20 mL) and brine (20 mL), dried over anhydrous magnesium sulfate, and concentrated. The residue was was dissolved in ethanol (0.5 mL), and 2mol/L aqueous solution of sodium hydroxide (0.2 mL, 0.4 mmol) was added thereto. The mixture was stirred under heat reflux for 6 hours. 2mol/L aqueous solution of hydrochloric acid (0.2 mL) and brine (20 mL) were added thereto, and the mixture was extracted with ethyl acetate (20 mL). The obtained organic layer was dried over anhydrous magnesium sulfate, and concentrated. The residue was purified by silica gel column chromatography (hexane-ethyl acetate) to obtain Compound 327 (10mg, 19%) as a white foam.
MS: m/z = 573 [M-H]-

### Example 52

The following compounds were synthesized according to the above examples.

**[Table 60]**

| | | | | | |
|---|---|---|---|---|---|
| 329 | 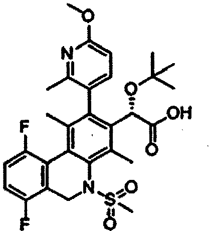 | 2.34 | | 575 | |
| 330 | 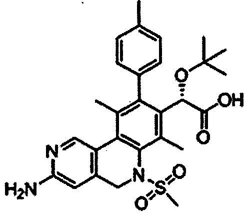 | 1.44 | * | 524.2 | |
| 331 | 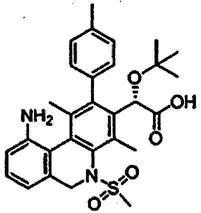 | 2.31 and 2.53 | * | 523.2 | |
| 332 | 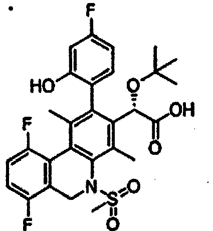 | 2.23 | | 562 | [M-H]- |
| 333 | 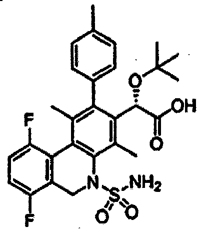 | 2.42 | * | 567.1 | [M+Na]+ |

**[Table 61]**

| | | | | | |
|---|---|---|---|---|---|
| 334 | 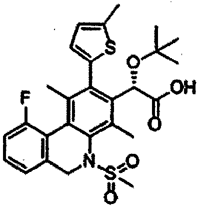 | 2.66 | | 550 | |
| 335 | 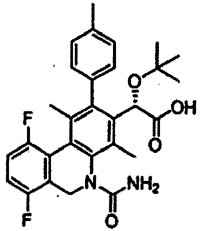 | 2.37 | * | 531.2 | [M+Na]+ |
| 336 | 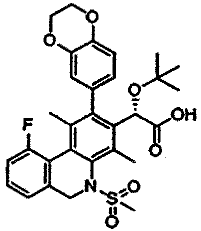 | 2.39 and 2.49 | | 568 | [M-H]- |
| 337 | 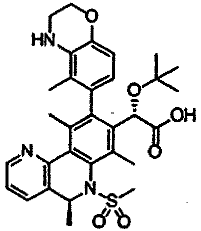 | 2.17 | | 580.3 | |
| 338 | 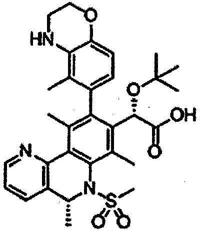 | 2.30 | | 580.4 | |

**[Table 62]**

| | | | | | |
|---|---|---|---|---|---|
| 339 | 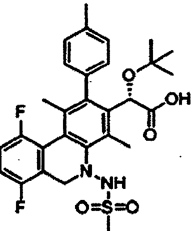 | 2.53 | * | 559.1 | |
| 340 | 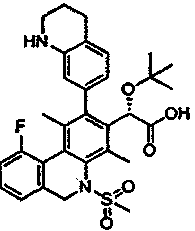 | 2.17 and 2.39 | | 567 | |
| 341 | 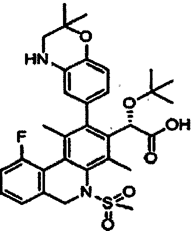 | 2.37 and 2.57 | * | 523 | [M-OtBu]+ |
| 342 | 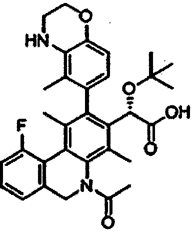 | 2.40 | | 547.4 | |
| 343 | 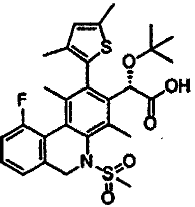 | 2.68 | | 546 | |

**[Table 63]**

| | | | | | |
|---|---|---|---|---|---|
| 344 | 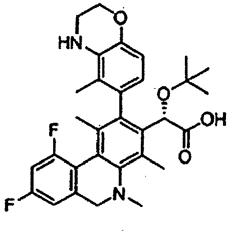 | 2.84 | | 537.4 | |
| 345 | 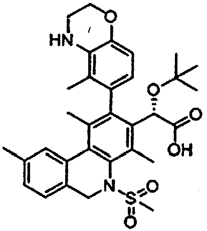 | 2.60 | | 579.3 | |
| 346 | 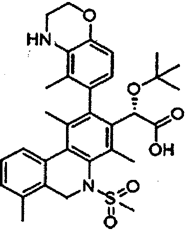 | 2.60 | | 579.4 | |
| 347 | 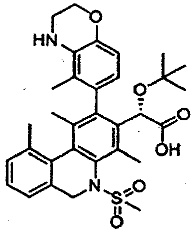 | 2.47 and 2.50 | | 579.4 | |
| 348 | 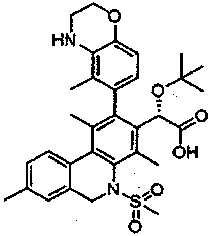 | 2.53 | | 579.5 | |

**[Table 64]**

| | | | | | |
|---|---|---|---|---|---|
| 349* | 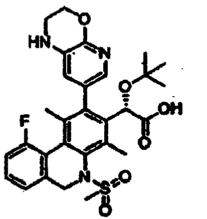 | 1.73 and 1.78 | | 570 | |
| 350 | 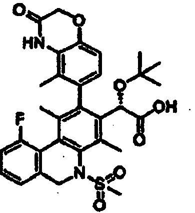 | 221 | | 595.2 | [M-H]- |
| 351 | 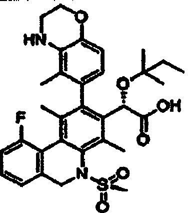 | 2.47 | * | 509 | [M-OBu]+ |
| 352 | 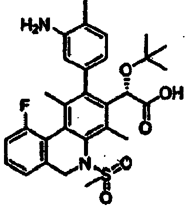 | 2.00 and 223 | | 541 | |
| 353 | 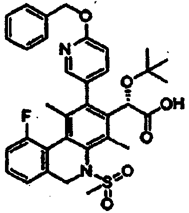 | 2.78 | | 619 | |

| | | | | | |
|---|---|---|---|---|---|
| *reference example | | | | | |

**[Table 65]**

| | | | | | |
|---|---|---|---|---|---|
| 354 | 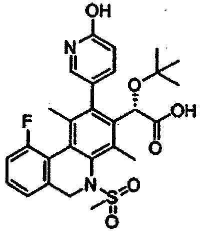 | 1.67 | | 529 | |
| 355 | 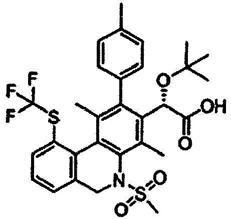 | 2.84 | * | 630.1 | [M+Na]+ |
| 356 | 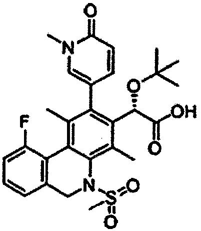 | 1.71 | | 543 | |
| 357 | 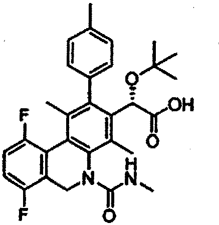 | 2.47 | * | 523.2 | |
| 358 | 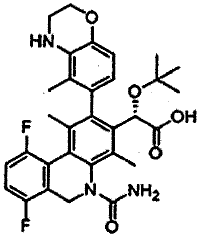 | 2.20 | | 566.3 | |

**[Table 66]**

| | | | | | |
|---|---|---|---|---|---|
| 359 | 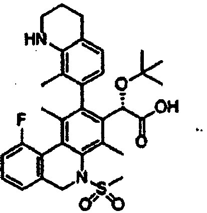 | 2.42 | * | 581.3 | |
| 360 | 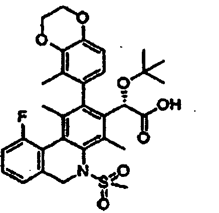 | 2.48 | * | 606 | [M+Na]+ |
| 361 | 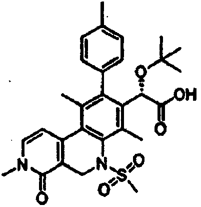 | 2.03 | * | 539.2 | |
| 362 | 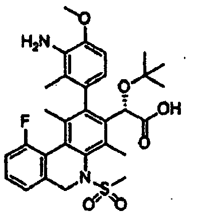 | 2.22 | | 571.3 | |
| 363* | 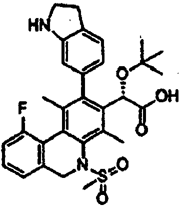 | 1.10 and 1.26 | | 553 | |

| | | | | | |
|---|---|---|---|---|---|
| *reference example | | | | | |

**[Table 67]**

| | | | | | |
|---|---|---|---|---|---|
| 364 | 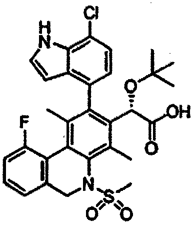 | 2.57 | | 583.2 | [M-H]- |
| 365 | 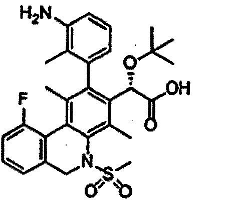 | 2.12 | | 539.3 | [M-H]- |
| 366 | 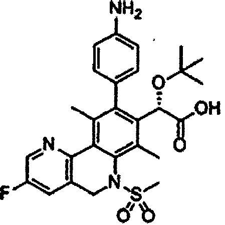 | 1.72 | | 528.3 | |
| 367 | 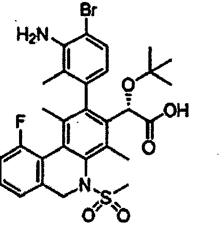 | 2.68 | | 617.2 | [M-H]- |
| 368 | 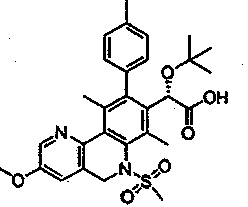 | 2.47 | * | 539.2 | |

**[Table 68]**

| | | | | | |
|---|---|---|---|---|---|
| 369 | 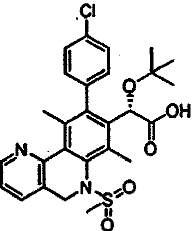 | 2.53 | | 529 | |
| 370 | 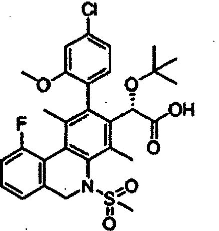 | 2.70 | | 530.2 | [M-H]- |
| 371 | 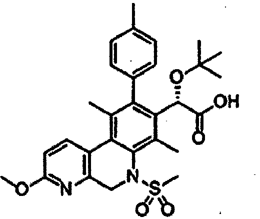 | 2.66 | | 539.3 | |
| 372 | 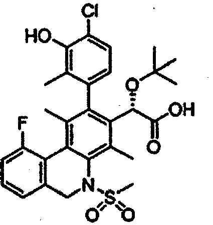 | 2.56 | * | 574.2 | [M-H]- |
| 373 | 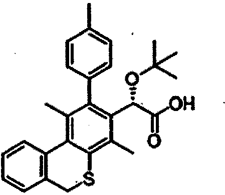 | 2.92 | * | 469 | [M+Na]+ |

**[Table 69]**

| | | | | | |
|---|---|---|---|---|---|
| 374 | 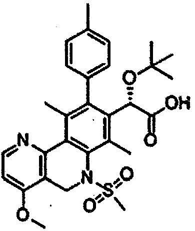 | 2.13 | * | 539.2 | |
| 375 | 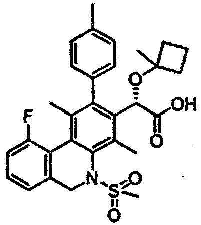 | 2.65 | * | 560 | [M+Na]+ |
| 376 | 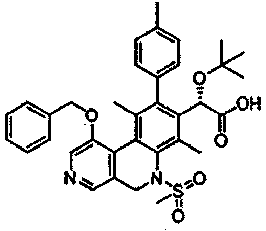 | 2.33 | | 615 | |
| 377 | 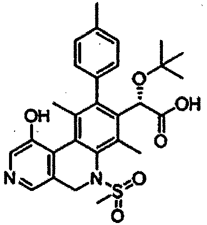 | 1.62 | | 525 | |
| 378 | 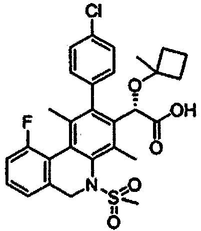 | 2.65 | * | 580 | [M+Na]+ |

**[Table 70]**

| | | | | | |
|---|---|---|---|---|---|
| 379 | 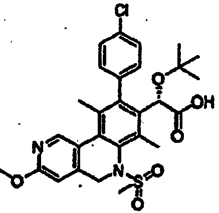 | 2.63 | | 559 | |
| 380 | 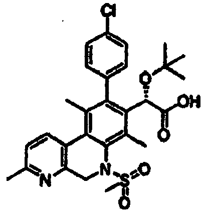 | 2.38 | | 543 | |
| 381 | 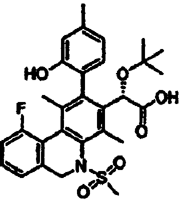 | 2.16 | * | 542.3 | |
| 382 | 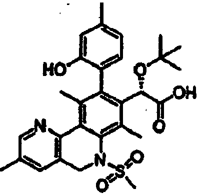 | 2.05 | * | 539.2 | |
| 383* | 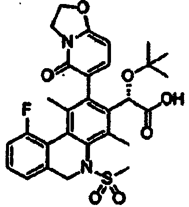 | 1.94 | | 571.3 | |

| | | | | | |
|---|---|---|---|---|---|
| *reference example | | | | | |

**[Table 71]**

| | | | | | |
|---|---|---|---|---|---|
| 384 | 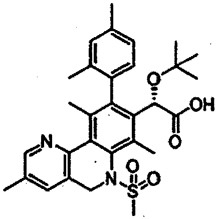 | 2.62 | * | 537 | |
| 385 | 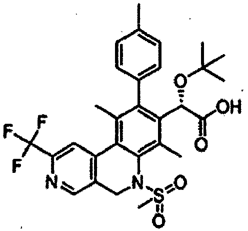 | 2.69 | | 577 | |
| 386 | 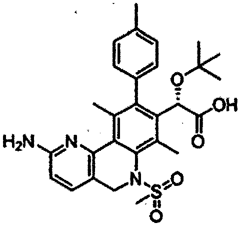 | 1.88 | | 524 | |
| 387 | 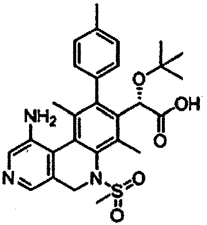 | 1.66 | | 524 | |
| 388 | 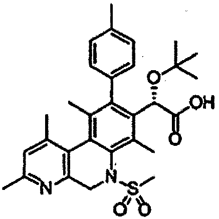 | 2.95 and 2.07 | | 537.3 | |

**[Table 72]**

| | | | | | |
|---|---|---|---|---|---|
| 389 | 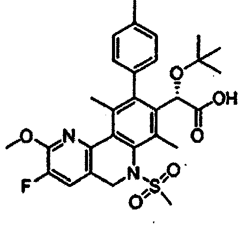 | 2.61 | * | 557.2 | |
| 390 | 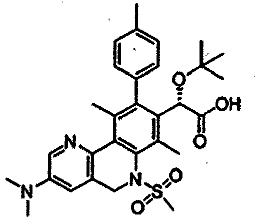 | 2.36 | * | 522.2 | |
| 391 | 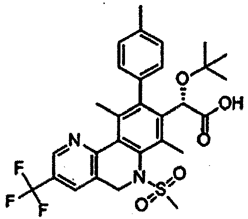 | 2.71 | * | 577.2 | |
| 392 | 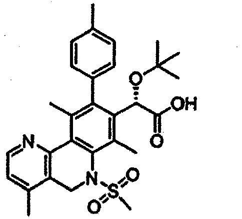 | 2.40 | * | 523.2 | |
| 393 | 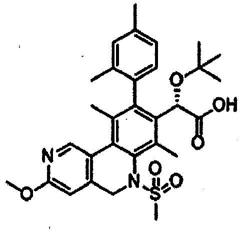 | 2.55 | * | 553 | |

**[Table 73]**

| | | | | | |
|---|---|---|---|---|---|
| 394 | 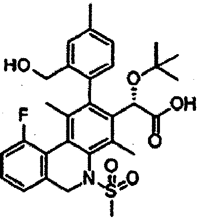 | 2.10 | * | 578 | [M+Na]+ |
| 395 | 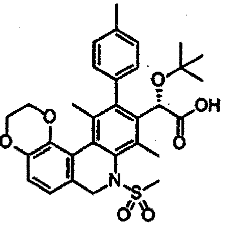 | 2.45 | | 564.4 | [M-H]- |
| 396 | 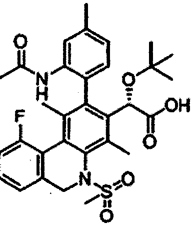 | 2.41 | | 583 | |
| 397 | 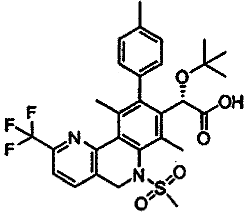 | 2.68 | * | 577.2 | |
| 398 | 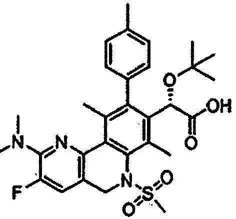 | 2.78 | * | 570.2 | |

**[Table 74]**

| | | | | | |
|---|---|---|---|---|---|
| 399 | 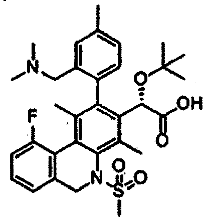 | 1.67 | * | 583 | |
| 400 | 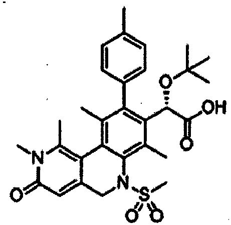 | 1.96 and 2.02 | | 553.3 | |
| 401 | 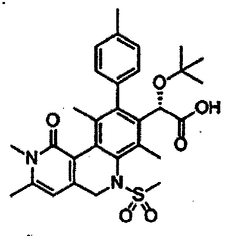 | 2.04 | | 553.3 | |
| 402 | 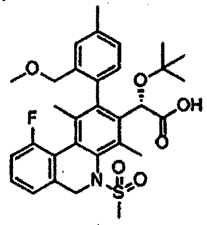 | 2.54 and 2.60 | * | 592 | [M+Na]+ |

### (Synthesis of the above Compound 401)

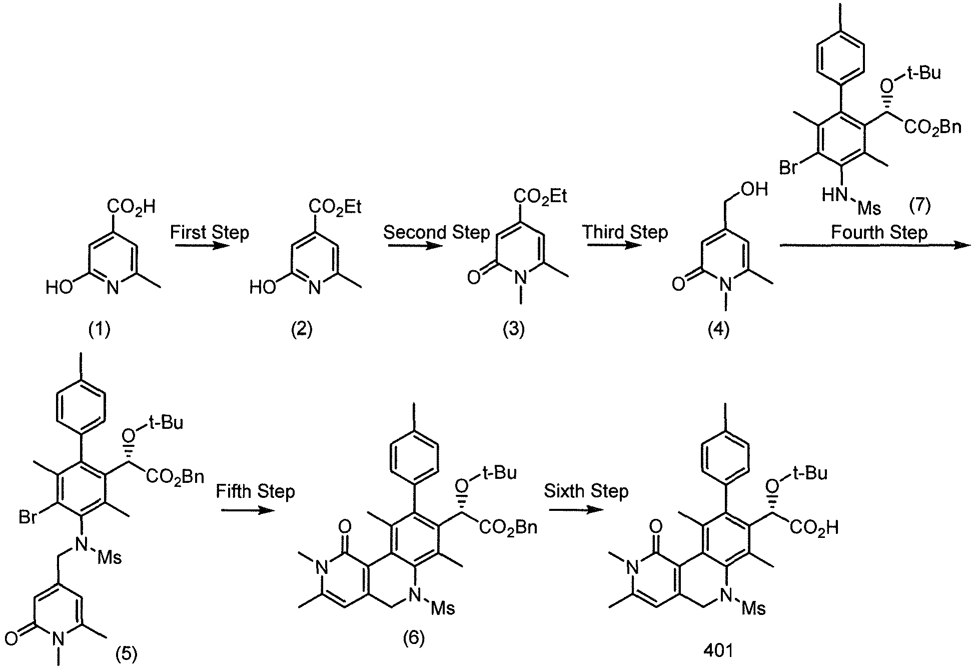

### First Step Synthesis of Compound (2)

Compound (1) (2.00 g, 13.1 mmol) was dissolved in a solution of ethanol (20 mL) and conc sulfuric acid (2mL), and the mixture was stirred under heat reflux for 2 hours and then concentrated. The residue was poured into a saturated aqueous solution of sodium hydrogen carbonate and extracted with chloroform. The obtained organic layer was dried over anhydrous magnesium sulfate, and concentrated. The residue was solidified with hexane to obtain Compound (2) (2.12g, 90%) as a colorless solid.
¹H-NMR (CDCl₃) δ: 1.38 (t, J = 7.1 Hz, 3H), 2.41 (s, 3H), 4.36 (q, J = 7.2 Hz, 2H), 6.59 (s, 1H), 7.06 (s, 1H), 13.24 (s, 1H).
MS: m/z = 182.0 [M+H]+

### Second Step Synthesis of Compound (3)

Compound (2) (1.00 g, 5.52 mmol) was dissolved in DMF (20 mL), and potassium carbonate (1.53 g, 11.0 mmol) was added thereto at room temperature. The mixture was stirred at same temperature for 5 mimnutes. Methyl iodide (10.1 mL, 85.0 mmol) was added thereto, and the mixture was stirred for 18 hours. Water (100 mL) was added thereto and the mixture was extracted with chloroform(100 mL, 50 mL×2). The obtained organic layer was dried over anhydrous magnesium sulfate and concentrated. The residue was purified by silica gel column chromatography (hexane-ethyl acetate) to obtain Compound (3) (982 mg, 91%) as a yellow solid.
¹H-NMR (CDCl₃) δ: 1.37 (t, J = 7.1 Hz, 3H), 2.41 (s, 3H), 3.55 (s, 3H), 4.35 (q, J = 7.1 Hz, 2H), 6.55 (s, 1H), 7.09 (s, 1H).
MS: m/z = 196.1 [M+H]+

### Third Step Synthesis of Compound (4)

Compound (3) (880 mg, 5.07 mmol) was dissolved in THF (19.8 mL), lithium aluminum hydride (385 mg, 10.1 mmol) was added thereto under ice bath. The mixture was stirred under ice bath for 1 hour. Sodium sulfate decahydrate was added thereto under ice bath. The mixture was filtered through celite, and the filtrate was concentrated. The residue was purified by silica gel column chromatography (chloroform-methanol) to obtain Compound (4) (281 mg, 36%) as a colorless solid.
¹H-NMR (CDCl₃) δ: 2.34 (s, 3H), 3.02 (t, J = 5.6 Hz, 1H), 3.50 (s, 3H), 4.48 (d, J = 5.6 Hz, 2H), 6.06 (s, 1H), 6.44 (s, 1H).
MS: m/z = 157.0 [M+H]+

### Fourth Step Synthesis of Compound (5)

Compound (4) (52.1 mg, 0.340 mmol), Compound (7) (200 mg, 0.340 mmol) and triphenylphosphine (107 mg, 0.406 mmol) were dissolved in THF (2 mL), and di(2-methoxyethyl) azodicarboxylate (96.0 mg, 0.408 mmol) was added thereto at room temperature. The mixture was stirred at same temperature for 18 hours. Water (30 mL) was added thereto and the mixture was extracted with ethyl acetate (50 mL). The obtained organic layer was washed with brine (30 mL), dried over anhydrous magnesium sulfate, and concentrated. The residue was purified by silica gel column chromatography (hexane-ethyl acetate) to obtain Compound (5) (102.7 mg, 42 %) as a colorless foam.
MS: m/z = 723.3,725.3 [M+H]+

### Fifth Step Synthesis of Compound (6)

Compound (5) (100 mg, 0.141 mmol) were dissolved in DMA (1.45 mL), and tricyclohexylphosphonium tetrafluoroborate (10.4 mg, 0.028 mmol), potassium carbonate (39.0 mg, 0.282 mmol) and palladium acetate (3.17 mg, 0.014 mmol) were added thereto at room temperature. The mixture was stirred under a nitrogen atmosphere at 130°C for 45 mimnutes. Water (30 mL) was added thereto and the mixture was extracted with chloroform (30 mLx3). The obtained organic layer was dried over anhydrous magnesium sulfate and concentrated. The residue was purified by silica gel column chromatography (chloroform-methanol) to obtain Compound (6) (7.6 mg, 8%) as a brown oil.
MS: m/z = 643.4 [M+H]+

### Sixth Step Synthesis of Compound 401

Compound (6) (7.5 mg) was dissolved in methanol (2 mL), and 10% palladium hydroxide (4.92 mg, 0.004 mmol) was added thereto at room temperature. The mixture was stirred under a hydrogen atmosphere at same temperature for 30 mimnutes. The mixture was filtered through celite, and; the filtrate was concentrated. The residue was purified by silica gel column chromatography (chloroformmethanol) to obtain Compound 401 (5.6 mg, 87%) as a colorless solid.
MS: m/z = 553.3 [M+H]+

### Example 53 (reference example)

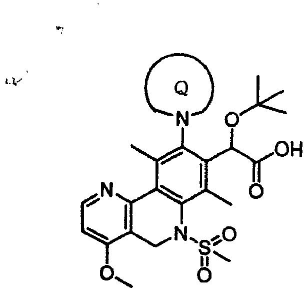

wherein examples of cyclic amine represented by ring Q:

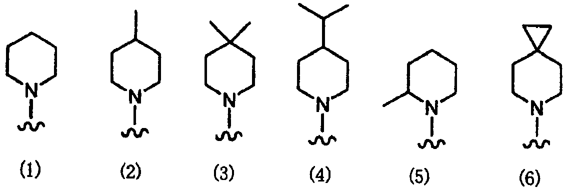

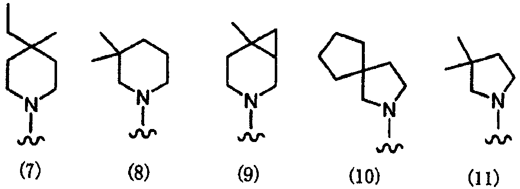

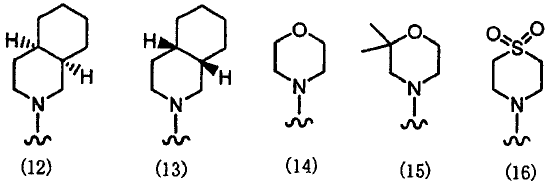

### Example 54 (reference example)

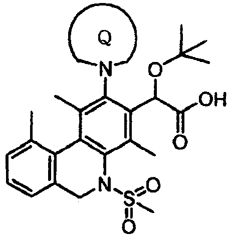

wherein examples of cyclic amine represented by ring Q:

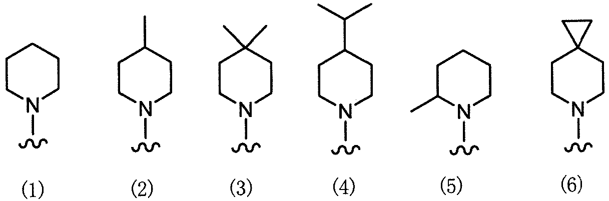

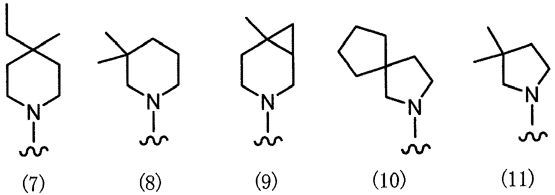

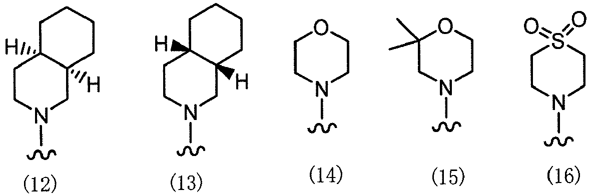

### Example 55

The following compounds were synthesized according to the above examples.

**[Table 75]**

| **No.** | **Structure** | RT (min) | | MS[M+H]+ | |
|---|---|---|---|---|---|
| 403 | 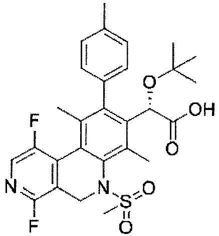 | 2.54 | | 545 | |
| 404 | 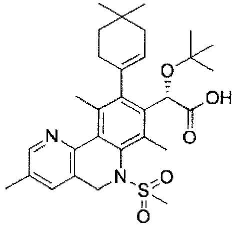 | 2.80 | * | 541 | |
| 405 | 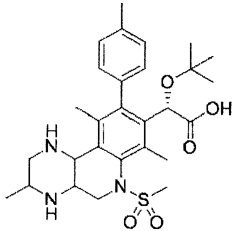 | 1.51 and 1.60 | | 530.4 | |
| 406 | 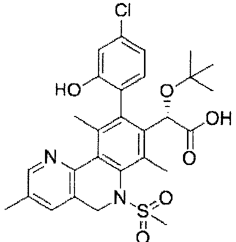 | 2.20 | | 559.2 | |
| 407 | 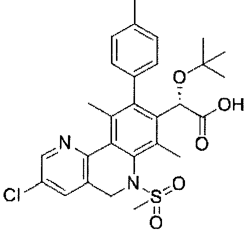 | 2.80 | | 543 | |

**[Table 76]**

| | | | | | |
|---|---|---|---|---|---|
| 408 | 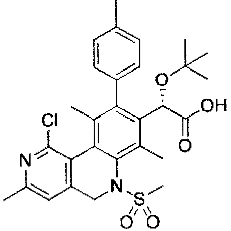 | 2.49 and 2.57 | | 557.3 | |
| 409 | 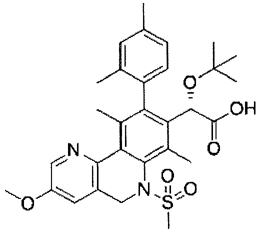 | 2.56 | * | 553 | |
| 410 | 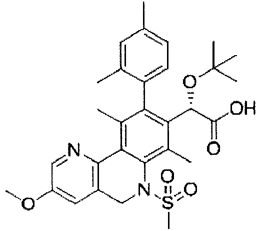 | 2.56 | * | 553 | |
| 411 | 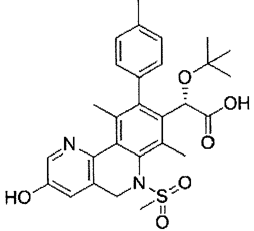 | 2.17 | | 525 | |
| 412 | 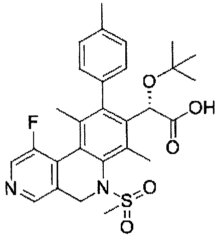 | 2.33 | | 527 | |

**[Table 77]**

| | | | | | |
|---|---|---|---|---|---|
| 413 | 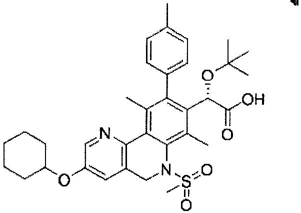 | 3.19 | | 607 | |
| 414 | 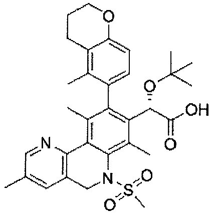 | 2.48 | | 579 | |
| 415 | 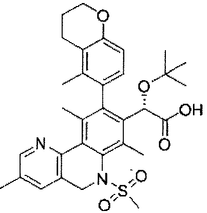 | 2.43 | | 579 | |
| 416 | 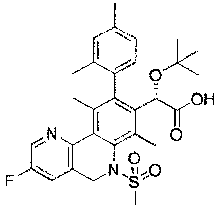 | 2.60 | * | 541 | |
| 417 | 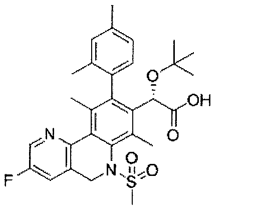 | 2.60 | * | 541 | |
| 418 | 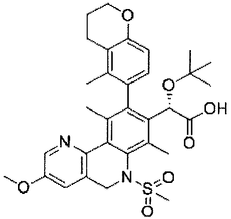 | 2.46 | * | 595 | |

**[Table 78]**

| | | | | | |
|---|---|---|---|---|---|
| 419 | 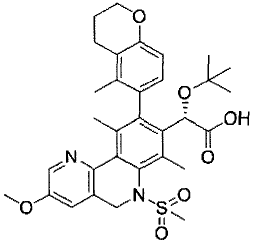 | 2.40 | * | 595 | |
| 420 | 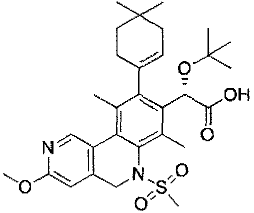 | 2.70 | | 557 | |
| 421 | 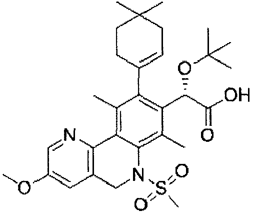 | 2.75 | * | 557 | |

### Experimental Example 1: HIV replication inhibition Assay

The biology assay of the compound of the present invention is described below.

### Experimental Example 1: HIV Replication Inhibition Assay

HIV (HTLV-III B strain) persistent infected human T cell strain Molt-4 clone 8 was cultured in 10% Fetal Bovine Serum-containing RPMI-1640 medium and the supernatant was filtrated, then the virus titer was measured and the solution was stored at -80°C. Each anti-human immunodeficiency virus active substance was diluted with the above cultured medium to the designated concentration, which was dispensed into 96 well micro plate by 50µL. Next, a MT-4 cell suspended liquid was dispensed by 100µL (2.5 × 10⁴cells), then the above HIV-containing supernatant diluted with the above cultured medium was added thereto by 50 µL (60 pfu (plaque forming unit)).

The obtained mixture was cultured at 37°C in CO₂ incubator for four days, then 3-(4, 5-dimehylthiazole-2-yl)-2, 5-diphenyltetrazolynium bromide (MTT) 5mg/mL in PBS was added to each well by 30µL, followed by 1hr -cultivation. In this step, as formazan was precipitated by reduction of MTT in living cells, the cell supernatant was removed from all well by 150µL, then a 150 µL of solution (10% Triton X-100 and 0.4% (v/v)-containing isopropanol) was added thereto, followed by shaking with a plate mixer, to elute formazan. The formazan was measured with a microreader at OD: 560nm and 690nm (wavelength) and the result was compared with the reference. EC50 means the compound concentration at which cell cytotoxicity by virus is inhibited 50%.

The result is shown below.

**[Table A-1]**

| Example No. | No. | EC50 (nM) |
|---|---|---|
| 1 | 3 | 64 |
| 2 | 7 | 9.6 |
| 9 | 8 | 62 |
| 11 | 10 | 2.4 |
| 36 | 17 | 59 |
| 4 | 20 | 13 |
| 26 | 42 | 10 |
| 36 | 48 | 29 |
| 19 | 49 | 12 |
| 30 | 53 | 5.5 |
| 31 | 71 | 16 |
| 33 | 79 | 15 |

**[Table A-2]**

| Example No. | No. | EC50_(nM) |
|---|---|---|
| 41 | 88 | 21 |
| 41 | 89 | 9.3 |
| 41 | 91 | 4.2 |
| 41 | 95 | 9.8 |
| 41 | 96 | 32 |
| 41 | 98 | 22 |
| 41 | 99 | 97 |
| 41 | 100 | 10 |
| 41 | 101 | 17 |
| 41 | 104 | 2.1 |
| 41 | 105 | 38 |
| 41 | 108 | 12 |
| 41 | 109 | 9.1 |
| 41 | 110 | 17 |
| 41 | 111 | 7.8 |
| 41 | 112 | 8.5 |
| 41 | 113 | 8.6 |
| 41 | 115 | 6 |
| 41 | 116 | 2.9 |
| 41 | 117 | 19 |
| 41 | 118 | 4.4 |
| 41 | 119 | 43 |
| 41 | 120 | 5.1 |
| 41 | 122 | 3.8 |
| 41 | 123 | 89 |
| 41 | 124 | 8.2 |
| 41 | 125 | 39 |
| 41 | 127 | 11 |
| 41 | 128 | 10 |
| 41 | 129 | 7.7 |
| 41 | 130 | 12 |
| 41 | 131 | 87 |
| 41 | 132 | 22 |
| 41 | 133 | 4.8 |
| 41 | 134 | 62 |

**[Table A-3]**

| Example No. | No. | EC50_(nM) |
|---|---|---|
| 41 | 137 | 46 |
| 41 | 140 | 3.1 |
| 41 | 141 | 3.5 |
| 41 | 142 | 4.4 |
| 41 | 145 | 39 |
| 41 | 148 | 8.8 |
| 41 | 149 | 23 |
| 41 | 150 | 22 |
| 41 | 152 | 16 |
| 41 | 153 | 47 |
| 41 | 155 | 39 |
| 41 | 156 | 23 |
| 41 | 157 | 4.3 |
| 41 | 159 | 5.3 |
| 41 | 161 | 21 |
| 41 | 162 | 13 |
| 41 | 163 | 15 |
| 41 | 164 | 4.1 |
| 41 | 166 | 1.7 |
| 41 | 167 | 8.5 |
| 41 | 168 | 24 |
| 41 | 169 | 9.6 |
| 41 | 171 | 21 |
| 41 | 172 | 42 |
| 41 | 173 | 9.4 |
| 41 | 174 | 1.5 |
| 41 | 175 | 2.9 |
| 41 | 177 | 34 |
| 41 | 183 | 21 |
| 41 | 184 | 53 |

**[Table A-4]**

| Example No. | No. | EC50 (nM) |
|---|---|---|
| 48 | 208 | 15 |
| 48 | 210 | 38 |
| 48 | 212 | 1.9 |
| 42 | 213 | 4.9 |
| 45 | 214 | 4.9 |
| 48 | 216 | 46 |
| 48 | 217 | 41 |
| 48 | 218 | 6.4 |
| 48 | 220 | 7.3 |
| 48 | 221 | 90 |
| 48 | 223 | 34 |
| 43 | 224 | 2.5 |
| 48 | 226 | 58 |
| 48 | 228 | 19 |
| 48 | 230 | 94 |
| 48 | 231 | 78 |
| 48 | 232 | 12 |
| 48 | 233 | 38 |
| 48 | 235 | 5.4 |
| 48 | 237 | 12 |
| 48 | 238 | 31 |
| 48 | 239 | 81 |
| 48 | 245 | 15 |
| 48 | 246 | 2.3 |

**[Table A-5]**

| Example No. | No. | EC50 (nM) |
|---|---|---|
| 48 | 248 | 11 |
| 48 | 249 | 5.8 |
| 48 | 251 | 2.1 |
| 48 | 254 | 3.1 |
| 48 | 258 | 3.9 |
| 48 | 259 | 27 |
| 48 | 260 | 14 |
| 48 | 262 | 25 |
| 48 | 263 | 12 |
| 48 | 264 | 19 |
| 48 | 265 | 11 |
| 48 | 266 | 7.9 |
| 48 | 273 | 4.5 |
| 48 | 275 | 15 |
| 48 | 278 | 3.7 |
| 48 | 279 | 11 |
| 48 | 282 | 8.8 |
| 48 | 284 | 16 |
| 48 | 285 | 55 |
| 48 | 286 | 3.2 |
| 48 | 287 | 33 |
| 47 | 288 | 37 |
| 48 | 290 | 53 |
| 48 | 293 | 3.3 |
| 48 | 296 | < 7.8 |

**[Table A-6]**

| Example No. | No. | EC50 (nM) |
|---|---|---|
| 48 | 298 | 30 |
| 48 | 299 | 26 |
| 48 | 300 | < 7.8 |
| 48 | 301 | 4.9 |
| 48 | 302 | 28.1 |
| 48 | 304 | 5.2 |
| 46 | 305 | 28 |
| 48 | 307 | 51 |
| 48 | 310 | 25 |
| 48 | 311 | 28 |
| 48 | 314 | 5.3 |
| 48 | 315 | < 7.8 |
| 48 | 317 | 70 |
| 48 | 318 | 17 |
| 48 | 319 | 8.4 |
| 48 | 320 | 3.1 |
| 48 | 321 | 15 |
| 48 | 322 | 11 |

**[Table A-7]**

| Example No. | No. | EC50_(nM) |
|---|---|---|
| 49 | 324 | 8.1 |
| 50 | 325 | 11 |
| 50 | 326 | 54 |
| 51 | 327 | 7.2 |
| 52 | 329 | 39 |
| 52 | 330 | 53 |
| 52 | 332 | 24 |
| 52 | 333 | 19 |
| 52 | 334 | 22 |
| 52 | 335 | 30 |
| 52 | 336 | 22 |
| 52 | 337 | 26 |
| 52 | 340 | 4.3 |
| 52 | 342 | 9 |
| 52 | 343 | 16 |
| 52 | 344 | 47 |
| 52 | 345 | 6.8 |
| 52 | 346 | 4.8 |
| 52 | 347 | 22 |
| 52 | 348 | 2 |
| 52 | 350 | 96 |
| 52 | 351 | 4.5 |
| 52 | 352 | 10 |
| 52 | 358 | 91 |
| 52 | 359 | 5.4 |
| 52 | 360 | 4.4 |

**[Table A-8]**

| | | |
|---|---|---|
| 52 | 363 | 14 |
| 52 | 364 | 16 |
| 52 | 367 | 7.6 |
| 52 | 368 | 3.9 |
| 52 | 369 | 44 |
| 52 | 371 | 4.6 |
| 52 | 372 | 81 |
| 52 | 374 | 63 |
| 52 | 375 | 19 |
| 52 | 378 | 36 |
| 52 | 379 | 2.3 |
| 52 | 380 | 13 |
| 52 | 381 | 5 |
| 52 | 382 | 49 |
| 52 | 384 | 8.4 |
| 52 | 385 | 39 |
| 52 | 386 | 86 |
| 52 | 388 | 29 |
| 52 | 389 | 67 |
| 52 | 390 | 2.2 |
| 52 | 391 | 4.1 |
| 52 | 392 | 31 |
| 52 | 393 | 4.7 |
| 52 | 394 | 27 |
| 52 | 395 | 34 |
| 52 | 397 | 33 |
| 52 | 398 | 80 |

**[Table A-9]**

| Example No. | No. | **EC50 (nM)** |
|---|---|---|
| **55** | **403** | 14.6 |
| **55** | **404** | <7.8 |
| **55** | **406** | 38.0 |
| **55** | **407** | <7.8 |

### Experimental Example 2: CYP Inhibition Assay

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmοl/L ethoxyresorufin (CYP1A2), 100 µmol/L tolbutamide (CYP2C9), 50 µmol/L S-mephenytoin (CYP2C19), 5 µmol/L dextromethorphan (CYP2D6), 1 µmοl/L terfenedine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, and a test drug in 50 mmol/L Hepes buffer as a reaction solution was added to a 96-well plate as the composition as described above, NADPH,as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tolbutamide hydroxide (CYP2C9 metabolite), mephenytoin 4' hydroxide (CYP2C19 metabolite), dextrorphan (CYP2D6 metabolite), and terfenadine alcohol (CYP3A4 metabolite) were quantified by LC/MS/MS.

A reaction system containing only DMSO which is a solvent for dissolving a drug was adopted as a control (100%), and the remaining activity (%) was calculated, then IC₅₀ was calculated by reverse presumption with a logistic model using a concentration and an inhibition rate.

As a result, all IC₅₀ of Compound 10 for the activity of CYP1A2, CYP2C19, CYP2D6, CYP3A4 was 20 µM or more

### Experimental Example 3: CYP3A4 fluorescent MBI test

The CYP3A4 fluorescent MBI test is to investigate the enhancement of CYP3A4 inhibition of a compound by a metabolism reaction. 7-Benzyloxytrifluoromethylcoumarin (7-BFC) is debenzylated by the CYP3A4 enzyme to produce 7-hydroxytrifluoromethylcoumarin (HFC), a metabolite emitting fluorescent light. The test was performed using 7-HFC-producing reaction as an index,

The reaction conditions were as follows: substrate 5.6 µmol/L 7-BFC; pre-reaction time, 0 or 30 minutes; reaction time, 15 minutes; reaction temperature, 25°C (room temperature); CYP3A4 content (expressed in *Escherichia coli*), at pre-reaction 62.5 pmol/mL, at reaction 6.25 pmol/mL (at 10-fold dilution); test drug concentration, 0.625, 1.25, 2.5, 5, 10, 20 µmοl/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution, and IC₅₀ was calculated by reverse-presumption by a logistic model using a concentration and an inhibition rate. A case where the difference of IC₅₀ values is 5 µM or more was defined as (+) and, a case where the difference is 3 µM or less was defined as (-).

As a result, Compound 10 was (-).

### Experimental Example 4: Metabolism Stability Test

Commercially available pooled human hepatic microsomes and a test compound were reacted for a constant time, then a remaining rate was calculated by comparing a reacted sample and an unreacted sample, thereby a degree of metabolism of the test compound in liver was assessed.

A reaction was performed (oxidative reaction) at 37°C for 0 minute or 30 minutes in the presence of 1 mmol/L NADPH in 0.2 mL of a buffer (50 mmol/L Tris-HCl, pH 7.4, 150 mmol/L potassium chloride, 10 mmol/L magnesium chloride) containing 0.5 mg protein/mL of human liver microsomes . After the reaction, 50 µL of the reaction solution was added to 100 µL of a methanol/acetonitrile = 1/1 (v/v), mixed and centrifuged at 3000 rpm for 15 minutes. The test compound in the supernatant was quantified by LC/MS/MS, and a remaining amount of the test compound after the reaction was calculated, letting a compound amount at 0 minute reaction time to be 100%. Hydrolysis reaction was performed in the absence of NADPH and glucuronidation reaction was in the presence of 5 mM UDP-glucuronic acid in place of NADPH, followed by similar operations.

As a result, the amount of Compound 10 in liver microsomes of human and rat was 75% and 91%, respectively.

### Experimental Example 5: Solubility Test

The solubility of each compound was determined under 1% DMSO addition conditions. A 10 mmol/L solution of the compound was prepared with DMSO, and 6 pL of the solution was added to 594µL of an artificial intestinal juice (:water and a 118 mL solution of 0.2 mol/L NaOH reagent are added to 250 mL of 0.2 mol/L potassium dihydrogen phosphate reagent to reach 1000 mL) with a pH of 6.8. The mixture is left standing for 16 hours at 25°C, and the mixture is vacuum-filtered. The filtrate is two-fold diluted with methanol/water = 1/1(v/v), and the compound concentration in the filtrate is measured with HPLC or LC/MS/MS by the absolute calibration method.

As a result, the solubility of Compound 10 in the artificial intestinal juice was 50µM or more.

### Experimental Example 6: Fluctuation Ames Test

The mutagenicity of the compound of the present invention was assaied.

20 µL of freezing-stored rat typhoid bacillus (Salmonella typhimurium TA98 strain, TA100 strain) was inoculated on 10 mL of a liquid nutrient medium (2.5% Oxoid nutrient broth No.2), and this was cultured before shaking at 37°C for 10 hours. 9 mL of a bacterial solution of the TA98 strain was centrifuged (2000 × g, 10 minutes) to remove a culturing solution. The bacteria was suspended in F buffer (K₂HPO₄: 3.5 g/L, KH₂PO₄: 1 g/L, (NH₄)₂SO₄: 1 g/L, trisodium citrate dehydrate: 0.25 g/L, MgSO₄ • 7H₂O: 0.1 g/L), and the suspension was added to 110 mL of an Exposure medium (Micro F buffer containing Biotin: 8 µg/mL, histidine: 0.2 µg/mL, glucose: 8 mg/mL). The TA100 strain was added to 120 mL of the Exposure medium per 3.16 mL of the bacterial solution to prepare a test bacterial solution. Each 12 µL of a test substance DMSO solution (several stage dilution from maximum dose 50 mg/mL at 2-to 3-fold ratio), DMSO as a negative control, 50 µg/mL of 4-nitroquinoline-1-oxide DMSO solution for the TA98 strain, 0.25 µg/mL of 2-(2-furyl)-3-(5-nitro-2-furyl)acrylamide DMSO solution for the TA100 strain each under the non-metabolism activating condition, 40 µg/mL of 2-aminoanthracene DMSO solution for the TA98 strain, 20 µg/mL of 2-aminoanthracene DMSO solution for the TA100 strain each under the metabolism activating condition all as a positive control, and 588 µL of the test bacterial solution (a mixed solution of 498 µl of the test bacterial solution and 90 µL of S9 mix under the metabolism activating condition) were mixed, and this was shaking-cultured at 37°C for 90 minutes. 460 µL of the bacterial solution exposed to the test substance was mixed with 2300 µL of an Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into microplate 48 wells/dose, and this was subjected to stationary culturing at 37°C for 3 days. Since a well containing a bacterium which has obtained the proliferation ability by mutation of an amino acid (histidine) synthesizing enzyme gene turns from purple to yellow due to a pH change, the bacterium proliferation well which has turned to yellow in 48 wells per dose is counted, and was assessed by comparing with a negative control group. (-) means that mutagenicity is negative and (+) is positive.

As a result, the mutagenicity of Compound 10 was negative.

### Experimental Example 7: BA test

Materials and methods for studies on oral absorption
(1) Animal: mouse or SD rats are used.
(2) Breeding conditions: mouse or SD rats are allowed to freely take solid feed and sterilized tap water.
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) is calculated by non-linear least squares program WinNonlin (Registered trademark), and the bioavailability (BA) is calculated from the AUCs of the oral administration group and intravenous administration group

### Experimental Example 8: hERG test

For the purpose of assessing risk of an electrocardiogram QT interval prolongation, effects on delayed rectifier K+ current (I_{Kr}), which plays an important role in the ventricular repolarization process of the compound of the present invention, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds was recorded. After the generated current was stabilized, extracellular solution (NaCl: 135 mmol/L, KCl: 5.4 mmol/L, NaH₂PO₄: 0.3 mmol/L, CaCl₂ . 2H₂O: 1.8 mmol/L, MgCl₂ • 6H₂O: 1 mmol/L, glucose: 10 mmol/L, HEPES (4-(2-hydroxyethyl)-1-piperazineethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using an analysis software (DataXpress ver.1, Molecular Devices Corporation). Further, the inhibition relative to the tail peak current before application of the test substance was calculated, and compared with the vehicle-applied group (0.1% dimethyl sulfoxide solution) to assess influence of the test substance on I_{Kr}.

As a result, the inhibition ratio of Compound 7 was 2.6%.

### Test Example 9: Powder solubility test

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and JP-2 fluid to reach 100 mL). In the case that all amount of the test compound is dissolved after the addition of the test fluid, the test compound is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### Test Example 10: Light hemolysis test

The compound of the present invention is disolved at the target concentration, and is mixed with the red blood cell suspension on a microplate. The mixture is irradiated with fluorescent UV lamps under the condition of 10 J/cm2 in the UVA and UVB. After the iradiation, a supernatant of the mixture is collected and is transfered to a microplate. Absorbance of the supernatants are measured at 540 nm and 630 nm, and phototoxicity of the compound is determined based on the optical density. In the study, two endpoints of 540 nm and 630 nm are indicated as a damage to biological membrane (photohemolysis) and hyperoxidation of lipid membrane (met-hemoglobin formation), respectively.

The formulation examples are shown below.

### Formulation Example 1: Tablet

| | |
|---|---|
| Compound of the present invention | 15 mg |
| Lactose | 15 mg |
| Calcium Stearate | 3 mg |

The above ingredients other than Calcium Stearate are uniformly mixed, crushed, granule, dried to prepare granules of suitable size. After addition of Calcium Stearate, the mixture is compressed to prepare tables.

### Formulation Example 2: Capsules

| | |
|---|---|
| Compound of the present invention | 10 mg |
| Magnecium Stearate | 10 mg |
| Lactose | 80 mg |

The above ingredients are uniformly mixed to prepare powdered medicine as powder or fine particles, which are put into capsule containers to prepare capsules.

### Formulation Example 3: Granules

| | |
|---|---|
| Compound of the present invention | 30 g |
| Lactose | 265 g |
| Magnecium Stearate | 5 g |

The above ingredients are fully mixed, compressed, crushed, selected the size to prepare granules of suitable size.

### INDUSTRIAL APPLICABILITY

The compound of the present invention can be a medicament useful as a therapeutic agent for virus infection disease such as AIDS.

## Claims

1. A compound represented by the following formula: 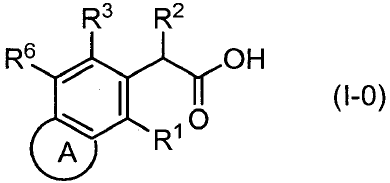 or its pharmaceutically acceptable salt,
wherein
ring A is substituted or unsubstituted heterocycle;
R¹ is alkyl;
or R¹ may be taken together with atom(s) constituting the ring A to form substituted or unsubstituted heterocycle;
R² is alkyloxy, or substituted or unsubstituted cycloalkyloxy;
R³ is selected from the following groups: 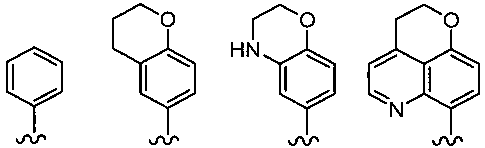 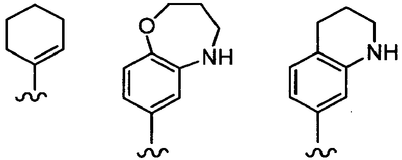 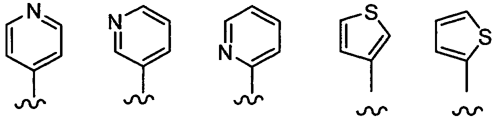 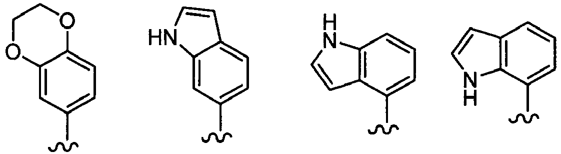 wherein the above groups may be substituted with same or different 1 to 4 substituent consisting of alkyl, alkoxy, halogen, hydroxy, hydroxyalkyl, alkoxyalkyl, haloalkyl, oxo, amino, mono or di alkylamino, aminoalkyl, mono or di alkylaminoalkyl and cyano; and
R⁶ is alkyl.

2. The compound according to claim 1 or its pharmaceutically acceptable salt, wherein ring A is monocyclic aromatic heterocycle or monocyclic non-aromatic heterocycle,
wherein the ring may be fused with another aromatic carbocycle, non-aromatic carbocycle, aromatic heterocycle, or non-aromatic heterocycle; the ring may form a spiro ring together with another aromatic carbocycle, non-aromatic carbocycle, aromatic heterocycle or non-aromatic heterocycle; two atoms constituting ring A which are not adjacent to each other may be cross-linked with alkylene, alkenylene or alkynylene; and/or, rings may be substituted with one or more of R^{A};
wherein R^{A} is each independently, halogen, cyano, nitro, oxo, azide, trimethylsilyl or -XA -R^{A1};
X^{A} is a single bond, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -CO-O-NR^{A2}-, -O-CO-NR^{A2}-, -NR^{A2}-O-CO-, -CO-NR^{A2}-O-, -O-NR^{A2}-CO-, -NR^{A2}-CO-NR^{A3}-, -NR^{A2}-SO₂- or -SO₂-NR^{A2}-;
R^{A1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A} is a single bond, R^{A1} is not a hydrogen atom;
R^{A2} and R^{A3} are each independently a hydrogen atom, substituted or unsubstituted alkyl, substituted or unsubstituted alkenyl, or substituted or unsubstituted alkynyl;
when X^{A} is -NR^{A2}-, -CO-NR^{A2} -, -CO-O-NR^{A2}-, -O-CO-NR^{A2}- or -SO₂-NR^{A2}-, R^{A1} and R^{A2} may be taken together with an adjacent nitrogen atom to form substituted or unsubstituted aromatic heterocyclyl, or substituted or unsubstituted non-aromatic heterocyclyl;
when X^{A} is -NR^{A2}-CO-NR^{A3}-, R^{A1} and R^{A3} may be taken together with an adjacent nitrogen atom to form substituted or unsubstituted aromatic heterocyclyl, or substituted or unsubstituted non-aromatic heterocyclyl;
R¹ and R^{A} may be taken together with an adjacent atom to form aromatic carbocycle, non-aromatic carbocycle, aromatic heterocycle or non-aromatic heterocycle, wherein the ring may be substituted with one or more of the same or different R^{A'} ;
wherein R^{A'} is each independently, halogen, cyano, nitro, oxo, azide, trimethylsilyl or -XA' -R^{A'1};
X^{A'} is a single bond, -O-, -S-, -NR^{A'2}-, =N-, -CO-, -SO₂-, -OCO-, -CO-O-, -NR^{A'2}-CO-, -CO-NR^{A'2}-, -NR^{A'2}-CO-O-, -CO-O-NR^{A'2}-, -O-CO-NR^{A'2}-, -NR^{A'2}-O-CO-, -CO-NR^{A'2}-O-, -O-NR^{A'2}-CO-, -NR^{A'2}-CO-NR^{A'3}-, -NR^{A'2}-SO₂- or -SO₂-NR^{A'2}-;
R^{A'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{A'} is a single bond, R^{A'1} is not a hydrogen atom;
R^{A'2} and R^{A'3} are each independently, a hydrogen atom, substituted or unsubstituted alkyl, substituted or unsubstituted alkenyl, or substituted or unsubstituted alkynyl;
X^{A'} is -NR^{A'2}-, -CO-NR^{A'}2 -, -CO-O-NR^{A'2}-, -O-CO-NR^{A'2}- or -SO₂-NR^{A'2}-;
R^{A'1} and R^{A'2} may be taken together with an adjacent nitrogen atom to form substituted or unsubstituted aromatic heterocyclyl, or substituted or unsubstituted non-aromatic heterocyclyl;
when X^{A'} is -NR^{A'2}-CO-NR^{A'3}-, R^{A'1} and R^{A'3} may be taken together with an adjacent nitrogen atom to form substituted or unsubstituted aromatic heterocyclyl, or substituted or unsubstituted non-aromatic heterocyclyl.

3. The compound according to claim 1 or 2 or its pharmaceutically acceptable salt, wherein ring A is the following ring: 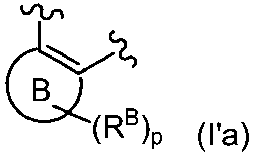 wherein
ring B is monocyclic aromatic heterocycle or monocyclic non-aromatic heterocycle; wherein the ring may form a spiro ring together with another aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene;
R^{B} is each independently, halogen, cyano, nitro, oxo, azide, trimethylsilyl or -X^{B} -R^{B1} ;
X^{B} is a single bond, -O-, -S-, -NR^{B2}-, =N-, -CO-, -SO₂-, -O-CO-, -CO-O-, -NR^{B2}-CO-, -CONR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- or -SO₂-NR^{B2}-;
R^{B1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom;
R^{B2} and R^{B3} are each independently a hydrogen atom, substituted or unsubstituted alkyl, substituted or unsubstituted alkenyl, or substituted or unsubstituted alkynyl;
when X^{B} is -NR^{B2}-, -CO-NR^{B2}-, -CO-O-NR^{B2}-, -O-CO-NR^{B2}- or -SO₂-NR^{B2}-, R^{B1} and R^{B2} may be taken together with an adjacent nitrogen atom to form substituted or unsubstituted aromatic heterocyclyl, or substituted or unsubstituted non-aromatic heterocyclyl;
when X^{B} is -NR^{B2} -CO-NR^{B3}-, RB¹ and R^{B3} may be taken together with an adjacent nitrogen atom to form substituted or unsubstituted aromatic heterocyclyl, or substituted or unsubstituted non-aromatic heterocyclyl;
p is any integer of 0 to 12;
R¹ and R^{B} may be taken together with an adjacent atom to form aromatic carbocycle, non-aromatic carbocycle, aromatic heterocycle or non-aromatic heterocycle, wherein the ring may be substituted with one or more of the same or different R^{B'};
R^{B'} is each independently, halogen, cyano, nitro, oxo, azide, trimethylsilyl or -X^{B'}-R^{B'1};
wherein X^{B'} is a single bond, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -COONR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- or -SO₂-NR^{B'2}-;
R^{B'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom;
R^{B'2} and R^{B'3} are each independently, a hydrogen atom, substituted or unsubstituted alkyl, substituted or unsubstituted alkenyl, or substituted or unsubstituted alkynyl;
when X^{B'} is -NR^{B'2}-, -CO-NR^{B'2}-, CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl;
when X^{B'} is -NR^{B'2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl.

4. The compound according to claim 1 or 2 or its pharmaceutically acceptable salt, wherein ring A is any one of the following rings: 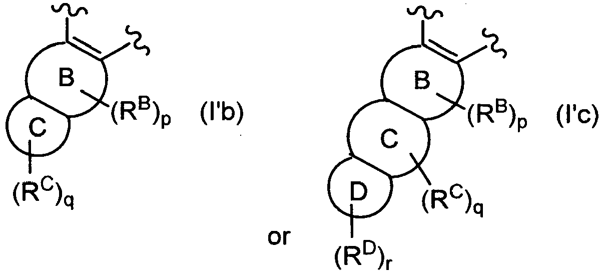 wherein
ring B is each independently monocyclic aromatic heterocycle or monocyclic non-aromatic heterocycle, ring C and ring D are each independently monocyclic aromatic carbocycle, monocyclic non-aromatic carbocycle, monocyclic aromatic heterocycle or monocyclic non-aromatic heterocycle;
wherein the ring(s) may form a spiro ring together with another aromatic carbocycle, non-aromatic carbocycle, aromatic heterocycle or non-aromatic heterocycle; and/or two atoms constituting each ring which is not adjacent to each other may be cross-linked with alkylene, alkenylene or alkynylene;
R^{B} is each independently, halogen, cyano, nitro, oxo, azide, trimethylsilyl or -X^{B} -R^{B1};
X^{B} is a single bond, -O-, -S-, -NR^{B2}-, =N-, -CO-, -SO₂-, -O-CO-, -CO-O-, -NR^{B2}-CO-, -CO-NR^{B2}-, -NR^{B2}-COO-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- or -SO₂-NR^{B2}-;
R^{B1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B} is a single bond, R^{B1} is not a hydrogen atom;
R^{B2} and R^{B3} are each independently a hydrogen atom, substituted or unsubstituted alkyl, substituted or unsubstituted alkenyl, or substituted or unsubstituted alkynyl;
when X^{B} is -NR^{B2}-, -CO-NR^{B2}-, -CO-O-NR^{B2}-, -O-CO-NR^{B2}- or -SO₂-NR^{B2}, R^{B1} and R^{B2} may be taken together with an adjacent nitrogen atom to form substituted or unsubstituted aromatic heterocyclyl, or substituted or unsubstituted non-aromatic heterocyclyl;
when X^{B} is -NR^{B2}-CO-NR^{B3}-, R^{B1} and R^{B3} may be taken together with an adjacent nitrogen atom to form substituted or unsubstituted aromatic heterocyclyl, or substituted or unsubstituted non-aromatic heterocyclyl;
p is any integer of 0 to 12;
R¹ and R^{B} may be taken together with an adjacent atom to form aromatic carbocycle, non-aromatic carbocycle, aromatic heterocycle or non-aromatic heterocycle, wherein the ring may be substituted with one or more of the same or different R^{B'} ;
R^{B'} is each independently, halogen, cyano, nitro, oxo, azide, trimethylsilyl or -X^{B'}-R^{B'1};
wherein X^{B'} is a single bond, -O-, -S-, -NR^{B'2}-, =N-, -CO-, -SO₂-, -O-CO-, -CO-O-, -NR^{B2}-CO-, -CO-NR^{B'2}-, -NR^{B'2}-CO-O-, -CO-O-NR^{B'2}-, -O-CO-NR^{B'2-}, -NR^{B'2}-O-CO-, -CO-NR^{B'2}-O-, -O-NR^{B'2}-CO-, -NR^{B'2}-CO-NR^{B'3}-, -NR^{B'2}-SO₂- or -SO₂-NR^{B'2}-;
R^{B'1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{B'} is a single bond, R^{B'1} is not a hydrogen atom;
R^{B'2} and R^{B'3} are each independently, a hydrogen atom, substituted or unsubstituted alkyl, substituted or unsubstituted alkenyl, or substituted or unsubstituted alkynyl;
when X^{B'} is -NR^{B'2}-, -CO-NR^{B'2}-, -CO-O-NR^{B'2}-, -O-CO-NR^{B'2}- or -SO₂-NR^{B'2}-, R^{B'1} and R^{B'2} may be taken together with an adjacent nitrogen atom to form substituted or unsubstituted aromatic heterocyclyl, or substituted or unsubstituted non-aromatic heterocyclyl;
when X^{B'} is -NR^{B'2}-CO-NR^{B'3}-, R^{B'1} and R^{B'3} may be taken together with an adjacent nitrogen atom to form substituted or unsubstituted aromatic heterocyclyl, or substituted or unsubstituted non-aromatic heterocyclyl;
s is any integer of 0 to 12;
R^{C} is each independently, halogen, cyano, nitro, oxo, azide, trimethylsilyl or -X^{C} -R^{C1};
X^{C} is a single bond, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-OCO-, -CO-NR^{C2}-O-, -O-NR^{C2}-CO-, -NR^{C2}-CO-NR^{C3}-, -NR^{C2}-SO₂- or -SO₂-NR^{C2}-;
R^{C1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, provided that when X^{C} is a single bond, R^{C1} is not a hydrogen atom;
R^{C2} and R^{C3} are each independently, a hydrogen atom, substituted or unsubstituted alkyl, substituted or unsubstituted alkenyl, or substituted or unsubstituted alkynyl;
when X^{C} is -NR^{C2}-, -CO-NR^{C2}-, -CO-O-NR^{C2}-, -O-CO-NR^{C2}- or -SO₂-NR^{C2}-, R^{C1} and R^{C2} may be taken together with an adjacent nitrogen atom to form substituted or unsubstituted aromatic heterocyclyl, or substituted or unsubstituted non-aromatic heterocyclyl;
when X^{C} is -NR^{C2}-CO-NR^{C3}-, R^{C1} and R^{C3} may be taken together with an adjacent nitrogen atom to form substituted or unsubstituted aromatic heterocyclyl, or substituted or unsubstituted non-aromatic heterocyclyl;
q is any integer of 0 to 12;
R^{B} and R^{C} may be taken together with an adjacent atom to form aromatic carbocycle, non-aromatic carbocycle, aromatic heterocycle or non-aromatic heterocycle, wherein the ring may be substituted with one or more of the same or different R^{B}; and
R^{D} is each independently, halogen, cyano, nitro, oxo, azide, trimethylsilyl or -X^{D} -R^{D1};
X^{D} is a single bond, -O-, -S-, -NR^{D2}-, =N-, -CO-, -SO₂-O-CO-, -CO-O-, -NR^{D2}-CO-, -CO-NR^{D2}-, -NR^{D2}-CO-O-, -CO-O-NR^{D2}-, -O-CO-NR^{D2}-, -NR^{D2}-O-CO-, -CO-NR^{D2}-O-, -O-NR^{D2}-CO-, -NR^{D2}-CO-NR^{D3}-, -NR^{D2}-SO₂- or -SO₂-NR^{D2}-;
R^{D1} is a hydrogen atom, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl or substituted or unsubstituted non-aromatic heterocyclyl, provided that when X^{D} is a single bond, R^{D1} is not a hydrogen atom;
R^{D2} and R^{D3} are each independently, a hydrogen atom, substituted or unsubstituted alkyl, substituted or unsubstituted alkenyl, or substituted or unsubstituted alkynyl;
when X^{D} is -NR^{D2}-, -CO-NR^{D2}-, -CO-O-NR^{D2}-, -O-CO-NR^{D2}- or -SO₂-NR^{D2}-, R^{D1} and R^{D2} may be taken together with an adjacent nitrogen atom to form substituted or unsubstituted aromatic heterocyclyl, or substituted or unsubstituted non-aromatic heterocyclyl,
when X^{D} is -NR^{D2}-CO-NR^{D3}-, R^{D1} and R^{D3} may be taken together with an adjacent nitrogen atom to form substituted or unsubstituted aromatic heterocyclyl, or substituted or unsubstituted non-aromatic heterocyclyl;
r is any integer of 0 to 12;
R^{C} and R^{D} may be taken together with an adjacent atom to form aromatic carbocycle, non-aromatic carbocycle, aromatic heterocycle or non-aromatic heterocycle, wherein the ring may be substituted with one or more of the same or different R^{C}.

5. The compound according to claim 4 or its pharmaceutically acceptable salt, wherein ring A is the following ring:

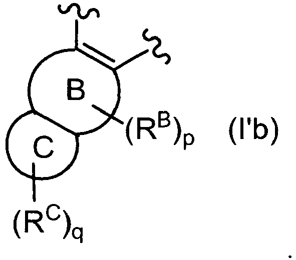

6. The compound according to any one of claims 3 to 5 or its pharmaceutically acceptable salt, wherein p is one or more.

7. The compound according to claim 3 or 6 represented by formula (I"a): 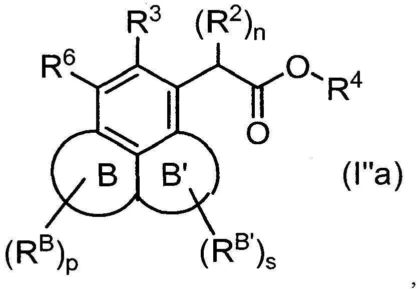 or its pharmaceutically acceptable salt,
wherein ring B' is monocyclic aromatic carbocycle, monocyclic non-aromatic carbocycle, monocyclic aromatic heterocycle, or monocyclic non-aromatic heterocycle; p is any integer of 0 to 11; s is any integer of 0 to 11.

8. The compound according to claim 1 represented by any one of following formulas: 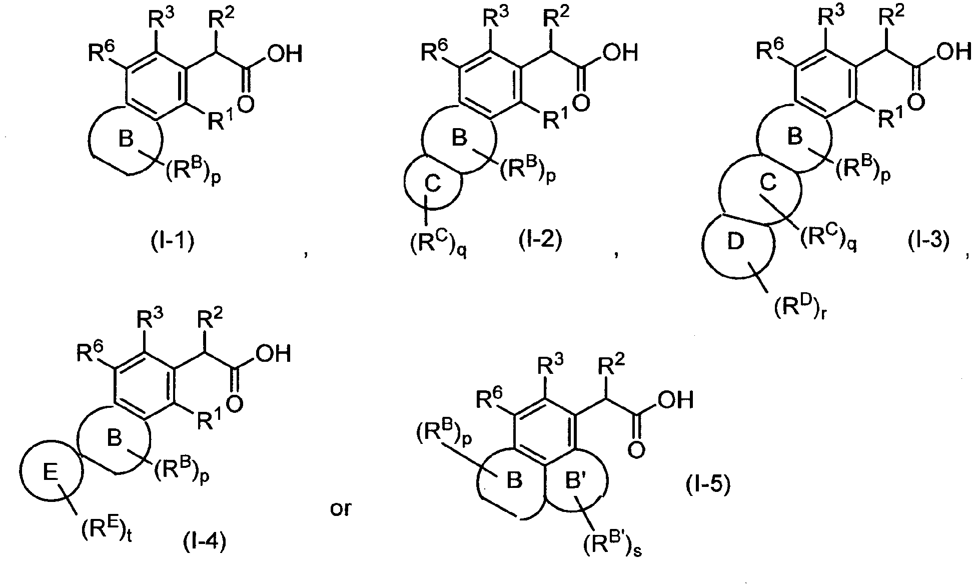 or its pharmaceutically acceptable salt,
wherein
R¹ is alkyl;
R² is alkyloxy, or substituted or unsubstituted cycloalkyloxy;
R³ is as defined in claim 1;
R⁶ is alkyl;
ring B is each independently monocyclic aromatic heterocycle or monocyclic non-aromatic heterocycle, ring C, ring D, ring E, and ring B' are each independently monocyclic aromatic carbocycle, monocyclic non-aromatic carbocycle, monocyclic aromatic heterocycle or monocyclic non-aromatic heterocycle;
ring E is the same as defined as ring C;
R^{B'} and R^{E} are each independently the same meaning as R^{C};
t is any integer of 0 to 12;
others are the same meaning as described in claim 4.

9. The compound according to claim 1 represented by any one of following formulas: 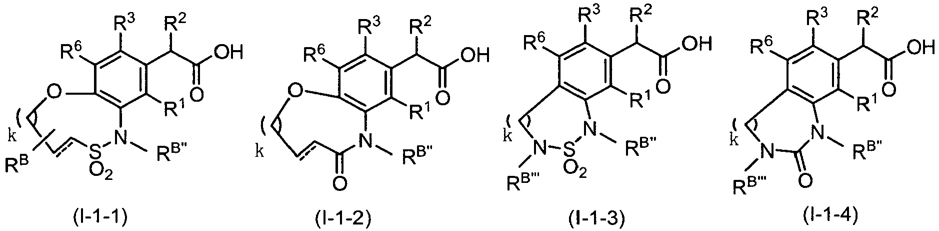 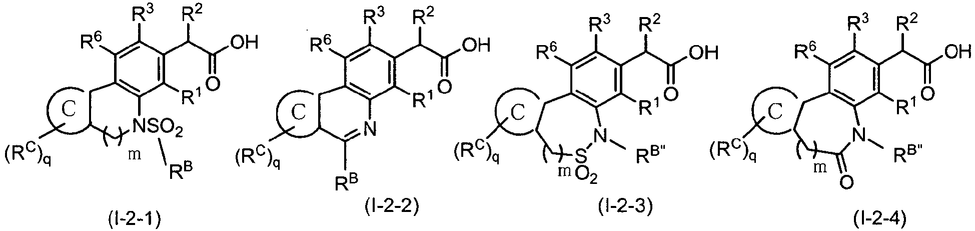 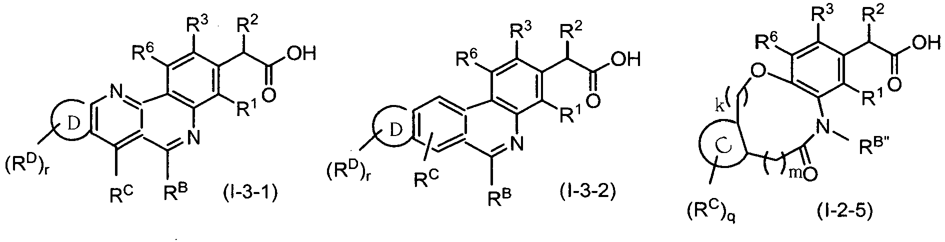 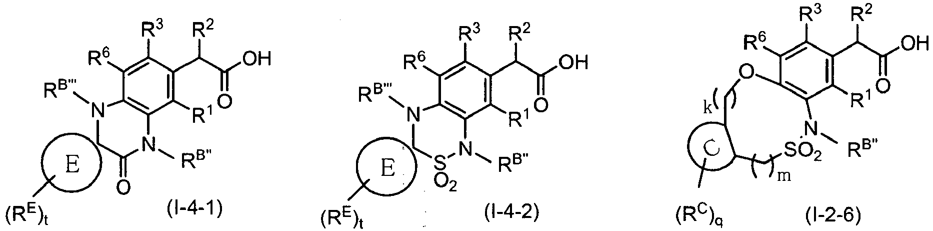 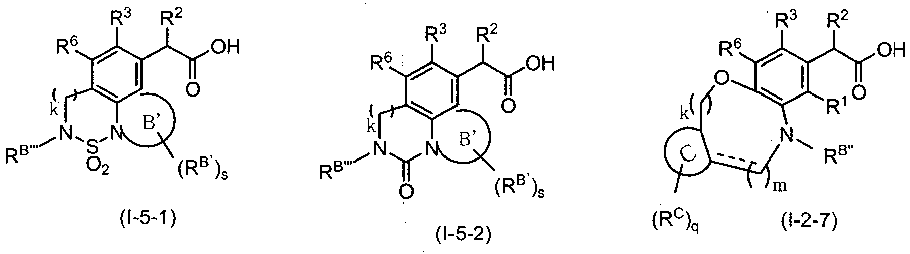 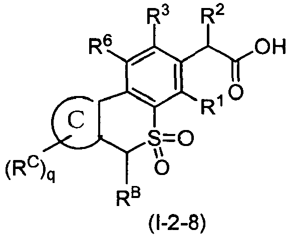 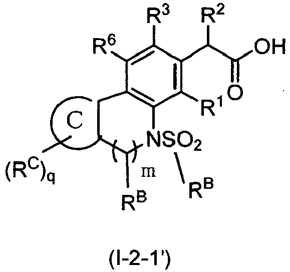 or its pharmaceutically acceptable salt,
wherein
ring C and ring D are each independently benzene or 5- to 8-membered heterocycle;
ring B' is 5- to 8-membered heterocycle;
ring E is optionally cross-linked 5- to 8-membered carbocycle;
R^{B''} and R^{B'''} are each independently a hydrogen atom, alkyl, cycloalkyl, cycloalkylalkyl, formyl, alkoxycarbonyl, substituted or unsubstituted aryl, substituted or unsubstituted aralkylaralkyl, substituted or unsubstituted heterocyclyl, substituted or unsubstituted heterocyclylalkyl, or substituted sulfonyl;
a broken line means the presence or absence of bond;
k and m are each independently an integer of 0 to 4;
others are the same meaning as described in claim 8.

10. The compound according to claim 9 represented by formulas (I-2-1), (I-2-2), (I-2-3) or (I-2-4), or its pharmaceutically acceptable salt.

11. A pharmaceutical composition comprising the compound according to any one of claims 1 to 10 or its pharmaceutically acceptable salt.

12. The pharmaceutical composition according to claim 11, having anti-HIV activity.

13. The compound according to any one of claims 1 to 10 or its pharmaceutically acceptable salt, for use in the treatment or prevention of HIV infection.

## Patentansprüche

1. Eine Verbindung, dargestellt durch die folgende Formel: 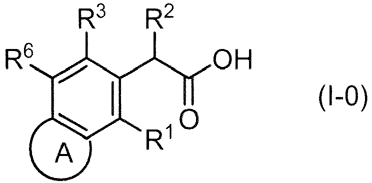 oder ein pharmazeutisch verträgliches Salz davon,
wobei
Ring A ein substituierter oder unsubstituierter Heterozyklus ist;
R¹ Alkyl ist;
oder R¹ mit einem oder mehreren Atomen des Rings A zusammengenommen werden kann, um einen substituierten oder unsubstituierten Heterozyklus zu bilden;
R² Alkyloxy oder substituiertes oder unsubstituiertes Cycloalkyloxy ist;
R³ aus den folgenden Gruppen ausgewählt ist: 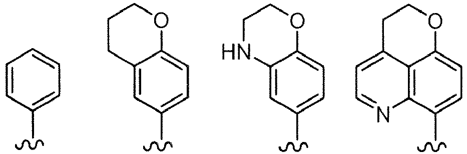 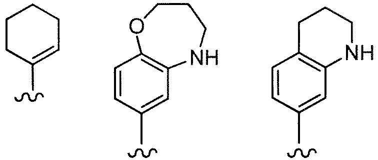 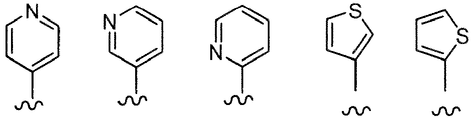 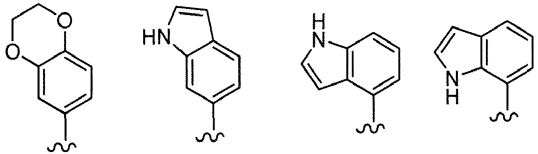 wobei die obigen Gruppen substituiert sein können mit gleichen oder verschiedenen 1 bis 4 Substituenten bestehend aus Alkyl, Alkoxy, Halogen, Hydroxy, Hydroxyalkyl, Alkoxyalkyl, Haloalkyl, Oxo, Amino, Mono- oder Di-alkylamino, Aminoalkyl, Mono- oder Di-alkylaminoalkyl und Cyano; und
R⁶ Alkyl ist.

2. Die Verbindung gemäß Anspruch 1 oder ein pharmazeutisch verträgliches Salz davon, wobei Ring A ein monozyklischer aromatischer Heterozyklus oder ein monozyklischer nicht-aromatischer Heterozyklus ist,
wobei der Ring anelliert sein kann mit einem anderen aromatischen Carbozyklus, nicht-aromatischen Carbozyklus, aromatischen Heterozyklus, oder nicht-aromatischen Heterozyklus; der Ring einen Spiroring bilden kann zusammen mit einem anderen aromatischen Carbozyklus, nicht-aromatischen Carbozyklus, aromatischen Heterozyklus oder nicht-aromatischen Heterozyklus; zwei Atome des Rings A, die nicht benachbart sind, vernetzt sein können mit Alkylen, Alkenylen oder Alkinylen; und/oder, Ringe substituiert sein können mit einem oder mehreren von R^{A};
wobei R^{A} jeweils unabhängig Halogen, Cyano, Nitro, Oxo, Azid, Trimethylsilyl oder -X^{A}-R^{A1} ist;
X^{A} eine Einfachbindung, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -CO-O-NR^{A2}-, -O-CO-NR^{A2}-, -NR^{A2}-O-CO-, -CO-NR^{A2}-O-, -O-NR^{A2}-CO-, -NR^{A2}-CO-NR^{A3}-, -NR^{A2}-SO₂- oder -SO₂-NR^{A2}- ist;
R^{A1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{A} eine Einfachbindung ist, R^{A1} kein Wasserstoffatom ist;
R^{A2} und R^{A3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{A} gleich -NR^{A2}-, -CO-NR^{A2}-, -CO-O-NR^{A2}-, -O-CO-NR^{A2}- oder -SO₂-NR^{A2}- ist, R^{A1} und R^{A2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{A} gleich -NR^{A2}-CO-NR^{A3}- ist, R^{A1} und R^{A3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
R¹ und R^{A} mit einem benachbarten Atom zusammen genommen werden können, um einen aromatischen Carbozyklus, nicht-aromatischen Carbozyklus, aromatischen Heterozyklus oder nicht-aromatischen Heterozyklus zu bilden, wobei der Ring substituiert sein kann mit einem oder mehreren der gleichen oder verschiedenen R^{A'}; wobei R^{A'} jeweils unabhängig Halogen, Cyano, Nitro, Oxo, Azid, Trimethylsilyl oder -X^{A'}-R^{A'1} ist;
X^{A'} eine Einfachbindung, -O-, -S-, -NR^{A2}-, =N-, -CO-, -SO₂-, -O-CO-, -CO-O-, -NR^{A'2}-CO-, -CO-NR^{A'2}-, -NR^{A2}-CO-O-, -CO-O-NR^{A'2}-, -O-CO-NR^{A'2}-, -NR^{A'2}-O-CO-, -CO-NR^{A'2}-O-, -O-NR^{A'2}-CO-, -NR^{A'2}-CO-NR^{A'3}-, -NR^{A2}-SO₂- oder -SO₂-NR^{A'2}- ist;
R^{A'1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{A'} eine Einfachbindung ist, R^{A'1} kein Wasserstoffatom ist;
R^{A'2} und R^{A'3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
X^{A'} gleich -NR^{A'2}-, -CO-NR^{A'2}-, -CO-O-NR^{A'2}-, -O-CO-NR^{A'2}- oder -SO₂-NR^{A'2}- ist;
R^{A'1} und R^{A'2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{A'} gleich -NR^{A2}-CO-NR^{A'3}- ist, R^{A'1} und R^{A'3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden.

3. Die Verbindung gemäß Anspruch 1 oder 2 oder ein pharmazeutisch verträgliches Salz davon, wobei Ring A der folgende Ring ist: 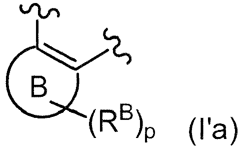 wobei
Ring B ein monozyklischer aromatischer Heterozyklus oder ein monozyklischer nicht-aromatischer Heterozyklus ist; wobei der Ring einen Spiroring bilden kann zusammen mit einem anderen aromatischen Carbozyklus, nicht-aromatischen Carbozyklus, aromatischen Heterozyklus oder nicht-aromatischen Heterozyklus; und/oder zwei Atome jedes Rings, die nicht benachbart sind, vernetzt sein können mit Alkylen, Alkenylen oder Alkinylen;
R^{B} jeweils unabhängig Halogen, Cyano, Nitro, Oxo, Azid, Trimethylsilyl oder -X^{B}-R^{B1} ist;
X^{B} eine Einfachbindung, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- oder -SO₂-NR^{B2}- ist;
R^{B1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{B} eine Einfachbindung ist, R^{B1} kein Wasserstoffatom ist;
R^{B2} und R^{B3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{B} gleich -NR^{B2}-, -CO-NR^{B2}-, -CO-O-NR^{B2}-, -O-CO-NR^{B2}- oder -SO₂-NR^{B2}- ist, R^{B1} und R^{B2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{B} gleich -NR^{B2}-CO-NR^{B3}- ist, R^{B1} und R^{B3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
p eine ganze Zahl von 0 bis 12 ist;
R¹ und R^{B} mit einem benachbarten Atom zusammen genommen werden können, um einen aromatischen Carbozyklus, nicht-aromatischen Carbozyklus, aromatischen Heterozyklus oder nicht-aromatischen Heterozyklus zu bilden, wobei der Ring substituiert sein kann mit einem oder mehreren der gleichen oder verschiedenen R^{B'}; R^{B'} jeweils unabhängig Halogen, Cyano, Nitro, Oxo, Azid, Trimethylsilyl oder -X^{B'}-R^{B'1} sind;
wobei X^{B'} eine Einfachbindung, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -CO-O-NR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- oder -SO₂-NR^{B'2}- ist;
R^{B1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{B'} eine Einfachbindung ist, R^{B'1} kein Wasserstoffatom ist;
R^{B'2} und R^{B'3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{B'} gleich -NR^{B'2}-, -CO-NR^{B'2}-, -CO-O-NR^{B'2}-, -O-CO-NR^{B'2}- oder -SO₂-NR^{B'2}- ist, R^{B'1} und R^{B'2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{B'} gleich -NR^{B'2}-CO-NR^{B'3}- ist, R^{B'1} und R^{B'3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden.

4. Die Verbindung gemäß Anspruch 1 oder 2 oder ein pharmazeutisch verträgliches Salz davon, wobei Ring A ein beliebiger der folgenden Ringe ist: 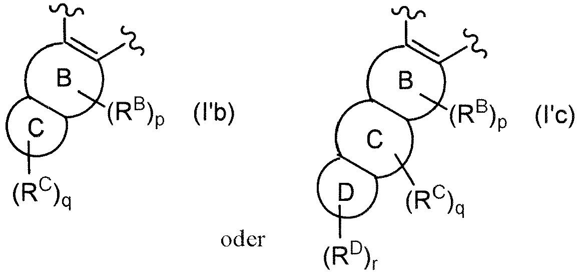 wobei
Ring B jeweils unabhängig ein monozyklischer aromatischer Heterozyklus oder ein monozyklischer nicht-aromatischer Heterozyklus ist, Ring C und Ring D jeweils unabhängig ein monozyklischer aromatischer Carbozyklus, ein monozyklischer nicht-aromatischer Carbozyklus, ein monozyklischer aromatischer Heterozyklus oder ein monozyklischer nicht-aromatischer Heterozyklus sind;
wobei die Ring(e) einen Spiroring bilden können zusammen mit einem anderen aromatischen Carbozyklus, nicht-aromatischen Carbozyklus, aromatischen Heterozyklus oder nicht-aromatischen Heterozyklus; und/oder zwei Atome jedes Rings, die nicht benachbart sind, vernetzt sein können mit Alkylen, Alkenylen oder Alkinylen;
R^{B} jeweils unabhängig Halogen, Cyano, Nitro, Oxo, Azid, Trimethylsilyl oder -X^{B}-R^{B1} sind;
X^{B} eine Einfachbindung, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- oder -SO₂-NR^{B2}- ist;
R^{B1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{B} eine Einfachbindung ist, R^{B1} kein Wasserstoffatom ist;
R^{B2} und R^{B3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{B} gleich -NR^{B2}-, -CO-NR^{B2}-, -CO-O-NR^{B2}-, -O-CO-NR^{B2}- oder -SO₂-NR^{B2}- ist, R^{B1} und R^{B2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{B} gleich -NR^{B2}-CO-NR^{B3}- ist, R^{B1} und R^{B3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
p eine ganze Zahl von 0 bis 12 ist;
R¹ und R^{B} mit einem benachbarten Atom zusammen genommen werden können, um einen aromatischen Carbozyklus, nicht-aromatischen Carbozyklus, aromatischen Heterozyklus oder nicht-aromatischen Heterozyklus zu bilden, wobei der Ring substituiert sein kann mit einem oder mehreren der gleichen oder verschiedenen R^{B'}; R^{B'} jeweils unabhängig Halogen, Cyano, Nitro, Oxo, Azid, Trimethylsilyl oder -X^{B'}-R^{B'1} ist;
wobei X^{B'} eine Einfachbindung, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -CO-ONR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- oder -SO₂-NR^{B'2}- ist;
R^{B'1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{B'} eine Einfachbindung ist, R^{B'1} kein Wasserstoffatom ist;
R^{B'2} und R^{B'3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{B'} gleich -NR^{B'2}-, -CO-NR^{B'2}-, -CO-O-NR^{B'2}-, -O-CO-NR^{B'2}- oder -SO₂-NR^{B'2}-ist, R^{B'1} und R^{B'2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{B'} gleich -NR^{B'2}-CO-NR^{B'3}- ist, R^{B'1} und R^{B'3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
s eine ganze Zahl von 0 bis 12 ist;
R^{C} jeweils unabhängig Halogen, Cyano, Nitro, Oxo, Azid, Trimethylsilyl oder -X^{C}-R^{C1} sind;
X^{C} eine Einfachbindung, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-O-CO-, -CO-NR^{C2}-O-, -O-NR^{C2}-CO-, -NR^{C2}-CO-NR^{C3}-, -NR^{C2}-SO₂- oder -SO₂-NR^{C2}- ist;
R^{C1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl, oder substituiertes oder unsubstituiertes nicht-aromatisches Heterocyclyl ist, unter der Bedingung, dass, wenn X^{C} eine Einfachbindung ist, R^{C1} kein Wasserstoffatom ist;
R^{C2} und R^{C3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{C} gleich -NR^{C2}-, -CO-NR^{C2}-, -CO-O-NR^{C2}-, -O-CO-NR^{C2}- oder -SO₂-NR^{C2}- ist, R^{C1} und R^{C2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
wenn X^{C} gleich -NR^{C2}-CO-NR^{C3}- ist, R^{C1} und R^{C3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
q eine ganze Zahl von 0 bis 12 ist;
R^{B} und R^{C} mit einem benachbarten Atom zusammen genommen werden können, um einen aromatischen Carbozyklus, nicht-aromatischen Carbozyklus, aromatischen Heterozyklus oder nicht-aromatischen Heterozyklus zu bilden, wobei der Ring substituiert sein kann mit einem oder mehreren der gleichen oder verschiedenen R^{B}; und
R^{D} jeweils unabhängig Halogen, Cyano, Nitro, Oxo, Azid, Trimethylsilyl oder -X^{D}-R^{D1} sind;
X^{D} eine Einfachbindung, -O-, -S-, -NR^{D2}-, =N-, -CO-, -SO₂-, -O-CO-, -CO-O-, -NR^{D2}-CO-, -CO-NR^{D2}-, -NR^{D2}-CO-O-, -CO-O-NR^{D2}-, -O-CO-NR^{D2}-, -NR^{D2}-O-CO-, -CO-NR^{D2}-O-, -O-NR^{D2}-CO-, -NR^{D2}-CO-NR^{D3}-, -NR^{D2}-SO₂- oder -SO₂-NR^{D2}- ist;
R^{D1} ein Wasserstoffatom, substituiertes oder unsubstituiertes Alkyl, substituiertes oder unsubstituiertes Alkenyl, substituiertes oder unsubstituiertes Alkinyl, substituiertes oder unsubstituiertes aromatisches Carbocyclyl, substituiertes oder unsubstituiertes nicht-aromatisches Carbocyclyl, substituiertes oder unsubstituiertes aromatisches Heterocyclyl oder substituiertes oder unsubstituiertes nicht-aromatisches Heterocyclyl ist, unter der Bedingung, dass, wenn X^{D} eine Einfachbindung ist, R^{D1} kein Wasserstoffatom ist;
R^{D2} und R^{D3} jeweils unabhängig ein Wasserstoffatom, substituiertes oder unsubstituiertes Alkyl, substituiertes oder unsubstituiertes Alkenyl, oder substituiertes oder unsubstituiertes Alkinyl sind;
wenn X^{D} gleich -NR^{D2}-, -CO-NR^{D2}-, -CO-O-NR^{D2}-, -O-CO-NR^{D2}- oder -SO₂-NR^{D2}- ist, R^{D1} und R^{D2} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden,
wenn X^{D} gleich -NR^{D2}-CO-NR^{D3}- ist, R^{D1} und R^{D3} mit einem benachbarten Stickstoffatom zusammen genommen werden können, um substituiertes oder unsubstituiertes aromatisches Heterocyclyl, oder substituiertes oder unsubstituiertes nicht-aromatisches Heterocyclyl zu bilden;
r eine ganze Zahl von 0 bis 12 ist;
R^{C} und R^{D} mit einem benachbarten Atom zusammen genommen werden können, um einen aromatischen Carbozyklus, nicht-aromatischen Carbozyklus, aromatischen Heterozyklus oder nicht-aromatischen Heterozyklus zu bilden, wobei der Ring substituiert sein kann mit einem oder mehreren der gleichen oder verschiedenen R^{C}.

5. Die Verbindung gemäß Anspruch 4 oder ein pharmazeutisch verträgliches Salz davon, wobei Ring A der folgende Ring ist:

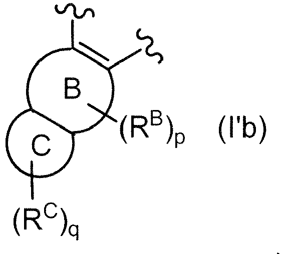

6. Die Verbindung gemäß einem der Ansprüche 3 bis 5 oder ein pharmazeutisch verträgliches Salz davon, wobei p eins oder mehr ist.

7. Die Verbindung gemäß Anspruch 3 oder 6, dargestellt durch die Formel (I"a): 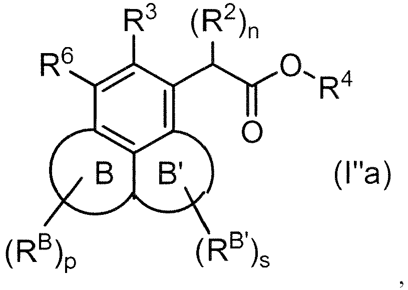 oder ein pharmazeutisch verträgliches Salz davon,
wobei Ring B' ein monozyklischer aromatischer Carbozyklus, ein monozyklischer nicht-aromatischer Carbozyklus, monozyklischer aromatischer Heterozyklus, oder ein monozyklischer nicht-aromatischer Heterozyklus ist; p eine ganze Zahl von 0 bis 11 ist; s eine ganze Zahl von 0 bis 11 ist.

8. Die Verbindung gemäß Anspruch 1, dargestellt durch eine der folgenden Formeln: 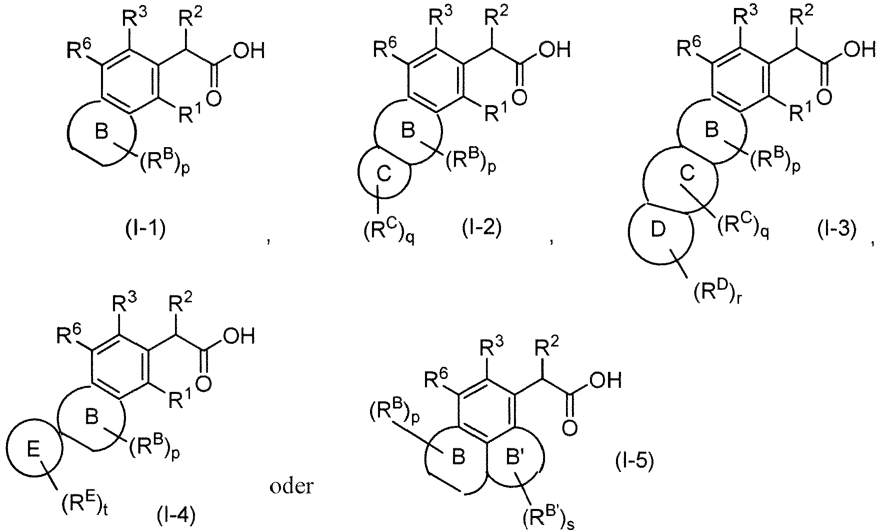 oder ein pharmazeutisch verträgliches Salz davon,
wobei
R¹ Alkyl ist;
R² Alkyloxy ist, oder substituiertes oder unsubstituiertes Cycloalkyloxy;
R³ wie in Anspruch 1 definiert ist;
R⁶ Alkyl ist;
Ring B jeweils unabhängig ein monozyklischer aromatischer Heterozyklus oder ein monozyklischer nicht-aromatischer Heterozyklus ist, Ring C, Ring D, Ring E, und Ring B' jeweils unabhängig ein monozyklischer aromatischer Carbozyklus, ein monozyklischer nicht-aromatischer Carbozyklus, ein monozyklischer aromatischer Heterozyklus oder ein monozyklischer nicht-aromatischer Heterozyklus sind;
Ring E dieselbe Definition wie Ring C aufweist;
R^{B'} und R^{E} jeweils unabhängig dieselbe Bedeutung wie R^{C} aufweisen;
t eine ganze Zahl von 0 bis 12 ist;
die anderen die gleiche Bedeutung haben, wie in Anspruch 4 beschrieben.

9. Die Verbindung gemäß Anspruch 1, dargestellt durch eine der folgenden Formeln: 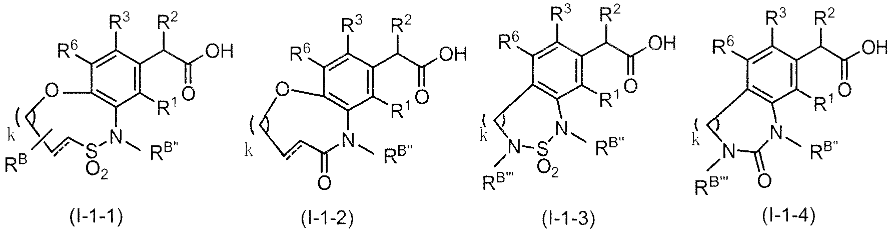 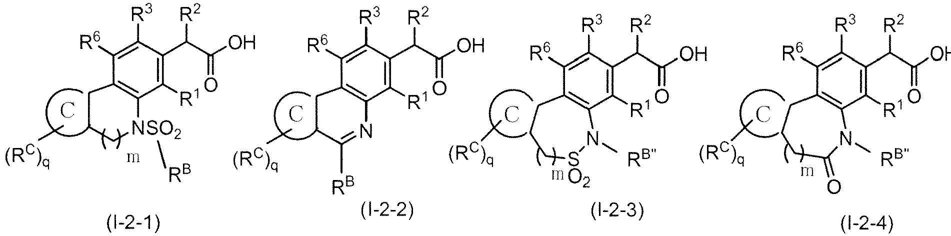 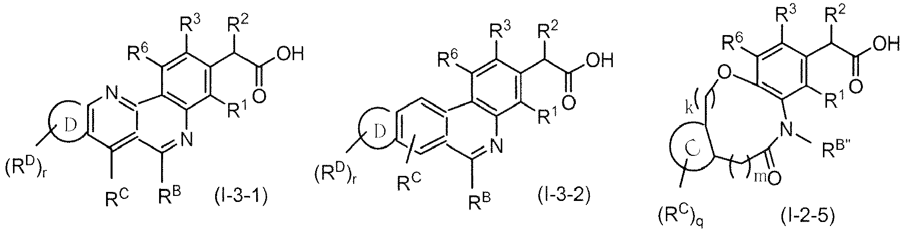 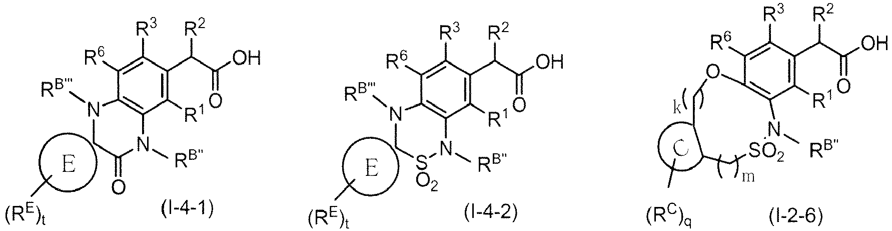 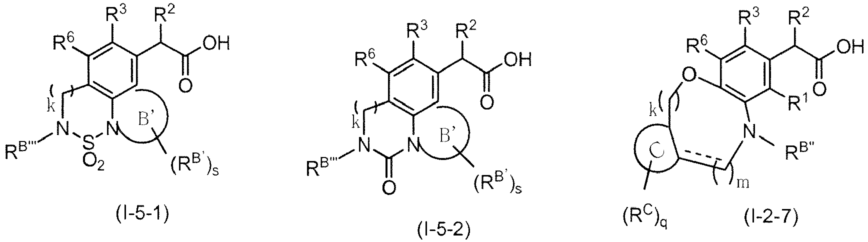 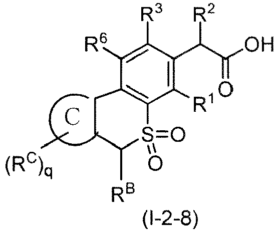 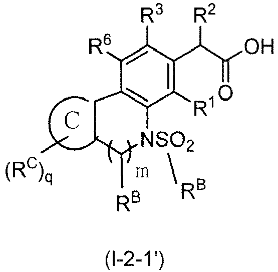 oder ein pharmazeutisch verträgliches Salz davon,
wobei
Ring C und Ring D jeweils unabhängig Benzol oder ein 5- bis 8-gliedriger Heterozyklus sind;
Ring B' ein 5- bis 8-gliedriger Heterozyklus ist;
Ring E ein gegebenenfalls vernetzter 5- bis 8-gliedriger Carbozyklus ist;
R^{B"} und R^{B'''} jeweils unabhängig ein Wasserstoffatom, Alkyl, Cycloalkyl, Cycloalkylalkyl, Formyl, Alkoxycarbonyl, substituiertes oder unsubstituiertes Aryl, substituiertes oder unsubstituiertes Aralkylaralkyl, substituiertes oder unsubstituiertes Heterocyclyl, substituiertes oder unsubstituiertes Heterocyclylalkyl, oder substituiertes Sulfonyl sind;
eine unterbrochene Linie die Anwesenheit oder Abwesenheit einer Bindung bedeutet;
k und m jeweils unabhängig eine ganze Zahl von 0 bis 4 sind;
die anderen die gleiche Bedeutung haben, wie in Anspruch 8 beschrieben.

10. Die Verbindung gemäß Anspruch 9, dargestellt durch die Formeln (I-2-1), (I-2-2), (I-2-3) oder (I-2-4), oder ein pharmazeutisch verträgliches Salz davon.

11. Ein Arzneimittel, umfassend eine Verbindung gemäß einem der Ansprüche 1 bis 10 oder ein pharmazeutisch verträgliches Salz davon.

12. Das Arzneimittel gemäß Anspruch 11, das eine Wirkung gegen HIV aufweist.

13. Die Verbindung gemäß einem der Ansprüche 1 bis 10 oder ein pharmazeutisch verträgliches Salz davon, zur Verwendung bei der Behandlung oder Vorbeugung einer HIV - Infektion.

## Revendications

1. Composé représenté par la formule suivante : 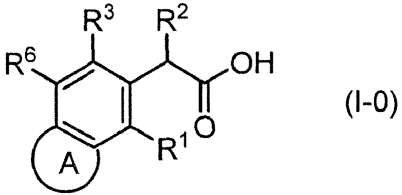 ou son sel pharmaceutiquement acceptable,
dans laquelle
le cycle A est un hétérocycle substitué ou non substitué ;
R¹ est un alkyle ;
ou bien R¹ peut être pris conjointement avec le ou les atomes constituant le cycle A pour former un hétérocycle substitué ou non substitué ;
R² est un alkyloxy, ou un cycloalkyloxy substitué ou non substitué ;
R³ est choisi parmi les groupes suivants : 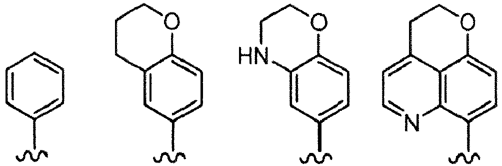 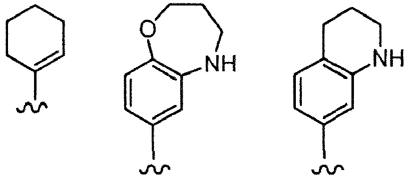 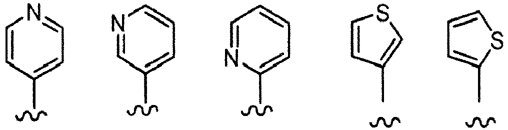 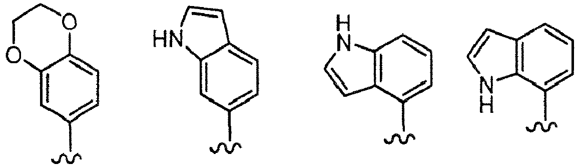
lesquels groupes ci-dessus peuvent être substitués par 1 à 4 substituants identiques ou différents consistant en alkyle, alcoxy, halogéno, hydroxy, hydroxyalkyle, alcoxyalkyle, halogénoalkyle, oxo, amino, mono- ou di-alkylamino, aminoalkyle, mono- ou di-alkylaminoalkyle, et cyano ; et
R⁶ est un alkyle.

2. Composé selon la revendication 1 ou son sel pharmaceutiquement acceptable, dans lequel le cycle A est un hétérocycle aromatique monocyclique ou un hétérocycle non aromatique monocyclique,
dans lequel le cycle peut être condensé avec un autre carbocycle aromatique, carbocycle non aromatique, hétérocycle aromatique, ou hétérocycle non aromatique ; le cycle peut former un cycle spiro conjointement avec un autre carbocycle aromatique, carbocycle non aromatique, hétérocycle aromatique ou hétérocycle non aromatique ; deux atomes constituant le cycle A qui ne sont pas adjacents l'un à l'autre peuvent être réticulés avec un alkylène, alcénylène ou alcynylène ; et/ou les cycles peuvent être substitués par un ou plusieurs R^{A} ;
dans lequel chaque R^{A} est indépendamment un halogène, cyano, nitro, oxo, azoture, triméthylsilyle ou -X^{A}-R^{A1} ;
X^{A} est une liaison simple, -O-, -S-, -NR^{A2}-, =N-, -CO-, -SO₂-, -O-CO-, -CO-O-, -NR^{A2}-CO-, -CO-NR^{A2}-, -NR^{A2}-CO-O-, -CO-O-NR^{-A2}-, -O-CO-NR^{A2}-, -NR^{A2}-O-CO-, -CO-NR^{A2}-O-, -O-NR^{A2}-CO-, -NR^{A2}-CO-NR^{A3}-, -NR^{A2}-SO₂- ou -SO₂-NR^{A2}- ;
R^{A1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{A} est une liaison simple, R^{A1} ne soit pas un atome d'hydrogène ;
chacun de R^{A2} et R^{A3} est indépendamment un atome d'hydrogène, un alkyle substitué ou non substitué, un alcényle substitué ou non substitué, ou un alcynyle substitué ou non substitué ;
quand X^{A} est -NR^{A2}-, -CO-NR^{A2}-, -CO-O-NR^{A2}-, -O-CO-NR^{A2}- ou -SO₂-NR^{A2}-, R^{A1} et R^{A2} peuvent être pris conjointement avec un atome d'azote adjacent pour former un hétérocyclyle aromatique substitué ou non substitué, ou un hétérocyclyle non aromatique substitué ou non substitué ;
quand X^{A} est -NR^{A2}-CO-NR^{A3}-, R^{A1} et R^{A3} peuvent être pris conjointement avec un atome d'azote adjacent pour former un hétérocyclyle aromatique substitué ou non substitué, ou un hétérocyclyle non aromatique substitué ou non substitué ;
R¹ et R^{A} peuvent être pris conjointement avec un atome adjacent pour former un carbocycle aromatique, un carbocycle non aromatique, un hétérocycle aromatique ou un hétérocycle non aromatique, lequel cycle peut être substitué par un ou plusieurs R^{A'} identiques ou différents ;
dans lequel chaque R^{A'} est indépendamment un halogène, cyano, nitro, oxo, azoture, triméthylsilyle ou -X^{A'}-R^{A'1} ;
X^{A'} est une liaison simple, -O-, -S-, -NR^{A'2}-, =N-, -CO-, -SO₂-, -O-CO-, -CO-O-, -NR^{A'2}-CO-, -CO-NR^{A'2}-, -NR^{A'2}-CO-O-, -CO-O-NR^{A'2}-, -O-CO-NR^{A'2}-, -NR^{A'2}-O-CO-, -CO-NR^{A'2}-O-, -O-NR^{A'2}-CO-, -NR^{A'2}-CO-NR^{A'3}-, -NR^{A'2}-SO₂- ou -SO₂-NR^{A'2}- ;
R^{A'1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{A'} est une liaison simple, R^{A'1} ne soit pas un atome d'hydrogène ;
chacun de R^{A'2} et R^{A'3} est indépendamment un atome d'hydrogène, un alkyle substitué ou non substitué, un alcényle substitué ou non substitué, ou un alcynyle substitué ou non substitué ;
X^{A'} est -NR^{A'2}-, -CO-NR^{A'2}-, -CO-O-NR^{A'2}-, -O-CO-NR^{A'2}- ou -SO₂-NR^{A'2}- ;
R^{A'1} et R^{A'2} peuvent être pris conjointement avec un atome d'azote adjacent pour former un hétérocyclyle aromatique substitué ou non substitué ou un hétérocyclyle non aromatique substitué ou non substitué ;
quand X^{A'} est -NR^{A'2}-CO-NR^{A'3}-, R^{A'1} et R^{A'3} peuvent être pris conjointement avec un atome d'azote adjacent pour former un hétérocyclyle aromatique substitué ou non substitué ou un hétérocyclyle non aromatique substitué ou non substitué.

3. Composé selon la revendication 1 ou 2 ou son sel pharmaceutiquement acceptable, dans lequel le cycle A est le cycle suivant : 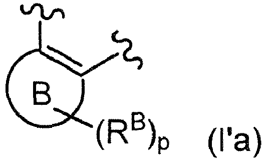 dans lequel
le cycle B est un hétérocycle aromatique monocyclique ou un hétérocycle non aromatique monocyclique ; lequel cycle peut former un cycle spiro conjointement avec un autre carbocycle aromatique, carbocycle non aromatique, hétérocycle aromatique ou hétérocycle non aromatique ; et/ou deux atomes constituant chaque cycle qui ne sont pas adjacents l'un à l'autre peuvent être réticulés avec un alkylène, alcénylène ou alcynylène ;
chaque R^{B} est indépendamment un halogène, cyano, nitro, oxo, azoture, triméthylsilyle ou -X^{B}-R^{B1} ;
X^{B} est une liaison simple, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- ou -SO₂-NR^{B2}- ;
R^{B1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{B} est une liaison simple, R^{B1} ne soit pas un atome d'hydrogène ;
chacun de R^{B2} et R^{B3} est indépendamment un atome d'hydrogène, un alkyle substitué ou non substitué, un alcényle substitué ou non substitué, ou un alcynyle substitué ou non substitué ;
quand X^{B} est -NR^{B2}-, -CO-NR^{B2}-, -CO-O-NR^{B2}-, -O-CO-NR^{B2}- ou -SO₂-NR^{B2}-, R^{B1} et R^{B2} peuvent être pris conjointement avec un atome d'azote adjacent pour former un hétérocyclyle aromatique substitué ou non substitué, ou un hétérocyclyle non aromatique substitué ou non substitué ;
quand X^{B} est -NR^{B2}-CO-NR^{B3}-, R^{B1} et R^{B3} peuvent être pris conjointement avec un atome d'azote adjacent pour former un hétérocyclyle aromatique substitué ou non substitué, ou un hétérocyclyle non aromatique substitué ou non substitué ;
p est un entier quelconque de 0 à 12 ;
R¹ et R^{B} peuvent être pris conjointement avec un atome adjacent pour former un carbocycle aromatique, un carbocycle non aromatique, un hétérocycle aromatique ou un hétérocycle non aromatique, lequel cycle peut être substitué par un ou plusieurs R^{B'} identiques ou différents ;
chaque R^{B'} est indépendamment un halogène, cyano, nitro, oxo, azoture, triméthylsilyle ou -X^{B'}-R^{B'1} ;
dans lequel X^{B'} est une liaison simple, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -CO-O-NR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- ou -SO₂-NR^{B'2}- ;
R^{B'1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{B'} est une liaison simple, R^{B'} ne soit pas un atome d'hydrogène ;
chacun de R^{B'2} et R^{B'3} est indépendamment un atome d'hydrogène, un alkyle substitué ou non substitué, un alcényle substitué ou non substitué, ou un alcynyle substitué ou non substitué ;
quand X^{B'} est -NR^{B'2}-, -CO-NR^{B'2}-, -CO-O-NR^{B'2}-, -O-CO-NR^{B'2}- ou -SO₂-NR^{B'2}- R^{B'1} et R^{B'2} peuvent être pris conjointement avec un atome d'azote adjacent pour former un hétérocyclyle aromatique substitué ou non substitué ou un hétérocyclyle non aromatique substitué ou non substitué ;
quand X^{B'} est -NR^{B'2}-CO-NR^{B'3}-, R^{B'1} et R^{B'3} peuvent être pris conjointement avec un atome d'azote adjacent pour former un hétérocyclyle aromatique substitué ou non substitué ou un hétérocyclyle non aromatique substitué ou non substitué.

4. Composé selon la revendication 1 ou 2 ou son sel pharmaceutiquement acceptable, dans lequel le cycle A est l'un quelconque des cycles suivants : 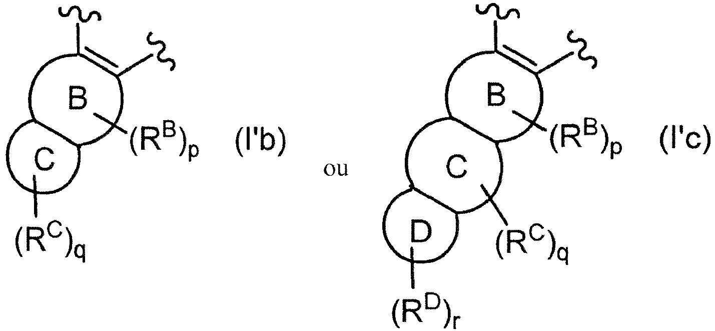 où
chaque cycle B est indépendamment un hétérocycle aromatique monocyclique ou un hétérocycle non aromatique monocyclique, chaque cycle C et cycle D est indépendamment un carbocycle aromatique monocyclique, un carbocycle non aromatique monocyclique, un hétérocycle aromatique monocyclique ou un hétérocycle non aromatique monocyclique ;
lequel ou lesquels cycles peuvent former un cycle spiro conjointement avec un autre carbocycle aromatique, carbocycle non aromatique, hétérocycle aromatique ou hétérocycle non aromatique ; et/ou deux atomes constituant chaque cycle qui ne sont pas adjacents l'un à l'autre peuvent être réticulés avec un alkylène, alcénylène ou alcynylène ;
chaque R^{B} est indépendamment un halogène, cyano, nitro, oxo, azoture, triméthylsilyle ou -X^{B} -R^{B1} ;
X^{B} est une liaison simple, -O-, -S-, -NR^{B2}-, =N-, -CO-, -SO₂-, -O-CO-, -CO-O-, -NR^{B2}-CO-, -CO-NR^{B2}-, -NR^{B2}-CO-O-, -CO-O-NR^{B2}-, -O-CO-NR^{B2}-, -NR^{B2}-O-CO-, -CO-NR^{B2}-O-, -O-NR^{B2}-CO-, -NR^{B2}-CO-NR^{B3}-, -NR^{B2}-SO₂- ou -SO₂-NR^{B2}- ;
R^{B1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{B} est une liaison simple, R^{B1} ne soit pas un atome d'hydrogène ;
chacun de R^{B2} et R^{B3} est indépendamment un atome d'hydrogène, un alkyle substitué ou non substitué, un alcényle substitué ou non substitué, ou un alcynyle substitué ou non substitué ;
quand X^{B} est -NR^{B2}-, -CO-NR^{B2}-, -CO-O-NR^{B2}-, -O-CO-NR^{B2}- ou -SO₂-NR^{B2}-, R^{B1} et R^{B2} peuvent être pris conjointement avec un atome d'azote adjacent pour former un hétérocyclyle aromatique substitué ou non substitué, ou un hétérocyclyle non aromatique substitué ou non substitué ;
quand X^{B} est -NR^{B2}-CO-NR^{B3}-, R^{B1} et R^{B3} peuvent être pris conjointement avec un atome d'azote adjacent pour former un hétérocyclyle aromatique substitué ou non substitué, ou un hétérocyclyle non aromatique substitué ou non substitué ;
p est un entier quelconque de 0 à 12 ;
R¹ et R^{B} peuvent être pris conjointement avec un atome adjacent pour former un carbocycle aromatique, un carbocycle non aromatique, un hétérocycle aromatique ou un hétérocycle non aromatique, lequel cycle peut être substitué par un ou plusieurs R^{B'} identiques ou différents ;
chaque R^{B'} est indépendamment un halogène, cyano, nitro, oxo, azoture, triméthylsilyle ou -X^{B'}-R^{B'1} ;
dans lequel X^{B'} est une liaison simple, -O-, -S-, -NR^{B'2}-, =N-, -CO-, -SO₂-, -O-CO-, -CO-O-, -NR^{B'2}-CO-, -CO-NR^{B'2}-, -NR^{B'2}-CO-O-, -CO-O-NR^{B'2}-, -O-CO-NR^{B'2}-, -NR^{B'2}-O-CO-, -CO-NR^{B'2}-O-, -O-NR^{B'2}-CO-, -NR^{B'2}-CO-NR^{B'3}-, -NR^{B'2}-SO₂- ou -SO₂-NR^{B'2}- ;
R^{B'1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{B'} est une liaison simple, R^{B'1} ne soit pas un atome d'hydrogène ;
chacun de R^{B'2} et R^{B'3} est indépendamment un atome d'hydrogène, un alkyle substitué ou non substitué, un alcényle substitué ou non substitué, ou un alcynyle substitué ou non substitué ;
quand X^{B'} est -NR^{B'2}-, -CO-NR^{B'2}-, -CO-O-NR^{B'2}-, -O-CO-NR^{B'2}- ou -SO₂-NR^{B'2}-, R^{B'1} et R^{B'2} peuvent être pris conjointement avec un atome d'azote adjacent pour former un hétérocyclyle aromatique substitué ou non substitué ou un hétérocyclyle non aromatique substitué ou non substitué ;
quand X^{B'} est -NR^{B'2}-CO-NR^{B'3}-, R^{B'1} et R^{B'3} peuvent être pris conjointement avec un atome d'azote adjacent pour former un hétérocyclyle aromatique substitué ou non substitué ou un hétérocyclyle non aromatique substitué ou non substitué ;
s est un entier quelconque de 0 à 12 ;
chaque R^{C} est indépendamment un halogène, cyano, nitro, oxo, azoture, triméthylsilyle ou -X^{C}-R^{C1} ;
X^{C} est une liaison simple, -O-, -S-, -NR^{C2}-, =N-, -CO-, -SO₂-, -O-CO-, -CO-O-, -NR^{C2}-CO-, -CO-NR^{C2}-, -NR^{C2}-CO-O-, -CO-O-NR^{C2}-, -O-CO-NR^{C2}-, -NR^{C2}-O-CO-, -CO-NR^{C2}-O-, -O-NR^{C2}-CO-, -NR^{C2}-CO-NR^{C3}-, -NR^{C2}-SO₂- ou -SO₂-NR^{C2}- ;
R^{C1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{C} est une liaison simple, R^{C1} ne soit pas un atome d'hydrogène ;
chacun de R^{C2} et R^{C3} est indépendamment un atome d'hydrogène, un alkyle substitué ou non substitué, un alcényle substitué ou non substitué, ou un alcynyle substitué ou non substitué ;
quand X^{C} est -NR^{C2}-, -CO-NR^{C2}-, -CO-O-NR^{C2}-, -O-CO-NR^{C2}- ou -SO₂-NR^{C2}-, R^{C1} et R^{C2} peuvent être pris conjointement avec un atome d'azote adjacent pour former un hétérocyclyle aromatique substitué ou non substitué, ou un hétérocyclyle non aromatique substitué ou non substitué ;
quand X^{C} est -NR^{C2}-CO-NR^{C3}-, R^{C1} et R^{C3} peuvent être pris conjointement avec un atome d'azote adjacent pour former un hétérocyclyle aromatique substitué ou non substitué, ou un hétérocyclyle non aromatique substitué ou non substitué ;
q est un entier quelconque de 0 à 12 ;
R^{B} et R^{C} peuvent être pris conjointement avec un atome adjacent pour former un carbocycle aromatique, un carbocycle non aromatique, un hétérocycle aromatique ou un hétérocycle non aromatique, lequel cycle peut être substitué par un ou plusieurs R^{B} identiques ou différents ; et
chaque R^{D} est indépendamment un halogène, cyano, nitro, oxo, azoture, triméthylsilyle ou -X^{D}-R^{D1} ;
X^{D} est une liaison simple, -O-, -S-, -NR^{D2}-, =N-, -CO-, -SO₂-, -O-CO-, -CO-O-, -NR^{D2}-CO-, -CO-NR^{D2}-, -NR^{D2}-CO-O-, -CO-O-NR^{D2}-, -O-CO-NR^{D2}-, NR^{D2}-O-CO-, -CO-NR^{D2}-O-, -O-NR^{D2}-CO-, -NR^{D2}-CO-NR^{D3}-, -NR^{D2}-SO₂- ou -SO₂-NR^{D2}- ;
R^{D1} est un atome d'hydrogène, un alkyle substitué ou non substitué, un alcényle substitué ou non substitué, un alcynyle substitué ou non substitué, un carbocyclyle aromatique substitué ou non substitué, un carbocyclyle non aromatique substitué ou non substitué, un hétérocyclyle aromatique substitué ou non substitué, ou un hétérocyclyle non aromatique substitué ou non substitué, sous réserve que, lorsque X^{D} est une liaison simple, R^{D1} ne soit pas un atome d'hydrogène ;
chacun de R^{D2} et R^{D3} est indépendamment un atome d'hydrogène, un alkyle substitué ou non substitué, un alcényle substitué ou non substitué, ou un alcynyle substitué ou non substitué ;
quand X^{D} est -NR^{D2}-, -CO-NR^{D2}-, -CO-O-NR^{D2}-, -O-CO-NR^{D2}- ou -SO₂-NR^{D2}-, R^{D1} et R^{D2} peuvent être pris conjointement avec un atome d'azote adjacent pour former un hétérocyclyle aromatique substitué ou non substitué, ou un hétérocyclyle non aromatique substitué ou non substitué ;
quand X^{D} est -NR^{D2}-CO-NR^{D3}-, R^{D1} et R^{D3} peuvent être pris conjointement avec un atome d'azote adjacent pour former un hétérocyclyle aromatique substitué ou non substitué, ou un hétérocyclyle non aromatique substitué ou non substitué ;
r est un entier quelconque de 0 à 12 ;
R^{C} et R^{D} peuvent être pris conjointement avec un atome adjacent pour former un carbocycle aromatique, un carbocycle non aromatique, un hétérocycle aromatique ou un hétérocycle non aromatique, lequel cycle peut être substitué par un ou plusieurs R^{C} identiques ou différents.

5. Composé selon la revendication 4 ou son sel pharmaceutiquement acceptable, dans lequel le cycle A est le cycle suivant :

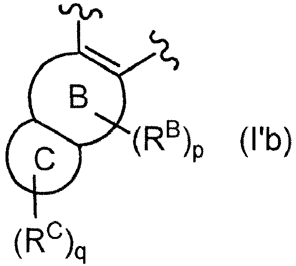

6. Composé selon l'une quelconque des revendications 3 à 5 on son sel pharmaceutiquement acceptable, dans lequel p vaut un ou plus.

7. Composé selon la revendication 3 ou 6, représenté par la formule (I"a) : 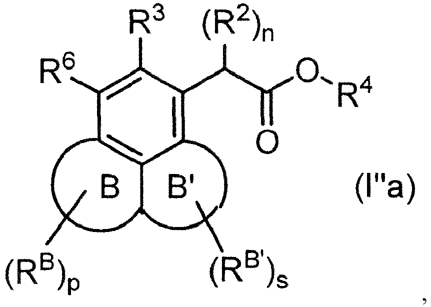 ou son sel pharmaceutiquement acceptable,
dans laquelle le cycle B' est un carbocycle aromatique monocyclique, un carbocycle non aromatique monocyclique, un hétérocycle aromatique monocyclique, ou un hétérocycle non aromatique monocyclique ; p est un entier quelconque de 0 à 11 ; s est un entier quelconque de 0 à 11.

8. Composé selon la revendication 1, représenté par l'une quelconque des formules suivantes : 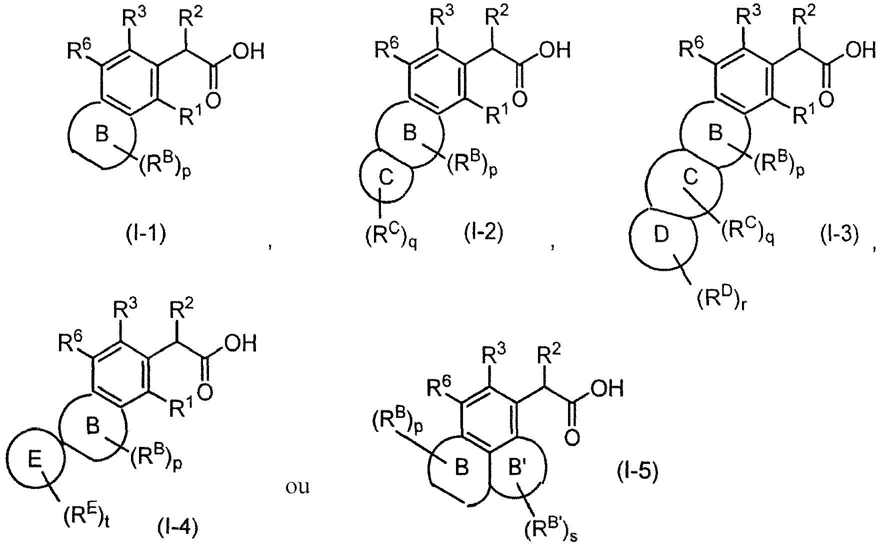 ou son sel pharmaceutiquement acceptable,
dans lesquelles
R¹ est un alkyle ;
R² est un alkyloxy, ou un cycloalkyloxy substitué ou non substitué ;
R³ est tel que défini dans la revendication 1 ;
R⁶ est un alkyle ;
chaque cycle B est indépendamment un hétérocycle aromatique monocyclique ou un hétérocycle non aromatique monocyclique, chaque cycle C, cycle D, cycle E et cycle B' est indépendamment un carbocycle aromatique monocyclique, un carbocycle non aromatique monocyclique, un hétérocycle aromatique monocyclique ou un hétérocycle non aromatique monocyclique ;
le cycle E a la même définition que le cycle C ;
chacun de R^{B'} et R^{E} a indépendamment la même signification que R^{C} ;
t est un entier quelconque de 0 à 12 ;
les autres ont les mêmes significations que celles décrites dans la revendication 4.

9. Composé selon la revendication 1, représenté par l'une quelconque des formules suivantes : 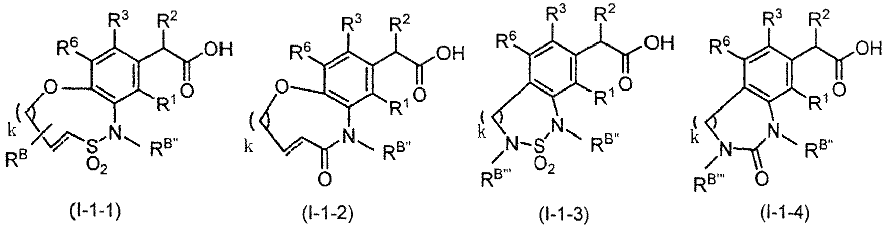 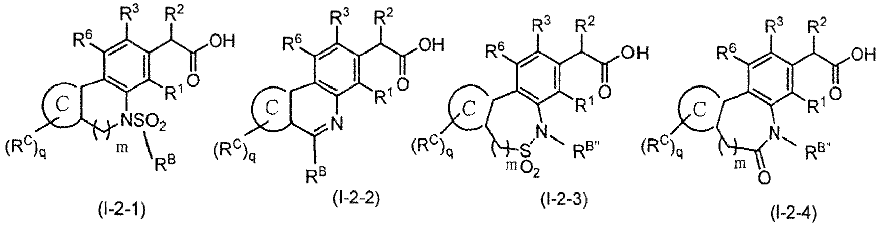 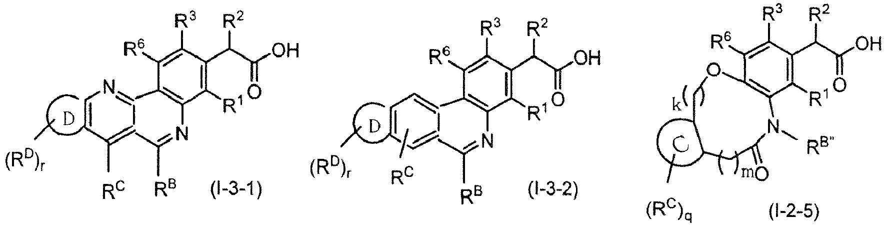 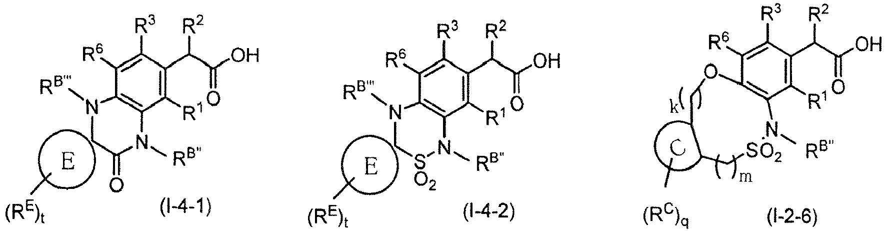 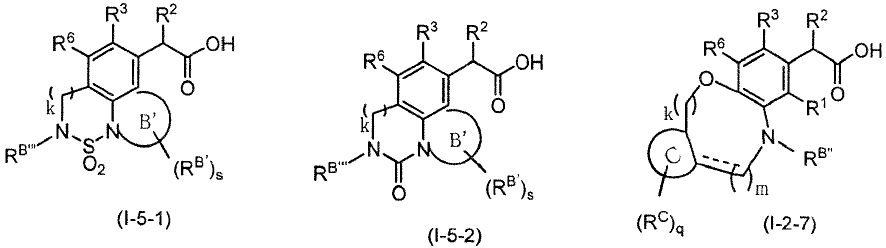 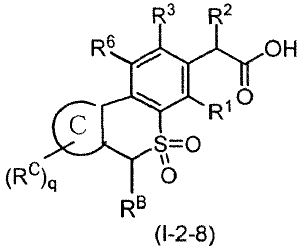 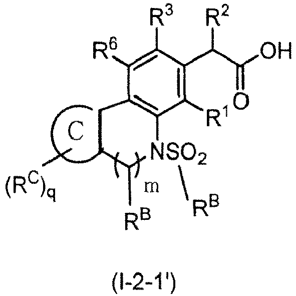 ou son sel pharmaceutiquement acceptable,
dans lesquelles
chacun du cycle C et du cycle D est indépendamment un benzène ou un hétérocycle à 5 à 8 chaînons ;
le cycle B' est un hétérocycle à 5 à 8 chaînons ;
le cycle E est un carbocycle à 5 à 8 chaînons éventuellement réticulé ;
chacun de R^{B"} et R^{B'''} est indépendamment un atome d'hydrogène, un alkyle, cycloalkyle, cycloalkylalkyle, formyle, alcoxycarbonyle, aryle substitué ou non substitué, aralkylaralkyle substitué ou non substitué, hétérocyclyle substitué ou non substitué, hétérocyclylalkyle substitué ou non substitué, ou sulfonyle substitué ;
la ligne de tirets signifie la présence ou l'absence d'une liaison ;
chacun de k et m est indépendamment un entier de 0 à 4 ;
les autres ont les mêmes significations que celles décrites dans la revendication 8.

10. Composé selon la revendication 9, représenté par la formule (I-2-1), (I-2-2), (I-2-3) ou (I-2-4), ou son sel pharmaceutiquement acceptable.

11. Composition pharmaceutique comprenant le composé selon l'une quelconque des revendications 1 à 10 ou son sel pharmaceutiquement acceptable.

12. Composition pharmaceutique selon la revendication 11, ayant une activité anti-VIH.

13. Composé selon l'une quelconque des revendications 1 à 10 ou son sel pharmaceutiquement acceptable, pour une utilisation dans le traitement ou la prévention d'une infection par VIH.
